# EUROPEAN PATENT APPLICATION

(11) **EP 1 619 504 A1**
(43) Date of publication of application: **25.01.2006**
(21) Application number: 04016948.4
(22) Date of filing: 19.07.2004
(51) Int. Cl.: G01N 33/68, C12Q 1/68

(54) **Novel use of the kinases RSK4 and PAK5**

(71) Applicant: Fraunhofer Institut für Toxikologie und Experimentelle Medizin, 30625 Hannover (DE)
(72) Inventor: Borlak, Jürgen, Prof. Dr., 31275 Lehrte/OT Immensen (DE); Niehof, Monika, Dr., 30161 Hannover (DE)
(74) Representative: Baumbach, Friedrich

(57) **Abstract**

Type 2 diabetes is one of the most common diseases. In the year 2030 366 million patients are expected, which is a doubling in comparison to the year 2000. Besides a defective regulation of the glucose metabolism patients suffer from diseases of the kidney and the brain, which are named as diabetic nephropathies and diabetic neuropathies, respectively. Worldwide large efforts are undertaken to understand the causes of this disease. There is evidence, that a dysfunctional HNF4α plays an important role in the onset of diabetes. With the help of molecular biological and molecular genetic methods new HNF4α target genes (RSK4 and PAK5) were identified. These two genes encode for kinases. In a diabetic animal model RSK4 and PAK5 were selectively regulated. The identification of these genes and their fundamental importance for a therapy of type 1 and/or type 2 diabetes and/or nephropathies and/or neuropathies, including diabetic nephropathies and diabetic neuropathies is described. This invention has a high economic potential, since agents can now be identified for the control of genes, which regulation is disturbed by these diseases. Therefore new therapeutic concepts are possible for the treatment of type 1 and/or type 2 diabetes and/or nephropathies and/or neuropathies, including diabetic nephropathies and diabetic neuropathies.

## Description

The invention concerns a novel use of the kinases RSK4 and PAK5 to screen for and to identify anti-type 1 and anti-type 2 diabetes mellitus and/or anti-nephropathic and/or anti-neuropathic drugs, including anti-diabetic nephropathic and anti-diabetic neuropathic drugs.

### BACKGROUND OF THE INVENTION

Diabetes mellitus is among the most common of all metabolic disorders, affecting up to 11% of flue population by age 70.

Type 1 diabetes (insulin dependent diabetes mellitus or IDDM) represents about 5 to 10% of this group and is the result of a progressive autoimmune destruction of the pancreatic cells with subsequent insulin deficiency.

Type 2 (non-insulin dependent diabetes mellitus or NIDDM) diabetes is one of the most common diseases. In the year 2030 366 million patients are expected, which is a doubling in comparison to the year 2000. This type represents 90-95% of the affected population more than 100 million people worldwide (King, H. and Zimmer, P. *Wld Hlth. Statist. Quart.* 41, 190-196 (1998)), and is associated with peripheral insulin resistance, elevated hepatic glucose production, and inappropriate insulin secretion (DeFronzo, R.A., *Diabetes* 37, 667-687 (1988)). Besides a defective regulation of the glucose metabolism patients suffer from after effect diseases of the kidney and the brain, which are named as diabetic nephropathies and diabetic neuropathies, respectively. Family studies point to a major genetic component (Newman, B., et al.

*Diabetologia* **30**, 763-768 (1987)). However, few susceptibility genes have been identified. Worldwide large efforts are undertaken to understand the causes of this disease. There is evidence, that a dysfunctional HNF4α plays an important role in the onset of diabetes.

The orphan nuclear receptor HNF4α is an essential transcription factor for liver development and hepatocellular differentiation (Chen, W. S. *et al.* Disruption of the HNF-4 gene, expressed in visceral endoderm, leads to cell death in embryonic ectoderm and impaired gastrulation of mouse embryos. *Genes Dev* **8**, 2466-2477 (1994); Li, J., Ning, G., & Duncan, S. A. Mammalian hepatocyte differentiation requires the transcription factor HNF-4alpha. *Genes Dev* **14**, 464-474 (2000); Hayhurst, G. P., Lee, Y. H., Lambert, G., Ward, J. M., & Gonzalez, F. J. Hepatocyte nuclear factor 4alpha (nuclear receptor 2A1) is essential for maintenance of hepatic gene expression and lipid homeostasis. *Mol Cell Biol* **21**, 1393-1403 (2001); Parviz, F. *et al.* Hepatocyte nuclear factor 4 alpha, controls the development of a hepatic epithelium and liver morphogenesis. *Nat Genet* **34,** 292-296 (2003)) and plays an essential role in a transcription factor regulatory circuit (Schrem, H., Klempnauer, J., & Borlak, J. Liver-enriched transcription factors in liver function and development. Part I: the hepatocyte nuclear factor network and liver-specific gene expression. *Pharmacol.Rev* **54,** 129-158 (2002)) to control a wide range of metabolic processes. This factor also targets genes in other organs including kidney, intestine and colon (Sladek, F. M. & Seidel, S. D. Hepatocyte nuclear factor 4 alpha in *Nuclear Receptors and Disease. Academic Press, London.* 309-361. (2001)), promotes expression of an epithelial phenotype, triggers de novo formation of functional tight junctions and contributes to epithelial cell polarity (Chiba, H. *et al.* Hepatocyte nuclear factor (HNF)-4alpha triggers formation of functional tight junctions and establishment of polarized epithelial morphology in F9 embryonal carcinoma cells. *Exp Cell Res* **286**, 288-297 (2003)). HNF4α dysfunction is associated with metabolic disorders including diabetes, whereas patients with diabetes harbor a high risk for nephropathies and neuropathies.

### SUMMARY OF THE INVENTION

With the help of molecular biological and molecular genetic methods new HNF4α target genes (RSK4 and PAK5) were identified. These two genes encode for kinases. In a diabetic animal model RSK4 and PAK5 were selectively regulated. The identification of these genes and their fundamental importance for a therapy of type 1 and/or type 2 diabetes and/or nephropathies and/or neuropathies, including diabetic nephropathies and diabetic neuropathies is described. This invention has a high economic potential, since agents can now be identified for the control of genes, which regulation is disturbed by these diseases. Therefore new therapeutic concepts are possible for the treatment of type 1 and/or type 2 diabetes and/or nephropathies and/or neuropathies, including diabetic nephropathies and diabetic neuropathies.

In one aspect, the invention features compounds that are agonists of a normal (functional) bioactivity of the kinases RSK4 and PAK5 and their use in treating type 1 and/or type 2 diabetes and/or nephropathies and/or neuropathies, including diabetic nephropathies and diabetic neuropathies. For example, to ameliorate disease symptoms involving insufficient expression of a RSK4 gene and/or a PAK5 gene and/or inadequate amount of functional RSK4 and/or PAK5 bioactivity in a subject, a gene therapeutic (comprising a gene encoding a functional RSK4 or PAK5 protein) or a protein therapeutic (comprising a functional RSK4 and/or PAK5 protein) can be administered to the subject.

In another aspect, the invention features compounds that are antagonists of a disease causing RSK4 or PAK5 bioactivity and their use in treating type 1 and/or type 2 diabetes and/or nephropathies and/or neuropathies, including diabetic nephropathies and diabetic neuropathies.

For example to ameliorate disease symptoms involving expression of a mutant RSK4 or PAK5 gene or overexpression of a normal RSK4 or PAK5 gene in a subject, a therapeutically effective amount of an antisense, ribozyme or triple helix molecule to reduce or prevent gene expression, as described herein, may be administered to the subject. Alternatively, to ameliorate disease symptoms involving the regulation via an RSK4 or PAK5 protein of an upstream or downstream element in a RSK4 and/or PAK5 mediated biochemical pathway (e.g. signal transduction), a therapeutically effective amount of an antagonist compound (e.g. small molecule, peptide, or peptidomimetic) that can prevent binding of the wildtype RSK4 and/or PAK5 protein, can induce a therapeutic effect. Further, to ameliorate disease symptoms involving a mutant (nonfunctional) RSK4 and/or PAK5 protein, a therapeutically effective amount of an anti-RSK4 antibody and/or anti-PAK5 antibody, as described herein, may be administered to the subject.

In yet another aspect, the invention provides assays, e.g., for screening test compounds to identify antagonists (e.g. inhibitors), or alternatively, agonists (e.g. potentiators), of an interaction between a RSK4 and/or PAK5 protein and, for example, a protein or nucleic acid that binds to the RSK4 and/or PAK5 protein. An exemplary method includes the steps of (i) combining a RSK4 or PAK5 polypeptide or bioactive fragments thereof, a RSK4 and/or PAK5 target molecule (such as a RSK4 and/or PAK5 ligand or nucleic acid), and a test compound, e.g., under conditions wherein, the test compound, the RSK4 and/or PAK5 protein and RSK4 and/or PAK5 target molecule are able to interact; and (ii) detecting the formation of a complex which includes the RSK4 and/or PAK5 protein and the target molecule either by directly quantitating the complex or by measuring inductive effects of the RSK4 and/or PAK5 protein. A statistically significant change, such as a decrease, in the interaction of the RSK4 and/or PAK5 and RSK4 and/or PAK5 target molecule in the presence of a test compound (relative to what is detected in the absence of the test compound) is indicative of a modulation (e.g., inhibition or potentiation of the interaction between the RSK4 and/or PAK5 protein and the target molecule).

Positive tested compounds can be used as anti-type 1 and anti-type 2 diabetes mellitus and/or anti-nephropathic and/or anti-neuropathic drugs, including anti-diabetic nephropathic and anti-diabetic neuropathic drugs. Those drugs can regulate the kinases RSK4 and/or PAK5. Instead of the kinases RSK4 and/or PAK5 also RSK4 and/or PAK5 encoding DNA or polypeptides having RSK4 and/or PAK5 activity can be used.

Yet another aspect of the present invention concerns methods for modulating the transcription of certain genes in a cell by modulating RSK4 and/or PAK5 bioactivity, (e.g., by potentiating or disrupting a RSK4 and/or PAK5 bioactivity). In general, whether carried out in vivo, in vitro, or in situ, the method comprises treating the cell with an effective amount of a RSK4 and/or PAK5 therapeutic (agonist or antagonist of a RSK4 or PAK5 bioactivity) so as to alter, relative to the cell in the absence of treatment, the level of transcription of certain genes. Accordingly. the method can be carried out with RSK4 and/or PAK5 therapeutics such as peptide and peptidomimetics or other molecules identified in the above-referenced drug screens which agonize or antagonize the effects of a RSK4 or PAK5 bioactivity (e.g. transcription) of a gene which is regulated by a RSK4 and/or PAK5 protein. Other RSK4 or PAK5 therapeutics include antisense constructs for inhibiting expression of RSK4 and/or PAK5 proteins, and dominant negative mutants of RSK4 or PAK5 proteins which competitively inhibit interactions between ligands (e.g. proteins) and nucleic acids upstream and downstream of the wild-type RSK4 or PAK5 protein.

A further aspect of the present invention provides methods for determining whether a subject is at risk for developing type I or type 2 diabetes or nephropathies or neuropathies, including diabetic nephropathy or diabetic neuropathy. The method can include detecting, in a tissue of the subject, the presence or absence of a genetic lesion characterized by at least one of (i) a mutation of a gene encoding a RSK4 or PAK5 protein; (ii) the mis-expression of a RSK4 or PAK5 gene; or (iii) an error or mutation in the promoter regulating a RSK4 or PAK5 gene that may lead to aberrant expression. In preferred embodiments, detecting the genetic lesion includes ascertaining the existence of at least one of: a deletion of one or more nucleotides from a RSK4 or PAK5 gene; an addition of one or more nucleotides to a RSK4 or PAK5 gene, a substitution of one or more nucleotides of a RSK4 or PAK5 gene, a gross chromosomal rearrangement of a RSK4 or PAK5 gene; an alteration in the level of a messenger RNA transcript of a RSK4 or PAK5 gene; the presence of a non-wild type splicing pattern of a messenger RNA transcript of a RSK4 or PAK5 gene; a non-wild type level of a RSK4 or PAK5 protein; and/or an aberrant level of a RSK4 or PAK5 protein.

For example, detecting the genetic lesion can include (i) providing a probe/primer comprised of an oligonucleotide which hybridizes to a sense or anti sense sequence of a RSK4 and/or PAK5 gene or naturally occurring mutants thereof, or 5' or 3' flanking sequences naturally associated with the RSK4 or the PAK5 gene; (ii) contacting the probe/primer with an appropriate nucleic acid containing sample; and (iii) detecting, by hybridization of the probe/primer to the nucleic acid, the presence or absence of the genetic lesion; e.g. wherein detecting the lesion comprises utilizing the probe/primer to determine the nucleotide sequence of the RSK4 and/or the PAK5 gene and, optionally, of the flanking nucleic acid sequences. For instance, the primer can be employed in a polymerase chain reaction (PCR) or in a ligase chain reaction (LCR). In alternate embodiments, the level of a RSK4 and/or PAK5 protein is detected in an immunoassay using an antibody which is specifically immunoreactive with the RSK4 and/or the PAK5 protein. Other features and advantages of the invention will be apparent from the following detailed description and claims.

### DETAILED DISCLOSURE OF THE INVENTION

In an chromatin immunoprecipitation assay the two HNF4α target genes RSK4 and PAK5 were identified. Both proteins are kinases, which are involved in cell cycle regulation and signal transduction. Notably, HNF4α, RSK4 and PAK5 were specifically regulated in streptozotocin-induced diabetic rat kidney and brain. This provide a molecular rational for diabetic nephro- and neuropathy, frequently seen in patients with HNF4α dysfunction.

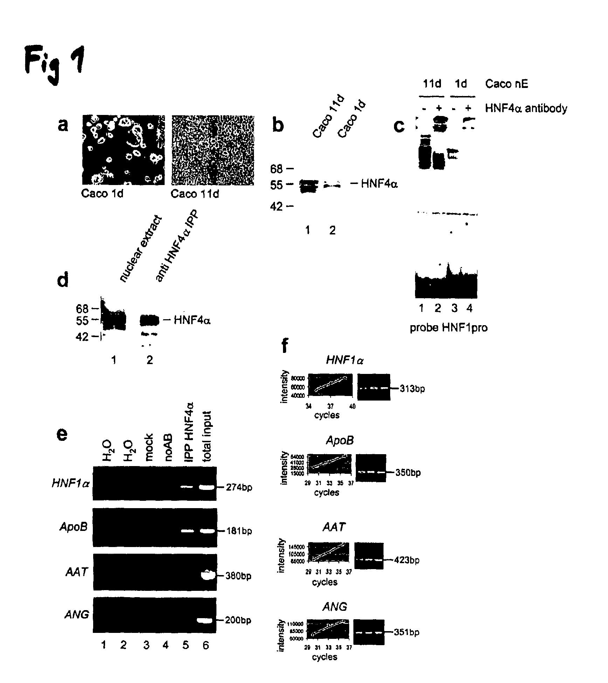

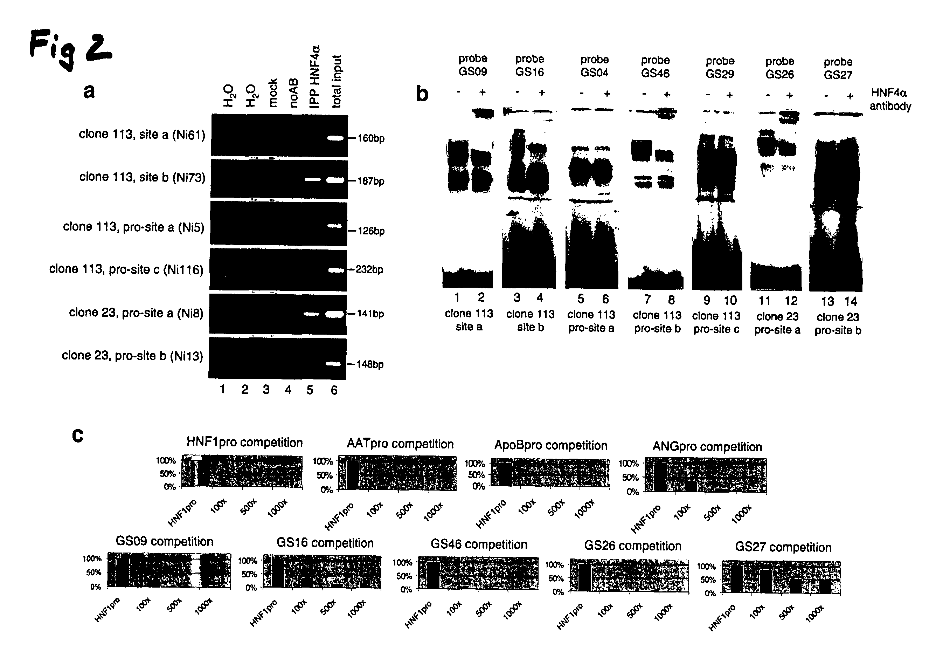

The ChIP-cloning procedure was used to identify novel HNF4α target-genes after formaldehyde crosslinking of nucleo-protein complexes in highly differentiated Caco-2 cell cultures (Hu, C. & Perlmutter, D. H. Regulation of alphal-antitrypsin gene expression in human intestinal epithelial cell line caco-2 by HNF-1alpha and HNF-4. *Am J Physiol* **276**, G1181-G1194 (1999)) **(Fig. 1a).** At day I 1 in culture, the HNF4α protein expression was abundant (**Fig. 1b**), as determined by western-blotting experiments. Further, EMSA-experiments evidenced a marked increase in HNF4α DNA-binding to the A-site of the *HNF1α*-promoter (HNFlpro) (**Fig. 1c**). The A-site is an established recognition site for HNF4α (Schrem, H., Klempnauer, J., & Borlak, J. Liver-enriched transcription factors in liver function and development. Part I: the hepatocyte nuclear factor network and liver-specific gene expression. *Pharmacol.Rev* **54**, 129-158 (2002); Sladek, F. M. & Seidel, S. D. Hepatocyte nuclear factor 4alpha in *Nuclear Receptors and Disease. Academic Press, London.* 309-361. (2001)). The ability of the HNF4α-antibody to immunoprecipitate HNF4α was confirmed by western blotting (**Fig. 1d**) and specificity of the ChIP-assay was tested by screening of immunoprecipitated DNA for enrichment of promoter sequences of well-known HNF4α target-genes (Schrem, H., Klempnauer, J., & Borlak, J. Liver-enriched transcription factors in liver function and development. Part I: the hepatocyte nuclear factor network and liver-specific gene expression. *Pharmacol.Rev* **54**, 129-158 (2002); Sladek, F. M. & Seidel, S. D. Hepatocyte nuclear factor 4alpha in *Nuclear Receptors and Disease. Academic Press, London.* 309-361. (2001)). Specifically, by PCR-amplification it was confirmed that immunoprecipitated DNA contained *HNF1α*, apolipoprotein-B *(ApoB),* α1-antitrypsin (*AAT*) and angiotensinogen (*ANG*) (**Fig. 1e**). Therefore evidence for enrichment of known HNF4α target genes in immunoprecipitated DNA and document specificity for the experimental procedure is provided. Further expression of HNF4α target genes by RT-PCR is evidenced (**Fig. 1f**) as well as for each CbIP-assay detection of the HNFlpro-site served as a positive control. Therefore the experimental approach prior to the cloning of immunoprecipitated DNA was thoroughly validated. ChIP-assays yielded clones with inserts up to 1800bp. The inserts were sequenced with vector-specific primers, and the genomic sequences were identified by database searches (GenBank, maintained by NCBI). Novel targets were distributed amongst different chromosomes, which demonstrates the utility of ChIP-cloning procedure and identified multiple chromosomal targets for this factor. Some of the cloned fragments were within intronic regions and this agrees well with finding by others (Greenbaum, S. & Zhuang, Y. Identification of E2A target genes in B lymphocyte development by using a gene tagging-based chromatin immunoprecipitation system. *Proc Natl Acad Sci U.SA* **99**, 15030-15035 (2002); Solano, P. J. *et al.* Genome-wide identification of in vivo Drosophila Engrailed-binding DNA fragments and related target genes. *Development* **130**, 1243-1254 (2003), Martone, R. *et al.* Distribution of NF-{kappa}B-binding sites across human chromosome 22. *PNAS* **100**, 12247-12252 (2003)). Proximal promoter sequences were also analyzed. Cloned fragments as well as promoter sequences were checked for putative HNF4α binding-sites and primer pairs were designed to confirm experimentally predicted sites. Independent ChIP-experiments followed by PCR-analyses with clone-specific or promoter-specific primers enabled robust identification of HNF4α target-genes.

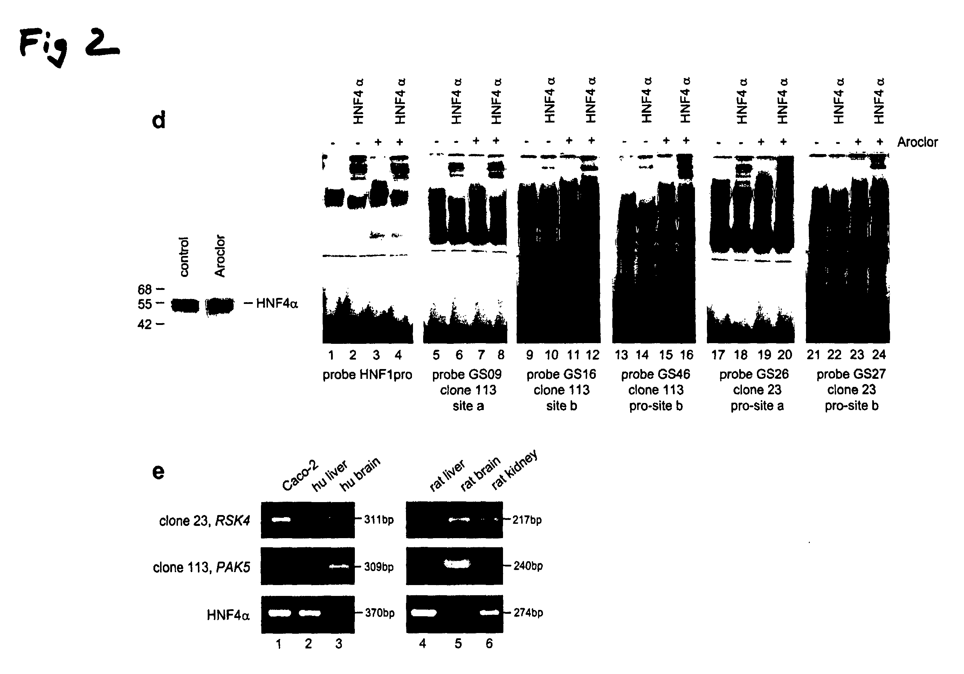

Here a detailed report of the identification of two novel kinases targeted by HNF4α is given. Binding in *vivo* of HNF4α was confirmed for two recognition sites of clone113 **(Fig. 2a).** Additionally by employing a bioinformatic approach two promoter binding-sites within clone113 and two promoter binding-sites within clone23, which were specifically bound by HNF4α in *vivo* (**Fig. 2a**) were predicted. Further the ability of HNF4α to bind to cognate recognition sites by EMSA with ³²P-labeled probes encompassing the predicted HNF4α-sites located in clone113 (GS09, GS16), in the promoter of clone113 (GS04, GS29) and in the promoter of clone23 (GS26, GS27) (**Fig. 2b**) was studied. Supershift experiments with a specific HNF4α antibody resulted in strong binding of HNF4α with probe GS09, GS16 and GS26 and weaker binding with probe GS27. No binding of HNF4α was detected with probe GS04 and GS29. Competition experiments with probes specific for several HNF4α target-genes were carried out to estimate binding affinity of HNF4α to the newly identified targets (**Fig. 2c**). The HNF I pro-site was used as labeled probe and competition was first analyzed with different known cognate recognition-sites, namely HNFlpro itself and HNF4α binding-sites within AATpro, ApoBpro and ANGpro. These sites were distinguishable in their binding affinity (**Fig. 2c**). With the AATpro-site (100x, inhibition to 10%) and the ApoB-site (100x, inhibition to 2.5%) binding was comparable to the HNFlpro-site itself (100x, inhibition to 2.2%), whereas competition with the ANGpro-site (100x, inhibition to 34,6%) was less efficient. By comparison, competition with the GS09 (100x, inhibition to 13.7%) and the GS26 (100x, inhibition to 9.5%) resulted in strong binding, whereas competition with the GS16 (100x, inhibition to 32.5%) was less efficient and binding affinity with the GS27 (100x, inhibition to 85.8%) was minimal. Competition experiments therefore complement findings obtained by supershift experiments. Clone 113 itself and two sites in the promoter of clone23 were thus confirmed for in vivo and in *vitro* binding of HNF4α. Notably, HNF4α may contact DNA through various protein-protein interactions, particularly through cooperative binding with synergized factors as formaldehyde crosslinks leads to both, protein-DNA and protein-protein complexes. Therefore in ChIP-experiments a three-dimensional, higher order structure can be crosslinked. HNF4α in *vitro* binding to the putative sites (pro-site a and pro-site c) in the promoter of clone 113 was not confirmed, but the surrounding regions of 126bp and 232bp were confirmed as in *vivo* binding sites (see **Fig. 2a**). The promoter harbors an adjacent HNF4α binding-site (pro-site b, GS46, localized about 800bp upstream to site a and about 400bp downstream to site c), exhibiting *strong in vitro* binding of HNF4α (**Fig 2b+c**), which would allow for immunoprecipitation of pro-site a and pro-site c through protein-protein crosslinks. Additionally, HNF4α may also contact another site within *the in vivo* confirmed fragments, which was not predicted by our computational approach.

Further, gene regulation of a broad range of CYP isozymes depends on promoter activation by HNF4α (Jover, R., Bort, R., Gomez-Lechon, M. J., & Castell, J. V. Cytochrome P450 regulation by hepatocyte nuclear factor 4 in human hepatocytes: a study using adenovirus-mediated antisense targeting. *Hepatology* **33**, 668-675 (2001)). Notably, treatment of hepatocytes with Aroclor 1254 resulted in induction of HNF4α and several CYP monooxgenases (Borlak, J. & Thum, T. Induction of nuclear transcription factors, cytochrome P450 monooxygenases, and glutathione S-transferase alpha gene expression in Aroclor 1254-treated rat hepatocyte cultures. *Biochemical Pharmacology* **61**, 145-153 (2001)). Therefore HNF4α-binding in liver nuclear extracts of control and Aroclor treated rats were analyzed by EMSA. Binding of HNF4α to HNFlpro as well as to the newly identified binding-sites (GS09, GS16, GS46, GS26, GS27) was significantly increased after Aroclor treatment (**Fig. 2d**), thus providing further evidence for our novel targets to be strictly regulated by HNF4α.

A summary of the cloned HNF4α targets is given in **Table 1.** Clone23 contained two ChIP-verified HNF4α binding-sites in the promoter region (around -1430 and -2053) and was identified as *RSK4,* a novel member of ribosomal S6-kinase-subfamily (Yntema, H. G. *et al.* A Novel Ribosomal S6-Kinase (RSK4; RPS6KA6) Is Commonly Deleted in Patients with Complex X-Linked Mental Retardation. *Genomics* **62**, 332-343 (1999); Kohn, M., Hameister, H., Vogel, M., & Kehrer-Sawatzki, H. Expression pattern of the Rsk2, Rsk4 and Pdkl genes during murine embryogenesis. *Gene Expr.Patterns.* **3**, 173-177 (2003)). Because RSK4 gene deletion was found in some patients with X-linked mental retardation, a role for this kinase in neuronal development was suggested (Yntema, H. *G. et al.* A Novel Ribosomal S6-Kinase (RSK4; RPS6KA6) Is Commonly Deleted in Patients with Complex X-Linked Mental Retardation. *Genomics* **62**, 332-343 (1999)). RSK4 expression during mouse development is, however, ubiquitous (Kohn, M., Hameister, H., Vogel, M., & Kehrer-Sawatzki, H. Expression pattern of the Rsk2, Rsk4 and Pdk1 genes during murine embryogenesis. *Gene Expr.Patterns. 3,* 173-177 (2003)), which suggests additional roles for this gene in development. Clone113 with two ChIP-verified HNF4α promoter binding-sites around -951 and around -2181 was annotated as *PAK5.* This kinase was recently cloned and characterized as a novel member of mammalian p2l(cdc42/racl)-activated-kinase-subfamily (Dan, C., Nath, N., Liberto, M., & Minden, A. PAK5, a New Brain-Specific Kinase, Promotes Neurite Outgrowth in N1E-115 Cells. *Mol.Cell.Biol.* **22**, 567-577 (2002); Pandey, A. *et al.* Cloning and characterization of PAK5, a novel member of mammalian p21-activated kinase-II subfamily that is predominantly expressed in brain. *Oncogene* **21**, 3939-3948 (2002)). Until now, its role was confined to induction of neurite outgrowth (Dan, C., Nath, N., Liberto, M., & Minden, A. PAK5, a New Brain-Specific Kinase, Promotes Neurite Outgrowth in N1E-115 Cells. *Mol.Cell.Biol.* **22**, 567-577 (2002)), whereas PAK kinases in general play a role in neurodegenerative diseases (Kumar, R. & Vadlamudi, R. K. Emerging functions of p21-activated kinases in human cancer cells. *J Cell Physiol **193**, 133-144* (2002)).

It is of considerable importance that HNF4α targets kinases important in neuronal development and function. Thus, next to its role in liver metabolism, HNF4α may also play a role in brain function. Specifically, HNF4α regulates several genes involved in glucose metabolism (Schrem, H., Klempnauer, J., & Borlak, J. Liver-enriched transcription factors in liver function and development. Part I: the hepatocyte nuclear factor network and liver-specific gene expression. *Pharmacoi.Rev* **54**, 129-158 (2002); Sladek, F. M. & Seidel, S. D. Hepatocyte nuclear factor 4alpha in *Nuclear Receptors and Disease. Academic Press, London.* 309-361. (2001)) and participates in the glucose-dependent insulin secretory pathways. HNF4α dysfunction does, however, lead to multifactorial Type 2 diabetes (Silander, K *et al.* Genetic Variation Near the Hepatocyte Nuclear Factor-4alpha Gene Predicts Susceptibility to Type 2 Diabetes. *Diabetes 53,* 1141-1149 (2004); Love-Gregory, L. D. *et al.* A Common Polymorphism in the Upstream Promoter Region of the Hepatocyte Nuclear Factor-4alpha Gene on Chromosome 20q Is Associated With Type 2 Diabetes and Appears to Contribute to the Evidence for Linkage in an Ashkenazi Jewish Population. *Diabetes* **53**, 1134-1140 (2004)) with patients developing diabetic neuropathies. Moreover, one form of a rare monogenetic disorder termed maturity onset diabetes of the young (MODY) was mapped to mutations within the HNF4α gene (MODY-1), thus confirming its role in pancreatic β-cell function (Schrem, H., Klempnauer, J., & Borlak, J. Liver-enriched transcription factors in liver function and development. Part I: the hepatocyte nuclear factor network and liver-specific gene expression. *Pharmacol.Rev* **54**, 129-158 (2002); Sladek, F. M. & Seidel, S. D. Hepatocyte nuclear factor 4alpha in *Nuclear Receptors and Disease. Academic Press, London.* 309-361. (2001)). Patients with diabetes harbor a high risk for progressive neuropathies for uncertain reasons (Vinik, A. I., Park, T. S., Stansbeny, K. B., & Pittenger, G. L. Diabetic neuropathies. *Diabetologia* **43**, 957-973 (2000)). Though the precise role of HNF4α in brain function is unknown, gene expression of this transcription factor in human and rat brain is demonstrated (**Fig. 2e**). Interestingly, expression of the splice variant HNF4α7 was also reported for mouse brain (Nakhei, H., Lingott, A., Lemm, I., & Ryffel, G. U). An alternative splice variant of the tissue specific transcription factor HNF4alpha predominates in undifferentiated murine cell types. *Nucleic Acids Res* **26**, 497-504 (1998)). Next to its expression in brain tissue *RSK4* is expressed at similar level in kidney, but lesser in pancreas and placenta (Yntema, H. G. *et al.* A Novel Ribosomal S6-Kinase (RSK4; RPS6KA6) Is Commonly Deleted in Patients with Complex X-Linked Mental Retardation. *Genomics* **62**, 332-343 (1999)). Gene expression of RSK4 is demonstrated in Caco-2 cells, in human and rat liver and in rat kidney (**Fig. 2e**). Unlike *RSK4, PAK5* is abundantly expressed in pancreas and minimal expressed in liver and kidney (Dan, C., Nath, N., Liberto, M., & Minden, A. PAK5, a New Brain-Specific Kinase, Promotes Neurite Outgrowth in N1E-115 Cells. *Mol.Cell.Biol.* 22, 567-577 (2002)). Expression of *PAK5* in RNA extracts of human and rat liver and rat kidney is demonstrated (**Fig. 2e**), but not in cultures of Caco-2 cells. Failure to detect *PAK5* gene expression in Caco-2 cells suggests lack of synergistic transcription factors acting in concert, even *though in vivo* binding of HNF4α to *PAK5* recognition sites was confirmed (see **Fig. 2a).**

HNF4α is a dominant regulator of the epithelial phenotype and is highly expressed in kidney (Sladek, F. M. & Seidel, S. D. Hepatocyte nuclear factor 4alpha in *Nuclear Receptors and Disease. Academic Press, London.* 309-361. (2001)). Further, in diabetic patients nephropathy is a frequently observed complication and treatment of rats with streptozotocin (STZ) results in diabetic nephro- and neuropathy (Bianchi, *R. et al.* Erythropoietin both protects from and reverses experimental diabetic neuropathy. *PNAS* **101**, 823-828 (2004), Gross, M. L. *et al.* ACE-inhibitors but not endothelin receptor blockers prevent podocyte loss in early diabetic nephropathy. *Diabetologia* **46**, 856-868 (2003)). A significant reduction of *HNF4α* gene expression in liver and *PAK5* gene expression in brain extracts of STZ-induced diabetic rats is evidenced (**Table 2**). Moreover, *HNF4α* itself, *RSK4* and *PAK5* gene expression was also significantly repressed in rat kidneys (**Table 2**). Therefore diabetic neuropathy and nephropathy is demonstrated to be linked to RSK4 and PAK5 deregulation, as a result of HNF4α dysfunction. Moreover, treatment of rats with Aroclor 1254 led to significant induction of *RSK4* mRNA in rat kidneys (**Table 2**) thus providing further evidence for a coordinate regulation of RSK4 and HNF4α gene expression.

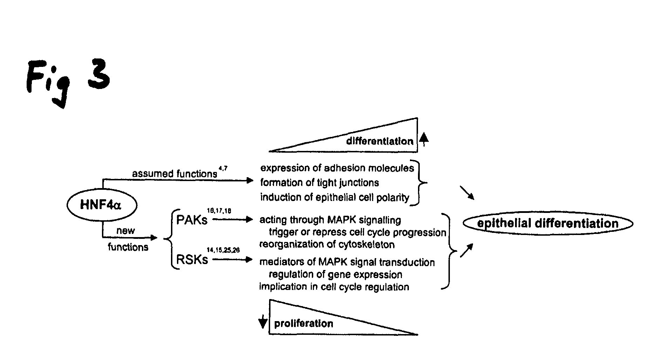

Noteworthy, RSK family members function as downstream mediators of MAP/ERK signal transducers of cell survival and cell cycle regulation (Nebreda, A. R & Gavin, A. C. Perspectives: signal transduction. Cell survival demands some Rsk. *Science* **286**, 1309-1310 (1999); Roux, P. P., Richards, S. A., & Blenis, J. Phosphorylation of p90 Ribosomal S6 Kinase (RSK) Regulates Extracellular Signal-Regulated Kinase Docking and RSK Activity. *Mol.Cell.Biol.* **23**, 4796-4804 (2003)), whereas PAK kinases play key roles in the stimulation of MAPK signaling pathways to support or to repress cell-cycle progression (Kumar, R & Vadlamudi, R. K. Emerging functions of p21-activated kinases in human cancer cells. *J Cell Physiol 193,* 133-144 (2002)). PAK kinases also regulate cytoskeletal dynamics by disassembly of stress fibers and focal adhesions (Kumar, R & Vadlamudi, R. K. Emerging functions of p21-activated kinases in human cancer cells. *J Cell Physiol* **193,** 133-144 (2002)). Thus, RSKs and PAKs are positive and negative regulators of cell cycle. Conceivably, cellular differentiation, as fostered by HNF4α, is opposite to cell proliferation. In conclusion, HNF4α has a unique role in targeting RSK and PAK family-members. This suggests a novel role for this liver enriched transcription factor in repressing cell-cycle progression to enable cellular differentiation **(Fig.3).** This provides the missing link between HNF4α dysfunction and late stage complications in diabetes frequently seen in patients with progressive stages of disease.

### METHODS

Experiments were performed after the following methods.

### Caco-2 cell culture:

Caco-2 cells were obtained from and cultivated as recommended by DMSZ. Essentially, cells were cultured in DMEM supplemented with 10% FCS and 200 pg/ml penicillin, 200 gg/ml streptomycin and 615 pg/ml L-glutamic acid. Cells were used between the 5. and the 20. passage and were checked for purity and morphological abnormalities by phase contrast microscopy. Caco-2 cells were seeded with a density of 4 x 10⁶ cells per 75 cm² flask and harvested after 11 days.

### Isolation of nuclear extracts:

The use of animals was approved by the local government of Hannover with project license 02-548. Sprague Dawley rats (n=3) were treated with a single i.p. dose of 100mg Aroclor 1254 per kg bodyweight and killed 72 h later. Nuclear extracts from rat liver were prepared as described by Gorski, K., Carneiro, M., & Schibler, U. Tissue-specific in vitro transcription from the mouse albumin promoter. *Cell* **47**, 767-776 (1986), whereas nuclear extracts from Caco-2 cells were isolated by a modified method of Dignam, J. D., Lebovitz, R. M., & Roeder, R. G. Accurate transcription initiation by RNA polymerase II in a soluble extract from isolated mammalian nuclei. *Nucleic Acids Res* **11**, 1475-1489 (1983). Eleven days after seeding cells were washed twice with ice-cold PBS, scraped into microcentrifuge tubes and centrifuged for 5 min at 2000 x g, 4°C. Cell pellets were resuspended in hypotonic buffer (10 mM Tris pH 7.4, 2 mM MgCl₂, 140 mM NaCl, 1 mM DTT, 4 mM Pefabloc, 1 % Aprotinin, 40 mM β-glycerophosphate, 1 mM sodiumorthovanadate and 0.5% TX 100) at 4°C for 10 min (300 µl for 1 x 10⁷ cells), transferred onto one volume of 50% sucrose in hypotonic buffer and centrifuged at 14000 x g and 4°C for 10 min. Nuclei were resuspended in Dignam C buffer (20 mM Hepes pH 7.9, 25% glycerol, 420 mM NaCl, 1.5 mM MgCl₂, 0.2 mM EDTA, 1 mM DTT, 4 mM Pefabloc, 1% Aprotinin, 40 mM β-glycerophosphate, 1 mM sodiumorthovanadate, 30 µl for 1 x 10⁷ cells) and gently shaked at 4°C for 30 min. Nuclear debris was removed by centrifugation at 14000 x g at 4°C for 10 min. The extracts were aliquoted and stored at -70°C. Protein concentrations were determined according to the method of Smith, P. K. *et al.* Measurement of protein using bicinchoninic acid. *Anal.Biochem.* **150**, 76-85 (1985).

### Western blot analysis:

Nuclear extracts or HNF4α immunoprecipitated complexes (collected with Protein G-Sepharose, Pharmacia) were separated on a 12% SDS-polyacrylamide gel and blotted onto a PVDF membrane in 25 mM Tris and 190 mM Glycin at 4°C for 3 h at 350 mA. The antibody directed against HNF4α was purchased from Santa Cruz Biotechnology (sc-8987x rabbit polyclonal IgG). The antigen-antibody complexes were visualized using the ECL detection system as recommended by the manufacturer NEN Life Science Products and recorded with Kodak IS 440 CF.

### Electrophoretic mobility shift assays:

Nuclear extracts were used as described in the figure legends. Binding buffer consisted of 25 mM HEPES, pH 7.6, 5 mM MgCl₂, 34 mM KCI, 2 mM DTT, 2 mM Pefabloc, 2% Aprotinin, 40 ng of poly (dI-dC)/µl and 100 ng of bovine serum albumin/µl. Oligonucleotides and nuclear proteins were incubated for 30 minutes on ice. Free DNA and DNA-protein complexes were resolved on a 6% polyacrylamide gel. The oligonucleotides were purchased from MWG Biotech and used as ³²P-labeled probes, for sequence information see **Supplementary Table 1**. Super shift experiments were done with an HNF4α specific antibody (sc-6556x, Santa Cruz Biotechnology).

Crosslinking and Chromatin immunoprecipitation (ChIP):
We employed two rounds of sequential chromatin immunoprecipitations (Weinmann, A. S., Bartley, S. M., Zhang, T., Zhang, M. Q., & Farnham, P. J. Use of chromatin immunoprecipitation to clone novel E2F target promoters. *Mol Cell Biol* 21, 6820-6832 (2001)) to increase purity and specificity of target DNA for ChIP-cloning and for validation of ChIP-derived clones. Other investigators employed a single immunoprecipitation step with obvious limitations for validation of targets (Tomaru, Y., Kondo, S., Suzuki, M., & Hayashizaki, Y. A comprehensive search for HNF-3[alpha]-regulated genes in mouse hepatoma cells by 60K cDNA microarray and chromatin immunoprecipitation/PCR analysis. *Biochemical and Biophysical Research Communications* **310,** 667-674 (2003); Horak, C. E. *et al*. GATA-1 binding sites mapped in the beta -globin locus by using mammalian chlp-chip analysis. *PNAS* **99**, 2924-2929 (2002)). Further, our highly sensitive PCR-assays did not always confirm exclusive HNF4α bound DNA after a single immunoprecipitation step, but rather resulted in signal enrichment compared to the no antibody control. We, therefore, performed two consecutive rounds of immunoprecipitation to obtain clean signals in relation to the no antibody controls (see **Fig. 1**). With some modifications all ChIP procedures were carried out as described by Weinmann, A. S., Bartley, S. M., Zhang, T., Zhang, M. Q., & Farnham, P. J. Use of chromatin immunoprecipitation to clone novel E2F target promoters. *Mol Cell Biol* **21**, 6820-6832 (2001). Caco-2 cells were treated with 1% formaldehyde at room temperature for 10 min under constant agitation. The reaction was stopped by the addition of glycine to obtain a final concentration of 125 mM. Cells were washed twice with ice-cold PBS, detached with trypsin and collected by centrifugation. Cells were resuspended in lysis buffer (5 mM PIPES, 85 mM KCl, 0.5% NP40 and 1x complete protease inhibitor, Roche) and incubated on ice for 20 min. The nuclei were collected by microcentrifugation and then resuspended in nuclei lysis buffer (1% SDS, 10 mM EDTA pH 8.0, 50 mM Tris-HCl pH 8.1 and 1x complete) and incubated on ice for 10 min. The samples were sonicated on ice until crosslinked chromatin was fragmented to approximately 0.2-1.6 kbp. Protein A-Sepharose CLB4 (Pharmacia) was blocked with 1 mg n-d-¹ BSA and 1 mg ml-' herring sperm DNA (Promega) and washed extensively before use. Chromatin preparations were precleared by incubation with 'blocked' Protein A-Sepbarose for lh at 4°C. The Protein A-Sepharose was removed by centrifugation and the precleared chromatin was diluted 1:3 with immunoprecipitation (IP) dilution buffer (0.01% SDS, 1.2 mM EDTA pH 8.0, 1.1% Triton X100 and 1x complete). Precleared chromatin from 2.5x 10⁷ cells was incubated with 1 µg goat polyclonal HNF4α antibody (sc 6556, Santa Cruz) or no antibody and rotated at 4°C overnight. Immunoprecipitates were recovered by incubation with a secondary antibody (rabbit anti-goat) and 'blocked' Protein A-Sepharose, washed twice with dialysis buffer (50 mM Tris-HCI pH 8.0, 2 mM EDTA, 0.2% sarkosyl) and four times with IP wash buffer (100 mM Tris-HCl pH 9.0, 500 mM LiCl, 1% NP40, 1% deoxycholic acid). Prior to the first wash, the supernatant from the reaction with no antibody was saved as total input chromatin and was processed with the eluted immunoprecipitates at the beginning of the cross-link reversal step. Elution was done with 30 µl elution buffer (1% SDS, 50 mM NaHCO₃) and samples were diluted 1:10 with IP dilution buffer. Two samples were pooled for a second immunoprecipitation step with the HNF4α antibody. After further recovering and washing steps, elution was done two times with 150 µl elution buffer each. Cross-links were then reversed by the addition of NaCl to a final concentration of 300mM, and RNA was removed by the addition of 10 µg of RNase A per sample followed by incubation at 65°C for 4-5 h. The samples were then precipitated at - 20°C overnight by addition of 2.5 volumes of ethanol and then pelleted by microcentrifugation. The samples were resuspended in 100 µl Tris-EDTA (pH 7.6), 25 µl 5x proteinase K buffer (1.25% SDS, 50 mM Tris-HCI pH 7.5, 25 mM EDTA pH 8.0) and 125 µg proteinase K (Roth) and incubated at 55°C for 2h. The cleanup of DNA was done by extraction with phenolchloroform-isoamyl alcohol (25:24: 1) and subsequent ethanol precipitation. The pellets were resuspended in 30 µl H₂O and analyzed by PCR. A mock probe, containing buffer without chromatin, was treated categorically through the whole immunoprecipitation procedure and through DNA isolation to control for DNA contamination.

PCR was conducted in a mixture containing 2µl of purified DNA or 2µl of a 1:200 dilution of the total input sample, 0.5 mM of each primer, 0.25 mM dNTP mixture, 0.625 U Thermostart-Taq (Abgene) and 1x PCR-buffer (Abgene, with 1.5 mM MgCl₂) in a total volume of 20 µl. For the PCR primers used to analyze immunoprecipitated target genes see Supplementary Table 2. The PCR-amplification products were run on a 2.0% agarose gel and analyzed by ethidium bromide staining.

We performed numerous independent ChIP-experiments but did not always identify the full complement of selected HNF4α target genes as our positive controls. Indeed, the second immunoprecipitation step resulted in very low yield of immunoprecipitated material. Further, formaldehyde crosslinking of nucleo-protein complexes did impact recognition of HNF4α bound DNA. Thus, the spectrum of HNF4α positive targets varied after immunoprecipitation. Consequently, for a given clone we demanded at least three independent confirmations from a series of independent ChIP-experiments until a target was considered to be confirmed.

### Cloning:

The immunoprecipitated DNA was treated with T4 DNA polymerase (New England Biolabs) to create blunt ends, purified, and cloned into the zero-blunt vector (Invitragen) using the zero-blunt PCR cloning kit (Invitrogen) according to the manufacturers recommendations. Colonies having inserts were identified by restriction enzyme digestion using enzymes in the polylinker.

### Sequence Analysis:

Plasmid-DNA was purified with QIAquick PCR Purification Kit (Qiagen), subjected to cycle sequencing with vector-specific primers using BigDyeTerminator v3.1 Kit and injected into ABI 3100 Genetic Analyzer (Applied Biosystems). Sequences were identified by database searches (GenBank version Build 34, maintained by NCBI) for human genomic matches. Detailed sequence information is given in **Supplementary Table 3**.

### Bioinformatic searching for BNF4α binding-sites:

Cloned fragments and respective proximal promoters (-1 to -3000 bp) were checked for putative HNF4α binding-sites with two different bioinformatic weight matrix-based tools, Transfac matrix (Biobase, www.biobase.de) V$HNF4_01 with cut-off core similarity 0.75 and matrix similarity 0.78, and Genomatix matrix (Genomatix, www.genomatox.de) V$HNF4_01 with cut-off core similarity 0.75 and matrix similarity 0.82 or V$HNF4_02 with cut-off core similarity 0.75 and matrix similarity 0.76.

### RT-PCR:

Total RNA was isolated using the nucleospin RNA Isolation Kit (Macherey-Nagel) according to the manufacturers recommendations. 4 µg total RNA from each sample was used for reverse transcription (Omniscript Reverse Transcriptase, Qiagen). PCR was done in a mixture containing a cDNA equivalent to 25ng of total RNA, 0.5 mM of each primer, 0.25 mM dNTP mixture, 0.625 U Thermostart-Taq (Abgene) and 1x PCR-buffer (Abgene, with 1.5 mM MgCl₂) in a total volume of 20 µl. PCR-reactions were carried out with a thermocycler (T3, Biometra). Reactions were done within the linear range of amplification and amplification products were separated using a 1.5 % agarose gel and stained with ethidium bromide. For detailed oligonucleotide sequence information and PCR-program see **Supplementary Table 4**.

Real-time semi-quantitative PCR: Real-time RT-PCR measurement was done with the Lightcycler (Roche Diagnostics). For detailed oligonucleotide sequence information and PCR-program see **Supplementary Table 5**. The PCR reaction was stopped after a total of 30 to 50 cycles and at the end of each extension phase, fluorescence was observed and used for quantitative measurements within the linear range of amplification. Exact quantification was achieved by serial dilution with cDNA produced from total RNA extracts using 1:5 dilution steps. Gene expression levels were normalized to *mitATPase6* as stable expressed housekeeping gene.

### Diabetic disease model:

Kidneys of streptozotocin (STZ) treated Sprague Dawley rats (6 months) were kindly provided by R. Amann. Conclusive evidence for this treatment to result in diabetic nephropathy was recently published (Gross, M. L. *et al.* ACE-inhibitors but not endothelin receptor blockers prevent podocyte loss in early diabetic nephropathy. *Diabetologia* 46, 856-868 (2003)). Further, liver, kidney and brain of STZ-treated rats (3 months) were kindly provided by P. Rösen, German Diabetes Research Institute, Düsseldorf.

### Statistics:

Data are given as means ± SD. Student's t-test or analysis of covariance (ANCOVA) with interaction between factor and covariable was applied for data statistical analysis. The results were considered significant when the p value was less than 0.05.

### EXAMPLES

In the following the invention is elucidated by embodiments.

### FIGURE LEGENDS

### Figure 1

HNF4α chromatin immunoprecipitation assay with HNF4α target genes.

(a) Phase contrast photomicrographs of cultured Caco-2 cells at day 1 and at day 11 (200x). (b) HNF4α western blotting analysis of 30 µg Caco-2 cell nuclear extracts prepared at day 1 (lane 2) or day 11 (lane 1). (c) Electrophoretic mobility shift experiment with 2,5 µg Caco-2 cell nuclear extracts prepared at day 1 (lane 3 and 4) or day 11 (lane 1 and 2) and an oligonucleotide corresponding to the A-site of the *HNF1α* promoter (HNF 1pro) as ³²P labeled probe. For supershift analysis an antibody directed against HNF4α was added (lane 2 and 4). (d) HNF4α western blot analysis of 30 µg total nuclear extract (nuclear extract, lane 1) and of HNF4α immunoprecipitated complexes (anti HNF4α IPP, lane 2) of Caco-2 cells. (e) ChIP experiments were performed with cultured Caco-2 cells and an antibody against HNF4α (IPP HNF4α, lane 5) or no antibody (noAB, lane 4). The no antibody control was used to monitor unspecific binding of DNA. Following DNA purification samples were subjected to PCR with primers designed to amplify promoters of different HNF4α positive targets. The primers annealed proximal to the HNF4α binding-sites of the *HNF1α* promoter, the apolipoprotein B (*ApoB*) promoter, the α1-antitrypsin (*AAT*) promoter, and the angiotensinogen (*ANG*) promoter, all of which are well-known HNF4α-targets. A mock probe (mock, lane 3) and a portion of the total input sample (total input, lane 6) were also examined by PCR. A mock probe, containing buffer without chromatin, was treated categorically through the whole immunoprecipitation procedure and through DNA isolation to control for DNA contamination. Routinely, two reactions containing H₂O instead of template were included in each PCR as negative control (lane 1 and 2). (f) Gene expression of *NNF1α, ApoB, AAT* and *ANG* in cultures of Caco-2 cells was analyzed by RT-PCR as detailed in the supplementary material. A linear range of amplification cycles was shown.

### Figure 2

Confirmation of ChIP clones by examination of HNF4α binding *in vivo* and *in vitro.*

(a) Independent ChIP experiments were performed with cultures of Caco-2 cells and an antibody against HNF4α (IPP HNF4α, lane 5) or no antibody (noAB, lane 4). Following DNA purification samples were subjected to PCR with primers designed for putative HNF4α binding-sites of clones and their promoters (clone 113, clone 113 pro-site a and pro-site c, clone 23 pro-site a and pro-site b). A mock probe (lane 3) and an aliquot of the total input sample (lane 6) were also examined by PCR. Routinely two reactions containing H₂O instead of template were included in each PCR as negative control (lane 1 and 2). (b) Electrophoretic mobility shift assays with 10 µg Caco-2 cell nuclear extract and oligonucleotides (GS09, lane 1 and 2; GS16, lane 3 and 4; GS04, lane 5 and 6; GS46, lane 7 and 8; GS29, lane 9 and 10; GS26, lane 11 and 12; GS27, lane 13 and 14) corresponding to putative HNF4α binding-sites within the identified clones and promoters as ³²P labeled probe. In supershift assays an antibody directed against HNF4α (+) was added (lane 2, 4, 6, 8, 10, 12, 14). (c) Competition experiments. Electrophoretic mobility shift experiments were carried out with 2,5 µg Caco-2 cell nuclear extracts and an oligonucleotide corresponding to the A-site of the *HNF1*α promoter as ³²P labeled probe. The A-site of the HNF1α promoter (HNFlpro), the HNF4α binding-site of the *al-antitrypsin* promoter (AATpro), the HNF4α binding-site of the *apolipoprotein B* promoter (ApoBpro), the C-region of the *angiotensinogen* promoter (ANGpro) and the putative HNF4α binding-sites (GS09, GS16, GS46, GS26, GS27) were added as 100 fold, 500 fold and 1000 fold molar excess. Dried gels were analyzed with a Molecular Imager (BioRad) using the Quantity One software (BioRad). HNF4α binding to the A-site of the *HNF1*α promoter as ³²P labeled probe was set to 100% and competition was quantified for each oligonucleotide. **(d)** HNF4α western blot analysis (left) of 30 µg liver nuclear extract of control and Aroclor treated rats. Electrophoretic mobility shift assay with 2,5 µg rat liver nuclear extract of control (lane 1, 2, 5, 6, 9, 10, 13, 14, 17, 18, 21, 22) or Aroclor treated (lane 3, 4, 7, 8, 11, 12, 15, 16, 19, 20, 23, 24) animals and oligonucleotides corresponding to different potential HNF4α binding-sites (HNFlpro, lane 1-4; GS09, lane 5-8; GS16, lane 9-12; GS46, lane 13-16; GS26, lane 17-20; GS27, lane 21-24) as ³²P labeled probe. In supershift assays an antibody directed against HNF4α was added (lane 2, 4, 6, 8, 10, 12, 14, 16, 18, 20, 22, 24). (e) Gene expression of clone 23 *(RSK4),* clone 113 (*PAK5*) and HNF4α was analyzed by RT-PCR in extracts of Caco-2 cell cultures (1), human liver (2), human brain (3), rat liver (4), rat brain (5) and rat kidney (6). In the case of PAK5 half of the PCR reaction was loaded on the gel for human and rat brain, in the case of HNF4α half of the PCR reaction was loaded on the gel for Caco-2 cells, human liver, rat liver and kidney.

### Figure 3

Function of HNF4α in cellular differentiation and cell cycle control.

For details see text.

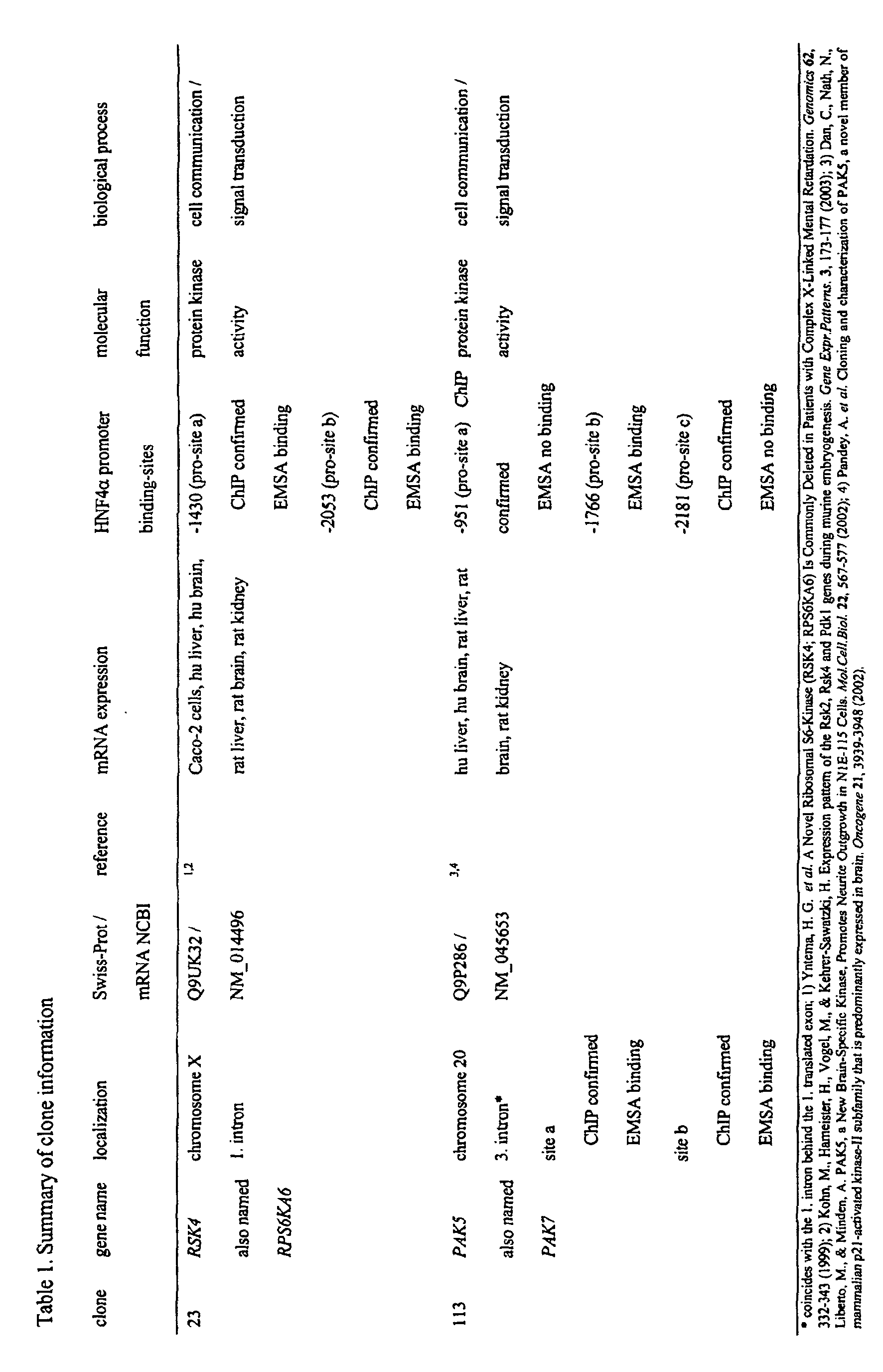

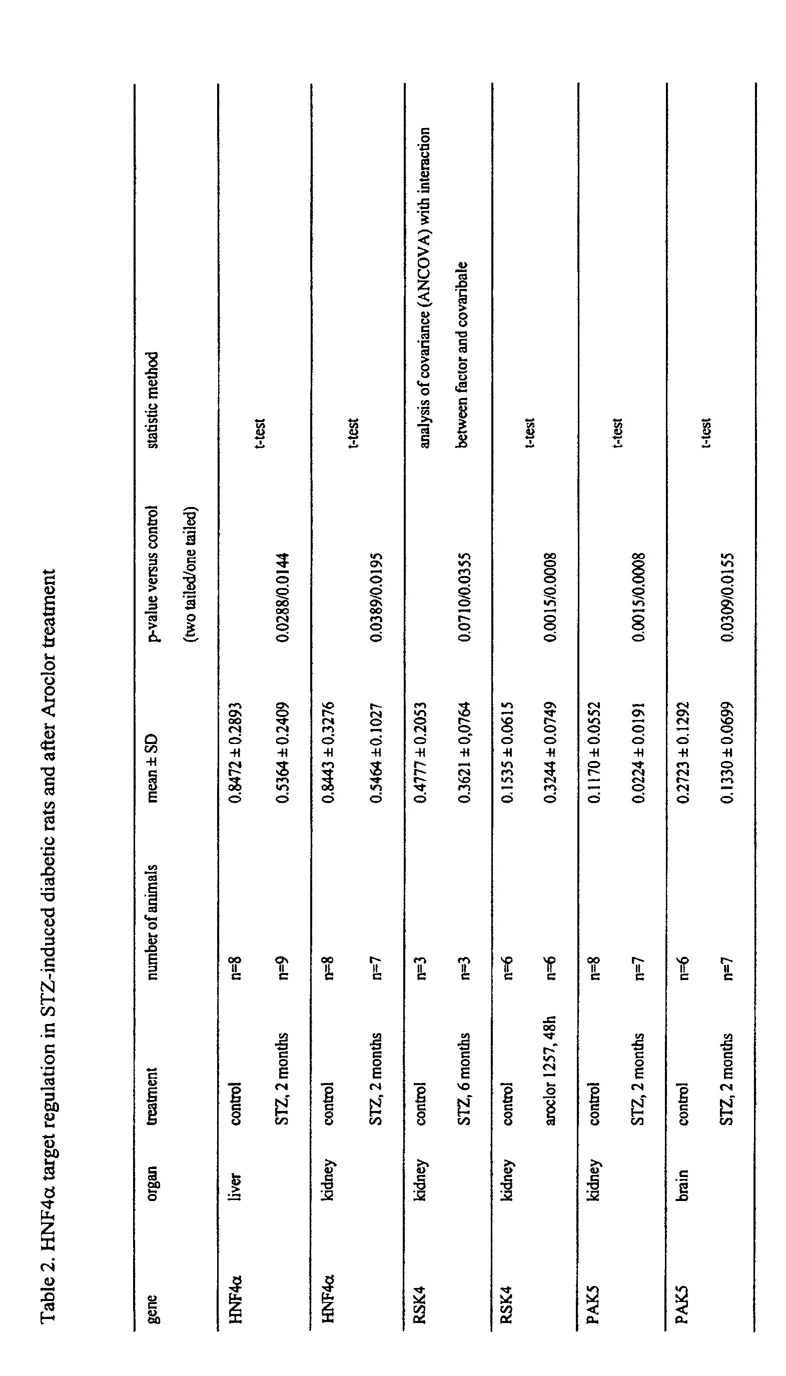

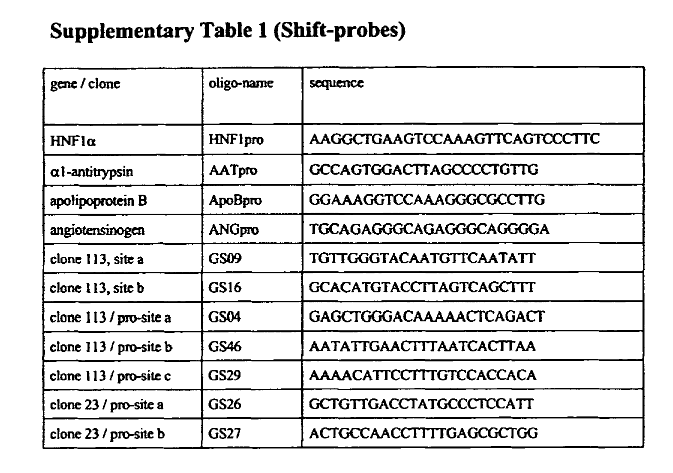

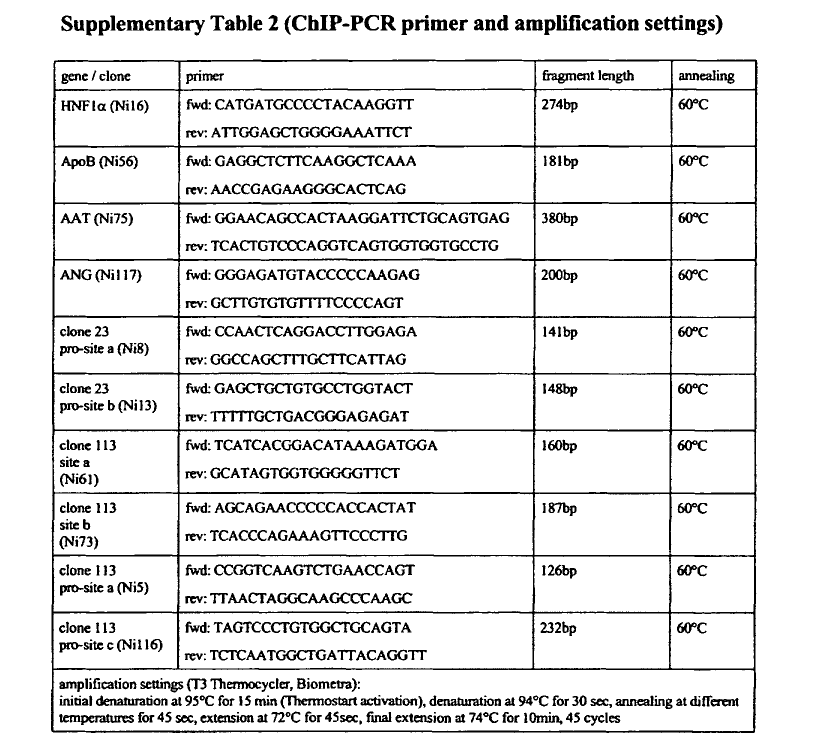

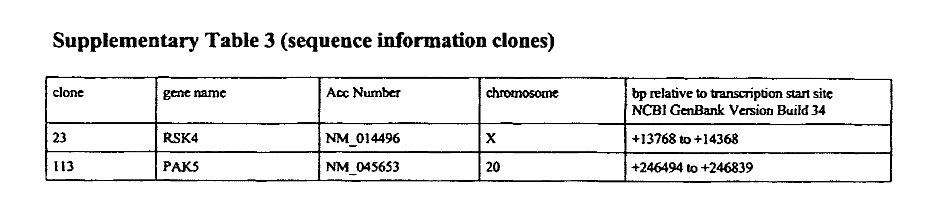

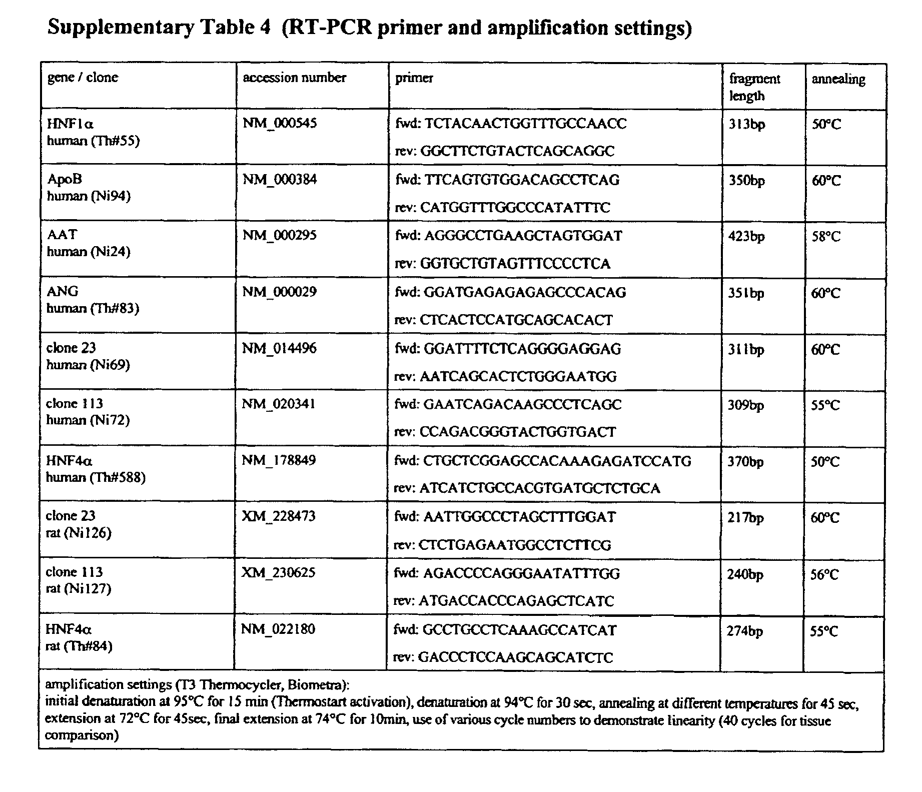

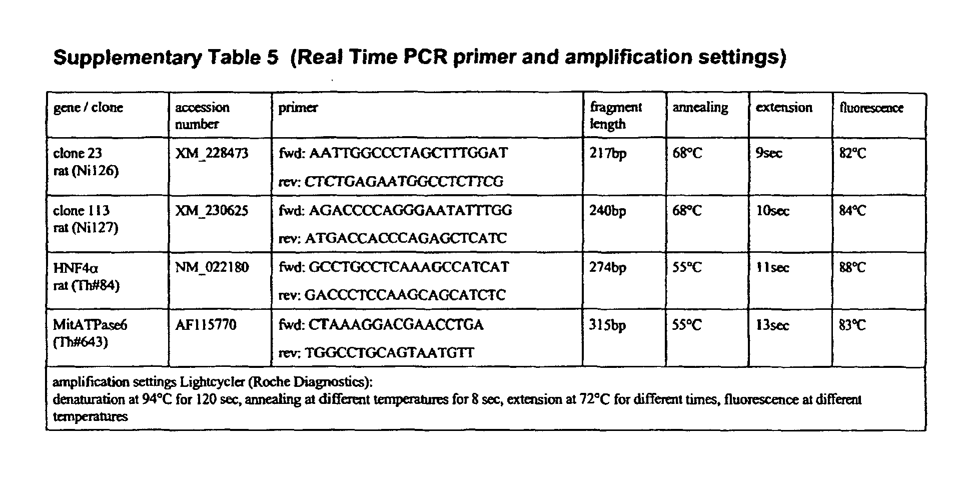

## Claims

1. Use of the kinases RSK4 and/or PAK5 to screen for and to identify anti-type 1 and anti-type 2 diabetes mellitus and/or anti-nephropathic and/or anti-neuropathic drugs, including anti-diabetic nephropathic and anti-diabetic neuropathic drugs.

2. Use as in claim 1, wherein drugs regulate the kinases RSK4 and/or PAK5 and are used for the treatment of type 1 and/or type 2 diabetes mellitus and/or nephropathies and/or neuropathies including diabetic nephropathy and diabetic neuropathy.

3. Use as claimed in claim 1 and 2, wherein RSK4 and/or PAK5 encoding DNA is used.

4. Use as claimed in claim 1 and 2, wherein a polypeptide having RSK4 and/or PAK5 activity is used.

5. Use of the kinases RSK4 and/or PAK5 for preparing a medicament for the treatment of type 1 or type 2 diabetes mellitus or for the treatment of nephropathies and/or neuropathies including diabetic nephropathy and/or diabetic neuropathy.

6. A method of identifying anti-diabetic compounds and/or anti-nephropathic compounds and/or anti-neuropathic compounds, wherein RSK4 and/or PAK5 is incubated with a compound to be tested and change in RSK4 and/or PAK5 activity is determined.

7. A method for treating type 1 or type 2 diabetes or for the treatment of nephropathies and neuropathies including diabetic nephropathy and diabetic neuropathy in a subject, comprising administering to the subject, a therapeutically effective amount of a compound that agonizes the kinases RSK4 and/or PAK5 bioactivity.

8. A method of claim 7, wherein the compound comprises a nucleic acid encoding a functional RSK4 and/or PAK5 protein.

9. A method of claim 8, wherein the compound additionally comprises an expression vector.

10. A method of claim 7, wherein the compound comprises a functional RSK4 and/or PAK5 protein.

11. A method for treating type 1 or type 2 diabetes or for the treatment of nephropathies and neuropathies including diabetic nephropathy and diabetic neuropathy in a subject, comprising administering to the subject a therapeutically effective amount of a compound that decreases or prevents expression of a mutant RSK4 and/or PAK5 bioactivity.

12. A method of claim 11, wherein the compound is selected from the group consisting of an anti sense molecule, ribozyme or triple helix molecule.

13. A method for treating type 1 or type 2 diabetes or for the treatment of nephropathies and neuropathies including diabetic nephropathy and diabetic neuropathy in a subject, comprising administering to the subject, a therapeutically effective amount of a compound that reduces the overexpression of a normal RSK4 and/or PAK5 gene.

14. A method of claim 13, wherein the compound is selected from the group consisting of: an antisense molecule, ribozyme or triple helix molecule.

15. Substances which regulate the kinases RSK4 and/or PAK5.

16. Substances according to claim 15 which regulate the kinases RSK4 and/or PAK5 to a normal (functional) level.


### Annex to the application documents - subsequently filed sequences listing

SEQUENCE LISTING

<110> Fraunhofer Institut für Toxikologie und Experimentelle Medizin

<120> Novel use of the kinases RSK4 and PAK5

<130> Fra 07/EP

<140> EP04016948.4
<141> 2004-07-19

<160> 4

<170> PatentIn version 3.1

<210> 1
<211> 126932
<212> DNA
<213> Homo sapiens

<220>
<221> promoter
<222> (1)..(3000)
<223>


<220>
<221> exon 1
<222> (3001)..(3089)
<223>


<220>
<221> intron 1
<222> (3090)..(26520)
<223>


<220>
<221> exon 2
<222> (26521)..(26580)
<223>


<220>
<221> intron 2
<222> (26581)..(34716)
<223>


<220>
<221> exon 3
<222> (34717)..(34833)
<223>


<220>
<221> intron 3
<222> (34834)..(42784)
<223>


<220>

<221>  exon 4
<222>  (42785)..(42866)
<223>

<220>
<221>  intron 4
<222>  (42867)..(43849)
<223>

<220>
<221>  exon 5
<222>  (43850)..(43930)
<223>

<220>
<221>  intron 5
<222>  (43931)..(45350)
<223>

<220>
<221>  exon 6
<222>  (45351)..(45430)
<223>

<220>
<221>  intron 6
<222>  (45431)..(55697)
<223>

<220>
<221>  exon 7
<222>  (55698)..(55804)
<223>

<220>
<221>  intron 7
<222>  (55805)..(56088)
<223>

<220>
<221>  exon 8
<222>  (56089)..(56126)
<223>

<220>
<221>  intron 8
<222>  (56127)..(70880)
<223>

<220>
<221>  exon 9
<222>  (70881)..(71023)
<223>

<220>
<221>  intron 9
<222>  (71024)..(73453)
<223>

<220>
<221>  exon 10
<222>  (73454)..(73524)
<223>

<220>
<221>  intron 10
<222>  (73525)..(73759)
<223>

<220>
<221>  exon 11
<222>  (73760)..(73848)
<223>

<220>
<221>  intron 11
<222>  (73849)..(74620)
<223>

<220>
<221>  exon 12
<222>  (74621)..(74679)
<223>

<220>
<221>  intron 12
<222>  (74680)..(83182)
<223>

<220>
<221>  exon 13
<222>  (83183)..(83285)
<223>

<220>
<221>  intron 13
<222>  (83286)..(83867)
<223>

<220>
<221>  exon 14
<222>  (83868)..(83998)
<223>

<220>

<221>  intron 14
<222>  (83999)..(84420)
<223>

<220>
<221>  exon 15
<222>  (84421)..(84543)
<223>

<220>
<221>  intron 15
<222>  (84544)..(85031)
<223>

<220>
<221>  exon 16
<222>  (85032)..(85121)
<223>

<220>
<221>  intron 16
<222>  (85122)..(86250)
<223>

<220>
<221>  exon 17
<222>  (86251)..(86409)
<223>

<220>
<221>  intron 17
<222>  (86410)..(88709)
<223>

<220>
<221>  exon 18
<222>  (88710)..(88871)
<223>

<220>
<221>  intron 18
<222>  (88872)..(93059)
<223>

<220>
<221>  exon 19
<222>  (93060)..(93136)
<223>

<220>
<221>  intron 19
<222>  (93137)..(94596)
<223>

<220>
<221>  exon 20
<222>  (94597)..(94714)
<223>


<220>
<221>  intron 20
<222>  (94715)..(125796)
<223>


<220>
<221>  exon 21
<222>  (125797)..(125937)
<223>


<220>
<221>  intron 21
<222>  (125938)..(126505)
<223>


<220>
<221>  exon 22
<222>  (126506)..(126932)
<223>


<400>  1
ggcattttgg tcaaagccat ttaacaagtc tctagaaagt ttcaaacatt cccacatctt      60

cctgtcttct tttgagccct ccaagctgtt ccaacctctg cctgttacat agtttgaaag     120

tcgcttccat attttccagt atgtttatag cagcacccca ctaccttggt accaatttat     180

tgtattagtc tgttttcata ctgctataaa gaactgctca agactgggta atttatttaa     240

aaaatagagg tttaattgac tcacagttca tcatggctgg ggagccccag gaaacttaca     300

atcaaggcaa aggggaagca aggtaccttc ttcacatggc gtctggaaga agtgcccaac     360

aaaggaagga agagcgcctt ataaaaccat cagatcttgt tagaactcag gcactatcat     420

gagaacagca tggtggaaac cacccccatg attcacttac ctccacctgg tccctccctt     480

gacacatggg gattatgggg attatgggga ttcaattcaa gatgagattt gggtggggac     540

acaaagccta accatatcag gtatggatgg gatatgggaa tacctagaac gctgaaaaag     600

aaagagggtg ggactgaacc ttgaagttcc aataggcaga attgggaaga ggagggatga     660

tccagtaaag gagacagaaa gatgtataag agaaacaatg atgtaggaga aaaacaaaca     720

ttatttcgtt ccagaggcca agtaaagaaa atgtttaaag aaagagtaag tcatgaactt     780

acatcaaatg ccagcaagtg ttcaagcaag ctgtcaataa catgtttttg ctgacgggag     840

agatccagta gagagaaaaa aatagatgat gttagtgggg gggcgggttg cagaacaaag     900

cctttgagta ggcaagaagg gatggatgag acccagcgct caaaaggttg gcagtaccag 960

gcacagcagc tcaggcctgt aatcccagca ctttgagagg ctgaggtggg cggatcacaa 1020

ggtcaggagt tcgagaccgg cctgaccaac atggtgaaac cctgtctcta ctaaaaatac 1080

aaaaattagc cgggcgtggt ggcgcacgct tgtaatccca gctactcggg aggctgagtc 1140

agaagaatcg cttgaacccg ggaggcagag gttgcagtga gccaagatcg cgccactgca 1200

ctccagcctg ggcaacagaa tgagactctg tctcaaaaaa aaaaaaaaaa aaaagaaaaa 1260

gaaaaaaagt tggccctagt taatactgtc agttcagcca aatatgagtt tatgggtgtg 1320

tgcgtgcaca cacagacaca cacactctca cacattttca cgatttatag ccccatgcac 1380

ccactacttt cgaagctttg acttttagag aaccctgttc gttaagctca ctttctcctc 1440

gagattttca gcagagtaag tgagaggaca ttatcagtta tccttgtttg taattcagtg 1500

caatctctgt ggccagcttt gcttcattag ctcttgagac cagatgatga aataaatgga 1560

gggcataggt caacagccct aggtgtagaa gggtaaaggg ggtggtgtgc cttttatgaa 1620

cgttagaaac ctctccaagg tcctgagttg gtttagccgc tgttacagtt gctcttttaa 1680

gtctttgaag tgtcaaatgt ggaataactt ttaaactttc agctctgaag tgttttgatg 1740

agaagcatct tgattatccc ccgtataaga ttatacttta tctctgttat atcgccagct 1800

cccagtacaa tgtgtggctt agagtagcga agtagtaaag ctcgctttga agaaggtact 1860

aaaaactatg attattgtca ctacagaccc aacattctca gagggggctg gaggatgggc 1920

tagcttcgga atcatgcctt tgttgccaca agtggagttc attaagtggc atatgtttgc 1980

ctgtgtgtgt gcatgtgtga agagaaacat tgtgtgttat caatagtgtg aaggcaacca 2040

gcaggagcat cctgagaggg acaagtaagg tgggggctag agaggacagg tatatttatt 2100

tttttaaatg ggggttatcc tgcctttacc ttctaggagg ataatgtatg atgtactaaa 2160

atccctgccc tagtatttgt cactaagtat cgaaatgggc gtggtttggc atataagaca 2220

cctgctcttc gtgagccctg caaagagata ctcttggagt cctaccctta ccctgattcc 2280

tcaagtgctc tggagggtcc cccagttcca gcatcctttt tgagaatcct gccgcccgcc 2340

agcaatgctg ctccgtggac ttgagagggc ctgctgagca tgtgtgaaga gcgctgcgca 2400

tgctccgtgg agtgggcagg tttaccgagg cgctagcagg ggcttttact actgttcagt 2460

cgcctcccca gcccagcacg cccccctccc ctctatccca ccacccacca cgcggtcccc 2520

cgcatccacc ccgcccctct atcccgcccc cttcacccca agctggggaa tctccattga 2580

cgtttgggtg acgccgctgt gccgccgccg tgggggggtc caggcgccgc acaggtaacg 2640

ccgccgctgc cgccatattg acagagcagg gagcggggag gggacccgag gaagtggggt 2700

gttgggggag tcaccaccga gcggctgtcg ttgtggtggc agtggtgagc gcgagccgag 2760

cggcagctct tggagaggca gcacagagtt gtcttctcta tgggaatcac cgggcggcgg 2820

cggcggcgac ccccagctcc cagcctctga gagacccggc ggcgagggca gcgctcgctg 2880

aaggcggggc tgggggtcgc ggcggcggcg gcggcggcgg cggcggcggc gggccagttc 2940

aatagacagg atgcgcggcc gcgccagcgg taggcggcag ctcctgtttg agtgctcctg 3000

aaggggagat gctaccattc gctcctcagg acgagccctg ggaccgagaa atggaagtgt 3060

tcagcggcgg cggcgcgagc agcggcgagg ttagtgtggt ggccaagacg ggctggagcc 3120

aacccccgcc tccttcccct caagaccttg gctggggacg gagagaggag aggcgtaggg 3180

ccgggaagcg ggctcccggg caagccccct tgatgctgcc tccactgccc tttcgctggg 3240

gagcgggcgg ggatgagacg gcgggctcgg gcttgcggag tgagtgcagc ctgggcccgg 3300

gctcggcctt gggcggagcg cctgcctggc gggccacgcc agagttgttg tgactaagtt 3360

tcttcacttt ccgacccgaa ggggaactgg gggactggcg gggtgctgtg cctgactccg 3420

cgcgtccctt tttctccgcc gcgcctagtt tgctggctgg gaacattttg ggggacagct 3480

gcggactcca gctcgctgtt gaaccccgga cgctttacga acccagtgca acaaacacac 3540

caaggtgact aagtaactct ggggacattt gcacataggc acgagtgttc cctggtaaca 3600

gctttctcgt ttctctgtgg cttgttatgg ttccttgtaa tgacccatgt cgagatacac 3660

tggcgaccta tctccagtcg ccaggtttca ggtgatgttc tactgcttcc tctcaggtcc 3720

ccttgattct actgcactct tgctccaccc cttctccttt ccacaaccgc tgtccctgcc 3780

ccgttggtct gtaaaagaag acgcaaagta aaccccagcc cttataaata agtcgaatct 3840

ttggtaagac ttagcgaggt tcaaccaaaa tactttggtt cacctctctt cccactcttc 3900

ttactctgaa actgttctgg ttgggctttg gtcagctgtg cagtgagaac ttagatgtac 3960

acaggtgctt tgtttttatt gttgttataa aagcgggcag catttaaaca caaaaacgtg 4020

tcttttgtga tagaaagttg gatccttttc tgataactta atcgactgcg tactactcca 4080

gtagaggcca ccatacacct cctaaaaata gttggtgaaa ttgattcaca gtaggatatt 4140

ttgaggtaag actctcaatc ctcgacatct aaagggtgca actttgtctt caagtacttg 4200

ctccatttaa aaaaaataca catttggctg ttgtatgatt accttgtttt tttttcttta 4260

aatttttttg ttatttatca actaaactgg ggttaagatt acatgctaga ataacatgtg 4320

gtaaggaata tagtggaaat aaattcagaa gtaccctaaa tctgaaccct ctatacttac 4380

aggaaatatt ttactagtaa accaagcaat tccaatatac gttttaatat tttacatctc 4440

gtaagaacaa gataaatgaa taggcgttcc tttcttgttg aaatgtggga atgctaactg 4500

tttgcacaat tgtctagcaa gtctattgaa ggggagagga actttgttat attcatgtat 4560

gtaacttact gaacttatag gcatttagtt tataaaaaca gttaaatgct ttgatataag 4620

gatgggatgg gaggtgggaa tccttttgca tgcctactat tttattattt ctgttaactt 4680

tgaatgtagt atgttttaaa ttgtaattac cacatatggt ttttttattg tttgcagtat 4740

gttttaggta ttgtagagat ttaacaatgg gtctctctac ctcagcaatt tggaaaaata 4800

ctcgagtgga aattgttaat ccctatgaag taaaacgaaa ggtgaaggta atgtggtgaa 4860

gatagtattt ctgccttctt tctctaaaac agttatgatt ggttagattt aaccctgcaa 4920

gttacttaat aaaaaatatt ataggtgaaa actaaatgct acatttgaac agacccactt 4980

agtacttaga agagtttgtt tgtttttaat tatattcttt tgataagaag taaacaagtt 5040

tacttttgat aatttatatt ttaattacaa atttgttgat gttggcttta aaaatgctaa 5100

ttttgagtac aagctctaca accctcaatt gtgataattt taggactaag agtgtttttg 5160

ttgtagaata tatattttta aaatgtgcta gaatccttaa agaaaatgta ggtaagaatc 5220

tgttaacttt aatgtcaaaa gaaataattt ccgtccattt ctgtgtgtgt tgaatctggt 5280

aagattaaag attatagaca ctaccatata ttttggagtc tttttgtatc ttttacctgt 5340

cttgcttgca tggacagttg ttaacctttg aattatcatt cagaagtgtg taaattggtt 5400

gtttgagact cttaacatgt tttcccacag gaaaaactta ttaagggtac ttatattttt 5460

agatcagtac ataatagcga ggtataatga tgccgagcct aaccactgtt tgtaaaattg 5520

ttctgtggtg aaatgtgtca ggtttcaacc aataactagc ttggttccag tgagaggtag 5580

agaatgggcc tgaaaagggg aaacctggaa tcttttggtt tctgggaaga gagttcttag 5640

aagaggggaa atagggtaag caagaaactg attgtgagag ctcttgcagg gactgtctca 5700

atgaggtcta ctgggaatag gaacttcaaa gggcatcacc cctcacgcag tcacaattta 5760

agcttttgac tggattctgt caggatcaag aaagaggttt tacttgagat gggatgggta 5820

ttggtcattc ctgggcaagg atcataaaga acaacctgtt tgactaatgt gaacttttca 5880

tgatttaatg ccatgcatgt tacagtttgt tcgcttagtt gtttgttttt tggtgaggaa 5940

ccaagtgctt ctttatcaag ttgatgatgt aattttcaca tttcatatca tagtaaacgt 6000

gtagtacttt tatattattt ctttagcaat gctgttttta tttaagcaag ttagtttttt 6060

tttttttttt ttttttttga gtcagggtct ttctatgtca ttgtacattg tccaggctgg 6120

cctcgaactc ctggactcaa acaatcctca tgcctcaggt tcccgagtag ctgggactac 6180

aggtgctcac aaccatgcct gtctaaacaa attagttttg agagtatgta gtttactgat 6240

aaaaccaatt tgggaaaaac ttctagtttt aattaatttt catgagaccc tatagtaata 6300

gaggaaagaa cagctttgaa ggcaggtaaa tctgggttct tgtccgagct ccatgccata 6360

ctcatgatgt gacacaatcc acgttaccta acctctctga acctcacttt ctttgtctat 6420

aagttggaga aaaaacatct tcaagagttg ttatgtaaag aaaattcagt gctgggttta 6480

ataaacttag caagatgttt tgcacttagt ctctagtaca caatagttcc ttctcttttc 6540

ccacagtcat catgcctctt cccatgcccc taaagtctga actattgtca tctatgttaa 6600

aatcctggga ttcagataac catggcctta gccgcagcct tccatttcgt aagcagtttt 6660
tcttaagacc ccttcctaaa tttgatcatt tcatctaagt atagtggtgc tctcactgtg 6720
aatgtttgta atcacagata gggtgatcca gagataaaat acaaagttat atttagaaac 6780
agtggaagga taagaagaga cagataattt catttattat atctggctga tgaggtgttt 6840
cattgaaatt tttaattttt taaaaaaatt ctgaactctt gtcatggtag gaattggttc 6900
aattcatgaa gcatttattg agtacaaggc agtgctgtac atagtgctgt aatgtgaaat 6960
gcctaaaaat ataccacaga gtcccaccct acccccacag gtttacattc aggtagtaag 7020
acaggcatgt atggaaattg ttattgtaaa aggcacaaga ttatcaacaa tggggtggtg 7080
gtggtggtgg tgggggtggt ggtggtttag gaatggatct ggtaaggctt tgtgaaggag 7140
gtggaatttg agcagggcat tagaaagtga taatattttc acagacagat gaaaggagca 7200
tatttcacgt ggatgggcac aatataagaa aaggcatgga aatgttctga aagaaggaag 7260
aatctgatac tgttgtaagt ctgtgatgtg ggagagggga tatagtgaga gataaggaat 7320
gtattaaagg taagctaggc ccaggttgaa tgggactttt taagccagtt taagggttca 7380
attttatctc ataaatagga gccactgaag gtttttgagg acatgagtga cattattaga 7440
tttgtgcttt gaaaaataat tgacggtagt gaggaatggg ggagatatgg aaggtgggga 7500
attccagcta ggaggctatt gtgttagtgg aagtagtagt gagatttttg aactagtaca 7560
gaaggggtag aaaagagatg actgatggga gagacctggc agaggtagaa tagttacaat 7620
ttggtaactg attagatgtt ggagagtaaa aagaaataac aaacaattga gattagaagc 7680
gtcctggtag gaattaattc aatgtaacag gtatttaatg catacctgcc aatataagtc 7740
tctgtattag ccaccagact aatataaaaa taaatagatc tttgcagtat agggagatag 7800
aaagctaaaa tgaaatctcc cctttctaat attgtattga ataatagcat tccactctga 7860
aggtttctga tcgtgcttag aatgcttaaa ttctttacta ccaaaggtga atacacttat 7920
tactagctaa aacattttta attcttttta aatgggcctt aggcctgtaa ttcttgtttc 7980
accaatgttc atcttcagta gtcagcagcc gtgagtacag agtcctattc tgaaaataat 8040
gaccagtact tatttctgga atgtggaagg agagcaggga catctgtatt ttttctatgc 8100
tttcaccagg tttatttgga atagacaagc ggggaggagt cagagggcag tggcccctcc 8160
ctgccacagg tgccttctca gttcctccaa gtcaatcttt ctttaagcca gtctgatctg 8220
gaaatattca ggtcagttcc ttcagggcag gtcctctata tttccctacc ttgattagtt 8280
catcaagaga gctaatcatt acctctgtgt tccatgataa ggcctactga tagtgtagtt 8340
gcttttctgg gccatattgt tttagtacct accctctcat agtctctctc atctagaatt 8400
ttgatgagga aatgcaagat ttttttccct tcctgtaatt tctttcttgg tgctatcatt 8460

gtatgtagca gcggttctta agatttgata agcacttgga atcttgaggt ataagccttt 8520

cagtactttg tcagaatttg ctatgtaatg tttattcatt tatgttattt ccaaggatta 8580

tttactgttg ctgccaatat ttttttttgt tgtgtatttt ctttattttt gtttgttttt 8640

aggtaggaaa ggagttagat gggagagaga tttagagagc tgagctaaat ctggcttttg 8700

cagataagag acgttttcta aattgccctg gagacattct gtaactgatt tcactgctag 8760

attttaatct gtatctctac attagaaaca ggtcctgctg tagttttgtg tgcacgtgtg 8820

tgtgtgtgtg tgtgtgtgtg tgtgagagag agagagagag agagagagag acagagaaag 8880

agggaaggtg gagtagagtg catgtgtgtt acttgtgatt tgcttcattt taccaagcat 8940

ttgagatggt atatgggaaa tataatctga taactatttt gatattgagg ataagggaaa 9000

acaaagtagg agatatatga aaagggtgga gggtagggga tgaagggtaa aatagagcac 9060

aaataagcag gctgtaaggg tctatgacat gtttaaaata tagttgaatt ataaatttgg 9120

ccttgagttt tcttagctgg tttttctcaa ctctggctgt gtgttatatt tacctgggag 9180

tgtaaaaaaa ttatccatgc cagtgttcca ctcagagatt cacatttaac taatctagag 9240

ggggcctggg catctattcc ccagatgctt atgatgcaca gccagggtta aaaaccacag 9300

ctctaggcca aagtaaaaaa agaaatagtc acgtagttgt cattattcaa taggaaaaaa 9360

agaatattaa tttgtaagct agcaattagc tctgcagtaa attatctgtg aattgtgata 9420

tttttctgtg tgttttattt tgttgttgtt ttaagatatg actggagcca aattgggttg 9480

tttgaactgc cctgtatgtt cttgctcttt agttttgagg aatttgcttt tccagtagca 9540

ttaaaaacaa aatttagtac cgtagctagt tttgatatag ttcagccata taactaagag 9600

gttaaataac ttgttcaccc attttactag ctgagtaagg agatagtgac tctgttttga 9660

atgacaaata tcccattaca tctctaacaa aaaatttcaa aacccatggg taattattac 9720

taagaaatcc ctctgcctct cctgtaaagt gattgaagac aaactttaag aggaaatata 9780

gcaatttaaa cacccaatac aaatttttta acctgaaaaa acgttggaat ccattccatt 9840

taaaggtatc gaatcttaag ggagaaagag ataatagcgt ttattgagca tgaactatgt 9900

accagctagt actagatgca tcatagatta tctcagtttt attcattctt caaaacatct 9960

tggtaagaca gttatgtctt tttaacagaa tagaaagata catgcaagtt agtaggaaat 10020

acgtctggaa tttgaaccta gattatttta aattcatgct ttttcctcta tacattgcta 10080

tctcccccct aaggacaata ttgaaaataa ggagagaata tagatacgtt gttagcaacc 10140

atattagatc agcagtcaga tgtgctgatt caatagccat gcctgtctat ttaagttaaa 10200

taaatgtcaa caaaatccca gcaagtctgt ttttttgttt tgttttgttt tgtaaacaga 10260

caagcatgtt ctaaaatgta tatgtaaaag tacaagacct agaagctcta aaacaatttt 10320

gaaaaagaaa aataaaataa ggaattactc tattggatat tacgttttac tatgtaacta 10380

taataaagac aatgtggtat tggtggaaga atagacacgt agatcagtag accagaatag 10440

agtacacaga aatagagcca cacaaataca ctcaactgaa ttttgagaaa aaatgcaaaa 10500

aaataaaaaa ataaagaaca agatcagctt ttaaaatctg aggaaatttt agggggaaag 10560

ttgctacctt tttcaaaaag gcagaacttg aaatttgcac tttctttctg aagattctgg 10620

ggaaattatt acataaaaca tttttgctat tttcagttac tacagttgaa attagtcatt 10680

aagacataag tgaaaatgga acatgaatta tcctaaaaat cataagcttg ataagagttg 10740

aggtataggg atttattact ctacttaaag gatcagagtg gacagaactt cagaaaacaa 10800

aggagaaaaa cgtcatatga ggggaaatgt ggactgaatg tcaggtttca aatgactttt 10860

agtttttctt aaaatgagag aggtttggtg tatgtgttaa agaaattgta ctaattgaga 10920

atagtcaaaa ttcaaaatta agggaaatag ataagccatt aggtctgatt tgataaaaat 10980

attttgcatt catgattgtt cacctggagt agatgcagtg atgaatgaat gctgttggaa 11040

acaattacat ttcaaactta atgggcatat gcagaaaacc atatgttgat tatagagaca 11100

tatacaagaa tgatagaaca tatataagat aatttgtgta tgattttgta ctttatttta 11160

cagatggaga agagtaactc tagaccttct ggcacaatca taaaacattt attgtgttta 11220

tcatttgtag agcaattaca ttggaataag tgaatactta tagaatctct taagggaggt 11280

cagtcaatac ggattttcaa aaaagacatg gtcattcagc aacttcatat ctatgaacaa 11340

tattaatttg attttatttg gtctttattc atgatattat gtgtatcagt agttctttct 11400

tttttattgc tgtgcactag tctgttatgt gaatgtacca cagttgattt gttcattaat 11460

aagttgaagg acatttggat ttgggttgtt accagtgttt ggtgattatg aataaagcca 11520

ttatacacat ttgtatgaaa atttcggttt ggacataact tttcacttat attgggtaaa 11580

cacttaggag tgaggttgct gcgtgagaat tccagctgct ctgagtactt gccagcactt 11640

agtcttgtca gattaagtga aaaaaaatag ctattctaat tggagtgtag gggtatctaa 11700

ttgtgatttt aacttgcatt tccctaatga cacatgttgc taagcatatt ttcatatgct 11760

tatttgccat ccgtatatat tctctgatga gttgtctttt caaatttttt gaaattgttc 11820

aagttgggtt gttttcttat tattgaattt tgagagttct ttatattcca aatgcaagtc 11880

ctttattaga taccgatttc caaatatttt tcccacatgg tctgtctttt cattctctta 11940

ataaagagtc gaggtttttc atcttagcga agtccagttt atcagttttt tttttctttt 12000

atgaattgtg tttttggtgt tgtatctaag aaatcctagc ctaatccaaa gttacagaat 12060

ttttccctta agttttcttc tagaagtttt ttagttatat ttcttaattt aggtctttga 12120

tccatttcta gttaattttt gaatatggtg ggtagtgtga tagaagtcta cttttttaca 12180

caggtatgtc cagttgctct gccaccattt gttgaaaaga ctacctgttc tctattgaat 12240

tgcctttgta ctatatcaaa aatcattttg tcacatatgt gtagttttca taataatttt 12300

aatgaattag tcctgtggaa cttcagaatt gcacttactg agcatagata tatggtgtta 12360

ataaaattaa ttaatacaat taataaaatt cttgaggttg tttctgtatt gggtttagtg 12420

aaatattctt tgttaaaaaa caaaataatt tctgtgatat attttctttg cagtcattaa 12480

ttttcaaaac attaagtgac tgttcttaga cccttttata gatgaggaaa cctaatggtg 12540

ctagaagttt tatgtgaact ttctcattct gcagtgatac ttctctgctt atctcctccc 12600

ccatatttgt acattacccc taacattttt tatatagcag tttgtgacct gttagttgct 12660

tagtattaga catgaaagct acaagactga ataagcttca gttcatattc tagaaactat 12720

ctctttgcca agaaaatggg agcctggatt gttggagctt tggtaaccat cattaacata 12780

gatttaattt tgctcttttt aaataaaatt attttggctt ggcacggtgg cttatgcctg 12840

taatcccagc actttgggag gcagcgtgcg aggaatactt gagcccagga gtttgagacc 12900

agcctgggca acatagtgag accctgtctc taaaaagaat aaaaaattag ctgtgtgtgg 12960

tggtgcatgc ctgtggtccc agctacttgt ggagctgagg tgggaggatc acttgagcct 13020

gggaagtcaa ggctgctgtg agccatgatt gtgccactgc actccagcct gggtgacaga 13080

gcgagacctt gtctcaaaaa atttatattt tagtttttaa aattgacaaa taattgttgt 13140

atattccttt tggttttaag aagagaatga tattcgttct ttgagaaaca ttttaaaact 13200

accttcttta accttggtta atgatactca gtgcctcatg tcttcctatg tgaaagagga 13260

aatatgagaa taatgtaggt tgggtttgta ttgagaatat tgtgctaatt aagttgattt 13320

taaacttaag gaaatataaa cagttgggtt agccatctca gattctttac aaattcagaa 13380

taccatagag gataatagct ggttccctag agctagaatg cccgagttta aattctggct 13440

ctttatttat ttactgtgtg accttggaca agttacttct ctgtagttta cttggtcgct 13500

ctatctgtaa aatggggata acagtagcat ataatatatg taaagcactt agaagagtgt 13560

cttttccatt gtaagcactc agtaaatgtt attattttta aatatatcac atctgcaatc 13620

tgagttattg aagattaaca ggcttgcaag ttaaaaaaat caactattgt ataatgatat 13680

ttaagtatca tatataatta tgcagttatt agtatttctt ctttaaaggt atttatatac 13740

aaatacacat ttctcctttt tgaccatctt cttctacctg tgacttgaat tttgaaacat 13800

tttattcttc cttgattagc tgtagtaagg ctgtcttttt ttcgtattca tttctcaaga 13860

ggatggtact gcttttgctg aacttatatt ttctttattc actgagcttc tgttttctgt 13920

agtctattga cttgtgattt tctctgagta gttttttgac agcaaacatt tattaatgga 13980

aaaaaaccca cacatatcag gtagaatttg acctcaagct aaggttgtgt aatagctgtc 14040

tatttttgac ttttttccac atataaaccc attttagttt ttaaattttt tcctttattc 14100

tccattgttt atgaagtgat gaaagaatag agtaatatgg tgtaggacat ttaggaactg 14160

tgactcactg ggataaagtt gttttatcct gtcactagct tgctgtgtgt ttttaggtat 14220

gtgttattta attaatcagt ttcttcacat ataaaatggg aattaacatg tttatgatta 14280

ttttgagaag taacacaatt gtactttgta tataatgttt gtcaagaata ataaagttct 14340

ttttcctttc aaaaaatgac taaagattta ggtgttctag acaaagtact tctttaaccc 14400

tgatatataa ctcagtggac aatttaaact taattcatac cccaaaggta gtattttaat 14460

gtgattatat agacttcata tcttgaagag gttacaacag tgagaattac tacctcagaa 14520

tgtcatgtgt gtattcaggt tgcatatgta catgacttca tttataccac atgaatactt 14580

atatttttgg aaagtaaact tcccaaatgc atgtgttgta gcccatatga gactggccag 14640

acagccagtg cctttgcatt gggaacactt tggagctatt caaaaccaat aaagtcatta 14700

gctctgattt cagctaagca gagtaaacaa attcctgcct gaatagaaac caggaagctg 14760

catttctgtc attgaaacaa ggttgtaaaa actcaaatgt tctggaaata ccttctaatg 14820

tttatgcaat attgacatac ttagtttaac tcagtagtaa cagtagttac tcagtgactc 14880

agtagtaact cagtagtaat agtagtacaa ctactagtcc tttttaaaag catgatgata 14940

attggatttt ttttaattgg ttctaaatat tattttgcat gcactcacca aattatgatt 15000

aaatatgtat agtgttgtgt tactagatag ggttcatttg gttattgctc tcaatttctg 15060

tcttgataac tgaaaactaa ttaactgtgt atttacgcat ttttccataa tgtacaaaga 15120

aatatctgaa ctgaaggaca atatattatg tcacaaattc caacaaattt agccttatag 15180

aaagattttt aaaaatattt tggttgttaa tgttttaatt ccatactcct gtggcattat 15240

tcacaggttc actaatagat ctttcccacc cttttgcttt tctaatatat taatacccga 15300

tttcctccta ctcaaataag ccatcaagaa agtagagcct cggtatgtat ggcagttcag 15360

tttgtgccgt attcttgctt tgcatagctc cccttactct tggctatgta atacctagca 15420

tataaatgct atgtaaatag ctgttaatag agataacatt attgctatgt aatacctagc 15480

agtataaatg ccatgtaaat agttgttagt ggagataatt taaaatatag aggaagagat 15540

ttaacagata aataatagag atcatttaaa gtacagagga ggatgtgcat agttatatgc 15600

aaatgccaca tcattttata tacaaaactt gagcatctgt ggattttggc atccacgaga 15660

ggtcctggaa ccaatctccc acgtatactg aggttcataa tgtgatttcc atgcaagata 15720

tgaaatacaa agaatttcta acattttttt tcttgaaaac aattttatga ctaacccaaa 15780

taatatattt ttaatgtgat tttgaaaaag ctgaattcct ctttgctgtt agcatttaag 15840

agtcagaaat gggaagtaaa atgacttact gtaacttcgt catgaaatta tgtttgaaag 15900

ttggttttaa acatttttag taagaaaact aataaaaatc gaaggaaaaa taatggccat 15960

tgcttattat tgaagttatt ttataaattt tattcattta gatcccagat tttagtcatt 16020

ccattttgta aatatttttc cagtgtctat tatgttccag atattatgct aggtactggg 16080
gtaaccaaga gtaatatgat gagatccctt tcctccagga gattacagta tgatagagag 16140
atggaagaga cacttaacaa gtaacacagt gtttactgat agaagtcttc atagggtaag 16200
gtggaaggaa aaagggaaag tgttaacgtt gagtcttgat aaagatgagt tgacattcaa 16260
aagcacaggt aattcaaaat ggcaaatact ccaaagatca ttcatattta actttggact 16320
tggttttatg ccttaatttg aacatggata tgttttacca gctaactgct tttacaacag 16380
agcctcatga ttgcatttaa atacctgact aaagtaatcg cttggaggct gtatactctt 16440
gtcccattct gagactcttt taaaaaaaag acatctcttt taaattgatt tgaaacataa 16500
ttgccctttc ttaagtcctc cctgaagcag tgacaataat taggtgggtg tatttcaaac 16560
ttcttgaggt acaaactaca agaatattgt ataattagaa agccagaaag gatcaaagta 16620
aatcatttaa aatgagtatt gtagttgtta tcagatttct cataagcagt atgttaacta 16680
tataatttaa tctaaaagtt caaaaagatt aaagtgtttt ggggaaatga ccatagatat 16740
taataaacct ttgaaaatat ttggtagtat aaaagacaca gtacaaatta aaacttgcct 16800
ggtgaatcca aaaaaacaaa ccaactaaat cttatttttg gaaatgtctt gcttaaaaga 16860
ctgttagata aattgataaa gtgtgttata gtaaaaatat acaaagatac ttttttattt 16920
ctctccaatt tgtgtttctt aaagcaaaga gtatgttcat gagtatgtat atacctttat 16980
gggttggttt gggagattca tttcacgttg accaaaagtg atcctatcat aattttaaaa 17040
gtgatctttc cacaacagta tgacatatca aaactacact agggctgggc actgtggctc 17100
atgactgtaa tccctgtgct ttgggaagct ggggcaggag gattatttga gcctaggagt 17160
ttgaggttac aatgagctgt gatcgcactg ccactgcact gcagcctggg gcgacacagc 17220
gagatcctat ctcaaaaaat aaataaatga tacaaaaaaa ctaaattaag actattgagt 17280
caatactatt atgatagtga tatggcagta agttttgatt cacatatttt ttattctcta 17340
tacaggcagc attattcacc tctgatgaat tattagctaa aatgttttta tttttttctg 17400
tattgttttt atcagaaaaa cattcacaca tcatgcattt gttgagcaca tgtcagacac 17460
tgtgccagct gctttaggaa cattgtatga atcctgacaa tcctaggttg ctcattttac 17520
agatgaagaa attgcaacta agaaagctta ataaactttt caaaggcagt aaaagtagag 17580
ctttagagtt tgaatcttgg tctgcttgac actgaagaat tgctgttttc atgacatact 17640
atgcctgcct aatgaatgaa acacagcatc attcctcaag gagctttaag tctagatgca 17700
catgacaaga aatttacctg atgtgatgta ttactaatct tatttttagc attaaaattt 17760
taaattgtct tatatatata aatatatata tgaaacataa aatttgccat cttaaccatt 17820
ttaaagaata cagttcaaaa gtgttgagta cattcacatt attgtgcagc caatctacag 17880
gactgttttt accttgcaaa actagaactc cttacacgtt acacaaaagc tttccattct 17940

cccctttttg ccagccccag caaccacctt tctactttcc atctctatga atttgactat 18000
tctagatacc ttatataaat ggaaccatat agtatttgtc attttatgac tggcttattt 18060
caattcatat aatatcatca tggttcatgt tgcagcgtgt gtcagaattt cctttctttt 18120
taaggttgaa tgatattcca ttgtatgtat ataccacatt ttgtttaact taccatctgt 18180
cgataaatac ttgggttgct tctacctttt ggctatttgt aaataatgct gctatgaaca 18240
taggtgtaca aatatctttt tgagaccctg ctttcgattt ttttggttct ataccctgaa 18300
gtgggattgc tagatcatat ggtaattcta ttttaaattt ttaaggaatt gctatactgc 18360
tttttatagc agctatacca ttttacgttg tcaccaacag tgcacagagg ttccaatttc 18420
tccacatcct tatgtatggt ggaattattt tgaaaaagta aattccagga tatattctgg 18480
atttactttc catttcaaca aagccatgct gttgataatt catgttgaga gggttagttc 18540
ttaagatact gagatatccc tgattgttaa aggaatatga aattatgttc ttgcggtgat 18600
tcgtcaagga tctaggacca gaaataccat ttgatctagc aatcccatta atgggtatat 18660
acccaaagga ttataaatca ttctaatata aagacacatg cacacgtatg tttattgcag 18720
ccctatttat tgcagcacta tatataaaca ctatatataa acacgcatgt ttattgcact 18780
actatttatt gcagcaaaca cttggaacta acctaaatgc ccatcagtga tagactggat 18840
aaagaaaatg tggtacatac acatcatgga atactatgca gccataaaaa gaatgagttc 18900
atgtcctttg cagggacatg gatgaaacta gaaaccatca tccttagcaa actaacacag 18960
ggacagaaaa ccgaacacca catgttctca ctcataagtg ggatttgaac aatgagaaca 19020
catggacaca gggaggggaa tatcacacac tggggtatgt cgggggttgg cgggaaaagg 19080
gcggggagag tattcggaca aatacctaat gcatgtggag cttaaaacct agatggcgag 19140
ttgataggtg gagcaagcca ccatggcaca tgtatacctg tgtaacaaac ctgcacgttc 19200
atcacatgta tcccagaact taaaataaaa ttttaaaaaa aggaattacg ttctctatac 19260
tgtgcttaag ttagtgattg atttttcgga aagcaggatt aaaggacaaa aatagaactg 19320
ctcaatttta agtttattga aactaaaatt aaaaatttgg ccccacttaa acatttgtcc 19380
tgggtcactt actagtatat agtttacagt tttatttcta ccaaatgaga acaaatatgt 19440
gagtttaata agtaaagatc tattttaaaa tgattaaact gtaaaatttg tctctggaaa 19500
gtctgtttca ggggagaatt tttgatcata gacacttttg attgtattac ctagtgctgc 19560
aaaaaattac cataaattta gcagctttaa ccaagacaca tttattatct cacagtttct 19620
atggatcagg aatctgggct tggctcaact agatcctctg cttagggccc taccaagctg 19680
taatcaacac atgggtcagc ttggtcctct gcttcgattc ccacaaaaat gtaatcaagg 19740
tttcagctgt tctgcattgt tatatagagg cttgactggg tgaataatct acttttacac 19800

tccctcagat tcttggtaga gttcctttcc ttgtggttgc aggactgagg gccctggctt 19860

cttgctggat gttgagtgga ggctgctttc aactcctaga ggctgcctgt agttcctaga 19920

ggatggccac agtccctcgt tatggggact ttctcaacat ggtcacttaa ttacttcaat 19980

caagtcagca aggaaagcct ctagagtctt atatgacaca gtgtaatcat atatgtaaaa 20040

tctcatcgcc tttgccatat tctattggtt ggaattcagt gataggccat gttcacattc 20100

aaggggatgg ggattataga agggcataaa cagcaagagg tgaggatcat gggggcctat 20160

cttagagtgt ccaacacatt gcttaattgg taggtttctt tctctgtttt attttgtcct 20220

gggcttaata tataggcata gatttttttt gttttgtttt ttgttttttt ttgcctttga 20280

gccagagtct cactctgtcg cccaggctgg aatgcagtgg cgtgatcttg gctcactgca 20340

acctccacct cctgggttca agtagttctc ctgcctcagc ctccccagta gctgggatta 20400

caagtgcatg ctgccacacc cagctaattt ttgtattttt agtagagatg gggtttcact 20460

atgttggcca ggctggtgtc gaactcctga cctcaggtga tccacccgcc tcagcctccc 20520

aaagtgttgg gattacagac gtgagccact atgcctggcc ggatctttat tttaggtaga 20580

tgtggaccca cctttatctt tagctctaat ttacttttgt tatttaaata aagtgaaaaa 20640

attgaataga agaagtcttc tttacaaaag ctatcagtga ctaatatgta aatgtaaagc 20700

ttatttgaga gacatatttt cttttttttt tttttttttt ttagaaggag tcttactctg 20760

ccatccaagc tggagtgttg tggcataatc tcggctcact gaaacctcca cctcctgggt 20820

tcaagcagtt gtgcctcagc ctcccatgta gctgggatta caggcgtgtg tcactatgta 20880

cagctaattt ttatattttt agtagagacg ggtttcacca tgttggccag actgatctcg 20940

agctcctgat ctcaggtgat gcacctgcct tagtttccca aagtgctggg attacaggca 21000

tgagccacag cacctggcca atattttttt ttactaggtt gattttgggg atcctggaga 21060

atacttgaaa atatataata ttgtcaaatt tattgtggaa actgctgtcc tgtgtatgtt 21120

ttgttaatca tttgcatcac ccatatttat tctcactttt tatctagttc ttatttgtgt 21180

atctctctac tgtcttcttc ggaaatgtgt atctctctac tttcttcttc agaggtgcac 21240

acaaataagg aaaaagtaga ttttattcta gttagatgtg ggcctacttc taagtttcaa 21300

tatatctttc agcaccaagc tacaagctct gttaacttcc tccttttgtg taacactaag 21360

gaacaggttt atgtatcata gataacagta aactctttta ctaaactatt tgtattcata 21420

tgttcactat cccataccac ctccaattcc aaggatctga aagggatttt ttttttttta 21480

atttctcagt tcagttttcc ttgatgagtt gctttggtca ataatagtaa gaaagcgata 21540

aatttgaagt gacctatgtg attttctggg atcagatgag tacagcaact cagtttagga 21600

tttctgtttg tcccgcatat ttgtaagtgc tttaagtctt ttctgataaa atgtgaaaag 21660

aatttttaaa gtagtcttct ctaagccctg gtacgagagt ctgttcactt ggattttgaa 21720

atatctgagt aaattattac tacctttatt gttgatgata aacattccag aatcatatgt 21780

agaatttttt tcctctcatt tggcacttaa ttggggaaag cagcatgtct gcttatcttt 21840

tcaaattaca aattttattt ttgaaataaa gatttattat cccagagaat tcagaaatta 21900

tttttgtttt tatgtggatg aaagtgattt aaattatagt aagtatcaat gagtatacac 21960

tgtggttctc cgtaggatga atattcttat attgaaaacc tgataccttg agggtagatg 22020

agatgggagc aatggagtgt tctgtttcta aaggccattt ctgactgaga taatttccag 22080

gatagagtga tcttcttgac tcattcacat tcattttatt tctagttcct tcagacccac 22140

agcttcatga taaattagtc ataagatgat atgaatagga agtaaccagt aactaaggta 22200

acagaggaaa gcaatatttt tcttttttta tggataatcc tcttcctatg cataatcctt 22260

ttttctatgg ataattctga gagtcatttt atggcaaaaa tttaattatc acgattctgt 22320

cttaatattg atttctggac tgttttccga atgctcttct ttctatatgc ttacaatgaa 22380

tctgtcacag ttcttttttt aatcccacat agtctgtttt gttgagctaa ggtggtagat 22440

ggtgttatca tttaatgaaa tgggaaaccc tgaagaaaga gcagatatga agcaggggtc 22500

aagaattcct ttttgtattg gttatttttg agagacatct gagataaaca tgtggaaatg 22560

tcaagtatgc agttggatat atggatcagg aactcagagt tagtcactga gtaaacgtga 22620

atcatgttag taaattagct gcgtggggaa agaatgtgga gtgggatgag aaggtgatcc 22680

tagtccattt cctgaggaat tacagtgttt caagggtatt gaaaaaaagc agcaaaggaa 22740

attggagtaa aataaaagga gtgtatggta ttacagaagc taaagactca aataggagag 22800

tggtcacctg tgacatgctg cagagagatc aaataagata atgactgatg gatccgttag 22860

caaagtagac atcattactg actttattga ccattgctgg ttttcaatgg gatttttttt 22920

tagaatagag atgctagttg agtaaataat gctaatttac tttttaataa attggtttcc 22980

aatttttcat atagtaaaat tcactctttt tcatgtaggg ttctgagatt aaaaaatgta 23040

tagagttgtg taaccatcat cacaatccag ataaagagtt gttcgatgac ccaccctccc 23100

cagcacacac acacacatac acacactctt cccatgtgat gcctctttgt agtcagcctc 23160

ttccctatcc ctaactcctt gcaaccacta atttcttctc cattcctttt agttttacct 23220

tttacagaat aacatataaa tggaatccta cagaatgtag ccttttgaat cttgcacctt 23280

ttatttacaa tgtgtttggg attcatccat gttgttgaat gtatcagtat attattcttt 23340

tttattgctg agtgatactc gtcttcctct acccccaacc cgttgaacca attatcttta 23400

ttctttccct acagttttgc tttattcaaa gtatcttata catgaagtca tacagtattt 23460

agccttttgg atttggcttc tttcagttag cagaaacgct tttaagattc atcgatgttg 23520

ttacaagtat caacagttct cttcttttta ttgatgagta aaaagtagta ttctttttat 23580

ccactcatca gttgaagacc atcttgattg tttccaattt atgacaatga taaataaagc 23640

tgctgtaaaa atgtatgtac agatttgtgt cttaacatgt ttccactgat ttcgggttaa 23700

tgttctctgt tttctggtct ccatgatttc ttacgagaaa tctgcagtca ttcagtttga 23760

tgttcctctt tatgtaatgt gtggtttttc tctcactcct ttcaatattt tccccatttt 23820

tcctttggtt taaagtagtt tacttaagat gtgtcttggg gtggatttct ttaattttat 23880

tctgtttggg atttactgaa cttcttgaaa ctgtaatttt ttttttcaaa tttgggaaat 23940

gttcagccat tatttatctt gaaaatacag ctttagtgag atagaattca cataacatat 24000

aattcaccca tttaaaatat acaattcaat actttttatt atattcacaa atatgtacaa 24060

ctatcacaac agtaaatttt aaaagttctt catcacatca aaaaggaaca ttgtaccctt 24120

tagttatgac ctccatctcc acatacctca ccccaaccaa ccattagtct gctttctgtc 24180

tctatacatt tccctgtttt cattatttca tatgaatgaa attgccttta tttcgttaag 24240

tgattttttc agtattactc tgttatttta tgggatgtta atgacatgga ttttagaatg 24300

tctcatagtg tttcatatgt ctctgaggct ctgtttattt ttcatcattt tcactctttt 24360

cttcatattg gatgacatct attgatctgt cttaaagttt atttcctcag ccacctgcat 24420

tcttttattg agtctatcca gtatttttaa aatttttcat atgattttta gttctagaat 24480

tacttacatt tgtttttttt catagtttct atttctctgc tgagaacctt ttatcatttc 24540

attcaggtgt gtttattttt atccagaaaa gcatagttat agtagctgct ttaagattct 24600

tgtcagatag ttcaactttg gagccatctt ggagttgata cttgttgata atttgtttgt 24660

ttcagcaggc aatctaccta gttaggttga ctccaaagtt atgtcttgtc ctctctggat 24720

aatggttcca atgttaattc agttttcaga gagtttgggt cagcactctg catgtgccac 24780

tcattggatg tgctaattca gtggttagtc tgagacgagc aattattggt attgtagttc 24840

agtactcaaa gactttgtta tgtttcttta gtcctttctg cacacatgca gttttttgtt 24900

gtgaccagga cttgtgttgg ttcatacaca gaattagggg atctgctctc ttcattctat 24960

cctctctgat ttcttccaca ttcttcaatt cccatgggcc ctttattctg ttctgctggc 25020

cacaaatatg agtttctctc agagtcttag ctttttatgc tggaatataa ctttacacta 25080

ttggagctgc cttttggatg aagtaaagaa gagagtgcaa aaaaaataac agggattacc 25140

ttcacatttt tcttataaaa gggacctctt ttcctgcttc ctctggccag agagagagat 25200

tttctcttga tcagggcttg ccttggagtt ggactcagca tcaaaaaaga gaaagtgtgt 25260

acatggggaa tcttggattc tccttatgtt tacaggctca aaagtgcgtc ttttcctggt 25320

cctttgtcta gaaaatggga tttttcttgg aatttttgtt gatgtctact gtgtaatttt 25380

gtgactcatt ctctcctctg ctcaaattca ggagataaag gtagaaaata aacctaggga 25440

attcatcaga gttaattttc aagttttgat cttccctaat taatttgttt ttgtttcatt 25500

ttcagagttc ttatgtagtt tctttttgta ttctacataa ggattttagt ttataatagg 25560
gggagatagg cttatgagct tatttcattt tgactgtcac aagaaactct aatttacttt 25620
ttaatttttt tttctgttga cttacaaatg ggcagtggta aaaatccttt ttatctagtt 25680
cgtcttcctg ttgcctaatt ttcaaacatc ttagttcagt gtttggcaga ttgtaagggt 25740
gcagatagga aatattttaa gctttgcaga catagggtct ctgtcacaac tggtcaactc 25800
tgctgttgta gtgcaaaagc agccatagat aagatggaaa caaatgggtc tggctgtgtt 25860
ctagtaaaat ttttacagaa acggacaaga ggtcagattt agctcatggg ttatagtttg 25920
tagaaccttg atctaagtct tctactttac tgtttcttga attagttgtt aaagaatgag 25980
tagtattacc tactgagctc tgcatgttga cttgttttgt ttccatgatt acttacatct 26040
ttctcctata gtttattttt tcttctgtat agtatgtcat gatgtttaat taactgaatg 26100
aaagaggaat tcacaacagt gcaattatat aaaactgttc ttattataat ttgctttgaa 26160
tataccccca tggtagattt ctgtggcatt ttttttcctg tcataaatac ttggttgggg 26220
acaatttaat gaaagtacat aggttatata cttagtgtat agttatctgt tattgcaaaa 26280
ctctaaacaa tatgaaatta agcataaagc tctgaaaatc aaagtttctc aaggataatc 26340
tgggaaccat gttaatagtg ttccttttct aagtacttct atgtgtcctt tgtccttttg 26400
tttttcagta agtgtggatc ttgttagatt gtggaaggaa atgcctaggg ttatattttt 26460
cttagttatc actcttgttt taatactttt gaacctcaat ttttgaacaa tgtttttcag 26520
gtaaatggtc ttaaaatggt tgatgagcca atggaagagg gagaagcaga ttcttgtcat 26580
gtaagtattt atgttaattt attaagccac atttttagca tataaggaaa aagaatacag 26640
ggaaaggaga aaagctccta tcaaacaaat atattcaggt aagttggtaa taagcaaatg 26700
gaattattaa taactcaact gcagtttaaa atataggaca ggttttcctg acagaaatac 26760
ttatgcatgc atttttaaaa ataaacagct ttattgagac ataatttaca tgctattgaa 26820
ttcacccatt ttaagtgtaa tggataaaat tttcagttgc cagtgtatga gtaactgaat 26880
tattatactt aaatatagaa tcctttgaga agattatctt caacctcagg gtagaaagga 26940
tatctgactt ggaaatattg aactgtataa gcataacttg aaatcattta aaatcaattt 27000
cctggctttt agaaaggatt atgtgtaaat tatttcagaa gtttctaact agttttaaag 27060
actgcttagc cattagacca ttctatgtaa tgcctagata ctagagtctt attttacaag 27120
atctagtttt acagagagca ttcttaagat ttgtagggag aatctgacta aatcagcatc 27180
tacatttatg ttgttatttt atggttgtta tttctataga tattttcata gagttataga 27240
tttttttttt tttttttttt ttgagacgga gtctcgctct gtcgcccagg ccggactgcg 27300
gactgcagtg gcgcaatctc ggctcactgc aagctccgcc tcccgggttc acgccattct 27360

cctgcctcag cctcccgagt agctgggact acaggcgccc gccaccgcgc ccggctaatt 27420
ttttgtattt ttagtagaga cggggtttca ccttgttagc caggatggtc tcgatctcct 27480
gacctcatga tccacccgcc tcggcctccc aaagtgctgg gattacaggc gtgagccacc 27540
gcgcccggcc gagttataga ttttttaagc tggaaagaac ctcagagata ttatgtaata 27600
cttcattgta tggattagtc tactgaaact tggagagtct gtgatttata ctaggtgata 27660
aaagtagcta gtagcagaat caagacttag ggcagtatgc ttccttttag gtcagatata 27720
ttatgtagac ctgaagaagt agaagatgag gagaacatta tcctttttct atttggttgt 27780
ggatgaaatc aactttgatg acccctccac caaaacttgt tcataattgg aattagctca 27840
gatagaaacc agcagaggga tttaaattgg gtttgtccaa aactctcctt tgaaatgttc 27900
tatgtaacta tttctgcagt tagtaaccat gtaacaacac ttttaatttt gattgacaat 27960
atgagagata caatgttgtc tttgtagctt ttgcagttta ctgaatatta ataatatatt 28020
gtaattttga gtataattaa atgtaaacta tcaaaaccat tatctagaga tttgtctaca 28080
gttcatatgg actaataggc atagtaatat taatgttgcc agtttataaa agataaagga 28140
gtataaggaa ctattaaagg cagtggagaa ggagaaggct gcttattata ttttagcatt 28200
aataaattca caaggatatc atggcttaca ccacagggga acagtgtgtg tggtgcataa 28260
actcaaggac acatacacag ggtgattggc aaatatttac ctttataatt ttagtagaag 28320
ataagtcagg aagttcagtt tttagtggaa aggcagtaaa aatatacagt gctagttact 28380
tacatgatct accttatgca atcaacttca gtggctaaaa ggtagagtgt gaaattttgg 28440
agagtaaagg acctagaatc acttccaagg atttgattta gtttgtggcc tgagttatct 28500
ggacttgaaa ccatgtactt tgggtgtgtg atggttaaag tactcaattt gtgtcaataa 28560
aactacatga gtgattaaaa acaccatatg tatttatccc ttaaattttt taaatataca 28620
gctacgattt ttttctttgt gattgtcatg tgtttttttg tggttggctt ttttttttac 28680
tgttttaaaa tagtgtagga ttgtagagac tgctctgtga tggtaagtat taggtaacag 28740
gttgctgata aactatatct aatatcacat ttcaccacct agtatcctac ttctttcagc 28800
aatgtcaagg ttagaaaaag tttgttatga tttgtctcct ttagtagttg gctgacatga 28860
gttaactttt tatttgttgt caaaataagc ataactgcct tatatttggt agtttatgat 28920
ccattggtat ttatttttaa aactctaccc agtatctgtt ttttctgaaa taaaatacat 28980
atttttaatg ctatcaatat cttaaaaatt tcaaagaata ttcagttagc aattagattc 29040
tttgttgatt acattagtgc aaggtcagca acatgtttag tctctaaagt gatttatatt 29100
tttattatgc agagatgtaa atgtcatagt gaactgaaaa tgtatatttt gaaaaacaat 29160
gctggatatc ctgatttttt taagccctga atttcttatt ttaaaaacca ttcttgacat 29220
gtgtctgtta agaattcttt caaaaatact tttttacaag tgtgttttac ttagactcag 29280

tggaaccaca ggtaggagct atttcaagct gagaatgcct gctttctgta tggtggattt 29340
ttttttcctc tatttgtctt tctattctga tgcacattta gccttagtat gcttcagtat 29400
tttttctctt gcaatgctga tgtttacttc taatttctat tcagttactt aaaaaactgg 29460
aaaccttgtc ctacagatat gcaaaactct cagatgaata attattgttc aactaaaaaa 29520
tgttggtcag ccactataaa gtcgagttat gctgcaccat caggtatgca aagataagta 29580
ctacagagag tatctgactc aggatggttc aacttatgag tttcaacttt atagtggtgc 29640
aaaagtgata ttcagtatgt tcctcaactt acaataggat taagtttgga taaactcact 29700
gtaagttgaa agtgaatttt caacttacaa taggtttatg gggacaacac tgtcataagt 29760
ccaggaacat ctgtacaaat aagttcagta acttcaattt ttatggatgt tttgtggttt 29820
tcaaagttct cagttgatcc ttataactga gatctgaaat agagtaagta ttactgccaa 29880
tttgtagatg agggaactaa ggagctgacc ctcgtaagta gaataaacca aaccaagtgg 29940
gaactagttt gccggcattc tttggttttc agtcataatg cagactttta caaaactaag 30000
tgttctgcag ataccttcca ttgtgtacat aattttgatg tgcttagttc accctcatgg 30060
cattttggca tttatattgg tattacgctt agctttcttt caaagtacac taatatttga 30120
catggttttc aatgacccca tattattcac tgaatgagaa gggtatatcg agctgctctt 30180
ctgggtcttt gcatatatgt ttcagcaaat ccacaactaa atgattcttg gatttttagg 30240
gtgatctttt caccaagctg gcctgtgtac ctacaaataa agctgatgtt tgaatagatg 30300
aatgttttat tattaaataa aaatgaacaa gttcaggctt taaaaatact tcttttgttt 30360
tgatttgttt tctggaggtt agttccacag tcatatgaaa actgctgtaa ttgagttata 30420
atagtatatt gctattaggt tgtcatcagc tttttctctt ttcaattaac aggtgattcc 30480
atcttcctaa acaaatcagt tatcaaatga atggagcaat agtagatttg acagttcatt 30540
ttgtacttta acagtgtgtt ttcccagtgt tacagcactg ttggccctct ggattaaaaa 30600
gatgatcaca gattatagag gtcagaacat actattttgg ttgtggttga gccttcatac 30660
tttttctgta tatcactgat tactttatgg tcgcaaaaaa ataataaaac ctaattaata 30720
gtcatcctgg cctcagattc agatttttta tttgatgtag aaagactagg ggtccttatt 30780
tagaggttat taaatcttca tttcacagta gcaacattat tttcaaagat cagctaagat 30840
tatgtaccat gtgtagtttg ctagggctac cctaaccaaa taaccacaga tgggtggctt 30900
aaacagcaga aatttatttt cccaagatgc tgcattctat aagattgaga tcaaggtgtt 30960
ggcagggttg gtttttctga atcctctttc cttagcttat agatgagtct tctccttgtg 31020
cctttacgtg gtcattcctc tgtaggtgta ggtattctaa tttcctcctc aaataaggat 31080
gtcagtcata ttgaattaga acccacccat atggcctcat tttaccttaa ttacttcttt 31140

aagggcccta tccctaaata cagtcatgtt ctgaggtact gggagttagg actttaacat 31200

attaactgtt aggggacata atttagccca tagcatactt gtagcttttt gtttctttga 31260

cggatgtgat gtttctgaca aagttctcta tcctcaaatt cacagtttat ttctgtatcc 31320

ccagtgtgca gcacagtgtc tgataatagg atatgtgctt aatgaatgtt gatttgctgg 31380

gtgaataata agagtgcata tatgggaaga taaggtgata tagcacaact ttatcatacc 31440

aggataggga atttttttaa ggatgattta ctctcaggaa aaaagaaata tatcacttat 31500

aagtacatta ccacctattt caggctagat tgctgctact atcaagcttt tgaaatggtg 31560

cagaacctga agacagggag gtggagggaa gaaagagggt ttcagttgac aaaaagggac 31620

attataatag taaaaatcaa taaacatagc cttgggatgg ctgagaaatt ttaggcaata 31680

gacaagaatg gggcagggag cagatagaat aagacagaat aagtattgga aatggtgaca 31740

caggagggta tcacatactt tgagagtaaa aaagagctag tggcgtagaa atgataaaag 31800

tgttaagtag ctatatataa acacatacac acatgtgtat atgtgtgtgc acgcatgtat 31860

gcacatatgt gtatgtattt gtgtgtatat ctattatgta tctgtataac tataaatctt 31920

agaagcaccc aaatattgtt tttcaatcct ggccatgtgt ccgaatcaac tggggaggct 31980

taaaaaatac acatgcttac gttttcctgt agttctactg aaccacaatc tctgagggtg 32040

ggacccaaac aagtgcagta aataaatatt tcttctacag gtgattctta tgggcaacca 32100

gggcattaca aattgtctgt gtaaaggcac agtagtcata atttggtgat ataatatgtg 32160

gtagttgttg gcctaggaac atatgtcttt atttaaaaag gcaaatggct aagcattagg 32220

ttaatattca ggggttattg tgtcctttat ttgtgttgag tacaacacat actcagcaca 32280

gtgtaataaa aatactgaca atggataata tggaagaagt ttatattctt cacagtgtat 32340

gggaagatat gcttatttta tttagaaata ttgttatcac aaagttggtt atattcaaag 32400

ctgacacaat ttcattagcc agaatgaatc tcctgtttcc ttatttcctt tatatattta 32460

aaaaaagaaa tcgtgggtat atatattttt tgttttgtag actatattta agaataattt 32520

tgagatttct tattgtttaa ttttaccttt aaaatcttta ttttctattg tatatattta 32580

aggtgtacaa tgggatgttt tcatatacat tgtaaaatta ttatgattgt taagcaaatt 32640

aacatccatc atctcacagt tgccctattt tttggtggta agcactgtct tcattttaaa 32700

aagtatatat tttttcctgt ttcttttata atattgagca gatttctttg tctttctgct 32760

ctcatgagat caaaaaagta ttgtgctaag cattaggaat aaagagatca atgtggtgta 32820

atacctgcat ttaagaagcc cacagtccag tgggaaagaa agacatggta attagttgtt 32880

cggacacagt gtaacatttt acattggctg tctcctggga aaagtgattt ttctcatcta 32940

gttgcatttt catattggga gctttttaga gtactgctgc tgaaaattga aagacattgc 33000

attttagagg atgggcatgt gagcattatt aataaaagtt aaaagggcat tggacaaaca 33060

ttttcatttg gaaaacctgc ttaggtttgg tgaaactaac tatagtacaa tttgtttcct 33120

aactctgaac acttctaaaa aaactgatgc caatacttat agtgataatc taaacataag 33180

cagggacctg aagtaccttc ttgaacataa aaatattata ttgtgttcag tatagaatca 33240

cagacattga ggggacctta gagacccatc taacaaaacc cctttgtcat gtgagaaaac 33300

tgaaggagag agtgatttgt gatttagcca aggtttctta gcttataggt gcatacagat 33360

atattatact ttaatatagt ccccaaatgt cagctacaaa gcatctatat ttgcattaaa 33420

aaaggttgtt tactttttta aaaaaggacc tctttcaaat agggaaattc acttccatag 33480

ccgattgatt ttaatttaat ttttattttc agtaaacatt tataaattac catcactatt 33540

taaaagggtc tggtgaaata ctattttgat gtataagaaa catctgatat actttcaaaa 33600

tcagttattc ttcatttctt gttgatttgc tggtagttcc taacacctac aattaaaatt 33660

tctttgccta ggtacccccc ttgagatagt gaatgcctga ctacctatat tccatggaag 33720

ttattttagc tgctatgaag tctagctcat ttgggaccat actcttgttc actttggctt 33780

caatcaccat tgactaatca gttaattgaa gagtgaatag attgagaaca ttcttggcat 33840

ccaggctaaa attagtatgc tataatggaa tgctaccaag atttatagac attgtgatta 33900

agtagtcatg taaagttaaa ttctgtgggt cttagttttc ttctttatac agtcagagag 33960

ttggataaga tatcccttcc aactctgtta ttttgtgata tacgtctgtc atcctagcta 34020

ttgtgcttct gtagggacta gtattaatta cagctcactt gaatgtagtc atcacagctg 34080

agtggagtcc tgcatcaagg ggaagaacca ctcactgggt ggctttcttt ctgcttcacc 34140

caggtggtac gaaatctggc atataattta tgtataaaat ttcagtaaac gggccatagc 34200

ctggaaacat ttgttggggc tctgcagatt ttgttggtct ttgccagttt ctttttcatt 34260

gatgtggtta gttggccatt taaacattta aaatagtata gtgaaatact atttaaaaaa 34320

gctcttgagg tcacgttttt tggtcatgcc ccacaattta caaggtttta ttttgacttt 34380

ttctgcctct acctcattag tcacagtcca ttgaagggtc ttgagactgt tgttaacaat 34440

tttattatat tgctaccaag ctggaaggga aggctgaagc atagatttct ttttacattt 34500

tatttacatg aaattatgta gtagggaagg tggtggtggt gcatctgtgt gtgctgtatg 34560

tgttagaaaa cagggaaata aggatactgg tagcccataa aactctctgc tacattgagg 34620

gagttgggta tttctattat gacttctgat tttaaatgga actaatcaga agatgttgaa 34680

aatggattca agtattgttt ggatttcttt ttacaggatg aaggagttgt taaagaaatc 34740

cctattactc atcatgttaa ggaaggctat gagaaagcag atcctgcaca gtttgagttg 34800

ctcaaggttc ttggtcaggg gtcatttgga aaggtaatga ataatttttt tccccatttg 34860

ttcctcttct aaacatttag caaaaaaagt gaacacatac gtagaaatct gattaatgag 34920

tcgtttctaa acttctgaac ggatgctcac aaaggtaatt ctttaatttt attcctttca 34980

tttctgttgt acttttatta ccatttcttt gtacttctct gtaacaatca aggttttcca 35040

attccctagg cttactgatt tcttgaggga aaaatatagc atactcttta ttagctgcct 35100

aaatcaaagt agcttgattt ttgtaaaaaa tggtattcct atctccttgt tcccatgctg 35160

gcatgaatac agcttgtttt ctcctggtga tttaatctac atttgttagc tatgacacag 35220

aaatcagaat ttatgtctta attcatgtat gaattcgtct gtcatctgtt aaagtagaac 35280

aagggtgtgt aaagtagaac aagggtagtt actgggtgat atgtggttta tttaagtctt 35340

cagagagctt acatttcaag gcagaaagca aaattgagta agaataacta ttatagtcgt 35400

tactaaagtt gtttattaag cacttaataa catggtttta gaaaagaagt gtataggtct 35460

ggttcctgac ctttatatac ctaacatcta atacatgaaa attattgcat acacatagta 35520

acagccttta gaaaaacagc aatttagaaa aaccgcaatt taaggggaga aagataaaga 35580

gaaactacta atttcaagtg gctgagattt agttttaaag gggaaaaata aggcaattga 35640

gcataggtta tttatagtgc tctggaacat tttaaaataa agacattttt attttattgg 35700

gttttagaaa tggggtcttg ctctgtcacc caggctgtag tgcagtggca tgattgtagt 35760

tcactgtagc cttgatctcc tgggctcaag tgatccttca tccccagctt ctcaaggagc 35820

tgatactaca ggtgtgcacc accacaccca gctaattaaa aaaaaaaatt tctggacaca 35880

aggttttgct gtgttgctca ggctggtctc gaactcctag ccttaagtga tcttcccacg 35940

ttagcctccc aaggaaagac atttattgaa cgctcagtgt gttcacaaca atgtataagc 36000

agtattagtg actataaaat agatgtcatc ttaaagtaac ctgatattta tgcattatag 36060

aatataacgt gaaatataaa gaaacatttt caggacacaa aaaagacaaa ctgagatgaa 36120

ttatggcttg gcagtgaatt ggtacaggag atatataaga cagggagata ggacttgaac 36180

agtgttctga ggagagaaaa actagaagga actgttaata gattggtggt ggtggtggtg 36240

gtggtggtga tgaagttggt ggggaatgtt tatagaactg atctgtagag taggcatgta 36300

tattatatac aactttattt taaagtttct aaaagtgtaa tattctttta gctattttgc 36360

actttatagc tatttttatg gtgttaattt tagaagaaca gagatgaagt tattttatat 36420

tccagatgta taaatgctta aatatttgac ttaaataaaa tctcaccagg caaacatttc 36480

aagctattaa aattacctgc tttgtctttt gatgtatgta tttactcata tatgcttttg 36540

tttatcttca gctttgttcc agaaaggatt taaggctttc atagatgaat attaaataaa 36600

attaaaaata agtagtgaag atgatggaaa cagaaagctc agagcaggaa agattagatg 36660

aagccaggag tacactctgt tcacaaaagc atgacataag ggtctgtaag tccttttcta 36720

gtaggtcaaa aattagactt aagttttcta gcagccatta taaagaagct aatatatttg 36780

agtgtattat aattgacagt attttaaagg tggaaacgga ttaaacagac cagagaaatt 36840

tctctcatgg gttttcttaa agaaaatact gtataacata atgaaatata ttcaataaca 36900

tcatgttaaa tatagcaaca tgatgaaaag ttgtcaatgt agtgcttaaa ttcagggaaa 36960

tcttagcttt gtctgtgctg ggatttagtt ttaagaagaa tatcattgaa accttttaat 37020

tcctaatatt aaagtacaaa atagtgatct ttatgtagtt catcaaccaa agaaatatta 37080

aggaggtggc ttttttttcc ccctaggata tgaacatact tgcttgtcag tgttacatca 37140

tttgcggagg cattgtgtag gtgtgacttg ttatatgctg aacctattca ggaatttggc 37200

caaggctgtg aatcagataa gtgtaggagc aattccaaga gactttgagc tgcagagtct 37260

taagtttttc ttcaaaagtt ccattgttaa aatgcagtaa atgggatttt agcccatata 37320

aagccttata acattaaatg tttttattgt ctatttaaca gtgggggaac cctgctaaag 37380

tcaacttaaa attgtagttt aaggaagcat gcattattat catgaaaggt gcatatacaa 37440

ttctgtccta agcacaccaa gtataatgct attaatgatc attaatgtat gggttgaaag 37500

gtatactaaa caacttacat taattatttt aaaacacaaa cacatgaaaa tacctttgaa 37560

attgtctgat tagtcgtcct tatatatcat gtgccaaata atatttatat aaaacataca 37620

ttttaatatt tgtggattat aaaaaaatac ttttgctttt gtgatagtaa agactttaag 37680

tacaggactt aaaaatgaat ttttgaaaat atcgaatact tgtactctta aataagtgat 37740

caactctcaa gtgatagttt ttaaaaacat atgcttacat cttaaaattt cctgaagttg 37800

gttctttctc ttcaaacagc atttaaatat gtgtgatctt ctagttcact gcagttactg 37860

aatattctta taggaaaata taaacgtcca tgtgattgag agaaaattat attggagtaa 37920

ctcacccatt cttttggaca atttcccatg attggattag ggtgactgaa aagaagcctc 37980

ttatcaatat agatacgttt ttaggtccac tagtttgaag ccatgcaaca acttttttac 38040

tttaattatt tctttaagca ccctatcctc aaatacagtc atgttccaag gtactgggag 38100

ttaggacttt aacatattaa ctttgagggg acataattta gtccatggcg tacttgtatc 38160

tatttcttta aagggcatga tgtttctgac aaatttctct gtcctcaagt tcacagttta 38220

tttctgtatc ccccagtgtc cagcacagtg tctgatacag aatatgtgct taatgactgt 38280

taatttgttg attgaataac taatgagtac atatatggga agataaggtg atatagcaca 38340

actttattat accaggatag ggaaattttt caaggatgat ttaggaaaaa agaaatagat 38400

cacagataag tacattgtca ccttatttca agttaggttg ttactactgt caagcttttg 38460

gaatggtgca gaacctgaag aaagtgaggt ggaaggagga gggagagttt cagatgacaa 38520

agaaggacat tataatagta aaaatcaata aacatagcct tgggaaggtt gagaaatgtt 38580

aggcaataga caagaatggg atggaaaaag aactactttt gtagttgatt ttgggaaggg 38640

ggcagtgaga tacgatattt tctcatgcta ggaaagtcac tcataagacc tgaaaaaaaa 38700

gaaaaaacag atcaggtgtt aaacaggtta cttctctaga ctgcgaatga tggatcctat 38760
taaatgccat attgggatac tactgaatat tagaagaact aggggaaaaa agtggctcct 38820
ttatgcaaca gtgtcttcag aaagcctaga ctctttcttt cttccttgct tgcctcttct 38880
cactcttcat attccgttct ttacaatccc actaagggct atgttttgta agagtggttc 38940
tgaaattgtg gtctccgtac ccctggacaa tcccaagact tttcagggag tcctcaagtc 39000
aaaactactt cacagtcatc ctaagatatt gtttgtacaa ctgctggcat gttggccaat 39060
caaggcagtg acttcaaatg gtaccagtag tcattgtagt cttcatttta ttacatatgc 39120
agtttaagaa tgtccttgat gagacattaa caattattac atttattaaa tctcaatcct 39180
tgaataccct tcttttgaat aatatgcgat gaaataggaa gtacatgtga agccccttag 39240
ccacgtaaca aaatagaatg gttgtctcaa ggcagaggac ttgtgcaaat tttttgagtt 39300
gtgagctgaa ccaaccactg tttttatgga tcaccacttt tccttgaaag gacaataaac 39360
tatggttacc taaatatgct tatttcgtag gtgttttctc aaaaatgaat ggagtgagcc 39420
tgttcataga aaaacaactg atactctttt tgtcaatggt agaatctgag ctttccagca 39480
aataattgaa tttaggcaaa tgtatatttg ccacattgac gtcattttct tgatgcagct 39540
atacttattt ttattatgct tcccttcatt gcacttcaca gatatccttc acttcattgc 39600
gcttcacaga tatgtatgtg tgtatatata tgtatatgtg tgtgtgtata tatatatatc 39660
tatatatatc ctatatatct atatctatat atatcctata tatctatatc tctatatatc 39720
ctatatagct atatatatat ctatatatcc tatatatatc tatatatcct atatatctat 39780
atatatccta tatatatcta tatatatcta tcctctatat atctatcctc tatatatcta 39840
tcctctatat atctatcctc tatatatata tcctctctat atatcctctc tctatatatc 39900
ctctctatat atatcctcta tatatatatc ctatatatat cctctatata tatatcctat 39960
atatatcctc tatatatata tcctatatat atcctctcta tatatcctat atatatcctc 40020
tatatatata tatcctatat atatcctcta tatatatata tcctatatat atcctctcta 40080
tatatatatc ctatatatat cctctctata tattctctat atagtccgtg tctcatattt 40140
tggtaattct tgcagtgttt caaatgtttt ccttactgtt atatctgtta tggttctttg 40200
tggtctgtga tctttgatgt tagtgttgtc attgtttcgg ggtgccatga accacgcaca 40260
cataagacag tgagcttcat tgataactgg tgtgtgtgtt ctggctattt tactgaccag 40320
ctgtttcccc atctctctct gtctccttgg gcccctctat tccctgacct gcaagaatat 40380
tgaaattggt ctaagtaata actctatagt ggccttggag tgttcaaggg aaaggaagtg 40440
ttctgcattt gtcactttaa atcaaaagct acaaattatt aagcttggtg aggaaggcat 40500
gtcaaaatct gaggtaggct aaaagctaag gttcttgtgc caaacagcca agttgtgaat 40560
gcaaaggaat gttcttgaaa gaaattaaaa ttgctactcc agtgaacata taaataagaa 40620

gaaagtgaaa cagccttatt actgatatgg agaaagtttt agtggtctaa atagaagatc 40680
aaagcagcca caacactccc tctagccaaa gcctaatcta gagcaattcc ctagctctct 40740
ttaattctgt gaaggctgag agaggtgagg aagctgcaca agaaaagttt gaagccagca 40800
ggggttggct catgaagaga aaaaggaaag aagccatttt cgtaacataa aagtgtaagg 40860
taatgctaca gatgctgatg taaaagctgt agcaggttag tcagaagatc tagctaaagt 40920
aactcttgaa ggtggttaca ctaagcaaca ggttttaatt gtagaagaaa cagccttctg 40980
ttggaagaag atgccatcta gcctttcata gctagagcga agtcagcgcc tagcttcata 41040
gcttggaagg acaggctgac tctcttgata gaagctagtg atcctggaac ttttaagttg 41100
aagccaatgc tcatttgcca ttccaaaaat tctagggcct ttaaaaatta tgttaaatct 41160
actatgccta tgctctataa atagaacaaa gcctgaatga cagcacatct gtttatagca 41220
tggtgtgcta agtattttaa gcccactgtt gagacctact gttcagggaa aaaaaaagac 41280
atttctcaaa atattactgc tcattggcaa tactcctagt tacccaagag ccctgataga 41340
gatgtacaag gagattaatg ttgttttcat gcttgctatt acagtatcca ttctgcagcc 41400
catgaaacaa agaataattt tgactttcaa gccctattat ttaagaaata cattttgtaa 41460
ggctatagtt gctgtagatg gtgattgctc tgatgaatct gggcaaagca aattgaaaac 41520
ctctggaaag gacttgctat tctagatgtc attaagaaca tttgtgattc atgggaggaa 41580
gtcaaaatat caacattaac aggagtttgg aagaagttga ttccaaccct tatggatgac 41640
ttggaggggc tcaagacatc attggaggaa atcactgaaa atgtggtgga aatagcaaga 41700
caattagaag tggaactaaa gatgtgacgg aagtgctgca gtctcatgat aaaacttgaa 41760
tggatgagga gttgctcctt gtggatgagc aaagaaaata gtttttttga ggtgaaatct 41820
acttctggtg aagatgctat gaacattgtt gaaatgacag taaaggcttt agaatattac 41880
gtagacttct ttaataaagc ggtaggattt gagaggactg agttcacttt tgaaagagat 41940
tctactgtgt ataaaatgct atcaaacacc atcaaattct atagaaaatt atttcatgaa 42000
aggaagtcaa tctgtgcaac aaacttcatc attgtcttat tttaggaaat tgccacagcc 42060
accctaacct tcagcagcca ccaccctgat cactcagcag ccatcaacat tgaggcaaga 42120
ccctccacca gcaataagat tatatctcac tgaagactca gatgatcatt agcatttttt 42180
agcaataaag catatttaat taaggtatat agggtcataa tatttgtttg ctctttatta 42240
aggtaacaaa aagtgggcct gggtcttaaa aggaggtatg tagctcaagt ttgtctttca 42300
ttatgttatt tatctcttac atcacaaaat tctcaaagtt cagtaccagg tcatctattt 42360
taaaaatttt cctttctata gtagtattta tatgcattat gatttattaa atcataacag 42420
tttttttgat taaaaactaa attaaatcta agaaaagtga gaaaataata atgtaaatta 42480

tagaataatt tataggagaa ggtataactt ttttcattta tttttttaaa ttgacaaata 42540
gttgtacata ttcgtggggt acatagtgaa gtttcaatac gtataatgta tggggatcag 42600
atcagggtaa ttttgcatcc ttcaacaaat ctctcactat ctctcccttc cctctatggg 42660
aaggcatatg tttataaaat tagagaaaat gcaaggatat aagtgtatgc tggtgtgttt 42720
gtgtagagca agaatgacta gaaatgtgaa ttttcctttt tttttttgtt caattttcta 42780
ttaggttttt cttgttagaa agaagaccgg tcctgatgct gggcagctct atgcaatgaa 42840
ggtgttaaaa aaagcctctt taaaaggtag aaaattagaa agtttattaa aatagaattt 42900
gacaaagtgt agtttaagaa atatgctgtg tattacttgc ataaggtgaa tagatgtagc 42960
tatttttaac attggtattt gtggcagttt ttctaacctg taggtaatat tactagattt 43020
gatattggca tatgccttaa cttagtgaga gtgcaccccc gtaaaaattt gatgctagaa 43080
aaagtgacat atttcattta atttaaactt attaacttaa ttaggatata ttctcttctt 43140
tcaagattct tggtttttcc ttttctatct gtgtcagtat tacatattga gaaagcacaa 43200
ccatttgcac acactatctt ttagggtgtg ataaaaatac cgcttatgat cagttttaat 43260
cagtaaactc atttgaacaa ttaaaagaga ttaataggcc gggcatggtg gctcacacct 43320
ataatcccag cagtttgaga ggctgaggtg ggctgaaccc ttgagcccag cccaggagtt 43380
caagaccagc ctgagtaacc tggtgaaacc ctgtctctac aaaaattaca aaaatgagcc 43440
aggtgtagtg gcatgcacct atagttccag ctgctcggga agctgagatg ggaggattgt 43500
ttgagcctgg gaggtggagg ttgcagtgag ctgagattgt gccgctgcag tccagcctgg 43560
acaagagagc aataccctgt ctcaacaaca acaacaacaa aagatgaaca cataataaat 43620
gcatattagc tacatacctg tttatgccac cttaacctgt ctctagatgt aatgaatatt 43680
tttgctttct gagaatatgg tcattatcta ggaatatgaa aactgcaaat ttcggcattt 43740
aggaagttac gttttgtatt tttatatttg ttgtttggta aattattgat ggactcttaa 43800
ctgaaatgat gtaagagagc aatatattgt acctttatgt aatttttagt tcgagacaga 43860
gttcggacaa agatggagag ggatatactg gtggaagtaa atcatccatt tattgtcaaa 43920
ttgcactatg gtatgtaatc tttttttatt cttttattta ttttaatcct tatttattaa 43980
tatggaatta gagaattatg atttcctttc acacaatact ataacaggaa ataaatactt 44040
tagacttagt atattcagct acgtaaaatg tatttgtctg aaataaatat cagtatttag 44100
ttattagtta tatgtccttc atttttctaa gttgattaac ttgtttaaag taacaagata 44160
gtgtttttca cttacctgtc tggtagtatt tatgtacttt gctaggctgt aacttcataa 44220
tcttagacta taaactattg tgataattta cgtaaaacat tcagttgctt gttcataatg 44280
accacactta tctgtttcct ggttatattc cagaggacca tgtagttatg ttagtctact 44340
tgagttattt ttttatactt aatgggagag gactaccagt aagaagcagt agatgtaatg 44400

gaaagaacat gagctttgga gttagacatt tctgaatttg aatcatggct ccattgctaa 44460

tttaacatat ggttgagatc aactctatct atttatctat cgtctatcta cctctgatat 44520

agtgtacttt gtcacttcca tttttctata tttaaaaaag tagcagctgt tgattttatt 44580

cttagtgtaa ttttgacaaa taatacatta aaatatatac ccagttatca ctattaaaat 44640

cccataagaa atttccaccc attattcttt tactccttgt tatactaaca gaataaaaaa 44700

agtaacatct aaagaccttc ccgtgtctgt agtaggtcca tcagtaaaca cttattgtgt 44760

gcaaaaatac tgtattaaat tcagtgggct atagaagtga aacacttttt gacctcaaga 44820

aatttataac ctatagagaa cacagagatg taaagaaatt aattgcataa atcaaaatgg 44880

aataagtact attaaggagg tataaatata ataagatctg aaaagtagca gcaagtaatt 44940

tcaataaaga agaggttgag gaagctgttg aaaaatgtga aaagggggaa atcctggaaa 45000

gtttaataga ggaagtggca tttttcttca gctttataga atttgtagat tttcagtagg 45060

tttgataggc taagtttagg caattgcagt ataacatgct tcataaatta ggaaattttt 45120

ggtggtagaa gttctgtgta aagccacatt caagggtgat tatgttctgg gaaggggttt 45180

atagctattt ctacactgga ttaaacactg ggtttaattt tgagatgtta tccattaatg 45240

aaccatgtga gtaaatactg taaaattcta aacatatatt aatttaaaag catactaagc 45300

tttttaagcc tttgagatta tcctgttact atattctaat ttttaattag cctttcagac 45360

tgaagggaaa ctgtacttaa tactggattt tctcagggga ggagatgttt tcacaagatt 45420

atccaaagag gtaagtatct gtacattttt actgtatttt cttaaaacaa aactaagaaa 45480

aatcactttt gttcttctgc taattacatt tttcctattg aaattttaaa tggtaactac 45540

tctgtttcat tgcatagtat ttggtaatgt gcctgttaat ggcattttct atcatctcaa 45600

tgtgctactc attattttct taggcaaaag aacaatatga ataactggat aaagtagata 45660

cgggcaagcc taggactttg gctactcaag ctacctaaac acctctggga gtagtgttta 45720

ttcattttaa aatagcttta tggaggtata attgacatac aataaactgt acatactaaa 45780

gtatacaatt tcataagttt ttacatttgt atataactgt gaaaccatca ttacaataag 45840

aataattagc atatctgtca ttcccgaaag ttttcttgtg tccctttgtg atgccgtcct 45900

cctgccctaa ccctactgaa tctgctttct gtcactattt ggtttgtact ttctaggagt 45960

ttatataaat ggaatcatat agtatattca ttttttttgt ctggcttaac ataagtactt 46020

tgagattcat ctatgttgta tgtactaata gtttattcat ttttattgat gggtagtatt 46080

actgattgta tggatacatc atgatttgtt tatcattcac ttgttgatgg atttttgggt 46140

cattgcagtt tttgatatta ctaataaaac tgctataaac attcatgtac aagtgattgt 46200

atcatatgtt ttcgtttctc atgggtaaat acctaagagt ataatgactg ggtcatgtgc 46260

taggtatatg tttaactttt aaagacactg ccaaactgtt ttccaaagtg attatataaa 46320
ttttacattc ccaccagcag cgtaagagag tttcagttgc tccacatcct tgccaacatt 46380
tggtatggtc agtcttttaa aatttttgaa cattagatta gtttttagtg gtatcccatt 46440
gtggttttga attacatcac actaatgact aatgacattg agcatctttt catgagctca 46500
tttgccatct atcctcttgg taaaatgtct gtttaaattt ttagcctgtt tttttttatt 46560
ggattgtttg ttttcttatt gagttttgag agttcttcat atattttgga tgtaagtcct 46620
tgatcatata tgttatttgg aaatattttc ttctggtcta tagtttgtct tttcattctc 46680
ttaacagtgt attgaaaagc agaagttctt aattttggtg aagtctgatt tataggattt 46740
tttcccttta ttaattgtgt ttttagtgtc ctttgtaagg catatctgcc taatcacagc 46800
taaagatttt ctcttatgtt ttctagaggt tttgtagttt gcattttaca tctagattta 46860
ggatccgttt tgagttcatt ttagtatatg gttacaggta tgtgcagaag tccttttcat 46920
ttgttcacat agatagccag ttgttatagc atcattattg gaaaagtctg tcttttcttc 46980
actgaattac cttacatttt tcttgaaaac cagttgatca tatatatgaa ggcctatttc 47040
tggatactct gttctgttca ttgatccatt tgtctatctt tatgacaata ccacactgtc 47100
tgaattactg tacctttata atatgtcttg aagtgaggca gtgcgagtcc ttgagctttg 47160
cttttctttt tgaaagtttg gctcttctag gtcctttgca tttccatatc aattttaaaa 47220
tcggcttttc actttgtaca aaaatgtcct gctgggatat tggttggaat tttattgaat 47280
gtataggtca atttgcagat aagttacatt ttagtaatat tgagtctttt aattgatgaa 47340
cgtggtatct ttctcaattt atttaggtca tctttaattt ctcgcagtat tgttttcagt 47400
gtaattcttg caattctttt gtcagattta cctctaagta aatcctgttt ttaatgttat 47460
tgtaaataga attgacttcc taatttcaat tttaaattgg atggaaacac aaccgatttt 47520
ttgtgtattg agcttgtttc ccacagtcta gccaaactta atacttctag cagcttttag 47580
aaatagattt caaaggttga gaaagttttc tgtaactatt tttgagagtt ttataaaaaa 47640
tcaggaatca atttcagatt tattcaaatg ctttttcttc atttattgag attataattt 47700
tttttagtct tactgtggtg aattacatcg attgatagtc agatgttgaa ccaaccttgc 47760
atttatggat gaaccccact tagtcttgat gtattaccct ttttataaat tgttggacga 47820
tatttgctga atatttaaga atttttgcat ctatttttat gagggatatt tatgattttc 47880
ttgtattatt atgtctattt ttggtattag agtaattctg actccataga atgagttggg 47940
tgtagttttt gcctctttaa ctttctggga aagtttatgt agattcaatg ataaattgcc 48000
cttaaatgat tgatagaatt caccagtgaa ggcatctgaa cctgaagttt ttcttatagg 48060
aaagtattta actccaaaat caatttatgt aatagacttg ggttatttat ttttgagtga 48120
gctttgctaa cttgtgtctt ttaaggaatt tgtctatttc atcaaagtta tgaaatttat 48180

tggcataaaa atgttcataa tattccctta ttgtcctttt tgtagttgtg gaattgttag 48240
tgatgtcact gctctcactt ttgatgttgg taattcatgt ttgctctgtt tttgttctgc 48300
ttagcttcac tagagattta tcaattttat tactctcaaa gaaacagctt ttggtttcat 48360
tgatttttct gtattctatt tctgttctat tacattgttt tctgctttaa tcttttttac 48420
tgcttatttt atgtattagc ttttcttttt ctggttttta agatgggagc tgaagttatt 48480
gatttgaggc ctttcttttc taataatgta ggtgtttagt gttataaatt cccctctgaa 48540
tactgcttta gggtcatctc acaagttttg atatgcatca tttttatttt ctctcagttc 48600
agtgtacttt atgatttcct ttctatttct ttgacccatt gatatttaga agtctgctta 48660
atttacaaat atttgggcat tctttcacat atctttctat tgttgatttc tactgtaatg 48720
ttcttgtggt cataaagcat actttgtatg actttaattc ttttaaactt atatttgctt 48780
tatgcttcag aatgtcgtgt atcttggtaa atgttccatg tccacttgag aagaatgtgt 48840
attctgctat ttttgggtgg tggaaggttc tataaatgtg gactggatca agttgtttga 48900
tagtgatatt aaattgccta tattctttct aattttctgc ctattctaca aatttttgag 48960
acaaggattg acaccttttt actttaatta tgaatttgta tatttcttct tgaatttctg 49020
ttagtttttg tttcatatat ttcaaagttc tgttattagg tacataaacc tttaggttta 49080
ttattccctc ttgatgaata gaccccttca tcgttatgat atgacatgac tgactttatc 49140
cctggtaatg ttcttttctt caaaatctta tttgatacta acatggtcac accaactatt 49200
ttctaaaaaa gtagtgttag tttggtatat ctttttccat attttaactt cagcctatgt 49260
gtgtctttat attttaatgt gtggtactta aggaagcata gtttccttgt tttttaatcc 49320
aatctgagag tccacgtctt ttaattggga tattagacct gttacattta atatgattat 49380
ttttgtagtt ctatttaaat ctactatctt gctatttggg attttttttg gtcccatttg 49440
ttctttgttc ttgtttccct cttctcctgg cttcttttta attacttttt ttttttttac 49500
aatttcattt ttatctctgc tgttgtcttg tagatattat ccataacact ttgttgtgta 49560
atttaatagt taccttagga tttgtagtat aagttttaac tcagaggtca ggaaaatatg 49620
gtcttcaggc taaatttggc cagttatttt tctttgtaaa taaagtttta ttggaacaca 49680
gacatactca tttgtttaag tattgtctgt gccttatttt gtacttcaat ggaatcatac 49740
ttgcatagtc atttttactg gatagcaggc atggtggatt taattttctt acatgctaga 49800
tacctttatt ttcatataaa tagtatttag cttttttcta ggatgcagtt cattttcttg 49860
taaacacttt gatcattttg ggccttgtgt ttaagctttg tcatgtggga ctagggaagt 49920
atttagttta agattaattt ttcctcagta tgaagcatgt cctttcttat actctaacaa 49980
agactctttg aattatgagg cttttcattc taacttttgg gaagaggagc tatctcagtc 50040

ctttgtgagc tgagtgggtt ctttctttta atcttttaag gtttttcttt tcctgggctt 50100
gggcttgggt agtttcctca gatccatgtt tgactatata tatatatatg tatgtttttt 50160
tttttttttt ttttgagatg gagtctcagt ctattgcccc ggctggagtg cagtagcgtg 50220
atctcagctc actgcaacct ctgcctcccg ggttcaagtg attctcctgc ctcagcctcc 50280
tgcatagctg ggactacagg tgcacagtta gcccggctaa ttcttgtatt tttagtagag 50340
atggggtttc accatgttgg ctaggctggt ctcaagctcc tgacctcaag tgatctgcct 50400
gccttggcct cccaaaatgc tgggattaca ggcatgagcc ccagtgcctg gcccatgatt 50460
gacaattttt tattagatgg cagacattgt gacatttagc ttgttaaggt gatgaacatt 50520
tttgtatttc tgtaactatt attgatctct gttctgagat ataaaactag tttgattatt 50580
ataggtcttt taaagttcgt tagattggac taacacagct ttcagtcggg ttaattttgc 50640
tccactactg aagcaaaatc cttctgatta ctttatctga tgtcctatga attatgagat 50700
tttctgctct ggctgaaggc aacaggaact attcctggcc catcgtgagt tcatcgactg 50760
gttcctctaa cccttcgttg ttttcctggt ttagggtaat gtcctcactc atatgtgctg 50820
agtatttctc cactgaatac ttgaaggaga ctgtctgcat atctacagag ttctctttgt 50880
gcaattttct cttctctaat actccatcct gtgaattttg tcctccttct ctaagactct 50940
ctgttctggc ttatctactc aggaagtctt tcaggctcca cctgacttag ccctccctgt 51000
actaccactt ggaaactctt tccaggtagt aagctaaggc agttgtgggg catacgtcat 51060
ttgtcccctg tctctcagta ttcagtgtcc tttgttgcct gatgtccaat gtcttaaaag 51120
tatcatttca tacgttttgt cctgtgtttt agatttttca ggcatgagat taaatcctgt 51180
ctttgtctgc ttcatttttc tttaagtagt agtctgggag tagcatttta attgggtgat 51240
agtagttcaa tgtcttactt ccagaattta cagtgtagaa tgtgggagag ctactgttga 51300
ttcccagagc tctggaactt gctgtgataa aggaacagtg tgtttctggt atgctagcca 51360
gtgcttaagt ggctaaggtc tctggcttat atctgtctac ctccagtgta gaatgtgcca 51420
tgcgtgagca tagggtgtgt tattggtata ttattaacta tagaacataa ttcaatatct 51480
gaaaaggatt gtctaggacc cccttctaag gagggtcttt aaaggacctt gttaaaaact 51540
ggtaccaagg catagaagga caacagtata ataaggctcc attattcatg atttcttgcg 51600
ttagatttta gaagtgtcac aaatatgttt gagaaattct tgtagtcatt tctaattttc 51660
ttttaaactc ttcagacctc cattctcgat gtgggactat ctaaacccat actagcacag 51720
agatgcagtc agtaagtttt gattcctaag tctggaatat taggtaagag caagcatttt 51780
aaatgcgtta cccaaaagat gataaattag tacattgatt gagcagcctt ttaaaaaatg 51840
attcaatggt tacttctgta gacagcttat taataattag atgtttgttg gttatacttc 51900
acttgtgtgt acaaaaaatt gttaatccaa aatgaggaca tgtttttata ccttagaaaa 51960

agacttttta aatacatttt tgtatgtttt aaagaatttt ataatgtatg tcatttgtat 52020

tttaaaggga aagataccta cccaaatttc ccttcctcag aatgtacaca cacacacaca 52080

cacacacaca cacacacaca cacacaaata cacaggtaaa caaacagaac tcttaagctg 52140

tgctctattt tccttattca agaaaatgtg gtagaatgaa tggctgatta ttgtaaagct 52200

gtcagttctt ctaaaattaa tttattggct taacaaaatt ccaaataaag ccacaatagg 52260

gatttttgtt tgtttgatgt tgacagaatt attttatagt ttccttggaa aagtaaacaa 52320

atgcaaagaa ataatatttt tgtttgtttg ttttttgaga cagggtcttg ctctgtcccc 52380

aggctggagt gcagtggcac tatcctggct cattgcagcc tcaacctccc atgctcaggc 52440

aatcctcatg cctccgcctc ccgaatagct tggactacag aagcgtgcgc cactatacca 52500

ggctattttt ttttttcagt agagatggga tcttgctatg ttgcctaggc tggtctcaaa 52560

ctcctgggct ccagtgatcc tcacgcgttg gcctccccaa attctcttgt ttaaaacttg 52620

gtaatgtgca taagcaaaac attatagtat caacacaaga ttggaaacat actgtatagc 52680

ccaaaaatat cctgttatag attacttacc ctatatcatt atgataaagg aagaatcaca 52740

aattgtggca aagaggttgt tcaataaaca gtatttgaga aattgcttaa atatttccaa 52800

aaatcatttt aaaatgccat ctctaccata tacaaaaata aatcttactt acataaaaaa 52860

gctaaatctt gaaaaacaat taccctataa taaaatgcag ctaaatattt tgttgaccat 52920

tggatggaga atgtctcctt aaacattaaa ataatgaagg aaaaatcata cagaaatcat 52980

catacatttg actagcaaaa attaaggatt actgaacatc aaaaactagc acaaaattgg 53040

ataaatattt gcagctaata tgatagaaaa ctgacatttt taacatttaa gtgacttgta 53100

ccaattgaca aaacagtaag tcagtaatag aaaataggca caaacattac tgataattca 53160

caaaaaagta gaaagactac taaacatgaa taacaatgtt cagcctaaaa atcaaaactt 53220

aaatttaaaa aataatgcca ttttcacaca tcaaatttga aaaggctttt tacagggtat 53280

attccatgct atcaaggatg tggtaagaca gatgctttcg taaactgctg atgggagtgg 53340

aaattactat aacctttttg gaaaataatt ttatcgtgta tgtcaggaat cttggactat 53400

ttatgctcct tgacccagca ttccattttt aaaaaaaaat tcttaattca ttttgagaaa 53460

atgatcataa atatcagtaa gatgtacata tgcatattgc agagttattt caaatagaga 53520

aaaaatggaa ataacctaaa tgtcaagaag gggaatgttt aggtaaatta cagtaaattg 53580

gtatggttat attgtgcagc tattgaaaac tatttataaa taagttttaa agacatagaa 53640

tgcttatgat atagtattaa ctggggataa atgaggtcac taaatagtac ttatacagta 53700

gtgtgatttc aactgtatta aaatacacat ggacacattc acacacagat atgcacaaaa 53760

aatttgaaaa gcagatgaca aaatattaaa agtgctttac tcaagagtac tggagtttag 53820

gataattttc attttgtagg gggggcaatt tatgagtagg acgattagtg ggttggaaag 53880
cattattggt agtaaaccta aaatttgttt ttagaaaagc aaacattgca tcacagcatg 53940
tggcagaagt ggcttctggg aaagagttca ttcttatgga caagtatccc tgagttcatg 54000
tcttgggcct ccttcttaac tagctgtgtt attttggaca agttacttaa tcactctgag 54060
tttctttctc catttgcaag gtgttcctaa tacctacctc atagagtttg gaagctataa 54120
taacataatg attgtacagt gcctggcaca taattgctac ttagtaatgt tttatttgca 54180
tctatcatga ggctttctac caagacaacc tcatgacaag gtgataggct tgcataactg 54240
taactaggca aagttttttt ctaatttagt tttaaataat ttttatttag ggttttctta 54300
cgtattagct ttcagagaac attcttatta tccttggtta gaagttgaat aggcaacagt 54360
tatttttatt cttaataaaa agtcaagtga aagtgatgtt tttatatttt taccattata 54420
atattcaaat taatgctccc aaaacttaga taataaacaa tctttgtttt tctattcatc 54480
attttaaaaa gtagttagca tatcagaagt tattaactac taattctctg caattttact 54540
agtaacctgg atctgcattg tgaaatgcag tggccgtaat tctaaatggg caagcaggtt 54600
gtaagctatt atgacatgta ctttatgcaa ttttgagcct tcttaataat ccagaggttt 54660
ctttatgaaa ataacccatt tttagaaaaa tatttgattt tgccaatccg gagttcttgc 54720
taaaccttaa aaattacatc taatggctga ttagtgtcca gttctatgtg taaatgaata 54780
ctcactagtg aatgaattta gatgtaaacc tataataata gtaccatatg gttatatata 54840
tagtttttta aagtttacaa agcttttttt ccacataaac tgatttgatt cttacgacaa 54900
ccttgttagg tatatacagc agatattatt gtcctgtttc acagatttag aaactgaggc 54960
ccaaagggct aagtgacatg tctgatatta cactgccaat tctgatttca agttcattac 55020
tcttcacttc atcatagcag ccgtatttcc agacacaaaa tgattaatac agtgccatta 55080
cactcgtata cattactaac agaagaggct tggatccacc ccttaggtca gagatgagtt 55140
cacaattgtg tgtaagataa gggtactaat ttaacaactt gtggcagtgt agtaaaagct 55200
ttgggtttgt agagccccta aaagtgaaat ttctgagaaa tgatactaga cagtaaaaga 55260
gagaatggta taaatagtgg aattctcaga gctaaaacaa aataacttct atatttcaaa 55320
taccctaagg gttactatat tatagcagat tactataatt aatattggta ttatgaggca 55380
aagtcacttt ataaaaggtg aatcttgttc tttgcatttc gtgtgagaag atatacaaac 55440
aacataatat ttcagaaaga tttacattaa aatggttaat atcgaagaga cacattctta 55500
aagagtttag tttcatttat atagaaatct tatttctctg gtattgccaa taattatgcc 55560
agtcaaataa gaattgactg tgcttattat cttattattt tcctagattt gtctgtctac 55620
tcattttgaa agaagtattt ttaaatatac tgaatattga aagaaagatc aaatgataaa 55680
tcattatttt gttctaggtt ctgtttacag aggaagatgt gaaattctac ctcgcagaac 55740

tggcccttgc tttggatcat ctgcaccaat taggaattgt ttatagagac ctgaagccag 55800

aaaagtaaag ctgtattctt ttgttttcat gttgtctgta tttctggaac tgctttttgt 55860

caaactctgt tagatctttt aaatattata gggaattcta tatgaattat atgaaactta 55920

tttgtaaaaa gtaatctttg tgagacgtac attgacttta agttaatcat ttaaattttt 55980

tacatatatt aaatagatac tgacctatct tagttttatg atttttatgt aaatgtttta 56040

tgtaaaatat tttattccat cttgttgaat gtgaattttg atttacagca ttttgcttga 56100

tgaaatagga catatcaaat taacaggtat gcagattttc ttattatacc cttagccact 56160

tgattcatat tttttattat tttaatataa atttgtctaa gcctgttttt ttctctttct 56220

gaatttgatt tgcaatttat ttgtctcttt agtgaattaa atgttacttg atggttagac 56280

tgctctcaat atgttctaat tgtaatatta aaaaattgta cattagatac acttatcctg 56340

aataagaaat gatcgttttc taggtaacaa tacatcatct tagtttgagt ctagacagaa 56400

agatgtatca caagtagtca tcacatttga aatatagtga caaccttccc tccaccttcc 56460

ctaactcctg gcaaccactt atatgtccta catctctata attttgttat tttgagagtg 56520

atatagaaat agaatcatac agtagtcaca ctactgagca tttatcctag aaaaataaaa 56580

acctatgttc acataaaagc ctgtatatga atgttcataa caactctatt tgtaatagcc 56640

caagactgga aattacctaa atgtccttca gaaggggaat ggataaatgt aaattgtcat 56700

acttccatat gatgacatac tactcagcag taaaaaggaa agacctgttg agacatccag 56760

caacttggat gaatctcaag ggcattgtgc tgagcgagcc agtctaaaaa cgttacatgt 56820

gttttcaata gatagtaaat tctgtatttt attatttgaa tgcttgaaca gagataagat 56880

gaagatgtag tctttacaga taattttaaa taggtacagt ttaaaggtaa gttgctttaa 56940

attcctgtgt atactgcatc aatgcaaaaa taaaatatca aacagttacc tcaaaagctg 57000

acataaatat gaaaaagttc aaataaagtt tagtgactct tattgattca cagcctgatt 57060

atctttttca tgtacaaaag aaatgatgtg ggttcgaaga tatttaaaga gaaaaaggaa 57120

aggaaaataa cagtagggaa cagtagagtt ttgagatttt tatataatac ataaatttta 57180

ttttatagca attaaaaaga taattaaaaa ttaattcctc ttttcatttt tgcttaataa 57240

agtatacctc ttttgtcaga ggtgagaaag agaggtggga aaaagagtag aaaaaaacct 57300

atttttctct ctctacatta aggttaatta cgtaaataat gatgccaagt ttatttgtat 57360

ttagtgttct cattttaatt ctgcttttga tagattttag agacttaaac tgtctgagga 57420

gggaagaata gataggatca atattaattt ataagcaagg gacacaaagc atgtaacagg 57480

aagaaatgtg atttataatt ttttttaaca attcaattta ttttccttgc aatggcacaa 57540

acatttcttt taaagaatgt gtacaataat aaatattaga taatttacag ttttatgatt 57600

ataaccttaa ttcaaggttt tataatgact acatttatgg atttaccccg acagcattcc 57660
aatgaggttg gttaaagtat gaccactcac agcaagtatt ttaacccatt tatgcctgag 57720
gctgcaatgt tttgaatttt tgcagtcaga ctttgatgat gaccttgagc agtagaatat 57780
aaataactcc cacatgctta gcgttccaat aatggaacac taggcataaa tgggtttaag 57840
agttctttta taatgtttct tttgatttat tttatattct ttatctttct ctttaatttt 57900
tcttgtttgc tttctttctc ctagggaggg tgtttgctag cattctgttt tccaaaaata 57960
attgcaggag aatggcaata cttttttttt tttcactagt ccactttaaa aattctccag 58020
acataaggaa tggacctagt atctggaatg acaggctact ctgaaattac aatggactat 58080
agaaagtttc ctgttgtaac tgtgctttga gtctgattgg cctcgttctg agattatgat 58140
tgttataaca gatcagtaga atgagagtgg atgagagtgg atattcccaa agatctacat 58200
ccttgctaat atattattta tattatatat tattatatta taaaacttaa gtttttttcc 58260
tgaaatcctg catccttttc atagtctgta taaaatccct tgtatagttt atataaaaat 58320
aactttataa tatgacttgc ctctttagaa aaattatttc atcataatat cccattaaaa 58380
agtacagctc gaatacagaa tttatttgaa ctgtggttgc tataatctgt tgcatttttt 58440
aaattttatt ttttttaaga gacagagtct tactctgtca cccaggctgg agggcagtgg 58500
caggatcaca tcttatcaca gccttttttt aaattattat tattatcaca gcctttaact 58560
cctgggctca agccatcctc ccaccccagc cttctgggta gctgggactt caggaacatg 58620
ccaccacacc cagctatttt ttgtgtgtgt ggcgacggag tcttgctata ttgcccaggc 58680
tggtcttgaa ctcctggcct caagcaatca tcctgccacg gcttcccaaa gtgttggggt 58740
aacaggtgtg agccactgtg cccagcctat aatgtgtttt ctgagagaca taattcagaa 58800
gcaacttcta caaagagagg gaagaaaaat atacagtggt cagagatgta aatcacacag 58860
tacttgagct gagtatttaa gagaaaaatg tatacttgag aaaataattc tatgtagttt 58920
ttatagttac ttgcaaacta atcactgaga ttgcttttat ttatagtaac tattcatcaa 58980
attattatat tctgagtgtg cttttaaagt ttaaagtctt agaaaagagt catttagatt 59040
acaaagatgt gaaacactga ccctcaaacc taccccccaa aataaccctt ccattatgta 59100
gttgaattgt acattttctt ttattttcaa attagtgatt tggaaaaacc actgtataac 59160
atatggcttg tctaatcttg tagctagttt tatagttgta caattttcgt cagttgattg 59220
ctgagcaaat tatagttgtt aaataacagt ttagtcagaa atagtacggc aaaggaactc 59280
tgtggattgg cataaatctc tgaaaggtaa cagaagaatg agaaaattct tctctccccc 59340
cttctctctg gaatggccaa agaaactagt gttcttctcc acaagctgtg ttatattcag 59400
ctgaactgac ctcgaagact agatgttaat tatatttact tttgtaaatg gaatgtgcag 59460
agtttacaag tttcagtata cagagagaac atagtggcac agtgaaaaga tttttaaaaa 59520

ttataccaaa agttaaaata cattaagcta ttatctaaga ataagtgatt tttttaaatg 59580
aaaatgttga gcatagttag aaaatcagtt ttgaggggac ctcaaattaa gtgttaatgt 59640
tgatatttta ttgtaaacaa gcaatatagg gcttataaac ttttttattt ttaaaatgaa 59700
agtatacttt acatatatta aaactcatcc ttctgatgtc ttttggtgtt gggactatcc 59760
cgtgtcttga ctgcatcaat gtcagtgtcc tggctgtggt tatagtacta tagttttgca 59820
agattttacc atgggaaaca ctggataaac aggatccctg tagtttttca tgtaacagca 59880
tgcgaatctg caatcatctc aaaatataaa gtttaatttt taaaaatcac ttttgttgta 59940
tgtatagttc tgtaagtttt gacaaatgcg tacagccttg taactatcac caaaataaaa 60000
atatgaaaca gttccataac cctcctcccc caaattctcc ccttataagt cagtcctccc 60060
tttaccccaa gccaactacc agtctgcttt ctgttcctgt atatatgctt tttccataat 60120
gtaattatga acagaggcat acagtataca gccttttgag actggcttca tttaattagc 60180
atagggcatt tgagattcat ccatattgtt ggctgtatca gtgagtgttt ttttaaattg 60240
ctaagtagtg tttcatttta tggtggtacc atgatttgtt tatccattcc tcaatgagga 60300
ggcatttgag ttatttcctg ctttgggcaa tgatgaataa agccactgta aaacttcatg 60360
tataggtttt ctttttacca taaattttta tttcagttaa gtaaatacat aaggagtgag 60420
attagttgat catatgttga gtatatttaa ctaagtgaga aaacttcaaa ctgatttcca 60480
aagtggttgt aatattttgt atacccaccc acaatgtcca gtggatctgt atcctcacta 60540
gcattttgta ttgttagtat tttttatttt agctattcta ataggcatta ggtagaatta 60600
tcatggtttt attttgtatt tttctaataa ctaatgacac taagcacatt ttcatatatt 60660
tatttctttg agaagtgtct attcagatct tttgcccatt aaaaaattgg attgtttgtt 60720
tggttttcag cctagaatgt tttatatttt ttaaaattta aatttaaatt ttgtgggtac 60780
atacgtgtat atatttatgt gttgtatgag atattttgat acaggcatgc aatgcctaat 60840
aatcacatca gggtaaatgg ggtatataca tcacctcaag catttatcct ttatattaca 60900
aacaatccaa tcatatgcat ttagttattt aaaaatgtat aattaaatta tttttgacta 60960
tactcaccct gttgtgctag ctaatattaa gtcttattta ttctaactat tttttgcacc 61020
cattatccat ccctcatccc ctaagtccct tcccccgtac ccttcccagc cactagtaac 61080
catccttcta ctctatttcc atgagttcaa ttattttaat tttcatctcc cataaataag 61140
tgagaacata agtttatttt tctgtgcctg gcttatttca gttaacacag tgacctccag 61200
ttccactcac aatgcagatg acaggttctt actctttttt atggatgaat agtactctgt 61260
tgtgtataag tgccacattt tctttatcca tttgtctgtc aatgggcatt tagattgctt 61320
tcttatcttg gttattaaaa acagtgctgc agcaaacatg ggagtacaga tatctcttca 61380

ttgtactgat ttcctttctt ttaggtttgt acccagcagt gggattaatg gatcatatgg 61440
tagctctgtt tttagttttt tttgaggaac ctccaaactg ttctccatag tggttgtgct 61500
gatttacatt ctcaccaaca gcgtatgagg tttccttttt ctctccatcc ttggcagcat 61560
ttttttattg cctgactttt gaataaaggc ctcattttag ggagaaatta tatctcattg 61620
tagttttgat ttgcatttct ctggtgatca atgatgttga gcaccttttc atatgcctgt 61680
ttgccatttg tatgctttct tttgaggatt ttttattcag atcttgtgct cattttttaa 61740
ttggattatt agactttttc ctgtagagtt gtttgagctc cttatgtatt cttgttacta 61800
atcccttgtc agatgggcag tttgcaaata ttttccctta ttctgtgaat tgtctcttca 61860
tgttattgat tgtttccttt gccgtgcaga agctttttaa cttgatgtga tccagtttgt 61920
ccatatttgc tttgatcacc tgtgcttgtg ggctattact taagaaatct ttgcccagtc 61980
caatatgctg gcaagtttat ccaatgtttt cttgtagtag ttttattgtt caaggtctca 62040
gatgtaaatt tttaatccat tttgttttta tttttgtata tagcaagagc tagagatgta 62100
gtttcattca tctgtatatg catatccagt tttcctagca ccatttattg aacacaccgt 62160
cctttaccca atgtatgttc ttggcacctt gtcaaaaagg aatttactgt agatacatgg 62220
atttatctct gggttctcta ttctgttccc tgttctatgt ttctgttttt atgacagtac 62280
catgttacta tagctttgta gtataatttg aagttagata atgtgattcc tccagttttg 62340
ttctttttgt gtaggtagct ttatctatac tgggtatttt gtggatccat ataaatttta 62400
ggactttttt ttctatttct gtgtagaatc tcattctact ttgaaaggta ttgtgtttaa 62460
tctgtagatt gctttgggta gtatggacaa ttttaacaat attgactctt tcaattcatg 62520
aacttggaat atctttccat tttttgtgtg tctttctcaa tttcttgcct cattttagta 62580
cattcctttt tacatgtctt taatttcttt ttatcagtgt tttgtagttt cattgtagag 62640
atattttact tcttggttaa ttactatgta tttgatttta tttttagcta ttgtaagtgg 62700
catcactttc tggatttcat tttcagattg ttctctgtcg gcctgtagaa atgctactga 62760
tttttgtgtg ttcattttgt atcctgcaac tttgctgagt ttgtcagttc taatagtttc 62820
ttggaggagt ctttaggttt ttccaaatgt aagataatac catcagtaaa aaaggataat 62880
ttgacttctt cctttctcac ttggatgccc tttttctttc tcttgtctga ttgctctggt 62940
taggacttgc agtactatgt tgattaacag tagtgaaagc aggcatcctc attgtgttcc 63000
aggtctcagg ggaaaggatg acagtttttc ccaattcagt atgatactaa ctgtgggtgt 63060
gtcatatatg gctttgatta tgttgaagca tgttccttct atacccaatt ttctgagggt 63120
tttttttttt atcatgaaga gatgttgagt ttcattgggt gctttttcaa catcagttga 63180
aatcatcatg tggtttttgt ccttcttttt gttggtatga tgtgtcatat taagtgattt 63240
gcatatggtg aaccatcctt gcatacctgg gacaaatctc acttggtcat gatgaacagt 63300

ctttttaatg tattattgaa ttcaatttgc tagtatcttg ttgaggattt ttgcattatt 63360

attcatcaga gatattggcc tgtagttttt tgttgtctta tctttgtctg gtttgggtat 63420

cagggtaata cttgcctcat agaatgactt tgggagtatt ccctcctgct ctattttttg 63480

gaatattatg aacaggattg gtattaattc ttctttaaat gtttggagga gtgtagcact 63540

gaagccatca ggccctgggc agttcattac tgggagactt tttattatga tgttgatctt 63600

actaattgtt attggcctct tcaggttttg gatttcttgg taggttttac gtgacaagga 63660

atttgtccat ttctctagaa ttttcccatt tattggcatg tagttgctca tagtagccac 63720

taatgaccct ttgaatttct gtggtatcag tcgtaatatt tcctttttca tctctaattt 63780

taattattgg ggtcttctcc ctgtttttct ttgatagtct ggctaatggt ttatcaattt 63840

tgtttgtttt ttcaaaaagc aagtttttat ttcattggtc ttttatatta ttttcctcat 63900

ttaaaattca tttatttctg ctctgatatt tattacttct tttctccgac taatttgggt 63960

tcaattcact tgcttctcta gttctttaag atacatcata aagttatttg aatttttttt 64020

tgatgtaagc actttcagct ataaatttcc atcttagtac tgctttcact gtattccata 64080

ggatttttta tgttgtattt ctattatcat taatttcaag aaatttttaa atttctttct 64140

taattttttt attgactctc tggtcattca ggaacatatt gttttatttc tatgtgttta 64200

tatagtttac aaaattcctc ttgttgttga cgtctagttg tattcagttg tgatcacaga 64260

agatgcttga tattatttca gatttttgaa tatattaaga cttgttttgt gatctaacat 64320

atggtctatt cttgagaatg atccatgtgc tgaggagaag aatgtgtatt ctgcagcctt 64380

tggatgaaat gttctgtaag tatctattag gtccactttt tctatggtgc agattaagtt 64440

cgatatttca ttgttgagtt ttctgtctgg gagatctgtc caatgctgaa agtggggtgt 64500

tgaagtcacc ggctgttact gtattgagtt ctatatccct ctttatctct aataatattt 64560

gctctgtata tatggttgct ccagttttgg gtccacagat atttataatt tttctatctt 64620

cttgcaggat tgaccccttt atcattatat aatgacctcc atttcccttc ttataatttt 64680

tgccttcaaa tctattttgt atgatataac tactcctgct cctttgtggt ttccattggc 64740

atggaatatc ttttcccatt tctttatttt cagtctgtat gtatctttat gggtgaagtg 64800

tgtttcctgt aggcaacaga tcattgtttc ttgttttttc atggttcatc cagtctgtgt 64860

attttgattg tttagtccat ttacataaaa tgttattatt aataaatcgg gacttactca 64920

tgccattttt ttagttgttt tctggtcttc tcttccttgt tttcttcctc cctgccctcc 64980

ttttactgaa ggtgattttc tgtggtgctg tgatttagtt tctttctctt tatttattta 65040

tttatgtatt ttttgagatg gagtcttgct ctgtcgccca ggctggagtg aagtggtgca 65100

atcttggctc actgcaagct ccacctcccg ggttcacgcc attctcctgc ctcagcctcc 65160

caagtagctg ggactacagg cacctgccac cacacctggc taattttttg gtatttttag 65220

tagagacagg tttcaccgtg ttagccagga tggtctcaat ctcttgaact tgtgatctgc 65280

ccgcctcggc ctcccaaagt gctgtgatta caggcatgat ccaccacacc cagccttttt 65340

ctctttattt tttgcatatc tgttgtatgt ttttcggttt gagaatacca tgaggcttgc 65400

caatatgatc taatacctct tattttaaac tgatgaaaac ttaacactga ttgtccaaac 65460

aaacatgcaa aaaaacctga taaaaacact acactttgaa ttcatccccc cacttttcaa 65520

gttttcattg cttctcttta tgtcttattg tactatgttt tgaaaagttt ttgtagttat 65580

tatttgattg gttcatcatt tagtctttct acttaagagg aatttatacc ccacaatgac 65640

agcataataa tattccctgc atttttcttt atgcttacta ttatagtgag ttttgtactt 65700

tcagatgatt tcttcttgct tattacatac ttttctttca gattgaagaa ctccctttag 65760

catttcttat aagacaggtc tggtgttgat gaaatctctc agttattgtt tgtctgggaa 65820

gatctttatt tctccatcat gcatgaaaaa tatgttttgc cagttataat agtataaagt 65880

aatttttttt cagcacttta aatatgtcat gccactctct cctaacctgt aaagtttcca 65940

ctgaaaagta tgctgccagg tgtattgggg ctccattatg tcatttgttt attttctctt 66000

gctgctttta ggatacttta tcaatgaact tttggagttt gattattaaa tgccttgagg 66060

tagtcttctt tggtttaaat ctgttggatg ttctataact ttcttgcact tgtatgttta 66120

tttctttctc taggtttggg gagttctctg atattatccc tttgaataca cttcttatcc 66180

gtctttctct gtctactttc tattcaagtc cagtagctct tagacttgcc cctctttggc 66240

tgttttctag atcttgtagc catgctttat tctttttttt ttttaatttt gtctcctctg 66300

tgtattttca aatagcttgt cttcaagctc acaaattatt ttcactcctt gatcagttct 66360

tctattaaga gactgtcatg cattctttga tatgtcaagt tgcatttttc aactctagaa 66420

tttctacttg attcttttta attattttaa tctctttatt aaagttatct gatagaatta 66480

taaattcctt ctcttcgtta tctttagttt cgttgagttt cctcaacaga gctattttga 66540

attatctatc tgaaagcctg catatctatc tgtttctcca gaattggttc ctggttcctt 66600

atttagttca tttggtgagg tcatgttttc ctggatggtc ttgctgcttg tagatgcttg 66660

ggagtgtatg ggcattgatg agttagattt ttaatttggt cttggactcc gggcttgttt 66720

gtacctgtcc ttcttgagaa gactgtacag gtattcgctg ggagttgaac cccaagccca 66780

ataatgctgt ggttttatag gctcctagag gtaccccatt ggtggccttg gataaattct 66840

ggaagaattc tctgtattac cagtcagaga acctcattct tttccattat tttctcccaa 66900

acaaatggag tctatctctg tgctgagcca cctggaatgg ggatgtggtt atgcaatcac 66960

ccctatggcc gccaccacac agactgcatt gaatcaaacc ggaagccagc acaacactgg 67020

gtctcgacct tggctcacta taactactac ctgggtaaaa cctattttca ctcaaggcca 67080

tagtgctcta cagtcagcag atgttaaggc caaacaggtt tgtgtccttc ctttcagggc 67140

aatgagttcc cccaggcccc gggcaggacc aggcatgctg tctgggagcc aaggattaga 67200

gtcaaatacc ttagaaattt acctgaagtt ctattctact gcagctaagc tggcactcag 67260

accataatcc agaatctttg ttgcacttcc ctcccctctc cacagggaga agggcctctc 67320

cctatagcca tcagcaccag cagcccatag caggggttct gccaggccac tggcaatgtt 67380

cacttaaagc ccagtggctc ttcagtcaac aagtggtgaa tgctgccagg ctgggaactc 67440

gttcttcaga gcactgggct cccatatgcc ccagggcaga tccagaaatt atatccaaga 67500

gcctaggctc agactcaaga gacctcaaga gcctgcttat tggcctaccc cactgtggct 67560

gagctggtac ctaaggtgca ttacctttct gtctgctttt ctcaaacaga aggaatcttt 67620

ttctgtagct accacagctc tcaatttgtt ggatcacacc tgaagccagc atttctcaga 67680

gcccaaggcc catggtgtac tacctgggta tattgctgct ggtggttgtt catggcccaa 67740

gagctcgtta atcagcaggc aatgaatcct gccaggactg ggttcttctc ttcaagacag 67800

tgggtttcct tttggcccag gatgtgtcta gaaataccat ccatgagcta gggcctggaa 67860

tgggaacctc acaactctgc ccagtgtcct gtcctactat ggctgagctg atatctaaga 67920

tgcaagacaa agtcctcttt actttttgtt ccttcctcct taagcagaag gaaggagtca 67980

cttttgttgc tgtgctgtct ggggttgggg gaatggtggc acaagtactc ccttagccac 68040

cctggctggt atctccctag gtcacatgcc accctagtcc actggctcta attccagttc 68100

aacactagta gttgcctagg aattgcaggg tttgcatctt agactgcctt ttaagtttac 68160

ctaggacccc agagcacttt ggcccaagat ggcgaggctt gctgaggagt tcaaatttag 68220

accactggga caggcaattc ccttctgtct agttcttgtc caaaagcttc ctctggccgg 68280

gtgcagtggc tcatgcctat aaacccagca ctttgggaga ccgaggtgga tggatcatct 68340

gaggtcagga gttcgagacc agcctgacca acatggtgaa accctgcctc tactaaaagc 68400

acgaaaaaaa ttagctgccc gtggtggcgg gcgcctgtaa tcctagctac tcaggaggct 68460

gaggcaggag aatcacttga acccgagagg cggaggttgc agtgagcaag actctgtctc 68520

aaaaaaaaaa aaaaaaaaaa aaaaaactta aaaaaaataa caaaagcttc ctccatatgt 68580

gggtgctggc tgaggccagc atgactttgc tgtctgctgt aacagggcag cactgaattc 68640

aatgtaaagc cccccagttg ctgagctttc cctcccctat ctgcaaagac tctcctcact 68700

gcatggctgc tgctggggca tggggaaggg gtgtcatcag tgattcatga ctgtctcttc 68760

taccctcttc agtgcctctt tcagcaatat gaagttaaaa ctaagtactg tgattactca 68820

cccgattttt gattcttgtg atggtgcttt tctgtgtgca gagagttgtt aaactttggt 68880

gttcctgcag aagctacaaa tggtataggc ttctattttg ccatcttgct ccatcgtttc 68940

ctcctgtagc ttctttttca ttttcttaat accattttct gaagaacagt tttgtttatt 69000
ttgatgatgt ccagtttgtc attatttttc ttttatggat tatacttttg atgtatctaa 69060
gaaatccttg tctaacccaa gatcacaaag aatttctatt ttcttttttg taaattttat 69120
agttttaggt tttacatggt ttacgatcca ttttgagtta attatttatg gtatgaggat 69180
gaatttttct atatctctat cctacttgtt ctggtaccat ttgttgaaaa gactgctttt 69240
tctccatgga attgcttttg cacctttgca agaaattaag tgacaatata gagccagttc 69300
tgttacagtg tgacatatgt gatctaaatc actgcagtat gcataaatcc atagtatata 69360
aaccacagaa cttatgggga aaaggaggca aggggcataa ctttaaaatg tttgatcagt 69420
gatacacaaa agaaaagaaa gtaaaaaggg tagcattgtt ctgtatgttc taaatgatta 69480
agaaatacat aaagactgca ataaatacag ttccttacct tgaaaaagac ccgaagtttg 69540
cttgtggaag tgggtgtcag aaaagttaca gtttgtaagt tataatgatg tagtggaagt 69600
tagattatct ggaattggaa ggaaaatttt aacgctagag gtggacagga gtggctcata 69660
acaaatacag tgaattagct ggtagatgtt tgaggtgtgt gcatatgtgc attttatgta 69720
tttctacttt cctaagctca gctgattcag ttttctgtgc tcatgtaacg cttcctgcag 69780
acaaaatcac tgataagcca gtgtgaaatt cacgttatac ttagattgtt tgctaagata 69840
tcaattgcat tagaacaaat ttgcattttc aaaagaagca ttatagcaga attaactgta 69900
tatgtcagtc tatcattgta cactcttctg ttccattgat atatatgtcc aacctttttc 69960
cggtacccca ctatcttgat taccgtagct ttatagtaag tgttgaagtc aggtgtagtg 70020
taagtccaac tgttttcttc cttttcaaaa ttgtttggac tattttattt tcttgacttt 70080
tttatacaaa ttgtagaatc agcttattga tttctacaaa agaaattttg ctgggatttt 70140
attgaagttt cattgaattg gtatatcagt ttggggagaa ttgatacctt aacaacatta 70200
aatcgtccaa ttcatgaaca tggtataact tcccatttat ttaggtctac tttgatttct 70260
ttcatcagtg tttccagttt tcagcatata attcttgcat atatatttta tagctgtaga 70320
tgtatattta tacctgtaga tttcatgttt gatatttttg gtggtattat aaatggtact 70380
ttttaaattt cgtcttccag ttgttcatta gtaataccaa gaaataagat tgatttttat 70440
tattgacatc tttagctcct tgaaggcttt gaacaaaaat ggaagctcac caaagccaaa 70500
gcaaaatttg cttttttaat attttaaaaa gtataagtta ctatttctgt tttgtcttag 70560
tctattttaa aacttagatc aatacggcag ctttgattta attgttgttc cctcacattt 70620
tttgcactat attctagtgt agacctgaaa gcatgtattt tgaggatgag atttcatgga 70680
aggatcttta aatgacctta aagagtattg ttatgaaata caggctgctt tctcttattg 70740
atctcacagg aactttctta agagtaactg taggccatcc ataaataatt gtaatgttta 70800
atacattatt ttgtttcttt tgacgttttc tattttctga agtttttcag acacattttt 70860

tggtaatact gttattatag attttggact cagcaaggag tcagtagatc aagaaaagaa 70920

ggcttactca ttttgtggta cagtagagta tatggctcct gaagtagtaa ataggagagg 70980

ccattcccag agtgctgatt ggtggtcata tggtgttctt atggtaagta gcattatttg 71040

ttttaattat gccaacctgt gattgatatt acttataatt ttattttaat tcaaattttg 71100

ggatttagca ggtcagaaag gggttctgat aatttataat taacaccaaa aaccttgtga 71160

ttggctagct gttacacaat aaaaattaag attttggtgt tctgatttat tttaaagttg 71220

ctagtaaaaa ggtaaatgac agcattgtta ttataaacta ttcatatttg atacttatga 71280

aaaggtctgt cagattctat atttcagaat gatgagttta tcagttacat ggtattttaa 71340

ttgaatgtta cagaacttag caccttacca tatagtgtct tattgtcaga ttgttccatg 71400

tattccctag aaaggttata aacctgttga gcagaggaat catgtcttat actacttttc 71460

atctcccata gtatcaagca tagtgagcat ttcagtagat gcttaataca gactgactaa 71520

tttggtatga gattaagcat agaatttgca gtagtcagta ggatgataga taaatagctg 71580

ttgaattaga tataatttgc tttggaggtc tgatccattt tgtccattta tcactgagta 71640

gcacatcaaa tgacacatca gcatgttaag tgtctcttat aagcctgaca tattaggagg 71700

cctgtggggt aggaactgat tctaagtaaa atatttggtc tttttcaatt ggagtagcta 71760

aataaactct caagaagtac cttaggagtt cagggaagat ttaatgtaag ctgatgtagg 71820

caaactgaaa gagttggaac ttaaattaag ccctgaagga tggataaaat ttaaatagtt 71880

gtgtgtcaag aaggagagtt attgtgatca tattagcaaa gacttggaga accagtttga 71940

ctagagaagt ggagaaagag ggcaaaggtg gaggaagtga agctataaac ctataagcat 72000

ttatacatga ttctctgggt aggaggaaac ttttagattc ttaaacaggc tggaaattag 72060

agaaaattgt attttaggag gattagcctt gtaagtggat tgtaatggaa gaaactagaa 72120

ataggaagac aaaagagatc tttaaagaca attgtaagat tatagagatt tcaaagtaag 72180

cagttgttaa ccttggtagc aaattagatg acacagatca aaaagaaatg aattgtcaaa 72240

gataaaaatg cctataaaat ttgtgagctt tagggggttc aaagaatggt aagttaatag 72300

aagagatagc tgaagaggac agctggtttt ggaagtggga aaaaataatt tttaacatgt 72360

agcataggtt actgtaggtt aatgactcac cactgggtgg tgataaacta ggaggggtag 72420

tggagagaga atcagaatca tttggagacc ttaatacgta tatattaact acccaacgtg 72480

tagtttcttt gagggaagat gtgagtccta tatacgatcc ttccttcatt ctctccctga 72540

agttctcacc tctactcctc cagtctttgg tggcgtacca ttacttaaga tttgggaagg 72600

aaagctggag tttgagagag attaagcctt gaacagcaga ttttgggaga cattaagata 72660

cagaagagac ttgaatgtat aagctcactt agattgtgag ttcagataaa tgagtagaag 72720

actaaggcca aagccattat tgaatctaag tttgagattt aaatactggt gatacaaaaa 72780

cttctcttta atgtaccgta atgattttta gaggtagtct ttttttatac cattatttaa 72840

tgttctagtt agtcacattg cagtcagttc tctttgagaa aatgttttcc ttcttctgtg 72900

tacaactaag gtgtatgttt cttttttaaa gctattggca tgtgtgtgtg tggcaggggg 72960

tggtggtggt ggtgtgttct gctgcttcta cttctttttt ggctcctgtt ttgggggcag 73020

tggagggaat gagaatggtt aaatctttct tgggcatgta tgaatttgaa atgcttttct 73080

gcttattttt ccttttcctt tattattaaa cattctcttc aaaagagtat atttgtttta 73140

acagaaaatg cattgtgtgt attttcaaat tgctttggct ttgagaacat agccgaatta 73200

gaactgagga agaattggtt ggtttcatct gctacaggtc accatcagtt gagctatata 73260

tatagagaga gagagtggga taccagaagt atatttcttt aagtgaccta gttttctggt 73320

cccattttga tctactatca tgtagatctt acaatttgat gattatatag aagtctcagt 73380

ttttttctag agaatcttat tatatattct aaggtgttct aattgtgtgg tgtgttttgt 73440

ttgtttgttt tagtttgaaa tgcttactgg tactctgcca tttcaaggta aagacagaaa 73500

tgagaccatg aatatgatat taaagtaagt gtaaacagta tgtttttctt ttgggaaaaa 73560

aatctcttgc tttcagtttt gaaaaaatta ttgcggtttt aaaataatac aagagtacct 73620

ttttctcact gtcttgttcc cttctaccat atacaggact gtacccttgt aaaagcactt 73680

ttaaaagttc tttttgagat ttttcaaatc aaaaatgtgg ctaaatttaa gtgaaacttt 73740

ctaaaatcct ctgtttcaga gcaaaacttg gaatgcctca atttcttagt gctgaagcac 73800

aaagtcttct aaggatgtta ttcaaaagga atccagcaaa tagattgggt attgcttatt 73860

ttcttagctt ttatagaagt aactgcatgt tttgggagga taattaaatc tttatgtttt 73920

cttgtttgat ttgagcacat tacttctata cgtagaattt ttcattttgt gtgtgcattt 73980

cagacaaagt aaactctgtt atgtccatga tttaaaactg aagtagtcta gttaatgaaa 74040

gcttctgttc aacctattat tatgtatata ttatgggtta cttcttttga aataataaac 74100

agattctccc ttgtacaggt aatcttttat atatttgaag atagatattt tgcccccccc 74160

caatattctt caaagtaaat attatgtttc tgtaactact tttttgaccc ctttctggta 74220

gcctttccct gtatccagat gcgatcctat tatttatgcc cttgtgtgaa aatttcagtg 74280

tcactttatt cctttgtacc tttcaatttg gcatataaat atgaggtcat aagataaatt 74340

aggaatgaaa aaatatgtat ttaagtttaa gtattatgtt taatactatt tgatttgatt 74400

tgggagacac tttattgtag ttcaatatga atgttagtta ttttttatca cagtaaaatg 74460

aaaggaccag ctaataaaat gccagataag aaatcatgta tttttctttt caaggaatgt 74520

atgaaatatg tgtattcctg tttacagata gcagtctgga agccagggag caagactaaa 74580

aaaaatcata aaaataaaat atcatttctt ttttttatag gatcagaagg agttgaagaa 74640

atcaaaagac atctgttttt tgcaaatatt gactgggatg taagttaagt agaaagcttg 74700
ttattgaata acttcatatg accaacacag gatattaaag atttattgct tttaatctaa 74760
ataacaaatc agtggaatat ttacatacat atttattttt ctttttctta aaaaaataat 74820
ttagatctgg gggtgcattt gtgggttttt tccgtggatg tattgcataa tggtgggttt 74880
ggatttctaa tgtacccatc aaccaaacag tgaccagtag gtaatttttc aaccctcaat 74940
tcccttttaa cttctcccct tttggagtcc tcggtgtcta ttatttccat ctgtatgtcc 75000
atgtgtatcc attgtgtagc tcccacttat aagtgagaac atgggatatt tgattttctg 75060
agttatttca cataggataa taatctccac ctccatccat gttgctgcaa agataatgat 75120
tttattcttt cttatggctg catagtattc catggtgtct atatacctat tttctttatc 75180
caattaacca ttgatggaca cttagattga ttccatgact ttgctattgt gaacagtgct 75240
gtgatagaca tacaagtgca ggtgtccttt ttatataatg atttattttc ctttaggtac 75300
atacctatta gtgggattgc tgggtcaaat ttttttgatt attagttctt tgagaaatct 75360
tcatatggtt tttcataaag gttgtactaa tttacattcc cattaacagt gtataagtgt 75420
acctgttttc tcccacatcc atgccaacat ctgttatttt ctaacttttt aataaaagcc 75480
ctcctgactg atataaaatc atatttcatt gtggttttaa tttgcatttt tctgatgatt 75540
agtgatattg agcatttttc catgtttgtt ggctgcttgt atgtcttctt ttgtaaaatg 75600
cgtgttcata tcctttgccc accttttaat aggtttgttt gtttgtttgt ttacctgttg 75660
agttgtttga gtaccttgca gatctcgata ttagtctttt gtcagaggca tagtttgcaa 75720
atattttatc ccattctgta ggtcatcttt ttactctatt gattatttat ttggctgtgc 75780
aggaaattaa ttaagtccaa tttgtctact tttgtttttg ttgcatttgc ttttgggatc 75840
ttagtcataa attctttgta tagatcaatg tccagaagag ttttgcctag gttttcttct 75900
aggacctttg tagtttcaca tcttatgttt aagtcttcaa acaatcttgt gttaattatt 75960
gtaaacagtg agagataggg gtccagtttt attcttcttc atgtggctag gcaattttcc 76020
caataccatg tgttgaatag agtgttctct cctcattgtt tattgttgta gattttgtag 76080
aaaatcagat ggctataggt atgcagcttt atttctgggt tctctgtttt gttccattga 76140
tctatgtgtc tatttttgta caagtactct gctgttttag ttactatagc cttgtagtat 76200
aatttgaaat taggaaatgt gatgcgtctg gctttgttaa ttttacttag ggttgctttg 76260
gctatttagg ctgttttttg gttctgtgta aacattagga ttgttatttc taattctctg 76320
aaggatggca ttcatagttt gataggaatt gtattgaatg tggatattgc tttggtcagt 76380
atgttttttt ttgttgttgt tttgtttgtt tgtttgtttt ttgacagcgt ctctctctgt 76440
cacccaggct ggaatgcagt ggcacaatca cagctcactg cagctttgac ctcctagact 76500

taagcaatcc tcccacttca gcctcctgag cagctgggga tacaggtgta tgccaccgtg 76560

tctggctaat ttaaattttt ttggtaaaga tgaggtctcg gtatgttgac taggatggtc 76620

tggaacttct gggttcaagc gattctcctg cctcagcctc ccaaagtgct gagattacag 76680

gcgtgaacca ctgtgcctgg actggtatgg tcattttaat gatattgatt ctttcaattc 76740

atgagcatga gatgtttctc catttgtcca tttgatctag tttctttcat ctgtgtttta 76800

tagttctcct tgtagaactc tttcacattc ttggttaaat gtatttgtag gtattttact 76860

ttttgcggct gttgttaatg agattgttaa ttagaacttt catttggttt tcagttagaa 76920

catcactgtt gtatagaaaa gctactgatt tttgtatgtt gattttattt ttttaaacat 76980

tactgaattt gtttatcaag tctaggagtc tgttggagga gtcttttctg ttttctcagt 77040

atcatattat atcatcaatc aatagaggta ctttgacttc ctcttttcca atttggatgt 77100

attttatttc tttttcttgc ttgattgctc tggctaggac tttccagtac tgtgttgact 77160

aggaatggta aaagtggaca tccttgacct gtttcagttc ttgaaaggaa tgctttcaac 77220

tttcctgcat ttggtatgat gttggagtat gttccttcta tgcctattat atggagggtt 77280

tttatcatca agtgatgttg aattgtctcg aatgcttttt ccgcatctat tgagatgatc 77340

acatgatttg tgtttataat tttgtttatg tggtgattta catttattga ttagtaaatg 77400

ttgatccatc ctttcatccc tagaataaaa tccacttgat catgattaac tattttttgc 77460

atgtgctgtt ggatttggtt tgctagtatt tcgttgagga tttttgcatc tatattcatc 77520

agggatattg gctggcagtt ttcttttttg gttgtgtcct tacctgattt tgctctcagg 77580

atgataccgg ttttgtagaa ttaattagga aggattccca actcctcaat ttttgggaac 77640

agttttagta agattggcac cagctcttct ttgcatgtct ggaaaaactt gtttctagac 77700

ctgtgtggtc ctggcttttt ttgatggaac atttttttat tactggctca gtttcactgt 77760

tcctttggtc tgtttgtgtt ttctgtttca tcctggttca atcttgggag actgtatgtt 77820

tccaggaatt tatccatcat ctagcttttc tagtttgtgt gtatatacat gttcatagta 77880

atcactgatc atattttata tatatttccg tggtattcat tgcaatgtgt gcccttttca 77940

atttctgatt gtacttgtta gaatcttctc tcttttcttc ttggttaaac catctagagg 78000

tctatcaact ttgtttatta tttcaaagaa ccagcttctc attttgttga tccttttttt 78060

ggtcgcaatc tcattttatt cagcgaaatg agacttagtt attttttgtc ttctgctagc 78120

tttgggtttg gtttttcttg ttattctact ttcgtgtagt ttgacaatag gtcattgatt 78180

tgagatcttt ctgtgttttt gatttaggca tttaacacta aaaactttcc tcttagcact 78240

gctttagctg tgtctcagac gttttgttat gttctgtctc tattttcatt catttcaatt 78300

tttttattcc tgccttgatt ttgttgttca cccaaaagtc attcatgagc aacttgtttc 78360

atttccatgt acttgtgtag ctttgagagt tccccttggt attgatttct aattttattc 78420

tactgtggtc caataggata cttgatgtga ttttgatttt cttgaaagta ttgaaatttg 78480
ctttatggtc aagcatatgt tcagttttgg agagtgttac atgggcggtg gagaaaaatg 78540
tatatgctgt agttgttgat tagaatattc tgtaaacatc catttggtct agagtccagt 78600
ttaagtccag agtttctttg ttgacatttg ccttgatgac ctgtctagtg ttgtcacttg 78660
gtattgaagt cccccactat tattgtattg ccatcaatct cttttcttag gtctattagt 78720
agttgttttt gaatctgagc gctctggtgt tggatgaata tatatttagg ataattaaat 78780
cttttggttt aattgagccc ttatcattat ataatgcctt tctttgcctt ttcttactgt 78840
tgctggttta aagtctgttt tatctgatat gagaatggct acttcggctt tgttgtgttt 78900
tttatttgca tgctatatct ttttccaccc ctttacttta agtctgtaga tgtgcttagc 78960
tattctgtgg gtctcttttt ggcagcagat ggttggtttt tgtctgttac ccaacttgct 79020
ggtctacgta ttttggatgg aacatttagg catttatgtt gaaggttata ttgatatatg 79080
aggttttaat cctgtcataa tttgctagct agttgccttg gattctcaat tgtatgattg 79140
ttttatagga tctttgagct ttctacttat gtgtgccttt atgatggtga gtgacatact 79200
tttgtttcca tctttagaac gtctttgagc attttttatg gggcctgttt aatggtgata 79260
agttcctctc gtgttttctt ttcggttaat gactttatgt cttcttcatt ttatgaagct 79320
tattttggct ggatataaaa ttcttggatg gcacttttta aaggaagcta aaattaggtc 79380
cccattctct tctggcttgt taaaatttct gctgagaagt ccactgtcag tctgatgaga 79440
tttcctttat tggtgatttg atgcttttct ctagcttctt ttatgattta ttctttcaca 79500
ttgaacttgg atagtctggc gactatatcc ctcggtcatg tttgtattct gtagtgtctt 79560
ctaggtgttc tctgaattgc ttatatctgg atatctacgt ctctaacaag attggggata 79620
ttttcctgaa ttagttcctc aattatgctt tccaaacttc ttttcttttt tctttctgcc 79680
tataagttgc aagtttggtc actttacatt atcccatatt tctcaaagac tttgttcatt 79740
ttttaaaaat tatttattct ttaattttat ctgatttaat tcaaaagacc agccctcatg 79800
ctcaaaatta tttcttatgc atggtctagt atgttattaa agttttcaat tctgttttga 79860
aattcctttc atcttttttt cacttccaga atttaaatat atatatatat atatatttat 79920
ttatctttta tctcctgaat tgttttctga tttctttgtg tttgtttgta tgtgtttgac 79980
tttattttgg atttcattga gcttccttac aatccatatt ctgaatgttt tatctgtcat 80040
ttcaaagttt tcatttgatt aagattcatt gctaggtagg tactgtgacg ttttggggat 80100
gtcattacat tctttttctt cgtagtcctg aagttcttat gttggttcct tcttatctgt 80160
agaagctgtc acatattact tttgaattta ttttcatgtg gacgtggttc accccctgcc 80220
gccgcccctc ttggggggtg tgactgtaga gtaggttggg aagtagccag aagtcttttg 80280

gctttgtttc tataggcctg tgcacttctt agtcaggttt ttatgttggg ttttacagtt 80340
caatctatag ggcagtaggt ggcatttacc agtacaagcc agctgtggca aaagcagatc 80400
ggtatgcact tgatatttgt ttattgtgtg gtgctctatg ttgtttcaga tgatgggctg 80460
gacagtggaa tgccaggttc cctgaccttt ttgtcccatg aggggtgagg ggaaagggag 80520
ctaggcagag cttgattgtc tggcttgcac acaaataccc caatgatgaa ctggggagcc 80580
agccctgaca ggcatggttg ggggagtacc tggtgagcag tgccaaggtc tcagcagaga 80640
gtaggaagac tagcttcaca tcctagacag gtagcaatat gttctattta tctatcacac 80700
ccctgactca gagctcatgg ctctcagttc agatccacaa ctgccgtgta tctccagacc 80760
gcagtatagc tgatagccac agaagacact tgtctatggg agttgtttct gggtggaacc 80820
tcctgaatca gtccaataca cacagctttg tgactcacct gtttgccaat gggtaatgct 80880
tctgctttct gtagatatca cagaggggct ccacttttta gcacacacag ttgggtatta 80940
gctgtggcag tatcgtctgg ttgggtccct ctgagctcag accccaggtt gaggcatggt 81000
catgtactag cagtggtaga caggactagg caaacttcca attccttatc ccccagaatg 81060
cccaggagac agcatgtatg agtcctgtaa gaagtggacc aggaccaggc cagtggactt 81120
gttaagtctc tagactcctg ctgctggcta tgatagggag tgatgggctg gttcttggag 81180
tcacaggcag aactctcaag cagaggcagg gagagtactc tggaagtggg agtcctaggg 81240
tacatcatgt gcctgtggag gctgggatct cagaagggcc tacaaaacac ttaggcagtt 81300
gcagaatgct caggcagttg gtgcatcaat gcatattgca gacctgtggg gctgggctct 81360
cgggagcgcc cacagaatgc tcagaatata gcaaaacact caggtgattg ggtccctatg 81420
gctgatcaca ggcctgtggg agctggtctc ttaaaagggc ccacaggatt ctcagattat 81480
cacagaacgc tctggtggtg ggattcccat ggctgatcac atgctttggg gtctgggctc 81540
tcagaatggc ctgcagaatg cttatgtggt cgaagtccca aggcccatca caggcctatg 81600
gggggctagg ctctcagaag gcctccaggt aacaggtgaa atagttggga gcagggccgc 81660
tgggctgctg gcctttcact ggggaaggca gaccccttca gctgaagcaa tgaaggttgg 81720
cagctatggg gtgcttggcc cacctgcact tccctcctga aggagcagca gtggatttca 81780
ctttggtggg cacacaaagg tgcccagttt ccccactgtc tccttggccc aggagtggca 81840
agtgtgatgg tggtgttggg cgcagctatt gagggagctg aagagcgggt cacgagcctc 81900
tgggggctgg gctctaagaa gaacaccagg ctgtggccag tatatacatg taagggcagg 81960
gcagctgtgc tgtagaccca acactgaaga agggaggcct catttagaag ggaacagctg 82020
agaccggcag tctttgcagc gtgcattctg ctcactcctc tgtactgcag ctgcagtatc 82080
tgtctttgag gtgtgcacaa gtaccagcct ccctgctccc tgcctagtga ggtggcagca 82140
gccggcgcca ggctgctcag ggatcaaaag tccatgggat tccacatggg cttgagcagt 82200

acctctgcac agattccagg cagctccgtg tattaaactg gaggcctagg ggattcaagg 82260

gggtttacct atggttagca ttgaaagtct gtggcagaat tgtggaatcc tgggggtctc 82320

tcactcaatc ttcccttccc tgcatcgaag atctcctggc tccacaccag tctcagttca 82380

cctgacggca tggctttgtt ctcttctttc tgtgattccc aattccatca cttctctgat 82440

gaattctagt gtggcctctt agaaaatcta cttgaagtgt caatatttat tgctattttg 82500

attcctcacc atgagagagg catccactag cccactagct gtttctagta agccataatg 82560

aactaaaagt catgtttaca tatttaaata cataaaaatt actcaaaagt aattccctat 82620

ttatttttcc ccactgtggg actgtaagct ttttaaaaaa tagatttcaa aagaagtggc 82680

ttttatatat tattagaaat tttctatgcc tttcaaaagc atagatttta gcaagataga 82740

ttgttagacg actaaatttt caattatctg atacaagcct aaacagtaca tttaacgtat 82800

attttttatg tgaaaacaat attttagatg aaatatgcac tttttaaaaa taattattga 82860

ttttgaaagc cttgagcatt ttagttatta ctgtgctgta tttgcatggt tatattatca 82920

atgagcattc aaaaacttct gatatccaaa attgagcatt ccttgtgaat tcagcttgtg 82980

gcatcattgc tatgtttctt ggcctcatta catcttgaac attatctctc aaaattttag 83040

aatttggtta acagttatct gaaactatga aattgttagt cttctattat ggactgtgaa 83100

tattatgttc tgctttgttt tacaaagtat aataattttt aaagtcttaa tacgttttgt 83160

gttatctggt tgtcccttct agaaattata taaaagagaa gttcaacctc ctttcaaacc 83220

tgcttctgga aaaccagatg atactttttg ttttgatcct gaatttactg caaaaacacc 83280

taaaggtaat gcatgtttat ctgtaaaatc agagatttta tttaagtaga tagtttaaaa 83340

ataaactttc ttgtgaagta aaaactaaaa gaatggtaaa tttgcagaga gttgatttgt 83400

ccatgggaaa tatggatcct atacaaaaca ttgcttgggc agctgtcaag tagatgtttt 83460

aaatgggaaa tatttatcag gaaggaagga acttggtcaa agggtggcaa gtttatttct 83520

ttgttttata agtcctttgg catgctaatg atttacagga tggggtagga ggtaagacgt 83580

gagatatatt actctgtctt ttacagtttc atacatacac tcaagaaata ttgaatacct 83640

actatgtact aagcaatgag gatgcagtgg tgaaaagaac agacaaaaaa tactgactaa 83700

aatggagcat gtatacagat gggaggagac agataatata taataaatca aatatataat 83760

atgttagtag catttaatgg tttcctttat agttaaagaa attctgacac tttatatcat 83820

agaattctgt gtgtagttat aattaaatgt aattatttgg acattagatt ctcccggttt 83880

gccagccagt gcaaatgctc atcagctctt caaaggattc agctttgttg caacttctat 83940

tgcagaagaa tataaaatca ctcctatcac aagtgcaaat gtattaccaa ttgttcaggt 84000

aaattccact taatcacatt attcagtttt gggataatta tttctctgtt attgctgatg 84060

tatttttaat cgtttttttt aaagtgattt ttaacttctt tgtttatcta ttggtataaa 84120
tcatgaatta aatatgtccc tgtgtgtttt ggaaaagagc agacagtggt aacttttaat 84180
tatatttatg tggcctcttt tgaataaaat agaagggtga tgtataagct agttgcttta 84240
ttatgttgcc tgtgggtatc aagaaaggat tggttcacgc catttataaa tgtcgataaa 84300
acttctgtag aatgaattat tctagtttaa ggcttaagat gaatgtatat ttaggacaaa 84360
agacagaaaa tgtgaaaata tttcattctc cttagtattt tactttttaa aaataaacag 84420
ataaatggaa atgctgcaca atttggtgaa gtatatgaat tgaaggagga tattggtgtt 84480
ggctcctact ctgtttgcaa gcgatgcata catgcaacta ccaacatgga atttgcagtg 84540
aaggtattgt ctctgagcct agctttcttg tttgttttat gcatgtaatt taaattatga 84600
gtcaactgac aaacattttc agtggagtaa atgaagatag gaaagataag atgatgagaa 84660
agaactctga gacttaatat tttcattttt gaatttcttt tcaagccttt gttatgtagg 84720
aacagtgtat ttcatgaatt tttgcatttg aataattttg ttatacatgt attatgttaa 84780
gattgagcag ttgtgataat tacaatcacg tcatcattgg tacaatttca gtgaaatagg 84840
atataatgtt aaaattttat ttttttctgt taagtttttt acttatgttt ttaaatgtta 84900
aaataatatg aggtaagtat tgttcttgag tttttaagca tttcttcaaa aatataacca 84960
aatggaaaat actcatttta gacaaaataa tttgtttgcc tcagatattt ttcttttttc 85020
gtatttctta gatcattgac aaaagtaagc gagacccttc agaagagatt gaaatattga 85080
tgcgctatgg acaacatccc aacattatta ctttgaagga tgtaggtatt tctaaatcta 85140
ggtatcagct aagctaatga agttatgaat tggaacttta tttttcttat tgaaactttt 85200
aatgtcttta gcatatttat tttcatgcct caaaaatctc aggactctta aattgccact 85260
ttaattcttt tttttcccat gattctatac ttctagtctt atagaatatc aaaaataact 85320
atgatgattg gtaaatgaga atttcctcat tacatgaata tattctggct ggttaactag 85380
ttaacattgt agctaacacc aattattatt ctgtagtgct atcaatacag aagatacagg 85440
agactgggga tctgccaaat tcttgaatat tttactttct tgattgtata gtaattggct 85500
atagcattat ggattgtttc tggagtgaaa tagtcttagt ctatagtttt gtaaaatgaa 85560
tgtataactc attaatatgt tactgaatgc tcacttttca cataaggaga taatgttctt 85620
ttctctttat acaagacaga aaagtcaact atcaccttga tcaatttgtg tcagaaaatg 85680
atatgcactt tgatttgtaa tctttaaact actgtggtgt gtgtgtgtgt gtgtttatca 85740
tacatgccct gagtgaaaag tgtataaata cttttcttat ttgttttcct tggtatacta 85800
ttggaattca accagtattt aagataccaa atcagataat gaactgaggg aagggtataa 85860
aaaataaata atgtatttac tttcagaaag aagcttacgt ttggggaaga gaagtagcta 85920
taatacagta aaaaaattga gatagaggtc atttgagagg cacagagttc tattttttaa 85980

tttctttctt tgatgaactg caacctataa cttcaaaagg caataaaatt atatgtttaa 86040

catggtttca tgggactgtg taggtgactg tttagccaat tcaaccttta gtacagcctg 86100

tactgcatta agagtaaagg aataatgtct tttttgtttc taatactaga gggaagtcta 86160

gtagaaaggt aaatataatt aaaatctaat ttaagaatta tagaattgta attataaata 86220

ataacatttt aaaactgcgt tcttttgtag gtctttgatg atggtagata tgtttacctt 86280

gttacggatt taatgaaagg aggagagtta cttgaccgta ttctcaaaca aaaatgtttc 86340

tcggaacggg aggctagtga tatactatat gtaataagta agacagttga ctatcttcat 86400

tgtcaaggag taagttgtag ttaacttttt tctttctatt ctgggaccta tatcttcatg 86460

aaaatatagg agatgatgcc acctaaaata agttgctttt attcacatat atttctatag 86520

agaatatcct aatagaactc acaatgtagt gaagtttatg gatttaggaa ataattattt 86580

tctctctgcc aattggccct gatttaatag catattcatt taaaaagcgg tttgaattat 86640

ataaatgtac ctaactttga ccttgaaatt atgaaactta gacttaaaag tcaaacataa 86700

aagaattaac caaagacata cagtgactat tattcaagtt acctaacata taaagttcag 86760

attacctaac atgtatcttg aaccttgcca gctccttccc taaatacagt aagaaatgtg 86820

tattctaaac atttcaagta ttactatatt aaattttgtg gtgaaggggc aggcttgtta 86880

ttttgcttcc tgtcctttca taaagaaggc aaatgaactg agttacaaaa aaagggaact 86940

tttaatatat ataactgtat aaaaaaacaa agtagggagt aatttaaaaa tttaagtttg 87000

aatcagttgt aatagtagat atgtaccaac ttaataaaga aatttaaaat ggcaatttag 87060

aattgacata atgaaaactc tgcatgagcc cctcttataa ttagtaaaaa tagtactcaa 87120

ttttgtgtgt ggtttaataa agtgtgtttt cagtattttt taatatataa tgctaaacag 87180

tagcttttta taatttaatt atttgattgt ctttaccaac cctctctaaa ataaagcatg 87240

aagagaattt ttgagaaaag catcataggc gtctcttctt tattattgta tgaaccttca 87300

ggttcttact tcaattaact aaattaataa gacacatatt gagcatttct acaggccagg 87360

cattgtaaaa agtgttgtgg attcaaacat gagtgagacc tggtccttgt ccttaaagag 87420

ctttagcata gtaaactctg attaaagata aggataactt acatgaaata gagagtacta 87480

gtagggaact tatcacatgg gggaatttca gcattttttt ttaccagtcc tacattttat 87540

ttgtttcttc tttattgttt ctatttttct gctgagactt cccttttttt gctgagagtt 87600

taatgcattg aaagcatgtt ttgttttact ttgttgagga tcattatgat agtcacttta 87660

aaatccttat ctgttttagc atggttcatc ttggaatcag aatttattga ttttcttaag 87720

actgtgttct attttcttgg ttctttgtat aacagataat ttgggattgt atcctaagca 87780

ttataataat cagttataga gaatttcata tttcattatt tttctccaat agttattatt 87840

gtttcatttg tttaagtggg taattttctt ggctgggctt gaactgaaaa ctagttttgg 87900
ggccagcagc tctggttttt gttcatatta tttgtcttta tacaaattat tttgatcctg 87960
ttccacacat ttttgataca aggatcagtt ctggctcttt cctttataat attcctccac 88020
tcagcattca gagttttcca gcatctcctt tctggtcctc taagccagaa ggtctgaggg 88080
tttttccaca tgtctttacc accactgtgt accataggga ttgcacttaa acccaggcta 88140
aaagccacag atatggaaac ctccttgtat ggtttcctgt gttccctcct tcaacaaagt 88200
gtgtactcct catcagagta tatctgcttc tgttcattct ccagtgcctt cagatagttg 88260
atttttgtat tatatccagg ttttatggtt gtttacttca aggagggggg tgttgagtag 88320
ggtcttagtc cattatggac taagtctaaa cttagtttag tttgtaagtc aggaactgtt 88380
cttatatgtt atgtataatg ctaagtgata taaattatgg catgtccaaa agtggatctg 88440
agaattgagc ttagatatat tgagattaga aatggttaac aaaactagtg gcttttgaga 88500
caatagaaac tggaacatct atctgacaga cataatttcc tgtgtatatt ttaggctatt 88560
aagcttgagc aaaatgtcat tttcaaaatt ttgtaatttg gaaagtttct ttttctcaga 88620
tatttagtta attatataga tataatgttt tatttctcag atatttagtt aattatatag 88680
atataatgct tttttccttt atattcaagg ttgttcatcg tgatcttaaa cctagtaata 88740
ttttatacat ggatgaatca gccagtgcag attcaatcag gatatgtgat tttgggtttg 88800
caaaacaact tcgaggagaa aatggacttc tcttaactcc atgctacact gcaaactttg 88860
ttgcacctga ggtattcctt aaaaacttca ccatttgcct ttcattcttt tcctttagtt 88920
gaatctatat gatgccatat gaatgttatg gtaggcagat aaaagctgtt accttgccct 88980
tttcagtttt aaagttcttt aaatcaggga attctcatag cccctaatat aaatatttac 89040
actggtaact agcatctaaa accaaccaga atgtaataat caggctattc tattctgttg 89100
ggttatatac taatttcaga atcatatcat tttagagttg ggaggtaggg aaaagatagc 89160
cacagcttca atatattatg tattctaaat gatgaaactg gctcgaggta cttcaatgac 89220
ttattcaaga cccagcattc acttaataac aaagctagga tagattcatc aggtatgtta 89280
aattgtactg aacattaggt aatttatata gattagcatt ataaataaca tataagggca 89340
gtgctatcct cgattgtctt taggatcttc taactcccgg agaaaatagc cacacagggg 89400
tttttgtaag ccgtggggat ttttcttgga aaataatatt tatatctaaa tattagggtg 89460
aagaatcagg aaaggtgatg ctcttaggga gaagagtcag gaaaggggag gctgtggtac 89520
agatttcaca tttgggaagc tgtaacattc attctaacca tggggatgag agctttgaca 89580
gtagcaattt ccatagcagt gaaagtgaag ttggggaaaa atgaatcaag agatggcatt 89640
ggccatataa gttctttata agctttcttt gtcaagtcag tagctgttct tatatgttat 89700
ggataatact gacatgtaat tacatattca atgaaaatca aaatacctaa aactcttcag 89760

atttgaaaaa attgatgtgt caatcaaagg agaagcagag taaaataatt tgagaagaat 89820
tcctatcttc tatatatttt atgattttat ttttatatca aatactagct ttttcaggga 89880
aatattgcac atcagatata ttaacaataa gccacttgta ttttaatgta aatcagtctt 89940
tattttaaag tagctactat aaaaataact ttaataatga aaatagaaat atagaaaata 90000
ctatactgat attgaagaac aggcagaaca tctgttttat aagcttttta tgataagaga 90060
tttgactaag aaattatgta tgtatccgga gaatgtgatt taatctatat tttaataaaa 90120
ttcataaaat aatgtacaag tttttatctg ttacaacata ttgtactgcc ctaaggtgta 90180
tgagataata gcctgcacat aaaaaaatta gcctttttag gacatttcag tggttttata 90240
ggaggacaga atatttgaga tagtgctctt ctgttatgta tagcatatat ggtcatcata 90300
aaggtcctca taaatattct aacagattta tacagtagac ataagagcag agtatagaat 90360
aaccatttta atagccaaat atttaatatt gacttaatct tgtggaccta aactacttag 90420
tattatggca acttctcact ggaaaaatgt taaggtgaat atgtaccata tgaacatatc 90480
aacaacagac attaaaatga aaaatatata atatgtccgt gtttagatta atggttaggt 90540
aaacagtttt agattaatgg ttaggtaatc aaacccagag agtagaatac agcccattga 90600
tgtttttttc ccaacagacg tacactgtag taagacactt ttaccattgt aatagtgaag 90660
gttttcaggt gggcatctgg agaccacttc tctctaacta gtgcttgaac tacctttgac 90720
tagtatttgt attttcaaaa tactttgaaa gttttatttt tgagtgatga agcccatgct 90780
aggaatcaat aatatatttg taaattgtta ttaatgaacc acagagagta tttttagtga 90840
ggtttgtttc tgaaatttca gacgctttca aaaggtatta gtaaaatata aaaatgcatt 90900
cttttttaaa ctagaattgg aaagattcaa atatcgatac agacatttca ataaaaaatg 90960
gcatttatgg gggtatacat tcattatttc acaatgcata aagtttttgc taagagctac 91020
actgattatt gcaattttag cttaacaata aggagaaaat attcttttag tttttagatt 91080
atgcatcagt ttgatcaacc tcttggggtt tattaggtag atgtgctttt ctactatacc 91140
tatgttgcaa aaagtgaata tatctctaaa atattttatt cttcgataaa ggcaaatcca 91200
ctacgttata ttattcaact gttattataa gcctactgag gttaaagaag ttcctgaatt 91260
aatggggctt acaaaaaagt tgagggatac tgtgtctgta gctttttata aactaatttt 91320
gaagttaact ctcctttgct ctttctctgg gctgtcatat agtttgcccc aaacgttaat 91380
tgatttgagc aacaggaatg aggagctctg tgtatgttac tttctgctga cttgggaagc 91440
caaatgagag agagacttat gccactgact actttaattg tgacttctat ctttgtattt 91500
aacttgatac aagtaaaaag gtgtgaaaca taacctctgt actctttcat tcatcctacc 91560
gctctgcctt gtttctttta tgattatatg ctcactaggg cattgagact agctctagaa 91620

aaaaagtgga ctgtttcttc tttacctaaa acaagttttt catgtttggc cctaattaag 91680

caatatgaca cctcctgcgc aagtctgtcc tcataactag gaaggttata ggagtatatg 91740

gtacctacag ctgacttgat taattctcac ctgtccaagt agtttctgtg cagtggccct 91800

caaccttagc tatgcattgg atcacttggg aaactttaaa aagcttccta agggaatcag 91860

ggaaaacatg gtgatgattg gaactgtaag atgtgatgag aaaaatgaag agataaggga 91920

ataaaatcac tagaagtcta tgttttacta gagtatgcca agttaacatt taccatgata 91980

tgagtggttt agctatacct tgttatttgt taagtacttt atagttccct acttgtatta 92040

tttaaataag ccaattatgc ttttatatcc tctttaccct cttaattttg cacatatgtg 92100

tgtttttatg aatacactaa gactaggaaa aaagcatatt tcaaatttta agtttgttat 92160

atactcttta ctcatgtttg gcagttgtca aagccaaaca ctaacatttt tagcctttta 92220

tagatgagaa attcatgctg gtggttatat aatccccagt gttaatgcat taataatgtt 92280

tctattttgt tcctatgcct agaggcttta gcaatgggct tgtttttcta aatggaaaga 92340

aaatggcctc atttcatctc tttatttttc taacgaactc tattaacttc gtagctaaat 92400

ctcacatgaa tacatagcaa atgtattatt tgatttttat taacactggc cccaaaaaac 92460

agcttcctaa attattgtat gaaaaataat gaagccattc agtatttttc aaatgtgtac 92520

ttgtggtcca gaaatatact gctgttttca agacattgac cttgtatggt aaaatataaa 92580

ttcattctgc ttttgtgaga ttatatatta tcttgccata tcttgtgtag taaagaggca 92640

gttatcatag atattaaaaa cacaggctta aataagactt cgtgttctac tacttactag 92700

ttttgtaatg ttgggtaagt tacttaacat tcacttcagt ttcctcatct tcaaaattgg 92760

aatattgtct acctcacaga atcagtgaca gaattaaaag ccttagtact gtatatagta 92820

tacattaaaa tctctgtaca tgttaatctt aagtttagat ttctctatat ctggtttcct 92880

aagaggagga gagaaaactc attgaggtgt caggggttat gtctttacta caagtgcata 92940

aaatgattga agtttttata ttatgaactt ttgtgagatt attagaagta aaacaacttc 93000

tttgggagaa aattgagttt atattgagta acaccatata atgagtattt ctttcttagg 93060

ttcttatgca acagggatat gatgctgctt gtgatatctg gagtttagga gtcctttttt 93120

acacaatgtt ggctgggtaa gaaacatata ttcaaaagca ttaacattgt tatttgaatt 93180

tcatctgtat atcttcatct agaaagagaa tagattaaaa taactttaag gaaaatgaca 93240

tttacatttc aaaatattaa tattgattgt tacacataaa attaaaaaac ataaagcaag 93300

gtgtagaatg aggcactact aatttatgac ataataattt taaaaggcag ttgtcaccca 93360

gaactaaaac ttttaaaaat aaagtgcatt tctattttaa tcttgaaaat atttatttaa 93420

tcataaaata attgcatgtt tcaccaacaa cttgcccact gaacttgttg atattgagtt 93480

gatttttttt aagcacttga attctaacta ttctggcact agagatttag taagattgtg 93540

caaatctaag tagtaacaat actaaactgc agaaacacac acaactacct tcccttgaaa 93600

ggataaagaa aagctcaaaa caaatgtaat aacaattgga ctatgttaat gttgatactt 93660

tacaaataat acaagtacta ttttactctc aataatgagt agcactttta tttagaagtg 93720

aaaacttatg gatgtagaaa ttattccaag agggttgtga tagagaaaat ggccaagttt 93780

agcaagtaat tataatgtac ttagacatat attttaaaga gtgcatttag tatagtttaa 93840

atatgcttga cataccaaga gctctataaa tatgttataa aaatggtgac tctaaattgt 93900

ttaatggctt ggtgttaatt acacatctca gtacatttca catactttta ggtattacaa 93960

atttatgcaa aatatttagt tatagacaac agtaaatact gaagttaagg gtagctaaag 94020

tttgcatgtc aagcacaatt aatctacatg attgatctac ataataaatg taatggctat 94080

ggagttttgt gtcgatatac atatttaacc aaccaagttt tcatagatat taataaaaag 94140

taatgataac cgtttccact gatgataatg atgaaatgtt aatgaataaa ttgaaacaag 94200

acaataaata cattatgaat atatgaaatt aacattttct ttttactagg tatatttgtt 94260

tgctaaccta tgccttgaat atgtgtgttt tagtgttgtc tagttttaag actgaagctt 94320

tacatgaaaa gccaccttta ttttatttta cattgattag ctaatcagag acaaaagaaa 94380

taattgatgt aaacaagatt ctccatttta ttaaacattt tattaaggtt aaatttatgg 94440

tttgaaatat aaaatttgac tttaacagtg aaaggaaata tgagatgata ttcttattca 94500

ctgaagtaaa tatttgcatg tagtaaatat atgtatctct ttcattgttt gggtatgtta 94560

ccctctagtg tctattaaaa tctattttat taatagctac actccatttg ctaatggccc 94620

caatgatact cctgaagaga tactgctgcg tataggcaat ggaaaattct ctttgagtgg 94680

tggaaactgg gacaatattt cagacggagc aaaggtataa attataatgt tttgttttgt 94740

tgcattcatg ttaaattgct caaatcccta agttatagct tagtgtgtgt ttgcagagta 94800

tctaatggtg tcatatgtaa gtgttttgaa ttaacacatc tatgagtgaa ttgaacagtg 94860

tacaaaggat aacttttggt tttaccatgt ttctgggcca ccttcctttc tgatcaattt 94920

acatatgtgc agggaatatt aactagttcc tgtattaacc aaaaccaaaa atgtaaattt 94980

tggaaacagg gtatatttaa agccaactaa agtttatttc tagatttacc atgtatttcc 95040

atatcatttt cttttagctc agaaattgac ttgattatgt agacaatttt tataatgcta 95100

acttattctt agagaatgaa tggactatgt atctgtggca cctggatatt cattcctgat 95160

ggcttagagt ggtccagtag aaatataatg caaggcaaat acactttaaa aatttctagt 95220

agccacatta aattttttaa gtgaaattaa atttaataat atgtgttgtt taatctaata 95280

tatccaaaat actattcaat ataaaaattt agatattctt tactttttct tactgagtct 95340

tcaaaatgta ctatatattt tatacttaaa gccgtgtgaa tatggactac ccacatttta 95400

atacttaata gccacatgtg gccagtggct gccatattag tgaagtttca gcataactgg 95460

ccttatttat ttgggggaaa aaatagtcaa cccaggcttg aaaactaatt tcttacattt 95520

ataattttta aattttaagt tttaaatgaa aactatcact tgtcaccatg tggcttttag 95580

gtcccagaag cttttaagct acaatttgaa gtagaacaca ttttatgaca aaagtaattg 95640

cttatagtta acatgtgcta attatctcaa gaacactagc cagtttgtag aatacattgc 95700

aaagtaactc cagcatcctg attattgtca ggataaataa actggggatt tgtccctaac 95760

ttttaaacat tagaaaaaca gacaagggat tgttcttgga tcttaacctt agtatgagta 95820

tgctgtgtgc tatagtagaa ggaacacttg aatggaagcc aggaaaactg agcgctaatc 95880

tttctttggt tccttataac tccaaaattc atttctatgc aatttggttt atttgtaatt 95940

tgcaaaattt ccccatgtaa tgtgattaga actactagta ttattcatta aatatgtatt 96000

ttctagtcat ccagtttatg cttcagaaaa tacattttac caaggatcaa ttttaatgtt 96060

taaagccatg catatgaatc tcctgtgttc cttttaaaaa tatctgctga gatcaggctg 96120

ttgacagttc ctggttggct cacagttacc catgtcagtt atctccgtta acatttccaa 96180

gaatctttgt agaacaagtt ctccacttgc aaggtctttt atttatttat gtatttattt 96240

atttatttat ttttattttt tttgagacgg agtcttgctc tgttgcccag gctggagtgc 96300

agtggcgcga tctcggctca ctgcaagctc tgcctcctgg gttcacgcca ttctcctgcc 96360

tcagcctccc aagtagctgg gactacaggt gcccgccacc acgcctggct aatgtttttt 96420

atttttttta gtacagatgg ggtttcatcg tgtttgcaag gatggtctcg atctcctgac 96480

ctcgtgatcc gcctgcctcg gcctcccaaa gtgctaggat tacaggcgtg agccaccaca 96540

ccaggcctcc acttgcaaaa tctttaaagt agaactcttt tttttttttt tttttttttc 96600

cctttaaggc agttagccca ttgccaaaag gtttactgac ttaaggctgg aaatgtcttt 96660

ctgagctata attccaagga cttctccaca gctaagtgag atgcctcata ccagtattag 96720

gtgattcttt gtgtggacag aacagagcat tttcatcttg tgtttaaagc aatttgttgg 96780

cttcagctcc tcaccacttt ctataccagt ctcctattac taacccagta atccctatgc 96840

aaaacaaaca aacaaacaaa caaacagaaa aagaaaacgc tgatggtgat tcctgtaggt 96900

ttaagggctt aaatctcaaa ctttgtgtta ggagtaacgg gagtgtgctg aaagggcaag 96960

caatacaaca agtcatacca aaaagccaca ttgttctctc ctaagcccca accccactcc 97020

actcctgtgg ccagtggtcc aaacagaaaa taactggaga aattgaggag gtcaaaggat 97080

tagggaacta agcgttatgt gaattcacta gcaagatgta cagaatgctt gtgtttacat 97140

tgttttttat ggaattagca gaataaaact gatctatttt aaaaatgcaa aaaaaaatct 97200

gctgaaatag atggggactt aacattgcca attgtgctca aattttcagt tttctgtgtt 97260

ccatgtattt tatgtttagg cctatctggt gtttaagtaa taatacatat taaagcttgg 97320

ttttattctt acgtctcttt tagcctggta atctggaaaa cttctcatag cataagatat 97380
tagggatagt gttgacatat ctcataaggt ctcttagata atcccttttt atggatcggg 97440
tgactttagt tctgtctttg aaacttttgt aggagctatg agaacctttt aagaaactat 97500
ctcatgtacc ttttaacagt tctactgctt acatttgttt taaactaggc atgaaagtaa 97560
acttgttcag tttcccataa cctcttaaac tagtacactt tagttgtgag aactgtgaca 97620
tttgctgccc atcagacata gattatttat tgcctcttca tttgtctctt ttaaaaagat 97680
taacttgtac ccagtgaaca atctctcacc atctcctcac ccaccactcc ccatcctctg 97740
gtaaccacta ttctactgtc tatttctatg agaacaatgt tttagattcc acatataagt 97800
gacatcatgt gatatttgtc tttctgtgcc tggctgtttc acttaacaca atgtcctcca 97860
ggttaatcca cgttgccaca aatgatagga tttcattctt ctttatggct gaatggtgtt 97920
tcattgtgta tatacacact atattttctt tatcaattca tatgttgata gacacttagg 97980
ttgattccat atcttggcta ttgtgaataa tgcttcagtg aacctgagca tacagatagc 98040
tctcctacat actgattcca tttcctttgg atatatatcc agcagtggga ttgctggata 98100
atatggtaga attttttgag gaaccactat actgttttcc ataatggctg tgctaatttg 98160
cattctcacc agcagtgtgt aagggttccc atttctccac atctttgtca acacttattt 98220
ttttattttt ttgatagtag ccactctgat tggagtgagg tgatgtctca ttgcattttt 98280
tttttttgat acggagtctc actctgtcgc caggctgcag tgcagtggcg tgacctcggc 98340
tcactgcaac ctctgcctcc caggttcaag cgattctccc acctcagcct cccgagtagc 98400
tggcattaca ggcaggtgcc accacaccca gctaattttt gtatattcag tagagatgat 98460
ctttcatcat gttggccagg atggtctcga tctcttgacc tcgtgatctg cccacctcag 98520
cctcccaaag tgctaggatt acacgcttga gccatggtgc ccagccctca ctgcagtttt 98580
gatttccatt ttccagatgg ttagttattt tgagcacctg ttcatgtagc acctggtgct 98640
ttattttgtc tatttggtga ggtcatggtt ccctgaatat tttttttttg aaaagtataa 98700
tttttttata attttaagtt ctgggaaaca tgtgcaggat gtgcagattt gttacatagg 98760
taaacgtgtg ccatggtggt ttgctgcacc tatcaaccca tcacctaggt aataagccct 98820
gcatgcatta gctatttatc ctgatgctca ctctcctcct gcccctcccc caacaggacc 98880
aggtcctgat tgtgtgtggt ttccttccct gtgtccatgt gttctcattg ttcagctccc 98940
acttatgagt gagaacatgg agtgtttggt tttctgtccc tgcgttagtt tgctgaggat 99000
aatagcttcc agctccatcc atgtccctgc aacagacatg atctttttta tggctgcata 99060
gtattccatg gtgtatattt acctcatttt ctttagtcta tcattgatgg gcatatgggt 99120
tgattccatg tctttgctat tgtgaatact gctgcagtga acatacacgt gcatgtatct 99180

tcataataga atgatttata ttcctttggg tatataccct gtaatgggat tgctgggtca 99240
aatggtattt atggttctag atcctttagg aatcaccaca ctgtcttcta caatggtcga 99300
actaatttat gttcccatca acagtgtaaa agcattcctg tttctccaca gcctcaccag 99360
catctgttgt ttcttctact tttcctctac tgttttccca ttactatttg gcatgtactt 99420
tattagagca tgtactacat tctgcctggt catatattca cttgggtata tttttgttct 99480
tcacacttag gaagccttaa ataacatact tattttgcat tttctatcac atctagcata 99540
atgagtactc aattggatac tttttcataa gctaatcatt tcttctggta tatcttgcat 99600
tgttaattta cacagcaagt gtttttgata cattttatac acatgttgtc ctattgtgag 99660
tgactataat ttgcagtaag cagtgagatt ttacataaat atttattttt cattttggct 99720
tgaagtcgct tttaatagaa gatgcttttt tcatgtgttt acaggttata gatttataga 99780
gctagaaggc aagtagtccg tcacctcatc attttgcaga taaataaacc aaggcatata 99840
gaatttaact tacacattaa tgagtggcag agttggaact gattttcagt ttagtactct 99900
tttcaatcca ctcacattag ggaaaacttc ctagaggaaa aaaggattta ttaccttgtt 99960
tcatgtagat taaatggagg aatgagaggt ctgtaaccct ttaacaattg aaggctcgaa 100020
ggaaagctat tattgttgac ccactttgtt tctgtagtaa tggacatgag gtatacagaa 100080
ccttattacc tgatatgttt tggctgtgtc caaactcaaa atctcatctt gaattgtaat 100140
ccccataatc cccatgtgtc aagggagaca acagatggag gtaactgaat catgggggca 100200
atttccccca tgttgttcgc gtgatagtga atgagttatc atgagatctg atagttttat 100260
aagtgtttgg tagttccccc gcgtccattc cccttcctga cttccttgct tgcgctcctc 100320
cttctaccgt gattgtaagt ttcctgagac tgtcccagcc atgcaaaact gtgagtcaat 100380
taaacctctt tatacatttc ccagtcttgg gtctgtcttc atagcagtgt gagaatggac 100440
taatacatta tcctttacat gatctgtact acagagcagt tttacagtta aaataatata 100500
ttgaagttca tctagaactc atagtagtac cacagttttc aaatgcctca tgccaaatta 100560
tactagtgaa agactacact atctaagcca agctaggaag gtacattagc aaatgcaata 100620
atcagaaatc aacccttgtt ttttctgaaa gataattatt tattattatt tgtgatcaga 100680
gcaggtgttg ctgaagtata tatggaagtt attcatttat actgaggata tatgaattgt 100740
tacacatttg accaattaaa catgcttaca gaatgagtaa taaaaaagtt ttacctcaga 100800
tacatgatta cattgaaaat agaagtgatg aattattaat cagtattttc caaactttaa 100860
tttggaatta aatttgagta ctgtgagaat aggaaaattg ctatgctcct atattcatgt 100920
atgttcaaag tatttagagt tttgctggct tttatccatt taaaatggac attaaaaata 100980
agaaacctga gtacattgag aggggccaag atggccaact ataagtaggt gcagtccaca 101040
gctttcatgg agagcagtga aaatggtgtg ttaattcagc accttcaact gaaatataca 101100

gtttcgtgta ttgggactga ctaggcagac aactcgaccc atggaggaga aagaatagca 101160

gggtggggtg atgacacacc tcagagtggc acggagacaa aggaaccccc agccaaggca 101220

agtggtgagc gattgtgtga ccccacctag agaaccgtgc ttctcccatg gatctttgca 101280

acccatggat cagtagattc cctcatgagc ccatgccacc agggcctttg gttcaatata 101340

cagagctgtg aggagtctca gtggggcagc tgctgaggca catacagaga cccaagaccc 101400

aagagttttg catactctgg ctccaggaat tccagcaagg cagaagaccc atctatactt 101460

ctatcctagg aagggacctg aatccaggga gccaggcagc atcattctgt gggccccact 101520

tccatggcac ctcacaagtt aagacccact ggcttggaat tccagccaac cagcagcaac 101580

aggctggaaa tggtctgaga tggaccaagt tccaggggga ggggtgacca ccaactctct 101640

ggtttggttg ggtcagtcat tctaacctgc cagctccgag gagtccaggc agtccagacg 101700

aggaggagtc cctgatgaag cagcacagca gctgtgccag attgtggcca gactgcttct 101760

ttgagtggga ccccaatcca tctctcctca ctggacagat cctttctgga ggacttcagc 101820

acttcagcca gggttatata gagagaaatt tgatgtttcc ctaggatgga gcccctggtg 101880

ggaggagtgg caccagttac tgcagttcag tcaacagcca ttctagcctg cctgctctgg 101940

gaagtccagg cagtccagag gaggaagagt cccccacaac ccagcacagc tgctgtgcca 102000

gatcatagcc atactgcttc tataactggg accctgatct atccctgttc actgggcggg 102060

gcctccctat gggaatttca gccactccag ccaaggttat tgggacagaa ctctgatttc 102120

tccctgaggt agggcccccg gggaaggggt agccaacatc tctgcaggtt gttcgactca 102180

gtctttccag cctgctgtct ctggggaggc caagcagtca ggacaaggaa gggttccccc 102240

aatgcagcac acctgctcta ccaaaaagca gccagactgt ttcttaaagt gggatcctga 102300

tcccatccct cctgactggg tgagacctcc caacagggat ctccagacac ctcctacagg 102360

agcatacagg tcagcaacag gtcagtatcc ccctgggaca gaactcccag agtaaggagc 102420

agattgtcat ctttgctatt tcacagcctt caccagtgat acttccaggt aagggaaaat 102480

caatgaaagc tagggtgtgg agcagtcccc cagcaaacca cagcagctct atggaaaagt 102540

ggcttgactt ttaaaagaac aacaaacaga aaacaacaca aaacaacagc accaacgaaa 102600

aagacaccac aaaaacctca ctcaaaggtc agcaacctca aagatcaaag gtaggtaagc 102660

tcacaaagat gagaaataat gcaaaaatgc tgaaaactca aaaaggcaga ggacctcttc 102720

cccttcaaat gactgagatg gctgaattga cagaagtggg cttcagaagg tgggtaataa 102780

caggctttgt tgagctacag gagcatgttg taacccaatg caaagaagct atgaatcatg 102840

ataacacaat agctggtaac aggagctgac aactagaata gccagtttag agaggagcat 102900

aagtgacctg atggagctga aaaacacaag aacttcacaa cgtaatcaca agtatcagta 102960

gcagaataga ttaagcagag gaaaaaatct cagagcttga agactatctt tctgaaataa 103020

gacaggcaaa caagaataga gaaaaaagaa tgaaaacaaa caaaacttca gagaaatatg 103080

ggactatgta aaaagaccaa gcctccaact gattggggta ccggaaagag atggggagaa 103140

tggaaccaag ttggaaaaca cacttcagga tattatccag gagaacttcc ccaacctagc 103200

aagaaagacc aacattcaaa ttcaagaaat ccagagaacc ccagtaagat actccaccag 103260

aagatcaacc acaaggtaca taatcatcag attctccaag gttgaaatga aagaaaaaat 103320

gttaagggta gccagagaga aaaggccagg tcacctacaa aaggaaactc attagactaa 103380

cagctgacct ctctgtggaa accctacaag ccaaaagagc ttgggggcca atattttaac 103440

attcttaaag aaaagaattt ccaacccaga atttcatacc tgggcaaaat aaacttcaca 103500

agcgaaggag aaataaggtc tctttcagat gagtaaatgc ttagggaatt cttcacgacc 103560

aggcctgcct tgcaagagct cccgaaggaa gcactaaata tgaaaaggaa aaactggtac 103620

cagccactac aaaaacacac tgaagtacac agaccagtta cattatgaaa caaccacata 103680

aacaagtatg caaaataacc aactagcatc ataatgacaa gaccaaattc acacataaca 103740

atattaacct taaatgtaaa taggctaaat tcctcaatta aaagacacag aatgccaagc 103800

tggataaaca gtcaagaccc aatggtatgc agtctttaag agacccatct cacctgcgaa 103860

gacacacaga gacgcagaat aaaaggatgg aggaaaattt accaagcaaa tagaaaacag 103920

aaaaaagcag gggttgcaat cctagtttct gacaaaatac actttaaaaa atcaaaagac 103980

aaagaagggc attacatgat ggtaaagggc tcaagtcaat aaggagaact aactatcata 104040

aatatatatg cacccaataa aggagcatcc agattcataa agcaagttat tgagagactt 104100

ataaagagac ttagactccc acacaataat agtgggcgac tttagaactc ctgtgacaat 104160

attatacaga ttatcaagac agacaattaa caaaaacctt caggacttga agatttttgt 104220

atcaagagga cctgataggt atctttggaa ctctccacca aaaatcaaca gaatatgcat 104280

tcttctcatt gctaggcggc atttacttta aaattgatca cgcaattgga aataaaacac 104340

tcctcagcaa atgcaaaaaa aaagaaaaac tgaaatcata acaaacagtc ctctgaccac 104400

agcacaatca aattagaact caagattaag atatttactc aaaaccacac aactacacgg 104460

aaattgaaga aactactcct gaatgactcc tggataaata atgaaattaa ggcagaaatc 104520

aagaagttct ttgaaactaa tgaaaacaaa gagacaatgt accagaatct ctgagactca 104580

gctaaaacag tgttgaggga aatttatagc acaaaatgtc cacatcaaaa agttagaaag 104640

atctctaatt aacaacctaa catcacaaca gaaagaacta gagaaccaag ggcaaacaaa 104700

cacaaagcta tgagaataca ggaaataacc aaaatcagag ctgaactgaa ggatatagag 104760

acacaaaaaa ccctcaaaaa gtcaacaaaa ccaggggctg atttttaaaa aacaaaatta 104820

ataaaataga caactagcta gacagaagag agaagaatca aatagacaat agacacaatc 104880

agaaattata agggggatat caccactgat cccacaaaaa taaaaacaac caccagagaa 104940
taaacacctc tatgcacata aactagaata tctagaataa tggataaatt cctggaacat 105000
gcaccctcct gtgacaacca ggaagaaatt gaatccctga atagaccaat tgtgagttat 105060
gaaattgagg cagtaatagc ctaccaacca aaaaagccca ggaccagatg gattcacagc 105120
tgaattctac cagaggtaca aagaagagct ggtaccattc cttctgaaaa tattccaaaa 105180
aatcgaaaag gagggactcc tccctaactc attttatgat gctagattca tcctgatatc 105240
aaaacctgac agagatgtaa caacaacaaa taactccagt tcagtatccc tgttgaacat 105300
caacacaaaa atcctcagta aaaactggca aactgaattc agcagcacat caaaaagctt 105360
atccaccaca atcaagttgg tttcatcccc aggatgtaag gttggttcaa catatgaaaa 105420
tcaataaatg tgattcatca catagacaga actaaagaca aaaaccacaa ttatctcaat 105480
aatgcagaaa attcaacatc acttcatgtt aaaaatgctc aataaactgg gtactaaagg 105540
agcatgcctc aaaatgttaa gaaaataata tgagaaacct acagccaata tcatactgaa 105600
tgggcaaaag ctggaagaat tccccttgaa aacaggcaca agacaaagat gccctctctc 105660
accactccta ttcagcatat aattggaatt tctggccagg gcaatcaggc aagagaacaa 105720
aataaagggt attcaaatag agaagtcaaa ttttctttct ttgcagatga cttgatctta 105780
tatctagaaa accccattgt ctcagcccaa aagcttctta agctgatgca acttcagcaa 105840
atctcaggat acaaaatcaa tttgcaaaga ttgctagcat ttctatgccc caacaacagg 105900
caagcggaga ggcaaatcat aaatgaactc ccattcacaa ttgctacaaa aagaataaaa 105960
cacctacgaa tacagctaac aagggaagtg aaggacctct tcaaggagaa ctacaaacca 106020
ctgctcatgg aaattaggac acaaacaaat ggaaaaacgt cccatgctca ttgttaggaa 106080
gaatcgatat catgaaagtg gccatactga ccaaagcaat ttatagatta aagggtattc 106140
ccactaaact accattgaca ttcttcacag aattagaaga aactatttta aaatttatat 106200
agaaccaaaa aatagcccaa atatctaaga caatcctaag caaaaagtac aaagctggag 106260
gcatcacact acttgacttc aaactatact acaaggctac agtaaccaaa acagcttgat 106320
acttgtacga aaacagacac atagaccaat ggaacagaat agaaactcag aaataagact 106380
acacatctac aaccatctga tctttgacaa acctgagaaa aacaagcaat ggggaaagga 106440
ttccttattt aataaagggt gctggggaac tggctagcca tatgcagaaa attgaaactg 106500
gaccccttcc atacacctta tacaaaaatt aactctagat ggattaaaga cttaaatgtt 106560
aaacccaaaa ctataaacac cctagaagaa aatctaggca ataccattca ggacatagaa 106620
acaggcaaag atttcaatga caaaaatgac aaaagcagtt gcaacaaaag caaacaattg 106680
acaaatggga tctaattaaa ctaaagagct tcttcacagc aaaagaaact atcatcagag 106740

tgaacagaga gactacagag tgggagaaaa atttttcaat ctatccatct gacaaaggtc 106800

tagtatttac aatctgcaag gaacttaaac aaatttacaa gaaaacattc aaaagttgtc 106860

aaaggagaag aacagacact tctcaaaaga agacatttat gtagccagaa atataggtaa 106920

aaaagctcaa catcactgat cactagagaa atgcaaatca aaaccacaat gagataccat 106980

ttcacaccag ttagaatggc aattattaaa aagtcaagaa acaacaaatg ctggcaaggc 107040

tgtgcataaa taggaacact tttacattgt tggttggaat gtaaatttgt tcaaccatta 107100

tggaagacag cgtggtgttt cctcagtgaa ctagaatgag aaataccatt tgacccagca 107160

atcccattac tgggtatata cccaagggaa tgtaaataat tctcttataa agatacatgc 107220

gtacatatgt tcactgcagc actatttata atagcaaagt catggaatca acccacatgc 107280

ccatcaatga tagactggat aaagaaaatg tggtacatat ataccatgga atactatgca 107340

gccatgaaaa ggatgttctt tgcagggaca tggatatagc tggaagccat tatttttagc 107400

aaactaactc aggaacagaa agccagacac tgagtattct cacgtataag ggagctgaac 107460

aatgagaacc catggacaca tgtcggagaa caacacacac tgggccctgt tatggggatc 107520

aggggaggca gagcctcagg ataaatagct aatgcatgct gggcttaata cctaagtgac 107580

gtgttaatct gtgtagcaaa ccaccatggc agacgtttac ctatgtaaca aacctgcgca 107640

tcatgcatgt ttttttcttg taaaattgtt taagttcctt gtagattctg gatattaacc 107700

ctttgtcaga aggatagatt ccaaaaattt tctcccattc tgtaggttgc ctgttcactc 107760

tgatgatagt ttattttgct atgcaaaagc tctttagttt aattagatcc catttgtcag 107820

ttttggcttt tgttgccatt gctttttgtg tttttgtcat gaagtagttg cccatgccta 107880

tgtcctgaat gatattgcct aggttttctt ctagggtttt tatggtttta ggttttacgt 107940

ttaagtcttt aatccatctt gagttaattt ttgtgtaagg tgtgaggaag ggatccagtt 108000

tcagttttct gcatatggct agccagattt tttaatgcca tttattaaat agggaatcct 108060

ttcctctttg cttgttcttg tcaggattgt caaaaaccag atggtcgtag atgtgtggca 108120

ttatgtctga ggcccctgtt ctgtttcgtt ggcctatata tctgttttgg taccagtacc 108180

atgctgtttt ggttactgta gccttgtagt atagtttgaa gtcaggtagc gtgatgcctc 108240

cagctttgtt ctttttgctt aggattgtct tggctataca ggctcttttt tgcttccata 108300

tgcaatttaa agtaattttt tctaattctg tgaagaaagt caatggtagc ttgatgggaa 108360

tagcattgaa tctataaatt actttgggca ctatggccat tttcacggta ttgattcttc 108420

ctatccatga gcatggaatg tttttccatt tgattgtgtc ctctcttatt tccttgagca 108480

gtggtttata gttctccttg aagaggtcct tcacatcgct tgtaagttct attcctatgt 108540

attttattcc ctttgtagca aatgtgaata ggagttcccc cattatttgt ctctcttttt 108600

gtctgttatt ggtgtatagg aatgcttgtg atttttgcac actgattttg tatcctgagg 108660

cgttgctgaa gttgcttatc agcttaagga gatttttggc tgagatgatg gggttttcta 108720
aatatacaat aatgtcatct gcaaacagaa acaatttgac ttcctctcct cctatttgaa 108780
taccctttat ttctttctct tgcctgattg ccctggccat aaattccaat actgtgttga 108840
ataggagtgg tgagagaggg cattattgtc ttgtgccggt tttcaaaggg aatgcttcca 108900
gcttttgccc attcagtatg atattggcta tgggttcctc ataaatagct cttactattt 108960
tgaaatatgt tccatcaata cctagtttat tgagagtttt tagcatgaac gggtgttgaa 109020
ttttgttgaa ggccttttct ggatctgtta agataatcat gtggtttttg tcattggttc 109080
tgtttatgtg atggattaca tttattgatt ggcatatgtt gaaccaacct tacatcccag 109140
ggatgaagct gtctttttcg tggtggataa gctttttgat gtgctgctgg atttggtttg 109200
ccagtattta ttgaggattt ttgcatctgt gttcatctgg gatatgttgt tgtttctctg 109260
gaaggtttgc tctcaggatg atactggcct cataaaaaga ttagggagga gtccctattt 109320
ttctattatt tggaacagtt tcagaaggaa tggcagcagc tcctctttgc atctctggta 109380
gaagttggct gtgaatttgt cttgtcctgg actttttttg gttggtaggc tataattact 109440
gcctcgagtt tagaatttgt tattggtata ttcaggcatt cgacttcttc atggtttcat 109500
cttgggaggg tgtatgtttc caggaatttc tctatttctt ctagattttc tagtttattt 109560
gtgtagagtt gtttatagta ttctctgatg gttgtttgta tttctgtgag atcaatgatt 109620
atatcccctt tgtcattttt tattgtgtct atttgattct tctctctttt cctctttaat 109680
agtgtggcta gcagtctatt ttgttattct tttcaaaaaa ccagctcctg gattcattga 109740
ttttttgaag ggttttcgtg tctctgtctc cttcagttct gctcttagtt atttcttgtc 109800
ttctgctagc ttttgaatct gtttgctctt gtttctctag ttcttttaat tgtgatgtta 109860
gattgtcgat tttagatatt tcctgctttc tcctgtgggc atttagtgct ataaatttcc 109920
ctgtaaacac tgctttagct gtatcccaga gattctggta cattgtgtat ttgttctcat 109980
tcatttcaaa gaacttcttt atttctgcct taatttcatt atttacccag tagccattcg 110040
ggagcacgtt tttccctttc catgtagttg tgtggttttg agtgagttcc ttaatcctga 110100
gttccagttt gattgcactg ttttcctaga gactgtttgt tatgatttcc attcttttgc 110160
atttgctaag gagtgtttta ctttcaatta tgtggtcaat tttagaataa gtgtgatgtg 110220
gtggtgagaa gaatgtatat tctgttaatt tggggtggag agttctgtag atatctatta 110280
ggtccgcttg gtccagagct gagttcaagt cctgaatatc cttgttaatt ttctgtcgtg 110340
ttgatctaat attgacagtg gggtgttaaa gtctcccact attattgtgt gggagtctaa 110400
gtctctttgt aggtttctaa gaacttgctt tatgaatctg ggtgctcctg tattgagtgc 110460
atatatatct aggatggtta gctctttttg ttgcattgat ccctttacca ttccctttac 110520

atctttgatg tcggtgacct tcggatgggg tttttgtctg gacatccttt ttgttgatgt 110580
tgatgctatt cctttctgtt tgttagtttt ccttctaaca ctcaggcccc tctgctgcag 110640
gtctgcctga gtttgctgga ggtccactcc agaccttgtt tgcctgggta tcaccagcag 110700
aggctgcaga acagcaaaga ttgctgcctg ttcctttctc tggaagcttc atcccagagg 110760
ggcacctgcc agatgccagc cagagctctc ctgtatgagg tgttaggggc acctgccaga 110820
tgccagccag agctctcctg tatgaggtgt ttgttgaccc ctgctgagag gtgtctccca 110880
gtctggaggc acgggggcca gggacctact tgaggaggca gtctgtccct tagtagagct 110940
cgagcgctgc gctgggagat ccactgctct cttcagagta ggcaggtagg aacgtttaag 111000
tctgctgaag ctgcatccac agctgcccct tcctccaggt gctctgtccc agggagatgg 111060
agttttatct ataagcccct gactgtggct gctgcctttc tttcagagat gctctgctta 111120
gagaggagga atctagagag gcagtctggc tgcagcagct ttgccaagct gcagtgggct 111180
ctgcccagtt tcactttccg ggtggctttg tttacgctgt caggggaaaa ctgcctactc 111240
aagcctcaat gatggtggac gcccctcccc ccaccaagct caagtgtcca agtttgactt 111300
cagactgctg tgctggcagc gagaatttca agccagtgga tcttagcttg ctgggctctg 111360
tgggggtggg atctgctgag ctagacctct tggcttcctg gcttcagccc cctttccagg 111420
ggagtgaata gttctgtctt gctggcgttc caggcatcac tgaggtatga aaaagaaaaa 111480
aaaaaaaact gcagctagct cagtgtctgc ccaaaaacgg ctgcccagtt ttgtgcttga 111540
aacctagggc cctggtggtt taggcactca agggaatctc ctggtctgtg ggttgcaaag 111600
acggtgggaa aattatagta tctggtctgg aatgcatcgt ttcttatggc tcagtccctc 111660
acggcttctc ttggataggg gagggagttc cctgacccct tgcgcttcct gagtgaggcg 111720
atgccccacc ctccttcagc tcgccctctg tgggctgtac ccactgtcta accagtccca 111780
atgagatcaa ctacgggtac ctcagttgga aatgcagaaa tcacccacct tttgtgttga 111840
tctagctggg agctgcagac tggagctgtt cccattcggc catcttatca gccctcaaga 111900
acttatatgc tagactgttg agttttttta ttttttttta agcaactata gtggggtaga 111960
attgataaca aatgaaatgc atgtatagta tatagtgtta tgacatatat acacacccat 112020
gaaaccatca tgaaaatcaa gataatgaac atatccatta ccactaaaca tttcctcatg 112080
ctgttttgta attccctccc tcctgcccta tatcttcaat ttataatctt ccacccacct 112140
cattctctgg cacacacagt tgtgctgtct gccactacgg gtttgtatat ttttttaaat 112200
gtttataaat taaatcataa gtctgcagtc ttttgatctc ttttatttaa taataatttt 112260
gagatttatc tatgttgttg aagtacagca atagttaatt ctattttatt gctggataat 112320
atttcttttt atggatacat cattgttcat ctgttcacct gttgatggac atttgtgttg 112380
ttttgtgttg ttaaccaata aagctgctat gaatatctat gtgccagtgg ttttatgtgt 112440

gtatatcttt tttttttgta taaataccta tgagcagaat gattgagtca tgtgtttaat 112500
gttttaagaa accactcaat tgttttacaa attatctgag ctattttaca ttttcaccat 112560
tagcatatga aaattttagt tcttccacat ctttggcaac actggatatg atccttttta 112620
aaaaaattag ctattttaaa aggtgtgtga tactatttga aagtagtttt aattcgcatt 112680
tccctagtga ctaatgatgt tcagcatttt tcatgtgttt atttgccatc catttatctt 112740
ttacctttcc ctttttcact gggtttttga gtgttaaatg caagttctct gtcagatcta 112800
tgctttgcaa ttattttctt tttatggatt gtctcttaat tctcttaagt gtctttcaaa 112860
gaagtcattt ctaatttgat aaagattatt ctttttcttt cgtatatcat gtttttgata 112920
ttagagctaa taaacttttg ccttatgcaa gatttcaaat acagaggttt cccaacttat 112980
gatggtttga ctgactgatg attttttgac ttaggataga gtgaaatcaa tatacattca 113040
atagaaactc tactttgaac ctttgaattg ttatcttttc ctgggctagc aatgtgcagt 113100
aaaatactct ttggcaatgc taggtagttg cagccagcca cagctcccag tcagctatgt 113160
gatcatgagg gttaacaatc agtagtctgt atttttcaag gtgtaacccc ttaataaatc 113220
agagcatctg tattttcccc tgtgttttct tttagaagtt gtatcatttt aggttttatc 113280
tttaggtcaa tgttcttttt tgattcaaca tatatatata tggtacaaca tatggtcaaa 113340
gttcattttt acatgtgaat atgcagttgt tccagtacca ctttttgaaa ataatatcct 113400
ttcttcacca aattgcattt gtatttatat caattgtcca aatatccatg gttctgtttc 113460
tacacttcct attctgtttc actgatctac ttgtctgtct ttacactatt accatactat 113520
cttcgttact gttgctttat aattaaattt tgaaatcagg taccatgagt gtttcgactt 113580
ttttcttttt cagagttttt tgagtttttt tgttgttgtt ttgctatttt gggtgctttg 113640
cattgccata tgaattttat aatcagcttc tcaatttcta tcagaaaaag aagcctgcta 113700
ggattttgaa tgagatggtg atggagctat agatcaattt gggaagaatt gacattttaa 113760
cagcattgac ttttcttatc tataaacaca gtatctcttt ccagttaggt ctttactttc 113820
tctcaacaat gttttatacg tttaaatata caggtcttaa acatctttta tcatatcttt 113880
tccccaaata ttttgttttt aatattaatg gcattaaaat tttattttct tttgttgcta 113940
gtatgcagaa atgtgattat attgatcttg tatgttagaa gcttgctaac ctcacttatg 114000
aattctagta gcttttttct agattctatc agtttttcta catagatggt aatagttttt 114060
ataaggacgt tttagtgctt gatttctaat atggatgtct tttttcttaa ttttcaatgg 114120
ctagaacttc cagtatagtg ttgtagaagt ggaaagaatg cacattcttg ttttgttcct 114180
gatcttgagt gtaagcattt atttttcacc attaaggatg atgttagctg tgggttttat 114240
atggatgctt ctaattaggt aaaagttcca ttttgtttct ggtttgatga aaatttgtat 114300

tggagatgaa tgttggattt tgtctaagcc ttttcctatg tctgctgtga taatcacatg 114360
attttcttta ttgttgttac attgatatac atttgaatta cgtttatttt caaatgctaa 114420
gccaaccttc tattcccagg ttaagtacca ctcgatcata atagactata tatagctaaa 114480
tttgatttgc taaaattttc cttacaattt ttgcatctgt gttcatgagg gatgtggatg 114540
tactatttta ttgtaagtat ctttgtctga ttttgatatt ggtgtaatag tggtcttaaa 114600
tgagttgaga aacattccct cctcttcaaa tttctgaaaa aagttattta gaattagaat 114660
ttcttattct taaatgtttg ttgggattca tcagtgaagc catctggacc tacagttttc 114720
tttgttggaa atatttaact ataaattcaa tttcttaaaa aggtatatgg ctatttcggt 114780
tagttgtttc ttcttgagtg agcactggca tttttttatt tcaaagaatt tgtccattgt 114840
caaacacatt ggcacaaggt ttttcttata atattttctc actaactggt taatatctgc 114900
tattaaccac tgtaatctat actgatgtct attttctcat ttcagctatc atggatttgt 114960
gtatttctct ttgcagttat aatggttttt gcttcatgta ttttgacgct ttgtttttac 115020
atgaataaac atttataatt ttcttctctt gattacttat cccctctgtt atgaaatgac 115080
cttctttatg acgtgtaata tttgccatta tttttgaaag atatgttcac acaatataaa 115140
attattggtt gacttttttt cagtatgtta aatatgttgc tccatagtct tcccatttga 115200
attttttttt tctgacagga aatctgttgt tatccttgtt tcacatgtaa catgtccttt 115260
tttctctagc tacttttaag gttttctctg tgtcttgggt tttgagtgat ttgggtttga 115320
tgtgccttga tgaaattttc ttgatttttc ttgtacttgg gttttattga ccttattgta 115380
tctgtgaatt tatggttttc atcaaatttg gacatttggg ggccattatt tatttaaata 115440
ttcttttctg ctcccaattt tcttctggga cttcaactgc atatatatat tagaccatgt 115500
gaagttgtct cacagctcac tcaaactgct cttttgttgt tctatactct cgtgtttctt 115560
tttggaaagt atctattgtg tatatttgat ttagtttcat tatttattat ctcacagttt 115620
ttatctctag aagtttgatt tgaggttgtt tgtttgtttg tttgtttgtt tgttttgcga 115680
cggagtctcg ctctgtctcc caggctggag tgcagtggcg caatcttggc tcactgcaat 115740
ctccgcctcc caggttcaag taattctcct gcctcaacct cctgagtagc taggattcag 115800
gggtgcacca ccacgcccag ctaatttttg tatatttagt agagacgggg tttcacaatg 115860
ttggccaggc tggtctcgag ctcctgacct catgatccac ccgccttggc ctcccaaagt 115920
gctgcgatta caggcgtgag ccaccatgcc cggccttgag tcttttttta tatcttctct 115980
atctctactt aatttctgaa catcaggaat acaattataa taactattta acatccttgt 116040
ctgctaattc tgacatctgt gtcagttctg ggtcagtttt gattgattct tttctttctc 116100
ctcatttttg tgctttttgc ctgcccacta atttttgaga agataccaaa tacggtgaat 116160
tttaccttgg tgggtgctag atacttttaa attcctataa gtattctatt ttcttaatgt 116220

caaatttgtt tttcttaaga tttattttta attaacacat aaaaattgta catatttatg 116280
aggtatagtg tgatgtttca atacatgtgt atattgtatg atgttcaaat caggataatt 116340
aacttatcca tcaccttaaa catttagcat ttctttgtga tgaaaacatt caaaatcctc 116400
ttttttagct gttttgaaat gtataataca gtatcgttat ttatatttac tctactctgc 116460
aacagagcac cagaacttac ttctcctatc tgtctataat tttgtaccca tttgctaatc 116520
tctccccatc cctccctcaa ccctaacctc ctcattccct agtaaccact gttctactct 116580
gtacctctat gagatcagct tttgtaaatt ccacaaatga gtggatcagg tggtatttgt 116640
atttctgtgt ctggcttatt ttctttaaca taatgtcttc caggtttatc tatggtgcca 116700
caaatgacag catttctttt ggccaatagt attccattgt ttatatttac cgcattttat 116760
aatccactat ctgttggtga acacataggt tgcttttata tcttgtctat tgtgaatcgt 116820
gctgcagtaa atataggagt gcagatatct tttcaatata ctgttctcat tttgtttggg 116880
taaatagcca gtagtgggac tgctggatca tatgttagta ctgtttttaa tttttgaggg 116940
aaatgccatg cagttttcca tagtggctgt actagtttac attcccacca atagtatagc 117000
gcatttccca tttctttaca tccatggcag catttgtcat ttttgtacta gccattctaa 117060
ttggggtgag ataatatccc attatggttt tgatttgtat ttctgtgata attagtgata 117120
ttgagcattt ttccatatac ctattggata tttgtattta ttcttttgaa aaaaaaaagt 117180
ctatttagat tttttgacca ctttttaatc aaattatgta tttttttgct attatgttgt 117240
ttgaatgttt tatatattct ggatattaat cccttttcag atatgtactt tgcaaatatt 117300
ttctactatt ctgtagattg tttctttcct ctgttgatta tttcctttct tgggcagtaa 117360
ctttttagtt tgaggtaatc ccatttgtct atttttgctt ttcttgcctg tatttttgaa 117420
ttcttgccca aaaattctta tccaaaccaa cgtcatgaag catttccccc atgttttctt 117480
ctagtagttt tatagtttca ggtcttacat ttaagtattc aatccatttt gtgttgattt 117540
ttatatatgg taagagatgt tggtctagtt ttattcttct gcatatagat gtccagtttt 117600
attggcacta tctattgaag agaccatcct ttacccaatg tgtgttcctg ctgcctttat 117660
tgaaaatcag ttggttgtaa gagcatcaat acatttctgt gttgtctgtt ctgttccatt 117720
ggtctatatg tttattttta tgctgactcc atcctgtttt ggttactata gctttataga 117780
atactttgaa gtcagtgtta tgcctccagc tttgttcttt ttgctcaaaa tgactttggc 117840
tatttggagt ctttcgtgct tccatacgaa ttttataatt ttttctatgt ctgtgaagaa 117900
tgccattgat accttgatag agattgcatt gaatctgtag attgggtagt atagacatat 117960
caacaatatt aattattgct attcatgaaa atggaatatt tttccattta tttgtatcct 118020
cttcagtttc ttccatcagt gttttatagt tttcattgta gagatcattc acatccttgg 118080

ttaaatttat tcctctctat ttttttgcag ctcctaaatg gaattgcttt ctcaatttct 118140

ttttcagact gttcactctt gtcatataga aatgctagtg atttgtgtat gttgcttttg 118200

tatcctgcaa ctttactgca ctttgtttat tctaacaatt tttggtggca tctttagtgt 118260

tttctataaa taagataatg tcatctgcaa atagtaacag tattatttcc ttctttccaa 118320

tttgcatgcc ccttatttct tttgcccaat tgttctggct aggacttcca gaactatgct 118380

gaataaaagt ggtaaaagtg gacatatttg tcttattcca tatcatacag gaaaagcttt 118440

caaccttccc ctattcagta tgatattagt tgtgggtttg tcatacgtga cctttattaa 118500

gttgaggtat gtttcttcta tacctaattt gtttaatgtt ttttatcatt aatggatgtt 118560

gaattttatc aaatgctttt tctgcatttt aaatttatca tatggcgtct gtccttgatt 118620

ctgttgatgt gatgtattat gtgttaattt gcatgtgttg aaccatcctt ttattcctga 118680

gataaattat acttgattat gttaaatgat gtttttaatg tgctgttgaa ctcagtttgc 118740

tggggatttt tgcatctatg ttctttaagg ctattggcct atagtttttg ttgttgtgtt 118800

cttgtctggt tagaacagtt gggttattct ggcttcatac aatgagtttg gcaggattct 118860

atcctcttta atcctttgga attgtttgaa aaggattggt atttgctctt aagaattcag 118920

cagtgaaggc atcagatgat gggattttca ttgatgggag actttttgta actgatttaa 118980

tcctgttact ttctattggt ctgttcagat tttctatttt gtccattcaa ttttggtaga 119040

ttttatatat ccaggaacgt atcaatttct tctaggtgtt ccaatttgtt ggcatatagt 119100

tgtttgtaat agtctcatag ttctttctat atctgtggta tcagttgtaa tgtcttcttt 119160

tccattggat tttatttatt tcaatcatct ctcttttatc ttagtcccat taaaggtttg 119220

tcaattgtgt ttatcttttc ataaaaccaa ctctttttca tttatcattt tttttattct 119280

ctattttgtt tatttcttct ctgatgttta ttatttattt tcttctaatt ttaggtttag 119340

tttgctcttc attttcctat aagtatgctt gaactttatt ctgggcagca gttaagttac 119400

tggaaacagt ttcatacttt tgggtcctcc ttttaaattt ttttaggcag gaccagacta 119460

gtgtttagtc tagggctact cactatttag gcaaggtgct tctaaatact ctgtaatata 119520

cttcatcaat tatgatgttt tccattttgg cgggtggaaa caggcaatac accccacctg 119580

tgtgagtcct gattttcatc cagtgctttc agatagttct ttccaaggtc ttgggttgtt 119640

tccccacgtc tttatcagta gtaatctgct agataattta gtgagacctt ttatagagcc 119700

ttgtaattct ctctctctgc tcctcacacc cctctcatac cctgtcctgc taactatgtc 119760

tgccttggtc tcccctgagt ctcagttcta tctcctcagt tcaaacccac aaggcttcac 119820

ctgagtttcc cttacctgca cagcagctag aaaactctct caggttggta agctgaagca 119880

atcacaatgc taacctgatt gtttcccact attaggaatc actttccttt attgcctaac 119940

atctgatatc ctgaaaactg ttgacatatt atatatttta tgtttttttt taattggttg 120000

tttcgggcag ataggtacat ccagtccttg gtatttcatc ttgaccagaa gaagaaatcc 120060
ttggactatt gaaatttttt catttttcca ataaccattt aggtttgttt cattgaaatc 120120
attgttatct tctttttagt attaattgtc catttatttt tgcacatgtt tccccagtat 120180
ggcttttgat tggttgccct agtcccttac atggtttcaa ccaattacgt tattcttcag 120240
aatctgatag tactacttag agtctttagt ttcactgctt ctgtgattga tgaaacgttt 120300
gcatacgatg attttgtttc tatattagaa cagttttttt ttggttaatt ttactttaag 120360
ttctggaata cttgtgcaga acgtgcaggt ttgttatata ggtatacatg tgccatggtg 120420
gtttgctgca cctagcaacc cgtcattcag tttttaagcc ccgcaagcat taggtatctg 120480
tcctaatgct ctccctcccc ttctccctaa cccccgacag gccgcagttt ttaatgttcc 120540
cctccctgta tccatgtgtt ctcattgttt aactcccact tatgagtgag aatacctggt 120600
gttttgtttt ctgttcctgc gttagtttgc tgacaatgat ggtttccatc ttcatccatg 120660
tgcctgcaaa ggacatgatg gctgcataat attccatgtt gtatatgtgc catttattct 120720
tcatccagtc tgtcatcgat gggcatttgg gttggttcca agtctgtgct attgtaaata 120780
gcgctgcaat aaacataaat gtgcatgtgt ctttatacaa gaatgattta taatatttgg 120840
gtatataccc agtaatggga ttgctgggtc aaatggtatt tctggttcta gatccttgag 120900
gaattgccac actgtcttcc acaatggttg aactaattta cactcccacc aacagtgtaa 120960
aagcattcct atttctccac atcctcgcca gcatctgttg tttcctgact ttttaatgat 121020
caccactcta actggcatga gatgttatct cattgtgttt tcaatttgca tttctctaat 121080
gagcagtgat gatgagcttt ttttcatatg tttttggccg tataaatgtt ttcttttgag 121140
aagtgtctgt tcatatcctt tgcccacttt ttgatggagt tgtttctttc ttctaaattt 121200
gtttaagttt cttgtagatt gtggatatta gacctttgtc agatggatag attgcaaaaa 121260
ttttctcccc ttctgtaggt tgcctgttca ctccaatgat agtttctttt gctgtgcaga 121320
agctctttag tttaattaga tcccatttgt ccaatttggc ttctgttgca agtgcttttg 121380
gtgttttagt cataaagtct ttgcccattc ctgtgtcctt aatggtataa tccaggtttt 121440
cttgtagggc ttttatggtt ttaggtttta tatttaagtc tttaatccac cttgagttaa 121500
tttttatata aggtgtgagg aaagggtcca gtttcagctt tctacatata gctagccagt 121560
tttcccagca ccatttatta aataggggat cctttcccca ttgcttgttt gtgtcaggtt 121620
tgtcgaagat caaatggttg tagatgtttg gtgttattgc tgaggcctct gttctgttcc 121680
atcggtctat atattggttt tggtaccagc accatgctgt ttcggttact gtagccttgt 121740
agtatagttt gaagtcaggt agcatgatgc ctccagattt attctttatg cttatgattg 121800
ttttggctat atgggctctt ttttggttcc atatgaaact taaattagca ttttctaatt 121860

ctgcaaagaa agtcagtggt agcttgatgg ggataacatt gaatctataa attactttgg 121920

gcactatggc cattttcacg atattgactc ttcctatcca tgagcatgga atgtttttcc 121980

atttgtttgt gtcctctcat ttctttgagc agtggtttgt ggctctcctt gaagaggtcc 122040

ttcatgtccc ttgttaagtt gtattccttt attctcttta tagcaattgt gaatgggaat 122100

ttaactatga tttggctctc tgcttgtgta ttattggtgt gtaggaatat ttgtgatttt 122160

tgcacattgg ttttgtatcc tgagactttg ctgaagttgc ttatcagcat aaatagtttt 122220

taggctgaga cgatagggtt ttctgaatat acaattatgt catctgtgaa cagagacaat 122280

ttgacttcct ctcttcctat ttgaatacct ttatttcttt ttcttgcctg attgccctgg 122340

ctggaacttc caatactatg atgaatagga atggtgagag agggcatcct tgtcttgtgc 122400

tggtattcaa tgggaatgct tccagctttt gcccattcag tatgatattg gctatgggtt 122460

tgtcataaat agctctattt tgagatatgt ttcatcaata cctagtttat ggcggttttt 122520

agcatgaagt agtgttgaat tttattgaag gccttttctg cgtctattga gataatcgtg 122580

gtttttgtca ttggttctgt ttatggaaag catttagccc atttacattg aaaattaata 122640

ttgttacgtg tgaatttgat cgtgtcatca tgatgctagc tggttatttt atacattagt 122700

tgttgtagtt tcctcatatt gtcattggtc tttatatttt gatgtttttg cagtggctgg 122760

taccagtttt tcctttccat atttaatgct tcctacagga gctcttataa gcagacctgg 122820

tggtgacaaa aatctcacag catttgcttg tctggaaagg attttatttc tcctttgctt 122880

atgaagttta gtttggctgt atatgaaatt ctgggttgaa aattcttttc tttaagaatg 122940

ttgaatattg accctcactg tcttcaggct tgtagggttt ctgcagagag atctactgtt 123000

agtctgatga gcttcccttt gtaggttaac ccgacctttc tctctggctg cccttaacat 123060

tttttccttt gtttcaaact tggagtatct gatgattatg tgtcctggga ttgctcttct 123120

caaggagtat cttagtggtg ttctctgtat ttcctgaatt tgaatgttgg catgtcttgc 123180

taggttgggg aagttctcct ggatgatatc ttgaattgtg ttttccaact tggttccatt 123240

ctccccatca ctttcaggta catcagtcaa tcgtagattt gattcacata gacccacatt 123300

ttattggagg ctttgttcgt tccttttaat tattttttct ctaatcttca tgttttgttt 123360

cattaatttg atcttcaatc tctgatatca tttcttctac ttgatcaatt caactatcca 123420

tacttgggta tgcttcacga agttctcttg ctgtttttca gctccatcag gtcatttatg 123480

ttcctctcta aactggttat tctagttagc agttccttta accttttatc aagtttcttg 123540

ccttccttgg attgggttag aacatgctcc tttagctcag aggagtttgt tattacccac 123600

cttctgaagc ctacttctgt caattcgtca atatcattct ctgtccagtt ttatgccctt 123660

gctggagagg agttgcgatc atttggggga aaataggcat tctatttttt ggaattttca 123720

gcgtttttgt gctggttttt cctcattttt gtggatttat ctgccttcga tgttagaggc 123780

taatgacttt tatatggagt ttttgtgtgg gagttatttt tgttgatgtt gaagttgttg 123840

ctttctgtgt gttagttttt cttctaacag tctggcccct attctgcaag tctgctgcag 123900

ctttctggag gtccactcca gcccctgttc accttggtat caccagtgga ggctgcagaa 123960

cagcaaagat tgcttcctac tccttcctgt ggaagctccg tcccagagcg gcaccagcct 124020

gatgccagct ggtgctctcc tgtatggtgt gtctgttgac ccctgttggg aggtctctcc 124080

cagtcaagag gcacaggggt cagggaccca ctttaggagg cagtctgtcc cttagcagag 124140

ctagagcact gtgctgggag aatcgttgtc aggatcagct gctctcttca gagctggcgg 124200

gcaggaaagt ttaagtctgc tgaagctgcg cccacagttg ccccttcccc gaggtgctct 124260

gtcccaggga aatggggttt tatctataag cccctgactg gggctgctgc ctttcagaca 124320

cgccctgccc agagagaagg aatctagaga ggcagtctgg tcacagccgc tttgctgcgc 124380

catggtaaat tctgcccagt ccaaacctcc cggccccctt agcattgtca ggggaagacc 124440

gcctactcaa gcctcagtaa tggtggacac ccccccgccc ccgccgccac caagttcagt 124500

tgtcgcagtt tgacttcaga ctgctgtgtt ggcagtgaga atttcaagcc agtggttctt 124560

agcttgctgg actccgtggg agtgggaccc actgagcaag accacttggc tccctggctt 124620

cggcctcact tccagaggag tgaatggttc tgtctcgctg ggattccagg caccactggg 124680

ttacgaaaaa aaaaaaaaaa actgcagcta gctcactgtc tgcccaaatt gtctcccagt 124740

tttatccttg aaacccagga ccctggtggt gtaggcacac cagggaatct cctgatttgt 124800

ggattgcaaa aaccatggga aaagtgtagt atctgggctg ggtagcacag tccctcacag 124860

ctttgcttgg ctgagggaga gaggtcccct ggctccttgc acttctcatg ttgggcgatg 124920

ccccacccta cttctgctca ccctccgtga gttggaccca ctggctaacc agtcccagtg 124980

agacgaactg ggtacctcag ttgtaaatgg agaaatcacc caccttctgc tttggtctca 125040

ctgggagctg ctgaccggag ctgttattcg gccatcttgc cagattctcc gaaaatgctt 125100

attaaggagt ttacttctca tttctctaaa agatacttgg gagagacagt ttatttaaaa 125160

gtattacatc tcttaatata ttgaaaactt ttcctgtgag gttatattaa atttacaact 125220

tttttaattg caacagagat aacataagca aacgtacact tactttgatg aaaaacttaa 125280

aatgcactgg ggactactat gatagaagcc acttgctaac attggattta tggtttgctt 125340

tcttaatatg ttatatagta agccaccatt tattgaatgg attaactaca tgggaccctc 125400

ccttctatgt tttatctgtg tacacatacc cttacacctt ggggcccagc ttaacagcaa 125460

agttcaatag ctcctgtctc ctgctgaaca tttagcatat tccaggataa cttctgccat 125520

tttacatttt cctcctccgt cactaatcac acatccaatt ccacagattt ggcatcttcc 125580

gatttaaaag tggtgtataa ggatttagta tatattgaaa aatatctctc aagattctct 125640

tgagagttaa ctgtgtagag tatagatttt actttgcata aatgtaggtg aaataaacag 125700

attgcaaggc tattgatgct gtcacatttt cagcaaatgt aaaattatgt atgtatacat 125760

gtatatatat atacacacac atttttttta atttaggatt tgctttccca tatgcttcat 125820

atggacccac atcagcggta tactgctgaa caaatattaa agcactcatg gataactcac 125880

agagaccagt tgccaaatga tcagccaaag agaaatgatg tgtcacatgt tgttaaggta 125940

aaacaaacat acttttatgt gccttcgaga tatcagatac ctaataaacc cattgtgtgc 126000

cacttgctga tgacacttaa ataattcctt aaaatatctt tattttttaa agatagattt 126060

ttttaaatta acaaccatgt acttttctta gcttacttta ctactgcctt tcagatgaaa 126120

caataaaaat ttcttgattg tacctgccgt gcttttgaga gatgctggaa ttaggattgg 126180

gctaaatgca agtttcatat tgaaataaaa gattgctgtt agttcttaat ttataactgt 126240

tcgtgaaacc attcctatat ccatatacat tgaatcaagg ttttaaatgt tagtgtttta 126300

acaatttttt gtaaaaagca tagttattga aatgaaaaac atacaagtaa tattgattca 126360

tcagtgcaca gatctaatat attcaattat tttcctagga aaacattttg ttctagatat 126420

atgtctcatg atatcatgtg aaagtttttt ttttttttaa ctccagaatt tgtacttagt 126480

ttgtggcatt ttctcattac tttagggagc aatggttgca acatactctg ccctgactca 126540

caagaccttt caaccagtcc tagagcctgt agctgcttca agcttagccc agcgacggag 126600

catgaaaaag cgaacatcaa ctggcctgta agatttgtgg tgttcctagg ccaaactgga 126660

tgaagatgaa attaaatgtg tggctttttt cctattctta tcaaaggcat cgttgtctgc 126720

taaattactt gaatattaag taatattaaa tccccatttt taggggaagt gagatttaaa 126780

aaaccattca caggtccaca atattcatac tatgtgtttg cagtagtgtt caagtgttta 126840

tttaagcata taattggtgt ccaccaggtc ctcacaactt ctctgcacac aagcttctaa 126900

aattcctttc aaataaagtt actttaatat tt 126932

<210> 2
<211> 304486
<212> DNA
<213> Homo sapiens

<220>
<221> promoter
<222> (1)..(3000)
<223>


<220>
<221> exon 1
<222> (3001)..(3113)
<223>


<220>

<221>  intron 1
<222>  (3114)..(57368)
<223>

<220>
<221>  exon 2
<222>  (57369)..(57517)
<223>  Exon 2 unterliegt einem alternativen Spleißen, wodurch die Regula
       tion der Expression beeinflußt wird. Die Proteinsequenz bleibt da
       durch unverändert

<220>
<221>  intron 2
<222>  (57518)..(130439)
<223>

<220>
<221>  exon 3
<222>  (130440)..(130589)
<223>

<220>
<221>  intron 3
<222>  (130590)..(197535)
<223>

<220>
<221>  exon 4
<222>  (197536)..(197750)
<223>

<220>
<221>  intron 4
<222>  (197751)..(260945)
<223>

<220>
<221>  exon 5
<222>  (260946)..(261731)
<223>

<220>
<221>  intron 5
<222>  (261732)..(275491)
<223>

<220>
<221>  exon 6
<222>  (275492)..(275983)
<223>

<220>
<221>  intron 6

<222>  (275984)..(278851)
<223>


<220>
<221>  exon 7
<222>  (278852)..(278985)
<223>


<220>
<221>  intron 7
<222>  (278986)..(284141)
<223>


<220>
<221>  exon 8
<222>  (284142)..(284268)
<223>


<220>
<221>  intron 8
<222>  (284269)..(297381)
<223>


<220>
<221>  exon 9
<222>  (297382)..(297507)
<223>


<220>
<221>  intron 9
<222>  (297508)..(299155)
<223>


<220>
<221>  exon 10
<222>  (299156)..(299290)
<223>


<220>
<221>  intron 10
<222>  (299291)..(302258)
<223>


<220>
<221>  exon 11
<222>  (302259)..(304486)
<223>


<400>  2
   tctgagcaaa tgtcctgctt catttctaga atctatgctg gttcccttat ggttgcggtt      60
   catctttcaa gaattccagc catttgtaga tgcatagcct ggagaaggta aagagaatga     120
   gctgggtagt ctgaacctgg gttccagcat gctgtcttac acatccctgt cagccaatct 180
   accaagttct ccacccctca ggttcatctt gtcacagttt ttggtgtcaa caaggttggt 240
   tgacattagt ttctcatgaa atcttgacta gtcagtagct ctgttttatt gaaaaacaca 300
   cttctattta ctatcaaaca cttattccta ccccattaca aattacaaat acagattttg 360
   acagagaaaa ttatgtctta aatggtagac aataaataca tgcctgctca ataaatcata 420
   attaaactag tcatataaaa aatgcataca gccagatacc taaatagata gtatactctc 480
   aatggctgat tacaggttat tttccaactt ttaaatcttt gagccagaga gaaagtctat 540
   tacttattca aaaataaagt tttaaaatct gtctgacaaa tcagtagagt tctactttgt 600
   gtgttcaaaa gtgctgtatt caataaatac atcaatgtgg tggacaaagg aatgttttgc 660
   ctaaattccc cttaagtgaa ggatttgtta ctgcagccac agggactaca gctgacagag 720
   gcctttagct gttgttacct gtaagaattg ccagagctgc ctgtcttaag ttcgtgcccc 780
   tcctaggaac agcccatgct caatgattga tgtggatgta aaggactgac tatctcagac 840
   caactcatgg tcattctgaa ggatcaccct agctagtgag ctccataagg ggctgacaaa 900
   ggctgcaatt ggcttgcatc acagcttagc ttctacctct gcctgctcct gcttcctgta 960
   tccctcccac ccacagatgt tggtccttag tcaaatactg caggctaaac cctcaagaaa 1020
   tggcattaca tataaagaaa acccttagga attcatgcaa tgcagtattt acagaaatag 1080
   cattattatt catctataat ttttaccttg cacgcttcca aaataaattt gaggcagcta 1140
   acaatcatca tcatatttaa ctagcagaaa caaatgtaca aacacaaact ttatgtataa 1200
   caggaaggct attttaagtg attaaagttc aatatttcaa ataaaaattt taactgatac 1260
   taaccaatga caaataaacg atatctcagt aaaaatatgt tctggctttt gcatctatgt 1320
   gagcacacat ataagaaagt caaaagtaga aaatatttat gcatatagtt gtgggggtga 1380
   cgatggtggc agtgggtagg ttgttagggc tgtgtgcagc tttgagaagg aagagatgag 1440
   tatgatacac aaggacatgg gactactgag ggagaaaagt aatcatccat ttatcctgtg 1500
   gtttctgttt tgatatggcc agtacaatat taaaggtact aagtgaatgg aaaacaagca 1560
   aggggaaatg ataactacta tactattagc tagaaaactg tctaaggtca ttgagtataa 1620
   taaggaacgg agagatacaa atccttattt tgtaacctgc tatctagtta tgggatcaga 1680
   ttccatatcc actactagct cataagggta ctcttgtggc agctgaagac agacaagagc 1740
   gtggcttctt catttccaag ggtgaaaact caggactact tcgtgaatgt ctttcattca 1800
   ctagagtctt gataatgctg gagacttcaa gagtctggga gggcagtagg caactgggaa 1860
   gtttaagttg aaccacaaat atcgttgcaa acttcagccc aaacatcact gaacacaaag 1920
   gcctgacatc tcttggtaca aaaatgcgct ctatgtttgt cataatctgt cggctttctt 1980
   aactaggcaa gcccaagctt gctattagat gtacaataaa cactttgatg cccagtctga 2040
   gtttttgtcc cagctcagga aaggcaaggg gacagccaag gcagactggt tcagacttga 2100
   ccggggcagc tcatccagca actggcttac tagccagcta gcaaagtgag catgaaaagg 2160
   tccaggaatc cttcttcccc tgggccttaa attcgaaatc agacagcagc caaggagaag 2220
   ctgggtggtc acagcctagc cccacaaaca ctccttccag gtaaggctca gcaaccccag 2280
   gctggaggag tctctgtgga caccagagac cattgctagg ctggcggatt gaaggatggc 2340
   cactgagagg tcacagataa ggggttctct agatgtttgc tactcggagt ggtgggaggg 2400
   ccagcagcag cagcatcacc tgcgaggttg ttaggcttgc agaatctcag gacccagcca 2460
   attgaatctg aatctgcatt ttaattcaca ggcacatgaa agtttgagaa tcaccggtcc 2520
   aggtggtggc aaagaggcca gcccactgtt ctgcttctca gcccctcctc ctccccacct 2580
   ctgctagcct cttaagcaaa actcaatcct gcaatggtcc tgaacaacat ttctgcaaat 2640
   cccgagtgtt gggggagagg gcgtgcagga gctgcccccg ccggggacga ctgggagtaa 2700
   acgtgattgg attggtcgcc tgctccagct cccggctcct ggctcctgca ccctgagtgt 2760
   agacacgccc cccgaccctc cccctcacct ccacccccgg ccgccgctac agcctgcctc 2820
   ccgccggctg ccacgaaaac ccggagcagc tgcgtacgct catggacagt cctccgaggg 2880
   gcgaagccgg gcagctgggc atgctcagta gctgggggag gtttgggtgg agagtagaaa 2940
   gctgtggctc tgcctctcac cccctcccgc tggcccccgc cccccttgcc cctacccagc 3000
   cagtagtagt tccccagcgt gcgcccgggg agaccgggaa catggcgctg ggagcgctgt 3060
   agcagctgag aaggggctga ggcaccgccg cttcgctgac agccggccac caggtaggca 3120
   ggcgcgcctg tcgggactgc ctgggtgggt atcggcgaga ggagaggcgc ctgcggggat 3180
   gcgcgccgct ggtaaccgga gcatctctct ttgccgggag aggatgcgaa gaggaccact 3240
   gtgacagcgc gatcctgcat ctcctaggag atgcaaaggc tagggacctg ctgcctggag 3300
   ggtagggata ggggaccgcc caccctcaac ccatgcgcta ctatccagcc tgggctctct 3360
   accaaccaaa taaaggatgg ttccattttg ctgggcggga gggcgtggag gagaaccccg 3420
   gattccagcc ctgagagcgt gctggtaccc cgaactgctc gcaagcccag caccagttct 3480
   ttgtgtgtat acagcagcgc acgccctggg ggtgccacgg cgctgctttt cccgagaagt 3540
   gttggacata ttgccagagc aggattcccg catggttttt gtcttttggc aggtgtggac 3600
   tggaggatgc aatagctgag cctttatgag gctgtgtttt cctttgttat ttgtgtgtgt 3660
   gtgtgtgtgc gcgcgtgtgt gtgtgcgcgc gcgcctggtg ggttgtaatg gagagacaga 3720
   gagagaactt ctgcttgtgg gcacccttgt gagcagggcg tggaagagga tcttggttcc 3780
   aaactcttgt caaaattgtg tgccatgtca gcctcctaga gggtaaactt gagaagtcca 3840
   gggatcatca ctgctcagtt tggaggataa gcgcgatcgc ccttcttgaa accatgtgtg 3900
   tgtatatgga gctttcaact cgggctgagt taatttgtca acattagata tcaattaagg 3960
   gaacctgaaa cgctgaggct atttatggtg gtctttttat ggccagaata ataaacaaaa 4020
   agaaaagttg gcacaaagcc agaggaaagt gtcattgtta atagttgatg acagcttgaa 4080
   agactgaaca cacttttagg aaaatcagga aacctggcat tctacggagt tctaccaaga 4140
   catagccaca gggtcttagg taactcacaa aaatcttcta gtgcaaccgc ttctcttctg 4200
   gggcttggag gctgtagaca ctctttctct tacctgatat agatggacaa gatgcttttt 4260
   agaagtccct cctacactag aattctatga aattgacacc ttataataac agctgtgcca 4320
   ccttagattt gtgtatctgt tatattcaac agcacaacaa ctcaatcttg gggagaaaga 4380
   taaccaggtt agttagggag aaaggtaaat ttaaagcatg ggtttatttt ttgctttata 4440
   atttaaagga tgggcatctt agcatatgaa gtttggagac agtttcctct gcactcacac 4500
   tatttatttt tatagtcttg ttaggtggcg agaatgagtc agtatcatca aaatgaagtg 4560
   ggggaataca taaggaaaat gcatttaagt aaattaaagc cataaatgtc caatctgata 4620
   ataccttgac ttggaataca tatgtgcaag gcagatgcac tgctaaatgg aagcaatata 4680
   agccaatggc aagttccttg gtaacgctgg ccttagacta aaaacctagt gttcttctgt 4740
   gagtgggtag agattgtact aggtctctgc agcttgttat tttattaata aatgtgagtt 4800
   taattgaaaa ttgatatcta agcattgaat caattgatat ctaagcattg aaaatctagt 4860
   ctggcagtgc ctctccactc tgtcaggacc tgctgaaatg ctgtaggtat gggaggcagg 4920
   tgtttgaact ctgactcttc tatgtttagc tttgtgattc tgggaaaatt atttaacttt 4980
   cctaagcctc agtttcctca cttatagaac agtcctcgga atagtaagac ttatctagat 5040
   gagtagatgt acggattaat cataatttaa accaccagga aaatggtggt gactgttatg 5100
   agtaagaggg aaatggatat ttactgaacc tatgctatgt gccctatata atgattcagg 5160
   ctcaaacatg ttaattccct taaatttccc aactatatta gtttcctagg gcagttataa 5220
   caagttacca aaaactgggt ggcttaaaac cacagaaatt tattctctca tggttctgga 5280
   ggctagaggt ccaaaatcaa gatgttgtca gggccatgca ccctccaaaa cctctaaggg 5340
   aggatccttg cttgcctttt ccagcttctg gtagccccag gcattccctg gtttgtggca 5400
   gcataactcc aatctctgcc tctgtattca catggcatct tcctctctgt ctctgtccct 5460
   gtgtccaaat ttcccttttc ttatacggac atcattcata ttggatttag gacctgttcc 5520
   ttattcaata tgacctcaaa ttaaccaggt tacatttgca aatgcctgat ttctaaacgt 5580
   catattcaca ggttctggat gggcatgaat tcttgggaga cactcttcag cccagtacac 5640
   caacatccct atgaactgtg tttgatgaca acattaacat tatgttagca taagaggtaa 5700
   agatagagta gcatggattc agaaaatact ttaaagatta tttaagggaa ctaacctatt 5760
   tgaggcttta tatacacaac catggtaggg aagagcaggg ttcaaaagta cctcatttat 5820
   gcatgtcact tacctgggag ccaagacaac ccaggacctg gatcttctag agtatctcaa 5880
   acatactgtg cccccagttg atttgttgat gttgccttcc atccttcttg ctaaccttcc 5940
   aaacaaatgc aaaaaaacca aaatgcatta aataacaaaa acaataatag caacaaaggc 6000
   aagctaacaa ctttctagtc cttcttaacc tggtaaatgg caccactatc catcgagttg 6060
   ctttaaccag aaacccaaga atcacctttg acacctcctt tcccttgacc ctccacattt 6120
   aaaaccatca tgcagtctta taaatgctat tttctctata gatctctaaa tacatcttga 6180
   taaattattt ctatccccac caccaccact atcaaactgc aagagatgat aatctcttac 6240
   ctggaccact agaataactt cctaacaaga cccctggttc cttgcctggc tcttctaatc 6300
   cttcatccag agcagagagg ataatgtcag ttccctacat aaaataccac ccacacacct 6360
   gggacagatt ccagagactc ctagagcctc tgatacttgg ccagcctcat cttgtgcatc 6420
   tcttcctctt gatgctgtca cactgaccct ctgtctgttt ctggaacatc cccaaccatc 6480
   ttccctctat attttaccag tctgggtcat actcatccct tgggcttcct tgatctctct 6540
   tccccaccga cccaactctc tcatcatcct ttcactttta ttccacagca cttattccag 6600
   tttgcaaatg cctgtttgtt tggctatgtc tgtaagctct ataatggcca tttattttat 6660
   gtcttatatg cagaaggtgc tcaatgaact tttggtgaat gagtgaagga acgaatactc 6720
   tgcctcatag cattttcctg tgccaacttc aagatctgat ttttagaagg tttttctttt 6780
   ctagtataga aagagcaatt tagaacctta ttctgacttc aaaaggagag tattcttggc 6840
   tttaacaaat gcatctttta agaactgatt taggctagaa gttaagaatt gagtcaaaat 6900
   atttttttgg caactgaggt ttttgtaaaa atacatcaat cagtcaataa atattctgca 6960
   aatatttatt gcatgtgtat atttgccatg ctacaccctt ggaagacaat ggtgagctag 7020
   actttcagtc ttctggaaat taatggtaaa caataaataa taatcattat gtataataat 7080
   aaataaataa tacatttaat tatttgttga gcttctgtta tatgccagtg tctcttcaat 7140
   tcctgtccat aatcatcact ataaccctct aatctttccc aataacacag aaccccacac 7200
   ttactaggag cacaacaaat attttgtgaa ttaataattt gttggtatta ttattctgat 7260
   ctgttgaaat aggaaatgtg tgcttatctg ggaaaaaagg tatctgcaca tatatagaag 7320
   agcatgcaat tgctttctca ccatatctag gtgggcactg ttttatttta aaaatcatcc 7380
   tggacattat ctctggaggt aaattaggag agaaattgct ctttataagg cgacaagttc 7440
   ctggtgtgtt tctgtcaaaa tcctgaattg ttttatttcg tttaattgtc taaatttgtc 7500
   ctgtttaatc caagtgagaa gtgacttaat gtctctcatt cagagccctt tttgtagttt 7560
   tgaatagtaa gactgagtgc ccaagggaaa tgaatgtagt tcaacagagg aacacatgtt 7620
   atgttttaga tttctaaagt ggcttcaatt tcagattttt atattagtct cataaaaggg 7680
   tgatttttaa aaataaacag taaaatagca tttaatgcct atgtttaagt aaagttttta 7740
   atgtaagtaa ccaaaaatta gacagtgtgc cttgattttg tttaaaagtg atggatacac 7800
   aatgggatca attaaagcct aaaggaacta gtacaaaatt tcagtaatgt ttttggttat 7860
   aaaatacatg ttcaaaaatt gatggccacc caaaatgcca gtgttttact aactaaaaag 7920
   tagaatggaa aatgattcta ttcatgaatt cactaatata taaattgagt gggaaaaaca 7980
   ggaaacatac ataaaatact cgtgaataat aagaaatata taaaagtctt tgtggaaagc 8040
   tatacaattt tactcaaacc aataaactga ggagcaaaca aagaaagaca taccttatgc 8100
   tttggtagaa agatgaatat tatgaaacgg ttatttttcc cagatcaatt atatagtttt 8160
   attacattgg gatttgggag aaccttacaa aatgattcta acatccatct ggaagaatca 8220
   ccatataaaa ataaaagaaa acttttcaaa agaatagtag tggtggtggg ggaggtgagt 8280
   ggtggtggta agatgggagg tggtggtaca tacaggtggt gtggagagtg gagttctagg 8340
   ggtggtaaag gtggggggaa gtttgatggt ggagacgaat acatgaaggt ggtgggatga 8400
   ggtggtatgg atgggggtgg tgggtggtag atgtgggtgg ttggggggtg aaggtaggta 8460
   gatggaagtg gtagtggtaa tgggcgccat tgttgaaaca aaagtaaatt tgatcattgg 8520
   ctacaatcat tatagcaatg tagtactggt gtaagaattg attaatagct cagtggaata 8580
   gaagagcctg gaaacagatt taggtcattc ttagaaccag aaatatgacg aagaaggcat 8640
   tatgaaacag tgaggagtat ttgacaaaac gtcatggaaa ttggctaagt gtatagaaag 8700
   cattcaacta agaacctctg tttacatgac cctccaaatg aaattccaca taaattgata 8760
   agtttatatg caaggtggaa aacaaagcta gaataaaata tttaactaat tttaggggaa 8820
   gtacttttta aatataaaat caatgggaag aatcacaaag caaaataaag atttgaatat 8880
   attttaaact ctcatatgca gcaatgcatg cattttaaac agaacagaat catggaaaac 8940
   aatattgaca ccaaacatga catgacagtt catgatagcc ctgaggaact cttgcaaatc 9000
   agtgagtaaa caatagcacc ccaatagaat tgtccaaata tctgaatgca taattcaaaa 9060
   ggaaaaaata aaagaccaat gcacacgttt aataaatgtt attactatta atcacagaaa 9120
   tgcaaattaa aataacaatg aggtggtgtg ttttatgtat taaaaaagta aaaaatacaa 9180
   aaaataaact taatactggc aaggatgcag tgagaaggac aatctctttc attacttgtg 9240
   gtgggagggt tacataatac aagcttccta cagagaagtt taaaaatact tatcaaaatg 9300
   tctgccaggc acggtggctc atgcctataa caccagcact ttgggaggcc aaggcaggtg 9360
   aatcacttga ggccaggagt ttgagaccac cctggccaac atgggaaaac cccatctcta 9420
   ctaaaataca aaaaaatagc tgggtgtggt ggcacactcc tgtaatctca acttcttggg 9480
   aggctgaggc aagagaatca cttgaacctg agaggtggag tttgcagtaa gccgagatcg 9540
   cgttactgca ctccaggctt ggtgacagag tgatactctg tcaccattaa aaaaaagaaa 9600
   gttgtcaaaa cttttaaaag tttaaattat ttgacacact attcctcttg ttttttattt 9660
   tatgttttct tttgtctttg gtaaggaagt agatgcaggt aaaattgtgt attataatat 9720
   atttcatagt aataaaaatt aaaaagaacc tatataacta gaacttggga gcttggtaaa 9780
   ggtatttaag gtcccaagat aacagactat tatataggta ttaaaattaa ttattttgaa 9840
   gattatataa tgacgtgaaa caaacttcac cacaatttta agtggaaaaa aacaaaatgc 9900
   actactacat atattgtatt gttaatatgt gttttgtgaa atgaaaacat catgcaggta 9960
   tatttaaaaa aacagaacaa aattaaaaag ttaggcaatc tctgggtggt ggaaatatgg 10020
   atggctttta ttttgttaca cttttccaaa ttttaaaaaa tgtagtaggc cgggcacggt 10080
   ggctcacacc tgtaatctca ccactttggg aactcgaggt gggtggatca cttgagctca 10140
   ggagtttgac accaggctga gcaacatggc aaaaccccat cactacaaaa aatacaaaag 10200
   ttagctgagc gtggtggcgc acgcctctga tttcagctac tcaggaggct gaggtggaag 10260
   gaatgtctga gcccatgaag tggaggttgc agtgagccaa gatcgcacca ctgcactcca 10320
   gcctgggcga cagagtgagg ccctgccttg gaaaaaaaaa ttagtcaatg atgtgttgtt 10380
   ttatgaatca gaattaaaaa atccactatt ttacaaataa gcagtgatat acatttaggt 10440
   tgtaattatt catttatgta acaaacattc attgattatc tcttgtgtca ggggctctgc 10500
   caagtgtatc agcagtgact cttagattgc atgcaaatga aagaaactct gcctaactcc 10560
   agcaaaagga atttactaga aggatattag gtagctaaaa gaattgccag gaatgtggcg 10620
   ggggatgggg aatcccaaac acacgcacgc gcgcacacac acacacaccc cccaaaacag 10680
   cttggaagca gatgaagcta gtcagttcca ggtctagata tcaggcactt tggaaacaga 10740
   ctccagccaa gaatgacttg ttcagagctt gaccacagtc cactggctgc tatgcttggg 10800
   gttcttcccc cacacccagc ccactgggca tgggatagga gggggggtgc cttgattagt 10860
   aggcccacta cccctttcca atgaaagaaa cacaagagat gtgatgggtg ctaaaaagcc 10920
   aaaaccaaac caaaccaaac caaaaaaaga caacagaaat gcccattacc ctaggactta 10980
   ggggtaaaca gggttgaata ccactcatcc ctctctttgc atcccatagg aaacatttgc 11040
   tatgacatgg gagtttaact taaggttcat ggtctaatgt tcactgtgat gtgcatcatt 11100
   gaaggtgcaa tgtgggtgcc ccacacagaa ggtacatttt cctcttggag ccagtagtga 11160
   catgttggga agagtgtgga ttttcagcct gatccttata taccggcagg aattttgaaa 11220
   gaaaggtccc attttctcag gtaagagaaa atcctagaaa ggtataatct gaaagaaaca 11280
   atgaagcaga agcaagaggt ataatatgga aatagatcag gcaccaacac aggtctagcc 11340
   ttcttttctg aatgatctta cattcagcaa gcccaacagt catgtttgag tttagttcac 11400
   catgaatacc agcctcttgg ttcaattaac tttggttgat aaagttatta ttaactaaat 11460
   attaaaagat catacttaga tcagtgcata ttcttactgc agttctcatg ataaatagga 11520
   aatgtctgtg gctgtccagg taacttaaca atcatgttgt tgcttttata actctttata 11580
   aacttttctg tgaagaaaga tgacttgtaa gtggaagaga aaaaagaggt aattgttctt 11640
   cacttgtgct taaaacagga cagagaaaat aatcttaaat tagcaagaca ggtccctgtc 11700
   ctggtcactg tatattagag ggctctgcta ctctgcactc caacttctca gcacctggac 11760
   agcactgctc ctctgcaaca aagacccctt gagctctttg cctccttctg agccttgctc 11820
   tgcatagctg ggaccttgga attcactgct catggggctc ctgggcccag tgggggttga 11880
   aaaaagactg ttggtgggag gtgtaaatgg agcttggact tgtggacttt gtagtctcca 11940
   cacaagtgct ggaggccatg gtagaaagag aaagggatgg ggaggggagc aaagactggg 12000
   tctccttgct ttgctatgtt ctggaattta agtactctaa atttaaatct ggcttctacg 12060
   ttgttatgaa ggcatatttg ttaagccagg aagatataac acatttaaat taacattttt 12120
   ttagcttgat ttatagtatt taaatattga gatctgtaac atgtgggact ccttttgtac 12180
   tcttgtctct caaatgttag gatcaggcct gctcaggtaa ctgttttaga aaggtaacac 12240
   tcaattgttg agacacacat gcataattag ctaatcatgc aacggcaatg tagattataa 12300
   gaaaaataga ttatactttt tgtgactata gctaacatat tgaacaagat gtgttaagcc 12360
   cacatccaag tattttacct atgtatatta attagtcttc ataagaacac cataaaatag 12420
   gtcactcaag gcaaaatatc atgcagctag atagagtcaa gaagctctgg atcatctgaa 12480
   tccaaaggcc attgtggtat acaacaattc aatggataag aaaagtttta tctgaggtcc 12540
   ttgaacctcc gataatttaa aagtcactag attaatcaac ccactttgac tctccaatac 12600
   tctcaaatcc tcagattcag catttctgtt cgtgcagatt tcctcatgaa aagggaaagt 12660
   aaatgaatgg actgccttgc agtgtttggt attatgatta actgaatgtt ccaaaccaaa 12720
   aatcaatatt tggttgtttg tataagaagc acatttaact tacattttta aacagatgtc 12780
   cagttgtggg atttccttag ctaattttca atggaattgc cattgtgtca ttgatgataa 12840
   aggtaaattg gccataaggt gtacctttgt ttctctaaag cactggttca tacccctact 12900
   actcttctat taccctattt tccaactgaa ccaagaagac tttctgcaat caatattggt 12960
   tccatcactt ttgataacaa cttacataat gggggaagga aaggaagaag ggagagaagg 13020
   ggaaagagaa aaacagagag agagagagaa actcccacct ttggttccaa atataagatc 13080
   tctgttagac acaacagtta ttagcttcat ttccaattaa aattcaagtt tggcatacta 13140
   tttaagaaat tctttagcta ttttctagag gaaacttcat gtgtttgctt ctaatggtca 13200
   ctggattcat gcaaatggtg tttgtataat aatgattttt tagctaattg tttttagctg 13260
   tcagctgtgt tgccccctta ttttatagct acattacagt ttactgaccc aggaagtgaa 13320
   ctttctggaa gaatagaaaa ttcacacttt atttgtttaa aagaaagatt cttcctctaa 13380
   agtaggaaca gttgctatgt gggtatgatt taacttctaa aaataaaaac actgacggag 13440
   gagggggatt taaaaaaatt ttacagaaac aacttagaat ttttctcatc ctggtggttt 13500
   ttaggacaat gttctctctt ctctaggtat tagagataga gaaacagaca aacaaaaata 13560
   atttgccaat aatatttgaa taatagttat tcacgccttg agttaagtag tggtttaacc 13620
   ctttttgagg gttaagataa cagctaccta cctgcaggtt gtgtgctatc tctttcagat 13680
   tccaagtaga gtctactctt ctcctaccct cacagccaag cagtatctaa attgcaactt 13740
   ggctgggcgc ggtggctcac gcctgtaatc ccagcactct gggaggccga ggtgggtgga 13800
   ttacctgggt caagagttcg agaccagcct ggccaacatg gtgaaaccct gtctctacta 13860
   aaaatacaaa aaaacattag ctgggcatgg tggcgcacgc ctgtgatccc agctacttgg 13920
   gatgctgagg caagagaatc gcttgaaccc gggaggcaga tgttgcagtg agctgagatc 13980
   acgccactgc actccagcct gggcgacaaa gcaagactct gtctctaaat aaataagtaa 14040
   ataaataaat tgcaacctaa aaaacatttt ttgcatgtgc ccccttccct gtctccactg 14100
   ccactcccct ttgcatctct caattagatc ttctgtcagc ctcctatctg ttctctttca 14160
   ttcttacctc caaaatgatc ttctgttatc tgagtatcac accttcattg aaaactttct 14220
   atacagctac cacattcccc caccctcccc ctcccccaaa atcctcccac atggttctgg 14280
   attgttcctc tcaaacagtc atcacaatgt tttgtagatt tttttttagc ttccttcttg 14340
   cactaaactg ggaactcttc gaaagcaggc atcttgtttc ttcacctttg tatccccagc 14400
   acctggcttt atgcctacac atgtcaggtc tttaataaat gtctatggat gaatggatat 14460
   tacccaggct aaaaaatttg aatgcaaatg tgaaaaggcg gtgctatgat ttaaaaggcc 14520
   actcaaggca aaatatcacg cagttagata gattcaagaa gctgagtcaa accctgtatc 14580
   atctgaatcc aaggcaatca atgctgtggt atacagcaac tcagcagata agaaaagttc 14640
   tgtttggagt cctaaacaaa aattggctaa tctaaaatca gctaatctaa aagttatctc 14700
   atccattttc tttaaaaaca cacaaactag tttcacacta atactggttt tctattttct 14760
   gtgaataaaa aagaggtaga gagaaagatt aaaccctcta cctgtttctg gttggagaaa 14820
   gaaaaaaaat tccaatattg gacatgggta taaggtagag attctaaaca catggaaaag 14880
   agagaaagtg gtgaagcaaa ctataagctg agagccggcc tccccacaat agagtaaata 14940
   aactgagctt cttgaaaaac attgagaata attatttgat tgctagatgc agcttaacac 15000
   ctactgctct attgtcccag acctggtatg aatatgccta attggtggtg tgattcctgc 15060
   tcaattatcc cttgatttgt gtagtgaaag ctggggtttt atatggttct gcccaagtct 15120
   tcggcaccaa tgtttccata gcaacagatg cactataaaa tgaggaagaa atcgaaagtt 15180
   tgggagagaa ataaatcaaa gcgagtgatc ccagcaagag gttccccagg gtgagtccca 15240
   agggcttttc aggcttctag tcagatatat ttgcggcaga attctcggag atagaagatt 15300
   ctaggatgat aaatgccagg gattttttaa agcaatatat ggatactatc tatgacagca 15360
   gttaccttgt gtgcttcagt ttggatttct aaggcaagtt cctgtgttgg gatattagga 15420
   gtctacactg gtcgaaagaa tcacttacca gttagagcct actaacccct aggtaacagg 15480
   atatgaaaac tggagatttt tactatcttt gaaattgaat tgagctatcc ttctaaagct 15540
   gaaaggtgta gaacactgtg aattctatta ggtactaaat atttgactat gtcaatttag 15600
   agcagtgtct aattttgtgg tatagatgtg ttctgtttaa cacagacagt gtgaaatttc 15660
   ccagaaaaaa aaaaatctag atttctgact tctcttattc aaaaaatcac acacacaaat 15720
   acagacacat acacttagtt gcaaaatttg tcaacactga ccatgtttct tcatggcaat 15780
   catttgctgt agctgagtaa cagctgcctt taacatggat cagtcatgga gtcttcactt 15840
   tgtcacagtc cccatcactc cctactgcct gtaatcaggc cattttaatt gtttatgtta 15900
   ccctcttggt cattaaagtc ttgaatgtat aacctttgaa tttgagtgaa tataattcaa 15960
   attataacct ttgaattgct ctatattttt ggatcatttt atcctcatat attgtgtctt 16020
   cttaattttg cttcccaaaa atatggacca acttatttaa taatttaaat ataacgcaaa 16080
   tgacatttta gaaagatgca ttttaaaaat tttatcctag aaaaaaaatt tagaagtaag 16140
   actttatgct ggtatgaatt ttatttaaaa gtatagagtt tagagatatt tggtaaaaac 16200
   atttgcataa ataggctgct gcaactactt tttcaaaatt tctttcaaca ctggacaatt 16260
   tttttaaaat ttctgttgtg ttttatttga actaaactga aatggaaaag aaatcatatg 16320
   gtcttaagct tgctggaggt agggattatc aaaacgcaaa aggatggtat catatttcat 16380
   atgatattat catctggaaa atataaaact ttcacaatgc aactgtgctc acagcagtga 16440
   agtagaacct tataaatgat atgcattctg tagaatggct ggaaaaatct caaaatcaat 16500
   attggaatca cccagggcaa ctaagaataa caatatcact gtaccttact aaaaaataat 16560
   aaaaaacaag tgagatagaa aattccagag ttgtccctag agtttttatt tgcttgggat 16620
   agaaggatga tagcagtagc ttcccatttc ttttcagacc tcaattttat tttaaacaca 16680
   gagcacagtc cacccactga aaagaatttt cttttctaca aagaccaggg tgtgcaatga 16740
   ctgtgagtat cactgtaaaa cagaagaaca tttggctcta agttactcac ttaactagta 16800
   ggatcaaaac aagataactg gatctgtcta ttaggctagt gtctgtttat gtctgctttc 16860
   tcctgtaaag actagattaa aaaataggaa acagatgtta caatgaacat tcccaacgag 16920
   tcactcaccc attcagcagc tgcaccttga gtactattct gcttacttca ggtggcatgt 16980
   accatgagga ggcatatcaa tgaatataga agataaaaat gttcttctgt gaacatagaa 17040
   gataaaaaaa gaaggagaga cagacaggaa acagcataat aaataaatgt atagaatgcc 17100
   agaaggtcat cagtgcttta taaaatagag tgggataagg gaaatcaaaa gggctgtgat 17160
   gggtggcagt gaagggcgtg attttaaata ggatggtcag aataggtata attgagaaag 17220
   tgacatttga aggaggagag gaaattgccg tgtagataca catgggacag catcccaggt 17280
   agggatgggg accatttttg cagtgcactc caaattgcat atgatttgtc tcctgcaaag 17340
   accctcaagt tttttcattt ggaagtagaa ttttagtgct ggttcaaacc ctaacctcat 17400
   tctaatgctg ctaaggacac cattactcca gtcctgccaa ttcttctcta gataaaccat 17460
   aactgctttt tggaaatttt tttttaagag gcagggcctc actttgttgc ccaggctggt 17520
   cttgaactcc tggctttaaa caatcctcct gccttggcct cccaaagtgc tggaattata 17580
   ggcataagcc accacacacg gccttccttt tggagattta atattctttc tccattttgg 17640
   tttaattcaa atgaaacaca acagggattt ttttgaaact gtctaatatt taaaaagtat 17700
   ggaattatgg cacatactag gtatttgttc agttttttgt ttggttatag aaagaaaaga 17760
   tgtgcctcag agttaaaaca atgattgagc agtaatcaag ttctttgaac atgtattata 17820
   tgccagatat tgtgttatat gttttgtaaa cactatctca tttaattctt gcagtaggct 17880
   tataaattag gtgttatcat tattcccatt ttatggctag taaatagcag attgtgctgc 17940
   cttgctctat tgattaagat taatcttcta caagcacaaa aaaaaggtaa aaagtatttt 18000
   ctcaagttct aagtgtaatt cctttaactt cctctatttt agttaggatt tcccccccag 18060
   aaacagacct tgaggcaagg attcgagagc aagtagttta cttggaacgt tcatggaata 18120
   ccgttaagag agagggcaag taacacatgg aaggggagtt tattagtctg ttctcacact 18180
   gctaataaag acatacccaa gactgggtaa tttataaagg aaagaggttt aatggactca 18240
   cagttctaca tggctgggaa ggccacgcaa tcatggtgga tgacgaagga agagcaaacg 18300
   atcgtcttac atggcggaag gcaagattaa ttgtgcaggg gagctcattt ataaaaccat 18360
   cagatctgtg agacttattc actaccaaga gaacagtatg ggggaaccac ccccatgatt 18420
   cagttatctc cacctagccc cacccttgac acgtgaggat tattataatt caaggtgaga 18480
   tttgggcggg gacacagcca aaccatatca gaaactgtca gtccattcct gctgctataa 18540
   caaaatacat tagactgggt agtttttaaa taatagaaat ttatttctca tagttctaga 18600
   ggcttggaag tccaagatca aggcaccagc agattcagtg tctggaaagg gcttctcctc 18660
   tgttcaagat ggcatcttgt tgctatgtcc tccagaggga aaaggatgaa tgctgtgtcc 18720
   tcacatggca gaagggtaaa aaagggataa acttgcttcc ttaagctttt cttataagga 18780
   cactaatctc atctatgaca gtggagctct gatggcctga tcacctgcta aagactctta 18840
   gtatttttac cttcaggttt aagttccaac atgtgaattt cagagggaca tgtacatcta 18900
   aatcatagca gaaaggctgc caaaattaga gtattaaaat agaggaactg ggaaatggta 18960
   taaaactcat gcctccaaat cagtccaccc aaaggggagg gaactgagat atttatacac 19020
   caactcccat cagtcattag ttgaaggctg ttcccagaga atgttagttt ctctgtactt 19080
   ccaaactccc atgatatgga aaagccctta gaaacacaga tactgactgt tggaagtcag 19140
   taggcatatg ggtggatact aataaggctt gctacatcta tacctgttgc ctcccttgtc 19200
   atgtgcctgc caaacttcag agatccttta gaagttgttg taagaaaaga aaatttatct 19260
   tctgctagtc aattgtgagc tattgattgg gcaatttatt agtgtagaca agaacattcc 19320
   atgtacagtg ggtaaaagag cattgagcca gagtgttaag ttgccctcaa agtcatttcc 19380
   aaatgaacaa gatgatataa ctataggtgt tcaattgtac ataattaagc agggagctgt 19440
   tattataaac gcatctaatg ttgaaaaatg aaacataagt aaggctacaa ctgaccttgt 19500
   aatgaaacag aaagataaaa tattcctaag agtaaatcat ttgactgtgt aatgtaatgt 19560
   aattgctttt tttttttttt ttttgagaca gatctcactc tgttgttacc caggctggag 19620
   tgcagtggca ggatctcagc tcactgcaac ctctgcctcc tgggttcagg caatccttca 19680
   gcctcagcct cccaagaagc tgtgactaca gagatgcacc accacacctg gctaattttt 19740
   tgtattttta gtagagatgg ggttcaccat attgcccaag ctggtctcga actcctgagc 19800
   tcaggcaatc tgcctgcctc agcctcccaa agtgttggga ttataggcgt aagccaccac 19860
   accgggcctg cattcttgta gggtactggt ggaccagttg ataagctgta tcttccatgg 19920
   cattatttgg aactctatat ccccaaaaat gttgttcatg cccatgatca tttttaggaa 19980
   tagctatctt gaaacttggg aaagctggag aatatataat acaaaatgat tttgagtctg 20040
   tcttgcaaga aaccccaaac caaaaataat tttgcatgga gaaagaagag aatgtaacaa 20100
   gtatgctgag agcagaaacg caagtgttag tcctctgtat gtgtggataa acagtaaatg 20160
   tcagtgaatt ttaactgagt gataaagtgt atttgatcag gaaaaagtga agaatggatg 20220
   ttgggcagtt agcagcctga agtaggatct caaaaataac ccaggtggct ttctcgatga 20280
   gaatgtatca gttgaggtcg tttaggatag tagagaaggt gttttaaaat tgtggagcaa 20340
   gagggggatc caaagttagg tcatatcagt gattgccaat aggatactca ggttaggcag 20400
   gctacaaagt attgtgttct gaaaataatc acagttaacc ttggatttga gatatggatg 20460
   ggttttaaga aactcatcat ctttttttgt ttgtttgaca gagtctcgct ctgttgccca 20520
   ggctggagtg cagtggcatg atcttggctc actgcaactt ccgcctcctg tgttcaagca 20580
   attctcccac ctcagcctcc tgagtagctg ggattacagg tccccaccac catgcccagc 20640
   taaattttgt atttttagta gagatggggt ttcaccatgt tggccaggct ggtctcgaac 20700
   tcctgacctc agatgatccg cctgcctcgg cctcccaaaa tgctggatta caggtgtaag 20760
   ccatcgcacc tggccattat cattggtatg gacaggaaac ctgttttaca caccagaggg 20820
   caccatgggt tctagagttt cccacttgga gtaacagaaa agtgttcaaa atttctggtg 20880
   ttcctaaaag aataaagaga agaaatttgg ggagagctat cagggctgga ggaagagtgg 20940
   tgcttagggg ctttgcatca ccaacattga gccttccttt accaccttgg gttactctaa 21000
   ttgttgaacc tgagtgttac agttctgctc ttccctggtt ctcatttcag ggctccccat 21060
   tcagttcctt tatattcagg gcttccgtgt tacacagctt cagaagacaa cattcacatc 21120
   tgaggtgatt ccacttttgt tgtgcctgcc ttggtccata ctttgtaaaa tgggtcccct 21180
   tgcttcttaa ttatgtgtgt tttgttctcc ttctttgtcc cattggactg gaagaggcag 21240
   gaaggaagag gctaaatttt atctagttaa gttagagcat ctggcatagc accttccacg 21300
   tacagttgct caataaattt ttactaaaca aatgaattca ataagaattc tgtgacctac 21360
   tgagacttac atctccttta atccaaatag cagccccatg ggttacatat tgccttcatt 21420
   tctccttgga ggaggtgagg agtagatcaa tgaatggaca cagtgagtaa aaacaactct 21480
   ttcaataaaa ataggttgtg gcccaattta attagactct aaaatgaaat aggagaggcc 21540
   ttctgcatat aggggaaatc tgagttttat aaaatgcatt taaagaaaat aagctttcct 21600
   tctacttatt tctaactctc aaattcattt tcatgacagt tgatattatg aggaggaaat 21660
   aatgaataga ttttctatag attaaacttg tctttgcagc tggatgttat gggaagcagg 21720
   gcgtatcttc aacaggattc ttttgtttga aaggggcaga aacccaactc aaagtggatc 21780
   aagaaaacta ggaatttgtt gacctgtata gcagaagcat ccagaaatac agttggatcg 21840
   ggagctcaaa aacattatcc tggtgccatt tcttccccat ctttcagctc tgagtgtttc 21900
   tgtatagttt tctttcttgg gcaagctatc ttatttttct ggcaacatgg acagcccccc 21960
   tacagctctt gggtttagat cctactgatc tcaaaatcca gcaaaaaata ctctctagcc 22020
   caatagttct aacaaaagtc tcaggattga atttcagtag ttctaatata acatttgtgg 22080
   gtcatgtgcc agtaccggtt ccttgtctaa ttactgttgc ctggggaatg tgtggctttt 22140
   gtattcccac ctgggtccaa acccacccct agagtcagaa atcagagtgg agttactttc 22200
   tcccaaacca ccttgacaga gaacgggggt agagaggatc tgcaaggatt atcaggaggc 22260
   cattatcaga agcatagcaa agaatgtggt caggcaaaaa aaaaaatgtt ttttaaatgt 22320
   tcattagtgt aggtaaaagt aaaattgagc aatataatta tagtctccac aaagatttct 22380
   gacccagtga aatttctctg gtcacaggtt cgttggccct aatttactgg actgattgca 22440
   tttgaaaata tctatctttt cctggaaatg aactataagt taccaaagac accatacaga 22500
   gactctttag ccctatcctc ttgtgatctt agctcttcat ctttgtgtcc tattttgcca 22560
   ttgtactgct gcttctattt tgaaaccttg gtaagaaata gatttgtgtt ggtttactga 22620
   tttagtctga aattaaaatg aaaaatcatt tgcatattca gaaccttact taatgggagg 22680
   gaaatcggtg ataaaattgc atcctaaaca tttctcaggg aaatgaactt atagtgagtt 22740
   agtgaggaca tattgtcatt acaaggtaac atgaaatata tgcacccaag ccctcagctt 22800
   atcctgatgc tttctttctt tggctttctc atgcagagta tattttctct cagtgattta 22860
   agtgatttgg tattattttg tcctatatag cttcatgttt tattagatca attcaacttt 22920
   tggacaagac aaattggaaa gtaactttga tagctactat atgttatttg gctgatatta 22980
   tttagttgaa atgatgtcca atacccatgt ttctttatat ttgcagtcct tcataccagc 23040
   ctgtgttcta tttttacagt gatacagcta tcattttgtt tggttttaga tgttcatgca 23100
   ttctagagaa agtggaaaac ttagaagcct aattaatgac tgtcttctgg acctctgaga 23160
   ccatgtttct agtgttttcc gtggaatatt atcaggtata gactttatga taattttcat 23220
   ccattcagag attaaatatt aaattgcttt aggaatatta cggtctatgt gcatctgttc 23280
   agatatattt gctttccttg gaatgctttg aattcttggt ttttgcagat tatcagtctc 23340
   caccaagatt catccttgcg agatactgtt cccatgttaa tggagggtgt ttcagtgata 23400
   ttcgttaaag cagagtaaaa aatactttat ttagggccat catgataggt agagggaccg 23460
   agcaatgaga tttcacagtg agggacagag atggagctta actctgaaca cagcatggac 23520
   cagtagtaat ttatattgcc aaggagcaga gtaggagtgg atggaaaatt accaagagga 23580
   aacatctggg taaggaggat ggctaaactg acctaatggg attcttgctt aagacagtcc 23640
   agcatgatca gacatacctg aggaataatg gaagaggagg aagaatctga tcagatatgg 23700
   agagtgatca gatgtcaagg tggaaggatt ttgctaaact gacttagcag ggtattttgt 23760
   taaaactgga ttttaaaagg aagtgaacag ataccaaggg tgtttactct gaatttgcat 23820
   ttcttctttt gtgtttcttt ttaaaaaatc cttctttgat tttacaacag aaggagttga 23880
   gtatttgttt tagataaata aaagctggat tttatgtggc aaagatagct gatgacccct 23940
   agctgctctg tgttcctatg aagaaagtcc cactgccaga gattggccat gcctggcctt 24000
   caccaggtgt gacctcatta actgagaatt gatagctttg gaatttgaca taaatcatag 24060
   agtgtgtgca aaggcagttt atgtaatggg taagagattg gattatggac ttggcaagca 24120
   tggattgtag accctgtcca gaccactagt tgtgaaaatt ggtcaaattt attagtttct 24180
   ctaagcactg atttcctaag cattaaaaaa atattattag gtaccttgtt tgggctgctg 24240
   tgggttgtca tcactccatc caaaatgtga ctctgattta ctttctacat atttttttca 24300
   ccatctgaaa ttatctcgtt catttatctg tttattttct gttatctttc ttcttcacca 24360
   gtaagatatc tttccccgga gattaaggat cttgcctatt ttgtttggcc tgctatctcc 24420
   agggcctggc acatattaag agatcactat tgtgtgtgtt gaaggaagaa atgaatgaat 24480
   aaatcgggct tagcagagaa gtaggcactt agtagtgctc aatgaatgta cattatgaca 24540
   tttgctggta cctgaagctc ttctatgtgt agacttgaaa gtctttcatt tggatagatt 24600
   tttaaaaaaa ttatacgtat ttaaaatcat ggaaactgag agcttgaaat tgtagttttg 24660
   agactccaat tcagccaaaa actgttataa tgttgagtgt tcttatttac ataattcttt 24720
   atttttaatt tttattattt attcatgtag ttagttttag agacaggatc tcactatgtt 24780
   gcccagcctg gactcaaact cctaagctca agtgatcctc ctgcctcagc cttccttgta 24840
   gctgggacta taggtgtgca ccaccatgcc tggcttgtat aattcttaaa ctcatattat 24900
   actataagtt tcctcctgta aaataaatac caggtctcag cagttagtag cagacacaag 24960
   attccaggtg tcccatattc tctatacctt gcatcatctg catagtaatg gttacagtag 25020
   aaaaaataga actgatacca gtagtaatag tcatggctgc tgtactagtc agggttctct 25080
   ggagaaagga gatttattat aaggtatggt attggctcac ataattatgg aagcagagaa 25140
   gtcccatgat ctgctatctg caagctaaag acccagtcaa gccagttgtg gggttcaaaa 25200
   gcctgagaac cagagagaaa gtatggattc caatctgagt cagaatacct gagaaccagg 25260
   agtgcagaga acaggagata agactgatgt ccaagctcaa gcagttaagc agagagcaga 25320
   ttcaaccttc ctctgccttt ttgttccatt caggccctca atggattgga taatgcccac 25380
   tcatgttggg gaaggcaatt cactttaccc aggctgctga ttcaaataca aatttcttct 25440
   ggaaacactc cgatggacat acccaggaat aacatttaac cagatatgtg ggcattctgc 25500
   gatccagtca agtcgacaca tgatgttaac catcacagca gttaaccttt atttcgctat 25560
   taatgggaca gataatatac tcataataac cctatgaagt tagtacaatt gtaattcctg 25620
   tatttaattt taatatttaa ttttttaaat ttcccaggaa cacagattca agttgcccag 25680
   aatatatgct ccctataatc acatatttca gaggacaaaa ctgaggcttt agaggccaca 25740
   ctgccacttt ctgaacccag tcaagtcttt ctccagagcc tgccctcata accctgaagt 25800
   actgacccca gagttccaat ctcctataat tcagaacttt catgaaatca gactgacttt 25860
   agacaccaat gactcagaac tagggagttc tattgtagtg tgactttgga gctctctgaa 25920
   tggtgcatgt aatttctgtt ttacagagca accttcatta tgagaagctg tgtgtctggc 25980
   ctgaaggaag ggaacactag agaaggggct ggttggtttg agttatgtga cagtctggca 26040
   ggaactgtca tttggcatgt gaaatagaag catattggat tttattttcc cctgagtagg 26100
   ttggtgggat taaatttaat taccatttta ataagcagaa gaaaggaaca atcccaaata 26160
   gtagaaacaa aaagaattgc tatggtagac cactaatcat tagaaagact tatttttgaa 26220
   tcatgaggga ccacagtgag aaaagcagaa aattcagaac acagagaatt ataaccgacg 26280
   ggaccattgc aaaattatac ctgaacgaca gtttgacaag tgaaagtaat tactgttcca 26340
   tgtcagagct ggggagggtc tgtcttaggg gatggagcaa tgaggatggc ccaaacctga 26400
   gtgtatggat ttgtagagct gcttcagaag ggagggatgg cccagtaatg tcaactgagg 26460
   cctaatcatg actttcttcc caagaaactc caaggactac acacatgcat ttcagttttc 26520
   actctgtgtt cctgaggaat gatggcaggc attcatattc atattttcaa tgcagatatt 26580
   aaaccagatt tatatggtgc tatggtttgg atatggtttg tttggccttg ccaagtctct 26640
   tgttgaaatt tgatcctcag tgttggaggt gggcctggta ggaagtgttt ggatcatggg 26700
   ggcggatcct tcatgaattt tttggtgcca ttctcgaagg agagagtgaa ttctcactct 26760
   tatttcctgc aagaactagt tgttgaaaag agcctggcat ctcctccttt ctcttttgct 26820
   tcccctcttg ccacatgatc tctgcacaca gtagcgcctc tttgccttcc accataagtg 26880
   gaagctgcct gaagccctca acagaagcag gtgcaggtgc tatgcttctt gtacagcctg 26940
   cagaaccatg agccaaataa accttttttc tttataaatt acccagcctc aggtatactt 27000
   ttatagcaac acaaacagac taagacatat ggctagcaat atagatagca aaagcgtcac 27060
   atgacttcca ggtgtccacc ctcgtgtcat tgctgttgcc tgcacaaatg ctcaaaatat 27120
   ggcatttggg gattctgaat ccaaaaatga ttcaggatag tcagattatg aatggacaga 27180
   tactttagta agaatttagc tgatttgttt ttcttatata tttatttttt atgtatgtat 27240
   tttatgaaag agtggcatat gatataaagc tctgactcag taaatctaaa agaacacttt 27300
   tacaaggtaa actaaaaact ctaatagtta acagtgttgt gatattttaa ttggcaactc 27360
   tttttctttt ctcagtgatt cctaaaataa tggtgcatct tgcaattctt ggtgtcttgg 27420
   tcaattaaat tcttggtcaa tgaaatatgg caatattaac cacaatcatt ttttcaagac 27480
   atgctagaca cagtgttaag cccttcatgt gcattatatg atttattttc attctcattt 27540
   cactgatgat gaaactgact caaacagcta aaaaacctca caaaaatgcc cataatttca 27600
   taacaattcc tatgtagaat ctttcaacgg cctcactttg gatctaaata aaatgtaaac 27660
   tccttgcctt gtttatagta cccttttcta acatagtcct gcctactctt cccacttcat 27720
   ctcataccat tctctgcctg gccatcctgt tgaagtccac tgcaattgca cttgcctaga 27780
   atgcacttga ccaagaacct gatggacctg gctcattctt gtttttcaga tctcagctta 27840
   tatgcttcct attcagaaag gtgtttctga cattcatcct aaaatagcca gttaatctct 27900
   atcctatcat atgggttcat ttttatttaa agtaattttt tgaaggttaa catatacaca 27960
   aaaaaggaca cacattttaa ggctataact ggatgaattt tcaccaagtg agcacacctt 28020
   tgtacaagca cccagaagcc cttgctggat ttccttctag ttgctcacta cacccaaggg 28080
   caaatgccat cctcagcttt gtcggcatcg attaaaattg gaatcataaa gtcctcattt 28140
   gtgcctggct tctttcattc actatgttat tagtaagatt catccgtgag gttttgtgta 28200
   gttgcagttc acttattctc attgctatat agcattccac tgtatgaata caccacagtt 28260
   ttttctgctc ttggtggaca ttggctggtt ttccatttag ggctattaca aacactgctg 28320
   ctatgaacgt tactgaacat tttttggtga acatcatgtg aattaatttt aactgtatta 28380
   cttaaaacta tgtggtatgt atttgcctat ttaaactttt tctgtctagc tttaccccca 28440
   tcacaatgtt actcctaaga tggtaaagac cttgtttata ttttttctta ttttattttt 28500
   gagctttatt gaggtatacc tgtcttacaa tatacaccca tggaattgtt actgtggtta 28560
   agctaatgaa catatctgtc acccccaaaa gtttgtcctt gctccatagc agtccctctc 28620
   taccacttct tcccgtagct cctatatctc ctaccctagg tccctattgc tctgttttct 28680
   gtgatgttca ttagtttgga ttttctagaa ttttttatat acgtgaaatc atacataagg 28740
   tactcttatt ttgctttcac tcatcacagt tattttggga ttcatctata ttgttgctta 28800
   tatcaatagt ttatttcttt tcatttgtga gtggcattcc attgtatggt tatactatat 28860
   agtttgctat agatttttta gctttaagtt actattaaac tttttatttt ttatttttta 28920
   tggataccta atagttgtac atatttgtgg ggtacttgtg aagttttgat acaagcatac 28980
   aatgtgtaat atcaaatcag ggtaactggg atatccgtca catcaagcat ttattatttc 29040
   tttacattag gaacattcca atttcactct tctagtaatt tgaaatatac aataaattat 29100
   tgttaaatgt agtcacccta ttgtgctact gagcattaga tcttattcct tctaactatc 29160
   taatttgttt atccattcat ctgttgatgt gcatttgagt tgattctaac ttttgcctat 29220
   tagaaatgat gctactatgg acatttatgt atgaactttc atatatatcc ctctacttcc 29280
   cttgggtgta catctaggag tgtaatgggt gggtcatatg gtaggtacat gtttaactct 29340
   ataggagact atcaacctgt tttctgtctg agagttccaa ttgttccaac actttatagt 29400
   aagtcttttt aaaatttttg ctaatctgat aggagtggtg atatctcatt gtggttttat 29460
   ttcacatttc cccaatgact aatgatgttg aacatcattt ttatatgtta cttgccattt 29520
   gtacatcttc tctggtgata ttgtctcttc aaattttgtg ttgattttaa acatcgtttt 29580
   ttttttgtta ttgagcttat gaaattcaaa aataaatatt ctagatataa ggccttttta 29640
   aggtatgtta ttcaaatatt ttctcccaga ctgtggcttg acatttcatt tttataatag 29700
   tcttttattt taatgctgtc ttcctaggaa ctagaatagt gattggcaca tgttggtgct 29760
   caaaaatatt ttcttgaaaa gacctagtga aaggtgatcc cctaggaaat atcttagatt 29820
   ctaaaacagg tttgagcaac cccattaccc ctaatattat caatatacat tacacaacta 29880
   ggtatgtata tgatgtaggt gctataattc attttcttta tgagaataag gaaactgagg 29940
   tggatggaga taaactcacc ttaatcaaag tcaccaagag agaagccagt gtttgaagaa 30000
   ggcagtctga ctccatagtt tttggagggt gtggtttgca gaagggatgt agaatggtta 30060
   gaaggagcat agtaacttct tccattgtcc aactttgcat tgtttcagtg gcggggtgag 30120
   cacatgttat tcttaaattt tggaagtgtg ctttaaagat aatatttgaa aagaatacat 30180
   gtattcaagt gctggactac ataatttgaa tatatagata catacaagat tgactgagtt 30240
   tctagatgta tagaccagct aggttgaatg cagccattct tgttcagttc cgtgggctgt 30300
   ccagtctcag gcagtcatgc aacctttaag agagtgaaag tagttggcct ctggtcatag 30360
   tgccacttct cccttctgtt ctcactctgt attattagac taatatcccc tgcatggtag 30420
   aatttgtcct ggactgttct ttattagtaa aaaccaaaac aaaccaaaag ctttcttttt 30480
   tacccctgaa ttctaggaac acatatttct ttttggggtg ggggagagag agagacagag 30540
   agaatgtttt agtagcattt aggagttact gggcggcctt tgggaagtgg catccatagg 30600
   agaactgtga gacagtatac aatgtcacga tgataagtac atttcaagaa atcaaatttg 30660
   atcatatttt ggggcagatt aatttataag gaggggatca ttggccagta ctttgtaaac 30720
   aatctggagc tcattttgct caaattaaat attccatagt cattaaatag atattcaagt 30780
   agtgcttagg cagcacagaa tatatgaaaa tctgcagcaa atcatactat ccacataaac 30840
   cattatactg cttctattgc tgatgtggct gccagatgtc tcttttgtga gctggattcc 30900
   atagtgtttt tcatatctct agagttattt gaatagttac ttcttgaata tctagcaact 30960
   gtatgtgcat atgtatgtat gtatatatgt atgcatgtgt atgtatatat gtctatgtgt 31020
   ctatgtgtat gtgtgtatat atacgtggtg aaacttaact ttgcaagtct tttattcatt 31080
   tttgtgagat atggtgaaat gtgatcttgt acatttttat taatgcaggt tgggaaacag 31140
   attctccatt agggtaatag cagagagggt acattttagg tacattttgc aattagacaa 31200
   ttttaacagc tactggtcaa agtattattt aaatttggca tcacacttaa gaaatataaa 31260
   ctgttgtgtc taaattaagc ctagttaaac actgcccttg atacattctt tcttgcttcc 31320
   ttttctttgg agacagggtc ttgctgtgtc acccaggcgg gagtgcagtg ttgtgatcac 31380
   ggctcactgc agccttccac ttcctgggct caagggatcc tcccacgtca ggtgcacact 31440
   tcctggctag ttcttttaat cttttgcaga ggcagggtct cactttgttt cccaggctgt 31500
   tctcaaactc ctgggctcaa gcaatcctcc cacctcagcc tcccaaagta actggactac 31560
   aggcatgagc cacaacggct ggctgtttga tacattcttg gtttgccttg ctatttgtat 31620
   tttcctaaaa gcgcatgctg gggtcacctt tggtgctttc aaatttacaa cctctggacc 31680
   ccattccatg tgattctggt tttattgatc tggtatgtag tgctgaaagc cctccaggag 31740
   cagaggaggt tgggaaccac tgctgcacag gtggaaggag cctggaagtg catctgtgac 31800
   catggactta actctgacaa aaataaggtg gtaaaatgaa aggaatgagt ctcgggagga 31860
   tgtagcaaat gtaattttag cattggaagg gagagcttcg gcgttaggaa aatgcttaca 31920
   aaatggaatg tcagaggaaa gataaataga atcctatggc cagagtcttc caaagtttga 31980
   cagcttaaaa gaatggagag gcttcctcag agtaaaattc tgaccttgca gccattcaag 32040
   tagtattctc cgaagagtgg ttctggtaaa cctgttatgt taggtagttc ataagctttt 32100
   aaaaatcagt gacagctatg agtcaatata aagttggttt aaatgaaatt gtaaataaat 32160
   aattgtacag gtgctgtgtg gtagaaatca ggaaggtggg atgtgaatag tttaagaaat 32220
   gtttgacaaa cagccattaa acacaaagca tcttaaaaaa actataatga cagcacaaag 32280
   agctcctgag ctcagagttt tgaaggagtg gtgcttataa atacacccaa ttaagtccct 32340
   gatgggagga aaaaaatcta gttatttgcc tgagtgtgat cattcagttg tctcagctgt 32400
   tactttctaa aagcagatac ttgtggcctc atggtatgtt cttgttttct cctttttatt 32460
   tttttaaaca ccaagttatt cccaaaccct agaatcggag tcattatcat gatgagcctg 32520
   ccaaaaaaaa aaaaaaaaat ttggattgcc tcaacagaaa agtagtatca cttgttctct 32580
   aactgaattg ttcattttgc agggtcaagt tacctttcca gacagtctga tttgagagat 32640
   ctggtgtgag gccctgagat ctatattttt aagtaagctt catagtatat ctagtgtatg 32700
   ctaaaggctc agaatgttgg ctgagaattt ggagatgatg ttgatgatgt ttcccacact 32760
   ggcaagggca cccatctccc ttccagaagt agcatctaaa caaaattata agcaatttca 32820
   tcacataaaa aatagaataa tataaagaac atcgacatac ctaccaccca gtgtaacaaa 32880
   tagcatatta ctaatatact agttgattcc tgtatacttc tccccagatt gcattacctt 32940
   ctgtttttct agagataatc attcttctca ttttttaaaa caacagattt actgagttat 33000
   attcatattg catacaatta aagtatacaa ttcaatgata ttcacagagt tgtacagcca 33060
   tcaccacaat caattttaga gccttctcat caactcagaa agaaactcag tcaccactag 33120
   aaagcatccc ctattttccc ctaccccacc aaactccaag ccttatactc ctaccagttt 33180
   actttctaac tctatgaatt tgcctgttct gaacatttca tataaatgga atcatacagt 33240
   acttattcct ttgcgatcgc ttctttcact tagcataatg ttttcaaggt tcatccatgt 33300
   tctagcatgg atcagtattt catccctttc tgtggctgaa taatttttgt ttagccattc 33360
   ttcagttgaa ggacatttga attgtttctg ctttttggac attatgaata ctgcttcttt 33420
   cataaacagt catgtacaag tttgtgtgtg aacatacatt ttcaattctc ttgggtactt 33480
   acctagaagt gcaattacca agccatatgg taactcagtt gaggaacggc caaactgttt 33540
   tcaaagaagc cactccctgg gtgttgatac ttcttatttt cacatacttt tttgtacttc 33600
   tccttgtgtt agtatttcta aacagtatgt agaaatgtta tgtattcttt caaacaatgt 33660
   aaggaatttc attttatatt atttataacc tgcaccttac tttgcttatc attgtgtttc 33720
   taagttcatc cctgggtaca cacaaagtgc taatacattc agtttcactg gtggtacaat 33780
   attcagttgt ctgaacacac ctcaacttgt ctattttctg tcttgtctct ttgtccgttt 33840
   tactgtcaga gtaaataata ccatctctgt taatacaaat tcccaaaatc cacaataaaa 33900
   tcttttgtat cattcatgtg aagttcaaag ttaatgcttc tggctgggga actctcctgc 33960
   atgtgctaac tcgggatctc agatttctcc atcttgcatg attgtcattt tctaggtcct 34020
   cccctgagtt tctagtggat catccagctg tctactaagc tagaaggcag ggttgcactg 34080
   gatttattat tattattatt attattatta tttatttttt gctggaaggg agtcgttact 34140
   gttgcccact ttgcattgac caaaacctag ccacaaggcc cccaaattaa ctgcaaggga 34200
   acctggaaaa catagtcttg ctttgtattc aggaagggaa tggggaattt ttagtatccc 34260
   tgccacattg acttgtaagt ttatttcaac tttttgcagc tgtaaacaat gccactaaac 34320
   tttcaatttt cttgttcatg tgggtctcag tttctctaga gtatgtatgt attagtggga 34380
   ttgggtcaca gggtgtgtta atgttgcatt ttattaggta ctttcaagtt tttctcaaag 34440
   gtatttatgc taatttaagc tccaccctgc cgtatgggtg cttcgctttc ttgccaacac 34500
   tgagtatttg ccaaaccttt aaatgtgggg caccagctgt taaaagggag atgacaaatg 34560
   gcatggcatt tgaaatggag gaaactagga tactaaaaat atagaatagt gtatgtggta 34620
   gacagaataa tgactcttca aatatgtcta catctcaatc cccagaacca cagaacacat 34680
   tcctttatat gacaaaaagg atttgcagat atgattaagt taaggatctt gagatgcgga 34740
   gattatctta tattaactga gtaggcccaa catgatcaca cgggagtcca tatgagaagg 34800
   aggcaataag gtcaaatcag aggagatgtg atgacagaag ctgaggttgg agtgatgtgc 34860
   tctgaagatg agggaagagg tcacaagcca aggaatgtgg gtagactgta gaggctgaag 34920
   aaggcaagga aatggattct gtccttgggc cctcagaaag aaccagctct gccaatacat 34980
   tgattttcat gcataagacc cattctgtac ttctgacatc cagaactata aaataataaa 35040
   tttatgttgt cttaagtcat aagtttgtgg tgatttatta tagtagcgat aggaaattaa 35100
   tataatatga ataataatag ctagcattta ttcagtacac agtcatgcta ggttctctta 35160
   tctgtggctc atttaatgga taacacactc cttagtgtca ccattttaga gctgaggatg 35220
   ctgacttaga attggagagg acttagtgaa aagagaaggt agctttgcaa agagctatct 35280
   ctttgcagga caaactcatt ttcgatggga aagcccacac agtgacagag ctctaaaata 35340
   cagtgagtta cagaaataag ggatatagat ataggaggta gggtctttac gttctgtccc 35400
   tggttctggg agggttctca aagaggtgac ttttgacata gggcctgaaa gatacaaatg 35460
   ctctgccttg tgtcttgaaa gaaaaagaaa attcaagatg caggaactgg agcaaagctg 35520
   aggcctggaa agttattttt accacatcat tctctgataa tattataatt aaataccata 35580
   tctactggct gggtacagtg gctcatgcca gtaatcccag cactttggga ggcagaggcg 35640
   ggcaggtcac ttgaggccag gagttcgaga ccagcctggc caacatggtg aaaccccatc 35700
   tctgctaaaa atacaaaata aataaataaa taaataaata aaattagctg ggcatgttgt 35760
   tgcatgcctg tagtcccagc tgcttgggag gctgaggcac aagaattgct tgaacccagg 35820
   aggcagaggt tgcagggggc tgagatcatg ccactgcact ccagcctagg caacagagtg 35880
   aaaccctgtc tcaaaaacag atgtaaaatc ttagctcctc atccatctct ctcccttagc 35940
   tattaaacca ttagtgtttt ctttggtagt gctactacca caactgaata aaaaaggatt 36000
   tgcgtgagaa ctgcacctga ttttgattgt tagactattg aaagagttct gcaaccaaca 36060
   ccactatcta attttagaac attttctata atccccaaaa gaaattctat gcccattatc 36120
   agaaactttc cactctctcc tcccccaggc ccctgcaaaa actaatcttc tttgtgtctc 36180
   catggatttg cttatttggg atacttctta tatgtgtaat ttatatctaa tataaatgga 36240
   gtatgtggtc ttttgtgact ggcttctttc acatagcaca atactttgaa agtccatcca 36300
   tttgcagcat gtatcagtat tttgttcctt tttatggtgg aacaatattc cattgtgtga 36360
   atataccaca tcttgtttat ccattcatca ttgatgaaca tgtgagttgt tttcactttt 36420
   tggcttttat gaataatgct tattctatga ccgctttgta taagtttttg tgtgaacgta 36480
   agttttcagt tttcttgagt atataccttt aagtggaatt gctatgtcgt aaggtagttt 36540
   tatgtttaat tttttgagga actgtcctgt tttctaaagt gactgtactt tagactctca 36600
   ttttacattc acctgagagt tttgatttct ccacatcctt gtcaacactt gttattatct 36660
   gactattcga ttctagccat cctagtgagt gtgaagtggt atctcattgt ggttttgatt 36720
   tgcatttcac tgatgactaa tgatgcgaaa catcttttta tgtgcttatt ggccatttgt 36780
   atttttttgg gaaatatgtc tattcacatt ctgtgtccat ttttaaattg agttaattgt 36840
   gttttattgt tgagttgtaa gagtttttta tattctggat actagaccat tatcagatat 36900
   aatattagca ttattttatc ccattttgtg ggtcaccttt tcacactgtt ttttgattgc 36960
   tgtttttgtt tgtttgttgt tttcttttaa tagagacaag gtcgcactgt gttgcccagg 37020
   ctggtcctaa actcctggcc tcaagcaatc cttccacctc agcctcccaa agtgctggga 37080
   ttacagacat gagccatcac acctggccca cactctggat agtgtccttt gcatgaaagt 37140
   tttaaatttt gatgaagcca aagttatcat ttttcttcgg ttgcttgcgc tttaggtgtt 37200
   ataactgagg aaccactgcc aaatttgatg tcatgcagat ttacccttat gttttcttct 37260
   aagagttata tagctttagc ttttacattt aggtctttga tctattttta gttaaatttt 37320
   gtatattgta tgaggtggta tggtcaaatt tattcttttg tatgtggata tttaattgtc 37380
   tcagcaccat ttgttgaaaa tatgattttt tcccatgtat tgtcctggca cctttgtcat 37440
   aaatcaactg accatagatg tatgaatttt tcttgaactc ttagttttat tccattgatg 37500
   tatatgtcta tcctctgtgc ctgatttgaa agattacttg aagtggttgc ttgatgcatc 37560
   tacatggctg atcttggaga gtcagtaagt ttgaaaagac acttgaaaat ctgtatttta 37620
   tcataagcac taccatgtga ctctggggtc aatgttgaga aactatgaca gcacctctca 37680
   gacttcaagg aacatacaga tcactagagc gcttgttaaa atgtaaattc tgatagagca 37740
   agtctgaatt agggcctgag attctacatt tctaacaagt tctcaggtaa tgatgatgat 37800
   ccacagacta cactgttggt ggcaaagggc tagaaggtct cccaccacag catgatccca 37860
   tgccttccag gtaggcctct ggaggaatca ctaggcttaa atgaatattt gaattttctt 37920
   tcccatgtta attgttgcct ttatttactt ttatgccact gagaaaacca acttctccag 37980
   ctcatttatg gtgccttaca aacctgattt gaggggatgg gtttttgaaa aggctttagg 38040
   attttatttc tggatttaca gaaaaagaca tggagttagg agtgcttgat ggaaaagtga 38100
   atttaatgga ttctgagtaa ttttcctact tatttaaatt ctacttgttt ccagaagagg 38160
   tgtattagca ggttattttt ctcttacaat gatactttaa tgcagatata taattgcatc 38220
   atgtttgcct aaaaattggt ttagataaat cacaagtgcc aggttacatc agcttttcca 38280
   acatccactt gatctacatt tatttcaaag gtttgtttag aaggctgaca tgttcttaga 38340
   ctcttctttt tctgctgttt gctttcagca agtcattcca attatttgca tataaaatat 38400
   ctccattttc acatagggac ccttatttgt ccctatattc tctcaggaaa actgctattt 38460
   ctcaagtact ttggtagagg attgcccttt atagttcgca aatgtgtgct tggatgtgcc 38520
   cttgcttatc cttccatgct ttctgggctt ggcgtagtaa gtggtcattg attttctgtt 38580
   caaggggttt tgtagtttca ggcaactgca gctgttaatt ctggatgtca atttctagta 38640
   aacctacatt tataaaatga gctactcttg tgcagtgttt gctttcataa cactagccgt 38700
   gttctgagtt acaagcactt gttttttttc ttgtttttgt ccttaattgt tttctaaaca 38760
   tttccggaag cttgggtgtt cttcaaataa tagtgtcttc aggttagttg tgattttctg 38820
   acttgaaata tttttagtgc agcaccttag tggctctgta cttagctagt atttgaagca 38880
   tggtttggag cttttttttt ctttaatttg tactaaatag tgtttttgct attagtaatt 38940
   catatatata tatatatata tatacacata catatacaat agtacagaag ggaataaaat 39000
   gaaaattaaa ccccgtttct agttctattc cttctgttgg ttgccactta gctttaacca 39060
   atattttggc atctctgttt attgaccgat gcagtctctt cttcaaccac tcctaaaaat 39120
   gggggatgca gaacaatcac atctcctaac tttcccaccc ctgtccaatg cccagttttt 39180
   ctctacttcc tgagattata tttttcttcc ttgtgatttt aaagtatgtg cttacgttct 39240
   ttttcaaaga ctttcagaat tattttgaat gggaaacatt catattcaaa acaataaaaa 39300
   atcaaaacac aaaaatgtat gcagcaagtc tctttacctc tctatgtaag aagttaatgc 39360
   ctccaacaga taatcaatgg taattatttc tggggttccc tttaagatgc tttcaatgca 39420
   taggaaagca gatgtgtgcg cacactactt cccttctccc atatttataa aatgtctgcg 39480
   tttatcattg ttcatatgcc cttccaatca tcttgccgtc actgtctctt cttccaatca 39540
   ctgcaatgac cactagccca ttaagacctc cagccattgt ctcgtgagta caacctagct 39600
   aacctcacat gatgcctaag ccatatgccc tttgcctcct gacccagatt gtccttgttt 39660
   ctagccaaag catgggactc catgagatcc cacttattgc ccacaaatgc acaacccaga 39720
   agtatgggaa gttcatgccc tatcgggcta atctgtgacc caagggaaac aaaagccggt 39780
   taaaaaaatt cttgctcttt tacttcccca gactgactgt cctaagaagc attttttatg 39840
   gattctcaga agatggttct ttgtgatcaa gcaaaatcag ccaaccttac tggtggccag 39900
   caccataaag catttttgta ttggctctgt ttgcttcctt gttttagtcc ccatgtctct 39960
   cactcttgct tcctgaaact gtactcccag ataaagtagc aatacatgaa gcgccatgcc 40020
   cttttcctag ttacttttat atgtggaatt aattttaatg tatctcaagc tatttgggat 40080
   tgagataatt taactcatgc tggcttttct tactagaatg aattaataaa tttttatata 40140
   ctgaaatgtt gaattacaaa tcttgccaat atcacattta attaaatgaa tttgaaatca 40200
   tgatggagat ggagatagaa agaaaatttt agtttttctt cgtgacagaa atagtttacc 40260
   tggtaatatt atatattcta taaatataaa agtctattaa agtctattaa aagctccata 40320
   tgaacagaag atttatatgt ataagtgtat gtgtgtgtgt atatctataa tataatgtat 40380
   atatatgttt atatatatat acaagtctca cagttccata agaacagaat atttctaagc 40440
   aaagcaaatt tctagacaaa cttcttagat agtttattag tcacttggga aaaaaaatct 40500
   ataaaacagt ttcataagaa ccagatttaa atatatttat atctatataa atattttgac 40560
   attgaaggaa ttagctgtgt gtgctatgcc taatattttc ccccatggct agtaaatatt 40620
   tgtagatagt gtcccttttc tccttgtagg ttacagtgaa ggggaagagt gacccactaa 40680
   attaccccag ccatgacctg aaaaagttac atgggaggtt ttcatcattt gttttctcat 40740
   agggacaaca gtaatattga agtaggaaac ctctatgtct tctattgttt actgattcca 40800
   tatcttactg taaaataaaa gcatgtgaga gtgaactgga gatctagata tcccctttaa 40860
   cttgcctgaa ctgttgttag aagacttttc aaactctcct gcagtctttg aattttcaga 40920
   agatgccttt atatttattt ctttcactag ttagctctcg gtggtgtctg tggcatctta 40980
   gctctcaatg gtgtctgtcc gtctggagct gggaatgtga tggagaatct ctaactcctt 41040
   agttggggga gatttgtaga tgagagaggc cagttagtca tgtttgtgat gttgggttaa 41100
   tctctttagg aaaagagcag gctggcagca ggttgatgtt tgcaactttg atttacttct 41160
   tggaaagtag attgtggagg taacaaaggt tgttaggcca taatcagtca tttcgtcagc 41220
   catggagaag ttggaagtaa atctaccaaa acagtcacaa tgaactgcat tattgtaaat 41280
   tcaaacagta gtaccagtgt tacacattaa tttttctact tactggtaat atttatcatt 41340
   ctgtcaaaac ttgtcctgct gtaagaatat tgagaactaa aaagtctcta agtacattct 41400
   tcttgtcatc ccacaggcat gaagttcaaa tagatgtcaa atgccacaat aaatggtcat 41460
   aaatttaaaa gattttttga tagtggttgg ctaataaagt caaagtttaa caaacaaaag 41520
   tgacctttat gcttgaagct aaatgagaat atcaaatcat ttggttggaa aaacaggctg 41580
   tgatagaaat ttcaatagaa gaaacagata aaatgagtta tctctatgaa aatgaagctt 41640
   agaatcctca aagcgtcatg aatctcaaag ttagagaaga aggtgaagat cgttgttcta 41700
   gtgctcagct aatatcattc aaccttggct aagaagccta aaggattagc gaagacagtt 41760
   ctattttatt aggaagggtt tttatagatt gtaataaaac ttgtcttctt ataaattcta 41820
   cacttgaatc ttttagaaaa ttatgatgaa gaagactaca ctatctttca tttgtcaact 41880
   atacagatat ttctttttct ttctttcatt tttttttttt tttttttttt tttttggaga 41940
   cagagtctca ctctattaca ggctggagtg cagtggtgtg gtcttggctc acaacaactt 42000
   ccacttcctg ggctcaagca atcctcccac ctcagcctcc agagtagctg tgactatagg 42060
   cgcacaccat cacactcagc taatgtaaac attttttttt gtaacagata tttctttatg 42120
   ggtgtcatgc tgctctctca tttctttatt ctcttccttt cacaaaagtc tatattctag 42180
   gccaaatatt cccaaaacat tcaattatac ctcatgttca gtggctgcag aaaggcacat 42240
   tatcattaga gtagaatttt agattataaa tctctgtctc acactgtggt atccagaact 42300
   gaatataaga cccatagaaa tcttcccagg acagaagtga atggaaatat cactgatatt 42360
   caaccacaca cacattctta ttaatggagc tataatcata ccatagactt gttgcatgtt 42420
   ccagttaaaa ctcttctggt ccttttctta catgttgttg agaagtagct ctatcctttt 42480
   catgtacatt tcattttagg gacataagtt caataaaaga taagcaaaca aataacaaca 42540
   caaaaacaac ccaaagatac gattgtcacc tattaagtgg acagcagtga aaaaagttgt 42600
   tgggtaagca tacattacag tgggcctctt ttgccctatg gatgactttg taaagtgttt 42660
   ggaaggccat tgtgcaatat gtattacagt ttaaaattgc acacctttta ttcaagaatc 42720
   atacttttta gaatgtattt tttctaaaag tggctgagat agatatctat aaaggatgac 42780
   ttcactactt atctagtttt tattccatgg atcatcagta taatcatggt cttgcaaata 42840
   ctttttaatt ctttcctttc ccttctttgc atttatgcaa agctctccct agatttatga 42900
   ttctcaaacg tttaagatcc atcagaatca cctgaatggt gaatcttgtt aaaaaatatt 42960
   gctgtgtcct atacccaggt attctgatat tagtagatct ggggtggggc ccaagaatta 43020
   ccatttttag taagttacca gctgaggctg atgctgctgg tccagtgact gtgctttgag 43080
   aaccatcatc cttgatgaac ccagccactc atgattgttg cttgcaagag tcacttggca 43140
   gattgatctc actacaaatt cacgagcccc aaatttaaat gtacataaaa cactacctgg 43200
   aaattctagg aagctacttt ttccattgtc tgcaaataat tttatatctt gcctttcctc 43260
   ttcaaactta cctgttcttc tcacatgcac taactgcaca ggctttaagc tgatgacttg 43320
   cctcatgctt ttttgagaaa acagaaacca ccagagggaa gattcccgac atcttcttat 43380
   cacaaatctg taaatcagct ggtgtctgaa tcctgttttc tccttctgcc ttttctttcc 43440
   tggagaaagt caaaggccaa ccctgtactt tgaatcctgt acagtctaac ctctacattc 43500
   agtagtttgc atgtttattc ccctctcacc atactgaatt attcccatta atacacaaat 43560
   ttgctctagg atttcccatc tttacattac acacgtagca atagctatga accatttttt 43620
   ttaccccata actcatacag ttactctact ttttctgcaa ccttgcaaag tcaaacttct 43680
   cctaagaatt gtcttcctat attttctatt ccctaataac tggtaaaccc attccaatct 43740
   tgttttggcc cccacattgc cactgaagta gattttcctt tggactccca acccctcagt 43800
   ctaacagagt tcttagtcct cctttgtgta acatatgcca cagctaatag ttctactggc 43860
   ttcttttcca gaacagcctt ctctaaatgg tctctacagg ttggggtact tctcttctct 43920
   caacctactt ttcccatgta gtcttattca tctctctctg gatgatatag aaccattcat 43980
   atactgatga atcctttatt ttatatctag cacaaacctt tatgctgaga tccagaccct 44040
   aatatacaca ggtgcctaat tggcatctcc acttggttgt ccaaaaggca acccaaaact 44100
   gacatatcca aagtggaact ctaaattttc tcataagtca gttcatctcc tctccttctc 44160
   ccattttatt ttattttatt ttttcttttt tttttcttgt gctcttattt ttatatacga 44220
   atatttttta ttatacttta agttctaggg tacatgtgca caacgggcaa gtttgttacc 44280
   tatgcataca tgtgccgtgt tggtgtgctg cacccattaa ctcgtcattt acattaggta 44340
   tgtctcctaa tgctatccct ccgccctccc cccaccccac aacaggcccc agtgtgtgat 44400
   gttccccttc ctgtgtccaa gtgtcctcat tgttcaattc ccgcctatga atgaggacat 44460
   gtggtgtttg gttttttgtc cttgcgatag tttgctgaga atgatggttt ccagcttcat 44520
   ccatgtccct acaaaggaca tgaactcatc attttttatg gcattctact catctgacaa 44580
   ggggctaata tccacaatct acaaagaaca caaacaaatt tacaagaaaa aaacaaacaa 44640
   ccccatcaaa aagtgggtga aggatatgaa cagacacttc tcaaaagaag acatttatgc 44700
   agccaacaga cacatgaaaa aatgctcatc atcactggcc atcagagaaa cgcaaatcaa 44760
   aaccacaatg agataccatc tcacaccagt tagaatggtg atcattaaaa agtcaggaaa 44820
   caacaggtgc tggagaagat gtggagaaat aggaacactt ttacactgtt ggtgggactg 44880
   taaactagtt caaccattgt ggaagacagt gtggcgattc ctcagggatc tagaactaga 44940
   aataccattt gacccagcca tcccattact gggtatatgc ccaaaggaat ataaatcatg 45000
   ctccttctct gattttagaa aaaggtgcta cctagttgct aacagtctgg aaggagtcat 45060
   ttgacaattc cccatctcat ctgctatatc gaatctatca ccaaattttg actgtagtct 45120
   ttgatcttcc acacatttcc atttccactg ccccaactct agaccaagcc ccaccactgt 45180
   cacttgcctg tttcctgtga taaccccaac aactttcctt gctggccttt cttcaatcct 45240
   tttttcctgc agcagtaaga gtgatcgttc aaaaatgcaa atctgatggt caccttcact 45300
   tagtaatttc ctactacatt tggaataaaa tttaaacttt tcatttagca tcttacaaga 45360
   tctattaatc ttccacctct ccagcctagc cttgggctac atgctatata caatagccat 45420
   gtcccatgtc ttccaatttc ctggaagcaa agtctctctt ggggccattt gtgtctgttg 45480
   ttccctttgc tttgatgcct cccatctctt tgcagcctgg ctttttttca tcttttagtt 45540
   cccaaattat cagttacatc ctcagaggga ccttcctacc catccaatag aagcctcttc 45600
   tgttgttccc tctgttcttc tccctcatat tgcaaattat tattgaaatg atatgttcat 45660
   ttttgctttt ttgcttatct gtctcttcta gtagatctaa gagttcatta actgtctgag 45720
   gttagagatc ttagctgttt tgttaatgtc taaattccca gtctggctca ttgtatggca 45780
   aatggtattt gttgggggac tgaataatat ttattagagc attcctgtta acagtgtaaa 45840
   atcaggctat agtgtaaagg tccatcaatt taatgattaa gttataggac gatccattgt 45900
   aaagaaaaaa gtaaatttat atatgctgat atggacatat atttaataaa tattaagtgg 45960
   aaaaaagcaa attgctaaac ctcatataga ataataccat ttttcttaag tatatttcca 46020
   tatttatatc tgcatgtttc tttctctctc ttcctctctc cctctgtgag tttccaggat 46080
   ttctactggg actttttcca ggtaatggca atacagagtg acaagaattt attttatttt 46140
   actctttctg aactattgga tttttaaatt tttataagtt ccatgtattc cttttataac 46200
   atcctccttc aaaagcatga agaagattta aagtgtaata caaaacatta cgtatgttca 46260
   ttcactccac aaattttatt caacatctac tatataccat gtactgctag aagctgcaca 46320
   tatagtgaga tccatttatt gtatataatt gggtgtcaac aaaagataat tttatgaggg 46380
   tttgtttctg gcagtggtga gattatggtt ggatttcatt gcaccttctc attttcaaat 46440
   ttttgatggt aaacataact ttgaaattac aaaagttatt tttaaagaca tttaaatatt 46500
   tacatggaaa aatgctcaat cattttagaa caatttactt ggacaggtga atacttttca 46560
   atttaatgga aaaataattg tataagtgcc ttttgtatag atgcaattat taaaatatgt 46620
   gtagcacata tagcttagaa agtttataaa taggtagttg tttccagtag gaaacaggat 46680
   actcaatctt tcttttcttt gcctatagac tgatttcttc aacatggcct actaatgtgt 46740
   tcctcttgga aaatgagtac ctaggagaga ttcaaggttc ttgttaatga gttaatattt 46800
   gtaacatatg cctgagggta tttatggaaa ccatgtattg gatccagaag ggcaaaactc 46860
   tccgggtggt ggaagctgag ttgtaagcag tgtgtgttga gaggaggcct aggggataca 46920
   gactgtcata gcttcatgag aaaaaaaaat taaaatgtgc tgtaggaaag ggaatgagag 46980
   tgagaagaaa agccatccag gggatctcat atactgaaga tatggttttt ctttcgtcga 47040
   atgtatgata cttggaggaa caaagaaaga taatatcaga tttgagaact gttgccttaa 47100
   aaaaaagata aaatgctgac tttctgcaaa tatctttatg accaactttc caaaaacagg 47160
   gaaactcaat gtgcctatgt tttttcctct ggtttcttaa ttccttgatg ttctcatttc 47220
   cagtgacctt tttagaccct cccaaggtca catgttggat gctattgtaa ccagaagtac 47280
   caccatgtcc aacatcacag tgcatatgtc ttgctctcta actaaatgtt ctaactcatc 47340
   tgcttatttt ctcctataag cagcatttct ccttcatcaa gaccaccagt ctgctaaccc 47400
   acattttctc tctattcttt agccttgtcc tattagtctt tattgcttat gcagagagat 47460
   tcaatgattt atatccactt tccagctcct ccatcaattc ttgccttttt tgcttgtttt 47520
   aaatttttta aatttttctt taagttctga gctacatgtg cagaatgtgc aggtttgtta 47580
   cataggtata tatgtgccat agtggtttgc tgtacctatc aacctgtcat ctaggtttta 47640
   agccccacat gcattaggta tctgttctaa tgctctccct ccccttgccc ccacccccca 47700
   acatgaccca gtgtgtgttg ttcccctccc tatgtccatg tgttctcatt gttcaactcc 47760
   cacttatgaa tgagaacatg cggtgtttgc tcttctgttc ctgtgttagt tcgctgagaa 47820
   tgatggcttc cagcttcatc catgtccctg caaaggacat gatcttattc atttttatgg 47880
   ctgcatagta ttccatggtg tatatgtgcc acattttctt tatccagtct atctttgatg 47940
   ggcatctgga ttggttccaa gtctttgcta ttgtaaatag tgctgcagta aacatatgtg 48000
   tgcatgtgtc tttatagtaa aatgatttat aatcctttgg gtatatactc agtaatggga 48060
   tttctgggtc aaatggtatt tccggttcta gatctttgag gaattgccac actgtcttcc 48120
   acaatggttg gactaattta cactcatcaa cagtgtaaaa gtgttcctat ttctccataa 48180
   cctcaccaac atctattgtt tcctgacttt ttaataatca tcattctgac tggcatgaga 48240
   tggcatctta ttatgatttt gatttgcatt tatctaatga tcagtgatga tgagtttttt 48300
   tttcatatgt ttattggcca cataaatgtt ttcttttgag acaggtctgt tcaaacaacc 48360
   ccatcaaaaa gtgggcaaag gatatgaatt cttgtctttt tctccctctg gaacttgttt 48420
   cacaattcta gaacactctg agtcagctct ctttccacct taactgacca acctataggt 48480
   aacctattgg tataggtagt ctgcccagta ggctactttg taacagcata cattgatact 48540
   atttacaaaa ttttatcttt ttaaaacatg tatgtgttta ccaatttgtt ttatacttct 48600
   gctatgaaac agtgagattc ataaagtcaa taatatagtt catatctcca ggaagttagc 48660
   acagtatctt ttgcagcgtg gatgcttaaa tgatgggtga ataaatggat aaagtcagct 48720
   ccaaagaaag aactgagagg tagagggagt gaccagtgta ttgatgtgat acataaactc 48780
   ttctgcccac atgggaaaaa aatggcatcc tggttaggct gtgcaattga tgagtcgcta 48840
   tcagttatta gaaaaaaacc tttcccacct aagttttcaa agtattctgc tgaggcagta 48900
   gagcgaactt gacttagtcc cttttctcta aacgagcatc atctcagttg cctgcctgtc 48960
   tgcctgcctg ttcgctttct gccagaacgt ttgattgatt gagtcatatc cttgcttcat 49020
   tcctagaagg attccaggtg ccaacttcca cttgatccga ggttaaatat atctcctgta 49080
   acctaggaat ctctcagtct tgctttctgc tgctggagaa tactaccata acatgtagta 49140
   aaatataaat ggaatatcag tcatctctga aatggtaata tattctccag ctttctattc 49200
   aaatattttt ctttgagctt ctttctttgg agtctgttgt tagatattct caaaaggatt 49260
   gtctccactt aagtttcttg aaaattaaat tgtagatgtg ttcagagatg tgtctgccat 49320
   aagttactgt cccacttaaa aagttcctgg caatttttgg atatttcctc atgtgaaaat 49380
   aactcctgat actgtcatgg actggggagt ataattcctg caaacagcac ttctgaaaag 49440
   cacttggctc ttggctctgt tgtagaacga ttatgacttc ttgacataaa atgtgttttt 49500
   aatctagagg ccagggccag agatttgctc accatgatta ccaaacttct cccgcaaccc 49560
   ttagacatgg tatcttagat tgtatgactt tctgcttctg tttttcttta gtgagagatg 49620
   gttacttgga atgatccttt aaatattcaa cacatcttca ttgatttccc ctgctaatgt 49680
   gaagatgtta aaaacatttt cttacattgt tagctcattt atttatttat ctgcctaata 49740
   aaaaagtata gcatgcactg catgcagggg ggatcaaaat agtcattgag tcacccctca 49800
   aggtgcttac agtctggtag gggagaaagg caggcaataa ataagaacaa tgatatggca 49860
   gaaattctca gatcaggagg gaagagggtg ttctatctgt gaaggaggta caggcagaag 49920
   aaattccata aatgaggttt cacatggatt tagttctcaa agacaaagtc ttgcaaagac 49980
   ttgggaggaa gccatttcag ctggagtcag cagtttgaat gaagccctgg aggtgtgacc 50040
   caatcaacag ggaggtgtcc ctgggactgt tgcctctatg aggctcatgg gtcacccacc 50100
   ggatgccttt ttaaaggagt ccttctttgc tttatcacca gcccattggc cttagctata 50160
   aagcagattt atgcaaagta attataatag agcagtttgg tttgagctat attttctaaa 50220
   aggattggtg aatcaccagt gcctgaatta tttactgctc tcattgagag tcaggtccaa 50280
   atccagcctg atgcacaggt gggaaaagaa ggactcagtg gtcagagaaa agtcatcaga 50340
   gagatgaagg ggccagcact tggaagtctt atataggtca agcagtaaag acattgtcgg 50400
   gtgaggggtg gatgtggcac acggcacaca cctgccctgt tggcacttgt cagaaaggca 50460
   ggaatcaaag ttgtgcccaa ggtcctgctt tgggtgactg ggtgagtggt gggtcttttc 50520
   ctacaacttc aaatggaaga gaaggtggaa catatttgtg aggacagaca aggagttcaa 50580
   ttgtagacag acatgtggac tgtgaggtgc ccaaagacac cctgctaggt ctttccagga 50640
   agcagggatt ttggagtgta tgcccagggc ttaaaggtga tatctagcct aaaacttaac 50700
   atttctaagt cgtctacatg tgagttgtga ctaaaggtgg tgaatgaata tgctacacaa 50760
   tgagaagaga aaggtgagag aaatgacgct gaatattgga ctttactgcc atgtaaggag 50820
   aaggcaaatg aaacagggac tgaaagctga tgtctatgaa gtaggagcag aaacaggaga 50880
   aggaaggtca tggaagccaa ggaatgtaac ccattcccaa cacccattcc ccgtgatgtg 50940
   gagaggccac ggaagatgag gaccagatct cattacaaga gtgtatggta cagagtgaga 51000
   tatctccaca aatgcctagt acctttcccc tttcatcact ttttattcaa gatcaaagta 51060
   taaaataacc aaattcaact gggtgcagtg gctcacacct gccatcccag cactttggga 51120
   ggccgagatg ggaagatcac ttgaggccag aagtttgata ccaacctagg aaacctgaag 51180
   ataccctgtc tacaaaaaat aaaaaattag gtgttgtgtt gtacacctgt agtcccagct 51240
   actaaggagg gtgaggtggg aggatcactt gagcccagga gttggatgct gcagtgagct 51300
   atgaccatgc cactgtacta cagcctggtc aacagagtta gaccctgtct ctaaaataat 51360
   aaaaatcaaa ttcaactctt tataagttgg ctttttttaa gagatcattc tacctttgtg 51420
   taatgtcccc agcccaaact ttctatcctc gcattcccct gggccttgga atggttttcc 51480
   ctctcccctc ttttcctctt ttcatgtgtt atctcaaaat ttaacttttc tggggcccct 51540
   ttatactgtt tgctctctga caagcaaaca aatctgatta atttgtttac ataagatgct 51600
   ataccttaat tatggcatag tgtgtcttca gcaccaataa ggtttgtatt gtaaccttga 51660
   ttctgaaagt cttgagataa agcagtagat tgcaggaact tcaaacacaa tagcaggacc 51720
   cgtagaggaa gttttagggg tacagcttcc tttgcctctg cataaagcag gctctttggg 51780
   ctgtcatgga aaagctgttt gggaaatggg gtagatgatg ctggagaatg aagctcctta 51840
   aactaaattc tgccagctcc ctcctcaatg tcttcaagca tttgctcatt catttacttg 51900
   cagcttgggc gtaaaaaggc ccctcctttc ctgacctgca tcagcatctt tttgcaatcc 51960
   cctgtggtta gaattagggg aaaatgagcc acttcatttt gttggtatca ttcctggggc 52020
   aatttttgac tgatgccaac ccatatggtg atcaagagtg actttgtaac catcactgga 52080
   ggttaaggga gaagggatgc tttatttcag cttctcctag aatgtcagca agaaattcag 52140
   gagacagttt gtctgccccc aaactcctgg tctttcccat cttccccaga ccatcatatg 52200
   tggaaccagg gaaaaggatc caggtctgga accagcaatg tccattaagt gttgtggacc 52260
   cttcctggtc tgaaataggt ctagcttgta gtgctcttga ggtcgcctct aatactgact 52320
   cttaataatg gaaaaagtaa acactgtata caagcaatgt ttttagaaaa attagaaaag 52380
   aatggcttaa tggaaacttt ttttttcatt gaaataattg agagtttaag tgagttttta 52440
   aagttaaagt ataaatgctg acaccttgaa gagccgagcc tagtttttca ctttgaattg 52500
   cgagggcatc tggtgtgtgt tctgaaaggg agagcagttt gatagccatc tgtccccaag 52560
   aaaaattcag atttggaagt aagatgaaac aagaatgaca cctgcttaag ccaagagttg 52620
   tgaataagac ataacttctg ctacatacag tattttttaa agaagaaaga aaacccacca 52680
   attagagatc ttacagaaat gttcagatgt acttgagtca ccaagggctt gcctttctgg 52740
   cagccagtac aaacttctac aaaggattga tttaccccaa atggcatact gagaacttcc 52800
   catagagctc agctaggaac cctcttcaca ccttccagtg gagggaggga ctcccatatt 52860
   ccaatagttg tatctttgca agtaaaccca gacatctttg tggatagtga ctggtcttta 52920
   ttcacctgca tagccttagt gtgtttgatg gttgttgaaa gctctaagac ctgaaattca 52980
   ccaacatgtc tgtgttccca gctgcttggc tcaaggtaag ttttaccccc ctgggtttaa 53040
   atactagtct ctctgcttct tggaggtggg ggtaaaactc taggattaag ctgaatcttg 53100
   gaaccccttt gttatccaag acaagaagaa taggaagagg gtctttctgt gaccaataga 53160
   gttatagata aaattgtgtc cacatatcca gtttaaacaa acatgaaagt acaacagtgg 53220
   cttggatatg accagaaaat acaaaaagac aaggcactgt atgtgatgct gttttttgca 53280
   aggtgataac caggtgtatc tctgctcttc atctgccaaa caataccttt tatagaagag 53340
   attttacctt ccagttctaa cactgtaggg cttctctcct cttttcatca tattccctct 53400
   ttggaattta atacagttga agagggttag ctgtttagaa tctgggtggt aacggggatg 53460
   gatttcatcc tgacctcaat ttaaagctta tctggatctg taatgacttg agtatgtgcc 53520
   ctgacaagtc tttggaaacc tgtcagaatg aggtatcaat aaagagacca ggcatctagt 53580
   gtgaccaaac actcatcaat gataggttct tgccttgtta ctggagatac atttgctgtg 53640
   cttccctggt tgagatgtct gtgtatttct tagtttaatg tgtctaggat tttctgcaca 53700
   ttacattgaa gctgttacta tagtcatcta tgaccatcat tggtataaaa tcacattgaa 53760
   caaagtcaat gctttcttta atttgtttct gtttagagaa aacaaagaaa atattgtggc 53820
   tttttccccc tatcaacagc actagctttt gtttattcaa ttgtatatgg gtactcacac 53880
   ataccccccc caacacacac agacacatgg gctggagaca gctaattttt agctattatt 53940
   aaaattaaat tatgtaaaca tacaattaaa taagttttgt tataaaacaa agaaataaac 54000
   actcaaaagt caccacttcc tcattatttt actgcatttt actattatct gtgattgtgt 54060
   ggttatttat atctattgca cctgtatgag ggaaacactg taccatggtg tctactgcat 54120
   atctattccc aaatctgcac ttagcagtat caagaatttt tttttggaaa gccagtagtt 54180
   aaacatttac cagcacacca ctgtaaatgt atacacatac acacacacac acacacacac 54240
   acacacacac acacatcctt ttcatagcca tgcagagtaa gcgtcagttt ttctatttta 54300
   tagatgaaaa actgaggctt agagagttta acttacccaa gagaccactc ccatgcttta 54360
   gctctcctac tctaaaaata taaaataaga agcccaggga atgcttaagg atgactgaag 54420
   ctgtattggg atataattta aaagcagagg tttgctagaa gtgagagcaa gagaaccatg 54480
   tggaaatcta gacaagcact ccaggcacag ggtgccacca gtgcaaagac ctggaggtgg 54540
   gattgttttg ctgcttctga ggatagtaag ggtactactg gagctggaat agaaagaggg 54600
   agagggacaa taggaagaag tgagacaagt agggcatgat cactaactac ctttatgaaa 54660
   ccaggcaagt aacttgaccc ctctatgctt tatttctctt atctgcagaa tgtttcactt 54720
   catgctcatg gtaagagtat atgtgaattt actgtgcttt agttaaatta ttgttactaa 54780
   agctaaacca tcactgttgc cttactagtg cttaaatata attttaagga agaataattt 54840
   tttcttgcca tcttcagcag ctgcatgagg agtgattcag aaatacttaa atttatccct 54900
   tgcaagtacc tattagccta ttttaaatct atttaaaatc cacagtgcaa tgcagtttaa 54960
   attcatctgg gattaatagg acttgccttt agctatatta ctgatggcat gatagaatct 55020
   caacaggaaa cttttgtgac atttttcaaa tgtccagtct gatggattat acaagactaa 55080
   aaaagtattg aaacacattc tgtattttga aaacccaaga gttcgatttt aacagtcttc 55140
   acttatccat aaaacagatt accatgtcct cttatccaca tatcaattat ataggtgtat 55200
   cttagagtat atttaaataa cggtatgaca taaaatcatg agcttctgtg gctttgataa 55260
   ctttcagttt cttcttaaat gatgtgttca ctttatcaaa attgatcagg ctgtacactt 55320
   aggttttatg aacttttctg tacatgtgct atactgcatt tttaataaat tttttcttaa 55380
   aagagtgagt agggctttga gaaaggggaa agtgtttgct ctcctggatc aaggaagcaa 55440
   ggtacaagat gagatgaggc cagtgttaga atgaacatgg catgtgtgtg tcacagccag 55500
   gacacaaatc atccacgaca tgtaaatgtt tgttgagcac ttcctggggg ccagtatgga 55560
   agatactgtg aactgggtac cctaaaatca ttcacagcct acttcttcct tgaaagctca 55620
   aatactcaac ttcccagcct cccttccagc tgggcatggg aatgtgaagt tgttgaatgt 55680
   gcttctggaa aagcctctta aaaagggaca ctcaatgaat atacccctta attatttcat 55740
   cttttgcttt ttgctgtctt catcctgctg gaatcaggct gtgattcccg gaagcaggtc 55800
   agccgtcttt caaccatgac atgacaagca tcaggacaaa ggcttatgta ctaagaatgg 55860
   aagatggaca gatgggaagc gtgaagcccc ctagagcagt gcctctgatt ggttgggtgt 55920
   ctgactgata ggcaggtggg gagggtcaag gatatgtggg tcagtctatt ctaggacact 55980
   acttttctga ccttgaggct gcccaaaaaa ttgccctttg ggaattttga catcatctac 56040
   agacatgcca ctttaacaat tatttgtttg ttttgacata ctggacatat tcttgcaagg 56100
   gacaacacat gatgaaatta atcaaagttt tgttggttgc caactttttc aaagttacta 56160
   gcagaaggtc ttcacaaata caaatacttt cctctcctat gccttagaca tttttttccc 56220
   ctctaagatg gtaatcttat gtcatcctca tctttgagca attctctgta aaatgaaacc 56280
   tttgcaacat ttccatatct agagatacca ctttatcttc acttggagac ttgataattt 56340
   tccaaggaga tacgctcaga gggaaataca aagagtggaa aagaatttgt taacttgaaa 56400
   agttggcaaa acagatgtgc tgtgattctt tcagttgagc tagcacattt catcatagcc 56460
   attttgaaat ataacattaa aattctgtgc tcaatgtccc ttatcaccaa accttttgtt 56520
   tctgtttgca ttttgttgtt gtaatcatac acgtcataaa tgagtatact aaggcaagtg 56580
   ataatgtgta taataaaatg tagtcaatac taatttgcca cctttgggga gaaaaatgac 56640
   ttccatctgc atgataacta tggaataaat attctacatc cttacctaaa ataatgtatt 56700
   aatgtcaaga attcttacga tgagcatagg ctatagacca gaaaaatagc caggggttat 56760
   gatatttgat taaagtttta acttgataca agttaaaact tgtatcttgg cttttaaaag 56820
   ccaaaagata cactgaaaat tgaattgaat ctagtataat atgacggctt actgaaatta 56880
   ttggaaagga ggtactatat taaaataaag gaaatgtctt cctttgagac taaactgcca 56940
   ttttgttcaa tttgatacac actttatcca cctatttctt tatttgcctg caaagagtaa 57000
   gagcttaggt aaggcagact gcgaaattca aagcaacagt gtggctacat gtgttgtttt 57060
   ttttttcttt ttttacttct tctcttgttt tttgcatgtg atcctgcctt tcacttcaca 57120
   gagaatacga tccatctatg tctgatgtct tcccttcctc ctgcaatgag gttcctatat 57180
   taatactatc aaacctcctg caaaatttga ggcttggatc tcatacattc ccacctgata 57240
   taggacttgg cttctccaac tgaaccctac attttacttg tatattcaaa acatttctat 57300
   cttggcttct tgtcctcaat ccacaaatac tatttacaaa gaagattttt ctctgctttt 57360
   atcttcaggt ttctgtctcc cagtataatc atatccagcc ccatggctct tactggggcc 57420
   tctgaaatgt ttatcaccaa atctgtgtct gcagttccaa cctcttccct gagcatcaaa 57480
   gctgtatttc caactttcgc tggatgcttc tatctgggta atatcttaag ctgaatttat 57540
   tcaaatatat atattcaaaa tggaattcat ttgacatctt cctcttatct ttgaagctac 57600
   tgtcattccc aatcctatat ctcagcagtg gcaatcctgt atcttagcag tggaagctga 57660
   acaagttccc tcataatatt gtagaagtac aaacagagca tcaagaatag tccttttaat 57720
   taaagtttaa gccagtaaaa acctatttta tctattctga gattcccatt atttggttat 57780
   atcattgtag attattcaga ggacactggc atctgctact taattttatt ttttttaaca 57840
   tttcatgaga cttaaattac agtctcatgg tagaattgta gttaattgat gtaccttatt 57900
   tcttgccaag attctgtttt aaacaatttg attaatgtat tgaatggcat gaatacaaaa 57960
   attgggttag ttaaaataat ttttacagat ggaagatttt tagcgtggtt aaatctatta 58020
   aatggggagg taagagctgt tttggccttc agaatctttg agaacatcat gttaaatgag 58080
   acaaccactg tttctaactc ttctttctta acttactgag agttacaaca ttgtattgca 58140
   aattataaat gggatcatag aattaaacaa tttagactta aagtatcaca tacttttagc 58200
   ctgtgtaata aatatcacta gacattattt gaacacaatg ttccgtaaca tatcttcctc 58260
   ggagaaacaa aacagacacc tttcctgagg gtgaaggtaa ccttcttgag atttatttaa 58320
   gcaaaaatag aatcgaagat gttgagatag agaagttctt agagttccct gcctccttgg 58380
   ttagaatgat gaaacagaaa ttcagaatat cacacggtgc ttagaggcag aaatggaaaa 58440
   aaagaatctg gtgttcaaat tcatacttca ggtaggaggc aagcagctat ctggacctta 58500
   aatgattctc ttgattgatt gataaataga tagatacgca gcaatacttt agttggatac 58560
   attatttggt gcctcttatg tcagtactct gatgttttcc attttcacag tttggaaata 58620
   agaaaaagta gacaaacttg gctgggtgcg gtggctcact cctgtaatcc cagcactttg 58680
   ggaggctagg gtgggtgcat caccggaggt cgggagttca agaccagcct gaccaagatg 58740
   gagaaacccc atctctacta aaaatacaaa attagccgat tatggtggca catggcatat 58800
   gcctgtaatc ctagctactt gggaggctga agcaggaaaa tcgcttgaac ctaggagatg 58860
   gaggttgtgg tgagccgaca tcatgccatt acactccagc ctgggcaaca agagcgaaac 58920
   tccgtcttaa aaaaaaagaa aaaaaaaaaa gaaagaaaga gaaaaaagaa aagaaaaagc 58980
   agacaaccat gcctactctc tctttagctt tgaaaatgtt tgacgatggt ggtgggtggg 59040
   gcctcctctg ggaccctata gtcatcacag cagttaacac attataattc aatttctgat 59100
   tctttgtcag acttcctact agactgaaag ttccttttaa gagcaggagc tgttttcttt 59160
   tctattactg tatctacaat gcctgatgca aagcaagttc ccagaaaaga cgagtttaat 59220
   gaatgaaagg gagaatggag tcagaagtca gagcacccag ggacccctct cctgtcatcc 59280
   ggttttaacc ctgtgtagga ggtaaggaat gatactccca gataaacact gcctgccacc 59340
   cacatgtcac ggattccaag gtacacatat ttttatattt taatatccct gacatcaaaa 59400
   tctaccttac aatcaatagc atgtcgtagt aagatactgg catgactcac agttgatggc 59460
   ttattggatc cagtgaagag tattattttt gtaggtaata agctcttaca taaatgagaa 59520
   caggtaagaa tcgttggatg ggctgggttg gagtatagtg gtgggagggg tttgcagtaa 59580
   taaacaggat ggcccaggtg ggcatcatta ttaaggacat gagattcaag aaagactaga 59640
   aagagctgag ggagggaaca tgtagatatc tgggggaaga atgttccagg aagatggaat 59700
   agatgacagt gcaaaacaca agtgcacctg gcacccaagg aacatgtcag gaagccactg 59760
   tggctggaac aaggggaggt aatagagaca atgtcagaga gagaaaggga ggtggggagt 59820
   agatcatata aggctctgaa ggttattgca aagacagatt ttactctgag ggacatgtag 59880
   gggcaaagca gagttctgag cagaaagttg aaatgacttg attcacttaa aaattattcg 59940
   gactgctgcg ctgagcacaa actatatagg ggaaagggga gaaactaaga gacctgttcc 60000
   aatacattta tattgatgca ggagagagtg ggtggtggct caggacagat gttaacagtg 60060
   gaggaagtga gaagcagttg gaatctggat attttatgaa gatagagcca acaggattcc 60120
   ctgaggcatg ggatgttggt acgggagagg ggaaaggggt caaggatagt gccaaggttc 60180
   ttggcacgag aactaaaagg aagaagttgc catcccctca atggagacag ctgtgggtga 60240
   agttagctca ggagggaaga tcaggaattc aggtttaggc atgctgagtt tgagttgtct 60300
   gttagacagg cgagtgctcc aagtgatgtc tagaaggctg ctggatatta gagtctcagt 60360
   gtgggaaaag atgtctggtc ttaatactag ttaaccacag catcccagtt tacacaaata 60420
   tggtgtgtga tatcttctta tcttattcgc ctctccaggc tccagaccag gcaaacacaa 60480
   catcctttta acttaccttt ctcacagtgg ccttctatgg gtgagctgtc acattgagac 60540
   ctgacccaca taagctttcc taaaatttct ccatttcttt ataccttatt ttagacaaat 60600
   ggtccatcct atcagcagta aattcatgaa caattatgca tacctttctc tcaggcctac 60660
   ccaattctgt tcctaaaatc tacaattgag gcagtgatgg aacccattag atgtatagtc 60720
   aattaagatt taatactcta aaaagaagag gcttcatttg tcaaatgcta tacagttgta 60780
   ttgataaata tcagtgatag ctgccaatat aaaagaaacc atatattatc catttgaata 60840
   tttttctcta ttcctgagga tgctgtatta ggcactgttt atttcatgaa ataaagagac 60900
   attcaatctg agacctctgt cagtaaagga aaaataatca tcataatacc tagataagta 60960
   aaggtactag cacattagag ttttgatgat atatgagagg ataaaataat aacttatgta 61020
   taattataaa ttttgaagtt tatgacatga atatagaaca cactcaaagc aagttaaatc 61080
   tctgtctact taacattcaa atggttatac catgtgaaaa gatttactca tagcaaacta 61140
   ctattcaatg tttgaatggt ttagagtagc agcttccaag gggggtgtgt actatatgag 61200
   aaaatgggta tgggaggaaa ataactaaga tgtattgttt tatgtattta tatatatatt 61260
   tctatgagta tagaaatctt acatttaaaa tcttgattag tcataataca tgtatacatt 61320
   tttaaataaa aatagatata ttgatggtat atgcctagtc ttattgggat gatgatgtgc 61380
   atggtataaa catctcagca tcttccaaga catggataac attttgcttt tgattaagaa 61440
   aatatgattt tagattcaaa ctagaaatgg agttagtaaa agaatgaata aactttgctt 61500
   tgaaaaatca aagctcaaga agcaaaagat gagacatttt ttccttggta aattttggcc 61560
   taactttcca aattgcaccc tattatgtca tatttatttc tgattttagc tatcattttc 61620
   ttcctctgat ttagtctttc acagaaataa accagaggtg aaaagggttc tagaagtgtt 61680
   atttattgtg tattttttat ctaattttgc cccatgctaa taaaagctca cttagagcta 61740
   ccttcttacc acaaatctag aaaagttatt tttggtcctt ggacccctag tctccaaagc 61800
   atgcagaaaa tgtgtctggt gatgtcaata gcttcatggg agatttttta aagaaatgtt 61860
   gttattgaga gttgggagcc tagcttaaat atggtaaagc atgcatttta ctataggata 61920
   cgtgtattag aattgatatg gggtacaaat cattattata cataatttgt tcaagattta 61980
   tgctttttgt gctttgatct attagtaaaa agcatatgat tgtaacaatt atcttggtta 62040
   agttgcatta atatatttgc cctttgctgc taattggaaa caagggcctt tagtagaaaa 62100
   tcttctacta acaatgctat taagtgtttt aagttgagtt gtaataatag ttctaagggg 62160
   ctataattaa ttttgcctat agtattaaat atagtataaa cataattaga aggaattatg 62220
   taaagatggc tactgagctc tctatctccc ttcctcccca actagtgcca agttcttaga 62280
   aactgacaga aaatttataa tcagttaatt taatgtagta tcaaccaaaa tgctaatata 62340
   attttatgaa gcatcaaatc tgatgctagg atgtatctga aaaaaaactc ctcagaaaaa 62400
   aagccaagaa aaatgtgaaa aaaagaacga tactgaaaaa gtctagccca gatttcagaa 62460
   tgtactgtca taattttaaa agtgaactat aaatttaatt atattttaga tatatatata 62520
   tattagacaa atgtccatta tatcagcagt aaattcatga agagccatgc acagctttct 62580
   cccaggccta cccagttctg ttcctaaaat ttagaattta gatagtaatg gaacccactt 62640
   agatacatag tcaataaata tttaatactc taaaaagaag aggtttcctt tgtcaaatgc 62700
   taaacagtta tattgataaa tatcagtgat agctgccaat gtaaaagaaa gccatatctt 62760
   gtccatttga atatttttct ctattcctga ggatgctgta ctaggcactg ttcatttcat 62820
   gaaaaaaaga gacgctaaat ctgagagctc catcagtaaa ggaaaaataa tcatcataat 62880
   acctagaaaa gtaaaggaac aagaaaataa ctatcccaaa ggccagaaga aagattacgt 62940
   gtagggaagg gcaagaggtt ttacatggga atggatattc tgagggttta gaggggctca 63000
   aaatgttctc tacctcaact tacgttgcgg tttcctgaga gttcacttta taattattta 63060
   ttaaactatg catgtatact gagcaccctt ttctgtgggt gaaaagggca tagaaaaaga 63120
   gtatggtcta gccacaggaa tagaccaaca tatcactgga aagaattggt agtccaggaa 63180
   tcaactcatg tttatatgaa aacttaggat cagtagcatt tccaattact atggagaggg 63240
   tgaactatca aatgggacaa ctcactgcct cattctatat accaccataa aagttaggtt 63300
   aaggagctaa atgtaatcta aaatctccca aagtattggc ctgtcgtggt gactcacacc 63360
   tgtaatccca gtcctttggg aggccaatgt aggtggattg gttgatgcca ggagtctgag 63420
   accagtgtgg gtaatacgat gagacccagt ctctacaaac aattaaaaaa ttagccaggc 63480
   atggtgatgt gcacctgtag tcctagccac atgggaggcc gaggtgagag aatcacttga 63540
   gcccaggggt ttgaggctac aatgagttgt aatcatgcaa ttacactcca gcctgagcaa 63600
   cagagtgaga tcctatcctt aaaacaacaa caacataaaa acctctaaat gtaaaaatat 63660
   taaaatatag cattggagaa cttactcttc atcacggaga agttatacac acgaacatgg 63720
   gagatattgt gaagactcaa gacccaaatc aatgaattaa aaataacaat aaacacaagt 63780
   agtgttcttt tggttaatat tttaactaat agttaaataa ttaaacacta gaacacattt 63840
   gtgaagggaa ctgtattcta cttataagag taccagccag gcatttcctc attctcatgc 63900
   ccatagagct tcacgtaaac ccacccaagt gcttagccta taccatccct tcttgtgatg 63960
   caggaaatat cattcttgtc taatccctaa tttgtgcctc tgtttgcaca tcctcacctt 64020
   gttcattctc ccctttttaa tgggattttt cccatcctca cacatgcata aaagagcagg 64080
   agcaccactg tcctcatcga tgaccctggc atagcccatg ctagattccc cactagtttt 64140
   gttctccttt ctagctaata tttcaaaaaa atatatgtat ataatgatct tccttatttc 64200
   tctttagtta ttctttcttt tactctaatt ggtccttgat ttctaccgcc ctgctatagg 64260
   tagttgtata agtctcttct tatgcttcta atagagacat actcatgact gggtaattta 64320
   taaaggaaag agattttatt gactcacagt tccacatggc tggggaggcc tcataatcat 64380
   ggtggaaagt gaatgaggag caaagtcaca tcttacatgc tggcattgcc tgccagggga 64440
   actgccctgt attaaaccat cagatcttgt gagacttatt cactatcata gagcagcact 64500
   ggaaaaacct gcccccttga ttcaattacc tcccaccggg ttcttcccac aacatgtggg 64560
   gattgtggga gtacaattca agaggagatt tgggtgggga cacagccaaa ccatatcacc 64620
   agtcttacca aaggaaccag ctatctacct ccatgctgcc atccaatgca cagttctctt 64680
   ttctcatcct ttatatctcg agtgaaatgt tcttgaactc ctgagctcaa gcaatcctcc 64740
   catctcagcc tagcctcctg agtggctgga actacaggga tgagacacgg tagctggctt 64800
   caagtgaaat gttaaattct cagagatgcc ttctcatacc taaaattgca ctaatacctt 64860
   tccatttctt tcaatccttt gttttcttcc tggcacatct catataatta gctcatagtg 64920
   aatatatctt tatttatcta tttaattgct atgtagcaaa ttaccaccat gtttcttggc 64980
   ctcaaaagca tttcttatcc catagtttct atggatcaga aattcaagag caacttagct 65040
   gggtgtctct ggctcaagtt ctcttatgag gttgtagtca aggattgatc atggctacgg 65100
   tctcatctga agatttaact ggggcttggg ggttggtggg aggtctgctt ttaggctcat 65160
   tcacatgggt cttgacagga ggtctcagtg cctcacttca tgggcctctc catagggctg 65220
   cttgagtgta cttacaacat ggctgctgat ttccccatca caagtgatcc aagagaaagt 65280
   cacatgtgtt agaccattcc tagaaatgat ataccacttc tgacttattc tgtttgtaac 65340
   caacaagtca ctaagtccag ttcacaccta ggaggaggga aattaagttc tacttagtga 65400
   aaacaggagc atcatataat ttgtggatat aacttaaagc caccatactc cccattagaa 65460
   tataagctcc cacagtgcaa ggaatgggtt actgcttaca cttacatcat tagtgcttag 65520
   aacagcaggc actttgataa acatctgttt aatgagtcaa tagatgaatg tctcttactc 65580
   ttgggcagca aagatataga ctatggagtt atctagacct ggtatgaaat tctgactcta 65640
   ccttttacta gttctgaact ttggacatat tagtttctat gagcctcagt ttcctcatct 65700
   gtcacctgtg ctatattcct gcttcacaag aatgctatga ggattaatga aattgcatat 65760
   ataaagcact tcttcaagca ttcagcacaa gataagcatt caaattctag tttctctgat 65820
   tttttccatc ttttcacttt ccccaaaact ttcaagaatc actatttctt tactacatga 65880
   taccaaaatt ttctatttaa ctttctcata atgaattaat catatgttca attatttaca 65940
   tgtcgaagaa aaatcaattt atcttagatt taaagaattt tttcattagt gcataatttc 66000
   tattagatct tcccaagaga attctaatat gtccctatgg aggggaaatt aacaaatggg 66060
   ccaattggaa ggaagattcc ttagcataag aggtacaact gcaatttaga ttcactattc 66120
   tgtctaaaag aaaagtatgt taccccataa atagatacat acttgttcaa gatttatgct 66180
   ttttgtgctt tgatctacta gtaaaaagca tatgattgta ataattacct tggttaaggt 66240
   gcattaatac atttgccctt tgctgctaat tgaaaatgag ggtcttttgt agaacatacc 66300
   tactgtgtac ccacaaaaat aaaaataaaa taaaaaataa aactgtgata atatttagta 66360
   cttgaaggaa agtcacaagg ctcatctgta gttgcaccat tttttcttgt ggaaaatgac 66420
   agcagacaga atgaatttgg gaaattattt tgtggaggga aatgaggcca tgggaccctg 66480
   tgggtggaat gaacagtact gctaaacagc ttttgttgaa tcagaaactg agcgaagaat 66540
   ttaccaagct gccctgaaac ttggtatcat tatgagtgag tctaccgacg cagctctttt 66600
   ttttcccctt aaaaaatcag gtgtaattta cattcagtga aaggcataga tctaaatttt 66660
   atagccctat gacacatata taaatatgta taacccacac cccaaccaag atagaacatt 66720
   tctatcactc taaaatgatc cttatgtctc tttccagtca tttgctacca cccccaatgt 66780
   aaccactgat ctgacttttg tcatctttag ttagtatagc ctcttctaga aagtacatac 66840
   tgctgtgctt attagtatat gcctctgaga tccattgttg tatgtgagta gttttttgtt 66900
   tgttcgttcg tttgtttttt tgaccgagtc tcgctctgcc gcccaggctg gagtgcggtg 66960
   gcgccatctt ggctcactgc aagctccgcc tcctgggttc acgctattct cctgcctcaa 67020
   cctcccgagt agctgggact acaggcgccc accatcacac ctggctattt ttctttcttt 67080
   tttttttaat ttgttagtag agacggggtt tcaccatgtt agccaggatg gtctcgatct 67140
   cctgacctcg tgatctgccc accttggtcg tccgtcagct cctctagtga gtagttcttt 67200
   tttgctttgc tgagtagtat cccaatgtat gactatatca gagtttgttt attcattcat 67260
   ctgatgaaga acatgtggat tatatccaat ttttagctat catgaataaa gctgccatga 67320
   ataatcctgt acattttttc tttgggatca atactgaaga atataattgc tagataataa 67380
   agctgatgct taattttata agaaaaagcc aagctttaaa aaattggttg ttctattttt 67440
   atactcccat cagcaatgta tgaaaagtcc agcggctccc caactccagg gatggtaatg 67500
   gctgtgtttt cactgttagg cattctggta ggtgtgagcg agtatccaca atttgctcta 67560
   tcacagtttt gtgcatgttc gtgttttctc caatcatagc tttcattctt tactttcaga 67620
   agagaagtgt tgggactgta tctgttcctc ctttccccca agctctttca gtagggagtg 67680
   ttcccatgga gtgaggtgtc acactgaatg aagtgttcaa ctgcatcagg tcttcagcac 67740
   cctaggtagt cccagattga cactttctta ccatggcgat gtaagaaagc atttaccata 67800
   gtaagcgggt ctgaccccat atcctgcttt gctttgtgga tctcccatct cctcctcatt 67860
   ctggcccttg ttctgaaata taaccttttg aaacataagt ttccctcact gtccagtgct 67920
   ctgtccagta tgagaaaaca gcaataggga acagttactg caaagcatga actcaggact 67980
   cagactgcct gagcacacat cgtaactctg ctgctgacca gtactttacc ttagactcac 68040
   ctcaagcctt gttttcttca cctgcaaaat gggacaacta cagtaacaac cttgtagtgt 68100
   tgttgtgaga attaaataat aaaatataca aggcacttag ccatagttat gccaaggctc 68160
   cagctctgcc gtctattatg cgaacatcta ctctgatgcc tacggacttg actttttacc 68220
   actctctgcc cccacttgtc ctgtgctttt atcttctttt ctttctcttc tctttctcat 68280
   atacttccta gccacatcaa gtcaatgtgt aacttggctt tgggctttcc tttttcactt 68340
   atgaactagt tataagaagg gtgacttcca gaaaaataga agtgaaattt accaaactca 68400
   tgcactgtct aagaattgaa caatgagaac acttggacac aggaagggga acatcacaca 68460
   ccagggcctg tcgtggggtc gggggaaggg ggagggatag cattaggaga tatacctaat 68520
   ataaatgatg agttaatggg tgcagcacat caacatggca catgtataca tatgtaacaa 68580
   acctgcacat tgtgcacatg taccctagaa cttaaagtat aatgataatt aaaaaaaaga 68640
   acttaaagag aaccaatgca agcaaaagcc acttctcttt ctgtgattat tcaagtggag 68700
   atggtacact tgcatgagct ttgggacatg ttgtacttct ttaggcaaag ccagcttgtc 68760
   ttccagagag atcttcttgg atggccttct acaccctgtt gaattcatca tagccctggt 68820
   ttgattttga agagtgagaa gtttgacttc tcatcatgag gatttatctc tcttagtttg 68880
   agtttcctca aaagcagatc tcctgaaaac cttcaaggag agacaatgcc tcagtttccc 68940
   atggaggggt gaggaagttg gaatattttt caaccaacat gtgcccttca ttggttgagg 69000
   gtcactcctg gagtgttaac tccctgacac atccagcctt ccctgaacat tctgcctgac 69060
   tattgggcca agaagaatag ggatgccttg aaataggagg aaagtctctg ctggaggacc 69120
   caggggatat gcatggcact gacaacatct acttcattat ttcgcaaatg taaattcaca 69180
   ttaaccacat ccaccaaaca cagggagtaa tgcatgaaaa gcaatcatgc tgatctatta 69240
   atattctatg agaaatacaa ccaccagaaa ttccaactat gtgtgctttg acagtggcag 69300
   cttctttatg gtcataacca aatgggaagc cagattatct tcacaaggag actggggcgt 69360
   ttagagccgg tgacatgagc ttcctggctg gtgtcattga cctctgacca atggcataca 69420
   tcccctagcg gggatattcc ttcacaggga tggtctagag actcctgcaa aaccctcttt 69480
   cttggatttg actctgtttt ttctttttat agacaccttg ctgatcatcc ttctcctaat 69540
   agtgcacttt cttatctgta agaattatat ttataatcat ccaattatac ctcccccagc 69600
   tcatcttatt ttagttctga gggtcaccac taatctcttg gtcatagctc caggtggctg 69660
   ctctcatcca ttacctttgg attctttatc atttcttcac tagaggttaa atttaacctt 69720
   agttttcgga aatttgttgc tgttaactcc catagctgcc tgcagacatt ttcttgataa 69780
   aataagaaag tgagtacaag aagtgggtaa taatttattt ttgtttttta ttgttttgag 69840
   atagggtctc actatgtcac ccaggctgga atgcagtggt gcgatctctg ttcactgtat 69900
   cttcggcctc tcgggttcaa gtaatcctcc cacctcagcc tcctgagtag ctgggaccat 69960
   gggcatgtac cagcacacct ggctaatttt tgtatttttt gtggagatag agttttgcca 70020
   tgttgggcag gctggtctca aactcctgct caagtgatcc gccctccccc ggactcccaa 70080
   agtgttgata ttacaggcat gagccactgt tccttgatgg caatagttta ttttaaaaat 70140
   atttgaattc attaggccaa gtgtggtggc tcatgcctgt aataccagca ctttgagagg 70200
   ctgatacagg aggattactt gaggccagga gttcaagacc agcctggaca aaagagtgaa 70260
   acgcccattt ctacaaaaaa aaaaaaaaaa aattaaaaaa ttgactgaat gtggtggcac 70320
   acacctgtag tcccagctcc ttgggaggct gagtgggagg gtcccttgag cccaggagtt 70380
   tgaggctgca gtgagcagtg attacaccag tgcattctag cctgggtgac agagcaagac 70440
   cctatctcag aaaaaaaaaa aaattttttt aaattataga tctgctcttt cttgacactc 70500
   cgctatagta gaaagaaaac tggagtatgt taggttgaat gacatgaagt tggcaagatt 70560
   caaccatttt tgacatataa taggtcaatt tcatatagtt cagcctacat aaagacaata 70620
   atgttctcta tcaaaaagtg agtcaaaata tcttatctct gttagggcta tgtggctttt 70680
   aagcagatcc tcccacctct ttgggtctta gttactcgtc tgtaaaatgg gtttgactag 70740
   ataaattata ggagctttcc cacaagtttt atgaagattg gtcattgcta tggctgctat 70800
   aagaagtctt gtttcaagga tgtttttctt tgttgtgaca tcttgctttc ttatattcat 70860
   gttctcttac gtttataccc aggagtctcc tctgtccttt tctaatcttt cttgctggac 70920
   agttttttcc ttaagacagc tctatctgag ttgtttgtcc ctatggcagc catgaacaat 70980
   agagtaacta tctttgctgt ttatctatcc ttgacaactt gatgtccaac ttggcactga 71040
   ggataacttc ttgagggctc atatttcctt tagggcctca actccaacga ttcttcaacc 71100
   ccactgtgac ctgaagtcca tgcatcctac cccctttctg ttgtctctca ctctcttcat 71160
   gtcccaattt tctactgttt cagctcacat tttactctgc attactctaa ttactgtctt 71220
   gcattttcta ggcacacatt gctcctttcc ccttcctttg taatcacttg gcaaaagtct 71280
   aacccagatt acattcaact cttcatgccc atcttgccta cactcaccca gctaaacaca 71340
   gatggagtga aacacaccat tgtgctggct cgtttcactt tacagccatg actacaaacc 71400
   acgcatgggt ccttagagct gccgagtagt cagattatgt ttctggagcc cctttactct 71460
   cctgttcttt taggtaacca ttttatacct tatcttctcc ctcattttca ccttcagctg 71520
   attttcttgt ttcttgtttc tttaagaaaa tagaagccat cagaatgaga catccccatg 71580
   tccccatcat cgcactgtca accgcacatc tgcactcatc tgcgctcatg ttctctgcct 71640
   acatttcttt ttcttagaat gaactgcctg tgcttcctgc agagtccaag ccctcctctt 71700
   aggaagtcga ccgtcctctt tcgcctgccc aaggacatca ctctggcaac tcttccccct 71760
   ctcctgcatt atctgttggc ctttctgtgt catacacata agctctattt tttccccatc 71820
   taaaaaagaa actcttttga ctctaggcct ctccagctac caccctactt ttctactgcc 71880
   ctttagaaaa aactcctcaa aagaattatt ctcacatttc tgcaggataa catcacacat 71940
   tctcctagtt ctatattctt ctactagcta atttctttgt accatattta gtttttaaag 72000
   agctcctttc tctataaata tttcatttca atgccagagg tcatatcaga tggagaatgg 72060
   ctggttggtg gtttaacact gattaaaaga agaggaaaag cagccaaata tttagagaaa 72120
   aggtgaagcc attccatccc atcacctttg ttggagcatt tcaatttgct ctctacctta 72180
   gatgtgctct ctagcccata gctgagctaa aggtatatta agtctgtcta accgggcttg 72240
   aagggaagct ggaagaccat ttgttctttt aaatctcttt agtttgcaat tgtcagattc 72300
   aattttgagg ggaagagttc agagcatcat tgaaagtaaa aaagatgggg gaaagtgata 72360
   gccacatgat aaattatatc aaagtcaaag tcatcgccct atggcaattt ttgcaaagaa 72420
   gtatttttag catggatagc taaagggtag agcatgtcac agagacacct gtgtagttat 72480
   gcagactggc agggatgtag aggaactatt agaacccctg ccttgtgtat caatggcata 72540
   ttttatccat gggcagaatt aacagataat attcgctata ctgattagga gagaagacag 72600
   cttcatctcc acacagctgg aaatatgtcc tgcttcaatg tcaatttgtt tatgctgtgg 72660
   gccaagtcat gcccccatat gtctgtattt tcacatctct aacagatatt aattgcacta 72720
   gatttgctgt gctgtacttt tgattagtca gaagttcggc ctagtaagtt agaactctgt 72780
   gttctcactg tgttaaaggg atgcttgtca ttatattgtt ttgaaattgt ttaaaagata 72840
   cagttaggga ggcatagtcc ttgatctttt aaaatccttt taaagaaatg aacggggcaa 72900
   ttagatagca aaactgaaaa cttatgggct tttatttttt aatttttaat tactatggga 72960
   acataatagt catatattct ttcattcttt ttttccctcc cttcttgttt aagttaggcc 73020
   agagtccagt tttctttatg gttttttttt tttctttctt tccctttttt tttttttttt 73080
   ttttgcgatg gagtctcgct ctgttgctct gttgcccagg ctggagtgca gtggtgtgaa 73140
   cttggttcac tgcaacctca gccgcctgga ttcaagcgat tctcttgcct cagccttccc 73200
   agtagctggg actacagccg cgcgccacca tacccgtata atttttgtat ttttagtaga 73260
   gacagggtta tgccatgttg gccaggctgg ttgagaactc ctcatctcaa gtgatccacc 73320
   caccttggcc tcccaaagtg ctgggattac aggtgtgagc caccgcgcct ggccccttta 73380
   ctttttattt attctttcta atttcaactt ttattataga tacagggggt acatatacag 73440
   gtttgttcca tgagtatatt gcagcaggta tgggcatagt acccaatagg tggtttttca 73500
   acccacagcc tcctccctcc ctcccccatc tatagtctac agtgtctatc gttcctatgt 73560
   ttatgtccat gtgtgctcga tgtttagctc ccacttatga gaaagtgtga gatttggttt 73620
   tctaaaccct gcacgtgtac ccctgtgtct aaaataaaag ttgaaattag attaaaaaaa 73680
   agtgaagacc ctggtataac ttaaacagga agaaaaaaag aattaaagat tatattacta 73740
   ttacgtgccc atagtaatca aaaattaaaa aaataaaagt tgtccatagg ttttcagttt 73800
   tggtgtctaa ttgccctgtt tctataagag gattttaaaa gatcaaggac tatgcttccc 73860
   taactgtatc ttttaaacat taagtcactt agggtaatgg cctgcagctg cattcatgct 73920
   gctgcaaaga catgatttca ttcttttttt atggttgcat agtattccct ggtgcagatg 73980
   taccacattt tctttatcca gtccaccatt gatgggcacc taggttgatt ccatgtcgtt 74040
   gttatcatgg aagtgtttag aacaaaacta gttgaaaaaa tatcccctag aatcccaggt 74100
   acaaggagaa ggggacaaac ccccaatacc actgacctgc atgggctgaa tgtggcagca 74160
   agaagcagag ggaggcaagg gggtgactga agaaccagag aaggtggtaa ccccaaacta 74220
   tccaagaggt actctacaga aagcttaaat attgcctgga aatgggcact tttatggatt 74280
   gcccactctg ctaggctcag ttttagatac tagagaggcc acattaaatg aaatgggaaa 74340
   ggtctctact cttacagagc ctatgtccta gaaaagaaac atagacaaat aatagaattt 74400
   cagacacata gatgtgctaa aacaaaggaa ggcaatagaa ggaacccatg tggctgggtg 74460
   aggaactctg cttttaatga ggaggtgaca tttaagcaga caggtgagtg gaccttagcg 74520
   gacctgattt aataaataga atgatgagga taagttcatt gattatttga tccaggattg 74580
   aacattattc acttaaatag gttaattatt ctcctttatg atctcatctt tcaaaatttt 74640
   cctccatgca atatcaaagg ctgcctgtat ccaaaatcaa aatgagtaat tttgatgaca 74700
   taactgcatg tggcctgata aaaatcatcc ttattaaaat gaattctgga aggggtccca 74760
   agattggtgc agtggttcag ccttgctgcc aagaatccag ctccccgcag tctgtcaata 74820
   tataccctca ggtctgtgta ttttggccga acgtgggggc catggtcagg catcagcatc 74880
   ttgtgggagc atatcctaat gttggaagga aaacaaagtt tgtctctttt ctctcttgtc 74940
   tcttctctct cttaaaaaaa aaaacatttt aatgaagaat tgtgcacata ttgttaggca 75000
   tatatagctt gatatgtttt cacaactgaa tgcatccagc attcaaatca aaaagcagaa 75060
   tatcattagt acctaagaag tctcccatat gccccttttc aatcactacc ttctccaaat 75120
   gtaattagtt gcatgatttt aagtttctaa catcttagat tagtttcacc tattttaacc 75180
   ttttgaactt taaatggaac ttcacactat gtactgtctc atgttggctt cttttactca 75240
   ttatgtttgt gggattcaca gccatttttg tgtgtaaatt atttttatta ctccatagta 75300
   ttccattgtc cattctgctt gaaaatatat atatatatat atatatatat attctgcttg 75360
   aatatatata tatatatatt ctgcttgaat atatatatac atatatattc tgcttgaata 75420
   tatatataca tatatattct gcttgaatat atatatacat atatattctg cttgaatata 75480
   tatatacata tatattctgc ttgaatatat atatatattc aagtaagtag aatatatata 75540
   ttcaagtaag tagaatatat atatattcaa gtagaatata tatatattca attagaatat 75600
   atatatattc aattagaaca tatatatata tttttttctt tttttttcct ttttgagatg 75660
   gagtcttgct ctgtcgccag gctggagtat agtggcgcaa tctcagctca ctgcaacctc 75720
   tgccttccgg gttcaagtga ttcttctgcc ccagcctccc gagtagctgg gactacgtgc 75780
   tcgcaccacc acacccagct aaattttgta tttttagtag aaacggggtt tcaccatttt 75840
   ggccaggatg gtcttgatct cttgacctcg tgatctgcct gcctgggcct cccaaagtgc 75900
   tgggattaca ggcgtgagcc accacgccca gcccattcta cttggttatc attttaaaca 75960
   gtaaccagtt tggggttatg agtagtgctg cagtgagcat tctagtgcat gtctcttggt 76020
   gatcatatat acacatttct tttgactttt tgaagtcctg tatctaattt cctctcaagt 76080
   cttcttgggc aacattaggt cacatgccct tcctatttgc agtagaacct gggaaagaaa 76140
   atatctaaga atgtgctgtt aaatgttcaa caactagctc ttagcaggag ggttgggcag 76200
   ggagaagcag ggagagggcg gcttgatggg aggagggttg atttgcagca tctgcagttt 76260
   cttggtgtga atactcccat catggccagt tttaagctac cagtgttata tcaaccacct 76320
   tgcaaaaaca gctctcatag aattagtgtt agccagctcc agaatgccac agaaaatagc 76380
   taacattttc actttttata attggaattt ggctctgcca gaaaaaaaaa atggaggcta 76440
   gggagggata ggaaatgttg ttccataggc aatcagtatt tttgcaccaa ttattgagtt 76500
   actactatga ataaattaat aaactactat cctaacccaa agatgattat tataaatttt 76560
   agattattgg tgaaaatgaa aactggcttt tagatactat taggataggc tagatttata 76620
   ctgtggttaa aaaaaatccc caaattctag catttcaccc ataggagttt gtttttcact 76680
   catgcaaact ccactacagg tcaagaaact ctccagtgca gctattcttc atgcaatgat 76740
   ttagcaaccc ggacagcatc atgttatggc tcattatttc aatactcagt ctccatggtc 76800
   accgctggag caaaggagac aggtgagggt tatacatgtg cttgtaatac cttcagtgca 76860
   aaagtgattt atcaattcca ctcataggtc attgcctaaa gctaggtact gggtccctcc 76920
   caactgcaag gacaattggt aaatctgtgg gtatatgatg tactgtaaat gtctctgcca 76980
   cagagaccat ccactcagaa ccatctgtta ttttttaaag atcctaaaat gggcctttaa 77040
   aaatatttag ttctatgcat ttccacccta acatatatcc agttactcta gaaaaataac 77100
   cacaactgta ctcgaagtta tccaccagta atgcattatc tcagaaaata gaagaaactg 77160
   tttcttaagc aataacaaga tcaatcctaa tgaagactct tttctatata tgagttaatt 77220
   ggcttttata ttggactata atctccagta taggtaatgc cttcagccat aatgcttatt 77280
   ttaccatcac cgtagtaata attgatgtag gtaagaatca tcaacagacg ctaaaaccac 77340
   tgggctaaag ggtgctgagc aagagaatat ttgcataggc ttcaaagtga tatacccaca 77400
   tttattactt acaaagggga aaaggtagca ttaaaatgga gagatttggc agatattgcc 77460
   tcttccaatt gatcaaattt aatattgcca ataatggggc aaactaaaac catgtgcccc 77520
   atacgataca ctgagaaagg gacctaatgg tatctacaca gtatgcctac caaaactgtt 77580
   aactatgaac ataatcttaa gaaaaaactc aggcaaatcc taatttagag ctgtcttgaa 77640
   aaatctcagc tgtactcttt taaaatgtca atgtcatgga agccaacaca gtgtccttga 77700
   tgaaaggaga tcaagaatga tgactaaatg cagtgtgtga ttctggttca gatcctggaa 77760
   aatgaaacta taaaggacat tattggttta aggtggagat tttgattata gactatatat 77820
   ttgataataa tattgtatca atgttaaatt tcccaaatgc aattagtatg tcatagttat 77880
   acaggagaat gggtttgtct tatgagaaat gtgttgatta gtggtgaagt gtcatacctg 77940
   caggtacatt caggtgattc agaaaaaaag gatacgcata tatttatatg tgtatgtgtg 78000
   tttaagtata tgtgtaatta tatgtacgta taaacacaat atacacacat acagagaaag 78060
   caaatgtggc aaaatgttaa tctttgaaga atctaggtga aggttgtctg ggcattaatt 78120
   cttccactct tgcaactttt cttcagtttg aaatgtttca aaataaaaaa aacgggaaaa 78180
   tactgtttgt tccatttttc ctgcagtttt gcaactcaat tcactcctgt catatttgct 78240
   aaaacatcag tcttattggg accaacttca ttctgttgct taatctgcac ggtttagtca 78300
   tcagatgaga gttgggcttc aagattgttt ttttttgttt tgtttttcat ttaacagctt 78360
   attggtttat ttttgatatg tgtctgtatc agttaggata agttaggttt tttaaaaact 78420
   acttttttgc agaatggttg acatgtaaga agctatacat atttaatgtg tacaactcag 78480
   tgaatttggg gataagtata cacctgtgaa gccactgcca ccattaaagc tgtaggcata 78540
   tccatcacct cccaaagttt cctctcaccc tgcccctctt attataattt catgtgtgtg 78600
   catgtgtgta tgtgtgataa gaacactgag ttctaccgtc ttggtacaat atagtattgt 78660
   taactatagg tactatgcta tatagtagat ctacagaacc tatttatctt gtataagtaa 78720
   aactttgaac cctctaacca ttgcttcccc atttcccctt cccctcagcc ccaggcaatc 78780
   agcattctac cttctgcttc catgattatg cctattttag atttcacgta taggtgagat 78840
   catacagtat ttgtatttct gtctctggct tatttcactt agcataatgt cctctagatt 78900
   catccatgtt gttgcaaata gcaggatttg tttctttttt taaggctgaa aatatttcat 78960
   tatgcatata caccacagtt taaaatccat tttcctgttc atgggcattt aagttgtttc 79020
   cgtttcttgg ctattatgtg taatgccaca gtgaacaagg gagcacaagt gtctcttcaa 79080
   catcctgatt tgaatttctt gggataaata cctagaagtg ggattactgg accacataat 79140
   tctattttta atttcttgag aactttccat actgttttcc ataatggatg tactaattta 79200
   ccttcccata aatagtgtac cagagtccca ttttctccat atctttacca acacttatta 79260
   tttgttgtct ttttgataat aaccattcta acagtcatga ggtgatatct cattgtgatt 79320
   ttgatttgct tttctctgat gattagagat gtcaggtgcc ttttaatata gttggccatt 79380
   tgtttgtctt ctgtggagat atatctgttc agatccttcc tgtgcccatt taaattaggt 79440
   atttgtttta ttttatttgc tattgagttg tatgagtttg ttatagattt tggatattaa 79500
   caccttatca gatacaagat ttgcaaatat gttctactat tccataggtt gtcttttcat 79560
   tttgttaatt gtttcctttg ctatgcagaa gcattttaat ttgatgtagt cccatttgtt 79620
   tatttttgct tctgtttcct atgctttggg tgctatatcc aaaaatttac tgccaagacc 79680
   aatgtcaaag aattttttct ctgtgttctt ctaggagttt tacatcttca agtgttacat 79740
   ttaagtcttt aatccatttt aagttgattt ttgtgtgtgg tgtaagagaa gggcccaatt 79800
   ttattatttt acatgtgaat attcagtttt cccatcactg tttattgaag aggctgttca 79860
   ttcccctttt tgtattcttg ctgcccttgt caacaattat ttgactgtat atgtgtgggg 79920
   tttatgtctt ggttttttat tctgttcaat tggtctatgt ttattttatt ttcctgtgaa 79980
   gaagaattct caacaatttt ttatatggct actctaccct taaggaggtg gagtatgact 80040
   tccaatccat tttaaaaaaa aattcttgtg tgtatttaaa atatgcaaca tgttacaaga 80100
   gatgtataga tgataaaatg attacaatag tgaagcaaat taatatatac ttcatctcac 80160
   atagttaccc acttatgtgt ctatttttat gccagtgcca tgctgttttg attactatag 80220
   ctttgtaata taattttaaa ttgggaagta tggtgcctcc agccttcttg ttcttgctca 80280
   aaatcggttt ggctattcag ggtctttgtg gtttcatatg aattttaaca ctgctttttc 80340
   tacttctgat gttaaaaatt gccattggaa ttttgataga attttgatgc aaaattttga 80400
   atctatagat tgtgcagggt agaatgaaca ttttaccaat atgaattatt tcaatccatg 80460
   aacaccgaat atccttccat ttatttagat attctttaat ttcatcagtg ttctacagtt 80520
   ttttgtgtac agatatttca agggcaggga ttcttaataa tcaccacctg attctaaacg 80580
   ggcctgtaac acaggaaaga gcccacccag aagcagagat gtccttctgt gtgtttggat 80640
   tgcacattca gttaatacag catcaggcct ggaagatgat atttattttt ctttgaaaaa 80700
   cagtgggatc aggaaaagca aagctaaaga taactttctc cttttaaact tctgttgcgt 80760
   taggttagcc tgtggtatta gagtcaaaca tatttgtcta ctctgaaatg acactagtga 80820
   tactttatat gcttatgctc taaggaaaac tacttagcat attctgtagt gttaaatgaa 80880
   tgtactgcct tcttagaaca ggccctgtaa ttttgtgata agagtatcaa tgtttcactt 80940
   tgggaggctg aagcaggcag aacacttgag gtcaggagtt tgagaccagt ttggccaaca 81000
   tagtgaaacc ttgtctctac taaaaataca aaaaaattag ctgagcatgg tgatgcatgc 81060
   ctgtagttcc agctactcag gaggctgagg cacgagaatc actgcaacct ggaaggtgga 81120
   gattgcagtg agccgagatc gtaccacggc actccagcct gagtgacaaa gtaatactct 81180
   gtctaaaaaa aaaaaaaaaa agaaaagaaa agaaaaaaga taaatgtcag atgaattgat 81240
   atagatgatg aacttcaaaa agaagctaaa ctcaaatata catttttttt tttttttctc 81300
   aaccatcaaa aacttgaaat tctctgttga gaacttctgg gatcttttct gaccattctg 81360
   tcaggagcag cccagtagat ttgtagaatt aatgttgaca cttcacacct tttatgagtc 81420
   cctatccaaa tgggagaacg gaagatttgt atcagacagt tttgctaaga attgaaaaca 81480
   agagccattt tttggctgtt aaccagaatg aattgttgat caagtagatc atttagccct 81540
   caatactgga actcttgatg aatagcatgg agaactaaat tataaagttt caagattttt 81600
   tcactatttt gtcattgtcc taaaagtctg gaatgagctt tttaagtttc tgtctctgtt 81660
   tttggctcta atttttgaca tcccccacaa aacaaagtag tacttcagtt attgaaagcc 81720
   attatgattt tcagtaaata tgtctcatgt tgctataaca ctctggtggt cacagctacc 81780
   caataacatc acatgggaag gacagctaca tggttcaatt taattacaaa tatttacaca 81840
   ctttcaatat aagccctgga aaacattagc gtagggtgac ccctcccagt aggatagcag 81900
   ctttttgcag ctcaaagctg tattctttcc taaaaggttt tatttgacta aatggctgac 81960
   aggcggatat tgaatagaga atgagatggt tggtatatgt tacgacttga atgtaaccct 82020
   agacacagag tttaaaatag tctgctggtg tcttataggg acttgttccc catgacccaa 82080
   cattcctgag accaggcaac attctcttcc tgcctccagg gagtcatttc gtagctgggt 82140
   ttgggtaaac gttgtgggtt tcaaaaaagc cccatgcatg ggaccaggag agcctccttt 82200
   ttcgaccttg taccacaggc atcaccaact actcaagatg taactttgtt cagggcaggg 82260
   ctactcaaag tgaggatccc tttccttcat ctgggagctt gatagagatg caaattcatg 82320
   ggctcctcac ctactgactc tgaatcactg gggtgtggcc aggaatctgt gttttaacaa 82380
   tctctccctt aaattctgtg aaatatcaca attttagatt tcccataggt gtgaggcaac 82440
   aagcagacat tagctgaata cttctcaaaa gatgatcgtg atcatctgtt caccttgcca 82500
   aatagaagtt tcccataatg caaacagagg aacacttatc tataggatat gataagaaac 82560
   aggttctgac aaatgtaaat atgctacaaa gtaattttat taaaacatgg tgttatcaga 82620
   tgtttagcag tgaaaagaga tagtaaaatt aaaaagccca gctacattct gcatcatctt 82680
   atataacttg ctaccctttc aattaatact tttcctggat ttgttatctt gtaggacgta 82740
   gtgttaagtg aaatcttcct tatttgattg tgttctcata ttgaatgctt gcttgctttc 82800
   tcagtaagag ttcctaggag ccaagcaaat attacattag tgcataattc ctccagggac 82860
   tgtgttgtgg ctggagtcac aaggggttct gtcccctttg gagagcagac ctggaactac 82920
   actcagtggg ctctgaaacc tgagccccag agcctgtttt gttggtaaaa actttgctgc 82980
   ctttcttttc cttagacatg gcctccaggc agaagacaaa gtagacaatt tataaagaag 83040
   ggagcctgtg gatgtcacag aggcatttct tgtccttact ggggcaagga cataaataga 83100
   acacatttta tttctataag ctgctatgtt cattttagtt cccacacatt tagtggtgaa 83160
   ctgataaata tttaacaacc tagactccaa gggggaaagc cttgaatttt agccttggca 83220
   agttttcaaa atctaaatac catggccaat ttcaagctac caatgcaatg tcacaaagtc 83280
   atggtagtgg ggctgaaaca agaggctgca atcagctatc actgttctat gcgagctggc 83340
   ttcagcatac cactatgcaa agtactccct tccaggtact cgctgagata gaagatggga 83400
   attgagctag cccatggtag gcatggatag gaaggagaca tcactgttgt tgcacgggct 83460
   tggcatgtgg ggagatctgt tggtgttcag tacatgagaa aacattcatg tgttcgatgc 83520
   cactcccttc tatgctaatg ttttggccac atgtgttggg gaggtaaaat gctggattct 83580
   atacttctgt taatagtatg atgagttcac cacaaagcat aaggctagaa aggacatccg 83640
   tacctcacat tttcagataa atgacttagc aacactatta ttccctgcta aggctgcctc 83700
   aagagcaaaa gcaggcatgc acacaattct ttgttgcccc agaaagtatc gcatacattt 83760
   gctattgtag gaaagtataa aaaagcttgc aatccaaata aggctatgaa gtagctccca 83820
   agggcagcat aacttctgaa gcaaactcct tctgcaaaga atcctgttat ttatatacca 83880
   agagcaacaa acatccatat ttacttttgt ctgtgcccga attcaacatt tgattgttct 83940
   tgtcactgtg aggcttggcg tctaaaacac tgtaatgaca ggttacgtgg tgtgagataa 84000
   taaagagtgg atgcatttta tccagggaac tgattctact actatcaaag tattttgaaa 84060
   gaagaggagg agaattcaag taaaagttta aaaatagaac agcatgaagg agatcagtat 84120
   acatgtgcat aaaaatgttc ttggaagacc tagaatatgg aacatttcat tagtgtattc 84180
   atcagtactt ggttcttgaa gtcaggccat tttatgaggg agaaaatgtt gaagcacttg 84240
   acctaaggat tcaagtccat gctgaaatat gacaaaaccc tccactgaat ctcagaacct 84300
   ctgaactctg gtatttcttt taaaaatcca gagcttccca aacaaaatcg aagaagagaa 84360
   cagagcttta aaatcttatt aaagactgga ctgagtggaa accaatgaaa tatctagagt 84420
   ggggttaaag tctgtatttg atttgatgaa atctaacttt tatattttct ttttatattc 84480
   atcatatttt attttcaacc tatcttggat aagcatgaaa ataggtctga tagtcataag 84540
   gctatcacta cacttcagct ttaagaagtt gttaggttta tgaggacttg taataaaact 84600
   ccttaatgaa ttaaaagatg tggaatatat gtaatagttt ttttttcata ttgtcttaat 84660
   ttgggttgta ccacaagtag accctgagaa aggattctaa cacaagtata gtttattgag 84720
   gaagtaaccc caggcaatac ctaggagagg agaaatgaga cagggtaggg aaagaattcg 84780
   gtgaaaagta tgttttcaaa tgagttgcca ttatggacaa ctggtgctca atcctgctgg 84840
   ggaactctag gagacattgt agaacatgcc cctgaattgt cccaagtgaa aggcaagaaa 84900
   gctggtgtaa ccagtagctc tctttctgcc atcagttaaa aactgcttcc agagtcatga 84960
   actatttgac actacggact ttccctgcac tagggctagg tgtgttccca tagtgagata 85020
   aaggccctct gggtaataag ttacagatgt ttgcagtaac cggcctttgc tgtgtagaga 85080
   gaggacatgc cttagttcag cactgtccaa aaaagtttaa tgtgagacac taacatgagc 85140
   tatatacatt gttttaattt tttcaggagc caattacgat gtaaaaagac acaagagaaa 85200
   ttaattttaa taatatattt cttaacccag tacatccaaa ataccatttt aacatgtaga 85260
   taacgtaaaa agtgttaatg agttatttca ctttttgtcc cagaactttg agatttggtg 85320
   tgtattttac aacgcatttc atatttcaac aaactagcca cttttcaagt gctcagcagc 85380
   tgccgggatg ggaggctggg ggtggtgtgt ggctactgta tcaaacagtg cagttgtagg 85440
   ggaatgcacc agtgtttgca ttgctattta ctggatttgg ggcatgtgtc ccaattaccc 85500
   tctgagctac actgaaggcc agctccaccc aatgcatcag agaaccagat acgtagatac 85560
   atttttggct caactaatgc tttactttct caactagatt gtgctcctct gatttctcaa 85620
   gacataaaac ctggaaaagg ttatcttcca catattttat agaatattaa tattagtatt 85680
   ttgaaaacta acattttcaa tgtcttcaca aatgcattgc tgctgttttt attagtatga 85740
   gtttgggatt taattagcac aaattcagtc ctcacttatt ctcttttttt ctcccttcac 85800
   ccagcaaata cttactgaac acttcctgtg ggccagcaca ggccctgtgg cccctcagtg 85860
   atgaaacaaa accctatctt catggagcta tgtttcagtg atggggaagg cagacagtaa 85920
   acagaatacc atatccatat ataagagcca tgaagaacag gttcttggca aattgtttct 85980
   gcaatgggtt gacaggtttc tgcaatgggt tgccatctta ctttaatcag atgccatatg 86040
   gaaaagcaag taagatggat agtggttatt ggttttaaaa gaggatttct cactttttgg 86100
   tgcatttcat ctcagtgttc ctttcattag agccttgatt tggattcttg ctttgaactc 86160
   atttacagga gactttttag gaactgaact gaactataaa atgagttgaa ctctgtctag 86220
   ttaggagggt tgtgtacaca cactgccgtg ttttgcatct ctgcatgcct aatttaaggg 86280
   cagctcacac ctcttcccca caggctgtca gatgcatggc tgaatcatgg tttgtgtcat 86340
   cttcatggca taaactttac agacctttga aaactagctg ccagcagggc aggtcaaaga 86400
   ctgtgtcttt gcatatctcc agtggagccc tatgaggcat ttatttgagt tgctaatgtg 86460
   agtgaggctg tgatctctct attaattaag tctgttttat tcattgggtg aaatgtgaaa 86520
   gcatttaata atttaaagac ttgatgtttt caaagtgtat ttgacttttg aggttcaaga 86580
   cactaaaaaa aggttaactt gaagcaatgg cacttgcaaa gattttactg cagctgattg 86640
   catatgcagg atatttctgg agtcatgtat tatgccagaa agattatttc agtaggattt 86700
   aactgcctga gtataataag tagagtgttt tattacataa taattgcctt gaatgggtgt 86760
   gggtatttca tttggctatt gaaatcatgc taatgtaact gtaattgaaa agattacatt 86820
   tactttttaa acatcttcgt taacacatca catttggtca gctgatgcaa tccttaaaat 86880
   catatgtttt attttcacag atttaatttc ctgagatata ttgcctcttg aaacttttta 86940
   aagacatttc ccgatagaca aaggtagttg agttcctttc tatacaactt attgaaactg 87000
   cctttgcaaa aattataaca gggagaaaat tgtgacagtg aaagaaatct gacctaacca 87060
   actccatctt gcttataacc tccaaactgt ccttgttcat tcctgggcac agccctaact 87120
   ttgggaggaa cttagtttat agtctaactt tgaaagaaac atgataacaa tgcttttctg 87180
   aaacaaaacc ccttcttgcc tatgaaccag actgcctttg taggactaac aaattaacga 87240
   caagattaga aattatggtt taggagtcat gcagctagag accacaagat tctaaacctc 87300
   cccaattgct cctagggata aaacatctct attgtcaaac ctaagactgg tgctcaagat 87360
   atttttcaga ccttgcactc aatggatcac ctggcaccac ccaaatcagt aaattacttc 87420
   atctggtctc atggccccac ccaggaatgg actcaacaga agaggacagt tttgatgccc 87480
   tgtaatttca tctctgaccc gaccaatcag tactctccac tccctggccc cctacccacc 87540
   aaatcatcct taagaaaccc cagtctccaa attctggggg agaccaattt aagtagcaat 87600
   aaggtgaatt aaagtttttc tttattgcaa ttcccctgtc ttgataaatc cacagttgta 87660
   ttaatatctg ggcagtgggc aaaatgaact cactgggcag ttacattatt tcctaatttt 87720
   cagtggttac caacaaagta aaaactgatg ctaactatct taagcaaaat gaaactttct 87780
   ggaaagatat gggaagggga gaattgattg gagtcttaag tatcaaactt tgcaaaaagg 87840
   catcttaggt aggagccctt ggatgcagac ctgagatgga gattctttgt gcaaatgact 87900
   tattgaggga atgctctctg gagaaagctg taaaagagtg agggaaactg gataggcagg 87960
   aggagaagcc aagcacaaaa taagggctca ggtgaagtct agcctcagct tgatccatga 88020
   ggagctctgg agcttaaatg ccatcagaga tattctggta ttctgctttg tctctctggt 88080
   cttcctccag tgttctttct tatacaacag tctacagatg agcacctatt tgggattgtg 88140
   ttttcagtag gctttggcaa aacataagaa atatattttg ctctttgtta gctttttgat 88200
   taggatttcc ctttatgggc tatcacattt catacaatga tgggattagg agagggatat 88260
   gagtatgagt gattgtgtgt gtatgtataa tgtccttctt ggtaacataa gaagtggaaa 88320
   attgtatgtt aattggcccc aagaattaag aaaaaaatac tcctctgtga aactctatat 88380
   acactcctgc attcttactt ggcattatta atgtgaagct taaagaatag gaatttatca 88440
   tatagttcat ggtcatagct gttaatatag acccaacctc aaacaataca ttatactata 88500
   tgctcgagta gcatggcctt ttttgatgat aataaatctt aatcaaaaaa acaaatgcaa 88560
   actttcttga gtagggtatt gcacttttat aagttacata gtgtaaacat acttgattct 88620
   ttactttccc actgtaacaa tagattattg ctatgtaaca cacaacttca aaatttagca 88680
   atttagaaca agcatttatg tcacttattg gtctccaggt cgtctgggga attattgtgc 88740
   atctaggctg agctaggttg ctgccagctg gttggctcat gtgtgtgagg tctgctggag 88800
   atcagcttgg ggttggctgg tctgggttgc cctcttctag gatggctgga acttctagga 88860
   taactgaggc cactttctgt gtagtctgtc atattgcaga agacttgttc tcatgccagt 88920
   gacagggttc caagtgaggg tggaagctgc aagccttctt gaagcctagc ctcagaactt 88980
   ttggcgtgct tctgcctcct tcaattggtc aaagcaagtc acatagccaa gcctagattc 89040
   aagtcatggg gacaacagct ccacctcttg atgagagggg ttgtgaagtc acattgcaag 89100
   ggcttggata caaggaagga tggcaaaatg tggtcatttt tacagtctgc ctcacgctga 89160
   ttccccatat taaatcagta attcttacat attttatcat tcaatttcct tagtcttatt 89220
   tttaaatttt ttaaattgat aagtacatat ttatgggcta tgtgtgatat tttgatgcat 89280
   atatacaatg tgtaatgatc aaatcagagt aataggatat ataccacctc aaaaatttat 89340
   catatcgtta aatctacctc ttaccactgc taatccaagc caccattatc tctcccctgg 89400
   tagaccacag taactccgta acttatctcc ctacttccac tcttggccat ccctaatccg 89460
   ttcttcacac agcaggcatg atgatttttt gaaaatataa ataagttttt tcctcttgct 89520
   cttagaataa atctatgctc ctttctgagg tctaagagtt gccacagtga tctgcccctt 89580
   gcccagttct agccttacct gtgtccttag aagtcctgga acctatcaaa ccccttctgt 89640
   ttcaaggact ttgtgattgc ttgaccctgc ctctggcttg tggcttctgc aacttttcat 89700
   atgatttatt tcttttcata caaccacatc ttccttagtc acctatgtca agcaggactc 89760
   ccctgattac tttccgctgt gtagctttgt gtacttcctt cataatactg ttcagtttgt 89820
   taatttacac gttattgcca atctcttcca ccagatgtgt gcttcataaa gtcaaagaca 89880
   atatctatct cacgaggttt ggtcatttac aaaaaagaaa aacacagcgt ctgattatgc 89940
   acatcattgc acatattagt tgctaagtaa atgtttgttg atgtcttaca gagcagactc 90000
   cctacatgaa ttttttacta aaaacaaaac aggaatcctt gtaacctttc cttttaaaat 90060
   gggtttttta tttccatatt ttcatagcaa catacccagt tacacatcat aagtattaat 90120
   catgacaaat gatcttttgt tatggaaata gaattgatat ggtccaagga attaaaggta 90180
   gatgcctttg atgctgtgta tatagtgatg actcttacaa aagaaatgta ttagtccatt 90240
   gcttacttga tacaacgtaa gcaaacattt tcagaaaggg gtctttgcta gataaaggcc 90300
   tcagtgttca gagcctcgtt ttccaaatgg agaagaaagt actataaaat tgaattttat 90360
   tcatgcttca gagagaaatg aaatctctcc atcatgaaaa atgggtatca ggtgcttttg 90420
   atttcctttt atcagctgct aatcagaaga gatgaggaga gagaagcaag ctaatgaaga 90480
   aaccagtcgt ttcaaagaca gttgtattgt aggaatcttt gtgattgtta attagatgat 90540
   ggccttggga aatacattgt gagggagaga aatggaggag gaatttgaat attgcaaagc 90600
   ctctcccgag atggaaagca tctaaatgtg tttgtgttaa aatattttga gtcataagac 90660
   tacattttga ataagacata gtgtttcaaa gacactagag gaccttttac ctcatagatt 90720
   ctgtatacat tagtaatgat ttatatctat acagtagtaa ttattcatat aatctcttag 90780
   catgactaat ttggaaacac attgcagtgc acatttgtaa ttttgggaag ctaccaaata 90840
   tctacaatct cttttctagc tgggagaact tgttttggga gactgcagaa gccaaaaatg 90900
   ccatacactg tcaactttct tggtagctag ggcacagaca tgtggcctgg gcttgggaaa 90960
   tcagataaac ctactctaga ctataatgta aagaagtgga gcaggagaga atccatttta 91020
   ttggtggagg tggtatcaat tgggaccata gggatggtag gaactatcat cagtactcat 91080
   ggtggttaca gtgtaagcga tcatggccct taatagcaca tatgtatcct cattgggcag 91140
   gttggggtgg aattctagtc atagaatcta ctgctgctgt agcttctttt ggtccttcct 91200
   tattctttag gcaacactgt gagctatctg ttatcctttc aataaatttc ataaattact 91260
   ttattcttcc agaaagcaag gtaacttttg gttgcttata cataaaaacc atgactaact 91320
   cactccaaca gaaaatgcta accattttga gtaccatata atgagtagtt gtatttcgtc 91380
   aaatacatag tgctttatac aattatgttg cccagtacca cccaaatatt gagaggattt 91440
   atttagaagg atttaaaagt tttgttgcat tggctttcca attcttcttt tattaattta 91500
   aaaaatcatt actataattt taaaattaga gcctccttcg tcaatgttat gcttcatgct 91560
   gtctgtttaa aatgatgtca cttttatctt aaagcagagt tgtgttaaat agaattagac 91620
   tatttaacaa tatttctgtt gagtctgatc atactgcttg tacgtgttag gaggtgtcag 91680
   ggaggcaaac atttgctgcc accaagagag tagtaatttc cctctggtag aatgtttagg 91740
   ccataaatga aaaagggtga tgcactcaat aactgaagat ctatgtggct caacaacaat 91800
   ttatttactt aaaatatttt attggatatt tactaaacag ccaggatcta taaaacactg 91860
   agaagtcaga actgaataat gtttggataa tttgtcttaa tgaaatgcag cttttaagag 91920
   ctgataacgt aaggcagtga cgctcaaaat ctggcctgag gacgtgtgcc atctgcaaac 91980
   tgagtgttac tggtcagcaa ggagatatgg aacttgggct accatgtaaa tcattcaaca 92040
   ccctgtccag tccagttaaa ctgtagtcca gctgatgctt ctctcacata agactttcct 92100
   gatgaaggaa gcaatgaatt gatttacatc cttgtgcgga ttccttgtct tgtcatggat 92160
   cagaacaaac agtttgtata ctggctactt tttgtagcaa taatttagtg ggtaataaat 92220
   attataaaag gcaggaaata catgtgatta catcaatcta tggaacagca ataatatgac 92280
   ttcccattaa cattcatttc aaaacaagca tttaaattag tacttatttc agaaccctca 92340
   tttatcgtct gctttccatg agtgagccct ggacaggtgc tatgtacaaa acagttttct 92400
   gtattttgtc agatcttaaa taatcattgt catcaggatt tttcaacctc ataagtggga 92460
   aaaggcataa aaccaattgt atacattcaa atatcagata tgttttataa gaattataga 92520
   ttattagaaa gttctgtctc tatattattt taaatgaata ctggataagt tgaaggttat 92580
   atttttatct gaattgtgtg tgtgtgaatt atgtatgtga gttggtagct ctcaatggat 92640
   atttcaaaaa ttaattactg ccaagtgaga actagtaagt atttgaaata ataatgtcaa 92700
   tgataataac aatagtaaag taagatactg agtttttttg tgcacagcta tggggttatt 92760
   tcttaattgc tttcatatta ttcatacaaa taaagcacca gatctaaaaa actaaaatat 92820
   gtttttcaat cacaataaat ttaaactaaa gataaccaaa ctaacctgcg gtaatggagg 92880
   ccagaagagg agttaagttt ggaggatact ggcaggaaca gggcacgaga gaaacttctg 92940
   aggtgctgga aatgttctag ggctgtgctg ttcaacatgg cagccactag ccactgtggc 93000
   tatttgaaat tgaattaatt aaataagttt ctcattcaca ttaatgagat ttgaggtgct 93060
   cataacactg tggctaatgg ctaatgaatt agacagtgca taattatgtt tttttatgga 93120
   acatttccag cattgcagaa aattctattg gatggcacag ctttagatct tggtctgagt 93180
   tgttatgtgg gtgtccaaat atgccaatat tcattgagcc atacacttaa gatttgtgca 93240
   ctttactgta gtatgttgta tatattgtag taagccatat gtcaatttga agagtacatt 93300
   tttaaaaagt tagagatttg gatcagatag ttgtagatat gtggtgttat ttctgagggc 93360
   tctgttctgt tccattgatc tatatctctg ttttggtact agtaccatgc tgttttggtt 93420
   actgtagcct tgtagtatag tttgaagtca ggtagcatga tgcctccagc tttgttcttt 93480
   tggcttagga ctgacttggc gatgcatgct cttttttggt tccatatgaa ctttaaagta 93540
   gtttttttcc aattctgtga aggaagtcat tggtagcttg atggggatgg cattgaatct 93600
   ataaattacc ttgggcagta tggccatttt catgatattg attcttccta cccatgagca 93660
   tggaatgttc ttccatttat ttgtatcctc ttttatttca ttgagcagtg gtttgtagtt 93720
   ctccttgaag aggtccttca cgtcccttgt aagttggatt cctaggtatt ttattctctt 93780
   tgaagcaatt gtgaatggga gttcactcat gatttggctc tctgtttatc tgttattggt 93840
   gtataagaat acttgtgatt tttgtgcatt gattttgtat cctgggactt tgctgaagtt 93900
   gcttatcagc ttaaggagat tttgggctga gacgatgggg ttttctagat atacaatcat 93960
   gtcatctgca aacagggaca atttgacttc ctcttttcct aattgaatac cctttatttc 94020
   cttctcctgc ctaattgccc tggccagaac ttccaacact atgttgaata ggagtggtga 94080
   gagagggcat ccctgtcttg tgccagtttt caaagggaat gcttccagtt tttgcccatt 94140
   cagtatgata ttggctgtgg gtttttcata gatagctctt attattttga aatacgtccc 94200
   atcaatacct aatttattga gagtttttag catgaaggtt tgttgaattt tgtcaaaggc 94260
   cttttctgca tctattgaga taatcatgtg gtttttgtct ttggctctgt ttatatgctg 94320
   gattacattt attgatttgc gtatattgaa ccagccttgc atcccaggga tgaagcacac 94380
   ttgatcgtgg tggataagct ttttgatgtg ctgctggatt cggtttgcca gtattttact 94440
   gaggattttt gcatcaatgt tcatcaagga tattggtcta agattctctt ttttggttgt 94500
   gtctctgccc ggctttggta tcaggatgat gctggcctca taaaatgagt tagggaggat 94560
   tccctctttt tctattgact ggaatagttt cagaaggaat ggtaccagtt cctccttgtg 94620
   cctctggtag aattcggctg tgaatccatc tggtcctgga ctctttttag ttggtaagct 94680
   attgattatt gccacaattt cagctcctgt tattggtcta ttcagagagt caacttcttc 94740
   ctggtttagt cttgggaggg tgtacgtgtc gaggaattta tccatttctt ctagattttc 94800
   tagttttttt gcgtagaggt gtttgtagta ttctctgatg gtaatttgta ttcctgtggg 94860
   atcggtggtg atatcccctt taccattttt tattgcgtct atttgattct tctctctttt 94920
   ctgctttgtt aatcttgcta gtggtctatc agttttgttg atcctttcaa aaaaccagct 94980
   cctggattca tcaatttttt gaagggtttt tcgtgtctct atttccttca gttctgctct 95040
   gattttagtt atttcttgca ttctgctagc ttttgaatgt gtttgctctt gcttttggta 95100
   ctgttggctt ttctggttca gctcaaggaa tgtctgtgcg gttcctgtgg cagccactag 95160
   ccactgtggc tatttgaaat tgaattaatt aaataagttt ctcattcaca ttaatgagat 95220
   ttgaggtgct cataacactg tggctaatgg ctaatgaatt agtgcataat tatctttttt 95280
   tatggaacat ttccagcatt gcagaaaatt ctattggatg gcacggcttt agatcttggt 95340
   ctgagttgtt gttacatggg tgtccaaata tgccaatatt cattgagcca tacacttaag 95400
   atttgtgcac tttactgtag tatgttgtat atattgtagt aagccatatg tcaatttgaa 95460
   gaatacattt ttaaaaagtt agagatttgg tactgttggc ttttctggtt cagctcgagg 95520
   aatgtctgtg cggtttctgg acaagaaagc ctgggcaggg atgtaggtga tagagaaaag 95580
   atgcgttcca agacctgcac tgccatgatg gaggcagccc tcaatgggaa agcgccttaa 95640
   gagacaggtg tccagggtgt gtggggcagg gcagggctat ggctgtatga agtctatcag 95700
   ccaaaaggga agtgtctggg aagtttgtcc tttggataac aaataaaagg ctatctcagt 95760
   ctttggaaga tcattaccag ataagagaag attttattct ctgtatggga gatgagaaag 95820
   ttttgggaac atttttattt aacattgggt tttctctgag tcatttttta aaactttctt 95880
   ctctttgatg catatcctct cttgattact gatttttagg ggagaaaaag ctgaggccac 95940
   aagtactgag aaaacaatat atattccacc aaactggatt catatttctc ttttttttta 96000
   gttgttacat tggcattccc ccttctttta ttatctcatt tttcttttca gatttagagg 96060
   atttaggcag aattaactca tttttctgtt cttcattcat ttaaaacgtt actacataaa 96120
   atgtgatcca tggagtggca gcattggtat caccaaaagc atgttaaaat gcagattttt 96180
   gggtcctacc ccagacctac aaagctagaa cctgcctttc accaaggcat tcatgtacac 96240
   attacaggta aagaggcact agtgtagaag tgtaagatca acgcatgaaa ataatataga 96300
   gtacccagaa tattgcaaca ctaataaatt aggttaatca ttttgtaacc cattcattag 96360
   ccaagatgag ttataggtat taaattgacc acctattatt tgcttaattt aatagctgta 96420
   aaggattttt tcctttttgt ggcccttttc atttcacaaa ctgagtcacc aatgtcacag 96480
   ttctacaagc ttgttctaag aggatgaact gtgacattgg tgatatgtcg aatgtgattc 96540
   atatattaaa agcaaaaatt gctttttgat gtggtgtcac tcacagcaaa aatgacatta 96600
   aaaaaagata aatgcaccat tttcagcttt tgactaaatg atggcaattt agtaagaaat 96660
   aacttgatac tgacaacttt caattaatcg ctaagattga aagaagaaaa agaatgaatg 96720
   agccccaatg tcaggtaatt aatattactt atagttagct ttgtaatata tgttctgttt 96780
   acttttgact ggggatggct tcatctcaaa tcaaatacta agaacatact ctgaaaatgg 96840
   gacttggaaa gagtgaagtg tctgtctact gtcctctctc tatccccatt ctcctgttgt 96900
   taaaattttt tcagcagttt ataccatggg caacctaaac cactcatttc ccaagatgct 96960
   tctcagtata gttaaaatga gctgattttg ctgaaaccac agagcaggaa ccaaatcata 97020
   gtagcatgaa aaacaaaact tgaaatttga gagtgatgtg gaaacaaaac aagaaaccat 97080
   gctgagggct gtgggtgtgt caaccattac cccaaaggca agaggagata ggctgaagtc 97140
   acagcatgca tgaccttgag ctggtgtgga acatcattca agctggtggt tgaccacaca 97200
   catctcattc atgtgaagtt ctgctgtgat gctgtttctt cagggagata atcccaaggg 97260
   tctgccaatt gtctattcaa tccacaatcc cccatttagt caataattgc tgatgtttat 97320
   tccaagctct tactttgtta atattttaac tcatttatcc acttactaaa cttattttaa 97380
   aggtctgtga agactgggaa tgccaatgtc atcagaaaag tctttgttta taatgagctt 97440
   aaatgatcta gctcttacat taggagcaca agccagtttt gccagttgaa tgagctatta 97500
   ggcataaatt atttgttcta gaagaagcca tattgcttac ttgggttttt gtttatactt 97560
   aatgaactga tgtttcagga ctttgtatta atctttaggc tctgtattaa tttcctgtga 97620
   ccactgtaac caattacctc aaacatggtg gcttaaaaca gttgacatct attctctcag 97680
   tctgggggcc agaaatctga agttattatc aatgacctaa aaataaggta tctacaggga 97740
   cccacttcct ccagaggcca taggagacca tctgttcctt gcttcttcca gcttctggtg 97800
   gattccagca ttctttggct tgtggccaca tcctgcagtc ttcaaggcca gcatcttcaa 97860
   atccctctct tcacattgct atctcctctg tgtgtcattt caccctgtct ctctgttgta 97920
   aggatatata tgatggtatt tagggtacac ctggataatc caggattatc tccacatctc 97980
   aagatcctga gcttaatcac acttgcaaag tttttgccat gtaaggtaaa acttatgcgt 98040
   tccaggaatg aggacgtgga tatcttttga ggggccattt tcagctcacc acagacttca 98100
   aagaattagg gcatttccct tgaaatattt acttactctt taagaataga tattgtttct 98160
   agcacattgt tgttccacaa agcatcatgg accctaccca acatagaaat gtcactgata 98220
   actagcaaaa aatatgagca aatcataagc acaatctcag tattccaacg tctatttctg 98280
   aaggggcctt gcacctctga tacacttcct aaagatcccc ttttctgcac tgggatgcat 98340
   tttggtttag gagtcactga caggcattac ctaagagtct gatacaagtt agttcagttt 98400
   aagccattta gatttagggc ttttcagggt tgtttttgtt tgtttgtttg ttttgttttg 98460
   ttttgtttcg tttgtttttt tttggcttgt ttgcttgttt tgagacagag tcttgctcta 98520
   tctgtcaccc atgctggagt gcagtggcac aataatggct cacttcagat tcaacctcct 98580
   gggctcaagt gattctccca tctcagcttc ccaggtagct tgggctacag gtgttcaaga 98640
   ccatgcctgg ctaatttttt tattattttc gtagagtcga tgtctccctg tgttacccag 98700
   gctggtctca aactcctggg ctcaagcgat cctcctgcct cagcttccca aggcgctggg 98760
   ataacaggca taagccacca tgcctggcca gttttttata ctttattagt cacacaggta 98820
   gggagcctac atgactaaag tccattctcc agcagactat gtctcataaa ttccagctaa 98880
   ttacctgaag caatcaaggc agaccaggaa taacctgcta aaaccttaga gaagtttctg 98940
   ggttatcgtc ctgttagtaa atcccatcac agtgtttaca agatctgttc ttgtctaggt 99000
   caatgcatcc tcagggacat cccaaggagg gaaagaacaa gtgacagatc cactgttaat 99060
   tctttttcct acacgtgaat tatgttttag gcatgtttga ggaatgtttg cttatgactg 99120
   tacccagata ttggtggaac ttttgaggcc ttaccatttg gagatcaagt atgtagataa 99180
   atccagataa ccaatttttt ttaggtccat cctgaaatga tagagagaga gttttctgca 99240
   acaggaatat agggaaaaca gcagaaatga aagaggacta tgtaacagca aaggttgtga 99300
   gtggaatgtc tgcagtttga acagaaattg gtatatttgc ttatactgaa gatgcctaag 99360
   ggacttcagc taagagagtg atcatcagtg tgtataattt atttatagga tatttagatg 99420
   cttttattgc acaagattaa aacaattaaa gcccacttcc atctgattaa tgagaaagtg 99480
   acttgggtga aataatgggg ctcttatttt tattacaggc cgatctgctt cagtttcata 99540
   gttaaccttt gtatggagac aataataaaa gtaagacaat tttctttcac tttttatttg 99600
   gcttttgcat agaagaagca gtgtacactt ggcacctgat atttacagtg ccatctgtct 99660
   gttgattttc ttgctgtgca cagattcaca gtaacattgg ggtgggggga ggatccttat 99720
   ttgaatcttc tctaagaagg tcagctctgt gtggcacctg gatacccttg ggtggtgagg 99780
   gttggatagc acatgacctt attcagctct ccctgctctg tgccttaaga aaatctgttc 99840
   accttgggac caccgttagc gtccaacctc catttatctt tctaaggtag ggtttaaagt 99900
   aaggacacaa aggcaagatc cctgagtagc tacttccaag ggtagcctga tatttttcca 99960
   agtattgctt tcaagacagg aaagagggag gagttttttt ttttctctgc tagatcattg 100020
   ggatccttct tagtgttgct tgaagaagtg gatgtatcaa agattccatg caagacctcg 100080
   agagatcccc aggtcttaac taagttagtt accttatcac ttccctaaca aagacctttg 100140
   cccttagcaa ctgtcctcat gatagagcct aacattttct gaagcacgtt agggttttta 100200
   aatcagtgtt acagatgttg tcacattttc tcctcagaat aaccctctga agtagtctgg 100260
   acaggtatta atctttaatt tatagatgaa aaaattgagg ctaaaagggg tcaagtagct 100320
   tgccaaagtc atatcattct taaccaagag agtggagact tgagctcatt cctccgaaag 100380
   cttaatccaa aagcaatatt gagctcattc ctccgaaagc ttattccaaa agcgttattg 100440
   ctaaccgtgc tagacatact tagattcctc ccgggactgt gtgtataatg taattgagtg 100500
   agattggctg ggataagaaa aataacggtg ggctcaaggg taatctaaag gagtgtgcac 100560
   aattcattaa ggtattggat tgtcttgttc ccttcttatt tcccttcttc ttgtgtttct 100620
   tccctctttt cttcctttct ctctctattc tctgtaagca aacggaagct ttactgggtc 100680
   tcatggagcc ataaatttgt cactcctgtc tttcaacttt gcagacaatt taaaaagtaa 100740
   agtgcacaaa tcttgtaaat tataattgtc cagcagatga ctttgcatag ataaggttaa 100800
   cttagaggca attgatgggg tagagtttga cagtgcattc tgtgataggt atgtctaagg 100860
   tgacagagaa aagattttga tgggtcagcc caagactttg tgttggttca gttgcagaag 100920
   gctgagctgt atggcacctg agagaccatc tagttaaacc ctttcatttc caatcatttc 100980
   agagagcatg tgtgccctat cagttatcta ctgctgcagt catgctgcat aacaaacaac 101040
   tacaaaacct cagaggcata ttgccatgaa tgtttattgc tcattgatct tgaatcagct 101100
   cttatgatct tggcttagct aggttcactt gcatttctgt acttggctgg ctgttggctg 101160
   attgaggctg gcctccgctc aaaagactgg ggcaactcga atctgctctg tctctcatct 101220
   tccagcaggc tagccaaggt acgttctcaa gttcagggca gtggtgcaag agcacaagag 101280
   aaaacacata ggtctctcaa gatgtagtct tagaggacaa atacgttgct cagtctgttg 101340
   gccaaagcaa gtcacacggg gtagagaagt ataactacct tttggggagc cactgcaatg 101400
   ttacatggca aaaggtatag gtaaaaggag ggtgaatgat ggaggcatta ccaaatctac 101460
   aggttaccta gcaggttagg gatagcaaaa agacagagcc aggcctctta ttccacctcc 101520
   acacaaatgc ctttcacatc atttctagag aataccaatt tgtcttagac atgccccact 101580
   ccccaaataa gcccctggta tttagagcct tcacattgct gccaggctcc cttctaatcc 101640
   ttcggttgtg cgagcacctt gttctctgcc agcagtcaac taatccctgc caaggtatta 101700
   acacttaatt aaggtgtttg ctgttaaagt ccagtagtgg ctccgacatt tttccaccaa 101760
   agaacgcttt taattccttt tccctataga ctccacattt ggaaatttct ttgtaccaga 101820
   gttcttaata attatttctc cctctaaaat atgaaatatg tgcaactgcc aaatatagct 101880
   tctggttggc atgagatact gaactgagca tgaaaagcta gacagaagca aagactttga 101940
   ctgcttcagt gacgacctgt gttttcatgg gtcgatgtcc aagtttgatt aggccttttg 102000
   accctcactt ttatcttaac tgattccaaa ttgagaatca tggtgagaag atatgcttct 102060
   atcttgagca ttttcttgta actggacttt tctgagaaca aaacatactt cccttctatc 102120
   ctccagtcag tcttgtattc tctcaaatta tgcttggaaa attattgatt attcaaaaaa 102180
   gtttgattat tcagtcacac tcaggtcact aatctgaccc ctgagaaaaa gttggggcag 102240
   aggtacaggt tcatatcgaa aattgatgtc atttagcttt tggaaaagac tatttaaaaa 102300
   tcaaaagaaa gtatagcata aattaaaaat ggcactgtcc cttgggcagg taatttagtg 102360
   tacctgtttc tgaatttctg gtgatttgaa attcaactcc cataacataa actaaggatg 102420
   atattagaag agccagcaat ttcagtatag taatatattt cacaaacatg agttgcactc 102480
   caaatgcatg caagtttctt gttatctctg tataacaaga gagattaaat acagagatca 102540
   ttttacgcag ctggaatgtt tggttatttc tatagctcta gccaagtaga gaagtcagtg 102600
   agcacttaga aaatggtttc aagctgtgat ttaaaaaaaa aaaaaagaaa gcaaagaaag 102660
   aacttgtgtt gattcaactg cagaaagaaa aatgtttcac tgtggtgtgg catcatgata 102720
   tttgagagac tttgcatata ttacagagta ttgtaaatac tggataaagt acctgaagca 102780
   tttgtgaaag gaagaataat ccctgtgtgc tttttaaatt tatcattcac ttattaaata 102840
   cagtagagga gttagagcag ttgtgtctag aagaactttc agcattaatg gaaatgttta 102900
   tatctgcaca gaccagacag tagccattag cctcacgtgg ttatcaagca cttgaaatgt 102960
   tactattgca gctaaaaaac tgaatgttta ctctccttaa aaaaatgaac agcattattg 103020
   cattgtaact gacacacaca cacacacaca cacacaaact gcccatattt aaagtatgca 103080
   atttgataca tgttgacata tgtatacaac catgaagtca tcaatttaat caagatagca 103140
   aacatctcca tcactctgaa gttttattac agccttttaa aatttctctc atgccttttc 103200
   ctgtctgccc aacctctgtc cccaggaaac cactgatctt ctttctgaca ctgtatgtta 103260
   agttgcattt cctagaattt tatataaatg aaatcgttca gtatgttttt ttcctctggc 103320
   ttccttaact cagcataatt atgctaaggt tcatacatgt tgtagcatgt gtcaatattt 103380
   tattctattt attgttaaac agtactctat agtatgaata tagcacaagt tgtttataca 103440
   ttcacctgtt gatggacatt tgtgtggttt ccactttttg gctattacaa ataaggttat 103500
   tataaatgtg tatataagcc tttgtatggg aatatgcttt tatttctctg gtgtaaatac 103560
   ctaggcatga aatggctgga tcatatagta ggcatatgtt taacattatt aaaagctgcc 103620
   aaactgtttt ccaaagtggg ttatacaatt ttccattccc accgagaatg tgtaagagtt 103680
   ccagttcctc cacattgttt ttttgtttgt ttgtttgttt gttttttgtt tttgagaccg 103740
   agtctcactc tctgtaacca ggctggagta cagttgtgcg atctcggctc actgcaacct 103800
   ccacctccca ggttcaagca attctcctgc ctcagcctcc caagtagctg ggactacagg 103860
   cacgtgccac cacacccagc taatttttgt atttttagta aagatggggt ttcaccatgt 103920
   tggccagggt ggtctcgatc tcttgacctc atgatctgcc cacctcggcc tcccaaagtg 103980
   ctgggattat aggcatgagc caccgtgccc agccccagtt cctccacatt cttactaaca 104040
   cttggtatgg ttaatctttt aaattatagc aattctaata agaacacagt gatactctat 104100
   tttgttttca ttttacattt tcctaatgac taatgatatg gaaaatattt tatgtgtata 104160
   gttgtcatct ctaaatattc atgaagggct attcattaaa ggcttttcaa aatcttttgc 104220
   ccatttttta gttgggttgc ttgctttttt ttaattatta ctaattacta ctgcaattaa 104280
   aacattttct ctgtatttat tctatcagtt attgagatag cattaaatct tcaataataa 104340
   ttgcatattt gtctagttct ccttgtagtt ctgtcagctt ttgctttatg tattttgaaa 104400
   ctctgttttt aggcatataa acatttagga ttgttcagtc ttgatgaatt ctctctttta 104460
   ttgttatgaa atttatcctt ggtaatattg tttgctctga aatctatgtt ttatgatatt 104520
   aataagcatt ccaactttct tttggttagt tctagcctat ccttttccat tgtttaattc 104580
   aatttatttg tgtctatcta tacactgtgt tccttagagg cagcacagag ttgagtcttg 104640
   cttttttatc caatctgata tctctgtctt ttaatggggt gtatagacca tttacattta 104700
   atgtgattat tgatatggtt cagtttatgt ccatcacctt gctatgtctt ttctatttgt 104760
   tccatcaggc tttttttttt gtctcaaaat atctttattt cactttcaat gttgtaatat 104820
   atttttgttg gacatagaac tctagattga tgggtttttt ttctttcagt aaagatttag 104880
   ctctactgtc ttcctatttg cactgtttct aataagaaat ctgatggcaa ctttattttt 104940
   ctctgttcat atattttccc ccactggctt cttctgagac tttcttatta tcactgtttt 105000
   tgagcaatat aattatgatg tgttatagtg tcattttctt catgtttctt gtgcctggag 105060
   ttgtgaacat aaaataacat ggagaccaga cttcagaatt ctctgaacag acaaagccaa 105120
   tttggccaca taagcgaaac ttaaagttat ttcttgtaaa cacttctgat catcgtaaac 105180
   caatcttaag ttgccttcta aagatggtat aagatgagca ctttcaacca atcctctgtg 105240
   atcttgaaac cttcatgata ataacccatc attttaaagg ttaaacaact tccccatttt 105300
   tgctgtgtaa gccagcctgt aacatcatgc ctctgagctt cctcttatga ctgggcttga 105360
   ggtcacttag ttcatgaact attattttat atgcacaata aacttaaaaa atttttaatt 105420
   tgatctgatt ttatttttga caggattcac tgggcttgtt aaatctttag atacattatt 105480
   ttcattaagt ttggaaattt attcaaccat tatatcttca aatgcctctt ttgtcccttc 105540
   ctttgtatcc tcttcactag aaaatccact gacacttaaa ttaggctact tgaagttgtt 105600
   ccacaggttg gtcatatgtt gttcatttat tttagatcat ttccctatgt gtttcatttt 105660
   aaattgtttc tattggcctg gcgcagttgc tcatgcctgt aatcccagag ttttgggatg 105720
   ccgaggtggg tggatcagct gaggtcggga gttcaagacc aggctgacca acatagagaa 105780
   accccatctc tactaaaaat acaaaattag ctgggggtga gggtgcatgc ctgtaatccc 105840
   agctactcgg gaggctgagg caggagaatc acttgaacct gggagttgga agttgtggtg 105900
   agccaagatc atgccattgc actccagcct aggaaacaag agcaaaactc catctcaaaa 105960
   aaaaaaaaaa aatagattgt ttctattgct atgtctcaaa gttgtatttt tagtagagac 106020
   gggtttttgt catgttggct gggctggtct tgaactactc ctgaccttag gtgatccacc 106080
   tacctcagcc tcccaaagtg ctgggattac aggcatgatc cactgcgcct ggccagtggt 106140
   ggatattttt atagtcctat caatattctt gagctttgtt ttgagacaca gttaagttac 106200
   atggaagctg ttttttgttg tttttgtttc tattttttgt ttttttagtc ttgcttgaag 106260
   atttcttcag tgcaactagg gcagcgctca atctggggta attattccca tctattttgg 106320
   caagaccttc ctgagtactt tacctaacgt ccacggatct tcggcctttc cagtcttgct 106380
   gatgaaaaca gaaaccattc ctggccttgt gtgaaggttg gccatggtaa cctctaaacg 106440
   ttttaggtgg ttctttcctc agcctcaagt agtttcctca catgcttgaa ttgatcacta 106500
   ctcagtcgaa tacttgaggg agacattgta caaatctctg attctctgga attttttatt 106560
   ttttcctttc actcctgcct cactttcccc ctaccccagt ctctctccag ctctcccctc 106620
   tggtactccg tctggaaaac tccagctcct tttgtctctt tggtctctta gctcagtcaa 106680
   caaaactcag ggaactgatc ttttcctggg ttccccctcc ctgcaccaca tcctggaaat 106740
   tctttcttag gccttgattt ttttaatatg aaaaaatgaa agtgtctaca accaattggt 106800
   agggttattt taaagaatga gatctgatct ttaaagcaca tagctagtaa atattcaata 106860
   aatgaaaaat ccttttcctg tacatagcat tatgtattct gtatactttc ctcaagaata 106920
   attttgacaa atgacagttc attcaccact tctgagtcag ccacagggaa acctgtttct 106980
   cccttttgtt gttctggggc tcttgtatcc catagcatct tctctcacca gtatggttgt 107040
   cctagactgg ttacctgtct cttccttcgt ttctgcaaaa cacctctaat gctaggggaa 107100
   atatttgcta gacgacttta ttaagattta cagagagtag ttattacaca caatgagaga 107160
   aaacttttag atcagtgttg gttattaaca tttctgaaag catcagacat ttttagctac 107220
   agggaaattc tcactggctg tgtgaattat tagcaggtcc tggggttttt ctctgactcc 107280
   caccccgtgg cctgtgtgta tgtgtgtgtg tgtgtgtgtg tgcatgtgcg cgtgtgtgtc 107340
   tatcagtctt tttctctctt gctttcttct tgtatctttc tgtgtacata tttctctttt 107400
   gtttctcctt ctattcccta atgtcatttc tgtatctctg tctgttagtc tgtctctccc 107460
   tgtatgtatc ccttctctta tcattccctt tttgatttat ttaatttccc actcaccatt 107520
   accaaattca ttttactctt ccatggaggc catcctagct ctttgacttt ttccaaacag 107580
   acctttggcc tgcttttgca tatactgaca ctcaatatag agcttcttgt aaacaaagac 107640
   ccaaacattt ccccaggttt tcaactttcc taatacagaa agctgtcaaa aactttgaga 107700
   ttacaacttt tcttttagca catctgagat caacttcaag cccacagtag agttaacagg 107760
   ataaattcac ctattaccac aggaaatcta acagaagctt cctcaagaca ccttcttctc 107820
   tatgatggtc tttggaagga tttctacttc acattaaaag gagagcatcc tcatgttaat 107880
   gacccaacct taggaagcca tttaaaatgt gttaaaacca gtgtaggaac tctccacaca 107940
   gatcattgtg tttggacttt atttttaatt tttattttta cttttgtacc gtagtggtat 108000
   gcttctgagc agagaatcag gtctggagag atgggtagag aaacttcctg aattcttgcc 108060
   agtctttcaa atgaccagcc atcatggcag gaactaagac gagtaatgtt gtttaaaaag 108120
   agaaagaaag agccaggtgc ggtgtctcac acctctaatc ccagcacttt gggaggatga 108180
   ggcgggtgga tcacttgagg tcgggagttc aagaccagtc tggccaatat ggtgaaaccc 108240
   agtctctact aaaaacacaa aaattagttg ggtgtggtgg cacctgcttg taatctcagc 108300
   tacttaggag gctgaggcag gagaactgct tgaacctggg aggcagaggt tgcagtgaac 108360
   caagattgtg ccactgcact ccaggctagg tgacagagca agactctgtc aaaagataga 108420
   aagagagaaa aagagagaga gagaaagaga gagagaagaa agaaagaaag aaagaaagaa 108480
   agaaagagag attggtaact atgtgaggtg ttgaatatgc taattagctt gattatggta 108540
   attaattcac aatgtataca taaattatca attatacctc aataaagctg aataaaagta 108600
   atgtattatc tcatctcaac atcaatattc actgccttca aagaatttat cccagtgtgt 108660
   aattatgcat gcatttgttt aattgattac aaactatctt ccttactaaa ctattacttc 108720
   tgtgaggtca aaagaccatg tctgatttat gcataattca ctatccagtc cctagccagt 108780
   atctgccctt ttaaacacct gataaatatt taaatctagt tctgagaaag taggttcttc 108840
   cagacacaaa ttagtgtcca gtaaaataca aacaaagtgt tatgtttatc agctagttcc 108900
   aacagtattg gtaagagaga gaagttgatg tcttttccag attatgctac tgttttaggg 108960
   ttttaaataa tcaaagatga taattggtgt ctacgcaagc accatttctt ctcaaataag 109020
   ggagaattac aaatcttaga gctaactgga agaaagcatg tccagacaga ggtcttacta 109080
   ttttggggat tttgttgggg tatgattgaa gaggatggag tgagagaaag agcaaaacag 109140
   gagacagata ttgacagacc aatccaagat tagagaaccc ttgaactccc tatagccctt 109200
   gggtagacag ttgtgtagga aagggaagtg tgtgggtatg tatgtaccaa gtggaattat 109260
   cagagctatt ggaggaatca gatctacaag agagctcaca tgagtttatt ttatcacaaa 109320
   gacctggcag taataattat gctaatttaa acctggaagg atgtagacta aaatgtcaac 109380
   aatgtcttga gtactggaat tctgagtgat tttttttcta ctttgtgttt ttatgcattt 109440
   tcccaatttt tctataatga acacatgcat aatatatcat cagaaagttt taaaaatgca 109500
   taactttttc catttatgtt tggtacaaac tatttgtttc ataatctcct ggaatgcaaa 109560
   tgaaaattta ccctagaaag cacactttaa gtattactga gttatcaggt aatttggcag 109620
   ccttaatgct tgaaatattt tattgatgtt agagtactta agctgttaaa actttttttt 109680
   taacttaaac tttcagaaga gtgagttttt tccatttttg atgcagtact aatttttggc 109740
   tagaagtttt acagcttgac atgccacctg acctctgtac cttctatttg acctaatgta 109800
   tctatagatc atttaaatga gctcatttga ctcaagagaa aatccttaca tgtcaatttt 109860
   ccatgtaaat gaattctgtt ttaaagacta atactttcat tggaagctga ttataaaact 109920
   gtgctattaa ttttgacatt ttatattcac ttcagacttt ctatattatt atgttttatt 109980
   acccacgctg cagaatataa ataaatgtgt tttattattt gcttgggatt ttaactaagg 110040
   cttaatggat tttattctgg catactttaa tgtccccaca attcgtctac ttgcaaagta 110100
   ctttagttac aaatgagcca atttccatta acatacttcc tctacactgc aaagtatctg 110160
   ctttagaaca ggcatatttt aaaactaaaa taaaacccca actagttggt gtcagctttt 110220
   gtcttgctag tataatttct atcaacatgt tttagaaatt ttattaatct gttatatttg 110280
   aaatgtcatt gttttcctct ctggttattg ctattccaaa gatcctttaa ccatcacagt 110340
   aaatcacaga gttcctctag acttgaatgc ccaatacagg agccactaac cacatgtggc 110400
   tactgagaac ttgaaatgtg gctactccaa attgatttgt gctgaaagca taaaacacat 110460
   actggatttt aaagacctat tatgaaaaaa atgagtgaaa atgtgttact gatacatgct 110520
   gaaatggtga tattttggtt atattttgtt aaataaaatg cattattaaa attaaactca 110580
   cctctttatt tttcctttta atgcagctac tggaaaattt aaaattatat ttgtgactca 110640
   catttgatta taatgtgatt ataaaattgt tctattaatt ttgacatttt atattcactt 110700
   cagaccttct atattattat gttttatggt tcacattata tttctattag acagtgatac 110760
   tttagagctt gtttcttcac caaggggata tgctcaacca aaatgtgttg gtgaatgaag 110820
   cccccagtcc ctacatacat caagccttgt caagataata tccacccaag ctttctccca 110880
   ctatgtcagt gttcactgga cctcactgat gtttcttgct gaatatggca gcacaggaat 110940
   tttctcttat ctaacctctg gatttgcttt tgggaaacgt ttcctgttaa ccattggcaa 111000
   atgtgaatcc cagtcatttt tcattagata aggctgagtc cttatataat agatcaaggg 111060
   ttagcacagc aaaccagcat acaagaattt gaaatcagca atcagagctt agtgaaacag 111120
   aaaatcactt gcttgggcta atagtggttg cttgtgtctg caatttagtg gtgtatgtgt 111180
   atgccttcaa tccacaggag tcagcatgac atgtaagaac acaccgatat catcatagct 111240
   tctccagttt gttgtcaaga gctttctacc ttttacaaag agtcttcaga gcctgagaca 111300
   ctcgtaagcg tgcatatatt cttgcttttt tggtaaaggc ttgtgacgca gaagaaatga 111360
   taaataccta aattgaaagc aagggcttga cattcctaag tttcttatgt aactgagaga 111420
   attgtttaaa agcagaaggt tccacagaag tgtacttttt aaaaatttat ctgaaaacat 111480
   agcagtatag tagcactaat aagttgaaac atcctcttgt catggtaatt ttattgatga 111540
   aagctggcat ttagtttaaa aatagtaaat ataaaaacat caatttgaat tgtactactt 111600
   tttgttagag atataaaatg ttaatagctc ttaagtattt taatttatag gttgtcactt 111660
   ctaatatatt ttaatacaca cttcttatat atttaataaa aataatctta aatattttaa 111720
   aatctgctat catataaata actaattatt ttacatgtct atttttatga ctgtttttag 111780
   gagaacatga tttagcattt gtttgaaaaa ttctaattct cttttttttt tttcgagaca 111840
   attctctctc tctctctctc tttttttttt tttttttccc tgttgcccag gcttgagtgc 111900
   agtggcatgg tcacagctca ctgcaacctc cacctcccag gttcaagcaa tcctcccacc 111960
   tcagccacca gagtagctgg gactacaggc acacatcacc acacctggct ggtggtattt 112020
   ttgtatttct tgtagagaca gagttttgcc atgttgggca tgagtacaag aatatgtaaa 112080
   aaaaacaaaa aaacaaaaca cagcaaatct accacaggtt tcttctatct ttgagctcta 112140
   cctgcagtgg ggatagatat atgagttctt tctcacttca tggtccaaga ggactgctga 112200
   aactccagcc atcacatcaa ttttctaggc aagaatcagt agaagcaggg aagatcaaat 112260
   agggctcatt cagcctgccg tctcattcta atgagctttc cctaaagtct cactaaaacg 112320
   cttgtgcttg cattccattg accacctgtg ggtacaagag atactgagaa atatggtctt 112380
   ttatataata cctcgtgcct atataaaatc aggattctga tgctatggaa gtggatattg 112440
   agacaacaac cagcagtatc atacacatta atcaaaggta ccaactttca gtttgctctg 112500
   attgttggcc accattatca gacatcctaa cttttcaaaa gaaactacaa acagaagttt 112560
   tatgtgaaat ctcctgtatt ttaaatgtta gcatctcatt caaataaatt ttaaattttt 112620
   attatgaatt tttttttttt gagatggagt ttctctctgt cacccaggct ggagtgcagt 112680
   ggcacaattt tggctcactg caagctccac cccctgggtt cacaccattc tcctgcctca 112740
   gcctcccaag tagctgggac tacaggtgcc caccaccatg cccagctaat ttttttgtat 112800
   ttttatgaaa taattttagg cctacagaaa acttgcaaaa aaatggtata aatatttcca 112860
   tatgttcttc atccttaatg ttaacatatt gtataaccat aatacaatta cccaaaattg 112920
   gaattttaaa ttggcacaat actataaatg aagaattggc attattttgt agtaagccag 112980
   ataggaacta ttttaggtgt ctgtcactac gacttaattc cgctattgta gtgagaaatt 113040
   agtcatagac aatgcgtaaa caaatgaagg tatctgtgtt ccaataaaac tttatttaca 113100
   aaaaaaaaaa acagatctag gaaggatttg gccatgggct gtagtttgcc aactcctgct 113160
   gtaaactgaa cttatagacc ttgtttgatt tcatcagttt tctcattaac atcctttctc 113220
   tgttcagtcc aagatccaaa atgtatttag ttgttgtctc cttagtgtct tgcaaactga 113280
   gttatttttc cttcctcttt tgtgaatttg actcttttga agtgtactat tcagctattt 113340
   tgtaaaatat ccctcaattt ggattagtct gaaggcttct tgtgattaga ttgaggttat 113400
   ctataataca gatgtgtgca catgcatggg ttagtacaca tatgtatatt tcctaactct 113460
   gagagaacct acagttaatg acaccccagt agcaaacagc gcacctctta ttttagttta 113520
   tttgtgcact ataacaaagt acccgggatt gggtgactta gaaacaataa aatttatttc 113580
   tcgtgattct ggagtctggg aaatccaaga tcaaagcacc accaggttca gtgtctggtg 113640
   gaggatcctt cctcatcgat ggcaccatct cggtgtcttc atgtggtgga aggaatggaa 113700
   gggcaaaagc agctctctga agcctctttt atggccgtag atcctgtcca tgagggcagg 113760
   gccctcattg cctaatcatc tcccaagggc cccacttgtt aatattatca ccttgggggt 113820
   taagttccaa catataaatt tttgggggga cacatacact caaactatag cacctagcac 113880
   ccagatcttg gtggtttcta aacaccattc tccaataaaa gaaaatgagt ctccttgaag 113940
   aagtgattga gtccatggct ggggcaggga aactatgaga actgctcaga atttttgtgt 114000
   tgccaaaaag gaaggtagca cacaaaaata ataagaacat gtacacagga cacggaaacc 114060
   aacatgcagg agccccaagt ggccagaact ggaaaaattg gggcaaccaa atataaaatc 114120
   attttgcatt atatgcaaga gttttgcatt ataaccctta gaataaaata aatatccaca 114180
   tgtacatggt gatataaatg aataaataat tgaataaata aataaataaa tgggggagaa 114240
   gggacagctc tccttgactg aagaattcta attaataaac atagaaggaa tgagagaaac 114300
   agaaaattac catttggcaa atagcatagt aacaattgtg gcagacacaa tctactgaca 114360
   gatactaaaa atattgggac aaagtttgta gagagcagga tgtttgcata atcttaaatg 114420
   tcaccctcaa gatatttatt gattgcaaag ggaaaaatat agtaactttt tatggtgtga 114480
   cagaccccac cttaaccaaa tgaagaaggt taacttcacc agtaataaca catgacatca 114540
   taaacctccc acttcaaaca ctgatttggt taatcagtga aaagattttg cccttgggtc 114600
   atcagtttgc aacctttacc gtatagtaca cttttgatag tatttaattt gctacagctt 114660
   tgccattaca aaagtaatac agaaataatt tatgttttcc tacctcttaa aaaataatgt 114720
   atttttttgc caattagaaa aatgttgaag tatgactcat ttacagtagg ttaaatgaag 114780
   tatacacgtt tgatattccc tgtattcaaa tgctaagatt ggtaggattt ctagaaagaa 114840
   aacaaaggcc atacaacttc aggaactgca ctagatttaa aaattatgaa gattctaatt 114900
   ggcaaagcct ttggaccagg cattccaaac tcataacttc cacttttact attctgttga 114960
   attaatcata tcccagagca cacctctttg gtgtctagca tacatagttg atcatccttt 115020
   ttggtcgcta atactttttt ttcttttact ttaagttctg agatacatgt gcagaatgtg 115080
   caggtttgtt acatagctat acatgtgcca tggtagtttg ctgcacccat caacccgtca 115140
   tctaggtttt aagccctgcg tgcattaggt atttgttcta atgttctccc tcccctttgg 115200
   tcactaatac ttttaaatac ttggtgaaaa atccttgtgc ctttcccctt cagccatata 115260
   atcaaagttc tatttatact tttatttgct taaaaaaaat ccctgtactc aattttgacc 115320
   tgcatttagt taacatcatt ttcacttaga atcacttatt cttatcagga tttcagttta 115380
   ttgaacacaa cacccaaggt agatgaattc cagaagttaa atcgctgtgt gcaagccatc 115440
   acaaatacag acagagctgc tgaggctatt actttttgac tgcgatgttt ctaagtcaat 115500
   tgaactgtga ttgtccttta tatatatttc agttgacttc ttaaatttaa acttctgagc 115560
   aatcatattc aagaaaagta gccaatggcc caccaaacag gatccataag cctaaaaaga 115620
   catgagtttg gaagctgtgt ctgtggattc aaacttaggt gagaatttgt ttagaatggg 115680
   aaacttattt atgacaaata accaagttaa aaagctgaca atgaaaacat cacaaaatca 115740
   gagagacaag gtacctttat taagaaactg cccaccccac ctctataata ccttgactcc 115800
   tacaaatatg ggctgtaaat gttttgatta tctctctacc tttaaatata aaaattattt 115860
   tgaaacatca ttctatagag aaaatagaaa tataacacag cctttcctct aggatgtcaa 115920
   attaattttt tttcttatta ctgaaatttt aaaaaatgcc ttttggagtc ataatttgtt 115980
   atttgtaatg gcacatactt tttttagcat tatcaaactt tggaaaatct ttattgcatt 116040
   tcttgaatat aacatatcag tgcatttaaa atttactcat ataatgacta gttttaatac 116100
   tctttaaact gattaaatac atcaccagtt gctcatcaat gtcttccttg ttctgactac 116160
   gacgagtttg tgttctctcc atacttgtta ctattttgtg tcaaatctgt aagaatttaa 116220
   atatttcccc atgttcatga ttcacttttc ttcattaatt aaatttatcg aataagaacc 116280
   aaaacattac ttctattcaa aattttcctt ctaataaatt gttgctgttg gtgtgtgttg 116340
   aaaattcttt tgccgtaagg ggcttcttga tataagaata atttttattg atttaatcat 116400
   ttaattgttt tttcatgttc cattcattta tttcttagct tttcttccat tacatcgtaa 116460
   ataaatttaa agatgaccaa aaatggtaca gtcttatttt tatgcaatca aagcagaaaa 116520
   gtttaatttc tctcttgttt taataataac cttcaaaaca tctttcctta ttgcagttaa 116580
   atgatatatt cccaccttga caattttcta aaccatattt ctgtaaattt ttgcaaatac 116640
   tttgtagcat cctcagcaca tagttgcacc catgttgcag ggcttatgaa gctttataaa 116700
   aggcagactt cctccaggca catacatcta gtgttgcagg gcacagttat atgcaatatg 116760
   ccctctcatc ctgcatgttt gtttgtgtgt gtgtgttcca ttttatttta aaaatttttt 116820
   aaatttttaa tttttgtggg tatgtagtta gtgtatatat ttatgggtta tgtcagatat 116880
   tttgatacag gcatgcaatg tgtaataatc acatcagggt aaatgggatg tccatcacct 116940
   caagcattta tcctttgtgt ttcgaacaat ccagttatac tcttagttat tttaacatat 117000
   gcaattaaaa ttttttttta ctataattat gctactgtgc tagcaaatac taggtctttt 117060
   tcattctttc atcctgaatc tttgtgctat gatgccggtt gagtcagtac atggagatgc 117120
   tggagaactc ctggaagcca ttcctacagt ggaatgacta gcgaagatga actataatgg 117180
   aaattgagtg caaaacacat acatatatcc cattaattac aaaccaaatg cattgccaac 117240
   aacctccctc ttagctggag cccccaaata accatagcta ccccaactcc aagaaacacc 117300
   tagaaagaaa tgtgatactg aaagtcattg ttaaaaattc ttatttgata cattttgtaa 117360
   agatatatga ccatgtgaac atatttctag ggcttttccc aagcccataa tgtccacgaa 117420
   caagtgagtg gccttgagct tttggcttca ttggcttcat gcaacataca cctctgttcc 117480
   ttaatgtgca agagaatatc gaaaaccaag tcaatgagga cactgatgac aaataagtag 117540
   ctgaaggttg gcaattcaaa gaaaacgagt gccttgaaaa cttacacttg agagaagttt 117600
   ctcaaatttg acaataactc ttaaaaatat catactttgg tactaaaaac ataaataaat 117660
   ctccgtaaac tacaaattaa aaaaaatttt tttggatcag ccaagctggg ccttttttgg 117720
   aatgtgcttg tggaagtaaa taacctcgat tttgtggttt agtcattcat ttatgcttca 117780
   gtttctgtta caaattttct gctacatttg taatgcgcaa gcaatctaga atggacgggc 117840
   agtgttctgt tgcccacaga ctaagattgg ctgacatgag gtgctattgc attactcata 117900
   tttgggtgaa aaggaaacat cctgagtcag gtctctattg ccatgaaaat gctacataat 117960
   aagcagccac aaatttattt atacatttat ttagctcaca aacttgtgag gttcagctga 118020
   tctagactgg gcttgctcat gcatcgtcag gcaactgcag tgggtggact agatagctcg 118080
   ggtgatttgg ctggatttgc acatacttct caggtgaccg ttccctaatc taggctgcct 118140
   ttgcccaggg tgaccagggc cacatggctt ctcttcttat gtgtctcatt ctgcacctgc 118200
   cagctggaca gatttcatca tgatggcaga gatgcaagaa cagttcgaaa tgcacaagta 118260
   tttgaaaatg tggagatgtc agtaatgtcc catggagcaa atcaaatcac atggctaaac 118320
   ctagagccag agagggagtg acctacaatg ttacctggta aaaagcactt atacagtgaa 118380
   agatgaagaa ttgaggccgt attttatttt tgtaatctat catggcatct ggaactgaac 118440
   ccttggtttt tctaaccaac acatttaaat ctgattctct ccagttcagt aaatagaact 118500
   cctatccatc ttgatgctca ggccaaaata ctttcccttg cacctcaaag ccatcaataa 118560
   gtcaactaaa gtttcaaaat acatctagaa ttgaaccacg tctcactgtt cccactactg 118620
   ctcctaccct tgtgcaaacc cgctgccatt cttcacctgt gcaaacttga tagcctctta 118680
   actaatattc ccactttcag tttttccctc ctgcagtgtt tccaccaatt ccaaggaaac 118740
   ttttaaaatg taagttagat catgcacccg ctgctccacg ctcgtcaatg gcttcctcgt 118800
   tttattggac tttattgtaa gaccaataaa atcttccaac agacaccagg cacggtggct 118860
   cacacctgta atcccagcac tttgggagaa tgaggggggg aggatcactt gaggtcaggg 118920
   gttcaagacc agcctggctg ggaaaccctg tctccactaa acatacaaaa attagctggg 118980
   catggtggca cacgcctgta gtcccagcta ctcgggaagc tgtggcaaga gaatcagttg 119040
   ggcctgggag gtggaggttg caatgagtcg agatggcgcc gctgcactcc agcctgggcg 119100
   atagagcaag ccttctaaaa aaaaaaaaaa tcttccattg gattctattg ggacttacaa 119160
   taaaatccaa aatatttact gtagcctgaa aagccatgcg tggtctggtc actggctact 119220
   taccttgcct ttttaaaaat taatttattt atttatttat ttgagacgga gtctcgctct 119280
   gtccccccag gctggagtgc agtggcgcga tcgcggctca ctgcaagctc cgcctcccgg 119340
   gttcatgcca ttctcctgcc tcagcctccc gagtagctgg gactacaggc gcccgccacc 119400
   atgcccagct aattttttgt agttttagta gagacggggt ttcaccatgt tagccaggat 119460
   ggtctcgatc tcctgacctc gtgattacac gaggtgtaat cacacgtgat tacaggcgtc 119520
   agccacgacg cccggccttg ccttgttttt ataccactct tttctcactg actctgcttc 119580
   agccaaacaa gtcttcactg ttatttataa acattgataa cctcaggaca tttgtacatg 119640
   ctaactccct ggcctggatg acattttgta catgctaact ccctggcctg gatgactgtt 119700
   gaccctgctg ttgtctataa cactcactca ctcactcagt tcattcaagt ttcccttcga 119760
   atgaacttct taacctcttt ctctagtaat tttctgtcac tctcattcta gaccagctct 119820
   ttttctttct tcatggtaca tgttaatagt ggatattata aagtatattt gaattttaaa 119880
   aaatagccta aaggaataaa gttctagaag cattacagct agaatgaaaa tatttccaaa 119940
   acatgatata tgggagaagt gacagcacag gaaaatccta ccactgaaat attaaagtct 120000
   attgcttctg gacattttaa atgtatagtt tttgtgatag ctaaatatta cctttacctg 120060
   gaaagctttt agtcatgctc tattttctga actatatttc ccagttaata ttagaagtac 120120
   attaaacaca cacacacaca caaaaactat gaatggcttt gatagcaagc tttgtgctca 120180
   ctggctacat tctttttttt tttttttttt tttttttttt tgagatggag tctcgctctg 120240
   tcacccaggc taggagtgca gtggtgcgtt cttaactcac tgcaacctcc tcctcccggg 120300
   ttcaagcgat tttcctgcct cagcctcccg agtagctgag attacaggtg cgagccacca 120360
   tacttgggct aagttatttt tgtattttta gtagagacgg ggtttcacca tgttggccag 120420
   gctggtctca aactcctgac ctcagctgat ccgcccacct cggcctacca aagtgctgaa 120480
   attacaggcg tgagccacca cacctggcca acattcttgc tcctatatag ctgtggagcc 120540
   ttcagggagc actccagcat gctaggaata tctggatcac tgttaagtga tgccaagccc 120600
   gagtccttcg attgagccac gggaagagga gcccatataa tggtgggaaa ccctttatat 120660
   ccagggttta atccttcgct gtgccacaga gagacgtctt tggcagtctc cagaagacca 120720
   ggtaccaatt tttacaaaat gttttatttt gcatatttat cgagataaaa tttatgaaac 120780
   aattaactcg tttaaaatga acaacttagt gacatttagt acattcaaaa agttgtgcaa 120840
   ctaccacctc tatctaattc caaaacattt ccattgctcc aaaataaaac cctgtaccca 120900
   ttaagcagtt actcctcatt ttcctgttcc caagtctctg gtaaccacaa atctgctgtc 120960
   tgtctctgtg gatctaccta ttctggatag ttcatataag tacacttata aaatatttga 121020
   ctttttgtgc ctgccttctt tcccctgaca tgtttttgag gttcatccac actagcatgt 121080
   atgagtactt tattcttttt tctgttgaat aacatccgac tgattggata tactgcatgt 121140
   tatttatcca atcacctctt gatggtcatt tggctgttac aacacctttt ggctattatg 121200
   aatagtatca ttatgaacat tcctatacta catttctgtt cagtgcctgt tttcagtttt 121260
   ttggggtata tacctgggag tggaattgct attatcacag tggcaacata attttttaat 121320
   gtgtaaaata aaatacctag gattattttt aaaaggcaat aatgttgata cagagctatc 121380
   aaaacattaa ataaaataaa tgtgtgttat ggtgatatat atttcttgat tttgcagtat 121440
   aagaagatct aacagcaagt ctactaactg ctgtgcattc aaagtataca tgatatttcc 121500
   aggcatctgc aacacttgta atatgaaagg gtttgtgttt ttttggtcat agctccttcc 121560
   agtattattg ggatttattg actgttaaag actaataaaa ataaaaataa ctttttttct 121620
   atccagattc atggaaccct tgaagtctat ctaattgagc tcagggacag ttacgacccc 121680
   cagttaagaa tccctgtttt aaagaaggag agtgatggtg aaaaactcac ttcctagtag 121740
   cacatttatc tctgtctaac tctctcacca ctcttctccc atctcatctt ttgtcttagg 121800
   aaatctaaac tcagcagaat cagcttggac aggtgcagat tcattgaagt gaaggttagt 121860
   gaattcacac actccaaaga cattacaatg gagttgtaaa ctgttatctt ttaccatttt 121920
   aggtgtcaga ttaaatttaa gttaatttcc atataactta aacacaggaa ttaaaatagc 121980
   ttttctaaac ctaatgttgt gtagtacctt acagtatcta aagttctcac acatacatca 122040
   ttgtatttaa tcctctgaaa attgtgtaag taggtgttat agaggaagta caccctgtta 122100
   ttaccttggt tttaggatga ggaagcccag aggcagaaca atgtagtcaa ttaagtttac 122160
   aaggcaaaca caatgggcta gaacctgggt catctagtta atacaagctg taagtgcttt 122220
   ttctattgga gcagaaaatc aatatatcta aacataattg ttcacttggc ttttcagtgt 122280
   aaagaagagt tactccaaaa ataaaagcca acacattata aattgctctc taaatgggga 122340
   aaaaaaagca gtaatgtgat aatagtatag ttcacagtcc tagaaactca attcttctat 122400
   tgtctcaaag gaaatgtctc aaaaaggcca gaaactcaaa ccagatattt tcctgtagga 122460
   aaaggtgaag atgttttcaa aaactgagtt ttattattac ctgaaaagaa aaataaaata 122520
   gtgctcaatc tgtatttgga gaaatcccca aacagaatat tgttctgtat gctctgagaa 122580
   ttttctctag agaggatttc cttcactgga caaatgcata aataatttct gccaaaatgc 122640
   agtcccgtat gtcatagttt tcttgttttg aatgaaagaa aatccatttg tgagtgggtt 122700
   aggttttcta tgcaccaaaa gttacttatt caatttttag agtttctatt atgttgcttt 122760
   ctcctctgga cacagaacat accttattcc aacagtgaag ggaaagaaaa aatgctgagc 122820
   tctggctata aatattttat catgtataga gacaaagatc acaggggggc agatgaggag 122880
   caggaaacaa atgtgatgtt gttttcaaat cctcagagga ggtttatatt aaactgtctc 122940
   tgccagcttg ttttctgcct ggcttttaca ctgtcaaacc cggtaatgga atgattttgt 123000
   gtacttggag ttcacctact ggatgaggaa tgcagcatct aactgaaagt gttaaaatta 123060
   tccgtgttat aaagtataca caaagaaaaa ttggcagagt gatgaagaca tatttctaag 123120
   aaagatgatc catattaatt ttttttggaa aggttctatc ctagtaaaga caataggtta 123180
   aaaaatatgt tgtatgttga gttttctcaa ttcagtttat tatttttctc ctcttaatct 123240
   aatgtctcag agttgttgga aggttttaat ctaatgggat ctagcatctt tcatgatttt 123300
   gcaacccatt ggagtttatt tggctagaac tcaatattaa tgaggacaag gtcttgagcc 123360
   cattctccat aaagggagga caaaatgcta ttttcctaac ataatggcac agaccatgta 123420
   tcaaacctgc tactcacaaa attgcatctc tgggtacaag gaaaagtgga tgaaagggtg 123480
   agtaatacaa cagccgttct aaattgcaga tctgatggaa cccttccagt ctctaagtta 123540
   ccttgaaatg tatttgcata aggggctgcc atccttgagt gtgaggaggg gaaaaaagca 123600
   tgcagagact gtttatacta gtagagctga tagtaaatct tccttccgtt ttatatacca 123660
   tagaatttta gagtttaaaa tgtttaaagt ttaaatttta gagtttttaa actctaaaat 123720
   tctatggtat ataaaacaga agaaggctca ttaactctga gatatgaata tattattgat 123780
   attggtcact taccaatgtg cctaatagta tactcatatc tcagagtcag gtgccatttg 123840
   taatgcagtt gttatatgtt ttgaatgcat atcatgaatg agcatctgat ttccaacatc 123900
   cccaatatag gagtaaaatg tccagagaat ctatttaaat gaagtacaaa atggcggccg 123960
   agcgctgtgg ctcacacctg taatgccagc actttgggag gccgaggcgg gtgtatcacc 124020
   tgaggtcagg agtttgagac cagcctcgcc aacatggcgg aaccctgtct ctactaaaaa 124080
   tacaaaaatt agccagtcat ggtggcgggc acctgtaatc ccagccactc gggaggctga 124140
   ggtaggagaa tcacttgaac ctgggaggca gaggttgcaa tgagccaaga tggtgccact 124200
   gcactccagc ctgggcaaca gaatgagact tggtccttgg tctcaaaaac aaaacaaaac 124260
   aagaaaacaa aacgaaatgg cataaactta tgtgaaaact tagttatgct tagctaggac 124320
   atttaggatt gtttgtttgc ttgtttgttt ctttatcaaa ttattcgcag atatcaggtc 124380
   actgattttt ggcttgaaga gctaaattcg gtgactaaaa tgctgcttgg aatggttttg 124440
   tacttggtaa aaaatgcacg ctaatttttt tactattcat tattggccag gtactctgca 124500
   taggccattt tatagaaaat ggtattcatc tccatctttc agatgagtaa ataatctcag 124560
   ggaataataa attgcctcaa atcgtgcagc tagtaaaagg tggagccagt ctgtggatac 124620
   gaagccaact ccttcctctt gaaggttgga aatgcaaatt ctaaatttaa ttgacatctg 124680
   taccttttgt taaaatatgc attatcaaca tgttcagaat ggtatagctg ttatctgaac 124740
   atagtctact aaacattcag cattcagatt tctttctttt tttttttttt gagataagtt 124800
   ctggctccgt cacccaggct ggaatacggt ggcatgatct cagctcactg caacctctac 124860
   ctcccaggtt caagccatcc ccatacccca gccttccaag tagctggggc tacaggcctg 124920
   caccaccaca cccagctcat ttttgtattt ttctgtggag acggggtttc gccatgttgc 124980
   tcaggctggt ctcaaaactc atgagctcaa gtgatcagct tgcctctgcc ttccaaaatg 125040
   ctaggattac agggtaagcc accacacctg gcccccagat tgctttttaa aagtcagtaa 125100
   tggtaatatg gctttagcag agatatatgg gtaaagtctt cttaatggca tattttaaga 125160
   gaaagaaata tgagaggaga gttttagtga tgatttgaga atgtcttaaa gtaattttat 125220
   gatatctaat atttacttga ttaatttggc tatttgcagc ataaatagaa aattaaaagt 125280
   agtacgaatc cttagaaaat aaactcaaaa tggttaatta tatagaaaat gtaatataat 125340
   gatgctaaca aaatacagaa atttacaaaa tgcatttagt tttatttttc tggatcaaag 125400
   tattacttct gtcaaatata ttgaattatc ttattattat gcagtagacc cacatgatta 125460
   tatcaatttt aaaactgtat tccatgacag gtgacttgtc tgctgatact ctgaactgtt 125520
   tgaatagggt atcaaaagat gcagaaaaga aattacatct acttatattt tagcctattt 125580
   tgaactaatt gaaaagcagc tgtcagaact gtccttctag aatttattgt tgcctgtgta 125640
   aggcattgct ttttactaag tcctagtgat cctagcaata atagtttaaa acatagagca 125700
   tgcttttttt ttttttaatt tttttttttt tattatactc taagttttag ggtatgcttt 125760
   tgagaagata ttttttttca gctactgcat gttctttctt tgattttgat ttttatgtac 125820
   atatttttta ttacacttta agttctaggg tacatctgca caatgtgcag gtttgttaca 125880
   tatgcataca tgtgccatgt tggtgtactg catccattaa ctcatcattt acattaggta 125940
   tatctcctaa tgctatccct cccccctccc ccaacccctc aacaggcccc ggtgtatgat 126000
   gttccccttc ctatgtccaa gtgttctcat tgttcaattc ccacctatga gtgagaacat 126060
   atggtgtttg gttttttctc cttgtgatag tttgctgaga atgatggttt ccagcttcat 126120
   ccatgtccct acaaaggaca tgaactcatc cttttttatg gctgcatagt attccatggt 126180
   gtatatgtgc cacattttct taattctgtc tatcattgtt ggacatttga gttggttcca 126240
   agtctttgct attgtgaata gtgccgcaat aaacatacgt gtgcatgtgt ctttatagca 126300
   gcatgattta taatcctttg ggtatatacc cagtaatggg atggctgagt caaatggtat 126360
   ttctagttct agatccctga ggaatcacca cactgacttc cacaatggtt gcactagttt 126420
   acagtcccac caacagtgta aaagtgttcc tatttctcca catcctctcc agcacctgtt 126480
   gtttcctgac tttttaatga tcgccattct aactggtgtg agaaggtatc tcattgtggt 126540
   ttcgatttgc atttctctga tggccagtga tgatgagcat tttttcatgt gtctgttggc 126600
   tgcataaatg tcttcttttg agaagtgtct gttcatatcc tttgcccact tgttgatggg 126660
   gttgtttgtt tttttcttgt aaatttgttt gagttctttg tagattgtgg atattagccc 126720
   tttgtcaaat gagtagattg caaaattttt ctccgattct gtaggttgcc tgttcactct 126780
   gatggtagtt tcttttgctg tgcagaagat ctttagttta attagacccc atttgtcaat 126840
   tttggctttt gttgccattg cttttggtgt tttagacatg aagtccttgc ccatgcctat 126900
   gtcctgaatg gtattgccta ggttttcttc tagggttttt atggttttag gtctaacatt 126960
   taagtcctta atccatcttg aattaatttt tgtataaggt gtaaggaagg gatctagttc 127020
   cagctttcta catatgccta gccagttttc ccagcaccat ttattaaata gggactcctt 127080
   tccccatttc ttgtttttgt aaggtttgtc aaagatcaga tggttgtagt tgtgtggtat 127140
   tatttctgag ggttctgttc tgttccattg gtctatatct gttttggtac cagtaccatg 127200
   ctgttttggt tactatagcc ttgtagtaga agataatttt agcaacaaat tacttcctat 127260
   gaagtacaag taaatcaatg ttaagggtaa ccattttaaa atattgagag gtaaacttaa 127320
   ttggaaaatc ttcacaacac ctttaaaaca taaattcata ttctaagggg gtcttaaaca 127380
   tttttcaaat gcactggaaa atagccatgt aaagtgtgat ctctccattg tacctctcac 127440
   agagtagttg catttttaca gaaaatacaa ggaactaagt atggaccttt tgtacctgca 127500
   gataactggt gcatataatg aaataaaagc tgttatttct tatctcatcc aaggagaaaa 127560
   atggaaaaat tttctaggaa tttaggttga cctataatag acaaggcgaa ctattaacct 127620
   acaattattt tcagggaaat tttctttgaa cttctctgaa aattggagga gcaataatta 127680
   atatcatgca gaagaaaata cccgggctct gggatttcat tttttataga tatgtcataa 127740
   ttgtacctat tttggggata catgtgatgt tttgatacat gtatacaatg tgaaaataaa 127800
   gtcaaggtaa ttgagatatc catcacctca aacatttacc ttttctttgt gttgggaaca 127860
   ttacaattct tctcttctag ttattttgaa atatacagta aattacggtt aagtgtaatt 127920
   ttcctatggc actcccaaat attagaactt attccttcta tctaactata tttttgtact 127980
   cattaatcaa cttctcttca tttctcttgc tccccacttt cctttccagt ctctggaaac 128040
   cattattcta ccatctacct ccgtgaggtc tacatttttt tagctcccat gtgtgagtga 128100
   gaacattttc tttgcctggc ttgtttcact taacataata accttcaatt ccatccgtat 128160
   tgctgccagt gacagaaatt aatttctttt tgtgactgaa tagtatttga ttgtatacat 128220
   ataccacatt ttctttatct attctttcag tgatggaaac ttaagttgat tccatatctt 128280
   ggccattatg aatagcactg aaataaacat ggatgtggag atatctattc aatacattga 128340
   tttcctttct gttgatatat actcagcagt gggattgctg gattgtatgg tagttctatt 128400
   tttagttttc tgagaaacct ccatgctgtt ttccgtaatt gctgtattac tttacattcc 128460
   caccaacagc atacaagtat ttccctctct ctgcatcctt gccagcattt gttatttttt 128520
   tttctttttg ataatagcca ttctaactgg agtgtgataa tatctcattg tggttttgat 128580
   ttgcatttcc ctgatgatta gtgatgttga gcattttttc aaacacctgt tggacatttg 128640
   tatgtcttct tttgagaaat gtctatttgg gtcttttgtc catttttaat tggattatta 128700
   tttgctattg agttgtttga gtttctaata tatcctggtt attaacctct tgtcagatgg 128760
   atggtttgca aatattttct ctcatactgt agattgtctc ttaactttgt cgattgtttc 128820
   ctttgctgtt cagaagtttt ttagcttgat gtaatcccat ttgtctactt ttggtttttt 128880
   tgcctattct tttgaggtct taccccaaaa atctttgcct agaccaatgt cttgtaatgt 128940
   tttctcaatg ttttcttcta gtagtttcat agtttaggtt ttacatttaa gtctttaatc 129000
   aattttaagt tgatttttgc atatagtgag agagaagggt ttaatttcat tcttctgtat 129060
   atgtatatca ggttttccta gcatcattta ttaaacagac tgtcctttcc tcaatgtatg 129120
   ttcttggagc ctttggcaaa attagttgcc tgtaagtgcg tggatttatt tcagtgacct 129180
   ttattctgtt ctattcatcg gcatgcctgt tttcatgcta gtaacatgtt ggtttggtta 129240
   ctatagcttt gtagtataaa ctgaaatcaa gtactttaat gcctccagtt ttattctttc 129300
   tgctcagcat tgctttagtt attcggagtc ttttgtgttt ctgcatgaat tttaggattc 129360
   ctttttctat ttctgcaaag aatgttatta atattttgat aagattgctg gaatttgtct 129420
   ttttaatagg acagcagaga atataagaaa taatatttgc tttcatttcc tgggtctttc 129480
   atgaagaata taaaatctgt atgtagtcct tttctaccct atctataata tttattattt 129540
   aatcatttaa atggcaagga tatgctgaac tataaacaag gttgggaatt tgtataatcc 129600
   gcatataact gtgattcagc caatatgtat atagttgatt ctcattattt gtgattttat 129660
   atttacagat tcaccttctc atatctttta catttttgtg cttaatgttg gtgtttccat 129720
   ggtttgaaat ggccccatag tgtagggctg acatgctgtc tcatgttcct aagcacaata 129780
   ggctgtcacg tgccttatgg agaaaacacg tgtgttagaa aatctttgtg gaggcataag 129840
   ctacagtgct gttgattgtg agttccatct taataaatca acaatatgta taaacagtga 129900
   cacacataaa acaaagttat gtattgatta tttgacaaaa atattggagc cacagagttg 129960
   tggaaaccta accctctatt tctcctagta acagtgcttt ggtatttgct aatttagtgt 130020
   ttgtggtgac ttcataaatc ataactactg caaatatcaa gaattgactg tattgaaaat 130080
   gtacgattac caggtagtgg gctaggtccc agagattcag gaaccaaagg cacagttcct 130140
   ggccccacaa ggttcaccat ctattggaga gatggatttg taatagaata attattcaca 130200
   gtggagaaaa tgctgtggta cagaagcagc caaggtacta agaggagtct aatcctacct 130260
   gaggtgtcct ggtaataata tacagatccg tagctgcatg cctgagaaaa tgatagaaac 130320
   agtaaaaaag aatgcactgc atgtgggaga atgtaaagta aatgtggcta tatgtttatt 130380
   tttaagcaca gcataatgtt agtggggttc tgagtcattt tggtactata ttgttacaga 130440
   aatacactgt ggtgaaatgc ttccacctct tgctaaaatg aacactgagg aaaaatgaag 130500
   aagactgaca agcaccagcg aaaagttgca gaatagaaat agccacactc ctctggagtc 130560
   tttaattcat ccacagccat catataaagg taggggaata aattacctta tggttttgtc 130620
   cttttaaaat gtattttaaa ttaaacaata ctaaaaacac agaaacatgc atgtattgat 130680
   tcaacaatat tctctgggtg actgctatag gcctgcaaac tgttgtaggc actggaaact 130740
   aaaagggcaa gcttatggcc ttacagaagc tgacattcta gtggaaagtg taagtaaata 130800
   aatgaaaagg aaactgacaa ccattgataa tgtttatgaa cacgataagc aggttaacga 130860
   gataagggat gagggactac tttagtttga gtgtcagagc agaacactta gagagtttga 130920
   gctgagaact gagtgataga aaaaaatcca gccaagtagg gtttgaagat cataccgtcc 130980
   atgtgtcatg aaccaggatc catgtggata tattcaaggt ttggaaacca ggccagtatc 131040
   actttaagag aggggctttg gagaagcagg atgtagaaga tgaagctgga gagctgttta 131100
   gatgtgggaa agtgtcttca ttttatttta ataaagatgg aagttattgg aaagtcatta 131160
   gcaggtgaat gaaagaattt atatttttta aaggccattc ttctttatta tagccaatta 131220
   gtatgtgtgg gggtcgctac taccccccac acatgggtac taccccccac gcagggtgct 131280
   cttacccaca taaaagatac tgctgacttg gcttagggat gtggcattgg agatggatga 131340
   atttgaaaga tattttggca tagatgacca cccaagctcc ttatgaagct ccaaatataa 131400
   ttctaatttc catatgcagg gtatgctgtc tatttgcgtt ttaatacaca atggtcagat 131460
   tgtaatttat tctactattt caaagaactg cttatgatgt aatatgtctg tggcctgctt 131520
   ctgaaactta gagggtgttg atgtcaaatt gtgaaaataa agaaaattct gcccaaaatc 131580
   tcatattcta ggtgggttat cttgtaacac tcggagactt gagcattcca gatgtgtttg 131640
   tagctcttct cctgcttcct ggatggctcc tgacttctaa gaacattgtt gctcaagcca 131700
   ctgagaggca gcctgtggga agggtgtcct tctagatgag aaacaagagt tttttaagga 131760
   ctagtgactt agtcaagcat aacttcaaca ctaaaacttt tatactgggt tattcataga 131820
   tggaatgttg aagggaaaat taaaatccaa tttcaaattg gtgctttaaa aagatgagaa 131880
   tatatggatt caagcaatgg ttaccagaga aacttatcct ctgtgcagtg agggtttcac 131940
   atcttgtatt atgtaaccat tgtgcaatgt ccatagggta gggaagagag gaaaagaaat 132000
   gtatgcaatg accttgtagg tccaaatttg caagaagcca acagctactg ttggagggtg 132060
   gacaaccctc aagtctgtgc tcagaagagt ttttcatgat agctcaactg aacgatattt 132120
   taagatctca gcaaagtgca gcttgctttc caatattcat aactgtctga aattgttcta 132180
   aacttgtatg gtgtaggaat tcttccttcc tccaccccag atccttctgt aaagcttttt 132240
   gtcgttagaa aacagaccca gtgcagcaat tatacagtgc tggataatta aaaagtcaca 132300
   tgagactcta caagttcaca ggagggccaa ggctcaagct tactgagggg tattttgtac 132360
   cacacagctc aggtagctaa aagcaagctt tgttaagaag taaacaggtg caaaccatca 132420
   atcagtacaa ttttgataaa cagcatggac aagctggtac aggatgctgg tggggtttta 132480
   gacttttcac agcaccttat aaatcactag ctagtacatt tcacctctga catggccaaa 132540
   atcaatacca tgtttccagg tgcccatcaa cataagcaaa gagctaccag tgcacaagtt 132600
   aaccctttcc tcatatactt agagaaatga gtcaggatga aaacaaagaa gacctaaaaa 132660
   tgacatacgc cattctgtga ttagtcttgt catccactgg actagataaa actctttgat 132720
   tagctggaga gatcattaaa aggctaggcc tttcaaatgg aagtcaaagt tccttaagaa 132780
   atgatgggat tctgaagcaa caagactaac atgaactaga aagagtaatg gtgccttagg 132840
   ttgatttccc cagaagcaga gtgaaacaaa aaaaatggag tggtgtttat tcagaggtga 132900
   tgccagcata cgtttgtagg gtagtgtgga tgtgagcaga ggaaggaagg aagacagaaa 132960
   aggtgcctta ttgtttctgt gatcaagagg ggtacaatca cattaggtaa ctttgggata 133020
   cagcatagaa cataacttgg atttatccca atcaagggca tggaaattag ggtatttagc 133080
   tatcttctcc catatgtctc gactaagggc tgctccctta ggtggaggtg tggagtgtcg 133140
   actccctggt atttgtaacg tgccatacat gtagaccaag aaaactccag cagccagagg 133200
   aagtcctcag gcagagtccc agatgtttgc ttttgaaagc caccatcagg tcaacttgca 133260
   caatgatgat aactgccaat gagatgagga tagagcacct acagtttctg ctacaagaag 133320
   tgacaacaaa gatgatggtg atgatcatga tcatatgaat agagctatca tttattgagc 133380
   aactagaagc caagtcctat attaaatgct ttacatgcat tttatgattt aattcttata 133440
   tgttggatat cccctctgtc aggtgggaga tatttataca tgattatata tatataatca 133500
   ccacaatctt gcagaatgta tgttattccg gtctttaatt tagagatgac cacacaagac 133560
   tcatcaaggt taaccaaatt atctgaagta acataggaaa tacagttgag attcaaaccc 133620
   gagtttgtct gcctccaaaa gggtcccttg gcttaggcaa cagtgtaatc cttaattaac 133680
   taattaatca ttgacatttt tctgacactc atctattaaa aagtcagcag gcactattta 133740
   gattctgaga tacaaaacaa aaattgctcc tgttcccaag gagttctatg tttatggaag 133800
   agaaagacca aaagactgta gtattgtaag ggctaaacta gaagtatgtt gtagctattg 133860
   aacaggatca gtaaaaagag tatcacttgt tgctgtataa caaatagccc cataactcag 133920
   tgacttaata taataagcat gtgttattga tcaggagcct gtgggtgagt tggggacttc 133980
   tactgatttg gactaagctt ggctgatctt agctaggttt gctctgtagt ctgctgacag 134040
   gttggctggg gctgaatagt ctcagatgct tcactcccat gtctggtgct tgactggatg 134100
   tggctagggc aactcacttc tccagcaggc tagccaagac ttatttatgg caactgagcc 134160
   atgtggatca cttattcaag agaataagtg taaggatgca cggctgctta agacctggcc 134220
   tcagaacaat cacttctaca acatatacat caaagaaatt cacagaatat ctcagattta 134280
   agggatgtgg aaatagactg tacctcctgg ttagaggagt tcaaagtcac attgcaaaag 134340
   acattgataa aggaaaaaga agaactagat tgcaaacaat ctaccgggaa gaaaacatct 134400
   gaataaaaga ttacagagta agtaggtatt ccctagccaa ataagattgg aagagaattt 134460
   aaaacaagaa ggggctgggg gccatgccta taatcccagc actttaagag gctgaggtag 134520
   aaggatcact tgggtccagg agtttgagac cagcctgggc aaatagatgc tatctctaca 134580
   aaaaataaat taactgagca tggtggcact gcctgtgatc ccagctgctt tggaggctga 134640
   ggtgggagga tcacttgagc ctgggaaata ggctgtgatc ataccactgc actccaggct 134700
   agacaacaga gcaagaccct atctcaaaag aaaagaaaaa agaaagaaaa gaacagaaaa 134760
   gaaaagacag caggatagta tgtatttaaa gacataggaa tatagtgttt cataatgctg 134820
   tcaggttact tgggaatggt gagggagaga gaggaggatt aaagacaaga aatatgtaaa 134880
   atgtaccaga attcaagaaa ataaaagtta atttgcagac agaataatat gtttatgaaa 134940
   aagtgacaga caaatgggaa gatgaacaaa gttaggtcaa tggatgaatg tatggttgat 135000
   tggacagtca ggtgtggtaa tagggttacc aaaattgtta agattatctc ttagaaaaaa 135060
   atttgtaggt tgggcttggt gactgacacc tgtaatccca gcactttagg aggctgaagc 135120
   aggtggattg cttgagctca agagttcgag atcagcctgg gcaacatggc gaaaccccat 135180
   ctctacaaaa caaacaacaa cagcaaaaaa aaaaaagaaa aaacaaaaat tagccaggaa 135240
   tggtggcatg cacctgtggt tccagctact ggggaggctg aggtgggagg atcacttgag 135300
   cctgggaggc agaggtttca gtgagccagg attgcactgc tgcactctag cctgtgaaac 135360
   agagcaacac accctgtctt aaaaagaaaa aaaaaaaaaa aaagagtttg tagaatgtgt 135420
   atttatttca acaagaaagg aaggaggaaa tacctttgta agtgattgaa gaaaacaatg 135480
   tggttgaaag cagacctgaa ggaaataact tttcagcatg tgactgtgtt tgtggcttca 135540
   gggagctatt gtgatgatgc agattgcagg atagaaactc tttctatggg acaactttta 135600
   taagcgtgct aagcatagtg agtaggtttt tgtgaaaacg aatgaagagt ttgacttatg 135660
   gtcacaagca aattccgagg ctctagattc tcacaaggcc aagtctctgt gaagactgct 135720
   gatgttagag atatatttat atagagattt gtgcatgtgt gtgtttccac tgcagtatgt 135780
   atgtgagatg gtaattttca attcatggat ttctccactc tgagactgag ttgtgtcaat 135840
   tgaatagcag ctctattcta cagttttccc tagccacttt cattgtactc ctgggtctgt 135900
   gttgcaagtg tggagctcgc ccaaatcacc aggtgcagca gattttactg tgaagtttaa 135960
   caggctaata ttagcatctc aagctcctca gaggcagaat ggaaactttt gtagttcatt 136020
   ttcacagcta agtaatacta cactgtgtta atatatcaca atctatctgt tcttttatag 136080
   attgatattt ctgttgtttc cattatctgc tattatgaaa aatggtagta tgatcatttt 136140
   ttatatctct cctggtgcct gtgcaagagt gtttcaagga agtatatgta gaagtggatt 136200
   cctaggatat aagatgtata ggtaaccaac catctcggtt tgcctagaac tgaggagttt 136260
   tccaggatgc aagatttttg gtgctaaaac caggacagtc tcgatcaaaa tggtataagt 136320
   ggtcaccctg aatacacata tgatcgattt tacaacataa tacatattaa cctctttccc 136380
   acagaattgg tagcaattta cactcccaac aagtggtata tgactattcc ctttgaacac 136440
   cattctcctc atgcctggtg ttcttgatat cttaattttt gccaatcaat tgagtgtgaa 136500
   atggaatctt gctgtaattt gattttccct ggttactaat gaggtttggc atcttttcat 136560
   gtttagtttt ccctttgtgt gttctgttct gtgaaatgcc ttccatatct ttcatctttt 136620
   ttccattttt tttttaatta gacacatctc atttatctct tcagattttc ttgatttttc 136680
   cagtacttag gttagggtct ctcaacagca gcagtgatgg cattttggac tgcataattc 136740
   ccatgggata ctgtcctgtg ccttgcagca catttagcag cacgcttggc ctttacccac 136800
   taggtgccaa tagcaccccc aagtaatgaa aataaaaaat gcctctagac ataaaactac 136860
   ctcttgccaa atgtttcccc agggaacaag attgtacctg gttgagagcg tcttgctcct 136920
   gtgggaaaga gaaggtagca gggcaggtaa attccatttc catttctctc tctgacgatc 136980
   tctctctctc aaggcctata cagcatgtct ctgtccagca tgtttagagg tgttgcgtgt 137040
   cacatagcag atgctttgct ggaccactgg ctgtatttct tcatggtaat gcagtggtca 137100
   cagcagtaac gtctcacttt gtcttgctgc tcacccttcc ctgcttcatt tcctatctct 137160
   tctctttcac acctttggat ttcatattcc aattaaaacc tcagcgctta gtgcatgtct 137220
   caggttcagt gtcctaggga aactgaacta aaacaagtag ttgcagacaa gataccctac 137280
   agataggatt ttggaatttg attactgacc tgtctgaaag caatagggat catatattta 137340
   gtggtaaatt gggataaaaa taaaccctgg caggcaggca gcagcattac aattacttaa 137400
   gctctcatct gcagtaaatt gggaaatggc ttaagtggaa ggtcaggtgt taggaggcct 137460
   actagctgca acacaaagtg gctattagag cactgataat tatgcagatt gtgaagtaga 137520
   tggctacttt tgacctgcgg aaagaaaatg ttaagctgag gatggttcat tgccaactta 137580
   aggcatgctc tgaaagccag aggacatcat ctatggctac tttaaaggag actaatctct 137640
   ggtcactgca gggcagactg tgttgtaaca caggcccagg atctgactat aaggctggta 137700
   gggctgccaa ggagacagga tacacagctt tgtaaaatct tctgtttcaa actcagggcc 137760
   atgttcggaa taaggagacg tgggatgagt tcatttggat ggatgagctt gaggacctta 137820
   aacttctaag ttcccttaaa ccaccctgga agacatgcgt catccttctt tttctgacag 137880
   agaagaacag ctttccttgc ccaacagtta tgtaggaatc tcacctgaag caggtgcctt 137940
   atgagataat tcttgcatgt cttaagatct ttctctgcca ctgctcattg cctccatgct 138000
   gataactagg gtgtagactc agcagagaag tatcagccct ctccaggggg aaatagccta 138060
   tccactgaac aattgaagta ccttgaaacc cacccgggag tggaccttgt attagagtgg 138120
   agggggggat atatgttgtg ctgaactctt attgacttca ctggggatgg caccaagttc 138180
   aagaggcaga agaagagacc cagagccaga aacaagacat tgggtctcat gaaggtctta 138240
   catacagggg agagagtcca gtggtggtga gctagaccgg agaaccacaa cacttgcaaa 138300
   aagcatacag tttatgtagc attttcactt agcaccctcc cccaataact tccacctggc 138360
   aaccttttaa cccaaaacac agggcctcaa tcccctgcac agcccacatt ccacatgaca 138420
   ggatggggcc cagatgttcc tcatagaaaa ggaatagctc tctgggttgg ccactcctgg 138480
   attccctagc tcggaattcc caactgcatt caggtgcacc atacagggtc attcttagca 138540
   tatgcttaag tagttgttat caggtacatt caccatacaa cataatgcta gatcagaggg 138600
   tattcattga tatagagaca ctcactcata agtcaagatt cagttgtttg tcaaggacac 138660
   ttggagctga tcccgatagt tttctggggt ggttcttcaa atcctgtatg ttctgatggc 138720
   ctacagcaaa tgaggaaatg ccacaagttc tttgacagtc tcaataaagg gtctgacggc 138780
   tcagagagga gggatgttag aatgcatgtt aaaaaggttt taggtacaat ctatcacttg 138840
   actatttcta actaagaagg cccagaggac actcccttca caaagacaat aagggatcct 138900
   gaaaaggagt gctgacacct ttgagaaggt ggtctgtaaa ggctgccatc tatgggctgg 138960
   gattgacatt tgaagaagct gtccaggagt taatcttcct aatattgctg ggatcatgag 139020
   gcccagaaca acagaggcta agtagcagtg cttagtgtta gaggtcaggt ggccttaact 139080
   actataatgc atagcaaaat taaagtggca gtcagggtgc cttgagccac agggatctaa 139140
   cagtggctaa gagatcctgg aaatctttgg gacaaaataa ctgggcagct aatagatctg 139200
   cagtttggat aacaggggag aacaactgaa gggttataaa ccatggaata tcacgggccc 139260
   tttataggtt tccacatcta agtcagttca gagatggcaa aggaaccatg gcagtcagct 139320
   catgttcatg tttcatgttt attccgtttt taattttttg tacattttac acatcgtgat 139380
   tgcatatcta cctgctaatt ctaatacatg atgtacaatc ggtacagtaa accagttgat 139440
   tgttatttgg tcatctgggg ctctgggtga ccaaagctcc tcaggtcatg ggtaatattt 139500
   gagtattgat gcaagcatgc aggaatcctt agggcctcta ctggggatgt tttcccatgg 139560
   agagtattgc tttgtgttct ctaggagtca gaaacattgg ctgtcttctg ggattccacg 139620
   ctagggactg actctcaggc ttggctgctc tgcctcccat tgcccagggc ctaggctttg 139680
   tctttagagc agtgttgtta tcatcatatt cctttagagc tacccagtca ttttcattag 139740
   tttaattcct aaatcatttc tctggtgtca tttctctgtc atttattttt gagggcaaag 139800
   gtggagggaa gctcggatat ttcatttact aactactgag cctagcaaaa cttattactt 139860
   ctttttaaat tttttttgag acagggtctc tctgttgtgc aggctggagt gcagtgatac 139920
   agtcatggct tacagcagct tcaagctcct gggctcaagc agtcctccta tctcaacctc 139980
   ccaagtagct gggactacag gtgcaggcca ccacacctga ctaatttttt atatttttag 140040
   tacagacgag gtcttgctat gttgccctgg ctgctctgga actcctgagc ttaagtgatc 140100
   ctcctgcctc agcctcccaa agtgttggga ttacagacat gagctaccat accaggccat 140160
   caaaattgac ctttttataa atgtatccag gatatatttg cattttagca tagggatcct 140220
   ttaaattacc tagactgcat actaccaaaa atgaaataac tttgtctctc tacttgcctg 140280
   aaaatgactt agagcaatta gcaactgccc agtgcaccaa ttataatggc attgagtttg 140340
   gtccaggaag tctttaattg aatgaatcag aggggtattc ttatctcttg tgcagtatct 140400
   ttttcacatg gttaacaggt attgatcaga agactgggcc tgtgggcata gatcctgagt 140460
   gacgtttctc tagtcagagg caagctcagc tggtcccctc tccacacatt ccttatttat 140520
   ggaacgctca cattcaacat gtactgagct gtccctctta tttagtcatt ttttagaaac 140580
   atgatagtaa gtggtacatt tcaggaatat tttcacacgt ttcaaagttt gacatgcaca 140640
   aagacaaact tttaacagtt cagaacactt tcatttttgg aactgctatc tctatatata 140700
   tagagagaaa tgtgaatgac tttgcttgac tcgtcaaagg gcggagcagg gttctcatgc 140760
   ctgtggagac cacacggtgt aaggcaaagt aggcaggggg tgagaacttt ttaaagaggg 140820
   aaacgtaggc ctgtgtaaat gcaatggctc ctacctatct ccaatctatc attgctctat 140880
   gggaatgtgg gccagatatt ccagtttttt aaaggaagct tgaaagctag agttttctaa 140940
   tataaaacct cctaattttt aaacattgct ttattaaaaa acaaaaatat gcaaacaaca 141000
   agtgacctga ttttctgggg gaacaaaaca tgtttggggc gccctatgtg ctgaatctag 141060
   cctgcagaca agttcaaata tgcagcatgt agcttgagat ggatgagttt caaaatgcaa 141120
   atcctatctg cactggcaat tggattattt tgtgtcaagg aaaatgactt ttctgtctta 141180
   aggtattttt gcttttgatt gcttctttta ttgcttggga ttttcttctc cctggtataa 141240
   cagcctagtg gatactttat catctaaagc tacatataag cctatactat gatccagcaa 141300
   tagcccccct aagtgtatac ccaaggaaaa ctagtgcatg atggatctat tcaaagtaac 141360
   acaaaactgg aaacaagcca gaagttcaac aacagaagaa tgagaaatct tggtttattc 141420
   attcagtgga atgctgtgca ataataagaa cacacttttg ttttgagata gagtctcact 141480
   ctgtcaccca ggctggagtg cagtggcaca gtcacagctc actgcagcct caaccttcca 141540
   ggctcaagca atcccctcat ctctgactcc ctagtatctg ggaccacagg tgctttccat 141600
   cacgtccagc taattctttt atttttgtag agactagttc tcactatgtt gtccaggctg 141660
   gtctcaaact cctgggctca agctgtcctc ccacctcagc ctcccaaagt gctaggatta 141720
   caggtgtgag ccgctgtgcc tggcagaaca tgctgttgat acacacaaca acatggatga 141780
   atcacagaca tgttgagcaa aagaggctgg actccccaaa aatgtatgta tgtgtgattc 141840
   tatttataag gagttcaaaa actggcaaaa ttaatctttg gtgttagaac tcaaaaaact 141900
   agtttgatat gctgagttaa ctgtctcctt ttgcagctca ggggtaactg cttagtaatt 141960
   tgaaggaggt tcttaagtat ccccatttgc cataggcatg aggggaccgg gtgcttgaag 142020
   gtccaccctt ccaacccttc accaactgag acatctccat cacaaatgca tgcatgtaga 142080
   gaggaaggca ctctgattcc ccatgtgaca cgatatttca cttttttttc caagtcctcc 142140
   aggttcccac ttgtgaaata caagttcctt atctatcaca gagatgtaga gtttttttta 142200
   ttcctaccaa gcttgaagga gtggctcttt gtggagcatt acttatgtat atgttgatct 142260
   cacatcttcc ccagagcatc ctagtctctg caaataaaat taaaaaaaaa aaaaaaaaaa 142320
   acaagaagca aaataaccct tctcttttcc ctcataagct taatattttt ttttcaagtt 142380
   tcaaaaggaa tagactgagg ggaagaaaat atacacccaa ggctatttct ctactactag 142440
   gaactggtgt ttccatacag attgtttctc atacctgggg gatgagtaag tgcagccttg 142500
   aaaggaagca attggaaagt acctaaaaga agctcccagc ctattttatt tggcatctgc 142560
   tctctagggc agaaagtgag acaaggagaa tctgcagtaa aaaccaaagg agtaactgaa 142620
   gaaggggaaa cattcactga gcccttaagc tagaaagacc tctaatatta caatcaggac 142680
   tgtgtatttc aaacaacctg taaatagtgg tggcttattg cagctgatag gacacaaaag 142740
   acagcaagca gagatctggt tgtagtacag gggatgcaga ggagggcatt gtgaccgttc 142800
   tttaaggatt tgttcctaat tcaaagtttc ctctatctcc acattattca aacatcacta 142860
   ctcaaaatag aaaacatgga aataactcag aatctataaa gtgcttcact tgcttattta 142920
   tagtgcagga aagataggaa aaattttaaa aatggttatt ttgttaactt ccagttgtta 142980
   catctctgtg ttttaaaatc aagttatatc tcattacaat aagcttcatg ggaaatttgg 143040
   cattttattc catggaattc tcactggtca aaatcaaagg tacttgacct aggtaggaat 143100
   caggtgtctt aaatcatgct tagctacatc acttatagac agcagtgcag tcctgagttc 143160
   acttcttaac ctctctgagc tccgggtgaa acaaagtgaa atccatgctt tgtctgttcc 143220
   ttaagtttgg tatgaaacaa agccaaaggc atcctaacaa agtgtccagt gtgaagtgaa 143280
   tgggaggtat gatcattctt agccctgtga cttagaaagc aaaccaaatg gcatgtctgc 143340
   aaacattcct tagatgaagt gctttttgat tcttgtaatt aatgtttgtt ttttattggg 143400
   aaatggatat aagtatcatt agttatggga aagaagttaa agatcatgca ttcatgggga 143460
   gaaaaggagc tatacagtta caatagaaag cactgggatt tatactatcc tctctttctg 143520
   gctggccaaa gagtataact ccatggagtt gtttttcctt atgtctaaaa agaaatacca 143580
   gtatctacca ccctgaacta cttaagagga gtgttgtaaa catcaaagga aatcatgtgt 143640
   gaaaaggctt taaaagttca aataaagaaa aagagtatat agttgagggg ttataccact 143700
   tttccctctt attctcagaa aggtttctaa gcattatatt gaggattctg ataagattaa 143760
   tcaagccctc tcccacttgt aatgctatct gatttttttc ttccttttac aaatctaaat 143820
   aagtaaagat agtatctagc aaaaggcatt gtatgtgtca aattcatcat atggtcaaac 143880
   agtactaaca tcatgggcta atgtcaatta gcccatgtca acattttatt gtattaaaac 143940
   attagtcaat gttttactaa atcaatgttg gccataactt gggaaaatgt gtattgtcca 144000
   aaccaagact ggtttgagag tgaaaaggag agctgttaat aattatgtca gcctaacaat 144060
   agtcaactaa gactgttctt ggacaagaag tattgtctaa aagcttcctt tttaaaataa 144120
   gtcataatta tgcctaatat gttctacaga ctaataaagg tatggggaag attttttaat 144180
   gagcaaacta aatgtattcc atggttctag ccttcaagta gccacaagtc tatatggtaa 144240
   gagaaagatt atgaaataca aggatgcaaa attagtaaat gaaagacatc atgctagcta 144300
   acaagtgttg gattaagtgg agtagaatct tggagattta gacacaacca taaagattca 144360
   gacaaccttg agttgttggg gagcttctct gagagggcca aagtctttaa ctgggtctag 144420
   aagactggtg ggagctaggt agatacaggg aatagcatat ttggagatat ttggggcagg 144480
   taaaacaaca tggagaaaca cataaattga tgagtttcca tggttgtgtg tagaggggag 144540
   agagaagggc tgtaatgaaa tttcataagg ataaaatcta ttgtttcaac aaatagttat 144600
   tcagcatctg ttacatgatg ggcagtgttc tgacttatag aaataaaaca gtgaactaaa 144660
   catctttacc cttatggagt tcgcgtttta acaaaagaga acttccctta aaatctgcac 144720
   cttctctaaa tgaatgttta gatggcaaga agtcttttgg gggaaggagg aatgctgctt 144780
   gaaaaacaga aaatggatga ttcaggtcat tttgtgctaa aataataagg gatttccagc 144840
   aactgagcta atttatcttc ctaagccaga atctaagaca tggagaaagg tttatcaagc 144900
   atccatctat ccattcattc attcaacaaa tgtttattga gaacttacta ggtgtctggc 144960
   tcttctgtgt gctggggata ctgcaatgag aagtttggag aaagttccct gcctctgcag 145020
   agatattcat ctagtaaaac tggatcttcc cagattgtat attctaatcc acaaaaatga 145080
   aaattaaaaa aataaaaact cttcaagtac ttctttacat attcactttc tacatacaga 145140
   gctaataaga tgaaagtggt tatttgcttt tctccataat acaattttct tactctgatt 145200
   attactgtgt gggtccaagt ctttgcttct gcaaatgtaa tagatgcctg tggcaattga 145260
   gaatcatcag gaaacagaaa aaagaaactt tctcccacac gtctgtctca gagagtagta 145320
   gaccatttgt cgtgccatgg gcagtcctat ttggaagatg tgagatgata acctgactat 145380
   gaggttaaat aaccccccaa aatgcttctg cttcagacaa gacagtatat ttacaatgtt 145440
   gtaatataga gtaaatgtca ctgtataaaa caaaaatgtg gatctgttat tcctagtttg 145500
   aaaactgggg cacacatgag aatcacagaa acacagattg ctagacccac ccactagaga 145560
   gcctgattcc aaaagaattt tcatttctcc caatccccag tgatacctat gctgatggtc 145620
   cagaggttgc accttgagaa ccactgtgtt cttctttcaa tatgtggctg tcatatgaat 145680
   ataaaatgga ttgatctgta gataatgtaa attgtaatat ttataaagga ggtcattgta 145740
   ttcatgtaac tgtctatggc tgggttaccc agtatggtag ccactggcca catggagcta 145800
   ttgaactctt gaagtgtggc tagtgccata tgttgaaata aaaatatttt ggatatctta 145860
   cgttaaataa aatatattat taaaactaat ttcacttgat atttaatttt aatgtggcta 145920
   ctaggaaatt taaagttaac atgtatgact cacatttcat ttctactaga taacactggt 145980
   ctatggagct tttctaaaca tgtatatgct gagggttggt ttttatagaa cttagaagtt 146040
   tttttagaat ttaaaagttg aatttttttt tttagaattt gagagttacc agcagaggaa 146100
   acgaaggtat tgtgctttct ttgtaaatag cttctcaaca gtatcctttt tattcagttg 146160
   ttttgagtag tagttaatga gataatattc actggaagat tccttcaata gatatttgcc 146220
   aaccttcaca tcttaattaa tgtggctgtt gggtcagttt ctcccaaatt taccttagtt 146280
   ccttggaagt gttaagaaat gctctccttc agaagcaagg gttattaaag gtcactgcat 146340
   ttcccttttt agaatccatt caagctccag ttgcaataca catagatcga tttcagatag 146400
   atcgtaaatc taaaagcagg ataatgtatt tatgaacttg gtgggagcaa agactttttc 146460
   aataggacat agaaggtaat aaatataatg gaaaagatat gtaaattgga ctaaactaac 146520
   aagtattcat caaatacatc tttgagcacg aaaaggaaaa ccataaagtg gaagaaaaca 146580
   tttcagtacg tttgttcgac atatccagaa tttataaaga actccatcaa atgagtaaga 146640
   aaaaggaaga gaacctaatg tgaaaatcgg caagggactt gaacagatat ttcacaaaag 146700
   aggatatcta catggccaag gcacatgaaa gaagctcaaa ctcactaggg aaatgcaaaa 146760
   taaagccaca atgagatgcc actgcacacc caccagaatg acaatcatga aaggattgac 146820
   aattggaaat gtgaataagg atgtggagca actcttaagc tattggcagg agtgtaaatt 146880
   gagcactttg gaaagctgac tggaacactc tactaaagcc aaatatactc atatcctttg 146940
   acacagcaat gctactctta ggtatgtata tacccaataa taatgcatca tacatgtcac 147000
   caaaagacac gtacttgaac attcaggcag cattattttt aaaagtcctg aattagaaac 147060
   aacccaagtg tccatcaaca gtaaaatgaa cttacaaatt atggtatagt catgctatga 147120
   tatactagac agcaataaga aagaacatgg cctatcacga catgaattaa tctcacaaat 147180
   acaaaatgtt gaatcaaaca cacttagaat tgtatgatgt aatgattcta ttcatattaa 147240
   gtccaaaatc aagcagacca atcaatctct ggtgggagaa gctaagataa tgttttgcct 147300
   ttgatggagt tggttacttg ggcacagctt gaggaaggct tctagcattt tcctccttgg 147360
   actgcactgt ggtttcagga atgtgttccc tttgtgaaaa gtcgttaagc tgtacccttt 147420
   tctgtataca tgttatacct aaataaaatg tttaacaaaa agagagaaga ggaaatacaa 147480
   agtcataccc catgaccctc ccagaaaaag taactcacct ctttaaaata cacacacaca 147540
   cacacacaca cacaaacaca cacacacaca aacacacaca cggccatcac tgtatctcac 147600
   tacttaaaat ttaaaaataa tatagacttt cgttttatac ttaaaactct agtttgcaat 147660
   tgaccttcag aaattgtttt aatataataa gattttttgc agctatcagt ccaatttatt 147720
   tatcttaaat tcctcattgg acatatatca ttgaaaatca aaactagaat tatagtgcaa 147780
   agttaaagtg taagtagtat attgttctat attcaattca atgtcatcta gctaatagag 147840
   atcttatgtg attcaacttg tcctgaaaaa ccttaaaaaa aaaaggacct tgatgatagg 147900
   caggttctat agaccatgag atttgaataa ctctatatac agttaattcc aaagatttat 147960
   gacaatcata aatttataca actgatatcc aattatttac attagattga agtgagaaac 148020
   tagatagagg tattatttta aaagtataaa ttgttcaaca cagcttattc aaaagtgttt 148080
   atgagtgcat ggttatattc cttacttgta ggcagctggt cgtaatgcgt atctttataa 148140
   agtgttaatg agctttatta tatgactcaa aatttatttg ctcaacagca tttttggttt 148200
   tagcatgtaa atgtatgatt atgatacagt aacacataat atatctgcag aactacattg 148260
   aaattctaca ttactgacaa gtttccacta atgtgaacta tatggctctt tgagttagaa 148320
   atgtctgtaa ttactgttct ttggctttat tggtattact tatggggaaa ttgtgtagtg 148380
   tatgtgcatg tgagagagag agagagggag agaaagggag ggaggggaag atggaaatgg 148440
   acaggtaaac gagagatgaa gagagagaga atggaagcag aagggaggag ggagagaaac 148500
   tactacaatg aattaaactt ttgtttctgc attgactttt tgcatttgga gaataaaaag 148560
   ccaaagccat ttctccttgc catctgtgtt aagagggtct tatcccactc ttagattatc 148620
   aagaaaaatg tcagatttac aaggagtgcc acttttatcc cctttctgaa agaggaatat 148680
   caattgcctt cacaaaggat caattgtatt ctcttatcat aaatgactta tttgccatcc 148740
   acggaaccag gaaagttgcc attcttcttt gcttaacctt aagtcaattc aactgcagga 148800
   cactcagaat aaagtggaaa tatattataa tattgaccca tgatttgctg gtagaggaga 148860
   aactagcatt gctactgtgg cagtcacttt atatacttta ttttatttaa ttcttttagt 148920
   aaactcataa tatagacttt ttgttttgtg agttcgtgtg ttggtgggga gagagtaatg 148980
   tatttattta tttaattttt tttaatgaag aaactggaga tggaagaaat ccaggatttg 149040
   tccatcaggc tcacacagct gccaagctgg tattgattgg cccaggtgaa ctctcactca 149100
   ttctaggata acatccttcc tcccagtttt ggaactttgt catgaccatg cctaaatctt 149160
   ttaaaatgcc aaaatttcca aagtttgtga aggtatattt tacaactatt tgaaaaggca 149220
   tcatgaatac ttaagaacta gggtgatgat taatttccaa attttcccct ctaaactaat 149280
   taaatgaatg atgttgaatc atctgtttta aaaaaatcac tgagagttag aattccttta 149340
   gtgttgaact ccttaattta aattatatta tatagaagtt tatccatgga ataaaacata 149400
   acctaactgc cagttgaaat aaaattgtgg ctgacccttc caaagttctg tggataaact 149460
   cctatgcata cagcagcgag gatgaatact taacactccg gaatcccatt ctgagagcat 149520
   atttgcattt tcatacggaa ggtgtgatgc caacaagtaa tttcgaaagc taattacaaa 149580
   agccatcttt aaccttctta ttgctttcac acaaagaatg tggctttaat atttcttggt 149640
   gttaatgtac agttagaagc gatactgatg aaacaggtcc tgatatctgg ctgttttctt 149700
   ttacatgaaa aattctgact atgctcaagt actctatcag agtgagcaat tgcttcagta 149760
   ttaattctct ggaaggaaga atgtataatc agcaaagttt gaacaattag tgatgtatgt 149820
   accactatgt acctgggcag gttgagtgat ttctttgcac atggacttaa acaggatgct 149880
   gcataataag gtaggctttg caatcttaga agcttttaga gggagggact accagcccca 149940
   tttcaagata agggctgtga ttgcctgtca aatcctttgg aaacaggacc aagtttgtaa 150000
   atcaaaggtc aaaactaatg acctcaaata aaaatgttta taaaaataac ttgcagactg 150060
   aatatggggt tttcttgata ggaaataagt attgttctgt gaagaaatgg gcaatattgt 150120
   ttagatataa agaatgtaag agggactttt attggagtac atttgtagtt ttcattgtaa 150180
   ataatagtaa tagactatga tttgtgactt atttgtgaat attaatcatt aaaattaggt 150240
   gattattata aataaacgtg actagaaatc catgatgtct tcacaaattt ttgtccagag 150300
   caaggaaagg acacctatgc aaaagagcct atctggaata aggttgttta ctatttaaac 150360
   cctaccaagt tcaggctatt cagtcaaggt ggtacagact ggatggtggt tgaaaaatga 150420
   ccgacagtct ttttctttcc gtagagtctt atgattttcg tgacagttgc ctaaagcttt 150480
   ttggatcacc tttaattcta ataacaactt ttcttcttta ccctcgctct tcctttcctc 150540
   actctctccc accaccgtct gagtgtcctc agtaatcaag tgctttcctc ttttggtaca 150600
   gatgtctgtt gggactctat gggttttatc ctggtaactc agggacccct gcgtttgaaa 150660
   cacctacaag tgcatttctt cttaggcatg tctgagaata tgtttctagg aaactagcag 150720
   gcagctgcaa ggtgcacaga tgttgttgcc ctgcctatat gcccttggct ttctctggct 150780
   gctgagcctg tgtgtgggga cagacagaaa tgctgaggaa ggctgggtac catggctcac 150840
   acctgtaatc ccagcacttt gggaggccga ggcaggtgga tcacctgagg tcaggagttc 150900
   aagaccagcc tggccaacat ggtgaaaccc catctctatt aaaaatacaa aaaattagct 150960
   gggcatggtg gtgggtacct gtaatcccag ctactcggga ggctgaggca ggagaatcac 151020
   ttgagcccgg gaagaggagg ttgcagtgag cctaattgcg ccattgcact ccagccttgg 151080
   caacaggagt gaaactcctt ctcaaaaaaa aaaaaaaaaa aaaagaaaga aagaaagaaa 151140
   gaaatgctga ggactacccc tggaagaatc tctctcaaac ctctcaaacc agtgatggat 151200
   aagagctgat ggacaaatgc tgcagcttcc tagatccagg gtgggaagca ggggctggca 151260
   gtggtatcaa ctctgaggta catttacctt ctctccagag aaggtcccca gcaggattga 151320
   ggcccagttg cccacggtac tcactttctc attcactcac cttgcattga cagtcttctt 151380
   ttccctgaat tatttcccta cttccctacc attgcttcct gggactaatt cccagataaa 151440
   ctaccctgca ctcaaatcct catctcagag tcagtcttct gaggaaaccc aatctaaagc 151500
   acatatttct tctttcaaaa atgatatgat ataccagacc aatgctgatt ttcatcaaaa 151560
   gctaattctt cctgggaatt agcagtccat attgaagaac agcagccact acacacccca 151620
   catgggggca ttttagatgg tgtgtcgctg tcagcctgcc ctctgcacaa ctccactggg 151680
   cattttgatt cttcttcctg tgaccaccac tctcctttgc ctttctgacc agggacagtt 151740
   cggctgacag tttttctatc agctgaccta agtgagtcaa ttcctatttt tatgatactg 151800
   ttgtctgact ggctgagcaa agaagattgt ttttgtgagg atggagaggt gtatgttttt 151860
   ttcaaatatc tagagtacag catcttgtta tcctcaaaga agaactgatc ttcatgtgag 151920
   ggtgtctgta tgtaaatgtt cttatgagct ctgatgcctg caggcctgct tggcaattat 151980
   gtgcctcaga atgacccaga agcccagatg tgttccagtt atctccatac acactttaaa 152040
   taagcacata tgagtctaca catgaattca cagagatatc taagagagat tagctgttaa 152100
   actcttcttc tcactctaat accaagatac ttcaaacccc aaggtaatca ttatggatat 152160
   agaggatcag aaagccattt cagataaaga cttagaatct gaataatcta tttatctagt 152220
   tcaagttacc gaaatagctt cctccaagcc aatttgcaga tgtcccttgc ttatcagcgg 152280
   tttcattgct gagttccaaa tcattaaaca cttgaagcat gaaggccagg gaaacgagta 152340
   attattggtt tggtgaagtt gcaagggagg ttagggaacc caagagagag ttaggctgcc 152400
   ggatgtagac acaaaggaag aagaggggtg aaggaggaga aataattaag agtctgcttg 152460
   caatcaattg agtgtccatc tgctattgaa gtcatcgtaa caccaaatgc aggtaattgg 152520
   tatttggctg agtagacaat taggctggcc acagaatgaa aggaagccaa agactggtga 152580
   gtttatcctg catccttatt tcctcctctt tctgaagcag agtttgagaa ctgagaagtg 152640
   gcagacagaa cctcccacgg tttctgcaga ggggagtttg cagagcagtg acacctcctg 152700
   actcagaaaa caaatgaagc agtacctgag gagagaaatg ttcaactaca gacgttaatt 152760
   atacaaacaa aggcaataag ggctggcggt tctttgggaa caatttgaaa tatcacttct 152820
   cttatataaa cgaagttgtt attgtgatgg gactattttc taatgattca ggatgctcat 152880
   ttagaatcat atgactgagt ccacattaat gtcactgcat ctgcagtgat gaaactacgt 152940
   tggtgcagtg gtactttata ttaattgagg cttctatcac ttggtaatct gagaatcatc 153000
   actgagtagg actaactaga aaaatgtaag ttagaattta tatagcacac gtttttgcca 153060
   gtcaccgtcc taaattgttt catatgacaa cacatttaac aactccagat tttttttgct 153120
   agtggggaaa ctgaagcata gagctgcaca ggtgaagtgt tggagtgccc ctgactcact 153180
   cctcacccca tcctgtgttc atgctatgtg agcttcaacc tcttggtgtc ctgggaggaa 153240
   ttaccaatgt ttcctggtct ctgcttgtcc ctggctttgc ttggaggata agaggaaggg 153300
   gccatcactg ggcagacaag gatgctatca gcagcaggaa agaagcccca agcaggggag 153360
   ccttgtgccc accaaggaca gatgggcagt cagcaggaga accagggacc tgagtcaggg 153420
   ttggttatca actgagtgga catacaggtg ttttccctct gttcttatag gccttggggt 153480
   atttcaggtc tcctttgaag aagccgctcg agtttaaaga gattctctgt cctctcctaa 153540
   ataacacaaa gtgcgtggtg ttggggaggc aggcagaggg gtgggaagta gaatttgttt 153600
   gaagcaagac atttcctgct ggcttctcat gggactctca gaagaagtct agcttggaaa 153660
   taggaaggaa gcaaagtctg ccctctgttt aaatctgcat tttcaccctg tacctgccct 153720
   tggtttccac catggctgtt ctctggccag ttttagacta tttccgtctc cccaaagggc 153780
   tctggcctct ctctcacctt ggtgtctgct tatatattta gcacatcctt atcccagtcc 153840
   ctgcccccac catcgccaat ccatccaacc ctttccccac atctcagagc cttcctggac 153900
   ccagcattca attctggaat ggataatccc tacacacata cacacagatg cacacacata 153960
   acacacacac acacacacac acacacacct ccctctcttg acttctcttc atctaagcac 154020
   tttttatacc ccagtgcaat tagatcattg ctttttcagg taattgtttc tctgagtgct 154080
   catggggaca aagattccct attctgtgca tctatgtgtg tcctcagtgc tgggcacatg 154140
   gtggacacag aataaatgtt aattgaatgg acaaatgaac tcaccctagt gcctcaaaaa 154200
   aaagtgtttg gccgtatctg aagccatgat gtgcgggttt caaaatcacc atgtatttct 154260
   atgtctggcc ccatgctttt gaaaaacatg aaacctggag aaactctgac ccagttaata 154320
   cccaaagacg cccctgtatg atgagcatca tgtaaagtaa tatttttggt aggcagtgaa 154380
   ggaaatcaga attcaaaatg atatggtaaa attggagttt tagaataaaa tccagtaaaa 154440
   ataaagagca caagggacca tgattgggca ggggaaatac tttacaagtt agacagaaaa 154500
   tgataaacta caagttaaag tgtcatttga agatatccag gagggatgat gtgactcaag 154560
   cttcctaaaa atgagaatct tggcactgta tctaaaaaga aaaatacgca gacttgatat 154620
   atgcagtaga tgctctctga gcgccaccca tagacattga gcttttccat ttgctctcag 154680
   ctgccagtat gggcagcctt ggggctgagg cctctttttt ccatcctgag caaagctata 154740
   cagcctgggc ataagatagg ctggaggttt cagtgaacta acacagtccc caagagcagc 154800
   tctctgccag ttaatctctc aggagggggc ggctcctttg cctcttaggt gagataattt 154860
   ggaaatactt gctttatacc tattctcaga gttccctagc agaactgagc ctcagtcaca 154920
   cactgagtca ctggctcacc agctcaccct taattagctt tcttctcttt cacatattac 154980
   atctttgctt ccctaacaat ggtccctgta cttcccaaat aaactcctta tactcgatgc 155040
   ttgcctcagg atctgcatga ggggcaactt taaacttcaa gaatgtatca gtcaggacac 155100
   agataagaaa gggaatccat cccaggtggt tcagacgaag gggtctcaat gcaagggtta 155160
   cctgcagagg tatggaagca aacaaaagat gttgaggcct ccagggacta gcagcaacag 155220
   gaagccttta ctgttcctag gtcgaagggg ataggggatg caagtgtttt cacaggagct 155280
   gtaatcatag aaggatgcag ctacttccag tgtgccaaag tgggctgcag gggcgcacac 155340
   ctgtaatccc agcagtttgg gagaggcagg aggattgcct gatcccagga agtcaaggct 155400
   gcagtgagct atgagcatgc tactgcactc ctgcctgggg tgacagagca agagcctgtc 155460
   tcaaaataaa acaaaacaaa aaagatacca aacaggaagg gaaaaggaag gaaataccct 155520
   ggcctgtgtc tcctcctgcc ctcacatcat ctacctgtgc ctcccagtga ctgaacccac 155580
   tcagacacca gtcaccaagg gagtctctgt caaatggtcc acatgggtca gcttctgagg 155640
   cacagagaag ggcagatact ggatttggca aagcacacaa agaataaata gcccacatgg 155700
   ttttcctgtt tattacactg gtgacttggc tcctgctgag agcacatcag attctagatt 155760
   ctattactga agaatttaga aagccttttt ttgccactac aaagatgatt taaaaattaa 155820
   ggcattgaga cccattaaga aaattattta aggcttattt agctcaaaga aaagcagtct 155880
   gaaggaccaa ttaatattgt tcattgcatg tagcaacact ttgctaatca agaagttggg 155940
   ctccaacatg ataatttttt tatcagaaac ttccaggcca gggatgcagg cagaatgggg 156000
   ctgtacttca taacacagag ggtgtcattg acatgacatg ttcagtagat aagtactagc 156060
   agaagtagta ccagctgata ttgagcacat gctatgttct agacactgtg ctatgagtac 156120
   catatcacct cttgaaattc tgagaacatc ccaattatac agatactgtt atcccctttg 156180
   tattagtttt ctggggctac tataacaaag taccacaaac tgggtgactt aaacaacaga 156240
   aatttatgaa atttattgtt tccaagttct ggagctggaa gttcaagatc aaggtgtcag 156300
   caaggttggt tccttctgag agctgtgagg gaaaaaatct gttccacata tttctcctaa 156360
   attctagttg ttttctggca atcttttact ctccttgtcc tgtggatctc tttcttcatg 156420
   attatttgtg gttctccctg tgtatatccg tgtccatatt ttccttttct ataaggccac 156480
   cagacatatt ggatcaaggc ccaacctaat aacctcatct aaactaatta catctataat 156540
   gactctattt ccaaataaag tcacattctg agaccttgga agttaggact tcaaaatatg 156600
   aactttagaa gaacccactc agacaccagt caccaagggg gaaaactgta agatgagaaa 156660
   actgagtcag caaaaattga agtgacataa ggtttaccca gttagaaaat gcttaaaaag 156720
   atactcagtg tgctagagaa catgtgtcac aagcctgaga tcagtgctat ggaaagacaa 156780
   gggaagtgct ggattcagat ttttagagat gttagaatca agctatattc aagaaggaaa 156840
   gagattaaat gaagggctgg aaggagagag tgcatttata gtcctgagaa gttggggctg 156900
   gcaagtggtc ctaggagaag gaaaggagaa aatctggtta gatcctctat acagcagcct 156960
   cctctgagaa tgaaggacat ttgccaaggc aggcatactc cccacttcca ccccaaagat 157020
   ccccagtgga catcagatct gccttccagc cagcttttag tcttaagaaa accttctacc 157080
   tatttattag aattcctgga aaagaggagg aaaagctatt aagaggctta aaaatactaa 157140
   tgcctaactg aagaaacatt cagcaggaat gagtgtaatc agtttggtca ttagtttttt 157200
   tgattgaagt ggagaataga aaaaaaatgc atttatagaa agcaacagag gccatgcagg 157260
   atgcagtgca aacatctaaa tgtccccggg aggaaggaaa agaggatcat catgttacat 157320
   tagcaaacca gtatgtccac atgcttcata ataaagaaaa ctggcaagtt atcaatagct 157380
   tttttctgca cgtttctaaa ccttgaatac tgcccttcct tgaagtcttt catggcttga 157440
   ctttcatatc ttcaatgccc tctgatttaa tctgagcagg cacatcacta taacaaacag 157500
   cttcaaaatt ctcagtgatt taatgcaata ggagtttctt cttcatactc cagtccagtg 157560
   tgcatcctgg aaccataatc cttagggagt tttccagggt cacataacta gtgtaagtgg 157620
   cagaaccata atctgagccc aaacagttta tttccagaga gtcagtgttc tcctaacaac 157680
   tacagctcct ggttccagta tctctgtagt cacagttggc taccttctat gacaaacagt 157740
   ccaaattccc aatggtttaa cacaataaag tcctatttct tattcacttc atagtccaat 157800
   gggactggtt cttgttggca gtcttcttcc acatgctcca tcagggaccc aggcttttgt 157860
   tccactctcc tctgtccgca gagaactatc tattcaactg gcatgtgagg agaaagagaa 157920
   agctaagatc agtcagggga attttctgga ccattccaag aagtcaagta aatgcactac 157980
   attcatattt cactggcctc actcagtcac atgaccatat cccagccctt tgggagactg 158040
   agacaggaag attgcttgat cccaggaagt caaggctgca gtgagctatg atcacactac 158100
   tgcacacctg cctgggtgac agagcaagag cctgtctcaa aacaaaacaa aacaaaaaaa 158160
   ggtaccaaac aggaatggaa aaggaaagaa ataccctggc ctatgtctcc tcctgctctc 158220
   acatcatcta ccagtgccta gatacaaggg agactgggaa acataatcca ctatatgccc 158280
   aggataaaca gggaaaggat ttggtcacaa ctttatatct cagacaaatc catgtgtaag 158340
   aagacaacaa ggaggaagaa aaagaagagg aagaaggaaa aagaagcaga agatagatcc 158400
   cccaggcttt ataatatctc ataaggagtt gtatttacct tcatctggtc ctatattaat 158460
   ggaaattaca ttgcagctgc aaataggcag catcactttc tgaaatttct gagaaataca 158520
   gattatcttc cacatgaatg agctagtgtg tagaaacttc attcccaaaa ggacagctaa 158580
   ccgagtctca ctctatgtag aaatataatt attcaccgac tgtggggcat gtatttttcc 158640
   attccttcgg agaacagagc cagaggcagg aacctaaatt gcagcatgag atatttttgg 158700
   gtaaatagaa atgtgaggga gtgtgtgtga atatttgaac agcattccta agaaggtttg 158760
   tggaatttgc tctggagatt tctagtgaac aatatagatt cacttcggtc cctaaaggga 158820
   agagtatcag cccaactatt agtatccaag tgttctgaga gtgcagatta cttaagcgct 158880
   aagttttaag tcctagcacc ctaatgtctg gaatggagtt agagtaaatc ctacagcttc 158940
   tttcacagaa tatataaagc taccttctag agcttgaaca tcattagaca tttttacttc 159000
   acctgtggat catcccatat acaagatgtt gtcattccaa tggatgagga gctgtacaaa 159060
   atatggcaac cccctcttgc ctcctttgac catcaaagtg aatatatagg catagacttc 159120
   ccttcttaca cctatttaag aacataaaga acttgtgtaa gaacataaag aacctcaaga 159180
   acataaagtg gcacagattg tggggttcac ctcaatatgt tttccctttc cccaggacct 159240
   tgggtccccg agtgtttgta gtcctaatat tcaagttgta attccccaga ccaataaaac 159300
   cattgcaagc tcacttcagt gctgcaacct tttggccact gctttgtgct tcacagcttg 159360
   ggttttgaat ctgtaattct tctaagtgag gaaacgcctt gagaggaaat gaagctcaga 159420
   atgtcagatt cacctcacat ctttcttttc tcaccaagat cctcacctat tgagttctgg 159480
   ctgccctagt aggttttttt gtttgtttgt ttgttttcta gacagactct tgctctgttg 159540
   cccaggctgg agtgcagtgg catgatcttg gctcactgca agctctgcct ccctagttca 159600
   taccattctc ctgcctcagc ctcccgagta gctaggacta caggcgcccg ccaccacgcc 159660
   tggctaattt tttgtatttt tagtagagac agagtttcac cgtgttagcc aggatggtct 159720
   cgatctcctg acctcgtgat ccgcctgcct cagcttccca aagtgctggg attacaggca 159780
   tgagccaccg cgcccagccc gccctagtag ctttctagta cctgtcaaca tagcttcaaa 159840
   ataaattgtc aaacttttct cgttgtaggt ggaagcgttt gtacaacacc cttttcagac 159900
   aatagaatta catctttagg ttctggaagg aaacctaaga ctctctatat agtgaagaag 159960
   cctccaaaaa ttaagtaaaa cggaggtgtt tatagactaa caaaaacagg gtatttttca 160020
   ccaggagacc tgaagtaaaa ggagaaattt aagcgagaga aaatcaagag ggagggatac 160080
   acagacatgc agaaaggaat aaagtatgag agaaataata aatgtaaatg aatattaact 160140
   atattaaata ataatgtttt aataaatggc tttcagtttg taaagaatta caaaagatag 160200
   tatgaagcaa atgcaattcc aaggctctta cattgaacag gaagtagtaa gcttactaac 160260
   ataaaaaagg ctataaagag ataaagattt atgttgccat ctccagggat attattcaaa 160320
   gaaaagcaaa ataatatgca attatctagc taatagaagg gggaaaagga atgataacaa 160380
   atacttgatt gacccaaaaa gccagaaagc agagaaaaat aaagatagga caagttgggg 160440
   gaaacatctt ttaagcaaga tagtttattt taacccgtct atattacatt aaatatgaag 160500
   ggactaaacc cttcaactga aatacagaat gtgtggctgg atgaaataaa aaagaaataa 160560
   gaagcatatg ttgcttacaa aagacatccc ttaaatataa tgatacagag aggttggaga 160620
   aataatacta ataaaagaga ccatgcaaaa aaataaaatt aaaaagccga tacagctaag 160680
   tttacataaa aactcaagtg aatgttaagg caagaagcat ttttagagat aaaaatgtat 160740
   tatactttat tgaagaatca ttccaccaga aagatataac gattcttaat atctgtgtac 160800
   caaataatac agcctcaaca tagacgcaaa aattgataga attaaaagga gacattggca 160860
   aattcacaac tcttggtaac tgttaaacaa accaaaagga ataaaagaag atttgacaaa 160920
   atagtttgca aacttaaact aattgccata cataaaacgc cgcacctaac aattgcagaa 160980
   tacacattct tctgaagtgg acataaaata tttgctaacg ctgatcctat cctagggtag 161040
   gaatgtccct ttttgaagac acctctcaga cagcctgtcc ttgggaaaac cttctttggc 161100
   agcacccctg ctccggtctg gagcagaccc agttaggact ccttagcatt taacattcct 161160
   ctacatgtct atatcttctt cctgcaaaca ctggggattc tctccggagg actttttctg 161220
   gtcacagaaa catgctcagg gccatgcaca cttcagggta gaaatgccaa ggagttaatg 161280
   tgtcaggtac agttcccaac tcataaaaaa tgggaattgc tgaataaatg tcccacttcc 161340
   ttgcccttca gatgggacca ccctggggta caacctatac cacctctcag agaccctcat 161400
   caggaccgag ctccagttgc tgacagtagt aacctgttgg aaatatgctc tgcactggct 161460
   gcttttttgg tcctatctca cttcctcatg ttcttaccat tcctcctgag atatctcagg 161520
   taaaggactt gcactcaaac tcttgtttaa ggatcagctt ctgagaaccc aaactaagac 161580
   agggccccat ctgtgcttgc tgatataact ttaacttact aaattacagt gatccatctc 161640
   cttttgtgag cattttgatg gtagagacta tgcttttatc tctgtggcat agcacctggc 161700
   acatgacacc taagaagtgc tcaactagtg ctcagtagcc attgtttaat cagtgaatga 161760
   agcctgattt gaaactcatc atccattagc tctcagcaca aggctgcctg aacggatcac 161820
   ataggtaggt tttgaaaaag agagagtaga aacaaatctg aactcttagg aggacaggcc 161880
   tagattacat gccacaggga atatccctaa acatactagt atgtccaaag aacccagagg 161940
   acctgctatg cagatgcaca tggcaccttc atggaagaaa agcaaggctg atgaaaaggg 162000
   atgtcagcat tcctgagatg cgcttttgtc catcacaatt ttgcctggag ccagccctgt 162060
   ttattacatg aagacatgct ttgttctgtt gggattttat ttgaaatcaa ttcttaattt 162120
   gattttaagg gaagcagtga tgttgggaaa tgagagcacc gtattattct cagagattgt 162180
   ccttgaaact aaatgggtct gccacatcac tgcacgactc tactgttttg ccctatttat 162240
   aatgctttgt ttatctgttg ttcccaggaa aataacctca ccttgacctc ttaagcatct 162300
   tttgaatgtc agcaatatga gaattactat gccaggagtt gggagatgag aaaaaaaagg 162360
   tgtttaccat ccagaaacat aagatataat ggaagagaca gatgcacaga cataaaatgt 162420
   tctcaagatt gaagataagg catgtaccaa gaatcttaaa agcacaaagg atagacagtg 162480
   gacccttgaa caatgtgagg cttaggggta ctgaccttta cacagtcaaa aatccatgca 162540
   taacttttga ctcccccaaa acttaaacta cctatggcct actgttgact ggaagtctta 162600
   ctgatatcag agtcaatgac acatattttg tatggtgtat gtattatata ctgtattatt 162660
   acaataaagt aaactagagg aaagaatatg ttactgagaa aaattacagg caagagaaaa 162720
   tatatttact attcattaag tggaagtgga ccattgtata tatcttcatc ctcgctttat 162780
   gctgaggaga ctgaggagga tggggaacag gagaggttgg tcttgctgtc tcaggggtag 162840
   cagaggtgga agaaaatctg catctaggtg aacacatgca gttcaaacct gttttgttca 162900
   agggtcaact gtgtattcag actctgcctg ggaatatcag ctaaggcttc tgatatggtt 162960
   tggctgtgtc cccacccaaa tctcattttg aattgcactt cccataatcc ccatgtgtcg 163020
   tgggagggac ccagtgggag gtaatttaat catgggggcg gtgacctcca tgctgttgtc 163080
   atgatagtga gtaagttctc atgagattta atggttttat aaggggcttt cccccctttt 163140
   gcttggcact tctctgtcca tgtgaagaag gacatgtttg cttccccttc tgccataatt 163200
   gtgagtttcc tgaggcctcc ccagccatgt aaaactgtga gtcaattaaa cctcttttct 163260
   ttataaacta cccagtctcg gtatgtcttt ttgtttgttt cttttgttga gatggagtct 163320
   cgctctgtca cccaggctgg agtgcagtgg tgcaatctca gctcactgca agctccacct 163380
   cctgggttca cgccattctt ctgcctcagc ctcctgcgta gctgggacta caggcgcctg 163440
   ccacaatgcc cggctaattt tttgtatttt tagtagagac ggggtttcac cgtgttagcc 163500
   aggatggtct tgctctcctg acctcatgat ccacctgcct cggcctcccg aagtgctggg 163560
   attacaggca tgagccacca tgcctggcct cgggtatgtc tttattagca gtatgagaat 163620
   ggacaattac agcttcttca agcttgttct ctctaataca ttctcgaagg gcacaatttc 163680
   ccacagaaat aaagatccca aggagctatg agatcaagac acaaggttgt gaacaaccaa 163740
   agactatttt ggaaaaaatg agtcttgtta gagcagaaac atagagaagg tagagaggat 163800
   tggtagaaac tgaggctaga gacaggatct tgacatttgc ttgtgccatg ggatggcaat 163860
   ctgatgagcc catggtccct tctcagaata atttcaacta taggtatttg aagttttcag 163920
   aaagttgaaa aaattgtaat atgatagcat atatgcttct ttattaacac attaaataac 163980
   aagacctaac agtaggccta atacctacta caattttaag tggtgatgag cataaacggt 164040
   atttcaagaa agccactata ctgtcctatg atatttaaat acctgtgatt tccatgagtg 164100
   aaagagtcac aggtactgct actacgatta tggtctgttg cttacatgca taatttagga 164160
   aaatatcagt ttccattcac agttaaaaaa gataaagtta acagttttct ggtcaaactg 164220
   aaatccaaat ggtcatagcc attctggatg aggttaagaa cacacaggtg agaaattggc 164280
   tgatgttcca agagttcaga cttttatcta caagtaaatg gaaactagtg agtgtttaca 164340
   atggaggggt tgctggaatc aacatttttt tctaaactta agtgtggtca cacccagggt 164400
   ttcatgtcta aggccacctg ttgcagccta tggtgcaggc cctgctccta gaaaatcaaa 164460
   gtagaaaaag aaagaagaga ccaatatatc tgagagtcta caagcctgag ctgctcagcc 164520
   ttagctcttc tactccctct agtaaggaca gagcgttatg ccgaggctgg agtgcagaag 164580
   ccggggtctg aatgtgatgc atcctattag aatgctgagt cacattagat ttgttggaag 164640
   agagatccca agtgtggagg aaaacctgag cttcatatta aagctgaata cattatgaag 164700
   tgaaagagaa tatttttatt gctttaaatt tttaaaaaag acattccttt ccttctgtct 164760
   tccatgcaaa ataattataa caacaaacac acacacacac acacacacac acacacacac 164820
   acacacacat gcacataccc tgattttgat ttccctgtgg ttttcaggaa aacatattta 164880
   ttttcctctg tctttagata ttcctcaatg ataaaatgtt agggtctctg aaatgctagc 164940
   caacacccat ttggactcag tattctctaa ttttattgtc agaatatcag atatgcatta 165000
   atgcacaaaa cataagcata ttaaatatca gcaaatatac aaaactgtga acacaattga 165060
   gagtggatta acaaagtttc tttgtccttc tgggtattga attatcacca atcaagatga 165120
   tttcatatca aagccaggac agtgtttcat gtatatgtac atatttatat gtatgtgtgt 165180
   atactgatct accttttcaa tattctccta aagactttcc tttattttca tattgagaaa 165240
   acacaattca gaattcagaa caagtccctt tctccctcta tctcacttcc caaccatgtt 165300
   ttccagcaaa gctgaaattg aaactatcct gttttctttc ctgtttgtta aaaaatgaca 165360
   tcaataatat tcacacagtt cccaggaata ggatcctact aaactctctc ttatttttct 165420
   ttctgtagaa attgtaattg tagtttatta ggtccttcaa aaataataaa tcataatttt 165480
   ataaaaagat caagtgtaat attttagtat tcacattaaa tatatagatt gacccaattt 165540
   aaataattca tatattgcct aaagcagttt gatatggttt ggctgtgtcc tcacccaaat 165600
   ctcatcttta attcccacat gttgtgggag ggactcggtg ggaggtaatt gaatcatggg 165660
   ggcaggtcct tcccgtgctg ttcttgtgat agtaagtctc acgagatttg atgattattg 165720
   taagggggag ttttcctgca catgcttttt tcacttgctg ccatccatgt aagatgtgac 165780
   ttgctcctcc ttgtcttcca acatgattgt gaggcttccc cagccatgtg caactctaag 165840
   tccaattaaa cctctttctt ttgtaaattg cccagtcttg ggtatgttct tatcagcagc 165900
   atgaaaataa actaatacac agtttttgtt gccttacttt accaatttac aattatagct 165960
   atgttgtaag tgttacagaa ttaaaaatta aacattgctc ataacaagtt gtcaccaaat 166020
   agccttgtga ctaacagaat aagcagcaag acacatggta tcagttgact aatttgtttg 166080
   ttatccataa atattttggc tgactatata gtttgccttt cttctttaga aacatgcctt 166140
   cctctcataa atgttaattt taagaatgtg aactgaataa tgtaacagaa tggggaagaa 166200
   atgaatattt ggaacaatta attattaatt ggagctgggt atggtgccat gcacctgtag 166260
   tcccagcttc tcaggaggct gaagtgtgaa gattgcttga gcctgggagc tcaagaccaa 166320
   cctgggcaac agagagagac tccatctcta aaataaataa ataaataata aataaataaa 166380
   attaattatt aacctgttaa gttttatcca ttcatttatt caaatgttga acaaatattt 166440
   aaaagcacct actgtatgcc atgctgtgtg ctaagtactg aggtcctact tgtccttttg 166500
   gttttaagtt agagctttgt cacccccaag ctgtatgatc cttattttat tgtccttgca 166560
   ttagaaaaga atctcctctg tgcttctatc agtatgcagc tggactgctt taactcttct 166620
   cctcttttat tttttgtcat aacttgtcat tttttaactc ttctcctctt ttattttttg 166680
   tcatatatat ttagttctgt ctactcagaa agcttctagg atctccatag ccttgcaatt 166740
   ctcagtctga ggaaacccct ttactcaatt aaaatacaaa caatttgtaa atgtactgtt 166800
   ttcctcagga gccagagttc ctatttagga ggaattgtgc agcagatgtt gtaagtggct 166860
   cacccagatc cccagttccc cctccttggg ccaccccagc tgctgtgagt gctgactact 166920
   aatgacctgc agctagctcc ttctctgagg cattactctc agccaatgga agcatctcca 166980
   cccaagatgc tgaactcaca ggtgatgact gacatgggtt acaaacatga ctgacatggg 167040
   gtacaagctc cctttgcctg ggaggcagct tatgctccag agttcccttg atattccctt 167100
   gatattagac tgagactaga ctccagctca ggtcacagcc tgttcagctc tcacttccac 167160
   ctgccctgct ttcctgaaag cactccctca ataaatctcg agcatttaaa tcactgcttc 167220
   aggctctgct tctgcatact caacttaagg cagcaagaat cctgccaaat tttgccttcc 167280
   tttgtctgcc aaaccaggac atctcagcct gttccatatg actttcctca tttcaaataa 167340
   caaaatcagt taagattcca atgtcaaaga aatgtcaaag attactccac agcaagatct 167400
   atagagggaa gtacctcaga gggaaacata taagaaagct tgtatcgtat tcttcctcta 167460
   gtattgatcc ccaagtacac tgacttcagg acacataggg tctcaagttc tttttgaggg 167520
   gtaggcaggg agagggttca attccacata tgcctgttga atctatgtat cttagtaatc 167580
   agagactctc ttggttagct caggaaaaaa aaagggtgag gagtcattgt tagaactgtg 167640
   gatctcaagg atagctaaaa ataggccaca gtagaagcag acttggaaac tcagtcttac 167700
   tcccttcagc aaggccgtgc cattctcatt ctgtttctgc tgctagactt cctggctttt 167760
   ggggcttttc tttcctcata attcccattt acatggggcc attatgcagc ccagagtctt 167820
   ctgtagcaca acatatcaaa atgtattaca tcagtcccat ttgcatgcta actagcagaa 167880
   tctcctagtt ttctgattct aaatccatgt ttccaattag cccagtttaa cttttctcac 167940
   cagattttaa gctccttatt gggcagctta tgagcagact gcactggggt cagtggccct 168000
   cccaggagtg ctgggggttg gtagggcaag tatgttgtct acaacaatta ggcttccatg 168060
   cggtaggatt gtgggcaagg gaaaatttcc cagagggggt ctgtgggcag aaatgtccca 168120
   taaaaactat aatacagtat actgatggac aagtaggagc tcaaagaaca gtttcaactt 168180
   gattattgtt agaacagata agaggaaaag ttaggaggca gataaactgc cattaaatca 168240
   accagataaa taatagtaga atcatttata aatactgttg acatctcctc agataacata 168300
   taacctccaa ggctttagag atgtactatc cagtgtggaa gttactagcc acatgtggtt 168360
   atttaaattg aacaaaatta atggaaatta aatataattt taaaaattaa ctctgcagtc 168420
   tcagcagcca catttccagt gctcaaaacc tccatgtagc tcacggctac tctattgaca 168480
   gatactgaca catgtccagc atttgaaaaa agttctcttg gaatgcacct atttaaaagt 168540
   attattgctt ttatttaagg aaagagattt gagagattca tttctttttt aattcttttt 168600
   ttttcttttc cagtaaatat gataataaac acaatttgaa tccctttcct ggaaacacat 168660
   taaggattag gtgccaggtt tcttccatct gttataacag gaaggccaac tggtattttg 168720
   tagttatttt aagtgtaata atatgtcaaa cacactaaaa acattctatg tctgtcactt 168780
   aatgttaaga tttctctctt gcccatgtta tttttttcct ttcatatggg caattgtttt 168840
   gcataaagat gaataggtag agagaagggg aggtgagcat tattctttga gtctctgtac 168900
   cttagagtca gaagggacct caaggatcta atagacattt cccatctaat gcaggaattg 168960
   catttgctgc ctcctttata ggtgaagcta ttgctgctca agaattacca aatgcagact 169020
   actgttcaag agccatctat ttgtatctat acttcctatc tgtatatgtg ggcaaagtaa 169080
   aaggaaagga aatatattat tgtaattctg agattgaaca gatacttatt ttcaactttt 169140
   tgctttttag gatttactga gtgtattagt ctgttctcaa gctgctaata aagacatacg 169200
   cgagactggg taatttgtaa gggcaagagg tttaattgac tcacagttcc acatggccga 169260
   ggaggcttca caatcacggc agaaggtgaa ggaggggcaa agttacatct tacatggcag 169320
   caggcaggag agcgtgtaca ggggaactcc cctttataaa accatgagat cttgtgagac 169380
   ttattcacta tcatgagaac agcacaggaa agacccaccc ccatggttca attacctccc 169440
   acaggcttcc tcccatgata ggtgggaatt atgggagcta caattcaaga ggagatttgg 169500
   gtggagacac agccaaacta tatcactgag tatctagtat gtgccaggat ttcacatgta 169560
   tattaaataa aataaccata catactgaaa tatatcaggc attcattaaa tgttatgtat 169620
   ataaatgggt tgcatttcca gttattgtca tgtaagcata atttggagta aaaaattttc 169680
   atggaaatta ttttaaaaat tgatgcgtta gttcctaggc tgccttcaaa agctgaatta 169740
   atctataata tatggctata tcaagattac ctctgtagga aaatctagtt ttttttgtcc 169800
   ttcctcgctg attatctctc ttctttcttt catttgttca ttccttcaac aaattccaca 169860
   ttcagagact aggaacaaag ctctttctta tctagatttc tgtagtgtgt ggctgatttt 169920
   tattaaggat caggagttct ttgacaggaa cagggcctat tttaaagtca ggggtacctt 169980
   ttactgaatg gacaaaaact ggaagcattc cctttgaaaa ctggcacaag acagggatgc 170040
   cctctctcac cactcctatt caacatagtg ttggaagttc tggccagggc aatcaggcag 170100
   gagaaggaaa taaagggcat tcaattagga aaagaggaag tcaaattgtc cctgtttgca 170160
   gatgacatga ttgtatatct agaaaacccc attgtctcag cccaaaatct ccttaagctg 170220
   ataagcaact tctgcaaagt ctcaggatac aaaatcaatg tacaaaaatc acaagcattc 170280
   ttatacacca ataacagaca aacagagagc caaatcctga gtgaactccc attcacaatt 170340
   gattcaaaga gaataaaata cctaggaatc caacttacaa gggacgtgaa ggacctcttc 170400
   aaggagaact acaaaccact gctcaatgaa ataaaagagg atacaaacaa atggaagaac 170460
   attctatgct catgggtagg aagaatcaat atcgtgaaaa tggccatact gcccaaggta 170520
   atttatagat tcaatgccat cctcatcaag ctaccaatga ctttcttcac agaattggaa 170580
   aaaactactt taaagttcat atggaatgaa aaaagagccc acattgctaa gtcaatccta 170640
   agccaaaaga acaaagctgg aggcgtcatg ctacctgact tcaaactata ctacaaggct 170700
   acagtaaccc aaacagcatg ctactggtac caaaacagac atatagacca atggaacaga 170760
   acagagccct cagaaataat gccgcatatc tacaactatc tgatcttcga caaacctgac 170820
   aaaaacaagc aatggggaaa ggattcccta tttaataaat ggtgctggga aaactggcta 170880
   gccatatgta gaaagctgaa actggatccc ttccttacac cttatacaaa aattaattca 170940
   agatagatta aagacttaca tgttagacct aataccataa aaaccctaga agaaaaccta 171000
   ggcaatacca ttcaggacat aggcatgggc aaggacttca tgtctaaaac accaaaagca 171060
   atggcaacga aagccaaaat tgacaaatgg gatctaatta aactaaagag cttctgcaca 171120
   gtgaaagaaa ctaccatcag agtgaacagg caacctacag aatgggagaa aatttttgca 171180
   acctactcat ctgacaaagg gctaatatcc agaatctaca atgaacgcaa acaaatttac 171240
   aagagaaaat caaacaaccc catttaaaaa gtgggtggag gatatgaaca gacacttctc 171300
   aaaagaagac atttatgcag ccaacagaca catgaaaaaa tgctcatcat cactggccat 171360
   cagagaaatg ctaatcaaaa ccacagtgaa ataccatctc acaccagtta gaatggcaat 171420
   cattaaaaag tcaggaaaca acaggtgctg gagaggatgt ggagaaatag gaacactttt 171480
   acactgttgg tgggactgta aactagtgca accattgtgg aagtcagtgt ggcgattcct 171540
   cagggatcta gaactagaaa taccatttga cccagccatc ccattactgg gtatataccc 171600
   agaggattat aaatcatgct gctataaaga cacatgcaca tgtatgttta ttgaggcact 171660
   attcacaata gcaaagactt ggaacctacc caaatgtcca acaatgatag actgaattaa 171720
   gaaaatgtgg cacatataca ccatggaata ctatgcagcc ataaaaaagg atgagttcat 171780
   gtcctttgta gggacatgga tgaagctgga aaccatcatt ctcaggaaac tatctcaagg 171840
   acaaaaaacc acacaccgca tgttctcact cataggtggg aattgatgaa aacacatgga 171900
   catacaggaa ggggaacatc acactctggg gactgttgtg gggtgggggg gaggcagaag 171960
   ggatagcatt aggagatata cctaatgcta aatgttgggt taatgggtaa agcacaccaa 172020
   catggcacat gtatacgtat gtaacaaacc tgcacgttgt gcacatgtac cctaaaactt 172080
   aaagtataat aataataaaa ttttaaaaat caaataaaat aaaaataaaa ataaagtgag 172140
   gtgtaccttt ataataactt aggcttggct gggtttcttc tcgtagggta aattagaaag 172200
   caaaattgga attagcttgt ggccacttct cagtacccaa accaaggcag agatgctctc 172260
   agcttggatg ttgcctagat gatgatagat aaagctcttc tctctttttc ttgagaaaaa 172320
   caatattatt ttttgatata ctttcctaca aaaacctcaa attttaatcc aaaaagaaat 172380
   tctagtccaa gcaaagtaga ggaggacttt tgatatgatt gcacctaaat atagaagaaa 172440
   aggcatatgt gaaccactta aaaataacat aatcacttaa aataaaagtt aaaaagggaa 172500
   attcagtttc tttaagatat ttctagaagg aggcttgtta tatatgtaac actgtcatgt 172560
   gtatatagat aagtatataa gaaataaaaa ctagcaagta aatatattgg taatctgagt 172620
   tcaagagaaa ccatcctaaa tacacttttt actagatcta aaataaaaac caaatgacat 172680
   tttacctcca aaaatggata ttgcccactg tttgaatatt tgctgtgtgt gtcttaaaag 172740
   atgtgttaca tatattgcat tacagcatgt gtaattatga atataacaat attttttgta 172800
   aatcactttt attatagaca tgtccactga ttctacaatc agcacaaggt tggattttgt 172860
   gttccattat acattaaaaa tgtctcttat gatcctgtgg atccaaggaa aagatctctc 172920
   gaatatgcca tgatattggg caccaggagg ccactgagtc agtgctgatc tgcatgcagg 172980
   ctcctcgtgc cttccccagc aatgcccagt atagatgtgg atattccctt ttatttctgc 173040
   acttaggtaa ctgccttaat agatccagat caaattaaca aaactgaaag catcttaata 173100
   aaatttatca gtagaacaga ggtgatgatg tcagtcaagt aaatagcacc ctggtatcca 173160
   ggaacagctg atacaacttc taacatgctt ttgtttattg gtttgagcat gccactccaa 173220
   tatgcaaaag tgtcgttctc gttctcagtg ttatgctctc gtaaaattca caggttccct 173280
   ttccaagtcg ccagtcccct tgttactggt ccatctgttg actcccttac ctaatcctgg 173340
   ctattttcct tcattataag aaagggcaaa tgagaaagag tggatgactc agccttcatg 173400
   atctggcatc ctgctgaagg caggtagata tcatctttgt gtataaacat aacattatat 173460
   ttttcatcta atcatgaagg atttaaattc aggcaagaat agactccctt atacattctt 173520
   caggcaacat atcacaaagg aaaaaaaatg aaaatactcc attaggctca aaatttcctc 173580
   tgaaaaaaca aagacataga tatgtacatg ctttatgttt aggtttctat taccactttg 173640
   gttggattta tgtgctttca gtatgaattt aggagtacat gtgctattgt cttaggttgt 173700
   atgagagagc tatgaattta aattcttaga tggataccct aacatgcggc ttctcttttt 173760
   aaaggacaaa ttatgtgaaa aagcaattct taaggaaaat agttctttcc tcttgttctc 173820
   agtggaagct gtcatgactc aaatggttca aggagattct actcaaattt ccatttctca 173880
   gcttctgatc tatttggtat aattattaac catatattca aaaatgaata tccttaatac 173940
   tggcaagcta ctattactta agtgatttta gatactatta aacacattag gcaggaaatg 174000
   ttttgaaaag gtactaacag agcatgcaac aatttctcat ttcatatgtg gaaccatttg 174060
   gaataaacac acatacataa atctgcatat atacattatg ggctttaagt tacgaaattc 174120
   tatgttctag tgatctactg ctgtgtgaca aatgacccca aaacttagtg ttacaacaaa 174180
   gacaacttta tgatatctca tgattcagtg ggtcaggagt ttgatcaaca tttggttggg 174240
   tgatttttat attccacagg gtattattag agcttcccca gtggtatgca actagaagat 174300
   gaactttctg gaaaatccaa gatagcttca catatgtaga ccttggtggg aacagatgaa 174360
   gggttgggtt cagctgggac tgttgactgg agcacttgta catggctagg gtagttgaat 174420
   tcaatactca gggtagttga actcaatact ccaagagcaa gtgctccagg agatagtgaa 174480
   tggaagctgc tagtctctta gggcctgaac acagactggc aaagcataac tctgccacag 174540
   tcactatagg cattccaggc tgaagaggag gagaattata ctctacaatt tttttttgac 174600
   atggagttct gctcttgtca cccaggctgg agtgcaatgg tgcgatctca gctaactgca 174660
   acctctgcct cccaggttca agagattctc ctgcctcagc ctcccaagta gctgggatta 174720
   caggtgcaca ccaccacgct cagctaattt ttgtattttt aatagagata gggtttcacc 174780
   atgttggcca ggctggtctc aaactcctga cctcaagtga tccaccagcc ttggcttccc 174840
   aagtgctggg attacaggca tgagccacta tgcccgatct actctacatc ttaatgaaag 174900
   aagggacaca aaatttgcag ccatgtttaa tctatcacac tctgccctgg ctgagaggga 174960
   gaaatgtggc atcatggtta agaatgtgga agactagatc tgtcactgat tcactgtaag 175020
   gttgtcatct tttcatctaa aactaagaat agtaacaata ctcatccata tctcatagag 175080
   ttatgctaaa aatcaatgtg acaagcaaca tacctataca tacacacaga tacaaacaca 175140
   catacaaagc actcaacaac tttttgctat ttgttttttt gttttgtttt gttttgcatt 175200
   ttgttttgtt ttttcttgtt catctgagct tatggtggtg gaggtgggga gacattgtag 175260
   ccagaagtaa gaaggccact tggagaacaa ggaaatgggt atcattgatc aggcaatcta 175320
   atgtcataac tcaaaatgtt gtggaacaga acttgtctcc acggtagtat ttcctacttg 175380
   gttttgcttt ttgtttttgt ggtttcattc tttctttctt tctctttttt ttttttcatt 175440
   ccgccttttg ctgttgttgt gcttttgttg aagttttttt tttttttcca agttctcact 175500
   gcattgaaga atgatgcctg acctttcatt ctttctgtta ttgtcactgg actccagctt 175560
   gctattagtg tcttcctgtg ggcagtcagg aggctatatc atgcttgttt caatacagta 175620
   aatggctaat ttcaattata tgactcaagt tatgccctca aatggagggc atgtctgtaa 175680
   atatctttgt aaagttatat cggattcttt ctcatttctg ttattctgag aaatagtgct 175740
   gaactctact tttcccttcc tccttccctt cttcttcagc tcatttacaa ctaaacagaa 175800
   agcactttcc tggcaggcta gttaaggaaa aggctgagat attgtagcaa agagtcttac 175860
   aacatcctat acattaaaga cggaggctta tttctctttc ttaatagcct gggccatgga 175920
   gtccacaaca taacccaggg gtccagtaaa actgaaaggc tctgccatcc ttaagatatg 175980
   gttttcaagg ttcttctaat tatcaccatt tctaagcagt gggaaggaaa aataggggcc 176040
   aagtgcaggg caagcatttc tatatcttta gaaaaatgac cagccatgca cagtggctca 176100
   cacctgtaat cccagcactt taggaggcca aggtaggagg attgcttgag gccagaagtt 176160
   caaggtcagc ttttggacaa cataacgaga ccccatctca aaaaaaaaaa aagaaaagaa 176220
   aagaaatatt gagacctggt ggagtgcaac tgtagtccca gctaatcagg aggctgaggt 176280
   gggaggatga cttgagccca ggagactgag actacagtga tctgtgatca tgccactgca 176340
   ctccagtctg ggtcacagag caagaccctg actcaaaaaa taaagaaaga aaagctagaa 176400
   gtcacacata atttccgctt atcttaatgg caagaatttt gccacatgtt cacagctagc 176460
   agcaagggag tttgaaaatt aaagtctcca gccagcattc atgtggccag gaggaatgga 176520
   tttgtgggcc caggggagtg ggtggatgag atattaaatt gaatgccaca tttcctgctt 176580
   tatcaaataa ctcagcattt taataactga tacacaatgg tgactatcca gttgtccttt 176640
   caagccttac aagttggcca ggcacagtgg ctcatgccta taatttcaac actttgggag 176700
   gccaaggtgg gaggatctct tgagcccagg agtttgagac cagcctaggc aacacaggga 176760
   aacttcatca ctacaaaaaa aaaaaaaaac agaaaaattt agctgggcat ggtggtgtgc 176820
   acctatagtt ccagctactt gggaggctga ggtgggagga tcacttgagc ccaggaattc 176880
   agggccatga tcatgactct agcctgggca acagaacaag actctgcctg ttaaaaaaac 176940
   aaaaagactc acaagttgta gtaccaaaat atgcctgcca tacccatttt ttgagggatt 177000
   ctttttttta gtaaaataat tagacattta tgggtcaagg cagcatctca atggatgttt 177060
   ttctgattgt aaatgtcttt tagccttgta tttatgaggc tacaatatgt aacatgaagc 177120
   tgaaggctat gaaccatttt ttaaaacaat aactatcatc caagcatgaa tattgccttt 177180
   ggtactactt aggacacaag acgataacta cagaagaatg tgggcgaaat aattaaaaac 177240
   cacagcagca gcaaatctct tggttatttt ataaaaacga aatgatgttc cctaagggtt 177300
   aaaggctatc tgacaaagtt ccattgtgta atcaaaatgt ttgtgtctgg gtagataaat 177360
   atagtataga tgctgtaata ttgcagtttg tcagccattt ttgatatatg ccttgttgat 177420
   ttccatagtc ctgattccca aaacagctaa gaatcataga attttagagc tggatgggac 177480
   tgactgaggt ctgatttact ggagggaacc attggaggtt gaagcatttc ttgtgatgac 177540
   ttttctcatg ttctttataa agagggtgtc aacccactga acagtttagg gagaaaagga 177600
   aatgctgagt tcctttcagt ctacattaaa gctctacact cttggttagg atctatggag 177660
   ccttgctcag gtgagataat tgagccccag agaagacacg tcactctatg ttgaccattc 177720
   tgatatgtca tctggggctc tttctatggt acagtgtaat cctgatggaa gggactttct 177780
   gcctgtgacc tatttataaa atcccccatc atgaggtaaa atatgcactt attcgtagct 177840
   gcctttttct ttcaatcaac tttatgtttg tggaattcat tgaacttttc caaatggttc 177900
   tttctccctg ctatgtagca ttgcaatgta ggaatatacc tcaatttatt tatgcattcc 177960
   actactgatg agcctatgga taatttctag tttgtgattc ttaaatggga ggcaacaaaa 178020
   gccagttaca catagaaaag tagaaatata aagcaatacc tttaagcact cgtgttattc 178080
   aaaaagttcg catcttctcc tactgaggga atatatttta aaactgaccc tctcaatggt 178140
   aatgtaagat tccttacctt tactcgagag ctcagaaaaa gcctgctgga tatggtctcc 178200
   aaggtgtagt cctgccttac atttccaaag ggaagggtaa tttttgctga gtttacatat 178260
   ttcataaatc tgacattact gaaagtagga tcaataagcc cctttccatt ttaaaagtgc 178320
   ctgagatccc actgttctac aaacttaccc aaattaaata gtagtattaa gttgaagagg 178380
   aaaatgcatt ttggcatttt tcaaagattt gaatttcaaa ggtttattat tatgcataac 178440
   agagagaggg aaatgacaat gctatttaaa taaacttcac tagaaacttc acattaaata 178500
   cctcttagac acagcttcaa gcacagaaat taggagttat tattctagca atatttcctt 178560
   cctcgatgtt attttgccag accaacaagg ggccagtcac atagtgggat tgcttcgtag 178620
   gaatgtaaaa cttctagact aaaatcaagg ttgggtaaaa acaaaatatt gccccaaacc 178680
   acatcagatt ttaccattgc aatggttttt gcttcattga tttatacatt taaaggagca 178740
   aaacttaatc tgctataatg tattacaata gaatccatct tctaaactat gtgataattt 178800
   tattaacaac tctattgact gacagtcttc cttaaaaatg ttactgctga aaattcacat 178860
   agtggctaat aaccaccatt gttgccattt tatatttgca ttccattcct tggctcatta 178920
   aatataatgt aataaacata tattaaaagc cagaggagtt tgacatgaat cctgcatctg 178980
   ccactagcta gctgagtgac cttaggcaag tgatttcatt ctgatactta gcttttttct 179040
   ctttgatgtg aggctaatgc tatttgctga cagcatagtg ttgttatcag aaaaaaaaat 179100
   gataagtata ttagagtaat gcccattgtt ataacaacta gaccccagaa cttcattagc 179160
   ttagtccagt agaatttaat ttcttgcttg catgaagtcc agtgcaggtg ctcctggtca 179220
   gcaagtggat ttcctccata cagcagttca gggatccagg ctccttctac cttgaagttt 179280
   taccaccacc cctgcacttt gttcttctct gcatccagcc tacataaaat ccctaatctg 179340
   gaagtgaatt atttcacttc tgtgcacatg ctattggcca ggataagtca catggcagta 179400
   cctggatgca aagggagctg ggcacatgtt tgcccagttg taagctgggc agctaatttc 179460
   tagggtgagt tccagattac agaaagggga gcatgtgaaa tccataaaca ttgtgaagct 179520
   tttgttgctg ttgttgctgg tgacatttta ggtagtcttc ttgttagcgc catcatccta 179580
   aaccagcatt cccataatca tattgattga acatggtgaa catcatcatg ttatcttaga 179640
   aaagatgacc aagaagtgat cacagttgga aatgcaagtg ttattaactc ccaatgcatg 179700
   gtgtatagca ctgacaatat tttgctgatt ttcccatcac ataggcacca aattatgcta 179760
   aattataaat tagatatatt aaaattccag tgccaaaatt attcaagaat catagtaaaa 179820
   gacctctaca gataaacccc aagcttctat tgtagtatat agttatctga agggccttct 179880
   gtcttccagg accagctatt gtccagagaa gagcccagag gggctgaaga aagcccccag 179940
   gagaccatgc taaggagaca tatcctaaat gatcatttca ctgtgctacc ttttgtggtt 180000
   gtaggagcca taggcttttg gcccctaaag attcactgaa aaatcactga cgtgaggaag 180060
   attgattaat aggagaaaag aaatacaaat ttatttaatg cctttagtag gggcattaaa 180120
   taaatgcctt tagaatgaag gcattctaaa atgcctttag aatgaagacc cctcttccaa 180180
   taggttacag aaacttatat tttatcttga ggttaaagaa agaatgggag cttgtatcct 180240
   ggtaagacca gttatgagag gtgggagaag aataattcta ttgaggggca ataaatgata 180300
   gaaaggggaa taaatattaa cttgtaaata gccttctcta gaatttacat agtcattgga 180360
   gacaattcat tatgcccata aaaaaggtct tctcaggtgt ggtcacatct tggtcttctt 180420
   ttctgcaaca gataatgaga taacagggaa aggaagaaaa aaacaattgt tcaccttggt 180480
   gggtctggat cttaggcaga taaaggaact tcagcttctt tgggagagac tttgggtggt 180540
   ggagggaggg aggccagagg atccttgagg cttcttcagt tcagcatgtc aaaatgccat 180600
   attttggggt attagtttct gagccccagc gtggtctagg aaaatgaata tacctggaaa 180660
   ccatacaatc acttaattac atcaaaactg aaatcattgt tttagattaa gtaattaaac 180720
   aatcaaatca attttcattc cactcagaga aaaaaaacca aagccctgca gtggcagctg 180780
   atgccctcaa tcacagctgc tcagacttga gtgcacatca gaatcatctg gagagctttg 180840
   tcaaaatgga tggctgagtc tccctgcacc acccctcaga gctactgagt cagtaggttt 180900
   gtggtggaac tccaaaattt gcatttttaa acaagctccc cagtgatgct gatgctgctg 180960
   gtctgcgacc acacttgaag aactactgcc ctgtgcggcc tgacttcctg ttccatctct 181020
   gtctcatgtc cctctgtttg gccctttgct tacccttctt caggttcaca agcctccttg 181080
   cttttccttg aacccaccag gaatatccac atctccaggc cttgcacttg ctgttgactc 181140
   ttactagaac tctttggcca gatactctaa tgaacggtat cctttctaac ttcaaatatt 181200
   tgctcaaata tcaccttctt aatttaagga catcttctca tccctatcca cacaatataa 181260
   aaattctttt cccctgccac agcattcgcc attccctttt ctgcttttat ttttttctca 181320
   cgggccttat catcttctaa tatactacgt aattttccta tttacttgtt tattgttttc 181380
   tctcctatca tgagagcccc ataagggtgg taaggatctt tgtctgcctc cctattgcgt 181440
   cttcagtacc atggacagtg cccggcttca gcaggtgcac aaaaaatact tgtcaaagaa 181500
   aagatggaga gagccctaag ggaagagaga aaggagagaa aattaaaata atcttaatct 181560
   accacagatt tttgcttgga atgcaaatca cttgtcactt gcccacagcc agtttactgc 181620
   gtaatccacg tgcagtgcct ctgttttgcc gtataattct gaaagccttt agtgccctgg 181680
   ttgcctttat ccccctcatg ccaggattat cctcaggttc ttctggggtt tgagagcctc 181740
   aggtggcagg ctgtaagtga gaatctgaaa agattttaca atagaagtgg gcatcttcag 181800
   atttggaagg gagtgacagt cagaggctag ttattattgt ttttgcttct cttattgtga 181860
   ctctcagttc atctgactta ggctgcacaa accaagccca ttcttgccct ttccctttgg 181920
   catacttgct gttatagaga agcaaatatg aagagaagag tcagaaggtg agacttatac 181980
   accatagcag gggtttctat acatggaaag ttcagtttga agctgacatg tcaacaggac 182040
   taccatttct atttgctcag aatgttagat tgtttgggga caaggcaata tccaatgaca 182100
   gattctagtg gtttaaaaga aagaaacaaa caaaacccta aagaactatg attttttatc 182160
   taaaattgta ctacaacttt gtagtattta actactcaga agattccatt ctttactcgt 182220
   aacagcaaat tcccttctta tttggcccag tcttcttcaa caggaaagtt tcttataccc 182280
   aatactctat cgtttaaaat tcatataatc aatgacatta aaagtggaca gcttgacaac 182340
   ttatttctac cataacttta agaaaacgta cagctctgag gttttcagac aagtggtagc 182400
   agtaacttat ttttggaaat aagtcgaaca ccattgcagt tgcccatatg aagcccatag 182460
   tctcaccagg aggtggtgct agcatatgac cctcagatgg aaaagagcag aattatgcaa 182520
   gtgtttgtgt gacccatctg accaatctaa ataaagcaaa attaaggtta cataaggttc 182580
   tctggcttga tgtgaaattc ctatgcatca gtttttatat tcttgaagat tgaaggaaag 182640
   aaagcaggct ctgatgttgt taaacttgac agtgtttcca gttatgattt atggaagatc 182700
   tcattttgat tgccagctgt gtgtacacat gcacacacac ccacaataca attttcaatc 182760
   acctgagaag tttgataatt agatgagaac aacacctacc acgtatctca tagttactgt 182820
   tgcaaagact tctaaagtct ttatacccaa ttatatgggg ggctttttac ctgttggtgg 182880
   gacatgtagc tccttgcaat tgccttttta agagagggca gaagatctca gtgtgatatc 182940
   ttgagtggtg aattccagaa attacagcat tcctctgcca actaccagaa ttagggttta 183000
   ttcttagtgt atctggagct ggctcacatg agcttgtgag agccaattgt gctcatcttt 183060
   aaaaatattt tttaattaaa aaaatgttct gcaatgtcac attggtagct tgaaattagc 183120
   tatgtgacag tatttgcacc ataaaagttg acagacatta caatcagggt ttgtttaccc 183180
   tttttccata gccagttttt aaccacttag aagcatgcca ctgttactta ttgtggtcgt 183240
   ttgcaaacat aatcctaatt ctctatgtct cacagtagcc aatcattggc aatgtgacat 183300
   tgaagctcct ccctttgaaa agcagactaa tttccccacc cattgaatct gcgttggtct 183360
   tatagctttc attgtttata gaagctagtg gaaccaatct tatgtgcctt ttcctagcct 183420
   aggtcttagg aggtatttct tctgtgctct cagaaccctg ataccaccac aagaacaagc 183480
   ccatgctacc ctgctgaaag atgagagtcc atgtggccat cctagaccag tcagtttcca 183540
   gacaacccat agctgaccac agaatatatg aataaacaca ggcaagacca taagaaccac 183600
   tcaactggac ccagcacaaa ttatcagccc acagtatttg gggccaaata aatggttgtc 183660
   attttaagtc actaaatgtt gggatggtgt gatatgcagc caaagctaac tgatcaacta 183720
   atctactcat aattcccagt aagttttaaa caaacaaata acagtgatat ttccaggatt 183780
   aaaatatggg ccagtatctg tttggggaaa ccctacttcg ctaaaagact tactgtgaaa 183840
   atactttatc tagttttcct agtcattacc ttcagaaagt agatgggtgt atttggatat 183900
   tagtgtcatt gatgtaaaat ataaaaaaat ctggagaatc tgggactatc tagacgatgt 183960
   ttacagatgg tggaccactt cattctcctc ttaaataagt attgcttcag ccacaggagc 184020
   tgcttttgtt tcttgaacaa ggcaaatcat tttctctctt gaggccttta tccttggttc 184080
   cttttgcctg gaatgctctg tttcaagttc gcaagacctg ccccttttta tccataaagg 184140
   ctgaactcaa agcattccca acccaatatc tgtatcacga gtaggcctgc agctgctctc 184200
   ctacaagtta cactttcata atgctgtcca gtagaaatat aatgtaaggt acatctttaa 184260
   ttttaaatac tccagtagtc atattttgaa acacaaaaga ggtaaaacta attttaatta 184320
   atctgttttg ttttatctaa aatgtttacc actttaatat ataaccaata taaaaatatt 184380
   agtaatacaa gcgatcaaaa aaataacctg tacagtatta ttcataccag ctttatgtgt 184440
   aatagccaaa aactagaaac aaccaaacta tccctcaatg agtgagtggt taaacaaagt 184500
   atggtacatg cataccatga aatactactc accaataaaa aagaatgaac tattgataca 184560
   tggaacaacg tggatggagt gctagggcct cgtgctgaga gggaaacagg accatcccag 184620
   atggtcatat aatctatgat ttcatttata taacagtctc aatattatat atttgtggag 184680
   atagaaaaca gattacaggt tgccagggct tagcaattgt gggggtagtg agggaacatg 184740
   taactttaaa agtatagcac atctttgtgg ggatagaaca gttctataca ctgattgtgc 184800
   tggtggttac aggaatcact acatgtgata aaatggcata gacatatata catacattgt 184860
   accaaagcca aagtcctggt cttgatatgg taccctaact atgtaagatg tacaaatggg 184920
   gggtacaatt caagtaaagt gtacatttat gtgtgtactt ttaaaaacat tattacaaat 184980
   tcacagaaag ttgtgaagat agtatggaga gatttgtgga ctcttcatct ctcttaacat 185040
   tttatttctt tcctttacaa taataataat agctaacaat tatggaatgc tcattccatg 185100
   ccaggcagta ttttatgtgc ttaacaagta ttaactcatt aaatgttcac aacatttgac 185160
   atagatatta attagctgca ctttattgaa aagctgaggc ataaagagat taagtaattt 185220
   gccccattaa gtagaagaac aaaggtttga acccagaaga ctgactctag acttcactgt 185280
   gcttggtttt tcttctccct tctactctat aaagtctcaa acaccactac cttgcctata 185340
   gtacttggta tacagaagta gttacttaat atttttgtag gagtgataaa aacatacaag 185400
   gaaaaaatca aagaggactg ggtcctctct cttccactct gcccctgaaa agctgtgtgt 185460
   ttatctgggt tccttagaag catttgggat gcattcctgt cacatgctgc tgatctcacc 185520
   ctgtgtgctg tctgcttgtt agaagccact ctgcttgaac catgaattgt ctctatcaag 185580
   ggaaatattt tgtctcagtg actattcatt caacaaatat tactgctcat ctactatgtg 185640
   ccaggcacag ataagatcaa aatgaaatta aggggcttac ggcttgctaa ggaagaccaa 185700
   ctttgatttc caacaatgtt gataggaaag gaaagtatct aggctttctg aaagccaact 185760
   tgctagcatt taaaaaagtt ttaaacatgc ttgctcttat gggatgaatt ctgtccacct 185820
   cccaagttca tatattgaaa tcctaacctc caatgcctcc aaatgtgact gaatttggga 185880
   ggtagggact ttaaagagat gattaaatta aaatgaggtc attagtttgg cccctgatct 185940
   aatatggccc ttgtccttat aagaaaagga aacttggaca cagtcatatg tagggggaag 186000
   aagtcatcta caagccaggg agagaagtct cggaagaaac caaccctccc aacaccttga 186060
   tcttggactt ccagcctcca gaagtgagaa taaaatttat cttgcttaag ccacccaatc 186120
   tgtgttactt tgttttggca gccctagcaa actaatacat atgccttccc tccattattt 186180
   ccacatctga caatctatgc cacagatgtg ctcgtattta tgcagaatga tagatacatc 186240
   tagcagagtt ctcacaggaa tgatgttttt aagagcccca gaacgtaaat gttaatcaat 186300
   aaaagaatca atagatgaat tatagtacat tccattttat aaaatgctat ggagacttta 186360
   gaagcaagtg atatgtgtgc tgatatggaa agatagccaa ggtttgcttt tttatttttt 186420
   tttaacataa caccccaaat aggctaaatg actcaaaaaa aaaagcaagt cccacacaat 186480
   gtacttagag tggtcttatg tttatgtata aaaaatcagg atgtgtgcag ggataacaca 186540
   aacaaataga aaatggtctg gatttgaaac tttgtgatag gaacaaagta gttccctttt 186600
   agcttttgtt ttgtatgctt ctttttgtta gaaccatcca caactgtgag ctgggataag 186660
   gggtttggat ttgtgtgtaa tgacaatgag aagtcataga aaaagtttag gagagagagc 186720
   ataatacaga catacatttt agaaacattg gtctgcctac agggtggtga actgcttgga 186780
   tgagtaagtc tagtggaagg gaaactgagc tagataactt tgtaatagcc caagagagaa 186840
   atgatggtgg cgtggacagg gtagacagaa tgtgttcatg catgtctgta atttgaaata 186900
   atttctgtag cccaatagac aaggaactgt gggaacaagt aagaaaaaaa gtattctggg 186960
   gtctaaagtg gcctccctaa ggaagttcca tttgatctga gaccagaaaa gagactttgg 187020
   gtgagggtca agatgagagg gtggagacag gcaggggagg gagaagttag aggggatgag 187080
   gggctttaag gtaatgggag tgactaagga tggtaaggct taatgaaagg gagaaggaat 187140
   ttatacagtt agagaggtac caaggggtgg tccccaggag ccttgtaagc tctgataagg 187200
   agtttggttt tgtttttaag gtcggtgaga agtcatagaa aaggtctaag agagaaagag 187260
   aatgatacag acttatattt ttcaaaagat ttgtctgcct acagggtgga aaactgcttg 187320
   ggaggggcaa gtttagttga agggaaactg agctagataa ttttatagta gcctgagaga 187380
   gaaatgatgc tgacatggag agagcagtga caatgggtgt ggtagacatc tccagcaaga 187440
   cgtcatcatc ctcatccctg aaatctgtaa ggggaattaa ggttgctaat aagataactt 187500
   taaaataggg agattatcca ggctggtcca atataataca agcatcctta aaagtagaag 187560
   agaaagacaa aataagagag tcagaggaaa atgggaaaga tgggccgggc acggtggctc 187620
   acgcctataa tcccagcact ttgggaggct gaggcaggca gatcacctga ggtcaggagt 187680
   ttgagaacag cctggccaac atggaaaacc ccatctctac taaaaatata aaaattagct 187740
   gggcatcgtg gtgcatgcct gtaatcccag ccactcaggt ggctgaggcc agagaatcgc 187800
   ttgaacccag gaggcagagg ttgcagtgag ccaagatcat gaagatcacc aagatcacac 187860
   tacagcactc cagcctgggg ggcagcacgt gaccctgtca aaaaaaaaaa aaagagaaga 187920
   aaagaaaaat aaaaagaaag tgggaaagat ggaataacag tcagagagag atgcagtatc 187980
   agccaaggaa tacaagtgac ttctagaagc tggaagagac aaggaaatgt attctgctct 188040
   agagactcca gaaagggaca tacccctgcc aatgccttga tttttaccag gtgagactca 188100
   tgttatactt ccaacctcca gaaccgtaag aaaatacatt gcattatcta agctgctaag 188160
   tttgtggaaa tttgttacag caactttagg aaactaatac agtgggaatt cagagagagg 188220
   tatagatgta gtagacatct ggggagcaga atggatagga attgatgatg attgaatgtg 188280
   caggatgatg cagaagagaa aggcaaagtg aactctcaga actggcttcc acaatttaat 188340
   gcaatgggat gctatctata taaggattga gatatgtagg aaagaaacag agcaagtagg 188400
   agatcatgag ttaagttttg gagatgctga gtgtcaaaaa tgagaataat ccaagaggag 188460
   ctttggaagc atagacacag agattaagag agaggtttgg gctctgtctc catatttggg 188520
   ctcattggca taaaggagaa gtaaacttat agtaaagaat gaaaccatgg tgaagtagtg 188580
   gcttttgatt gttatattta acacactttc catgcagttt ttctgagtac ttactacatt 188640
   gatgttactc taaaacccat gattttctct ttgctattat ctcaatatta tgttgtcatt 188700
   ctgccaatgt ggaaatgaat aaatttaaat atgttttcct taaagtgtgc cttttgtcaa 188760
   tactggatat tggatattgc agaaagttga gagggatgag ttgaagccag ggtgaggagt 188820
   tgaagcgagg agttagagca gaaaggtctg atgatgttga gggtctaaaa agtgtgcagt 188880
   tcagatggat gtatatgcta aatccaactg tgggcacaga tttcagaatc agggcagaat 188940
   cagaaagctt agataagaag aaaccagcag acaggtaaga tccaaacatc atgatcagag 189000
   ggaagggtct aagacattca cagaagtaga gaaggacaaa taagaaacaa agattcaggt 189060
   atgaagtcgg ggtttacatc ccagtggtag aaattagaag ccagaatcag atccacaaaa 189120
   gaagaaaagg ggttacagct gtttctacac ctgttgagaa ggcaagtgtg tttgaattat 189180
   gatccaggtc aaaaaaacag gcccaggtca caaagcaact ggataatgag catctgtgca 189240
   atagaaatgg ggaccaggta caaggataat cattgtattc acccaacagg ctttgataca 189300
   gtttctcctg gagtgggaag cacccagtgg aacatagtga ctgtccaatg ggtcctatta 189360
   ggagacactg tagtagatga atccaaggaa aggaaactat gtcatttata acaactttga 189420
   cacataatga ttttttttta gaaaacaact ttatttttag attatatgat gaacccatcc 189480
   caagattttt agattttcct atggatccca aatatgtaat ctatgtttca ttttccctaa 189540
   aatcagcata gctaaaagac tggagaatct tttgttcttg attctgaggc tataataaat 189600
   gtatatctga gcaatgacat ctcagaaagg ggatggtgtg gttggtgcca tcccctctgt 189660
   aatgtgattt ctgaaacagt cacctttctc ccgtaagtga aggaccagtc attaaaccta 189720
   aattatatga acacaggtag gtatttctaa agtacttaaa tttccattat tacatttacc 189780
   aagttctgaa aaatagatct gaggctaaga aaagaaaaat ctctcttcgt gagggagtca 189840
   ataagaaaag actcactctt aagaggtacg ttaaagtcac cattttaaaa ttctgtggtc 189900
   ttattctttc taccatgtct ctagctggct gaatgactgg aaaccttaat agttcagtaa 189960
   ttatattaac tcttcacttt attagaccaa cacagtttga catatttctt tcttaatccc 190020
   aaatgttata ttacaatttg agattttgag gggggatatg gacaaaagga ttttctaata 190080
   agtgacctcc tgctctgcct gccccacccc atcctgtgcg tgccccaccc cgtcttcagt 190140
   atggcttctg cctgccaact acttcctccc aggcaatcaa agcccctggg gcatatccat 190200
   gtcattatct tttattacta ttattattat gaaatataca caacaaaaaa tttaccattt 190260
   taaccaattt ttattgtact gttcagtagc attaaataca ttcacacttt cgtgtgacca 190320
   tcaccaccat ccatgtcatt aaccttaaaa tgttgccatg ggtgggacac aggactgagc 190380
   catgagtcat tgttttgaaa aggttgaaga atgctggaac cttctcaatt tgtaataggg 190440
   gtcaagtagc tgctcctcta cctctgcgtg tccacctgat gtgatgagag atggcacttc 190500
   agccactgat tggacctggc ttatagtaga gttcctactc cattccctaa aatcctaatt 190560
   tggattgcac ttgctaggag ccagacacag atctaagcca cttttcatgt cttagttcac 190620
   tgaatgttca caacaaccca ctgaggggga gaatattatt attcccattt atcataggat 190680
   actgaggcaa aaggaggcaa agccagttgc ccaagaatgc agaactgata aatgtagcag 190740
   gactcagatt cagatccagg aagtccgggt ccctgctttc aaccagaacc ctaaattaat 190800
   gagaaaatgg gctgcctgag gaggcaataa gattgtttga taaaattctt ttgccgttac 190860
   cctttagata attttgtaaa gaaagtaatt tcacagaatg cccatcttgc ttttttgaac 190920
   acctcaagct ttatgcccaa gagaaaaata aggcaacaaa aatattttta aaatagttat 190980
   aaaggaatac atgcaagaaa cgggggtctc catatggcta atgaagactt tggccccttt 191040
   taatgggtgg tggcttcttg tggatattcc ctagtcccta ggcattgctg gtgagtcctg 191100
   ggaagtgggg accataagcc ccacaagtat gctacatgcc agttagagtc ctggcgggaa 191160
   gcagaaggtg cactccagaa gggtttacct agcacatata agcaaggttc tgagaaccaa 191220
   taaaaaatgt aggaataccc acaaactaaa aactgcagga agatgtcaca tctagcccca 191280
   cagggactga aaactggagg gaaaactgga gggaaaaggg aatggcaaca gaagcttgta 191340
   aatattgggg aattttggca agaaaaccag agaaataagt ttagcaagaa aacaagggaa 191400
   ttttgcaaag aaaaccaggg taataaatac ccaacctctt tctccaccta caaattgcct 191460
   gtaattggtc aaatccccct agaaaccaaa gacaaggaag ttctagtgac acagtcatat 191520
   aaatcaactt aaaggcacag agaagagaag agactgaatc tgggtctggg gagagagttg 191580
   tagtgtatca attcagaaga accagcaaag tctcccttgc aagcagatat tatgagcagt 191640
   attaaccact cccttgtcta tcatggaagc aacattcaga tcatccagtc ccttgtaggg 191700
   ctttcttcta ataccttctc attgagttat ccaaacaatt acaggagcta tgcatatctg 191760
   gagttaactc catgtagaat ggaggactct ccatctgaga gaggctgcca gggctacaca 191820
   gcatgctgca ccatgggggc tggactagta ttcaagtcag cagagcatgg accagtttgt 191880
   actcaaaatg tggactgccg ggttttgaat tccatttcag tttcttccta cttgaatgaa 191940
   cttgggcaca ttttttttta actcctgtag gcctcagttt ccttatctgt gtagcagaaa 192000
   taataatagt ccttacaata tgaattactg tgaacagaat atgtgaattg gtacatatag 192060
   agcagtgtct gacacacagt tcatcctaga tgaatattat ttaagtagca tcttctggtc 192120
   cctaatgagg tgctgcgcca tcaccccaac cagcatgact cgaatagtac caggtgctgc 192180
   tgcatttgcc tgttatttct tctttgactc cctactccct atgcctgttc gtagagacct 192240
   gaattgggag atgcctgcat gttggagctt ttagactagg catatgctta aggcagggca 192300
   acccactaag ttagggacaa agctgggctg tccctgagaa caggttgagg gctgagataa 192360
   ccatgaagat gtcagagcag aaatggggta ttagagtctg gggatctgga tttccttcag 192420
   tctcttctat gacttctaag acttatattt catagaagaa catagggtga ctgaaaccat 192480
   ccatcaaagc agtgtttgct gtgagttttg cagtaaactt tcagaaacaa ggattctggg 192540
   atctgtttac tatccttctc taggaactaa actaatatta gcataataaa gaccttgaaa 192600
   tgtcccacaa agggacctta tgtaattcat tttttcccaa acttatttag ctaaggagcc 192660
   aacttggtta atgctgaatc aaactgccag gtgaatgttc acatactgaa acaaataata 192720
   aagtgtttaa taatgaaaca aagaaatgaa gaaatatttc aaattaattc ttgaattgcc 192780
   tacttctaaa cctatgtaac tttctgtact gaaacatacc tacacagctg tcatgaatta 192840
   ggtctgctgg acactgagga gtggaaggag gtatgctgtg cccttggttt acatgggtgc 192900
   tgagcagtag ggatcttttc ttctgtagga gaggctgctc agcttcacct gctcagtgac 192960
   acagtttgta atttctcaga gagggccttt atggagagac atgacaggac ccaagaaacc 193020
   tcaccatcca caccaactgg gttccctaag aaaggtagga tggtgtaact ggcagagaag 193080
   gtaaatacag ggcagccata cattgctctc acaagtgatt aaaagcttgt gttttgagat 193140
   tctaaatctc cagttttaca agtttctaac ctggactctg agcatctgtt tcctcttcta 193200
   tgaaagggcg atgataataa cacctcctct gcagaaaaga gttagtctag taggcttcag 193260
   gatgcatatc ctagaaaaaa agcctatttg caaggttggc ccttggctgg catctgggaa 193320
   cttggatttt agcaagttcc tcaccattcc ctgataaaga atggcttgct gtgcctaaac 193380
   tgtttatgca aaaaaaaaaa aagaggttta tgttcaaccc ctgttttcct cctggaattt 193440
   aaatatggac taagcaaggg gtgcctatgt gaccagtaca aaataaaatc ctcaggccct 193500
   tcatctctga tgggcttccc cagtagatgg ctttttacat gtgttgttac aatcagtcct 193560
   gggggaatta accacaaccc gtgtgagttc actaggagaa gacctctgaa agcctgcacc 193620
   tgtattctct ggacttggcc ccatgcacct tttcccactg ctaattttgt tttgtatcct 193680
   ctcatggtaa taaatcacag ccacgagtat gactgtatgc agagtcctat gagttctcct 193740
   catgaatcat caagcctggg ggtggtcttt gggacccctg acagagggct accatgaatg 193800
   tgggatgaga cgcattgaac ttttagtgcc ctagtaggta gagactgagc atgagcacat 193860
   cagagagaag aaatattctg ccaagaagat cttcacttcc ctactaggcg tccatctgtc 193920
   ccatggtgct aaaaggcact aggaccctgt ctctgtcttt cagctgagtg tgggcctcct 193980
   ccttgctaac agagtagtag ccactgctga tagaatgtca caataaatct tggagatttt 194040
   gtctctgcac ctgtgaactt cccaggaata ttgcttcagg atttagagct gttttgtgtt 194100
   ttccttttaa gttgttcctc tgtaactctt taactcaaac ttactggaga ggtagaactg 194160
   catgtttgac cattaaaatt atagttgtgc tgtatcacac aatgcagtgt aggtagatta 194220
   attcagctgt ggatttcctc tccgcaactc tcttatttaa aaacagaaac agtattagtt 194280
   cattgatatc ttcttttcaa tgctctcatt gagtggcata gcaaccactg ggaaattcac 194340
   aatgagagag ggaataataa gacatttcta ttctgcctct aaaaatattt ggcctagcat 194400
   tttcagtgct tgttcaggta tgaatacagg ccaaataact cttctcaaat gatgtttctg 194460
   aaataatttc catcttgcat ttttaaaaaa tggtcatagc taaaatttct tgaactgtca 194520
   agcggactca gccaacaaat ggccaaatta tattttcaaa tgtattttat gctcactaaa 194580
   taatttagag agatggtgta tagagctaat tattggtgta atttcctgcc actgaaaaca 194640
   ttccaggcct tcaaccagat agtaatatca caatcaaaac tcctttgagt tagcactcaa 194700
   ttgtgtaaca gctaatatag ttacccagat tggatgttgt tacttgaaac tttgaagcag 194760
   ctaaaataga aacctaaaaa tggcaagcat ctgaagatgc aaggtagagg atgataatca 194820
   gggtttttgt tatgttagct ctttctgtgt agcatggttt aaagcaaaaa aacatttatt 194880
   tgtgtcctga ttctgtaagt gataatttta agttcagttc aactgtgtag ttctgctggt 194940
   tttgactagg ctcactcatg tggatagctg ctggtcagct gggcagctct gcttctcagg 195000
   gtcgactagt tgctggctgg ggagacaggt aactgggata cacagcccct catcttccag 195060
   caggataggc tggctttgtt ttcatgctga ctgctgtttc taaggacagc aagagggcag 195120
   acaagccaca gtgtgcaggc acagtgcgta ttggtgtcat attttctctt gtctcactgg 195180
   ccatagcaaa ttatatatcc aagtccagag tcagtgtggg agagtcctcc ttgaaggcat 195240
   ggataccagg aagaatgaaa cattgtgggc catctctgca gcagtctatg acagcagggt 195300
   tcctttactt cttgacactt tggtttgggt gtgcccaagc gtttaaggtc agctgacttg 195360
   tatttattga cctacttgta gtaaagtact tacagaattg ttttaagtta taactatatt 195420
   ttccaggaaa ggtaatatat caaggtagtt gaagtcctta tactctttca tccgttttca 195480
   ttacagccct tccaaattag tctgaagtaa caaccagaat gaatgcgatg ggttttgacg 195540
   gagtccaggg agtgaaggct gtgaatggtc tgagcattgc tgagagaata tttatgtagg 195600
   tagaggatgg aaaaggaact tgagaaaact ggaaggcatg ggaagaccct gcttaccaca 195660
   cagggacaag tgtttaaaag aagtaacagt tttaacagag gatgaaggga agtaaagaag 195720
   ggagagtgag agaacatgga gctccaaccg cacatgctat ttactgaata aatgtgctgt 195780
   tcagggtgat tttagtccat caacaaatat tttgtgagcc ctactatagg ccaggcaaac 195840
   agtaatgaaa aagcaggaag catcctttcc ctcatggatc ttacattcta gtagttaaaa 195900
   cagacactga gcacaccatt atatgtaatt aattaatcgc aagtggataa atgctgtgaa 195960
   gaatatatac atagaagtgt aggaagatag gctgggcgca gtggctcatg cctgtaatcc 196020
   cagcactttg ggaggccgag gcgggcggat cacgaggtca ggagatggag accatcctgg 196080
   ctaacacggt gaaaccccat ctctactaaa aatacagaaa aattagcttg ggcatggtgg 196140
   agggcacctg tagtcccagc tactcgggag gctgaggcag gagaatggtg tgaacccggg 196200
   aggcagagct tgcagtgagc tgagattgca ccactgcgct ccagcctggg caacagagcg 196260
   atactctgtc tcaaaaaaaa aaaaaagaag tgtaggaata tatgacctac ctattccagt 196320
   ccaagggctg agataagtct tcctcaacaa atcaacatgt aggccaaaac atcatctttc 196380
   acaatgccca tgcagtgttg taaggattcc caaaatgatg attgttctat tcacagatga 196440
   gtagatgcag agaggaaaaa agcaattatc tgggatcaca gtgaataggt gacctagctg 196500
   ggatgcaaac ccaggttacc ttatgtgttt tcaacatcat aaagcatact ttgcctattt 196560
   tacatatttt ctagaggtat tctggtcact ggctatgttt cacctaggta aactatgttg 196620
   tttcaaacag gtaaagtaca ctgtaaaaaa ttgtaaatat aatatacagt gaaaagtgag 196680
   tcttcctccc tgccttcttc ccctggtacc cttctctgca ggtaatatca gtcaactttc 196740
   ttgtatattt gtccagaggt gtataattaa tacttgaagt aattactttg tgacagatgt 196800
   ttttccaagt gctttgtaaa tatttacttg tttgatcctt tatgtggtga ctatcattat 196860
   caacccattt ttcatgtatg catatatttt attccccaca aatgttttgg attttaacta 196920
   cataccaaac tttgaaatgt ttctccctgt gtactggtca gctttaataa tgctatataa 196980
   caaacaaggc caaaatctca gtggcttcat tacatacatt tatttctcat ttgcgagtca 197040
   gccgtggctc aataatatct cagttgagct tgcctggtct tggcttcaga ctggaggttg 197100
   ggcttgtgtc tgccctcatg catctctcct tccaagacct aggctgagga agtgtcatct 197160
   ccctgagaga gagatctttg tgacaagtga caggagcaca aggaaaaagt ggaaacatac 197220
   aatgccttca aaaacttctg ccctgaactt gccaactgtc tcttccatcc atagtcaaag 197280
   caggtcacat ggccaaatca aagtcaatgg ggcagggaac tatcatccac atggcaagtt 197340
   taaggaggga acaaaaaaaa attgaataaa attatctaat ttacacaccc tgaaaactca 197400
   acttttatct tttgcagctg ggatctagag gaataactga aaagatgttg ccctgagtga 197460
   attaacaata gctttctcct ggtcttctgc tggcaagata taaatggctg cctcttttcc 197520
   atttatattt cccaggtttt ggcatcatgt ttgggaagaa aaagaaaaag attgaaatat 197580
   ctggcccgtc caactttgaa cacagggttc atactgggtt tgatccacaa gagcagaagt 197640
   ttaccggcct tccccagcag tggcacagcc tgttagcaga tacggccaac aggccaaagc 197700
   ctatggtgga cccttcatgc atcacaccca tccagctggc tcctatgaag gtatggtgag 197760
   ggctccatct ttggctgggc ttaagaatca tgctcttatt taatgcactg catcagcttt 197820
   ataagcaagc tataaaatgc ctaaaccaca tattttttaa cattcacaga ttattcacaa 197880
   ttattctttc atgattataa ttttcaccat ttcttataaa ttgcagccca tgaaagggaa 197940
   ctttggcttt agatgtgatt tttgaataat cagacacata aaacacgatc acaaaatagg 198000
   acatttttat ttcgacttta aatggtgttt ttgttagact ttatactgaa gcatattatc 198060
   catccactct ggttaatatt agcaatagcc tctgaccaac attagaccac cttttgttgt 198120
   tttccatttg ctctcaaaat atttaaactt ttctttcata taaaatttac taccaataga 198180
   ttttaataat ggtattgata aatatatata catatgcata tttaggcata tacatgtata 198240
   tgtttatata atcaaataaa tatgtgtgta tatatacaca caaacatata cgcacatatt 198300
   tatttgtctt tatagctttc aaatttgtct ttatagccta aaagagagct atttcaaata 198360
   aaagcaagta atttgagtcc tagaatcaga tataatttat cttaaatatg taactaggaa 198420
   atgtttggca ataaatgctc ataaaagtaa ttacatgtaa gcagaaagca gacccaaaaa 198480
   gatttcagtt atgccaagtt actacaaaga aaatccaggg attgcagttt tcttctgctc 198540
   ttcagaatga taagaattct atagactagc aggcaattgc ccaaaagttc tctatgggag 198600
   ttaaacatca tggaaactta taatcatatt atccagtgga tcattccaga gtttcataat 198660
   ctacacaaag cttgaagttc agattctttc cttgttcatt atattagtag acatctgtgc 198720
   tatttgaata tgttattctg tgttaacatg aatactgcat aaactgtatt taaataatat 198780
   gcagaacaaa actgaataca tttttaaagg gatgcaaaga aagagaacag ctgatacatt 198840
   atgggaaatg cttatgaaat atcacctgac ctgactaaat tagaactgtg attccattgt 198900
   tcatgcacag tgctggtcat tctattctgg tattgaacat atgagtctgt tctgcccaaa 198960
   gatcaataca gagtcttata attctgcatt ttgtgagttt gattttctgg tagaatttca 199020
   aaacttttca aaccaactaa aatgttaagt acatacacac acaaaaaaat ggatatttga 199080
   gaagataaag ctttgggaac aaaaggaggc tgtcatttta aacagatatc aattgatatt 199140
   tattagaaac tgactaacca actctactag tttgcatgtg gaaatatcct actattgaac 199200
   ataattttct ccctaagaga aggctagcac ttcactaatg atcatataat aaggtggacc 199260
   atatctttaa aaagttggga ttagaaaaaa atctgcttgt ttagtttcat tatttttctt 199320
   atatcccaag tttttacaaa acccagaaaa tgcaatgagc agtgtgccct gaaaagtctt 199380
   ctactattta tcaccatggg gaaagaaaaa ttctcacatg aaactaagac ttattttctt 199440
   accctggagt atcattatca ttatcattgg ataagaaaca ttctagatgg aagcagagtc 199500
   ttaaagaaac attctcacac cagttagaat ggtgatcatt aaaaagtcag gaaacaacag 199560
   gtgctggaga ggatgtggag aaataggaac actttcacat tgttggtggg actgtaaacg 199620
   agttcaacca ttgtggaagt cagtgtggcg attcctcagg gatctagaac tagaaatacc 199680
   atttgaccca gccatcccat tactgggtat atacccaaag tgagaggtga cagcatgctg 199740
   gcagtcctca gagccctcgc ttgctctcgg cacctcccct gcctgggctc ccactttggt 199800
   ggcatttgag gagcccttca gcccccccgc tgcactgtgg gagccccttt ctgggctggc 199860
   caaggctgga gcccactccc tcagcttgca gggaggtgtg gagggagagg cacaagtggg 199920
   aaccggggct gcatgcggtg cttgcgggcc agctggagtt ccgggtgggc gtgggcttgg 199980
   tgggccccgc actcggagca cccagccagc cctgctggcc ccgggcaatg gggaacttag 200040
   cacctgggcc agtggctgcg gagggtgtac tgagtccccc agcagtgccg gcccaccggc 200100
   gctgtgctcg atttctcgcc gggccttagc tgccttccca cggggcaggg cttgggacct 200160
   gcagcccgcc atgcctgagc ctcccaccca ctccatgggc tcctgtgcgg ccgagcctcc 200220
   ctgacgagca ccaccccctg ctccacagcg cccagtccca tcgaccaccc aagggctgag 200280
   gaatgtgagc gcaccgcaca ggactggcag gcagctccac ctgcagcccc ggtgcgggat 200340
   ccactaggtg aagccagctg ggctcctgag tctggtgggg acgtggagag tctttctgtc 200400
   tagctcaggg attgtaaata caccaatcgg cactctgtat ctagctcaag gtttgtaaac 200460
   acaccaatca gcaccctgtg tttagctcaa ggtttgtgag tgcaccagtc gacactctgt 200520
   atctagctgc tctggtgggg ccttggagaa cctttatgtc tagctcaggg attgtaaata 200580
   caccaatcgg cactctgtat ctagctcaag gtttgtaaac acaccaatca gcaccctgtg 200640
   tttagctcaa ggtttgtggg tgcaccaatc gacactctgt atctagctgc tctggtgggg 200700
   ccttggagaa cctgtgtgtc gaaactctgt atctaactaa tctgatgggg aggtggagaa 200760
   cctttgtatc tagctcaggg attgtaaacg caccaatcag caccctgaca aaacaggcca 200820
   ctgggctcta ccaatcagca ggatgtgggt ggggccagat aaaagaataa aagcaggctg 200880
   cccgagccag cattggcagc ccactggggt ccacttccac actgtggaag ctttgttctt 200940
   tcgctctttg caataaatct tgctactgct cactcttcgg ggccacactg cttatatgag 201000
   ctgtaacact caccgtgaag atctgcagct tcactcctga gcccagcgag accacgagcc 201060
   caccaggagg aacgaacaac tccagacgcg ctgtcttaag agctgtaaca ctcaccgtga 201120
   aggtctgcag cttcactcct gagccagcga gaccacgaac acaccagaag gaagaaattc 201180
   tgaacacatc tgaacatcag aagagacaga ctccagacgt gccactttaa gagctgtaac 201240
   actcaccgcg agggtccgcg gcttcattct tgaagtcagt gagaccaaga acccaccaat 201300
   tctggacaca aaaggattat aaaacatgct gctataaaca cacaagcaca cgtatgttta 201360
   ttgcggcact attcacaata gcaaagactt gaaaccaacc caaatgtcca acaatgatag 201420
   actggatcaa gaaaatgttg cacatatata ccatggaata ctatgcagcc ataaaaaatg 201480
   atgagttcat gtcctttgta gggacatgga tgaagctgga aaccatcatt ctcagcaaac 201540
   tatcacaagg acaaaaaacc aaacaccgaa tgttctcact cataggtggg aagtgaacaa 201600
   taagaacaca tggacacagg aaggggaaca tcacacacca gggactgttg tggggtgggg 201660
   tggggggagg ggggagggat agcattagga gatacaccta atgctaaatg atgagttaat 201720
   gggtgcagca caccaacatg gcacatgtat atatatgtaa caaacctgca tgttgtgcac 201780
   atgtacccta aaacttaaag tataataaaa aaaaataata aacattctag ctgaagaccc 201840
   aggacatgtg ccgtcttgtt agagtgtatg cattatttgg ttaggacaaa gtatcattta 201900
   cttcaccacg gtttttcact tgtccacaac ctagcaggtc ctgtttgaga gcaaagaaag 201960
   aggctttcat cttagtggta acacatcatg ttctgtggat gtgggactta gcagaacctt 202020
   ctacctaatt tcttccattt cctaggtggg ctggcttgtt atcctttcct ctctgacaat 202080
   cagtcccact gagagttgga gcaagatttc ctcacttcta agctgctaaa ccttgtgctt 202140
   ctcttactca ctagtatggg ctttccagcc tttgtgccaa atgcatgagc attcagtcca 202200
   tatgaaatga aaagactaaa atattagtta tagacactta aatgttaact gggtgatgac 202260
   tggaagatac atctcaagta tagagcaggt tctggatttc tgcaaatgag acttgaggca 202320
   caattgtgcc gctgagggaa aaaaataaaa taaaaacaga caaggagtga caaaaacaag 202380
   atccagctag gttggatctc acccataaag agtaaagttg atcaaatcca tttaactgaa 202440
   ggacacccaa aaaatctagt acctcatact aacactaaac tcccataaag caaatcctat 202500
   ctttctccaa atatgagtgt aaatatgtct ccacatatga gttcagttaa catcactggc 202560
   agacagtaga caatattggt ggtcaaacag aatcctgtga ctaccagact cttacaataa 202620
   gtaaaaatta aaaggtttaa ggggacaaac tatgttatat agtttgtatg atcccatttt 202680
   ttgaaaatgg cttctctgga aaaatcttac aagtcaccta agaatgacat atgataaagt 202740
   atagagaata tatctcagaa gtcaaaaaag tccttgaaat aagaagtaat gaataaatgg 202800
   gcagaaaaga ctggatatag tcatgcatgt ttaaagccat tttggcaaaa ttatttaagt 202860
   taaatagaac caaccattct agttggtttg gtcaaagtgt cctttaaatt gagaaaggaa 202920
   aatgagaaaa gagatcatga cataaaaaca gtcaacggag gaagtgtgtc gcccctgatg 202980
   ttgtggaaaa tgccagttct tcagtacttg tcagcaagct ttgccaagct agttccgagt 203040
   ggaaatctgc cgttgccacc aacactcttc cttgattagt taggatttca gctgttcctg 203100
   tttagaacct caggcttacc cattgtcacg cttaccagga cacacctctc ctctgtgtag 203160
   acttatttag gccagggctt ctccagccat acagcagatc agaatcacct gaagagtttg 203220
   ataaaacaca gattgctggg ctccattctc agaatttctg attcagtaga actgggatgg 203280
   ggcccaagaa tttgcatttc taataagttt ccaggtggta ctgatgctgc tggtctgagg 203340
   accacacttt gactttgatt taagtagtat aaaagaatgt atccacataa ggtataatca 203400
   atgtgagttt acaccagccg ggatttccaa actgtcagaa atttaaccag aagccacaag 203460
   ttagcttgta cttgtgtcaa cagtttgtgt gcccaaactt ttgggctagc catgtaggcc 203520
   atgtagggct agccaagata acctgaatac cccaaggggg aggttccctt taaaacattt 203580
   ggcaaatatc attgagaaac tccttttata tctaatgtca tttcgtccat gagaacatca 203640
   taagcaatat taccaaaatc tgtgctgaat tagatgcatt atgtaaaggg caggttgaat 203700
   ggtcgtattt gtttgtgatt ggccagtaat tctcaaactt agactttcat aagaatttcc 203760
   ctgggggctt gttatagctc agattcccaa gccccaccct aggtattctg attcagtagg 203820
   tctggagtga gcctgataac ttgcatttct aacaagctct cagatgctgc tgatggatag 203880
   atgtacttta agaactgctg ttctaggtgg actaaagcca caacataatg atcacccttt 203940
   ccctttgtta cagcctagaa atcatttgaa aatgctaaaa aacaaaatac agaggataat 204000
   atttaaaagc ttttgtagct tctacctaaa atgaaaaaaa gcatatctta atatttgctt 204060
   catatttctt tttaattttc aggtagaatt atacaaataa tattagatag ttttattcgg 204120
   catatgtgag ttttatgaaa tgatgtcata atgtatataa tctctaactt gcttttttac 204180
   ttaatagtgt tttgagatta attcacactg atgtgtagaa aatgaattta tttaaaaatt 204240
   actctagctt ttttttcaat ttatccattt ctctactaag ggatattgtc aagtatttgc 204300
   tattataaat attaataata ttgtcatgga catctgtgtt catgtcttct tgggcctatg 204360
   gtgaaaagtt ttcttgcagt ggttctcaaa agtgtggtct aaacctatgg ggggttcctc 204420
   agactctttt agggagtcta caaaattaaa gtttgctttt tttaactctc gtttcctgtg 204480
   tatacaatgg agaagctaca ttacatgtga taatgtcatt cattggtgga tgcttaatgg 204540
   atcatgtgct ttatagtctt atgttttaaa ttttcttggg tgggcacatg cctgtaatcc 204600
   agcactctgg gaggccaaag caagagtgct tgagttcagg agtttgagac caacctgggc 204660
   aacctagcaa aacctcgtct ccactaaaag taataataaa aaaaatatta gccaggcatg 204720
   ctggtgtaca cctgtcatcc cggctactct agaggctgag gtgggaggat cgtttgagcc 204780
   taggtggtca aagctacagt gagccacgat tgaaccactg cactccagcc tgggtaacag 204840
   agcaagaacc tgtctcaagt aaattaataa gtttatcagt tttaatatct aatgcagtga 204900
   atattaatag atataatata aactaaggtc tcttttaggt cctcaataat tttaagaggt 204960
   tcctgagaca aaaagtttga gaaccactat cttggaactt atattaaatg tgattatctg 205020
   tggcttctgc aacttgcctt tcacacacta ctgacttttt tgttgtttta aatgaaaata 205080
   gagacttttg tcttttctag ttggccagca atcttctaat ctctacaacc ttttcaaaga 205140
   ttattgaata caatattcca tcccttttcg tgtatactat ataatttctc taaaccagct 205200
   gttttgaagt tttttaaatt gttagatcct tatttaccat cccttttcta atttagcctt 205260
   caaagtcttc ctaacaatct tctttttatc ttttaaagat tgaagagtca cagagaagac 205320
   caatacaaag ctggctctga cttagttttg tttgtctttg tagtcttcgg tattttataa 205380
   tctttcaaag ctgaggttct gtctcttcct tttttatcga ctgctgctga gtactgcttt 205440
   aaaaacttct ctattcacaa aaacaggaaa aatcaagaga ggtaaatctt taggtctggc 205500
   catcccattc ttgaggacca ctgttttatt ttctctccca tcgtagttta tgttgaaagc 205560
   tcatccaaaa ttgaatctaa tttaattact ctgaaattct ctacctttct ctatttttgt 205620
   gatgctcaat tttgcattgt atttagagct atatcatttt tcttagtttt atttccatgg 205680
   gattctctct ttcctttaat atttttaaaa atctgctttc ccaaaataca gggcaactat 205740
   ctgacatttt ctagcattat ttccctttga cctaaggtaa cagttttccc caaggttcct 205800
   tctcaattac gcataactga tgtggtcttc cttgttggtc agaattaagc cagaaaggag 205860
   ttatttcttt tagtgcctcc tttactttct gagagttgtt gccaagcagt ttaaactgtt 205920
   tccagatgtt tgccatgctc ctagctgagt gaagctccca gcaattctga gagctggggc 205980
   tgccccctca tgacttttct ggctccctta acaggtctct tcttaaagct cacagattag 206040
   gttccccatt actttcctat cctcttaaat cagtgttttt tgtttgtttg tttttgcctc 206100
   tcccttcttt tagacctgct tctgctaaaa tatagtgttg agctagaagc atcttttttg 206160
   caacatgaag tccagtgcct tcatgttcac tgagctgggt cacccctttt catttctaag 206220
   ttctcatctg gatgaacatc agggcacttc taagaggtaa caatggagcc aggagaagac 206280
   cttggaaagg gatatattca aaggaaggag gaagaactag tcccaagacc ctgtggcagg 206340
   aacaagcttg aggagtttaa aggaaaaaca gatcatctgt tgggtagagc agagcatgat 206400
   ggggagaggg gtgggggata tggccagatg gacaagtaga gattggatgg cacggggcac 206460
   ttttgaaagc cacagtcagg agtttgctat tttgtgaagc ctctgtgcat gaaggacaca 206520
   ggtctcagat caagaaattg tcctaataca aaatgaattg atttatttaa gggtcagacc 206580
   caatgtatag tgtaccaaat atattgatct aatatgtaat cctgcaggtg ttagaggtgt 206640
   gttagtcaaa tggttgtgaa cagttgggat gcaggaagtt cttccaactt aatgattatc 206700
   agaagacatt taataaaaat tcaaaccttc ttaaagaaga aagagtgtat atgttataaa 206760
   gacagttaat ttcattgcaa agtaaaagca attataaaat gaaacccatt agacagatta 206820
   cctatgtttt aaaacctctt gtcaaaaatg ccacttcaga agaactgcat tgagttcttc 206880
   agaaccaagg ccctcagcgg gaggagagat tgctgatcct ttgttaaagt tgtttatatt 206940
   atttttatac ttgcaaaacc agtgaggaca atggacttgg acactcacat ttctcttggt 207000
   gcctgttact tcctggaatc cctccttcca gtgtcagtat gccttttcag ccctcccacc 207060
   accacaaaga ctgtggaata gaaacatcac actggccccc attttctcat ttgcctaaat 207120
   tcaggagatg tgggcaaggt aatcttgttt ccaaataaaa gaagaaatcc aaaaccaaag 207180
   ctagatttat tatatttctt catttttatg atcccatcca atctggaaac ttcagccaag 207240
   cagtttttga ttatattaat gtggttattt tttgatgtgc accatgtcaa cacaccatct 207300
   ttccccataa atgtctcttt ctcacaccct ttaaaagtaa caaactacat caaccactaa 207360
   taagaaccaa ctttgtttac attagtgttt tgcagggttt tacataagcc actttaagat 207420
   tttcttaaac tttgaacaaa cttaagctca catggcctag ttattactct aaaaacaaaa 207480
   gctgatgttt tcagagaaga cagtatcacc ccagggaatt tatccctgtg ggtacccacc 207540
   acttcttcta tttggaggag ggccaatgtt aaccacacac ctccaggcct cttggcaggg 207600
   ccatcctggt acccagagtg acagctctgt aatcacaaat gggtgatgtc acatctatca 207660
   cacctgcagc tccctggaac cagcagagaa taactggaga ttcatggtcc atgatcctgg 207720
   catttggaag actgctccta gggtcccaac taaaattggg tcactgagac ctcatgaatg 207780
   aatgctctca gttactcccc atcaaggagt aactgtaatc tcctgccaag gattattaga 207840
   aaataatcct tctaaatcaa aagaaaaaag gcatgtggcc ctctgtgact cccactcatg 207900
   cttggaactt gaaagattta atctcctgcc acctcaagac caaggtaaac acaaattgaa 207960
   tgtaccattt taaagcaatt gtcagttata ggttatattg aaaagttatt taccaccaat 208020
   ggttagatct atccctgaag gcctcttctc atggttcaaa atgctgttcc cagcattcct 208080
   gcagtggctg tgttccccta ctgtaactcc tgggaggaag tgatagagaa gaacccacac 208140
   agggaccgca tatactgagg gttcctgtga gtacctccac ttgagtgttt taccccaact 208200
   ctactcccag agcagaagct gatgtgaagg caatgcaggt gtcaaatctg tagtaatctc 208260
   tagtttttgt tgaaatctct taggcaacag gcctgaccaa aatcaagagt atcattacca 208320
   aatatcggat ggtgttttac agagtaaaaa atgtttaaga tttattcttt tgtggcactt 208380
   ataataacaa atagttttaa cccagttgta cagataagtc ttgactaagt atgattgatt 208440
   agtgcaaatt cacccaattt ctaagttgaa cccagttatt tatttattta tttagagaca 208500
   gggtaatgga gtgacaccct gtctcaaaaa ctaaataaat aaactcaatt tttcattttt 208560
   ctcaccacag tctcatacag gactgagtac ttccccatcc cttgaatttg catgggactc 208620
   acatggccag tggggtgatg tgagcagact tctaatggac agctaagatt tccctctgtg 208680
   cttatgccaa catcataaga atatgcctta gatgacagac tgcctgcatc tctgaaactg 208740
   gaagcagagc cacccagccc caacctcatt gtatgaacct gagtgtgggt ggtttgttgt 208800
   gcaaccttat aggacaactg actgatacta tcttacttag ccccatacca gccttttcaa 208860
   ttagagagta gtaactccgt cttttgggta aggaaagcaa gtcttagaaa gtttaaagga 208920
   tttgtttaag atcccacttc tgtaactgga aaggcccagg actcaaacct tatacctatc 208980
   tgactccaag acctgggctt gtaatcacca catcactgtg cctcctggta ataagtatgt 209040
   ttgggcatga aaaactgtta tgtgcataaa cgatgctaag tcagaaaaca aaattgcttc 209100
   cttcttacct tttcaccttg tagattgtgt aactaagact caggtaggca cagtgttttt 209160
   tcctaagacc acagagcaca ttaatagcag atattatttc tgtgggtaag gaatgctttt 209220
   gattcgatca tgtagaaatc ccagatttag cccatgagct ctcccttgag aatctgacaa 209280
   ggacatcttt ataaatacct acctgactat gattccctat cattactgcc atatgacttt 209340
   attttttagt tgtagagtac tcagagccct ttgcagagtc tttcattatg catgtaatcc 209400
   acgcagtctt ctcaagtgag ttaggcagaa tatttaaata ggaaatgtca atgaaatcat 209460
   gcaacgagaa gcaaatgagt gagctttcaa acgtgtgcca ctcattcctt ctccactatg 209520
   gcctggatgc tccctgagcc catcatattt taggtcaaac agcatacccc ccagatcaca 209580
   agcctgtctc cagaactgtg ttggtttccc atagagtggc cctctctgaa ctcctgaaag 209640
   ctgaactcat caaataagga cagctgtgct ttgatagctg ggaaattcta tataatgtga 209700
   aatgatattc taggggacat cttcttttcc ccagctcttc tctcccagca gtggtgacct 209760
   aactgaaaat aatccttcta aatcaaaaga agaaaggcat gtggctctct gtgactccca 209820
   ctcatgcttg gaacttgaaa gatttaattt tatctctcat ttagcatctt atagaaattg 209880
   ggacactaag atggcagcta gaggctgctt tagtggaact atcaaatcct atccttttta 209940
   gtgttttatt gtgtaatttt aagtggaagc attatgctgt caactttata ctgatgataa 210000
   atctatcact ttaaaatcat ttccaagcag cacatcacta tctgattgga tgctactact 210060
   gttacctgta aggttaatgg ctgcctaaga tggtacctgt ctaagtgttt gctagaatca 210120
   ctgaatccat cacctgttga gaaccttcat atcagctttc tattactgca tgacaattac 210180
   cactaagtta atggcttcaa agaacacata tttattatca cagtttctct gagttcagag 210240
   tctaggcatg gcttaacagg atcttcttgc tcaaggtttc acaaggctgc aattaggggg 210300
   ccagctgggc ttcattccca tctggaggct caaatgggga agaatccact tccaagctta 210360
   tttagtcaga attctgtggg cagaattctt ttctttgcag ctgcagaatg cgtgacagct 210420
   tgcttcttca aggctagcag gaaagcaaga gctagtctgc ttggactata tatataataa 210480
   tgtactcaca gaagtgatat cccatcagtg ttgccatata atgtaagtta accatgagag 210540
   tgacattcca tcacctttga catgtaacga cacagaaggg acagcctctt acctttacca 210600
   tattctattg gttagaagca agccacagat cctgcctaca ctcatggaga ggggataatc 210660
   aaaggcatga tcaccaggag gtgggaatca ttgaggggtc accctagggt ctgtctacct 210720
   cgttcccaat gtcactccca catgcaaaat acattcatcc cagcccaaga tcccaaagtc 210780
   tcatctcatt gcagcatcat ctcaaagtcc aaactctcat catctaaatt gtgattaggt 210840
   attgatggag tgcttgggag taatctatta aatacagctg ttggggcaca attcctttcc 210900
   atccatggac ctgtgaagcc aaagagacaa attatatgcc cccagcctgt ccaacatgta 210960
   atggtgggac agcacaggat aaccggtgta aaaattctga ttcaaaatgg gggcagggtg 211020
   agggcaagtg agtggaagga aaaagggaat tactggtccg tgactcttct gaaatccagc 211080
   caggcaaatg ttaggagttc cctgattaca tttcaaggca tgggaatggc cctctgtggc 211140
   ttaggctcct ccctcagggt tcttggttct gcccttcctt ttcatgaaag gcagcacatg 211200
   tttgcatcct gagtagtttt atcagcatgt tttccactgc tagtagaatt ggaagggtct 211260
   gaaagcctcc tttatttcat actctctttg tccctttcag gccaagtggc cagtattcct 211320
   gatgaaacaa ttccttaaga actttgtaga gtttgtgtgg attcactgca gttcactcca 211380
   ttaggtgatg ccacatccac agatatttta aagataagcc ttcctgttcc ttggcctcct 211440
   tctgagatga ctgagggacg atgcccatat gcttccttga ggttttctgg cttgattgag 211500
   aagatctgta gggcagacct ttaatttctt aaaagacccc ttcgtgtaac tgcatagttt 211560
   tgtcttttga tctttctaag tttctaacaa agagctctac agtctcactc agacttttcc 211620
   ctagtgcatg ctttcctgag agtgaacctc ttcattttag catctgtgcc atccagagat 211680
   catcggaatt ttttacatca tcagattctg attctgtttt ttgagaaaaa ttgtcctccc 211740
   aatcttcctc tttcctcaca cattttacta caagcagaaa gaagaaacca gagggttcct 211800
   tcaacacttt tcttggaaat ctttatacag ccaagtatca ctttcaattc tgctttccac 211860
   acgacctcgg gagactattt caccgaactt cacattacaa aagattctcc cttctccttc 211920
   cttcagtttc cgattagtgt tcctcacttc tgagccttca ccatcagcat cctcaatgtc 211980
   cagttttcta ctctgagtct gttcatgtca tttaggcttt ttctaccatg ttcctcaaaa 212040
   ttcccccagc cttcacccac ctggttacaa aaccccttcc acattgttat ggattgaact 212100
   gtgtccctcc caaactttat acattgagat gctaatcccc aatgtgacat tgtttaaaga 212160
   tatggacttt aagaagtaat taaggctatg tgaggtcaca agggtaggac cctaatctaa 212220
   tagggctggt gcctttataa aagaaaggaa gagctcggtc atggtggctc acacctgtaa 212280
   tcccagcgct ttgagaggcc aaggcaggag gatcacttga gtccaggagt tcaagaccag 212340
   ttcaagaaca acatagcaag accctgtgtg tattttttaa atatttcaaa aaaaaaaagg 212400
   aggaggaaga gacaccagag gtgcccaccc agagaaaagg ctatgtgagg atacaggtaa 212460
   gaaggcagcc accccagtct gtgttatttt gttatagcag cccaagcaaa ctaatactca 212520
   tgtttttagg tatttgctat agcagttccc cacttccagg tatcaaaagc tatactagtt 212580
   ttctattgct atgtaacaaa ttaccataaa tataacagct gaaaacaaca cacatttgtt 212640
   agtatccttg cagtttctat gggtcaggag tctggcacag cttcactgac tcctcaggtc 212700
   tcagaggctg aaattcaggt gtcagctggg ctacattccc cactgccttc ccatccagag 212760
   gcttgacagg gcaagaatcc acttcgcaaa attcatttcc ttacagttct gagggaatgg 212820
   ggtactgagg ccagaaagga gaagaagcct ggtttgttga tgaacaggca accccatagg 212880
   caagccgact cagttgtgcc agggatgctc tgagatcaca tggagcacac ctcacaagta 212940
   gcccagcaag gagcaaggaa gttgggacat ctacccacca actccaagct ttcttggttg 213000
   tgcgttgctc ccaggagtta atttcctggc aactccacac ttggccatgc attccccaac 213060
   agccagagga caccttgtgg tagagagact cagggaaact tcagcatgga taggaaattt 213120
   ctgcaggtgg cctctggggt ggactgaggg gataaaggca cagcaccggt ggcaactgct 213180
   atcagtataa tatcagcttt catttcctta acagatgttt attgtgcacc tactatgttc 213240
   tgagagaaaa aacaacagta tttggtcttc accttcacag agtgcacagt ctagtgaggg 213300
   agaaagaatt gtaaagacaa atgaaaacat aatgttataa cagaagtata aatattttgt 213360
   tatgaaagct ttgagaacaa aatgactata cttcctacaa aatttggaga tgtaactttg 213420
   gattttatgg aagagtatag tcttatattc atacctaatg aatgaatatc cctatgtaaa 213480
   caaatagtaa ctttacaaag aggcagtgac aatgtattgc ttaagagccc tctggagcca 213540
   aaccatccaa gataaataaa aaatcttgga ttcttcatat tccagctata tgacctagtg 213600
   caagttactt aacctctgtg agcttctgtt tcctcatctg ttagaagtgg atataacagt 213660
   actacctcat agggctggag taaggattga atagcttaat acattaaaac attagcagtc 213720
   aagaaataaa aaatgaatca aaccaaatgg tcacatgtac aaagtataat gaaaattccc 213780
   ttttttaaga agagaacaat attttgagat tttctctctc aattaataaa acaaaaatta 213840
   tacatgtatg tcttcatcat tttaggaata ccatttggaa taatttatat gtaactctct 213900
   aaaccgtaca agtgtgagtt ctcagagttc ttataaattt gaatggaaag gccaggcgca 213960
   atggctcatg cctataatcc cagcactttg ggaggcagag gcaggaggat cacttgaggt 214020
   caggagttcg agaccagcct ggccaacatg gtaaaaccct gtctctacta aatttacaaa 214080
   aattagcctg gtgtggtggc gggcacctgt aatcccagct actcgggagg ctgaggcagg 214140
   agaatcactt gaacccagga ggcggaggtt gcagtaagca gagatcgtgc cactgcactc 214200
   cagcctgagt gacagagtga gactccattt aaaaaaaaaa gaaaagaaaa ggaaaagaaa 214260
   aaacattgaa tggaaaaata ccgcaggtct ctttcacttg tgggtgcatt caagtgtgag 214320
   gtttctaagg gcaggcttcc agtggtatct ttcacctgaa acctgcaggc tgctgcagtg 214380
   ctgcaggcca ttgctcctac catgaagcag gaggcagagc aatgactact tgcagcagca 214440
   tctgacatta gcagccctca cctcccgcct catgggtcag cagcatcttt gcaggacatg 214500
   tgagctgtga ggctggaaac ctcattggct ggaaacccca agccaagttc actttctgaa 214560
   gtctgtaggg gtggggaaaa ggagcacttt ttaaacaaca ctaaggagag gtgcatgagg 214620
   aaatagcttt cgtcaaatct ccatgtccta aagccagatc ccacttagct tagagcagaa 214680
   agctggagaa acagctgaat gtgataaacc ttctctagaa atgaaaccct gtggtatgtt 214740
   tagcagatat ttttatctat agaagaaaat gtgcttcctt tgagagttat ttctaaaaac 214800
   ctttcatttt tatgttttta ccacttgaga gcactgagta ttcatcatag gcagcttagc 214860
   aaaattagaa aactggagaa aaaaataaag acctgtcttt tttttgaatt ttgaattttg 214920
   gtgtattttt tacaccattt taacacagac acatgtgcgt acacatacac gaaagggtga 214980
   gattgttgac tgtatttaca cagttttgat ttcatttttt caccaaattt caggggttac 215040
   caaagtttct tggttctcag taacttgtca cagcacctcc tagaaaaaaa aataaataaa 215100
   aataaaagcc attccattta ttaggtaatt cagtccaaac tgtaagtatg gcataacaac 215160
   aaatacatgt aaattgaaat ttgcaaaata cttatatttc attctgaaac actggtgaaa 215220
   tgtgcaggcc tgctggacac cgcctcactt ctccaacctt ggaaccaggt tggataccac 215280
   accctcaccc tcatttcctg ttccacattg attttcacac aggacttttt gtttgtttgt 215340
   ttttggtcat ggcaactgcc agatacactg ctttacattt gatgtcatca aaatacatgt 215400
   agtgcaatct aatgttgaaa gatgcactag taatttagaa ggtgtctcac aagttaaata 215460
   tcgttgttgc cttcaaaatt ttaaaatagc ctgccacagc attgtaagtt cgctgcagtt 215520
   agttccctgg ggtaccttgg catatggttt ggaaactatg aacttagttt ccttttgaca 215580
   gcaagattat ttttattatt tttctttttt ttctttttct gtttttgttt gttttggatt 215640
   tgcaatatta ttttaagtgt ccacattaga attctacaga tgtgccataa ttttaaaacc 215700
   ctcttattgt tggaaattta tgttgattgt tagtttattt ttattgataa tttcattgtc 215760
   ttcaaacggg taagaaactc cattgtttta tgtaaaggtc gagtctttga atccacaatt 215820
   actacccata gttatttggc ctatttatat tcttaccttg cagtttcaga gcccagtgag 215880
   cccaattagc tggtggcacc tgcagtcctg aatgctatga tttttcacct tccaaatcct 215940
   atgatcttca tggttttgtc actgatttga ttttggaaac agtgagccta aaacaggaaa 216000
   ctatttttta acccatcagc tgcgcttcag attttcaagt taatgaataa aaataaagcc 216060
   aaggaattga caagcctata aattgtcagc aaccagcaaa gctaggagaa cacagggaag 216120
   gaatgaagac agagccaggc acagtggcat gcacctgtag ttccagctac tggggagtct 216180
   gaggcaggag gatcatttga gctcaggagt tcaaggctgc agtgagctag gatggtgcct 216240
   gtgaatagcc actgtactct agcctgagaa acatagcaag accttctccg taagaaaaag 216300
   aaaagaaaaa aatatgaaga caaataaaaa tcattgtgaa attcaaaaaa gtcttacttt 216360
   tgataacttc acaccaaaaa cccgtgcaca gcactatgcc ctacaccatc catgggcgtg 216420
   gtgtagcaag ggaagatgtg aggggactac ccagcatggc gcatagcttc ttggcacagc 216480
   gttgtagaaa gtggctcata tttgaagtta gctcttcaat taagggtaga tcctcaatgg 216540
   attattcttc cattagcttt aaactagcat gaatcaagtc aaatgagagg gtgtccgggc 216600
   agagagtgag aacatcagga tgaaggagtt atcaaatata aggagatggt gttgaagcta 216660
   gagcttatga aggtggtgga ggtcattgat ggtggagaag gaagtggttt ggaggtgatg 216720
   gaggtggtta aaggtggtgg aagtggtggt ggtggaagct gtggagacag tgatgccagt 216780
   ggagatggct ggtggatgtg gtggtggcag tggtggtagt ggtagtaata atactttttt 216840
   aaccttcatg aatggctact tgtagatgta aatggcccgc atctatttta tgcccagtta 216900
   taaagttctg ttgcccacaa accaatgtga agaatcctta cttaattgct tataacagat 216960
   tcataatttg tttgtttgtt tctgtgagat gcgaactcac tttgggatgt aggactgggg 217020
   ctcatagtta gtaacatatt tgaacacaca ttaccccatc taagcccctg tgaggtaggt 217080
   attatccttc ttttacagac tgggtgatct gaggctcaga gaacccaaag gactcaccaa 217140
   cagtcacaca actctcaaat agcagagctg aggcgtgctc cacccagctc aggagactcc 217200
   agagccaggg cctttccaca aagcacagag ctgtgtttga gatcgctgtg acttgtcaag 217260
   ggtataatgg gaacactagc acattataag acagagaggt aaggagaaaa catgaacctt 217320
   aattttaaga aaacagataa gttggaagaa attctgttac ctttataata tttctacccc 217380
   atcctggaca tggcataaaa atctttctag aagtaccttc caagtgtaca aattctactc 217440
   ttttctaagg tattcaccat atccatacct tttttaggta ttcaacattc aatttgttca 217500
   gcattattga atacccgtca tgggctaggc atgatgtcag tgcttgctga gatgtgaaac 217560
   tgagtatgtc agtgactgag atattgataa ccaagacagg ctaaaatttc tataggactg 217620
   gacaaaagtt gcattttctg atacagttct cctatgcctt acttattttt atagcaaaaa 217680
   ctctttctgc caaattttga gataagtatg tttaagtctt tctctagaat tgtgattttc 217740
   ctatgttttt cttacaatat tttattgtct aatattggac ctcatattca gcattgggtt 217800
   aaaattgtct tgactacaag acataccatg accctaaagt gggattgttg gatcataaag 217860
   cgtatgtacc attgaattta gtaagtactg acagatggct ctccagaatg acggtaccag 217920
   tgtactcccc accagtaaag cacaaggttg cacaccttac attctcacca tgccctggta 217980
   ttataaactg aatatttgca ggtctaagga ggatttcatt gtggttttta tttcctctct 218040
   tttcactatt gtgtttgagc atctcttcat atcttgttaa ctgttggacc attcatttgg 218100
   tgaattgcat attcaaatcc tttgtcaact ttcctgttgt tttgcgagaa ttcttgcatt 218160
   actaagagtt tctttaatgc atttaacttt aaatgccccc ttgtcttata ctttcaaacc 218220
   tgatttctct ttgtttgtac ctatcttagt ctaatactgt acttttattt gggtatctgt 218280
   aagcattgtc ttataaacag catatagttg tttagtttta tgttgttttg tctttattca 218340
   gtctgacatt tattggaatt tggtacaata atttggtaca tttatattta gtatcataac 218400
   tcatctactt gatttcattt cttccctatt acttaatgct ttatttaatg tacattttta 218460
   ttttctcctc ttccttattt cttgctgatt tgatcatgtt gcttttcatt ctgttcctct 218520
   cctttattaa cttggagttt ccactgttcc attccactaa tggattctcc actttctgtg 218580
   ctgttgtgat cagatacctt ccctttttac tatatttttc aacaaactcc caactcactc 218640
   ccccaataat attaactgta tctctcccca caaaatgcaa tatgtcctcc ctatttctga 218700
   ttccaataag atgagacaat tagaaaaatg tcttccattc ctcttcttcc ctttctccac 218760
   tccttgtgac acagagactt tgaaatactt ttatttccct aactcctaga tgttgatgga 218820
   ctcattttat acatacagca ctaaattatt attagaattt tctgcatatt gtgttttaag 218880
   agcccttatt tagcaatttt ataacacatc attcctagac agtcttactt atccattcag 218940
   aattaattca ataaagagag agagaaacag agagagagat acagactgag cacacaccag 219000
   gtgtgttgct taacacagca gtccccaacc tttttggcac cagggactgg ttttgtggaa 219060
   gacagttttt ccacagacag gggtgcggat ggtttcagga tggaagtgtt ccatctcaaa 219120
   tgatcaggca ttagattctc ataaggagca cacaacctag atccctcaca tgcttggttc 219180
   accatagggt ttgcaaccct atgagaatat aatgtcgctg ctgatctgac aagaggcaga 219240
   gctcagctgg taatgctcac cggcccacca ctcacctcct gctgtgctcc ctgcttccca 219300
   acagtcaaca gacactccgg gtatgggtcc atgacccgcg ggttggacac ccctgaatta 219360
   actgaattaa caagtttccc cctcctcctc atgtttactt catcttgagg tcaatgtcta 219420
   gattagcact attccgtaga actttctgtg atcatgtaaa tgtccactag ggtacctacc 219480
   agatacatgg agatatggag ctctggaaat atgttgagga tgactcagaa ttgaattttt 219540
   gatttgattt tattttaatt aattttaatt taaatagtca catatgtcta gtgggtacca 219600
   tattgaaagg gaagatctag atcatctgtt atttggataa agtcttgcct agcattacct 219660
   caaaatggtt agagataact gtaatatatg atcatgtttt gctttcttaa atctaacata 219720
   taatcacgta tatatttggt tttggttttg ctaatgagtg aattatatcg tggttgagta 219780
   taggtttccc agtcctttcc tttcaggctg ggcttgctag aagcagaacc tcagatgggg 219840
   attcttctgc aagtgactta ctgagggaga tgtctcagga ggcacccgtg acagaataag 219900
   gtaggcagga gagtgctgaa gaatattatg gacagagctg tggtttcagt tccagtctag 219960
   cctgagcctg agaccatgga gtatcaatgg caccatgggg ttgtctcacc tggagatagc 220020
   aattctgact tttattacca tttttcaata agtcattagc tgagaccatc cccaaagaaa 220080
   ggcataacac cgcaggcccc gctgcatgag gctgtgagca ctctctggat aactgtgggc 220140
   tgttctcagg aggagtggcc tgccattgcc cgtcagccac cacactcaga cctaagtcaa 220200
   ctgcaggact gtacttattt aaacttcagg tttttagata ataagcccat tggtattctc 220260
   attctttctt ggaagtatgt aagagttttt ttcattctta gaattaagga attttctagg 220320
   atatgcctac ataagtattt tttttttttt ttttcaaatc agcccattaa tattctctct 220380
   ttcctggaaa tatgtaagag acttttttta ttctaagagt gaaaaatttt tcaggatatg 220440
   cctaggtgtg tctttttttt tttttttttt aatcagtcct gccctgaact taatgacatt 220500
   tttttaagct gtctattcag gtccttagct caaggaaatt atcatctatt atttgttaaa 220560
   ttctgcccgc catctgttcc tttttattct tctgacactc ccttattttg catgttaagt 220620
   ctctttttcc cctatgactt tcctctcttt atatttttgc ataggttttg agacatctct 220680
   tctacttgtc agtctaaacc tcatgtctca gcagtgacca tcttctcctt caatcaatgc 220740
   tttgaattgc ctatttccag acactctctt ttgtgtcgtg cttgaatcaa cagagatgct 220800
   cttgttgctt tttacattca agttgtcatc tgtctcttcc ggtagctctg ttttgttggg 220860
   taagagttta atctgttctg agccattgtt ctctctctct ctctctctct ctctctttct 220920
   ctctctcttt ctctggctgc tggatacctt tggttacaat atattaatat atatatattt 220980
   tttctggcta ttttctttct ctgtgtggta gtagtcccat gattagagga agacaaaatg 221040
   gaaactacac ctggggactg atggcgtcat gcaggcagac tgtcaattcc ccattgagat 221100
   gcacggagga agccctctct tcctccacca tctctcagac tctcccaacc tccagggcag 221160
   agactctgta tagcttttaa acaccacatc tccagagctg ggaaatccat ggtgccatag 221220
   ttcacggtct tgggtcccca tatcactgtg agccatgttc ttcccaggac ctgagcctgc 221280
   tgtccaccca tgagcagcta gagcgcaacc tcttccccat ccaggtggaa gcaagcccac 221340
   aagctctctc tgttgagatg tcctcagcac ccaagccttg ctggtccaac tgcccatcag 221400
   ccccagacgc catgtggtca aaagaagtgg acaccctgga gttgaacatg gaggagaggg 221460
   agaattgttt aggtcatgat ggtcccagaa tcctcctcct gctttccgca atccttctga 221520
   agagacctga attcaaattt tcattgttcc cttttaggat ctttgtttca acaaatgcca 221580
   gccaaatacc aagcccagtg ctgaggacac aacattgaat aaatcaggga tgagtccttc 221640
   cttctaggaa tgtttactcc aaaggggaaa taattgtcca tgtgatctag ctctaattac 221700
   agagtgttgt gaatgggcaa ataaaggctg tggtggaggg cagtgttgct gattcatatt 221760
   tctgccctca ctttctgtac ccaccttgtt acaacagaac ttccgtccat gttatctgta 221820
   cactaaaggt gtccatttca ttccatctca tccttggaca acgtttaaca agttgcatgt 221880
   gaaaaaaggt ggctgagatg atggaaaatc tagaattcag gtgatctgat gattggctga 221940
   gagtactgat gattggctga gagcactaat gattggctga gagcactgat gattgtctga 222000
   gagcactgat gattggttga gagcactgat gattggttga gagccctggt gatcttctgt 222060
   caaagaatca agggagggaa gtaaacaatt actttcttca aacattgaat gactgtccca 222120
   cagcagggga attaataacc tctaggaatc ccctggaagg aaaaatagga agaggtaagt 222180
   gaaggcagca cggagagact ccagtacttt acctcaatag ggcaagactt ggaaatgtgt 222240
   tatccagcaa tgcaaggggc tgccttggaa aggcgagctc ctcatcttta ggaatgttca 222300
   aatatttatc caacggcacc cttaaaaggg acctgaacac agcagacagg gcaggagaaa 222360
   aagccaggaa gaactgggag ttgagaagtt tgttagggat ttggtgtctg agaaatgaga 222420
   gtattgggca gtgaaaaggg gtaagagaag tgtggccagg gttgccaagt gtgtctcctt 222480
   caaagagaag cgtggaagga atagaattgt tttcagtttt tatcttgcca tgtcctagat 222540
   aatagctaag tttcctgaga tcaaaagcag tcatggcttt tttcttccaa actttctttg 222600
   tttctatttt ctctggtcta cctaaagtaa aacaacagta aacaatagga tctggccagg 222660
   cacagtggct cacacttgta atcccagcac tttgggaggc tgaggcaggc agatcatttg 222720
   aacccaggag ttccagacca ggctgggcaa caaggcgaaa ccctgtttct actaaaaata 222780
   caaaaattat ctagttgtgg tggtacacac ctgtagtccc agctactcag gaggctgagg 222840
   tgggaagatc acttgagcca aagaggtcaa ggcttccgtg agccatgatc ccatcactgc 222900
   actccagcct gggcaacagt gagattaaaa aaaaaaaaaa aaaaaaaaaa aaaaaaaaaa 222960
   cgaaagaaag agagagaaaa gaatctgaga gaagccactt tctaccttcc tttcttcctc 223020
   ttaaaacctc cagtcctgaa acatatgata tgttatgaaa atatcgattc atctcagctt 223080
   ttccttaagt tgcttgtact tatagaactg tacctgtcac cctgctcaat aaagatttga 223140
   tgattgaatt aaaaataagt tccagccggg cgcagtggct cacgcctgta atcccagcac 223200
   tttgggaggc caaggtgggt ggatcacgag gtcaggagtt caagaccagc ctggacaaca 223260
   tggtgaaacc ccatctctac taaaaataca aaaaaaaaaa aattagctgg gcctggtggc 223320
   acatgcctgc aatctcagct actcgggagg ctgtggcagg aaaattactt gaactcggga 223380
   agcagaggtt gcagtgagcc aagattgtgc cattacactc cagcctgggt gacagagcga 223440
   gactctgtct caaaaaaaaa aaaaaaaaaa aagttccagg ccgtaatagt ctgtcctgtg 223500
   tatcaacata tgtctcagca ataacactgg gcaggggatc ttaacagaaa actctgtagc 223560
   cacatggagt ggagctatgt tttatcaatt tgattataat ttagaggggt taaaacttgg 223620
   gggggtacct gactctttgg agttatgtag tatttactgt atgttctggc acttcctaga 223680
   gctgttcctt ctcccttgat agtgtcgccc agcgctgtat gttccttgga gtatgctgtc 223740
   atctagttgt gtgagagcca cattacaagc ctatttgtca catgcttaca taatgtttat 223800
   ttttccttat actaatatta cctccactga atgttaattc gttgtagcgt ttcttttgat 223860
   aggttcgacc ttttcttctg agatgtatct tcaatttcag gacacaattt agaggcttaa 223920
   aggtatcttt gatgctttga caacaaacct catgtaaact tccacttttt ttatttttcg 223980
   tgctttggta gaagagctgt tcagataaga attgggattc ttgccgggcg tcgtggctca 224040
   cgcctgtaat cctagcactt tgggaggccg aggcgggtgg atcacgaggt caggagatcg 224100
   agaccatcct ggctaacacg gtgaaacccc gtctctacta aaaatacaaa aaatcagccg 224160
   gccgtggtgg tgggcgcctg tagtcccagc tactcgggag gctgaggcgg aagaatggcg 224220
   cgaacctaag aggtggagct tgcagtgagc cgagatcgca ccactgcact ccagcctggg 224280
   tgacagagcg agactccatt tcaaaaaaaa aaaaaaaaaa aaaaaaggat tgggattctt 224340
   gggtctcatc cctgcaaatt gagtaagtct ctcagcctca gttttatcat attacaatta 224400
   agcaattaag cgcttaactg tgctagctgt ttccaatgtc attacattaa ccaactggta 224460
   taaaaataaa atcgtctggg aagaattact taccgtacag atctggggcc caatccccag 224520
   agttttgact caatgtcttt gagaaccact gggctaactc gtttctttcc actgattgaa 224580
   attttaaatg gttaaagcag cctggctttc tcaagaaaag tacacgggcg ccacctagtg 224640
   tccctgaaaa gttcgtacag ctcacacatc agatgtattg aagctttaac ttggcagggt 224700
   taccagttat aaatgcaatc gaatttgcat tttaattagt gaagccgatt atgtattgag 224760
   ataaacaagt gttgtgccat cgactcactt ggaataatta gtttgatctc acttttgatt 224820
   tatgacttgg catttttctg gactcagtta tgcctctcag gcttattctg tctgaatcac 224880
   acacaattgc aatagtccag ctttttgcaa ttatccgtat ttttgtacaa ctccagaaaa 224940
   accaaagtga aagaataaac aaaaagattt tcccagaaga aaatggcaat ttctaacttc 225000
   ttcctatgcc tactggcatt gtgggacaga ctgccaagga attcaaccac attccaccca 225060
   ctacccaccc ctggacttct atccttagac tctcactgcc cccagaatag ggaaggttat 225120
   caggccccta aggaaggacg catgctttgc ttggctctct gggccttgtc tgggcactgc 225180
   accccagtgt attatcaccc tgctgtgcca tagagcccca catcccacag tgcaagccac 225240
   aggctagcag cagcagcatc ccctgggagc ttgttagatg tggaatctca gtccccagcc 225300
   cagccctgaa ggatgagaat ctattttcgc aagatgcccg ggggatgtat atatgtgtta 225360
   aagtttgaga aaccctacca tagagaacac aaaggcttta gccgaagaga acgctgattc 225420
   ccttcccaac atgctacttg tctttgccgt gtttccagca gcataaaata gtctggaagg 225480
   atgaacattg ggcacatctg gcgttgacat tagaatctgc tactcgctac tggcgtaatt 225540
   tgaggagcac ctgaaactct ctgtgctttc agttcctcat ctgtaatata ggaactgccc 225600
   tttttaattc aggaagttgt tgtgggctaa catgtgtact gtatttgaca tcatgcatgg 225660
   ttcatagtga taaatattaa tttcatttct acccatcccc tgcttccagg tagaagtaca 225720
   atggagtgta gtttcaacat tgctttattt taaaaattag ccacagtgtc tttgacatgc 225780
   ttcccatcag aggtggtgtc taccttccct ccccttgacg tgggactggc tcctgattct 225840
   cttgtaacca gaagaatggg gaagaagtta ctctgaggct tctcagcact agaaaaggaa 225900
   aggccatgta gctttcaccg gttttcttgg gttgcttgct gtgggggcag catgcagcat 225960
   tgagaaaact ccatgctgga gaggcctcat gtggtgctcc agcagactgc ctagctgtgc 226020
   ccccagacaa gagtcaccat ccgctgccag gccacgggag tgagcatcat ggatgtctgg 226080
   cccttgtgcc ctcaggtggc tgcagcccca gccaatatct gactacagtg atcgacaact 226140
   gcccagcaga tgcccttcca aaattcctga cccacacgat tatgcaaaat caaagagttg 226200
   tttgggggtg atttattatg cagcagcagt aactggaaca cactttggtt ggtggttttt 226260
   gttatagttt ttacttcttc aacgaaggac ttattctaag tcaagattcg acaaactgta 226320
   gccctctggc caaatctagc ctacatctgt tttaggttat tgtgaatttt ctttaataga 226380
   ctttgctttt tagagcagtt ttaggttcac tgcaaaatta agtggaaagt acagagattt 226440
   ccatatgccc cctgccccta cacatgcaca gcctccccct tgatcgacat tctctaccaa 226500
   agtggcatgt ttgttgcagt caaagaacct ccatggacac gtcattatca ctcaaagtcc 226560
   atagttcaca tcagggttca cttttcattt gcacattcta tgggttttga caagtgtctg 226620
   agctgtatct accattgtca tatcataaag aatcgctgcc ctaaaaaatc ctctgtgctc 226680
   tacctgttca tccctcccac accctaaccc cggacaacca ctaatccttt tattgccacc 226740
   ttagttttac ctgttccagt gagtcctgtg gttggaatta tatattacat agcttttctc 226800
   agattggctt cttttactta gtaatatgca tttgtttcct tcatatcttt tcatggctcg 226860
   gtagcacatt tgtatatatg ttaatacatt gtggctacaa taattttgaa caaattgatc 226920
   tgttagatta agagtaacaa gttaaaaatt tcattttacc ttcacttatt tcttctctga 226980
   tgttctttct tttgttatgt agatatgacc ttctgaccta taacaatttt tattgccttg 227040
   aataacttct ttgaacattt aacaaggcag atctgctagc atcaaattcc ctgaattttt 227100
   gtttgtctga gaaagccttt aagtctcctt tacttttgga ggataatgtc acagagtata 227160
   gaatgctaga ctggtggagt ttttttctca acacattcaa tatttcactc cactgtcttc 227220
   ttgcttgcat ggtttccatg aagtcagatg aaattcttac ctttgttcct ctaaggaagt 227280
   aaggtgtttt tcacctttgg catcttttgg gatttttttc cttatctgat ttttctgtag 227340
   tttgaaaaca atgtggctag gcttaatttt gacattcatc gtggttggtg ttctctgagc 227400
   ttcctggatc tgtggtttgg tgtctgattt tcagtcatta ttgtttccaa ctttccatct 227460
   gttcctttct ctctgtcttc tccttttggt attcctatta aacatatacc ttttctaatt 227520
   atcccacagt ccttggatat tctatgcctt tttcattcag cattttttcc ctttgctttt 227580
   cagttctgga tgtttctatt gagatatcct cagcctcaga gagtctttct cagctgtgcc 227640
   cagcctacta atgagcccgt gagaagcatc cttcatttgt tagtgttttt gatctctcgc 227700
   atttctcttt tattctttct tagaactttg atctctctat ttacattgcc catctgttct 227760
   tgcttactgt ccactttatc cattagagcc cttagcacat gaatcatagt tattttaaat 227820
   ccccagtctc atagttccaa catccgtgcc atagctgagt ctggttctga catttgctct 227880
   gtcttttcag actgtggtct ttaccttttt ttatgccttg tgattgtttg ttgatagcca 227940
   gatgtgatgc actgggtcag aaacggtggc aaataggcct ttggtgaggt gtggggaagg 228000
   gaagggttcc atagccctgt gattaggact cagtgtgtag acctgtgatt aggacccagt 228060
   ctgtcttgag cctgtgccct ggactgtgag cttcattcag cttctcagtt tttccctgag 228120
   ctaggacagg gtggctaggg ggaaatggag ttgggttttt attttcctcc aggtccatta 228180
   ggctctgcaa aaaaattaaa aaccaacagt ttaagctctg ataaagtgat ttctattgag 228240
   ggaaggcctt gttaagaact aaatgctatg gcatattttt aagtggtttc tttttccctc 228300
   ctcctgacag aagcatgaac agatttttct ctgatagtca ctgtgagaac ctggttaagc 228360
   tcctggagat aaaactcaca aaagggtggg tgccccctca gattgggccc tcctggagat 228420
   tttaactccc agacctgtgc acactgagct cccagcaact cctcaatccc agttcagatt 228480
   tcctacccag tactggttcc catggacagt tctgcctgtg ggtttctgct ctggtaagct 228540
   gtgatcttct gtatcctcct gtctgcctct ccaaatttgg aggagcagtt tgccctgtgg 228600
   ccttacttct ctgacagaac taagaagatt cttgaaattt tctgtttgtt tagtttttaa 228660
   attattgtta ggatggcatg gcaacctcca agcttcctac gtgtcactgg aaaatggaca 228720
   tcccatctgt ttttgtcaat aaagttttat tggaatacaa ccacactcat tcatttctac 228780
   attttctatt gccacttctg tgctgtggca gagtagttaa gtagttgcca cagagacttg 228840
   atggcccaca aaacctaaaa tattagctct ttggactttt gtagacaatt tttcatcatg 228900
   tctgttctag gtaataggga ttgaataata ggtaaaataa agtctatttt taatagattg 228960
   tatagtctaa tgggggggga gatacagtaa aaactaataa acatcacgtg gtgataaatg 229020
   tcatgaggaa actagagaaa aggggaaaaa gtgacagggt aatggggtgg tccaagaagg 229080
   taaagtaaca tttcagcaaa gatgagaatg ggttgaggga gcgcctctgg gggtagctgg 229140
   gtgatatggt ttggatcttt gtaacctgct caaatctcat gttgcattgt aatcactcat 229200
   gttggagcct gggcctggtg ggaggtgatt ggatcggggg ggtggagttc tcacgaacgg 229260
   tttagcacca accgtcttgg tactgtcctc atgataatga gtgagttctt gcaagatctg 229320
   gttgtttaca aatgtgcggc ccctccccac tctctctctt gctcctgctc tggccatgtg 229380
   acatggctgt ttctcctttg ccttctgctg tgattgtaag tttcctgagg tctccccaga 229440
   agctgagcag atgccagact tatgcttcct gtacagcctg cagaaccttg aaccaattaa 229500
   gcctcttttc tttataaatt acccagtctc aggtgtttct ttatagcagt gtgagaacgg 229560
   ccaaatacac tgagaaatat gttctccaga cggagacgac attaagagga aatgcgctaa 229620
   gtgtgccagg aggaacaaca gacagggccc agtgagaact ggagccgcgt gtaggagctg 229680
   agggcagggc ggccccagag gacgggcctc attgagagca ttagatttgg gggtctgctg 229740
   tctgctctgt ttcagtgtct aatcagtaca tatccctgca gcattttcca tggggggcca 229800
   gacaccctgt gcctcagggg catgggggtg gcagaatgac attgttttgc ctgcaaagag 229860
   gagaacagag tttttcagga cggctcccta gaagagacat cacagtggta gttcctgcac 229920
   aaagaattta tggaggaagt gttcccaaga aaaactagta attgcatgaa ggaaataggc 229980
   aggaaaggga caaaagtgag taatggacag tctcaagtaa attcccacct ctgtctgatt 230040
   cccagggagc tctggagcac aagtaactcc ttagactttc ttctctcttg aggcagagtt 230100
   gggctctcac actcccatat agggagggaa aaactttttc cactgtcttc tgggttcttc 230160
   agctgaggcc ctgcaaatta gagttatgaa agataggtga agaacaacaa taaaaacaaa 230220
   cagacaggct gaagtttatt agcacgtgca gcttgcatat acacgggaga actccgtggt 230280
   gagtaactca aaggggtgac tagaccttgg ggcttatatg gcatcttaac aaagatggat 230340
   aattttgtag agaagtgaca agaaaagagc ttttgatgat tggggtggca aattgtggga 230400
   aggtaaacac atggggaaac atataaaaga tagggttagt gagtaaggtg tgtttgtgta 230460
   gactctctca gtaccatctc ttctctgata ataaggccgt tatccccttc ctggaatggc 230520
   agtttgggtg ggggcaggga caccttcaca acaggacttt atgttctgtt tttgggcaga 230580
   taggaggaga gtagagagtt cattctgaat ccactttttc tcaattgcct ccacttaaaa 230640
   taattcctat tccagagtgg catattactc cccggcatta ccacttctct gcctatgcag 230700
   gcaaagggat tccaataggc caagaacagt ccattggaga tgaatatctt ggtcctttcc 230760
   tgctgctgta acaaaataca gggaatggaa aatttaccaa caataaaaat ttatttctcg 230820
   cagttctgga ggctggaagt ctaggatcaa ggtgctagca ggttttgcgt ctggtgaggg 230880
   cacagtctgc ttccgagatg tccatttgaa tgagcactga aggacaaaaa ggccaaaagg 230940
   ggcaaaggcc atgtcctctc gtggcagaag agatggaggg gaaatgggcc cacctagctc 231000
   ccttaacctt ttataagggc actaatctca ttcatgacat agccacctcc tgacagcccc 231060
   acctcacact gggatttcac tcccaacctg tgaaatgtgg agggacacat acattcaaat 231120
   gacagcaata aattacgggc acagaccatt agaaacaaag cacacagatg ccgagggagg 231180
   gacacgccag aacagttaaa gaaatccaag cgcatctatg tagcctgtca acagagtctg 231240
   ctacaaagac caaaaaggaa atgccttagg atcttttgca catgccgaca tcttatattt 231300
   gcctatttgt taattcaagc atcaggtaca tcccttgttt gctatttggc taaatatatt 231360
   aatatatatg atcttaagta ataatgctgc aagcacactg caccatgcag aatcggtcta 231420
   cagtcatggg atggaaccac atcaaaaccc catgcccagc agttttgttt tagaattcca 231480
   gagaaacaca acagagatag ctgccagtac aggcttactg tgcaaggcca cattcaagct 231540
   ctacctaggg gcagcatgca agggaagtgt ttagtaatga ttatctggag gatgcaagct 231600
   aattcttcca tttctttgat gccttccata gtggctatac caattctgaa gacattcaat 231660
   ataaaaataa ctgtgaagtt tattagccaa ttatatacat tcggctttgc gtatggcggt 231720
   gtgatttcag ctctcaccaa gatactttca ggaagatgac tttaagacct tgcggttgct 231780
   tttatctatc ttcacaccag ggggcaggct tgagtcgttt ttaattcctt tgtgtgttgt 231840
   tcttagaaaa caaaggctgt gtcttattta aaacaaaaca aaacaaaagc tactacactt 231900
   ggttacctca aaggaaaagt accatttggc acttacttct accgtcacag gctgattaca 231960
   gaccttccat agaatggatt tagagtgtta catatccatc tgtcctgtct ggatttcttt 232020
   ccactccatt tggaaccatt tttgaggatc catttgaatt ttaaacagga agacataatg 232080
   tctatgctaa agttccaccg actaatatac aggccaaagt ttccaaattc ctaaaattga 232140
   aaatttgtca aaatttatag tcatttttaa tgttttgtgt tgctggtgta aaatcttcca 232200
   ggatagtatg gccagtgccc aaatgcaaaa acaccctcat gttcctgcct ctgatggctt 232260
   tgaccactgg tggctgaggg ttggcagcag aactccagga ctgtgccttt ggtggactca 232320
   tttgctccat gtgaccccca ccataaacgc aggccttgca gaaggaaagg aagagtttgc 232380
   atctcggcct gctctgtccc cttgagtgtc tttgcctgga aattgtcagt gttttcacac 232440
   ttatgttttg ccctcgacct ggatatgtgg gcacctatgc gaggttaaac tgtgatactt 232500
   tgattttcat caatctggaa agtggtggca ctagctcagt tgcttagggc aggacaaacg 232560
   gggttgcaac tgcaggtgat gtcttcattt gactcattct cttggctgtg actctacctg 232620
   aaagccaact catcagcttg caaattacag ccactgaact gggcctgggg aggcaaaggc 232680
   aagtctgtct tcattactag agaggctgtg taaagtgctt tccccttccc acccagcatc 232740
   atgtggttgc caatgccttg ttacacaaag cccaggtgtc tgactgcaaa gagaagcggt 232800
   tctctcatcc tgagcccttc caccaggggg ttgaattcat atgttcccga caataagtac 232860
   aacttggtga agaggcatca ctaattcacg aaatctgcag atacgttgct ttattcttca 232920
   tcatgttgaa gatccttcat ttctaaggtt ttccttttct cttcttttat tcttgttgcc 232980
   atcaccagct gcatcagata gatgaaggag cagcagctgg acagtttaat ttattagggc 233040
   tgtcagggcc aatctactgc tgataataga acacagtaga agtggatctg tctagtaaag 233100
   cacatttaat ctaaatgatt caaatagtgt ttcagcagct tcctataaat taattcattt 233160
   tagaatcccc ctattaactt gcttttaatt tcagaatatg tgcacattgc acacacattt 233220
   ttctcttaaa aataccctgt ctgctcatgc agagaagata tttgatattt aaaatatttt 233280
   tcctctgatt ttgaaaacaa gattgaaagt caaattaaaa gacatcactt gagatcatgg 233340
   atgtctgaac accatgtctt acctagagac aatggaggat tacttgccag ttcaaatcca 233400
   caccaggaag gaatgcttcc acctcaagtt ttggactgac tggctgtcct ccaagcgctg 233460
   gagtcctctt ggctaaggca ttcatagaaa cttcctaact tttcattatt tcaaataatc 233520
   tcaaatttac agaaaagtta caagtaccat ataaagaact ttatgtcctg aatctattaa 233580
   gttgccaacc tgacattctg tcattccaaa taccatagtg tgttacctac ctgctataga 233640
   atgaatactt gtatccctcc aaaatttgta tgttgaaatc cttaccctca gtgtggtgat 233700
   atttggaggt ggggcctttg gcaggtgatt gattaggtca ataagggcag agcccttatg 233760
   aatggtatta gagtccctgt caaagagacc ccatacagct cccttgtgtg aggttatagt 233820
   gagaagatgg ctgtctgtga accaggaagt gtgccctcac cagacactgt tcctgctagc 233880
   accttgatct tggaattccc agcctccaga actgtgagaa atagatttct attgtttaaa 233940
   agccactcag tctgtgacat tctgttaaag cagtctaaat cgactaatag taccaaaaag 234000
   aagcatcatt ttctttttct ccttttcttt tttcttttct ttttttttag acagagtctt 234060
   gttgcattgc ccaggctgga gggcagtggc atgaagatca tatctcactg tagcctcaac 234120
   ctcccacgct caagtaatcc tcccacctta gcctccggag tagctgggac tgcaggcaca 234180
   tgctacctac catgcctggc taatttttta aaatattttt tgtagataca gggtctccct 234240
   agtctggtct ttaattcctg ggcccaagtg atcctcccac cttggcctcc caaagtgctg 234300
   ggattacagg tgtgagccgc catgcctgcg ttttctacac agccacatgc caccatcaac 234360
   attaagacat taacactgag accttactgc cacttattta tcaggtccca agaaggttct 234420
   ccagttgccc taataatgaa gatccaattg aaagtcacat gttgcatatt ttgtcatatc 234480
   tctttttagt cttattgatt ccagaacagt tcctcagcct ttctttatat tggccttggc 234540
   acttctgaag agcacaggcc agttgtttta cagaatgtcc ctccattttc ctttggctga 234600
   cactttctca tagtcaggtt caggtgatgc acctttggca ggactatcag agaagccata 234660
   gtgggctgca ccattgttcc agattcagtg tgtttcatgg ctggcggcgc tcactctgat 234720
   cactcagtcg aacatggtgt ctgccaggct tttcaactcc aacgttactc ttttcccctc 234780
   tgtaattaga aggtatggga ggaggaagtt agtagagaat atgaaatata gactttcatt 234840
   tttaaaacat aaatcagcaa aagatctttt atatcaaatt ttagttaatt ttctaaaatt 234900
   gattggcttt cttaagtctc tttgttctag atgcacacag aaggaaatta tctcttggtg 234960
   tctctgtttc tcatttcatg gggaggcagg tcctacaatg cttgaaaacc aaggcctata 235020
   ctctctcacc cctatgtttt taagttttaa ttgacattta atgtattctt taagtgcaca 235080
   agttatgaag tgtacagccc aacacatttt cacaaaggaa acacatcaga tcaagaatca 235140
   cgatgttata gacccacccc agcagattct caccttgtgt ctgcttccat tctcaatcac 235200
   ctccatgggt caccgttgcc aactatacat tttgctggtt tttgaactta attttcatgg 235260
   aattctttag tacctgtatg attgtgcctg atttctttca ctcaacatta tgattatgag 235320
   attcagcctt attcatgcca ctgtatataa ttgtagttat tgcccctcct gggctacggt 235380
   ccacatgtgg ggtcttttac tccttccttc acttgacaag cacctgtgta ttagtccatt 235440
   ctcacactgc tacaaaaaat taactaagac tgggtaattt ataaagaaaa gagtaattgg 235500
   ctcacctcct ctgcaggctg cacaggaagc atgatgctgg catctacttg gtgtctgggg 235560
   aggcctcaga aaacttacaa tcatcgggga aggcagaggt ggagcagata catcacatgg 235620
   ctagagcagg aggaagagag ggcggcagga ggtgccacac acttttgaag actgagatct 235680
   tgtgagaact cactcattat catgagaata gtaccaaggg gatggcacta aatcattcat 235740
   gagaagttca ccccccatga tccagtcacc tcccaccggg ccctaccgcc aacattggag 235800
   attactattt gacatgaaat ttgggcaggg acacagatcc aaatcatatc aacctactaa 235860
   gcatccagaa atttaagtaa agtgagtaac actggtgctt acacttttga ccatctagtc 235920
   tgtctaaatt ctcttcactt ttttttgaga tggagtctcg ctctgtcacc caggctggag 235980
   tgcagtagcg ccatctcggc tcactgcaac ctccacctcc aaggttcaag ccattctccc 236040
   acctcagcct ccccagtagc tggtattaca gctgcatgcc accacacccg gctgattttt 236100
   tgtactttta gtagagacgg ggtcttacca tgttgaccag gctggtctct aactcctggt 236160
   ctcgagtgat ccgcctgcct tggcctcaca aagtgttggg attaaaggca tgagtcacct 236220
   tgcctggcaa ttctcttcac ttttaaccca gtggggaagc aatagtagtt gtccactgta 236280
   tacctgacac tgtgccaggt gttaacacat accatgtctc acttaatcct tcctccaatc 236340
   tagcaatata tcattatgtc tgttttttag ggaaggaatc tgatatttac agagaggttt 236400
   ccatagttcg cataagacct tatagttagg tgacagagca gatattcaaa cctacatcta 236460
   tccatgtgca aagctggcta cagcagtcat gaagatgccc tgcttcatat cccctgaaca 236520
   cacctgagtt tacctgcagc tttggtggac tcatctcatg cacactaaca gtttctctct 236580
   gctctgtgtc caagggcttt ctctgacatg acaatcctgc ccagaaatgc caggcaatga 236640
   atgcccccaa aggtaatcct taaccaatgt ggaaaggaag ttcttggata aatacccccg 236700
   cttctctgac ctgcatttga catggctctg cagggcatct gctgcagcac agaggcccag 236760
   ttgcccacag cattaaccag ctcaatgggg cacccaatgt ggatttctct cattttcatt 236820
   gcactttctt cagtctgtca tgtggctttc tggaatctcc tcacaaataa agaacctgca 236880
   ctaaagtcct tcttccttga gttgctttca agggaaccca aattagaaca cttattctct 236940
   ctacagttcc ttgtactggg gtacctagtg cttctgaata aagctgcatc ccagataacg 237000
   ccccagtgag gagttgtaca agcctgggct tttcctccct gccatagaat gcctagccta 237060
   cagcattcca tggccccttg gaggcggaaa actgacaaga caaccaggta tcttttctct 237120
   aaatgtctaa agagggagga gaaagaggtg tcattttggt gtgtgggtgg caaagggaca 237180
   aagcagatca agctggaggg actcacaaag caggaggagt cattagaggt cacaagtttg 237240
   gatgcagggg tgtgaatagg atttctagct aagagaacaa gctttggctc tagagagacc 237300
   tggcttcaaa acctgattcc tccacttaaa ctatagcttt gtgaccttca ctgagtggct 237360
   tgtcctccat acttcagtgt ccttgtgcca tatgctgttg tgtcaaactc ctgttaacct 237420
   caatagcaaa ggggacccca ttcaagaggc cagagaagag acccagagcc agaaaatgag 237480
   acatggggtt ttattagggg gctcacatac aggggagagg gtccagtggc aggaaaaccg 237540
   caattgcttg caaatagcat gcagtttatg tagcattttc acatagcacc ttccccctaa 237600
   tgacatccag ctggcaacct tcatttaacc caaaactcag gacctcagtg ccctaggcag 237660
   cttgtgttcc acagaagagc ccaggggctc tgatatttgt cattgacaag gaactaatca 237720
   ggttggccac tcccggatgc cctagcttgg aacacacatt caggtgcatc tgccatacag 237780
   ggttattcta aggatgtgtt taagttattg ctatcaggtg tgtttaccct gatatcaata 237840
   atttgtggtg tggtgatcat aattctgcca atcttatagg attttttagc ggattaaatt 237900
   tgaaaatata tgtaaagctc ttaacacaac tgtcaggaag taaagctttt tttatgacgt 237960
   tagcaatgtg aatgtagcag tgaagatgtg ggttctgtag ctagacggcc tgggttcaga 238020
   ggcacagctt ttctacctcc tggctcaaga ctcagtttcc acacctgtaa aatggggata 238080
   atagtgcaaa tctcatacgg ttgttatgga gattatatac agacagagag ggagagcagg 238140
   cacttcaaag ggtgtgtata gcagcacagc ataagcactc taatgttatc tgtaaatcta 238200
   gaaaaataac ttgaatccat aagctatctg gagtcaggaa agccctatca tgggtgagga 238260
   tgtacacagt tttgcttaac taacgtacca gttggggtat tagctcatca agaagaacaa 238320
   tatggaaaga cagtcttaaa tgtttttctc aggcagaagc tgtatgtgag ctggggatca 238380
   gccagggaga ggcagaaacc tttgggtgcc aggaggagct gattctctga tataaatagg 238440
   gcagaacttg gagtcttcag ggaatgaggt ggaatgaatg gaagggattc acagaagcgg 238500
   gaagaagaaa acagagaagg tggagttgac acagataaag aaaatggatg gctacctcct 238560
   ctcttccagg taaagcttgc tgctgccaat gtgttctccc tccccaacta gaaacacttc 238620
   ccaggaaagg ccaaaagtgc ttgcattact tcctgagagc tccatctacc cagaaagtca 238680
   ctgtgtcatt ggcaggtagc ttgtaggcca tttggccatt ttctacaccg gaggcgtcta 238740
   taatctttcc cctccagggc accattcctt ccagcttgct gcactttgtg atgcttccgt 238800
   gttgaaagta gtccctccca ccttcctggg ttctttgcta agataaaatg aggcaaaata 238860
   aatcaacttg gctttcacat caaagccacc tctgtctaaa ccatctgtga cttagaggaa 238920
   ttagaactga aattcgtgag tgatgacatc tttgttttgt ttgcttgctt tttggttttt 238980
   gttttgttat tttctagtta gagtagattg aataaatgga tagttttgcc tgctaaagct 239040
   ctgttacatg tacagttcag gcaagatggg gaatatcctt ggattcagtt ctaattattc 239100
   cctcagagga aaccgaactt tgattgatga agaggatcct ttctgtgttc agctcagatg 239160
   ctgtggtcac gtttgactga tttggagcag ctctgaatct cagctctgtc aaatattgca 239220
   tgtaaaccaa gcttcttact agctactaat ctagcatgga aaagtcctgc agtgcaaaat 239280
   gatgagaata gaatgatctg caaaaagaac ttttagattc tcagaaactg actgacgaat 239340
   cagcctgaag cactagccat aaagataatt aattcatgac aggtgagcat ctgtagtaag 239400
   cacatcccag acaccaaggg gctgtgctcc acctgaatac aatgcagcta gccatgggca 239460
   ctattattcc ctaggttgta tggtagcaac attaggagca aggggtctgc attcagacat 239520
   tactggagtt tattttattt atttatttat ttatttattt atttatttat ttatttattt 239580
   atttttgaga cagagtcttg ctttgttgcc aggctggagt gcagtggtgt gatctcggct 239640
   cactgcaacc tccgcctcct gggttcaagt gattctcctg cctcagcctc ccaagtagct 239700
   gggactacag gagtgcacca ccacacccag ctaatttttg tatttttagt aaagacgggg 239760
   tttcactatg ttgaccagga tggtctcgat ctcttgacct cgtgatcctc ccgcctcggc 239820
   ctcccaaagt gcattcctgg agtttaatcc cgagttcacc agtgtattag tcaactatgg 239880
   ttgccaaaat gctacatagc aaacaaccct acaaatgcta atgacttata acagcaaaca 239940
   tttctttttc ttgctcatgg gtcttaggct ggtagggtta ctctgctttg aggttcaggt 240000
   cacttagagt tggctgcggg ttttaggttg ggttcaggtc tgttctggac gcctcttggc 240060
   attttttttg gaccagcaac tgcccaggtc aggttttttt cagggcagat ggcaaagact 240120
   caaggaacca gtcgatatat ataatagcct ctgcttttgt cagttcacca acattccagt 240180
   ggccaaaaca agacacgtgg ccaagttcaa agtcagtggg tcccactcta ctgggaggct 240240
   cttaaaaagt cacatgcaga cggtgagata tatcatcatc acagggaagg agggaagaat 240300
   cgagagaata tttcacctgc tacaaccact aaaaaagcta tggaacctca gtcaatgctt 240360
   gtaacttctt ctttctgatt tgtaagatga ggtgtaataa taatataagt ctcctagggc 240420
   tattgggacg atcaaatgca agagacagat gatagcacat aaagattaat aaaggggaac 240480
   tttcttgtta tagaaccaac cctttctgtt ttaacccttt ttgtctcctg acctctttcc 240540
   cccattctgg tagcaactct cttcctcaat ttctaaattc ttggaaattc tgcttgcttt 240600
   cctctcattt ccagggctaa ttcaaattat tatgcttaca catgaaatta aggcaatgtg 240660
   aatatctttg cagactctcc cttgtaatag gctgtgggaa agatagatgg gaatccagat 240720
   atcagcttta ttattataaa gccccattta ctaaggaggc cttgtacaat atacacctct 240780
   atctcaccct ccagcttaga tataagagag agctctgcaa ggctgatact ggtcaacctg 240840
   ctacagagga gtcactttac acacacttac ctgcctgttt ggggtcagag tgggactctt 240900
   aacctccaga agagaagttc tgggtcctta ccccatggaa gtagaaaaca caggctatgc 240960
   agacaggaaa actctccaga aaaaatgaag agaaggtcct ttgagccatg catgggccct 241020
   aaccgttctc ctttctcccc tgccagataa gccacactcc ctgccacatg cagatgagta 241080
   ggggaggtgg gagagaacag aagtattatt ccagtggaag actctgcagg gaaatgtgag 241140
   gacagcagag aagcagttcc cctgacaacc tgttttgcag ataagaaagg ttaatccctt 241200
   gctgggagct ttctatccag cagagaaatc agcagcctta gagtcagtgg cctggactaa 241260
   gatcaataat ggttatctat tatgacaaat aagtctgtgt aacaaagcac tctgaagcta 241320
   aagagcttaa aatacactca aactgcaaaa cattactgag agaaagaaga cataaataaa 241380
   tgaaaagaca tcctctgttc atacattgga agacataata ttgttaaggt gaaaatgcta 241440
   cgaaagtcat ccataggttc aatccaatcc ctatctaaat gtcaacagtg ttttttacag 241500
   aaataggaaa accatcctaa aattcatatg gaatcccaaa gggcccaaat agccaaaact 241560
   ctcttgaaaa agaacaatgt tagaggattc acacctcctg atctcaaaac ttactacaaa 241620
   actacaatta aaaccaaaat aatattataa tggtataaga acggacatct aggccaatgg 241680
   aatagaatat agcactcaga aacaaaccct cacatatgag gtcagatgct tttccacaag 241740
   gcaaccaaga ctatttaatg gagaaaggat aatcttttca acaaataacg ccgggagaac 241800
   tggataccca acagcctaaa aaatgaaggt ggccccttat cttataccat atacataaat 241860
   taactcaaaa tgggtcaaaa tgtaaccaca gaagttaaaa ctataaaact tttagaagga 241920
   aacatggcgg aagcttcatg acattggatt tagtaataat tccttgaaaa ttacaccaaa 241980
   agcgcaagca ccgaaagaaa aaaaatgata aattagactt atcaaaatgc aaaacttttt 242040
   gcactgaatt acactgttaa gagagtgaaa atccgtagaa tatgagaaaa tagttgcaaa 242100
   taatatatct gataagggat taatacccag aatatagaaa aatctgtaac aactcagcaa 242160
   caataaaaag aaacagccca actcaaatac aagcaaaaga catagctaga tactgctcca 242220
   gagaagatat atagatggct aataagtgca ggaaaagaaa atcaacatag tctgagaaaa 242280
   tgcaaatcaa aaatcacagt gagatactaa gtcacaccca ttaggatggc tatgattaaa 242340
   ataatatttt taatggaaaa taacaagtgt ttcaaggatg cggagacatt ggcatcctca 242400
   tgcagtgctg atgggaatac caaatggtgc agctgctgtg gaaaacagtt tgttgattct 242460
   tcagaaggtt aaacgtggac ttaactctag caactccact actcagtaca cccaaaatag 242520
   ttcaaaacaa ggacttgaac agattattgt atatcaatgt tcacaggagc attattcacg 242580
   acagccaaaa ggtgcaacaa tccaaatgtc tgttgacaga tgaatgaatg gataaacaaa 242640
   atgtggtaga tattattcaa cttttaaaag gaatgaaatt cttatacatg ttacaacatg 242700
   gatgcacctt gaaaacacta tgcgaagtga aataaaccag ggacaaaaga acaagtcttg 242760
   tgtgatttca cttatatggg gaagctagat aggcaaagtc atagaaacag aaagtagatt 242820
   cgaggttacc aggggctgga gggaagggga attattgttt aatgggtaca aagtttctac 242880
   gtgagatgat ttaaaaaaag ttccagaaag gaatagtggt gatggttgta caacactgtg 242940
   gattgactga ataccaccaa attttatact tagaaatagt taaaatggta aattttatgt 243000
   atattttacc acagtataaa aagagtttaa aacaataagc atttattcaa atttaaaaga 243060
   aaaaaacata cagccccatc aaaagtgggc aaagaacatg aacagacact tctcaaagga 243120
   agacatttat gcggccaaca agcatatgaa ataaagctca acatcactat cattagagaa 243180
   atgcaaatca aaaccacaat gagataccat ctcacaccag tcagaatggt gattattaaa 243240
   aagtcaagaa acaacagatc ctggcaaggc tgtggagaaa taggaatgat tttacactgt 243300
   tggtagaaat gtagattagt tcaaccattg tggaagacag tgtggtgatt cctcaaggat 243360
   ctagaaccag aaataccatt tgacccagca atcccattac taggtatata cccaaaggaa 243420
   cattaatcat tctattataa agatacatgt atgtatatgt ttcttacagc actatctgca 243480
   atagcaaaga cacgggatca agccaaatac ccatcaatga tagactggat aaagaaagtg 243540
   cgatatgtat acaccatgga ataccacaca gccataaaaa ggaatgagat catgtccttt 243600
   gcagggacat ggatgaagct ggaagccatc atcctcagca aactaacaca ggaacacaaa 243660
   acgaaacacc gcatgttctc actcataagt gggagctgaa caatgagaaa acatggacac 243720
   agggagatag gccaggaaca acacacactg ggcctgtcat ggggggaggg aaagcatcag 243780
   gataagtagc taatgcatgc agggcttact acctaggtga gagattgata ggtgcagcaa 243840
   gccaccatgg cacatgttta cctatgtaat aaaactgcac gtcctccaca tgtatcctgg 243900
   aacttaaaat aaattttttt aaaaactgtt tattgttcct cacaattcag caagttggca 243960
   gtttggcctg tgcttgaccc atggttctag cctcatcagg gctcactcgc atcagcttct 244020
   gagttggcta gaggctggct ggcctagcat ggcctcagct ggaaagactg gtctctgttc 244080
   ctcgtgtctc tcatcattca ccaggctagt acagacttgt acacatggta accaggcagg 244140
   attcaaagct gcacagcctc tagaaacgta gacttgattg ggcacaacat catctctgct 244200
   gtattccatt ggccagagct aaacccaaag tcaagggaaa gggaaacaga ctccatctcc 244260
   tgatgggagg aggtgcggag tcacactcca aaggacatgc atacagacag aagaggagaa 244320
   ttggaaccac ttttgcatca acctactcga gtttggttct gacattgtta agaatgtgtc 244380
   aggacaccaa atggcatgtg acaggatgtg ctcatgtaag taacctagga tttgttcaca 244440
   taaagggtca gtgcttgttt taggaattta aaagtgaagg aaagcataca caaacctcag 244500
   ttcatgcata cagttttctc tcccagtagg taagcaccta tcagattgtt tttcaatcaa 244560
   gccagctgct ttgcactttg taggagctgt aactatctgg attagctgag acatggagag 244620
   agccttcaaa gcaacagaat tttgaagcta gaagggtgcc agggaaaccc tctcagtaca 244680
   gataaggacc atagccccag caagggaaag tgatttggtg aaggcttcat agttgccaca 244740
   gatggaactt gcactagaat ccagattgcc agatttataa tccagcactc tctctaggac 244800
   atggtgctga tttcattttc cctggaatat gtaaacccca gttcagccac caagggaaca 244860
   caatctattc tttgagacct tgcaactaag agcatgctct gcagccagca gtattagcct 244920
   cacctaagag cttgttagaa attcagaata ttgagcctta tgccagacct gctcccttgg 244980
   agtctgctgt ttaaggagat acctggggca tttatgtgcc aagtagggtt taatacgcac 245040
   tgcattagga cacgggtttt caacctcaaa tactaggaat tgcctgggag aggtttaaaa 245100
   ctaccactgc ctggtcttta ccccagagat tcttatttat taagtatttg ctcttgggtg 245160
   cagtctgggc taaaggattt ttaaaagctc ctcaagccac gacttctctg tagtcttttt 245220
   tttttttttt tttttttttt gagacggagt ctcgctctgt cgcccaggct ggagtgcagt 245280
   agtgctatct cagctcactg caagctccgc ctcctgggtt cacaccattc tcctgtctca 245340
   gcctcccgag tagctgggac tataggcgcc caccaccacg cccggctaat tttttgtatt 245400
   tttagtagag acggggtttc actgtgttag ccaggatggt cttgatctcc tgaccttgtg 245460
   atccgcccgc ctcggcctcc caaagtgctg ggattacagg cgtgagcctc cgtgcctggc 245520
   ctctgtagtc tatttctaag atgctgaaga cattggcagc tttttatata gttttctgtc 245580
   tgtgcagtgg ggtagtattt tcctaaagag taaacagcct tcagaagacc ctagttgatt 245640
   gacctgctcc agcatcctgg gtcagatgta agatcaaggg attttaaaat tgctgaggac 245700
   tacttaaata cattttcata agggaggaaa ttaaagacca gaagtggtca ttgagaggaa 245760
   ttttttaaaa atcaactttg tttcccaagt ggaatagcat taatgtacaa tgaaattcca 245820
   tgtaataaag gtgtggcaaa ttacatagtt caacagtgaa ggccaagaaa cacgtaaatt 245880
   atggaaggaa gaagagacag aagggaatgc aagtagacta caggccttac acatgaacaa 245940
   aatcaatagt taagctataa atttgatttt tttcaagcct ctggggagtc catttgaaac 246000
   agttacaaga tttctatcaa cagaaaaggg gagtgggaaa agcagaattt cctctaggaa 246060
   agccaagctt tctaactcta atttgtaaat aagtgtgtca ggaggccttt ctagggggac 246120
   acttaatgaa gtagcgaacg atgtcttcaa gaacattcct gggccaaaac acccctggat 246180
   tttatgtttc ttgttaaaga acccctggat tttatgtttt tgttaaatct gtggtcttta 246240
   tgtgaaaaca actgatagaa tacaattttg gaaacaggaa agataatgtt gtttgagtct 246300
   cttctgatgt gtgtgttttg ctttatctaa gatataacta agctttatct gagatataac 246360
   ttagagaaga ggagcttcaa tttggcaagg cagcttagag aggaaagaag tggatcatct 246420
   ctctttgttg gtacaagggt ttgtgctgtt ctgttagtat cattgaggga atcaatggga 246480
   ttattcagta aactcttttc agagtcctgc attctggact taaacttgcc ctcttggata 246540
   aacacatggg ataaatacgc tccatcattc agtcacagaa tgcattctag gagcataatg 246600
   tccagaggtg cccttggtgt tgtgaatcaa tcatttttcc ttttctgttt tgaattgctg 246660
   tttctttttc tattaaatga aagaatgacc ctattaatct gaagtttagg ggctagattt 246720
   tttttatttg atactatctc tctctctctc tatatatatg tatatgtgta tatatatgta 246780
   tatgtgtata tatatatgta tatgtgtgta tatgtacata tatgcacaca cacatctaaa 246840
   tacacctaaa tatacatata tctaaatgta tgtgtatatg tgtttatata tatcagtaaa 246900
   ttctctccgt aataaatata tgattttata tatacacaga gggaatggac tgattttaaa 246960
   atccttattg agttgaaatg gtttggatca tgaaaagggg accttaggag taaagactaa 247020
   atcaaaggca aatggttgtg gaccaaaatt agggctgtcg aagcagaagt tgattaaaaa 247080
   gatttattgg aagtcaaatg tgagaaatga cctgggaaaa cacaccagca aagttgggaa 247140
   tgatccaaag tctgttacaa gttggaatgc ttttatagga aagtatggga gaaaggagag 247200
   ggactcccat atcagagttg tcctttttca ttggagggta caatacaggg attattgtca 247260
   ttgtctacag attgcaacat acaggctaac acatctatgt gcaagacaat cagtgaaatt 247320
   tcctgattca gatacaaacc agcaaaactt cataattcag aaacaaatca gcatcctttt 247380
   caatgtcagt aggttactca ttaatcagtg tgtcaataag ataaggaact cagataattc 247440
   tttactcagg aacaggatgt tgccatgaat cacaagacct ccccaaggca gactaattta 247500
   gaagcctgca aatgtgacct gcaggttgtc ttggttgacc agtgctgtcc agacttcaat 247560
   gtatatttta attacctgga gattttgtta aaatgcagat tgtgattcag tagatctgga 247620
   tggggctgca attctgcatt cctaacaaac ttccaggtga tggtgatgct gctggcctag 247680
   ggaccaagct ttgagtggca ggaggagaag ttctagcaat atggttccca aacttggctg 247740
   cccattggaa tcccctgaag agcttcagaa attactgaac cctagaacga tgatcagaga 247800
   tgtaattggt ctgggaagag accctggcct ccaccctgat gattccaatg tgcatggctg 247860
   ggagcccctg caattgaagc tgccagatga agcggaatct ctcactgcct tagcctttcc 247920
   tacaggttat tagaaatcct caaggtgcat tcttaaacct tccattgggt ttcaaaggaa 247980
   caaaatattt taaatgtgaa cacatctctt acccctagat aaggaggggg agagagagag 248040
   agagagagaa agagagagag agagagagat ccttgggata aatgtctcat tttgcaaaaa 248100
   gaaaagacat gatttgattc tgtatatagc ttatctaaat gaaagaaatg ttgcagagtt 248160
   agcgtttctg cggtgcaaaa ggaaagcacc ttaaggaatc gccacactgt cttccacaat 248220
   ggttgaacag agttgaacaa tgagaacaaa tggagacagg gaggggaaca tcgcacaccg 248280
   gggcctgtcc ggggagtggg ggtcaagagg agggagagca ctgggacaaa tacctaatgc 248340
   atgtggggct taaaacctag atgtaacaaa cctgcacgtt ctgcaaatgt ataccagaac 248400
   ttaaagtaaa atttaaataa ataaataaat aaaaaggaaa gcaccataga cttgcaggtg 248460
   tctctgggta agccaagtta gtacatagag atgcatcagg ctccactggt gggattggta 248520
   aagagattga ggatgaaggg agcctaacct accaactctt tttttttctt ttgatacagg 248580
   gtctcactct gttgctcagg ctggagtaaa gtggtatgat tatacctgac tgcaaactcc 248640
   aactcctggg ctcaagggat cctccgacct cagcctcttg aatagctggg accacaggca 248700
   cttgccacca tgcccagcta tttttttttt ctttgtagaa aaaaaaaggg atctagctat 248760
   gttgcccagg ctggtctgga agtcctggcc tcaagcagtc ctcctacctc agccacacaa 248820
   agtgcttgga taacaggttt gagccaccgt gcccagccct gccaactcct aatagtatga 248880
   atagcatcat ttctttggca tttgctaaga gttactggaa tatttcagct acgggcttgt 248940
   atctctgatt gggtttaaga gatgggattt tcttcccctc tgaaccagcc acccacccac 249000
   agccacatcc cacaacacct gttctctctc ccttagactt tcagtctttg accttgctgc 249060
   attctcctta cttatcagca ctctccttta ataatctcct ttcagcttaa actgcatggt 249120
   tcatccacat tctagtgccc attgctacct acctacttgc atgacagctg cctggcaaac 249180
   acccattctg agtatatcca atgtcccacc tttgcagaag ctgcccatag agtgctgcat 249240
   atttgctgga caagtcaagc aaacttttgg tttggtgcaa ttccaatgta ttttgacaaa 249300
   ggaataattt atgtgtaata agatatacaa atcataaacg taaagtatgc caaattttta 249360
   caaatgtatg tattcttgtg accacaacca agatcaagat atacaacatt ttcatcaccc 249420
   agaaagttcc cttgggcccc atcccagtga gcacctttcc cctaggggta actagtactc 249480
   tgactttgat caccaaagag aaattagtaa tgaatatcaa ggaatgtttg gtaaagctga 249540
   ctaaggtaca tgtgcttgtt tgttacatgg atattacacg catagtggtg ggggttctgc 249600
   ttctagtgta cctatcactg aaatattgaa cattgtaccc aacaggtaat ttttcaaccc 249660
   tcattcctta tccaccatct cccatccctg cttttagagt tcttagagtc tattctctcc 249720
   atctttatgt ccgtgatgac cttaatattt aatggggtta gaagtttcaa acttttgaga 249780
   catatggaag acttggattg caacttttct cttctatttt ttgcataagg catactcttt 249840
   tcttggcatc taagaacagt caatttctga aatatgaatt acagtgcata gttacaatac 249900
   caactttata gtttaaaatg ttatttgact gcctactggt tttatttcca gattaacaag 249960
   tttcttaaca tttattatgc ctccaagaga taatttccac atttgtaaag attgtttgaa 250020
   atgtcaggta aaacaacaac aacaacagaa atttaaattt aggacatgca aaggtaaatt 250080
   tttcttggct aaatttaaaa taaatatgcc ttgtactata agagaatgca atacattgtg 250140
   gcccttagca ttgcatatta gcaatattta gagcataatt taatcagttg gttgatttgg 250200
   ggaggtagtt gcaattgata tgtagactac atgttttatt agttattaca tttttaaatg 250260
   tcagaatctt gagtaaatgc aatgtaacat ttcctttgtt atagagaaag aattttcaag 250320
   ttgctttaaa ggctaattat ttcaaagacc agtcaactta tttttcccca atttccataa 250380
   attctgagtt ttgaacatcc tttctttaaa aagctttgtt tctggaaata ttttatattc 250440
   tgccccccac agaatatgct tacatttctt tcattgtatc tcaaagggtt tggagcttgt 250500
   ctttctgggt tttgactatc ctttctactc ctgagagttg aagttgaggg tgctgacagg 250560
   gtgttgtggt tttcatattc tatagagagg cagcatttgt aacaggtagg tgtgagcctt 250620
   gctttcaaac ctcccacctg ctagctgagt gacactggga aaattccttg gcctctctga 250680
   gcctcacttt cttccttctt ataaaggggt aataatgcca taccttggag tcatattatg 250740
   attattatat attggaaaaa tacttctaat catcttggca cacagcaagt gtttaatcca 250800
   tggtagtgac tagaaactct tattaaacca atttacggac tgatggcagt cattttattc 250860
   gtcagttcac aaattcttta aagttcagat catatggagg acagggcatg agggaggaaa 250920
   tgatggattc ttgtttatgc ccacaacacc tactgttgcc taaggtgacc ttaccaccca 250980
   gcaagaactt ggccagtctg cttctcgaat tgctgttcca gaaaagctca tttgcgaaca 251040
   gtgttgggtt gtttatttgg gttgcttatt ctagtcaatt gacttggcca acaaactgca 251100
   tttcttcagg agtggggaga actgtgtttc atgtcctcct ctgtgacaga actgcagaca 251160
   gtaaacgaag tgatgcctgt ggtgccatat gactccctcc ctctaagaaa ccacattcag 251220
   cttttgggga ggcttatttc tttttttttt ttttttggca cagcccatga atcaaatact 251280
   agcagagttt tccctccaaa gatgagacct gtgtatctag atcagggttc agcaaacttt 251340
   ttctgtgaag ggcctgagag taagtatttt acaccttgca ggccatatag tctctcttgc 251400
   aactcaaccc taccattgtg gtgcacaaat ccagccatac tctcaccata tgtaaatatt 251460
   tacaaaaaca ggcagagggc cagatttggc ccatgagtca catggcttcc tgacttctgc 251520
   tctagatcag ttctgctcaa tacaagtaaa atgcaagcca cgtatttaat tttacatttc 251580
   ttagtagcac attaaaaaca attcttggcc agtcctggtg gctcatacct gtaatcccag 251640
   cacttcagga ggctgttgca tgagatcact tgaggccagg agtttgagac caccctgggc 251700
   aacataatga gacctcctct ctacattttt tttttaatta gccaggcatt ttggcacatg 251760
   cctgtattcc tagctaccca gaataacttg agcccaggag tttgaagcta tagtgagcta 251820
   tgattgcacc actatactcc agcctggaag acagagcaag accttgtctt caaaaaaaaa 251880
   aatttgtaat atattaaagc atcttgattt ttaaagatga aatgttttta agtcttagaa 251940
   tagaaagaga cagatgcctt acctttatga gtaacctaag tttgctgaga ccagcctagc 252000
   caacatggtg aaaccccatc tccactaaaa atacaaaact aactggtcat ggtggtgcat 252060
   atctgtaatc ccagctactt gggaggctga ggcatgagaa tcgcttgaac ccgggaggca 252120
   gaggttgcag tgaacagaga tcacaccatt gcactccagc ctgggcaaca aaggtgaaac 252180
   tccatctcaa aacaaacaaa caaaaaaaca gaaattggct ttttggattc catgtgatta 252240
   agattattac ataatttgtt aaagagggat tttctggtta gaatccctta ccactacctc 252300
   cactctgaaa tatgttgcct gaattatctt gttttccagg gatatgcaat gctcaaatgt 252360
   tcacccaatt agccccaacc actgttgtta ccaaataaaa ggtatatgta gagaactgaa 252420
   agaaaataga taaaatatct attggtaata gcgagtgaaa atatttattg aaaatatcta 252480
   atgatattac aaaatccaaa caagagttac cgtttcttcc tataaggatc taagtagatc 252540
   aaatctgttt gttgtgacta tgttacctga tgtttccagc tacctgcctt aataaaaaca 252600
   aggaagacag ttctttacca acagggtaag aatttagata gacagtttaa aagtacataa 252660
   gttctgagag aatctttgtt tatacttcag ttgtggaaat atttagctta ttaacctcct 252720
   gagctatatt tacctgaaag acaaaatgaa acacgtttga ggcacacaga agatactgct 252780
   tttgaaaaag agatgtgagg tcctattagg gcaaagctgt taattacaca cttcattatt 252840
   gtcaaggtta atacagtctg tgagggcaga ggcacttttt catctctttt cggggaacaa 252900
   ggggagacag ctgttttcac agacagatcc attatagctt atcaccagcc aacagcccat 252960
   ggaaggagca ctatgaaata tggccactgt cactgggagc ctgaatagat gctgagctgg 253020
   tccagggtac aatggaaaga caatgagcac catggcttca aactcaaata gattgggttg 253080
   cattgatttt taatgcctgg aggttccatg gcataaaatc caccaatcct cttgcagtta 253140
   ttgtattatt gatctctata aaagtgcaca attttgcttt aatgaacacc agagacggac 253200
   aataataatt agtggttctt ctgtttggaa gcctggttaa accgtgttgg aaaactctgc 253260
   atgtttggat tttcattatt actgctttgc cacttatgat tctataaagt gaaacctgta 253320
   tcagcaaaac aaatactgcc ttggttctga tttaacaggt cttcttttct tatacttttt 253380
   tgggtggggc actaatttgt ttttaacaaa catttacatt ttgcaacccc ctgcttatta 253440
   acactgagct aatagttttc attgaaacgg aagagtgtgt gctttattta aagtgtgata 253500
   catttatacc atcaacaaac attttcaaat gctaaccatg gaaatgaaat ccaagaaaag 253560
   actgtcttag gacatgcctt agtttctttg cccttttgtt tggttggttg gttggctttt 253620
   agatttttct ttcatttttt tactattggg tgattaaaat ttcttctttc taaatgtttg 253680
   taaccacatc atatcttttg tagtgttcgg attttgttca tcaataactc taaggcttct 253740
   agccttatag taagagcatg gtgctaaaga agcaggtgta ccaaaatggt gaaataaatg 253800
   gaagtcatcg ttttggttaa gctggagaaa tgtttcatct tcaaagggaa tgagaaaatg 253860
   aacatgtaat tcccaatgtg tatatatagt gtaaacgtac ttagcgctat gtatgtatat 253920
   atagaataca atacatatag tacatttaac actatattat agatagttaa taaaatgcat 253980
   atttaataca acttatatat atgtatgtat atacatataa tgctgactct tcttatacag 254040
   aaaagcatac ctcagtctaa cttttttgtt ataaaggtaa ccctgattgt agaagatcta 254100
   gaaaatgcat taaccccatt gtatacacag tgtaaccctc cgaccatcat cctcatcacc 254160
   tgagagcttt ttggaactct agcatcccag gccccaccca gacctactga atcacagtct 254220
   ccctttaaca agatacttgg gtgatttgtg tgcactttaa agtttgagaa gcactggact 254280
   gaaatataaa tcacccctag tcttcccacc ctgagatgac agccactatc aaaattttac 254340
   tgtatttcta tcaacttttc ttttctatac ctatctttcc atttaaatct tgaaagtcta 254400
   acttggttaa tttttctgac tcatacatta cgtttttaag aagaataact atagaatgct 254460
   gattgttaat ttgaaagtat tccgcttttt agatctcaga aggaatattt atgtaacaat 254520
   tgtaattaat ttgagataca tctgtttaaa ttcaagaaat atgtattttg tatctaaagt 254580
   tactaatagt actttaaagt ctcccacatc aattatccct tttttcccac catatgaagc 254640
   tagatttatt tcttctaggt gttgtttgta gcttcctttc ttcttcattt attatcaacc 254700
   taatttaaaa gtaaaattta ccactgcagc ccgccacccc gccctggcca atagttaagt 254760
   aactagcact tataagtact ttgtatacgt caggaatcat gccaagtttt ttacacatat 254820
   tactgtatta aataatcaca ttaactctgt gaggtaagga tctagtctca atgttttatg 254880
   gattaagaga cagtgactta aaaaggtttg ataacactta atattaccta tctcataagt 254940
   gctataagcc taagatacaa aataatgata ataaatacta catttttaaa caatgtttct 255000
   tatcaattgg cagtctttgt catttcttct aaatagcatt tagcttatag cagtattaat 255060
   atttttctcg ggatacctgg tgacaagtgc acagaaagag caggtcccca gttctgaggc 255120
   cacacattaa ttcacatgat gaaatagtga ccctctaatc tcctatagaa atgttaatga 255180
   acacacttgg gagacaaaaa atccctatga aattgtattg attccttttg tttattaatt 255240
   tcctaaatat aagatattta tagcacgatt aatgactgaa aaagctctat ttaataggcc 255300
   ttcattatgt tgttcttagt aatctaaaaa tattgatagg tagtataggc ctagacacat 255360
   agatactata gttctgtttt ttatccatct atagatattt taggataaat agatgcctct 255420
   ttttgacatg taaattttat tatttcagta tttctcaaaa atgaagtgtt agtttaaagt 255480
   ctacccaatc aactcattaa tcaaattgga agggttttag tgtaaaaata gggagattta 255540
   agggacctgt aacttccaaa ggaggtgttc aataatgctc ttatctcaaa ggaatcacac 255600
   agggaatgat tttttttcct tctaaattac ctgtgtccta gtggtgaatt tctaacacta 255660
   acagtctacc ccccatcaca gacatttgtt aatttgtgaa gatgaactaa taggtttttt 255720
   ggacataaat tccaaacaaa taattgggat accaatttga tatgagcaga gagcctttct 255780
   tctgtattca caatttccat cctgacccca aagatggtag catctgaagg catacagatc 255840
   acctctgagc ctcagttgtc cagtaaatga tgagctggtg attatctctt ttttctctct 255900
   ctctagttac atctattgct tcctcctaga tgtgcttcat agttcttttc ttccagaaac 255960
   tttccctaga cttttagctt gcttttctct gatcattttt taaataatct tcatgctttt 256020
   cttagccttc attgatgaat gtgtagaaat gttcttaatt catatcatat ttacaaattt 256080
   ccgtccactg aaggcagtgt tttcgttgag ggcattagga tgttttaaaa ctgttctcta 256140
   gcacctcctt ctatctgcag ctacaattct agggggaagg ggatcactaa ataccatcaa 256200
   gtgataatat ccctattaca aataagagaa caggtaatga aataaagaat gaaattaaat 256260
   cacatagcaa gtgtgcactg aaattgaaat tggaatctag aattatccat attccgtaca 256320
   ttcagcaact aatttctgag agtggagcgc acgatagaaa tatgcaccac aatgaccaat 256380
   ggccaatttg ctgggttgtg atgtcacaca ccaacgttgc attgcttact ggtaatattt 256440
   tgtttagcct aaaagagtct aaagcatacc atagaaagag acctagactg ggagaaagga 256500
   gacttgggtt caaatcccct gtttgttaaa tattgttggc aaagtcaact ccaggtttca 256560
   ggttttactc agtaaaaaga agaggtgacc tagactcttt ccaaaattaa gatgctggaa 256620
   gtgattccat ggtagcagac tgaatatttt gtgtattcag agatgggaag gcatgggggg 256680
   caggcaagaa agaataattg gaatattgtt taagatggat agaatttaaa gaaataatat 256740
   ttttctagga tatagagcta aaggtaacat tgaatatgaa aaaaatctaa tatagttaag 256800
   cattgtttat caagttcaga gaaagtctgg ataagataag atttttgcta aaggattatc 256860
   cgagtagatc cagtgattca gaagggccct ttaagacctt ggtgtgcctt acactcatta 256920
   cccatgtggc acatctgtca acacaaatgc ctgggattaa acacaccagg aagctctttt 256980
   ggaggaacca tgaattcctg atgacatttt gtttgagtaa cctgcctatt gttcataaag 257040
   agagggagta gaaaatgtgc aagagtataa tttctgtcca aaaggaaaaa cttttagttg 257100
   atacactgtg atacaaacaa actgtttctg ttttctttta aaaaacagtg caagacatta 257160
   ttgaatagga aaggaggcca gaagtttaaa tccatagaag gaagcagaac agatgtctaa 257220
   gagggcttga aggccaccct gaggaaatag agaagtggta aacaaggcca ggcgctgtgg 257280
   ctcacgcctg taatcccagc actttgggag gccaaggcgg gtggatcacg aggtcaggag 257340
   tttgagacca gcctgaccaa catggtgaaa ccctgtctct actaaaaata caaaaactag 257400
   ccaggcgtgg tggtgagtgc ctgtaatccc agctactggg gaggctgagg caggagaatt 257460
   acttgaaccc gggaggcgga ggttgtagtg agccaagatc atgccactac actccagctt 257520
   gggtgacaga gcaagactct ttctcaaaaa ataaaataaa ataaaataaa ataagtggta 257580
   aacagggaag atacaactga aatgaaatag gaagctgctg aaagagactt gatattttta 257640
   aggctgagtt atagctgttt catctttcct ctgccaaggc tgctcatctt ttactcttca 257700
   cagttattgt ttctcccaag ttaatctgtg gggtgaggat ggatgttgtt cctctgagga 257760
   aaggtcccag gttatataca tgattctttc tttctctaag ggaggctcca acagagcatc 257820
   ccctactttg gcaatcaatc catcaattta tgcaccaccc tcaacaatga ctacattcca 257880
   tataatgaat tccacactat ggaataaaaa aggaataggc aaaaaaaaga ctaggatatt 257940
   agtttatgaa cactgactat attatgctca gtgaaagaag tcaagctgaa aggctccaga 258000
   ctgtatgatt ccatccatat gatattctgg aatagacaaa acaataagga cagaaaacag 258060
   atcagtgata tcaagggttt ggggagaggg taatgtttga ctagaggaaa ctcaggagat 258120
   atttttgcag ctggtggcac taatttgtat ggcactctaa cagtgaatac atgatgcttt 258180
   gcacttgtca atacccatag agctttgcag cacaaagagt taattaatgt acgtagtttc 258240
   aaaaaacaac aaaaaataaa ccagaagatc agaggatcct gggatagaat gcagaatgtg 258300
   accaaagaat ccaacagcta tacatttata acataacctc attgaagggg gtgcaggaaa 258360
   aaagacacta acttaaggaa ctttggaaac agtgctttga atgcaaacta taagtgtaaa 258420
   ggaattttaa agaactggac ttaaacatgg tacttgagtt gacaaagttg tttctcatgg 258480
   tagagtgagt taatctgaaa ctgctgaaca gttatactag cactgaatag gcaaatgact 258540
   ggcagatggt ggggcccatg ttttttactg ttgcagagga agttacaggt aaggaaatgg 258600
   caagaatgaa ttatgtggta cttgattaga ttcagagaca ttaatgtgga tttatgttta 258660
   ggttaatata gatacagatg gatggatata attataggtg tgtgagtgtg ttcatgagtt 258720
   agtctacaaa catattattt cggagctctg tttgctgagg cctggcaatg acacccagta 258780
   gcaatgagta ttcctggaac caaatcttaa tttctaatac tattagcgtt ccttggagaa 258840
   atgcctgatt tggggggaag ggcagggaaa ataaaagata aacctggagc ttcttgtagg 258900
   gacagaaagt aaggaagtgc tcaaaaaata aaaggaagag ggatcatcaa agggacacag 258960
   cagccagcct gagagaactc ccagtgacaa aagctggaac catttgagca acaaggcaaa 259020
   taacagagtg ttggcttata acccaaagta taaaataaat aaccataagt ccatccatcc 259080
   ttatatataa ataaatgatt gaatgagtag gtaaatggga gcaaataagc aagtctcctg 259140
   tgcagaagaa ttccaactac tttatataga taccctaccc ccaccacaca cacacaccca 259200
   agaagataaa gcttaaatcc ctgacccttg agtagacgct acacttactg acttgttccc 259260
   aaagaataca atatacagaa agctagggaa ggggagtgtg actgtgtgta taagaaacct 259320
   ggaaaatatg acctcaccca ggtagtcagg gctaacatca tcattcctaa gtcgtgtcga 259380
   cgatatgtac cctaaacatt atgcgatgaa atgtgacttg actctctgat cttcctcctc 259440
   aaaccaaata acctctgttt aaacatggga aaaacaccag aaaaccccaa tggaggaaca 259500
   ttctccaaaa tacctcacca gtactcctca aaactgtcaa ggtcatcaaa aaataaggaa 259560
   aatctgagaa actgtcacag atcagaggaa actcggaaga tataacaaat aaatgtaatg 259620
   aactatcctg gatgggactc tagaacccta ggagaaaatt aatgaaatct ataggaacat 259680
   ggagtttcct taatagcaat gcaagatatt aactcaaggg gaaactggat gagaagtcta 259740
   tgagaactct gtattctctt tgcaacttct ctgttaatct aaaactattc tagaataaaa 259800
   attttatttg aaagaaaagg gtatagtaca aaactattag tccaggactc ttggaaagaa 259860
   aaaaaataaa ccctttgttt caccatggat agatctataa aacatcagct ccaggacaac 259920
   tttgacttct tcctccagtt tgaaatcttc tattgtacca tccaagatag ttttgaactc 259980
   cttgctactt gctcaaaagt atttttctcc taaaatcctg gaagtctgaa gtcacaacac 260040
   tttgttcttt gttcttttat ccacccccat catatttgcc tgcaaatcct gtcattgtca 260100
   aggcacggct gacgggcttt gtaagtggtt tgcgatgtct ttcagttcct ggtattcttg 260160
   tgttatttag gttcattaaa gggaacaatt tgtcactaag atccaggtgc aaccatcatt 260220
   actaagccat ccaaaattca gcggtgatgt tacacagaag tgtggccata acaaacaagc 260280
   agtatagtat aagtatatat acttaataag aatatcacat ttgatcataa atcacacaaa 260340
   taatcttcag tctgtttcct aattacagtt tcaggaagtg cctgcctcat ccataaaatt 260400
   tcataacctc agttattatg tcttccatat aagggcaaaa aatgtacacc catttctagt 260460
   tatgttaata gttgggtgat aatgccttct ggttttgaaa atcatctgaa taatgaatgt 260520
   aaaacagtaa taaatattat tttttattac caaacagtta tagagtgtta aattataatt 260580
   atcagctcat cattaggtgg ttagggctta ttatttaggt agaagaagta gggggaaggg 260640
   atttgcctgt aaactggtaa ataaaacagt tatattctat gtagtctcct acatggcttt 260700
   ttgatggtta tttgatgtct ctgttgagat aggtaagaag aaaaatgcaa tttgagattg 260760
   tttactattt gatttatttc accactgaag tcaatcaaca cattttcttt atcattttgt 260820
   gttaagtgtt cccggggtct tttttcttct gtagtttaat ttggatggag tgggggtggg 260880
   atgccaccat caatttaaat ccatgaaata ttttctttaa acaatattcc ctctattatt 260940
   cccagacaat cgttagagga aacaaaccct gcaaggaaac ctccatcaac ggcctgctag 261000
   aggattttga caacatctcg gtgactcgct ccaactccct aaggaaagaa agcccaccca 261060
   ccccagatca gggagcctcc agccacggtc caggccacgc ggaagaaaat ggcttcatca 261120
   ccttctccca gtattccagc gaatccgata ctactgctga ctacacgacc gaaaagtaca 261180
   gggagaagag tctctatgga gatgatctgg atccgtatta tagaggcagc cacgcagcca 261240
   agcaaaatgg gcacgtaatg aaaatgaagc acggggaggc ctactattct gaggtgaagc 261300
   ctttgaaatc cgattttgcc agattttctg ccgattatca ctcacatttg gactcactga 261360
   gcaaaccaag tgaatacagt gacctcaagt gggagtatca gagagcctcg agtagctccc 261420
   ctctggatta ttcattccaa ttcacacctt ctagaactgc agggaccagc gggtgctcca 261480
   aggagagcct ggcgtacagt gaaagtgaat ggggacccag cctggatgac tatgacagga 261540
   ggccaaagtc ttcgtacctg aatcagacaa gccctcagcc caccatgcgg cagaggtcca 261600
   ggtcaggctc gggactccag gaaccgatga tgccatttgg agcaagtgca tttaaaaccc 261660
   atccccaagg acactcctac aactcctaca cctaccctcg cttgtccgag cccacaatgt 261720
   gcattccaaa ggtaacgttt gctacctcct ttccctcacg tgtgcaaaaa cctccacagg 261780
   ggtggtgctg gcacgacctt tttatacgat tggattgtaa agtacttgtt tgccttttaa 261840
   gggaaaatca atatcaaaac ccaccctatc tccacttgtg aggccccagg gcagaggttt 261900
   aggataaggc atatatattc attaaaataa aaatctatct tttgggccct cctacgtggt 261960
   ttctcattaa aaggcgaata aacttattga tttgaaagat agaattctga tatatgagtg 262020
   ataaatatga tgtgactgta tttgactaag ttccctgtta aggagtggat cagtcatata 262080
   caatcataga gcattagatc gagccatcca gtggttccta catcctggca aagtttgcat 262140
   agacctgcta aaaaggaaag aaactcagta taacatggta atttttaaat aaagccaatt 262200
   ttgttcaact caggggatgg cccttcatat taagagtatg tactttctac tatatttgat 262260
   ttgaaatatt ggtttttata tgaaatttgg gggtgacaga tggttgctgg accgctggac 262320
   catttttgtc cccttttcac tttgttttgt agtaattcct cttttaaatt ttgtttaaat 262380
   gttagcagct ccaagtttgc tttgaaattt gactggtcta tgatatccta aagtttggga 262440
   atcactgccg gagaagttac tgaaactaaa agcacaggca aaggtctatg ggtccgaagg 262500
   tctaaagtct tggaaaatac ctttgagaat gtgcaagtga tttatttttc cctgtcattt 262560
   agaaatgttt ttgctacctt acctgtccag caaaagaaca tggaccgtag ggtgtggaat 262620
   gtggattaca tagaggtctt gaagcaggtg agagggacat gacttctcac agggcagtca 262680
   tgtactttat ttataccaaa ctcttgacag tttggtgagc agcacagaaa actctggaac 262740
   ccaagcctgc ctgcattcac ctgatttatt gattatagat agtcgatatt tttcctttgc 262800
   ttcttatttc tcattaacct catccaagat gttgaagaga agccatttaa gtgtattgtt 262860
   ggagttactt cagccattct ttagaagaaa caaaaaagct gtcaccttag ttcccaggat 262920
   ataaaattaa aaacaaatct tgataaatag aggcctgaga tatgttcctc cagttaaaat 262980
   tcacatagtg acagtgtttt ttttttatct cgtggtatta atgtacttga acacccacac 263040
   tcaggatatc atgactgctt tctcttcctg tgtgcttgcc agggtgactg ttggaaagag 263100
   gtctaattaa ccacaagcaa aatctacttg aactcatacg catgtctcat gatacatgag 263160
   atagtttatt ccatttcttg tgcatatcac ttgtgcattt ctgtgagtga tatgaaataa 263220
   ataagttaac atgttctata gctgaattga gttacatcct gaggctttga atatcagtgg 263280
   ttattttttt atctaagatt ttgtgtgcgt gtggcttatt tgcatatttg tgttagggag 263340
   ttgtttgggg tcatagttac agagaaaaca ttgcccagat gatgctgcat tttggtcctg 263400
   gagccttatt tggctcttcc tggatgttgg aaatgaatat tgggtgggct acacatgtct 263460
   atagtccaaa tagttttaga ggtcaagagt tttagaactc ttaattctgg agtttcagtc 263520
   ttctctcttt tttattttta tttttttaaa aaggatgaac tggtatccct gagtccacat 263580
   tttcacatag catgggcttt tgggatttaa tgtcaccacc agcaatccct gcacactcat 263640
   ttcctagaag gggaaactga ggcccagaga gccacatgac acacttacag tttcataatt 263700
   agttggttgc aaaacaagga caagaactca agtctggtgt ttctccctgc tcaatatttt 263760
   cctattattt gggcgtatgt tatctcgggg actcaactgc atttgccaag aaaatccatg 263820
   agaagtccca gtggccacca gaactttcgt ctaagtgaca gaagaacgag ggtgggaggg 263880
   gtgattcact ttaattccct acaggctccc tctattctgc agacttccaa atataagaca 263940
   aagcagacct gccctgaaat ctggctctgc cagcccagaa acaatgcaca aactattgtg 264000
   gctcaccacc accaacaaca acaacaaaaa cagcataatc actcatggga acttctacca 264060
   agggtgttca gcccttacga tcagaaatac aaagaagcaa gctgctgctc catgtttcac 264120
   aggaaactcg tgagagattc atgagcaggt ttttgttttg tgggaggata agaggtggga 264180
   ggcaggttag taattaaaac gttcacttga ttaaaaatac aacatttctc tgaaaagacg 264240
   gaggtgcctg tgtctttaga acggtactct tagagtgtac caatcacatt tggatttcca 264300
   gctgagggaa gaaatagcgg tcacgactca gatgagtcgg ccttgcttgc agagactcct 264360
   aaaggaggct gggtcatgtt ttcatctcca gtaataaaag gaagcacaaa acttgtgtag 264420
   gtctccagat gaagcagaaa tgaggagggt catgtgtgtg tacacatgtg tgtgtgtaca 264480
   cacgtgtgtg tgtttgacac agttttacat tttgtcctaa agaaaaaaaa agattaatgt 264540
   taacccacag aaaaaaaaaa tccttcaatg ttatctacct acttaatttc cctgctagca 264600
   ggacaggaat caagaatgtt atgagtttgt tgatgttggc ctcctctccc tacatttaag 264660
   tgtattttgc tggaactaag cagatgaaac cctggggttg ggagggtggg aagcaaagcc 264720
   aaacaaaaac catatagcac catacccacc acaaaattat gaaaactgcc agatgtggag 264780
   agagaatgct ttccaccctc gcctgcagac aaatctgcct tccacggaag gaagctagct 264840
   ggagaaattg ccgccatgta tttgactttg ctgattcctg tgctctccag agaccaggca 264900
   gccacctact tgtgtctttg tattggccca gcacatccgt tccctactga gaagccccca 264960
   gcataacaat cccaccagtt cattaagcat ttgctgtgta ccagatacat gccatgtttt 265020
   gttgagcctc acagatctgt aaggcaagaa ttcttgaccc ctttgtacag atgaggaaat 265080
   gggttttaag aggcttagta tgctcacagc agagctggga tccaactctt tgggcttaag 265140
   ccttttacca gacattgcat gcatttccag tagcacaggg gcagcttctg tgggtgcaaa 265200
   gcctgcaacc tgcctgatta taagctcctt ttaatacctg aagcaatgaa gttgcattag 265260
   cagttaatta gcaattaatt tcacaactag gttgcatcat cccctttcac tctgtagcag 265320
   ggtccaccac cttgacacta tgaacatttt ggacctagta attcttcatt gtgggaggct 265380
   gtcctgtgca tcatagggtg tttagcagca tccatggccg ttcccctcta gacgtccata 265440
   gcatccctcc ctcactccta acaaccaaaa tatacccaga cactgccaaa tgtcctgcag 265500
   gcagaagagg gcaaacttgg cccagttaag gacaactgtt caatagctat catttatcaa 265560
   gcacgttttg gcagttaaag atatagattt tggagataaa ctgcctgggt ttgagtcttg 265620
   acattgccac ttattaactg ggtgaacaag ttgcttaact gtgcctccac ttctttatgt 265680
   acaaaataag gataaagaca gcccgtgtct cattatatag tatgagggtt aagtaagtta 265740
   ataaattaca gcagcttaat tactaatatg agtaatctac acatcagtct acatgtggag 265800
   catgtagatg aatggtggca gtgaggtgag ggttactgtg ttccaagcac tgtgctaagc 265860
   attttgcaaa catgactcaa cttaatcttt gcaattccag aagaattcag gatcacctct 265920
   gctctacagt tgaggaagct gagactccaa atctttatgg acttccccaa gtttataaag 265980
   gcaaataact gagtcaggat tccaaactag gtcccactga ctccaaagtc tatgtgttcc 266040
   agaatattct catttccgta gaagaaaatc caggggcaga aaggtggaca tgaagtctcc 266100
   aggcccagac acaaatccta gaggtagcct caccttgctg gtgaagaatg ggtggtgact 266160
   gacaggtgga tttgctgtgc ccatggaatg ctgtttattc ttcctgttct tccatcatgt 266220
   aggatggaat ccttgagaag ttccttaatg tcacccagag tgtaaaccgt gggtaaaagc 266280
   tggaactatg ggctgtaaag gaagtgttag gtgcagaaat ccctaaggtg ctcatatgca 266340
   tttatttgac cttaagtggt agtcttgttt gattttttaa aagataatcc agattttctc 266400
   acccattaca gaaaaatgca agttctataa tcagattccc tggattcaag cgcttagtag 266460
   ctgtatgatc ttctctgtgc atcagtttca tcatctctaa agtgtttcag tacctacttt 266520
   gaactgggca tggtggcaca caactgtagc cctagctact ggggaggcca agacaatcct 266580
   ttgagcccag gagttcaagg ttgcagtgag ttataattac actactacac tccagcctag 266640
   gcaacagagt gagaccctgt ctaaaaaaac aaaacaaaac aaaaaggatg ggaagaaaag 266700
   gatggtgtga tttcccggca cttccttctg gggtcaacag ttcaatgtgt tttcactgct 266760
   tagctatcac tttgctttcc ctggaaggaa aaagcacttg aaccagtggg cccagtcttc 266820
   ccgcatcaat aatttactga ttcactgttc tttgtggtac ttgtcatgcc cagattcaac 266880
   catggctcca acaacagaac agcaacttct tttctactga ggatccctgc agaactcggt 266940
   tcagagatcc attcagggtt taatgggtta tggaatgaca gtgctagtga aaactgatgg 267000
   gctgcactaa aaatctgagg gggaaagaga gaagcacttg atcacttaat ataacaatgg 267060
   atatatttag atttgttttc tttacctaca gggtcttcat taaggctcat aaaacatcca 267120
   aggactgact gagtgcacta tctctaacgt ctgtcccttc tgatggagac tttaggtgtc 267180
   ctttgcatat gaatggacac ttcttcccat gaatgctctt gtgattcaaa ataaattgta 267240
   ttttggtcat tatgaatggg ttttcccatt accaaattaa aaacaaaatg aatttaacct 267300
   ggcattgtcc gttcctagct ctctcagagg ccctgacata tttgaacaat attctgaggc 267360
   tgcaaaccag tccccaaaga cgaagagtcc ctccacatca ctgcaggcga tgacggatcg 267420
   caggtcagac caatctgctc tgtctcactg ctcttgaata gaagacacat tctttgagtt 267480
   tgaagcagca actgtggggg gttaattgga tatgccaatt cagaggtgga ggaagagcct 267540
   ggaatacccg tatgcctgac cctgcacatc tcagatttat tcattgcttc atatagctag 267600
   gctttgctag tttgtagggt catgagccac ttgtccaact ctcagtgtgc atttgcaggg 267660
   cacaaccaga gagacagacg agttataggg ttcaatgagt cccattcccc taattgccag 267720
   tctctatcct gtccctacag agaatgcaag ttaatgattc tctccagaat tcttacaagc 267780
   agtcgttttc ccttcccaaa gccaagcctg gtcctgttaa attttccttt gagtcaagtg 267840
   aggacaattt aagatgtcct ttccatgctc agataataga ggtactgtga ttcactttta 267900
   tcttgcattt gatttatgga gagtaagcag cacccagcct ggcgttttca ttttcgagat 267960
   gagcagtctt tgttttattg tgctctgaaa aggtgtattg gagcatcaca ttcaggcact 268020
   ggaaccagct gccgcccact ccctgtaact cgccatcctt cacggaacat tccgagtcct 268080
   cattctgtgg gtacagaccc acactgtggc tgtgtccaca caccccagtc gagcacgctc 268140
   cagccacggc ccccctgccc tcagaacaga aggccacagt gccaaataga tgctcctgca 268200
   gtactgtttt ttccttctct tctttctctc accctcccct aaccaagctt tcttcccccc 268260
   aagacttggc ataaaatggt caaattatgt tctactgcag aattcaattt cacagttcag 268320
   ttaggtcctt gattacactg ccaaattcag attaatgtgc atttaactaa catggatcag 268380
   gggaattctg gctgacaacc agctgtaatc ggagcaattg atagctgtgt ggagttttat 268440
   gccccgtcca ccccagcgta gctctggtga aattgattga ttaagtgaat tcatagctat 268500
   aattatttat aattttgata catcacaagc ctgtgtctga ccctatcttt agagatgttg 268560
   agctcattgt agaaaggtag tccggctgag ttgttaactg gggattattt attcccttat 268620
   ataatatatt cttttttgct ctttttcttt tttgatcatg tttgaataga gacatgacaa 268680
   agcacatttt ttaaagaatt aatttatatg gtatacaaaa aagtgtcagg aagctaaaaa 268740
   acaagtgtat gttattagcc atttcaaaaa aaaaaaagag gaaatcacaa tcatgactgc 268800
   ttgttcattt tgtcaatatt tctaaagatt cctgtagaga gaattccttc ttaaaatgta 268860
   tccaattttc aatgacaatt aatgacctgg taaccctgca gctaagcagt ttagcagcct 268920
   gggtgataat ttgcaagaca attgacctgg tcttctctca tgacatcatg ccatatggaa 268980
   atccccctga aaactttgca gaccatggtc tcctttgtaa ggagttactc gttcacaaaa 269040
   tcacagcact gttctctttg gagggatggg aagaatgcct ttgttgtttt ttctcttaca 269100
   ttgcagtttc ccacccttgc tctgcactcc gtgcccttgc tgtaaggtct gcaagggtaa 269160
   actatgtaca taaatctttt ggaaatggtt tggatcatgg gtgatgagag atatgctgga 269220
   tttgcttatt aaatatcatt cctgtcacta gtcccattcc actgcattgt gttttcacta 269280
   aataatcaga tcccaaatcc ctttcacaat accaaagata ggtctgcttt tatgaacttc 269340
   aagcctactc attttttttc tagaatgtat ttgtaaatca ggtcatagaa aagattgcac 269400
   agtttaagct ggactgcttt gcacatcttt ctcacatgca tgaacatgtg tgtgtaaaac 269460
   agtaggaatg gacaaattca tcatttatgt tttctgcctt ccaaatatag gctatcatga 269520
   gaaagacaac ttagaatgga acacttacat gactatgaaa gtccaagagc tatgtgagga 269580
   ggtctcatcg cagaagaaag tttgcacttt cttccttgaa agaacttaca gaagaaagcc 269640
   tgagtgatgt tgtaacaatg cagagttctg ctttgaccaa aacaaaaagg aaaatgatga 269700
   atgatgtaca tagataatca actcgttttg ctgtgatgac tactcttttt tttagctcat 269760
   ttctctaagc caaaactgct caaaacaaac aagccaaaaa aaaaaaaaaa acccttaatt 269820
   tgtctttcac cccctctcct agacattatc atccatcatg ttctggtcca attcttgcat 269880
   tttgccccag gctgcccaaa catctgggcc cccagtgaaa caccctgacc aggctttatc 269940
   tctgtggtta tcatcattgc ccactctgcc cgcccctgtt aagaccacat cccccccccc 270000
   catcattcat gcctatctct gtgaattttt caaggaccca agtactgaag cgactctgtc 270060
   tggatttgaa tcccatttct cccttctttt agctttggga tctttgcatg acctacttaa 270120
   acccactgtg cctcagtctt cttatctata aaatggagga attacagagc ttatttaatt 270180
   gtccagcttg aggtttacgt gcaagaatat acacagtgtg cttagaacag tccctggcat 270240
   gtagaaagca ctcaataagt atcaaacatc attattctta taaatcaatg aatgttggaa 270300
   atgccaaggc aggcaaggga gccggcctgt gggccagtgt gctgggcgcc ctcccactgg 270360
   gagtgaagga ctgaccccgg cagaagaact aggttatgtc gtccccttgg aagtatggag 270420
   ctatttgccc tgcattgctt gggggcagtt tcgtctcctt gtgcctctat tttatcaacc 270480
   acaaaatggg attcagttgc cccctgcttg attcccagaa taaaaagaac aagacctgtt 270540
   gaaagtgtgt gggctggtga tacgcaccat catggaaccc cgcatgtggg ttaagatcca 270600
   acagaaggca atggttcagc accttggtgg ccccaaatgg ggtcacccat gcatttctcc 270660
   ttcacagagt cagctatttg tcctctgagg gaagactaca tgtcccctca cttgaagtga 270720
   acagttcatt ctctgatgag tatgccttga gtttcattca aaacattgtc tcttgtgata 270780
   ctagttgttc ctcaaccatt gcttaagagt ctgtacctct taccagcagc cgtgaaagac 270840
   aacagcacct ttttgtctca tggcctattt tgaagatggt tagctttcag caaagagctc 270900
   tgcattccat ctttgcctgg aaattgtccc cttggtgcag gcaggcttct tctttatgtc 270960
   atatgccagc tgttaagggt ggattctgat aatgctgctg acaggtgata aggaagaaaa 271020
   gtggcctaaa ttagtgcaaa gcggtgtttg tgattcctgc aggatgcagc tttcccattt 271080
   ttaatatatc ttcagagatc agttatacat gcttgtgggg aatgaattca atttcgttga 271140
   aagtacagga gccagtcctc caaggaagac acagggtgat aatggcaagt gatggctccc 271200
   aggacacact ttcagaaata aaagccaaaa cactatttcc cttcacaccc tggccttgct 271260
   gactagccta gacttgacta atctggagct tattgaagga agaatccagt tgtttaactt 271320
   acactgacac ttctctctcc ccattccatc gtagtttaac ttacactgac acttctctct 271380
   ccccattcca tcatacacac acactcacac acacacgttt tccttgtgta gcacaaatac 271440
   agaggctttg aaaagaatca gcttggtttt gaatatttta actatacatt gttcaatata 271500
   ttaatgttaa ctacatcttg tcttattgct ttgagtatta atatatagga atatatatac 271560
   cacaggggca ttgtatgtct tcaataaatg ctagctagtt tgataatatt gcagttacta 271620
   tttattttat tttgcattct cattactgtg ctaagcattt tatataataa taacaccaag 271680
   tagaacttac tggccagtca ttgtttgaat actttgatat gtatttgatc ctttagagtt 271740
   cagtgaccaa aggaagtaag tattattatt gttccatttt acggataagg aaacagactc 271800
   acagaggctg atttgactaa aattatcaga gtcagaagga aactaagact cagagaggtt 271860
   gacttgacta aaattatcag tcagaattca ttcctatgcc cgtcaaaagc atgtgttctc 271920
   tctgcaacca gggcaggagg gcagcttagc agctaccctg gttcctgctg gttgatgcca 271980
   aggctgtctg agtggtgaat tcacctagta aagtgtagtg tttttgaagc tggaagggct 272040
   gcattgctga atgttcagca ggccaggcac tgtcattgta gaaccaatgg gcagtgttgg 272100
   gggactgaga atggtgctat ggtagcaatt ttaaagagcc cactgtgtgc agttattgct 272160
   ccatgatgtg atagaatgac taccttctta tgcatcgtgg tggggcagga gcttgaaaca 272220
   cctgcttgcc tctaccatgt caccagatcc aatgagcaac atgccccatc ccacttctca 272280
   gtggcatttg acaggattca ttagcccttc cttcttgaaa tactttcctc ccttggctcc 272340
   aggacacctt gcttccttag tttcctttct acagcacttg tcactccttc tctgctccct 272400
   tttgcaagat ccctctgctc tttctggtat ctagacgttg gggtcctcag accatctctt 272460
   acacattcag tatcatggct tttaatatga actaagcacc gatgactctc agattcatgt 272520
   ctttagcttt aactcttccc tggacttggg gcatgcctaa gtctcctaag tgacacctcc 272580
   acatggatgt ctaatcactc tcccaaactt tacatggcta aaagaactct taattttcca 272640
   ccatgtatta gctatcaata ttgctgcata acaaattatc ccaaaacttc atggcttaaa 272700
   acaaactgcc tcatgaagca aaaaatagtg ctgttcaact aatttcccct aaactttaaa 272760
   taacaaacat ttactatttc acagtttctg taggtcacaa attgagaggg cttagctgat 272820
   tgattggttc tgtcttggaa tctctcatga ggttgcagtc aaggtttcaa acaggaacac 272880
   agtcatctga aggatcaact ggacctggag gatccacctc cagaatggct cacatggctg 272940
   tttgccagaa ggcctcaatt tctagctagc tcttagcagg agtcctcagt ttcttgctgc 273000
   atggacctct ctattttatt attattatta tttttattat ttctgagaca aggtttcact 273060
   ctgtcatcca ggctggagtg cagtggtgca atcatagctc actgcagcct tgagctcctg 273120
   ggctcaagtg atccttcagc tttagcctcc caaagctctg gcattacagg catgagccac 273180
   tgcacccaac ccttccaaag gattcttgag tgtcctcatg acatggcagc tggtgaaagc 273240
   caaaataaat gatgtaagaa agcaaaggaa cttgccatca tctctgcctt tttctttgat 273300
   cacacagatt gatcccaata cagtgtagaa ggggactaca taggaatatg aatgtcagga 273360
   ggcacaggtc attgagagca atttggaagc tgattatcac accccacaag ccctgttcct 273420
   gcccctggat tcctcatctc attaaatggc ctgttgctta ggccaaaatc ctgatagcca 273480
   tctttgattc ctgtctttca agatctcacc caatccatca gcaaatcctg tcagctccat 273540
   cttcaaatcc tgccctagat ttcaccattt ctcaccccat ctacaataac caccctaatc 273600
   tcagcccaca ttaactgtga taagctgctg gcatccctgt ttccactctt gccaccaaag 273660
   tctctcctcc agcagccaga gagtcagact gtgtcattcc tgtctgctta gcaaccccca 273720
   gtgcttccca cctcactcgg agcaaaagcc aacgtctttg ccaaggtctt catgatctgt 273780
   cccccggcag cttctcggcc catcttcctt agtggacaca ctccactata gctgagcgtc 273840
   tgctcttcct cacaaactaa gcctggatct gcctcagaat cctgcatgtg ccactcccac 273900
   tgcccaaacc acccctccta ggaaagggat cttgcttttt ccctcactca gccaggcttc 273960
   tgctccaacg gaaccccctc agagagtcct tctgtgacta ccctatccaa gatggcatct 274020
   cattactttc aaccccctca ctctgcttta tttttcttaa tattattcat tactactgaa 274080
   acacacactg tatatttact ggttttcttt attattatct atttccttct ctggagcata 274140
   aaccccagga gggcagagat atcattcatt ttgactgttg gtggaccctc cccctcacac 274200
   ctaaaacagt gcatagcaca tcatagttgc tcaaatatta atttttgaaa taaatgaatg 274260
   aatgtgctgc atgggaacaa tcaattcatc tagaatagtg ccatccaata gccctttctg 274320
   caatgaggaa aatgttttat atgtgtactg tgtgatgtgg taactactga gttcttaaaa 274380
   tgtgactagt gtgattgaga aactgaattt ttaattttta atctattttt gtttatttta 274440
   aataggcacg tgtagcttgt agctactata ttagaataga tctaggtcct tggggtggat 274500
   ggtaggggag tgggataagt taatgccatt ccatttacac ccagtagagt gatagagact 274560
   ttctggggca ctgcattaag aaagagcctc aagtccaatc aacagatctt ttctgagtac 274620
   cttctatgta tccagcactg tgttgtcatc gagatcttcc aaaacagagt gcattgatgg 274680
   attagcactt tctgccctgg gctaggagca taccacatga caagatgtgg tatctttacc 274740
   atcctggcct cagcaattcc aggagtggcc agagctcctt cctgattctt tccaggaagt 274800
   gactgctgga cctacatatt gtatcatata ccagtaatcc cctcactggc ttccccattc 274860
   tcctccaata aatcccctca tcccactctt ccaagtcaga gtggatgttc tttgctggga 274920
   tctctttcgg gtctagcatc attttcattt cacacatacc cctgcaaagt ctgatcactc 274980
   tcaattatat ttgtacttat ttggtgagaa cattgcacat gttcaccctg tggttcacac 275040
   cacttagcac agggagacgt attttcccct gtacctacat ctgctgtgtt atacctgtgt 275100
   tatacatata tgtccatacc accctctagt atgtgaattc agaagcatgg gaaaaggcct 275160
   cctacccctg aaatgtctcc caaaaatgct agctgaaaac taatgtcctt taatgaggga 275220
   tggggttttt cattactgaa caagaaaata gggcttaaca agcaagtaca ccttagatat 275280
   gacacacgtg aagtaacctc ctgctgtggt gcacgtgcac cacagaaggc ccttccctag 275340
   gtgtggccgg tcctgttggt gccccagcca gatcctctca tctcccactg gtgatcctca 275400
   ttctgccagc tgtcagtcag ctcactcacc actcggctgt ggcattcaga tccagcatgt 275460
   gaatattctc tatccatgtc tgtcttccca ggtggattac gatcgagcac agatggtcct 275520
   cagccctcca ctgtcagggt ctgacaccta ccccaggggc cctgccaaac tacctcaaag 275580
   tcaaagcaaa tcgggctatt cctcaagcag tcaccagtac ccgtctgggt accacaaagc 275640
   caccttgtac catcacccct ccctgcagag cagttcgcag tacatctcca cggcttccta 275700
   cctgagctcc ctcagcctct catccagcac ctacccgccg cccagctggg gctcctcctc 275760
   cgaccagcag ccctccaggg tgtcccatga acagtttcgg gcggccctgc agctggtggt 275820
   cagcccagga gaccccaggg aatacttggc caactttatc aaaatcgggg aaggctcaac 275880
   cggcatcgta tgcatcgcca ccgagaaaca cacagggaaa caagttgcag tgaagaaaat 275940
   ggacctccgg aagcaacaga gacgagaact gcttttcaat gaggtaaaga gtgtgtttgg 276000
   gtcatccttt ctctcctttg taacatttat ttcccatttc aaaagttata cacatgtaca 276060
   ttttaggaca ttagaaaagc actgaaaata taaagacaaa aatagcgtcc cctgtaatat 276120
   ttccttctag tcttttttct atgcacatat atggctgtta catctataaa gtacttcatg 276180
   tgtatcagcc actgtactaa gcattaatct agcttgatcc tcacaacaac tctgtgagat 276240
   agaaagatgt tatcaccatt tttacaagga aacctggact caggttaaga gatccggctc 276300
   agggtcaagc aggttgactg tagagctagg atctgaatct ctgtctagca gattctaact 276360
   cttatgctac actgtatcta catgagtatg tatatatttt acactactat gatatcataa 276420
   tataccctcc ataaacaatt tttaaaacag tagaccaggg tcagtttacc aatggactat 276480
   caccctttag gaactttaaa atatttttcc taactataaa aataatgaat tcttcctgta 276540
   gaatacatgg aaagtaaaga aaatgtccca cagatatccc agtcaaggaa tagcctacat 276600
   taatatttca gtgtttcctt tctatatttt tctgtttaca aatttctttt gtttgagata 276660
   attatatatg tagtttggta actctctaaa tttcaattaa tattaacatg aatactaata 276720
   aaattaatat cataaaatat attgacctgt attacttata tttattaatg taaagtagat 276780
   ttaatgacca ttttaatggc cttataatct tctatcatat acatgtatca aatttattaa 276840
   accagtcact gatttaataa atttgaggta actttagatg tatgtggttc tgtgactttc 276900
   taaatttcac ctaatgttaa cattaatact aataaaatta atattaaata aaatatatca 276960
   gcatacatta cttattttta ttaatgtaaa ataaaatata ttaacgtatt ctattagcta 277020
   catgtcaaaa cacaatttta atggcttaat aatcttttat cgtatacatg tatcatattt 277080
   aaaaccagtt tctgatttat actgtttcca atttgtcact tttattattt tgcagtgagt 277140
   gttgttacac ataagtcttt ggcttcatat ctgataattt attagattcc tagaagtagg 277200
   atgaacagat caaatgatat gtactttttt tagataaatg atatacagtg ttaatttcta 277260
   gaaagtttgt accaatctag accctcacca atagtattgt ttcaccttca ctagcgtcat 277320
   tgagactgtc atgttatctc tgttcatctg ctagatatga ataaacctta ctgttttcat 277380
   tagcactttt attactagtg tagctaaatt gaattttatg tggttatagg ccatttttat 277440
   ttcttcacta aatcatatgt tctgtacaca gttttcaatt agggtgctag catttgtttt 277500
   attgatgtgt aggagctatt gatatgttaa ggaattaacc cttctctgta tatttgttgt 277560
   actttaatgt ttaccttcca ctttctcatt taccttttaa acttatcact taagagctct 277620
   aaaataggtg gggaaacatg agtaattatg ttagaaaaca cataaagtat tttggagtaa 277680
   tattctttta gtaattttat gagagcattg aggggaaaga taagtttttg tggatgagag 277740
   atctaagtcc taaaatatca agtcagggta tcagtgttag atgttagtct ccaggaaagc 277800
   atgtacaatc cacatcttct aactcccaga acaatctacg gctgcttgac ctctttgtct 277860
   ttctattgta aggactccca ttctctgaga agtaagaagg tgtttggtcc ataaacaggc 277920
   actgtacaaa tgcactcagc tgcaattttg aactctgttt atcatggagg ctcatatgaa 277980
   gcccccagtg gcctccacac atctccagct cccacgggtg cttatttctt acacacgctc 278040
   caaatctcac agagagttcc aggtgactgg gcttcagcat ctttccagtc caccattcct 278100
   ggtttctgga atttattttc caaaccacaa tgccagaaca cacagagcaa tatgatgtag 278160
   ataaataatt ctgtagttta ttatttaaca ctagctctga aaactcgagt ggaaacagcc 278220
   acagggcagc aatttctgga agctctctcc agcaagcagg ctccttagcc tttttaattt 278280
   ctatgccata cttattttta tcttcaatat atattctttc aattcagccc aaatatctct 278340
   gaagttcaac ccatacctac ttccaaggag aagttaagtt ggtcttgtgt ggctccagtt 278400
   ctaataatag atagtttact tcattcactt gtgggactca ttccctcaat tactacgagt 278460
   tggtgaccct ttgctaggat aaccagcaga ggactgtgaa cagtgcattc taaaggagac 278520
   ccttagagat atctgatcat atccccaaag atggtcaacc aaactccctc ttgtgaatgc 278580
   aaatcttgct ttggttctac ttgttttcat tttctctctg tgtctctcca atgtcagagt 278640
   gacctgccat tttctctcaa atttgctacc ttgattcctt ttatttctac tagctaccct 278700
   tgatgctttt tgtctgtaat agattttctg aataaacttc cccacaatca gtacctctca 278760
   atcagtttac ttacaattgt agttgtggcc acaagagaac aaaaagcaac ttcatcttca 278820
   caagtcaaaa gtatatttcc cgtttcccca ggtcgtgatc atgcgggatt accaccatga 278880
   caatgtggtt gacatgtaca gcagctacct tgtcggcgat gagctctggg tggtcatgga 278940
   gtttctagaa ggtggtgcct tgacagacat tgtgactcac accaggtagg cttctttatt 279000
   atccagagaa ttcgaggtgc ttcttattct tctccaggat aaagccgcag tgactatgaa 279060
   ctcttcataa attgcaggca ctttggagtc agggaatatg actacccttt cccctacatt 279120
   ccccagaggg cccattatgc tctcaggcat gtatagttca ttcatttggt gaatgttctc 279180
   cctgccctca gaaggacatt aatacccatt gatttaaaat aagcaacctg ctaagaatga 279240
   agcttcagtg acaaataata gaatgtcctg tcgcctatat agatttctaa agctgaattt 279300
   cattctcctg agttactcta ccattgctgc atgctaagac accaaggata ccaggggaag 279360
   gaagggacag ggagaggatc acttgaagtt ttgtatcaaa ataatgaatc tgggtggcac 279420
   agagaaattc tgcaattctg ttttaaaata tgcttgcaag taacgatggt taattaccag 279480
   cctattgctt cagctatttg cctccctgat gtgccagttc cagccatttc attaagtatt 279540
   tattgagcac cttttatgtg ccaggctgtt ttgtaagtgg tagagatgca gaagatacaa 279600
   atgaatgtgg ctcagatata tcccctcagg gacatcactg atgagaaata cattataata 279660
   cctgtgcaga attaagaaga tatatatatg tagcatagaa gaatacctaa atgtctctct 279720
   cagaaagagt caataaatat ttctatttaa tataaggtga catctgagat ggattttgag 279780
   agcgaatagg agttcactaa gaaggcaagt aggaggaaaa gggcattgaa ggcagaggaa 279840
   agaatatgtg ctaggacagt gcctcttcaa tttcagtgtc cacataaatc actgtggatc 279900
   ttatcaaaat gcatatcctg actcaataga gagggtgtaa gattctgcat ttacggcaaa 279960
   ctttcaggca atggcaatgc tgctggttca tggaccatac ttggagaagc aaggttctgt 280020
   ggaatagaca tacaaaatag catgttacat ttgggaaact ttaactttgg ttttgatggt 280080
   aaataaaatg ccaaaaagtt aaggaaggat tgtcatgagg aagaggtatt gagtgttagc 280140
   tgatgttatt gaaatggtac ctgaatcaaa agtggatcca ggtacataat tggtggaaaa 280200
   caaggtaaca agaaagtact gggaaaccct gtgaagtata aaacatgctg ttttttctcc 280260
   actgaaatga gatgttacgt ggctctacag ttctcggtct ggagtaggct atctcaaaat 280320
   aaagctttgt gagtgaagac atttatcacc aacaattcag ctggcagact cttgatgttg 280380
   tagcaataag acatgcccaa caaaaaaaca cataagatca tcttaattgg tagaaacaaa 280440
   tatactggaa agatctaagc ataaggcaaa tgtgatatac attttttgct tttcacatat 280500
   aaagcaaaaa ataaaaatat attcagggga gaaacctttt tgttgccttc cagagcttca 280560
   tacagccact caggtctcca gctctacctt tgtcaaggtt cctttccatt cttattttgt 280620
   atccatggaa accactcaag acagtctgtc cctaaaatcc attttctttt ctaattttct 280680
   catccaggat tgtcaactga tttccctttt aaagacccag taactccaga ggcaaaacaa 280740
   aattgtgtgt caaggaacac catgaatact tggtattcga aatcttaaac tcagaattca 280800
   gagagcttcc tttaatagat attgaataac tgctttactg aatgatttat tgatcggttg 280860
   ataaaacatc taaccccttg caactccaag tgctgtctga ggacctggga gcttgttaga 280920
   aatgcagaat ctcaggtact accccagacc aactaaatga gaatccatgc tgtacccaaa 280980
   tactcaggta tttcttatac atactgaagt gttagcatta acttaacatc ttgttacgtc 281040
   agatccttct aatttccagg aaacagaggg ttatagaagg gaagggaaag tgccaagcac 281100
   catcaagctc ttcatatgtt aatcatgtaa ccatcacata tttctatgag gcagatgctg 281160
   atgtacctgt ctgccaggtg atgaagctac aattcaaaca ggaaaaatat tattggccag 281220
   gcatggtggc ttatgcctgt aatctcaata ctttgggaga ccaaggcagg aggatcacgt 281280
   gagcacagga gttcgagacc ggcctgggca acttagcgag accctgtctc tattaaaaaa 281340
   atttaaaaat tagctgagtg tggtggcatg tccctgtagt cctagctact cgagaggcta 281400
   agacaggagg atcacttgag cccagaagtt caaggctgca gtgagctctg attgcgccgt 281460
   tacactccag cctgggcaac agagcaagac tctatctcaa aattttaaaa ggtatccttc 281520
   ttaagaccac acatttaata actgatacag ccaggatttg aatttgaggt tactctagaa 281580
   ctgaagggtt ttttattcca tgccaagctt ttttcctata ggaaagaagt agaagaagaa 281640
   atgcaccaac gattctccag ctggccatgc taatttcaaa gacactggat taggaagtat 281700
   ttgtcacgtg ttccagtcaa ataggatctt tctgttagtg actagcggtt agattcaagt 281760
   aatttttgat atagtcgacc tgtaaagaat ctttatacct ctttcagata actcaaattt 281820
   cacggaattc aagagaaaat aattttatta aagatggtgg ttcttggttc tgaattaatt 281880
   tgaaggtata tgtgagcttg tccctgctgc atatactgta aacctctgag gaatgtgttt 281940
   ttccacatgt gaaaatggtc gtgtgaaaac cattcaggat aatgaccaac ataccacaaa 282000
   acccagaaca cagaagttgt aggcttcctc tgtttcaggc aaaaacattt cttgtcacct 282060
   gcctttgtat gagactggtg ctgtggaatc ctcttttgct ttcttttttt ttttgaaaca 282120
   gagcctcact ctgtcgccta gctggaatgc agaggcgcta tctccgctca ctgcaacctc 282180
   tgcctcccag gttcaagcaa ttctcgtgtt tcaacctcct gagtagctgg aattacgagc 282240
   gccggccacc atgcccagct aattttttgt atatttagta gagacagggt ttcaccatgt 282300
   tggccaggct ggtctcgaac tcctgacctc aggcaatcct cccaccttgg cctcccaaag 282360
   gccaaatcct ccttgagggg aagggtgtgt ttcttcatac tcatcaactt tatacctttg 282420
   gtattctcct aattggctac cactttatac cttacttttg aatcccatca ggattttcta 282480
   gcatcaacta aactgaaatg aaaattcaaa gccattcatt tccttattct gctgacctta 282540
   gatttacctg ggattactgg agtttcacaa gtttgtcctt aaatttgggc tctggtcatt 282600
   tacaccctct gtctctcctg acagtctcct ctgtttggat gatagttcgc tttaggacct 282660
   cttttacatt tattatttaa catgggatag ggaagaagtt ttgcacatag atgagttagg 282720
   cagctgccta aatgagtcat tttgagggca ctgaacaacc tgtgccatcc aaataattct 282780
   cacctagatt cctctgtggc tgaactattt cactcattag gaaaatccat tcattcaagt 282840
   ggtgagggca gtgtgggaaa atgggaaagt gggaaagtcg gaaagcgtgc gttctggact 282900
   aaaaaagagg tggggtttga atcatgacct ggccatttct tagctgcgca accttgggca 282960
   gttagctgac ttccccctcc ccctctaaaa ctgtggagaa ggaaaaactt tctccacagg 283020
   ggtgcagtca tgagatatgg gaggccataa ccaatgcttt gcttcgtttc tcattcccat 283080
   ggtccattta ttaaaatact atccattcac actctgttat tagtaacttc ctaggtccaa 283140
   tttccttttg agagaaggga cctgaagcga caaaaacctc tagctccttg gaggaagaag 283200
   agagagggtg aatgggaagt agggtgggtc ccagagggag gattagagat gcaaagacca 283260
   ctttgcctga gctcagcctt gaaaactgtg gctgtcccca tttctaagat gaagaaaata 283320
   agacccaagg aagttaaagg attttcttat gatcatttaa cagccacgcc tgaaaccttc 283380
   atctacctat tcagtgtgct gactctgtgg gaccctggag gcttgttgat gcctatggaa 283440
   ttatctgtta agagttcatg gagccccgca cagtggctcc tgtctgtaat cccagcactt 283500
   tgggaggctg aggcgggtgg atcacctgag gtcaggagta gttcaagacc aggctggcca 283560
   atatggtgaa accctatctc tattaaaaat acaaaaatta gccgggcata atgatgggcg 283620
   cctgtagtcc cagctacgtg ggaggctgag gcagagagaa tcacttggac ccaggaggtg 283680
   gtggttgcag tgagccgaga ttgtgccact gcactctagc ctaagtgaca gagcaagaca 283740
   ccatctcaaa aaaaaaggaa gactgcactc cagcctgggt gacagagcgg gactccatct 283800
   caaagaatga atgaatgaat gaatgaatga ataaataaat aaataaataa ataaataaat 283860
   aaataaatga gttcctggaa gtgcatttgc atgaatgacc aaaaagctga caaatagtga 283920
   tctctttcct ttgacttata tttggtccct gtcattctta gtgccaagcc ttttttctgg 283980
   gagtcagatc ccaaagactt ccttcccttg tctgcaggtt tttcagatat gggctggagc 284040
   acgtggactg tttcactaaa gcacaaggga gctgcctctc ttgagcattt caaagatgtt 284100
   cttaaacctg tcttgttcct taaatgttat ttcctttcca gaatgaatga agaacagata 284160
   gctactgtct gcctgtcagt tctgagagct ctctcctacc ttcataacca aggagtgatt 284220
   cacagggaca taaaaagtga ctccatcctc ctgacaagcg atggccgggt aagatgttca 284280
   tgtttggccc gttcgtagtt tttcttgtca ctctgtctgt catgggcata actcttttag 284340
   aaacactttt ttataactct aatttttctt agtcacaagt cacctacttc tcttcccaga 284400
   acacttacaa aaaagaaatt accatccctt cctctgtgtc accttgttag tcagaattgc 284460
   ctgactttga tggataattt gcctccttaa caatgagtat aggaagtaat agaaactatg 284520
   gctatttctt ttccttttct gccacatctg ttttttgttg gaggagaaga gctgactctt 284580
   ctaggggtgc atccaaccta ttcacatgaa tacatgtgtc cacattacct tccagagccc 284640
   gtgcaggtaa cagatgatca actccacttg ggtaatttga ggatggtgta ataaaggaca 284700
   gtttacaaaa gcgtgggcag atgtagaaaa atcataagga atagtgtatg aggattagtg 284760
   cagtacttta taatggaaca ttactcctac taccccatgc ctgaagtggt gaggcaaggt 284820
   cctggctact agaatctgga aatggagatt gctatagatt tcggagctgc ctggagggtg 284880
   ctctgatctt tggtagaagc caaaccaagg tgaacctgca ggatgagagc tgagagaata 284940
   aatcctccaa cctcactctt cttccttcta gtgtcctact ggtgctccct attggctgaa 285000
   atcagctagg agccaaaatg cagtcaatat aggttggttt cctaaggctt ggaggaggga 285060
   ggaaaatgga tccagtagga caaacagagg atatttatca cactggcaaa cctctaggcc 285120
   cagatttccg tatttcacat cactgcaacc tgtgcaaggg attctgctga aactcatcag 285180
   ccacaccagg gcacagagca tggaatataa cagccaagga aataaccagt tgacattcca 285240
   aggaaggctt agttaaatgg aagagtttca aagcatagct ctataccatt aggctctccg 285300
   gagccccctg aaactcccac cagaggcaat ggccttggca tgaatggagc caaccattcc 285360
   cttgatgttc ctgtctttta cacctgcaac ccctgccaca tggaaaatgt tcatgtcata 285420
   tcagaaattg cccaggaatg atcagagggc agccagaaga aactgtccaa gcactttggc 285480
   ttttctttct tgttgtttta aaaagattgt aaatattatt gtagaaaatg tagtaaactc 285540
   agaaaagtat agaggataag acaaagatca ctcctaatcc cattaccgat ttttcccgta 285600
   tttccatgta catgtataca caatttgcaa acttgggaat gtgtttaatg aggagtttta 285660
   agtcctgctt tttgcattgc tttaaccatt gcattgcttc tcttaagaaa gaacggtaca 285720
   gtagaagcat gtatcagtca ggttttgcta catccactca acagttagtg gcttaaaaca 285780
   accaccattt atttgctgac ttatctctga gccagtattt gtgctgactc ctctgggaca 285840
   actcatctct gtaccagctc atctctgcat catgtgacat cagctgagct ccctcttgca 285900
   gttgtgatca gcctatggtg atagccttac tcatttgctt ggcagtcagc tggctctcag 285960
   cagggacagt gcaggtaaca gggccaagta tcactcatct ccgagtcacc tggacttact 286020
   cacatgatac ctgtagcaaa gttcccaaga gcaccaaagg aggcaagccc tgatgtgtag 286080
   gccctttcaa atcctgtgct tatattatat ttactaaata ttcaagacca gactcaagga 286140
   gtagggatat aaactctgcc tcttgatagg agaagatgcc aagtcacatt gcaaagggat 286200
   gtggatacag gtagaagaag actgtgtatg cattttttct taatcttcca tatgagtgga 286260
   cagctatgag aaaaggtcaa gaatcctccc tggcctgact ccggaaagca cctacgcaag 286320
   gctccacggt gtcagaatgc aatttgcaaa gcattaaact ggtctagcag agcaatttca 286380
   agaaagactt tctttccctg tttctagaaa tttatggtac aggcttcctt gctgtaagat 286440
   catatccagt atcaaaatca gaaatgagac taagaagtca ttgtttaatc aaattactta 286500
   aagatctccc ctcccctcaa tgtgcaagat ttttaaatca ttgagttgtc attctcaaga 286560
   gcagaacctt tgtctcaaac acctcctgca tccctgtgca ccctgtaaag agctcacata 286620
   aatagacaca ttgtagatgc ttttagtcct tattggtttg ttctctcatt catttactga 286680
   acatttgctg aatgtctgct acgtgtaaca gacagttcta aatcctaggg atgaacaaag 286740
   caaaatcact tcctctagta agcttatttg gggactggga atgtgataat aaacaaatag 286800
   ataaataaac aaacaaaatc cccaccaaac aaggggtgat aaatactaag tttcctagga 286860
   ttaatttagg catggtaagg agaatgggga gtgatggaag tgatgggtac tgtcttatgg 286920
   aggctggtca gaagatccct ctgatgtggt ggcgttacag cagagacatg gggactggga 286980
   ccctgtggct gccagaaaca ggagcattcc aggcagagga agcaagagca tattgagtgg 287040
   atcttgtgcc tcttggccat tttgtccaga gtttactcat tcatgttcag aacagtgcat 287100
   catattgact ccagtggttt ctccagtatc tgtaggaaaa ggaaactttt cttttaaaag 287160
   agagcagaaa tggagtgtgg ctactccaag aaaaggctta gagtgtgtta ttatatgagt 287220
   ctattagtgt acaacattat atagtggaga agactttttc aattatctat tgctgttata 287280
   acgaagcatc tcaacattta gagtcttaag tcaacagtca ttttagtatt gtctcttata 287340
   gctcagcaaa acagtgctgc tctgacttgt ggttagataa tgcctggggc cagagttatc 287400
   tggaagtcat cctcactcac atgtcaggta gtcagtccag gcaggctagc tggagctgcc 287460
   accagatcac ctacaaaata ggtggcctcc ccatgtggct tgagcttcct catggcatgg 287520
   caactgggtc ccaagagtgc gtcccaagaa aacgaggtgg aagtgcatgg catttttata 287580
   acctagcctc agaatcaaat aacattactt tcaccatggt cacaagccct tccagattca 287640
   gggggcaggg gtttagagtc ctcctcagtg tgagaaaaat taagtcatat tgtaagaaga 287700
   gctcatggga taggcaaaat tattgttggc atttttggaa aatactgtct gccacacaag 287760
   agatattggg ttctcttcct gtttgaagat gttcaacatc attggccttt gagaaatgca 287820
   cattaaaact acaatgagct atcactgcac acatttaaga atgactaaaa tgaaaaatag 287880
   tgataacaac acttattcat acactgctca taggaactta aaatggcaca gccactcaag 287940
   aaaatacttt ggcagttctt tttcaaaatt aaaaatggac ttaccatacg accatcactt 288000
   gcactctgag gcatttatcc agagaaatgg aaacttattt tcccacagaa acatgtacac 288060
   aaatgtttat agcagttttc tttataatag ttccaaagtg gaaactacct aaatgtcctt 288120
   caaagggcga atgattaaac aaatagtatt aatatatcca tgtcatggaa taccacttaa 288180
   caataaaaac aaacaattga tacacacgag aacttgggtg aacctcaggg aagttatact 288240
   gagtgagaaa acaccaatct caaaaggata cacactatat gattccatat atttatatat 288300
   atacacacac acacacactc atgaaataac ataattatag aaatgcacag cagatgagtg 288360
   gttggcaggg ttttggggct gtgggaatgt gtctgtaaag aacagcacca ggaagtcctg 288420
   tgctgatggt acagttgggt atcttgattt tggtggtggt tataataggt tggtgcaaaa 288480
   gtaattgcca ttacttttag tggcaaaaac cacaatgact tttgcactgt tctacacagt 288540
   ctcccagact tcttcagcag gatcaagctc tagttgccca ccgtagtaac tcaccgggta 288600
   actagccttc ccttggttag ctgactagtt ggcaacacat gatacaattt cagagtatac 288660
   ttatatacac tacacacaca atgagttcat ttgtaactga tgaaatttga atacgttcta 288720
   taaattagac ctaatcaatt tacaagtgtt gatattgtac tgtagccgtg taagatgcga 288780
   atactaggga gagacgggtg cacgttgcaa cttcctgtgg atttatcatt atttaaaaat 288840
   taaaaagttg gcagggcgtg gtggctcaca tctgtaatct cagcactttg ggaggctgag 288900
   acaagaggat tgcttgagcc cagtagttca agaccaacct gggaaacata ttgaagtccc 288960
   atctcatctc taccagaaaa ctaaaaaatt agctggatat ggtggcattt gtctgtagtc 289020
   ctagcaccct ggaggctgag gcaggaggac tgcttgggcc ctagagttta aggctgcagt 289080
   gagccaagat cataccacta cactccagcc tgagtgataa agtgagaccc tgtcgccaaa 289140
   aaaaaaaaaa actaaaaaat tttaaaaatt ttttaaagtt aggaaaaatg accgagaatt 289200
   gctcaggttt gaaagaatga atgctttctg tcctctcaaa agactagata aattatcttt 289260
   tatcaactaa aagtaaatca ttcaattatt atctatggca taagtaatat ctaacgagtc 289320
   actaatttgt gttaagaagg gagaatgtgg tgtttgggat ttgaaagagg aaaataaata 289380
   gtatctcaca gagtggctta ggaatgaata atgtaatgtg tgtaaaacct tcagaagtat 289440
   accagggaca aggtagatgc taagtcagaa ggcatttcct ctgcccttgt aattaagtgt 289500
   tgtgtagaat catcatgacc tttccttggc ctagggccaa catttgataa atgaattact 289560
   taggggccag tgagcagtga gtgactgact ttgacaaatt cattggccgg aattcaaacc 289620
   catgacccgc taagtccaat aagtttatgg aaaagagaca cacacaagtg gtctttgtga 289680
   tctctttttc tttttactat aaattgattg ccacaaatca aaacttggaa gatgtcatat 289740
   aaaatgtcag attcctgtct tccttgaaaa atcaaagggt ctggcaacac tgtgcctgga 289800
   ttcacacatc atgataaggg aagtggtata aggagggtga gttgtaccac agcaaatgcc 289860
   gttggtgccc tgcccagatc ttcctgcaac ttcctatttc agtgatcatt agtttcacct 289920
   ccaaatgcca ccatctgcat ctttatgcct gaagacttct ttttctggcc actgttatct 289980
   gctttttcca taacgttctc taactttact gagcatcaga acccactgga aagcttgaca 290040
   agaaacagat tcctgggccc caaccccaga gattctggtt cactcaggct acgtggtacc 290100
   caggaagctg catttctaac aggctctcag gtgaggctga tgctacatac attgtacact 290160
   gagtgccatt gccttacaaa gaagcaccca gaaactaggc acacaagtag cccccttttg 290220
   cattagccaa gaccctagag cagacacaga attctttaga gtgcatgcag tttacagagc 290280
   aagggttttt acattctatc tttaaacctg tagctgccct gtggatagac tctcttttgc 290340
   ccactctcac tagcccccaa ccctccacca ttcttttgtg ggaaccctga ttcctggcca 290400
   ggacacgtga gagatggata ggatctcatg tttaaattgc agctttgtct gagttctaaa 290460
   ctaaagttcc ttgtgtggtc cttgtgtaac aagctacatt ttctcctttt agggggcgga 290520
   caacatacta gtggtagttc tcttagccta actgcattag tgagacgaag gtggtttcct 290580
   aagcagccca gttgcacaat tctacccaaa gtaaggctca gcttagcgtc tatgtgattt 290640
   gcttttaaga tctgcctaca gtgctttatt gtactcatta cttgagttct cattatctac 290700
   ttgcagattt atttggacaa atgttgtgtg caattatgtt cttttagaaa attgtccctt 290760
   ttatgtactt ggatgattaa agtatcatgc aaaagcactt atactcctac agcagcagaa 290820
   caggcctcac ttccaagtgt cgagtttcaa ttgctcatct cacattttgt cagggagatg 290880
   gttattagga tgccagattt tgaggggttt gggcattttt cctgccagca aagagaaacc 290940
   cacactagat atactgaaat ttaaaactgc caatagcaaa atgctgatac attcacctcc 291000
   ttaaccattc aggtttctta aatttatagg caaacaaaac tggaacgttt gttcctcctc 291060
   tactctctgt gtattggggt tgcaaattta cttacaccct agttttggaa ataaacacac 291120
   acacacacac acacacacac acaccacaat tatggtttct atgcttccta aaactaaccc 291180
   agacttcttc acagagtcct actggctaga ttctttttaa aaatatctat tttaattcaa 291240
   tgcattaaat ttagttggct tgaatgattt gaacattgtc tatcaaagtc aactacatta 291300
   ttatgaggta taaatgatac ctttataaac ccagtctttc agatttaacc accacctact 291360
   ccctcctctc ctctccacct tcacttattc ccaagatgga gtgtgggagg aaagacaagc 291420
   atggctcgtg ggacctcagg gtgaaggaga taagaagcat cttcttgttc ctttctcctc 291480
   cctgccttgc tttcattgga ctctcctgct acctggactg gtgaccaggt agcaagattg 291540
   actggtccta ctggtggctg ggctgtccag gggctcacct ctgctgtaca tcacagttgc 291600
   agcctcttca ctcacgggtg aaaaggtgcc ctacaggctg cctccttgtc ccaatcacag 291660
   gcttgtgcca gacccttccc cattccctca ctgagatttg gaggaacttc cgaacccctg 291720
   cacatcccac acaggccatg gaagacagta gcattgccac aagctcactt ccacccacac 291780
   tatagcgccg tgtctccatg gagtacattt tacatgctgg tgtaaaacga aacagtatct 291840
   gagacaggtg tcaatcaatt tagaagttta ttttaccaag gttaaggaca tgtccagaag 291900
   aagaagaaat gaaaaacatg gattcataga aactgtctgt ggtctgtgcc tttctccaaa 291960
   ggttagtttg agggaaaggg gaaaagtgag ctggagggga aagagggagg gtatggtcat 292020
   ccacttgttg caagagaaaa ggagcaggta ggagaactgt caattatgta ttgatctcat 292080
   gctcagcaaa tcggcacttt acagaatatg atgtgaacat agagtcacta cctatggagg 292140
   tatttaccta tgacctgtag ctgtctaatt aggaacaaaa cagaaggtag cttcttgcag 292200
   gactcagcgt ttagttcaat ttttcctctt ggcagaatga attggcgtcc tgagttttta 292260
   ttttcctttc acacggggta tggccaaggc tggtgttcaa tcagttttac cagcatgtct 292320
   acacctgttt ttaaaatatt gcaagatttt catgctagtt ggttgagagc cactgcttta 292380
   agtccttagg ctatccccca tcactccagc cccttcccaa gaccctcctc acctcattaa 292440
   aaatggtaat ccttcccagg gtaactctag gactttgctt gactttctat ctaggttcca 292500
   attggcctac gttaatctgg ttattgaata ttttgaatat tacctctgaa cacatgcttg 292560
   tggggcttct cctctccctt atttgtatgg ggatgtatgc aacacagata tttccaaagc 292620
   ccaccaccat ctgcttggag tgaaaaaaag tgatatatca taaggaggcc gggacctgac 292680
   atagtcaaca tcactcctct ttctctcctt ctgcaaatta tcccagacta caaggggtgg 292740
   actgtaggat ttttacttct gtgcattatc gactcctctt ataatagggc accatggatt 292800
   atggaccacg tatcacaact acaaggccct ttttttcata ctacatgtga aattctatga 292860
   tctgagtcct ctggggttga ttcttgataa gaaattgtga atatcacctc tctgctaaac 292920
   cagcttctct agcatttctt tgggaataat ctaatccctt ccaattcaaa tcgtaccacc 292980
   actggtccaa atcactggcc ttaagccaat tagtgagaat atcatgaaca aggaaatgaa 293040
   aaaaaatcac attaataatt tccaaaatgt tattttcatt tcatctgtgt tccaattttg 293100
   attctagcaa cagctatctt cagtgtgctt cagatgtttt cagaaagagc ttcaatgtgt 293160
   cttctgtcac ctggtatact attatgtatg tgtgtcagta taaaataaat ctagtagcat 293220
   ttgttttctg agtttctgct gtttgtttgc ccaactctta aggaacacag taaggctctg 293280
   caatcagaaa gcagagttgt tgtccccaag gaatgtcatc tctagctaga gatggaaaat 293340
   cagcaccaga aaacagttca ggattgagtg catgccaaat tctctgaatt tggaaaagag 293400
   ggtgcagcgc accagcatgg cacatgtata catatgtaac taacctgcac attgtgcaca 293460
   tgtaccctaa aacttaaagt ataataataa taaattaaaa aaaaaaagag agtgatcaac 293520
   gtgggctcct gagaattctg gaggatgtta gatttgataa agcccttgat gtatacctaa 293580
   gagttgaata tgcaagaaaa atcatcaaaa aaactcagag ttgggaatgg accataagat 293640
   aaccacctct ttctgatttg cccagaactt tctgggtttt agccctggat attctacatt 293700
   ctaggaacag ccctggtccc taggaatggt tttagtgtgt gcagaaagcc atgaggaata 293760
   aaattggcga cacgaaaagg caccagatta cagagggctt taaaaaccat tcaggtggac 293820
   tcgtgtgttt gcgccagcag taggaagatg gggtgtggtg aaaatgttaa ggcttttgtc 293880
   agtatgctaa agagattaga ctctgtgcaa acaaatgccc ccttatcccc acctgttcat 293940
   gtgacctcct ccctcttctg tttcttgact gaactgtgat ctatgattca agctataatg 294000
   tgtataccaa cagcctgtgg ctcttgttaa agtggaaact gactttagta ggtctggggt 294060
   ggggcttgag gttctgaaaa tctaacaagc tcccaggtga cactgctgct gctgctattg 294120
   atgaagatga tgctttaagt aacaagacct tggatcccat cccatccctc ctgaagggat 294180
   gctttaaaaa ccctgtcatc agttatcctc actctctcct ctttcttcaa catctaccac 294240
   tctgggaact cccttctttc agcctgtaac tttgctcttc agagcagcag agtggtggtt 294300
   aaattcccga atcctggctc ttccagttac tagctctgta tccttggcaa agtggcctaa 294360
   ccacttctgt gttttggctt acttctctgt aaaatggaga taataatatt tcctacctcg 294420
   ttagtttctt gggagctgtc aaatgagata catgtaaagt gtttaaagtg ttacctggca 294480
   ggtagtaaga gctcagtgag tgtattcatt tcctaggact gctataacaa attgctacca 294540
   attgagtggc attttcttct ctctgcatga aatgtattct catgattcaa gagtccagag 294600
   gtccaaaatc aagattttgg tagtgttgat tccttctgga agctctgagg gagaagtttt 294660
   atatacgttt ctcctggttt ctggtggttg tggcaatcct cagtgttgct tggcttgtaa 294720
   actcatccct ccagtctctg ctttgccttc acatggtctt cttatctatg tgtctgtgtg 294780
   tcctctccta tttttatatc agtcatgcaa tttagggcct gtccccaatc caatatgacc 294840
   tcaccttcac taattagacc tgcaaagact cttccaaata agatcacatt ctgcactttg 294900
   gatggatgtg aattctggga gatattcaac ccactgtaga gaatagtaat aattatcatt 294960
   gtcatcatta tcatattcac cacgtttgta aatgctggat ttcttctatc tatattaaaa 295020
   tacatctcat ttcttgaaaa gcactctgac tgaaatctac ctgactgctc tctgtattcc 295080
   accactgact caacctgaaa ctagaggcta ctagggatgc cctcattgct gagcacaaca 295140
   gcaactcccg gttcttacct ggtttcatct ccctgttgtg tcctagctgc tgctatccct 295200
   accttggctt tcccaattta ttctccgttg tttctctgat ttcttgatgt ctttctcttc 295260
   ggtctcctgt aatgcctcaa tttcgatcac cctaggtgcc cagtatttca tatttgacca 295320
   tcttcttttc tcatgctatg gcccttcaag ggtttcacta ccactcatat cccaaggacc 295380
   ttctcatcct accctagcga tggcaatacc cagatcttga tagtactggg aggtcaaaac 295440
   tcatcacatg ataaacctaa agtgtgtaca tttcacactg tgtaaatttt taaactgtta 295500
   aaaaaacaca ttgaactcca gttaatgcta aattaggagt gaggtgtact gctgtcagta 295560
   attcagtttg aaatgcatca aaaattagat gaatagatgg atggatagag agataggtgt 295620
   gtgataaagc ccacacagca aaacattaat aattatagaa tctactcatg cagataggga 295680
   tgttcactct ataattcttt cagctttgct atatgtttgg aaattaccat aataacatgt 295740
   gtggcagggg gaatcttgtc tctgggtcag acctccctgc tgaatcctga tctttataat 295800
   atattaatgc acttgttctt cattctccaa ggactctttc acttcttatg attgaggttc 295860
   atcaatttat gcaacaattt tgattttgaa aacctatcat gggccaagca ctattgtggg 295920
   tgttgaagcc acagagatag tttttttttt taactctaac caggaagact tggggtgaga 295980
   tagagacaag agtcaaggat gattttcttg gattgatgtt ggtgtcattc actagaacag 296040
   gactgatagg aaaggggtca gcttttggag cagagagagg gatgctgagt ttggaagtac 296100
   ttgtggtcat ccatttagaa atatacacta gttggaaata caggctttga atggaggagc 296160
   aaggtctgaa ctaagcatag aaagaaaaac agtatccagt aagagaatct aagaagagaa 296220
   gatgtgagag aggaaaccag aaccctaggg aaccccaata tttaagaggc aggcggcaga 296280
   ggtagagcca gaaaaaaaag gttggaagag gaagttcaga gaggtgtgaa gagaattaag 296340
   agagaattca taccctggag actgtactat ttacctttaa aaattttttc acttcaagtg 296400
   gtcattctga accattgttt tcttttgtgt tctattatca ccaacaactc taagtggctc 296460
   tttgatgtca ctttcataag gctgtttgaa gttcagacca ggtcatcatg actagaaatt 296520
   atggcttcca gaaataaact gaatcccggt catctagaat tcataaacca gcagggtgcc 296580
   taacacccag ggtgcagtat gttcttaaga gtacatgagc tctaggaaag aaatttccaa 296640
   gtttttttcc tccttgatga tatctaaggt aaataacatg tatatacact gagcccttag 296700
   gatgaccacc ttaaacccct gacaaccaag tgccaacacc caggttctat gcctgtgttt 296760
   ccacttgcat tgatgttcat gttggtagat tcaactgtca tggacaaagg agcaaaccaa 296820
   aaagggaagg gtatccagta ctaaaagtga catttccaca tcctggataa gcaaaggcct 296880
   tgcaatattt caaatataga aaagagatgg ttctttgatt cacctcatgg tacatagaaa 296940
   tctaaatgtg ctcaactcaa aagaatctaa attacatcag tttgttcaaa attcatatta 297000
   agaatagcaa cttatgtata tgaaagcatc acctgttaca ttatattaaa cttgtaggct 297060
   tgggatgtaa caaatttgtt gtttgtggtg tatttaattt gtaaagattt tttgttgttg 297120
   ttccatcatt ttatagaagt aatataagca gcaggagttg agacatttgg aagttgatcc 297180
   tggtgggccc gggagaagtt tctccattaa aatggaccat gcctttccaa taaaagggtc 297240
   tgacagtccc tcctctgata tgtccaaggc ctgttgatga gactgttttg ttttaaattt 297300
   gaaactctgt atgtttgtat tttcgtgtct agcagacatt gttttaaaat tgctagtttg 297360
   ttgcattcct ttccttttca gataaagttg tctgattttg gtttctgtgc tcaagtttcc 297420
   aaagaggtgc cgaagaggaa atcattggtt ggcactccct actggatggc ccctgaggtg 297480
   atttctaggc taccttatgg gacagaggta agggggtcac agtcataggc gtggcaggac 297540
   tggggacagg ctcagggacc cattggttct gagatagttg gtgaccacag gagatggggg 297600
   caatgacttt tagctccaca tttgatcccc actcactaga tgaggcacag agtagatagg 297660
   ctgtggcagg tgggagcctg gccacccctg gggcagatca ctgcagtatt tatccaaggg 297720
   ctgtttattg cagtcctaca gtggacactg tgggaaagtc actccaactc tgatcccact 297780
   ctcagtagca ggcacacaca caaagcaagg caatgcaact tgcaaagtgg ctggagacat 297840
   gcagatgagc aggcatgaga attcagagaa aggaaagagg atcaaagaaa gtgccacaaa 297900
   atagaggtga tatttaaatg acccactgtt ttcaacacag tttacatctc acagtgaagg 297960
   acctacactg ttttctcata ggaaaaaaat aatatgtgaa tcatagtcaa aaacatggca 298020
   caatttctgt ttccactgga atgaaaacaa tcactcttaa tcttttaaga ggtagggcct 298080
   ggaaatgtga aaatgcaggg ccagaggagt agaggaaggg ccttcttggg aggtaatagc 298140
   atgggtggaa gcttagaggc acataaggct acatacagat ggaagccaag ggtatgtttt 298200
   cggctaggca tagtgtttcc acactttatg gtgtggatgg tgaatctggg agaagagaag 298260
   catgagaaga catggccgtg agctaaggac atcagcataa gttaaggcag aatctttctc 298320
   tttctcttgt ttgcttgacc ttggatgagt cctcaacttg tttttctcac tgtggtcctg 298380
   agagagaaat tctcggtgaa ggtcactgca aggggtaata atagcaatac gttcatcaga 298440
   taagaggtat aatgaggccc taacaggaaa ctttcctaac aggaaagtga gtgggactgg 298500
   gagtttatag cacagtgatg gctcctcatt gtctcgactt cccattcttt actacttgct 298560
   tgtcatctct tccttttctt ttctccaatc cctgaaaatg cttttgtaaa gtgaggtttt 298620
   gtgcatgctg ttttgaaatg ataataaaag gtagtggaag gaaatcccaa agtaggggag 298680
   aaaagagcta tttttaaaag caaattaata aaagaatcag aatatattct gcatcctgca 298740
   aggtagccct ttgcagtttg ttacaaatat gtcaaaactt tcttatttgc ttatggttta 298800
   aatacaattt tcttctccat tttcatagca ttaaaccgga tgattttctc atctgccctg 298860
   atagacagtt aactactaaa gtttacagag taaaaatgaa agcactaaac tttagaatgt 298920
   taaaatattc atttatgtct gatgggtgga accttcactg aagttgtcca taaaaggcaa 298980
   agactgttag caaaaactcg gtaacccagc agtttagcct ataaatctat attgtaaatg 299040
   ttgagaggtt acaagtgcca tagtcacttg tgaatgagta tgaggttctg tggacacact 299100
   tgacatttca gaatgttctc cttgcctgag ataaccagta gggtgtgcct aacaggtgga 299160
   catctggtcc ctcgggatca tggtgataga aatgattgat ggcgagcccc cctacttcaa 299220
   tgagcctccc ctccaggcga tgcggaggat ccgggacagt ttacctccaa gagtgaagga 299280
   cctacacaag gtgagaaaca gcagctgttg ctaaagttca gaatagtttt tggattttcc 299340
   tgtatctctc tggtttttaa gactggctta ccacatccag caggaagatc ctggtttatg 299400
   agcatggact ttggaaatag tcacttaggt ttgaatctct gttcttctat tcgtttactg 299460
   ggcaagtgac tcagcctttc ctatgccaaa gttcttcatc tgtaaaatgg gttatgattg 299520
   gcctgtatcc caaagggttg gttagaagag taaatcaggt gctctatgcc atactcattc 299580
   attcagcatc tattacatgc tggggacatc taatagcttg ggatgtcacc gtgcatgacc 299640
   ccagcttttc tgactctggg gtctagactc tgcattctag ttggggaaac agatagtaaa 299700
   ccataaacaa acaaataaat aaaataattt cagagtgtgg tacatactat gaaagaaaaa 299760
   catactgtga tgtgacagag tgtgcaggag aagggattga aagctggttg tatgagtctg 299820
   tttttacact actataaaga aatacccaag actgagtaat ttacaaataa agaaggttta 299880
   attaactcac agttccacat gcctggagag gcctcaggat acttacaatc atggcagaag 299940
   gcgaagggga agcaaggcat gtcttacatg gtggcaggag agagagagcg cacaggggaa 300000
   atgccagaca cttataaaac tatcagctct catgataatt ccctcactat cttgagaata 300060
   acatggggta aactgccccc atgacccaat taccttccac caagtccctc ccttgacatg 300120
   tgaggattac aatatgaggt gagatttgag tgggcacaga gagccaaacc ctatcactga 300180
   tttaagctga agagtgaagg aagccctttc tagggaggga tcaaacgagc tgggacctga 300240
   ataggaagaa ggtaccagcc atgcaaagat cttggagaaa aaggtcccag gcacggggag 300300
   catcaagtgg aaaggcctta agccaagaaa gaaggcttac attgctggag taaagtcaga 300360
   aaggggaaaa aggtaggtct tgtcagagag atgggcagga gctagattgt acagggcaat 300420
   gaaggtcatg gttaatataa tgcatgtaag agaccgagtg gggtcttttc tgttttccaa 300480
   ggattgccaa ttaggacgag ctcagaaaat aatgatgctt catccaagaa tcaccactgt 300540
   aggagctgcc agatttccaa aggacttgct cagaggatca tttctgcagg actactacta 300600
   taaacactgg ccccatacac acctctcttc ctatcttatg acaataaaag agagtgtgtg 300660
   gctgcctttt ggaggagaat tatttgtaat ggtaatatct cctccttcct cacccctaaa 300720
   tcttagggaa cagccccagt agcatcactc atataggttg tgtggaccta caaaaacata 300780
   aacaaaaaaa gagagattga aatttttact ggattggact aagtctaaat aatgaaaagt 300840
   ggctgggtgt ggtggctcac gcctgtaatc ccagcactct ggaaggccga ggtgggcgga 300900
   tcacgaggtc aggagatgga gaccatcctg gctaacacgg tgaaacccca tctccactaa 300960
   aaatacaaaa aattagccaa gcatggtggc gggtgcctgt agtcccagct actcaggagg 301020
   ctgaggcagg agaatggcgt gaacctggga ggcagagctt gcagtgagcc cagatcgcac 301080
   cactgcactc cagcctaggt gacacagcga gactacgtct ccaaaaaaaa aaaaaaaagt 301140
   gactaaaaat gtatcatatg aaagtagtct cacacccagt gagtgccaat gttcttcaaa 301200
   accatgtaca agaacaactt taatcctaat ggtttcaaaa aaactggaaa ccagtccgag 301260
   gttgtttccc aaatggataa tgtgtggtat aattggacag tgaatactac acagtaattt 301320
   aaaagatata acttttgcca cacgtcggag atgaatctca cagatacaac actgagtgaa 301380
   agaagccaga tagagaagaa atcaaatata tgactccatt ttggtaaagt acaaaagtaa 301440
   gcaaaactaa gttatgctat aaattaaaat agtggtgacc ttttgcaggg agataggggt 301500
   tggcaggaag gaaacctaaa agtagcttcc tagagtggta gaaaagttct atatcttgat 301560
   aggagtggaa agtaaatagg ggtaaatgta tgtaaaaatc cattgagcca cactcttagt 301620
   atttgtgcag cggaacctgg gcatgctggc acatgcttgt actcccagct actcaggagg 301680
   ctgaggtggg aggatcattt gagcacagga gtttgagcct gggcaacata gtgagacctc 301740
   atctctaaat atatacataa agtaagatat acctttaaaa ggaaaattac aagaccttca 301800
   aagaaaaaaa tgagatgtgt tcgatatcct tacagaagct tgtttctctt aaagaaaggg 301860
   gttccacaga gcctgtgtgg ttgcagttgt tagaaaaata aaacggctcc ctgtttatac 301920
   tatgcttcat caagcacgtg tgaccagcac ctgcacatac taaatatttt gtaatagctg 301980
   ctcttatcac tgtgattgcc actgttattt tatgtagtaa ataagttttc attcttagtt 302040
   gagctgtcat ttacacctat tatttcacac ttgaaaaagg taaggaaaac acgaaatgtt 302100
   aacaaagaca agacatgctt ttgctttcac aacatctttc tccttaaggt aactttctgg 302160
   aatttgaagt tgttgatagg atgaatgggg aaaacatttt ggggactggg ttaggtgtct 302220
   gtgttagtcc tcagattggt atctgtgtgt tttcacaggt ttcttcagtg ctccggggat 302280
   tcctagactt gatgttggtg agggagccct ctcagagagc aacagcccag gaactcctcg 302340
   gacatccatt cttaaaacta gcaggtccac cgtcttgcat cgtccccctc atgagacaat 302400
   acaggcatca ctgagcagag gattcgtgta ggtggcaaag ctagatgagg acatgagaat 302460
   aattcaggag aacaaaagga aacacagaac atgcaaaagg cctgtgcatt ctagaccagc 302520
   caattggtgg gacagcgtga tgaccggcag ggttcaacag accagggcat cttcttgtgt 302580
   cttaaacagg catctctcca ctgacagccg gtgtggtcac ttggagcacg gctttaataa 302640
   gtcattatta tatttttcag cccttcatcc agcaaatcag aaggactcag tacaaactcc 302700
   gttatgatat atcctagcca catgcagggt aacatgtagg attttctata ttgaaagaat 302760
   acttttctgg caaaaaaaaa aaaaaaagaa agaaaggaaa acaaaaagca cttttttctt 302820
   aatggtagca gtataatgta ttttgcaacg aatttgtaat ttttctgtac gatagttttg 302880
   ataatttata gtactttgat gtcatgtagc cattgtatca gttgaagtaa tacttgttta 302940
   ctagaggagt ttgaacaaag cctttcctac ttttttatcc ctttaagaga accaatgatt 303000
   ctttaggaac tttgaatact gaatgactct caatcaccgt cagctttagt aaaatctctt 303060
   tcttatccta acaagtgtct tatttggtgg aagaagaatt aagagtgatg gtgatggtgt 303120
   gcacgtttca ttaatccaac caaaaataat gaaataaaat ttgagccaca gtataccact 303180
   ccttgggata aagttaaata tttttaaaga tcacattttc catgaacgcc tctagtagca 303240
   aaccattctt ttgcacacca caatgtttcc ctcagtgccc tttctcaaat gggtacaatg 303300
   ttcccttgtg gccaaatttc cctcccaggg agcaatttca gtgctaggat cattggattc 303360
   agttcccaaa atagaatgtt tcagtgagac catgagaatt ccaggctcac aaagggagag 303420
   gagagaacag ggcaagacgt ttggtttcat ttgtcaccat ttttaaaact ctgtatgcta 303480
   gcacaccaaa ctcttgtcta tatttacctt tgtaccacag tattaatcgc tattgttcat 303540
   gtatcgtgct ggaagtctga actgactcta gaggatgaat tagcaagagg gtattttacc 303600
   aggtatgatc tgacttcagt tgtgcccatg ttataatgtg tttccgacat aggagagtcg 303660
   tgctgctgtc tagatcttct tgaatgttga taaaaatgaa tgactactac aatacatttt 303720
   gtgttgcttg ttggatgaat ttgcatgtta actgtaggcc aatatagatt tgcctttaaa 303780
   actctggaag agctacatag tcatcattag tttctattaa ttatgcatca gacaaaagcc 303840
   atttgttacc aaactgggaa aacagaggct tttcttaact atttcacata ctgtaacaaa 303900
   tatgaattta aatttgtgat agcgctctgg ttgctctaag cataattaag aatttttgta 303960
   attaataggt tgctaattat ttatcactgc taaaaaggaa aaaaggcata aaatgacctt 304020
   ctactgatta gattttcagt tttctttcaa actggaaatg cctccataaa tatgatctat 304080
   gattttgctt cataaaacag caaatcaatg ttttatgtaa aatattaaag cattaatata 304140
   aatatgtgag aataaaaaca atctaaatcc agaaaatggc agtcctaaat gttcatgaga 304200
   cagattgtat taatttaacc aggactatgt agaagtagaa agaaaagaaa aagaaaatct 304260
   tttttaaacc agaataaaca ttaaaaacta ttgcagaaaa tagtggattt tggattccaa 304320
   acattttcga cagtgtaatg gaaatttttc tgtaattttc ttaccatcgg gtatttttta 304380
   aagtattcat tgagtttacc aaaagttact gtagcttaaa aggttttgtg agcactaact 304440
   attggcagaa actgcatttg caaataaaaa taaatgtttg cctttt          304486

<210>  3
<211>  745
<212>  PRT
<213>  Homo sapiens

<400>  3

Met Leu Pro Phe Ala Pro Gln Asp Glu Pro Trp Asp Arg Glu Met Glu
1               5                   10                  15

Val Phe Ser Gly Gly Gly Ala Ser Ser Gly Glu Val Asn Gly Leu Lys
            20                  25                  30

Met Val Asp Glu Pro Met Glu Glu Gly Glu Ala Asp Ser Cys His Asp
        35                  40                  45

Glu Gly Val Val Lys Glu Ile Pro Ile Thr His His Val Lys Glu Gly
    50                  55                  60

Tyr Glu Lys Ala Asp Pro Ala Gln Phe Glu Leu Leu Lys Val Leu Gly
65                  70                  75                  80

Gln Gly Ser Phe Gly Lys Val Phe Leu Val Arg Lys Lys Thr Gly Pro
                85                  90                  95

Asp Ala Gly Gln Leu Tyr Ala Met Lys Val Leu Lys Lys Ala Ser Leu
            100                 105                 110

Lys Val Arg Asp Arg Val Arg Thr Lys Met Glu Arg Asp Ile Leu Val
        115                 120                 125

Glu Val Asn His Pro Phe Ile Val Lys Leu His Tyr Ala Phe Gln Thr
    130                 135                 140

Glu Gly Lys Leu Tyr Leu Ile Leu Asp Phe Leu Arg Gly Gly Asp Val
145                 150                 155                 160

Phe Thr Arg Leu Ser Lys Glu Val Leu Phe Thr Glu Glu Asp Val Lys
                165                 170                 175

Phe Tyr Leu Ala Glu Leu Ala Leu Ala Leu Asp His Leu His Gln Leu
            180                 185                 190

Gly Ile Val Tyr Arg Asp Leu Lys Pro Glu Asn Ile Leu Leu Asp Glu
        195                 200                 205

Ile Gly His Ile Lys Leu Thr Asp Phe Gly Leu Ser Lys Glu Ser Val
    210                 215                 220

Asp Gln Glu Lys Lys Ala Tyr Ser Phe Cys Gly Thr Val Glu Tyr Met
225                 230                 235                 240

Ala Pro Glu Val Val Asn Arg Arg Gly His Ser Gln Ser Ala Asp Trp
                245                 250                 255

Trp Ser Tyr Gly Val Leu Met Phe Glu Met Leu Thr Gly Thr Leu Pro
            260                 265                 270

Phe Gln Gly Lys Asp Arg Asn Glu Thr Met Asn Met Ile Leu Lys Ala
        275                 280                 285

Lys Leu Gly Met Pro Gln Phe Leu Ser Ala Glu Ala Gln Ser Leu Leu
    290                 295                 300

Arg Met Leu Phe Lys Arg Asn Pro Ala Asn Arg Leu Gly Ser Glu Gly
305                 310                 315                 320

Val Glu Glu Ile Lys Arg His Leu Phe Phe Ala Asn Ile Asp Trp Asp
                325                 330                 335

Lys Leu Tyr Lys Arg Glu Val Gln Pro Pro Phe Lys Pro Ala Ser Gly
            340                 345                 350

Lys Pro Asp Asp Thr Phe Cys Phe Asp Pro Glu Phe Thr Ala Lys Thr
        355                 360                 365

Pro Lys Asp Ser Pro Gly Leu Pro Ala Ser Ala Asn Ala His Gln Leu
    370                 375                 380

Phe Lys Gly Phe Ser Phe Val Ala Thr Ser Ile Ala Glu Glu Tyr Lys
385                 390                 395                 400

Ile Thr Pro Ile Thr Ser Ala Asn Val Leu Pro Ile Val Gln Ile Asn
                405                 410                 415

Gly Asn Ala Ala Gln Phe Gly Glu Val Tyr Glu Leu Lys Glu Asp Ile
            420                 425                 430

Gly Val Gly Ser Tyr Ser Val Cys Lys Arg Cys Ile His Ala Thr Thr
        435                 440                 445

Asn Met Glu Phe Ala Val Lys Ile Ile Asp Lys Ser Lys Arg Asp Pro
    450                 455                 460

Ser Glu Glu Ile Glu Ile Leu Met Arg Tyr Gly Gln His Pro Asn Ile
465                 470                 475                 480

Ile Thr Leu Lys Asp Val Phe Asp Asp Gly Arg Tyr Val Tyr Leu Val
                485                 490                 495

Thr Asp Leu Met Lys Gly Gly Glu Leu Leu Asp Arg Ile Leu Lys Gln
            500                 505                 510

Lys Cys Phe Ser Glu Arg Glu Ala Ser Asp Ile Leu Tyr Val Ile Ser
        515                 520                 525

Lys Thr Val Asp Tyr Leu His Cys Gln Gly Val Val His Arg Asp Leu
    530                 535                 540

Lys Pro Ser Asn Ile Leu Tyr Met Asp Glu Ser Ala Ser Ala Asp Ser
545                 550                 555                 560

Ile Arg Ile Cys Asp Phe Gly Phe Ala Lys Gln Leu Arg Gly Glu Asn
                565                 570                 575

Gly Leu Leu Leu Thr Pro Cys Tyr Thr Ala Asn Phe Val Ala Pro Glu
            580                 585                 590

Val Leu Met Gln Gln Gly Tyr Asp Ala Ala Cys Asp Ile Trp Ser Leu
        595                 600                 605

Gly Val Leu Phe Tyr Thr Met Leu Ala Gly Tyr Thr Pro Phe Ala Asn
    610                 615                 620

Gly Pro Asn Asp Thr Pro Glu Glu Ile Leu Leu Arg Ile Gly Asn Gly
625                 630                 635                 640

Lys Phe Ser Leu Ser Gly Gly Asn Trp Asp Asn Ile Ser Asp Gly Ala
                645                 650                 655

Lys Asp Leu Leu Ser His Met Leu His Met Asp Pro His Gln Arg Tyr
            660                 665                 670

Thr Ala Glu Gln Ile Leu Lys His Ser Trp Ile Thr His Arg Asp Gln
        675                 680                 685

Leu Pro Asn Asp Gln Pro Lys Arg Asn Asp Val Ser His Val Val Lys
    690                 695                 700

Gly Ala Met Val Ala Thr Tyr Ser Ala Leu Thr His Lys Thr Phe Gln
705                 710                 715                 720

Pro Val Leu Glu Pro Val Ala Ala Ser Ser Leu Ala Gln Arg Arg Ser
                725                 730                 735

Met Lys Lys Arg Thr Ser Thr Gly Leu
            740                 745

<210>  4
<211>  719
<212>  PRT
<213>  Homo sapiens

<220>
<221>  2 mRNAs 1 Protein
<222>  (1)..(719)
<223>  Durch alternatives Spleißen des Exon 2 entstehen zwei unterschied
       liche mRNAs, die das gleiche Protein kodieren.

<400>  4

Met Phe Gly Lys Lys Lys Lys Lys Ile Glu Ile Ser Gly Pro Ser Asn
1               5                   10                  15

Phe Glu His Arg Val His Thr Gly Phe Asp Pro Gln Glu Gln Lys Phe
            20                  25                  30

Thr Gly Leu Pro Gln Gln Trp His Ser Leu Leu Ala Asp Thr Ala Asn
        35                  40                  45

Arg Pro Lys Pro Met Val Asp Pro Ser Cys Ile Thr Pro Ile Gln Leu
    50                  55                  60

Ala Pro Met Lys Thr Ile Val Arg Gly Asn Lys Pro Cys Lys Glu Thr
65                  70                  75                  80

Ser Ile Asn Gly Leu Leu Glu Asp Phe Asp Asn Ile Ser Val Thr Arg
                85                  90                  95

Ser Asn Ser Leu Arg Lys Glu Ser Pro Pro Thr Pro Asp Gln Gly Ala
            100                 105                 110

Ser Ser His Gly Pro Gly His Ala Glu Glu Asn Gly Phe Ile Thr Phe
        115                 120                 125

Ser Gln Tyr Ser Ser Glu Ser Asp Thr Thr Ala Asp Tyr Thr Thr Glu
    130                 135                 140

Lys Tyr Arg Glu Lys Ser Leu Tyr Gly Asp Asp Leu Asp Pro Tyr Tyr
145                 150                 155                 160

Arg Gly Ser His Ala Ala Lys Gln Asn Gly His Val Met Lys Met Lys
                165                 170                 175

His Gly Glu Ala Tyr Tyr Ser Glu Val Lys Pro Leu Lys Ser Asp Phe
            180                 185                 190

Ala Arg Phe Ser Ala Asp Tyr His Ser His Leu Asp Ser Leu Ser Lys
        195                 200                 205

Pro Ser Glu Tyr Ser Asp Leu Lys Trp Glu Tyr Gln Arg Ala Ser Ser
    210                 215                 220

Ser Ser Pro Leu Asp Tyr Ser Phe Gln Phe Thr Pro Ser Arg Thr Ala
225                 230                 235                 240

Gly Thr Ser Gly Cys Ser Lys Glu Ser Leu Ala Tyr Ser Glu Ser Glu
                245                 250                 255

Trp Gly Pro Ser Leu Asp Asp Tyr Asp Arg Arg Pro Lys Ser Ser Tyr
            260                 265                 270

Leu Asn Gln Thr Ser Pro Gln Pro Thr Met Arg Gln Arg Ser Arg Ser
        275                 280                 285

Gly Ser Gly Leu Gln Glu Pro Met Met Pro Phe Gly Ala Ser Ala Phe
    290                 295                 300

Lys Thr His Pro Gln Gly His Ser Tyr Asn Ser Tyr Thr Tyr Pro Arg
305                 310                 315                 320

Leu Ser Glu Pro Thr Met Cys Ile Pro Lys Val Asp Tyr Asp Arg Ala
                325                 330                 335

Gln Met Val Leu Ser Pro Pro Leu Ser Gly Ser Asp Thr Tyr Pro Arg
            340                 345                 350

Gly Pro Ala Lys Leu Pro Gln Ser Gln Ser Lys Ser Gly Tyr Ser Ser
        355                 360                 365

Ser Ser His Gln Tyr Pro Ser Gly Tyr His Lys Ala Thr Leu Tyr His
    370                 375                 380

His Pro Ser Leu Gln Ser Ser Ser Gln Tyr Ile Ser Thr Ala Ser Tyr
385                 390                 395                 400

Leu Ser Ser Leu Ser Leu Ser Ser Ser Thr Tyr Pro Pro Pro Ser Trp
                405                 410                 415

Gly Ser Ser Ser Asp Gln Gln Pro Ser Arg Val Ser His Glu Gln Phe
            420                 425                 430

Arg Ala Ala Leu Gln Leu Val Val Ser Pro Gly Asp Pro Arg Glu Tyr
        435                 440                 445

Leu Ala Asn Phe Ile Lys Ile Gly Glu Gly Ser Thr Gly Ile Val Cys
    450                 455                 460

Ile Ala Thr Glu Lys His Thr Gly Lys Gln Val Ala Val Lys Lys Met
465                 470                 475                 480

Asp Leu Arg Lys Gln Gln Arg Arg Glu Leu Leu Phe Asn Glu Val Val
                485                 490                 495

Ile Met Arg Asp Tyr His His Asp Asn Val Val Asp Met Tyr Ser Ser
            500                 505                 510

Tyr Leu Val Gly Asp Glu Leu Trp Val Val Met Glu Phe Leu Glu Gly
        515                 520                 525

Gly Ala Leu Thr Asp Ile Val Thr His Thr Arg Met Asn Glu Glu Gln
    530                 535                 540

Ile Ala Thr Val Cys Leu Ser Val Leu Arg Ala Leu Ser Tyr Leu His
545                 550                 555                 560

Asn Gln Gly Val Ile His Arg Asp Ile Lys Ser Asp Ser Ile Leu Leu
                565                 570                 575

Thr Ser Asp Gly Arg Ile Lys Leu Ser Asp Phe Gly Phe Cys Ala Gln
            580                 585                 590

Val Ser Lys Glu Val Pro Lys Arg Lys Ser Leu Val Gly Thr Pro Tyr
        595                 600                 605

Trp Met Ala Pro Glu Val Ile Ser Arg Leu Pro Tyr Gly Thr Glu Val
    610                 615                 620

Asp Ile Trp Ser Leu Gly Ile Met Val Ile Glu Met Ile Asp Gly Glu
625                 630                 635                 640

Pro Pro Tyr Phe Asn Glu Pro Pro Leu Gln Ala Met Arg Arg Ile Arg
                645                 650                 655

Asp Ser Leu Pro Pro Arg Val Lys Asp Leu His Lys Val Ser Ser Val

660                 665                 670

Leu Arg Gly Phe Leu Asp Leu Met Leu Val Arg Glu Pro Ser Gln Arg
        675                 680                 685

Ala Thr Ala Gln Glu Leu Leu Gly His Pro Phe Leu Lys Leu Ala Gly
    690                 695                 700

Pro Pro Ser Cys Ile Val Pro Leu Met Arg Gln Tyr Arg His His
705                 710                 715